# EUROPÄISCHE PATENTSCHRIFT

(11) **EP 3 055 313 B1**
(45) Veröffentlichungstag und Bekanntmachung des Hinweises auf die Patenterteilung: **22.11.2017**
(21) Anmeldenummer: 14777656.1
(22) Anmeldetag: 02.10.2014
(51) Int. Cl.: C07D 495/04, A61K 31/519, A61P 11/08

(54) **CYCLISCHE THIENOURACIL-CARBOXAMIDE UND IHRE VERWENDUNG**
CYCLIC THIENOURACIL CARBOXAMIDE AND ITS USE
THIENOURACIL CARBOXAMIDE CYCLIQUES ET SON UTILISATION

(30) Priorität: 07.10.2013 EP 13187487; 24.01.2014 EP 14152518
(43) Veröffentlichungstag der Anmeldung: 17.08.2016
(73) Patentinhaber: Bayer Pharma Aktiengesellschaft, 13353 Berlin (DE)
(72) Erfinder: HÄRTER, Michael, 51375 Leverkusen (DE); DELBECK, Martina, 42579 Heiligenhaus (DE); KALTHOF, Bernd, 42329 Wuppertal (DE); LUSTIG, Klemens, 42113 Wuppertal (DE); LINDNER, Niels, 42115 Wuppertal (DE); KAST, Raimund, 42349 Wuppertal (DE); WASNAIRE, Pierre, 40225 Düsseldorf (DE); SÜSSMEIER, Frank, 80992 München (DE)
(74) Vertreter: BIP Patents
(86) Internationale Anmeldenummer: PCT/EP2014/071113
(87) Internationale Veröffentlichungsnummer: WO 2015/052065

(56) Entgegenhaltungen:
- WO-A1-2009/037468
- US-A1- 2007 208 040
- US-B2- 7 947 692

## Beschreibung

Die vorliegende Anmeldung betrifft neue 2,4-Dioxo-1,2,3,4-tetrahydrothieno[2,3-d]pyrimidin-6-carboxamid-Derivate ("Thienouracil"-Carboxamide), Verfahren zu ihrer Herstellung, ihre Verwendung allein oder in Kombinationen zur Behandlung und/oder Prävention von Krankheiten sowie ihre Verwendung zur Herstellung von Arzneimitteln zur Behandlung und/oder Prävention von Krankheiten, insbesondere zur Behandlung und/oder Prävention von Erkrankungen der Lunge und des Herz-Kreislauf-Systems.

Das endogene Purin-Nukleosid Adenosin wird ubiquitär gebildet und moduliert als wichtiges Signalmolekül eine Vielzahl von physiologischen als auch pathophysiologischen Prozessen. Es entsteht zum größeren Teil beim intra- und extrazellulären Abbau von Adenin-Nukleotiden und zum geringeren Teil bei der intrazellulären Hydrolyse von *S*-Adenosyl-Homocystein. Unter physiologischen Bedingungen kann extrazelluläres Adenosin durch die Adenosinkinase wieder zu Adenosinmonophosphat (AMP) phosphoryliert oder durch die Adenosindeaminase zu Inosin umgebaut werden. Die extrazelluläre Konzentration liegt zwischen 30 und 300 nM. Bei Gewebeschäden, bedingt z.B. durch Hypoxie, bei Entzündungsreaktionen und bei oxidativem Stress kommt es zu einer vermehrten Bildung und Akkumulation von Adenosin, so dass die extrazelluläre Konzentration bis auf 15 µM ansteigen kann.

Die biologischen Wirkungen von Adenosin werden über G-Protein-gekoppelte Plasmamembranständige Rezeptoren vermittelt. Derzeit sind vier Adenosin-Rezeptor-Subtypen nachgewiesen: A1-Adenosin-Rezeptor (AIR), A2a-Adenosin-Rezeptor (A2aR), A2b-Adenosin-Rezeptor (A2bR) und A3-Adenosin-Rezeptor (A3R). Der A2b-Rezeptor hat von den vier oben genannten AdenosinRezeptoren die schwächste Affinität für Adenosin. Aus diesem Grunde wird er unter physiologischen Normalbedingungen im Gegensatz zu den anderen Adenosinrezeptoren nicht aktiviert. A1- und A3-Rezeptoren sind an Gi-Proteine gekoppelt, die die Adenylatzyklase hemmen, während A2a- und A2b-Rezeptoren über Gs-Proteine eine Stimulierung der Adenylatzyklase und damit einen intrazellulären Anstieg von cAMP bewirken. Über Gq-Proteine aktivieren sowohl der A1-, der A3- als auch der A2b-Rezeptor die Phospholipase C, welche membranständiges Phosphatidyl-inositol-4,5-bisphosphat in Inositol-1,4,5-triphosphat und Diacylglycerol spaltet. Dies führt wiederum zur Erhöhung der intrazellulären Calcium-Konzentration und Aktivierung weiterer Zielproteine, wie der Proteinkinase C und der MAP-Kinasen.

A2b-Rezeptoren sind auf pulmonalen Epithel- und Glattmuskelzellen, vaskulären Endothel- und Glattmuskelzellen, Fibroblasten sowie Entzündungszellen exprimiert. Die Expression des A2b-Rezeptors an der Zelloberfläche ist ein dynamischer Prozess und wird z.B. durch Hypoxie, inflammatorische Faktoren und freie Radikale stark gesteigert. Die durch Adenosin aktivierten A2b-Rezeptoren führen zur Bildung und Ausschüttung von pro-inflammatorischen und pro-fibrotischen Zytokinen wie z.B. IL-6, IL-4 und IL-8. Studien haben gezeigt, dass der A2b-Rezeptor im chronischen Stadium von Lungenerkrankungen beim Gewebe-Remodeling eine wichtige Rolle spielt und unter anderem die Differenzierung von Fibroblasten in Myofibroblasten fördert, was zu einer verstärkten Synthese und Deposition von Kollagen führt.

In Lungengewebeproben von Patienten mit idiopathischer pulmonaler Fibrose, COPD und pulmonaler Hypertonie assoziiert mit COPD [Zhou et al., PLoS One 5, e9224 (2010); Selmann et al., PLoS One 2, e482 (2007)] sowie in unterschiedlichen Tiermodellen fibro-proliferativer Lungenerkrankungen [Karmouty-Quintana et al., Am. J. Respir. Cell. Mol. Biol., publ. online, 15. Juli 2013; Karmouty-Quintana et al., Faseb J. 26, 2546-2557 (2012); Sun et al., J. Clin. Invest. 116, 2173-2182 (2006)] konnte eine erhöhte Expression des A2b-Rezeptors festgestellt werden. Im Tiermodell der Bleomycin-induzierten Lungenfibrose und pulmonalen Hypertonie an der Maus führte ein genetischer Knock-out des A2b-Rezeptors sowohl zu einer Hemmung der Progression der Lungenfibrose als auch des pulmonalen vaskulären Remodelings und der daraus resultierenden pulmonalen Hypertonie [Karmouty-Quintana et al., Faseb J. 26, 2546-2557 (2012)]. Man vermutet, dass bei der Entwicklung der pulmonalen Hypertonie assoziiert mit Lungenfibrose die durch den A2b-Rezeptor modulierte Freisetzung von unter anderem Endothelin-1 (ET-1) und Interleukin-6 (IL-6) aus den Gefäßzellen eine Rolle spielt. Die Stimulation von humanen pulmonalen arteriellen Endothel- und Glattmuskelzellen mit 5'-(*N*-Ethylcarboxamido)adenosin (NECA), einem Adenosin-Analogon, führt zur Freisetzung von ET-1 und IL-6, die durch A2b-Rezeptor-Hemmung verhindert werden kann [Karmouty-Quintana et al., Faseb J. 26, 2546-2557 (2012)]. Im Lungengewebe und Serum von Patienten mit einer pulmonalen Hypertonie konnten erhöhte Endothelin-1- und IL-6-Spiegel festgestellt werden [Giaid et al., N. Engl. J. Med. 329, 1967-1968 (1993); Steiner et al., Circ. Res. 104, 236-244 (2009)]. Des Weiteren wird vermutet, dass die vom A2b-Rezeptor vermittelte Freisetzung von unter anderem IL-6 und weiteren profibrotischen Mediatoren sowie die Stimulation der Differenzierung von Fibroblasten in Myofibroblasten in der Lunge zur Induktion von Fibrose führt. Die Stimulation von humanen Fibroblasten mit NECA führt zur Freisetzung von IL-6, die durch Hypoxie verstärkt wird und durch A2b-Rezeptor-Hemmung verhindert werden kann. In Patienten mit idiopathischer pulmonaler Fibrose und in Tiermodellen der Lungenfibrose konnte eine erhöhte IL-6-Expression gezeigt werden [Zhong et al., Am. J. Respir. Cell. Mol. Biol. 32, 2-8 (2005); Cavarra et al., Am. J. Physiol. Lung Cell. Mol. Physiol. 287, L1186-L1192 (2004)].

Der A2b-Rezeptor spielt auch beim Gewebe-Remodeling nach Myokardinfarkt eine wichtige Rolle. Im Tiermodell der permanenten Ligatur der Koronararterie bei der Maus führte eine Hemmung des A2b-Rezeptors zu einer Reduktion der Caspase-1-Aktivität und der eingewanderten Entzündungszellen im Herzgewebe sowie der Zytokine und Adhäsionsmoleküle im Plasma und zu einer Verbesserung der systolischen und diastolischen Herzfunktion [Toldo et al., J. Pharmacol. Exp. Ther. 343, 587-595 (2012)].

Es wird daher angenommen, dass der A2b-Rezeptor bei vielen Erkrankungen, Verletzungen und pathologischen Veränderungen, deren Entstehung und/oder Progression mit einem entzündlichen Geschehen und/oder einem proliferativen und fibro-proliferativen Gewebe- und Gefäßumbau in Zusammenhang steht, eine wichtige Rolle spielt. Dies können insbesondere Erkrankungen und/ oder Schädigungen der Lunge, des Herz-Kreislauf-Systems oder der Niere sein, oder es kann sich hierbei um eine Erkrankung des Blutes, eine Krebs-Erkrankung oder um andere entzündliche Erkrankungen handeln.

In diesem Zusammenhang zu nennende Erkrankungen und Schädigungen der Lunge sind insbesondere die idiopathische Lungenfibrose, die pulmonale Hypertonie, das Bronchiolitis obliterans-Syndrom (BOS), die chronisch-obstruktive Lungenerkrankung (COPD), Asthma und zystische Fibrose. Erkrankungen und Schädigungen des Herz-Kreislauf-Systems, in denen der A2b-Rezeptor involviert ist, sind zum Beispiel Gewebeveränderungen nach einem Myokardinfarkt und bei der Herzinsuffizienz. Erkrankungen der Niere sind zum Beispiel Niereninsuffizienz und Nierenversagen. Eine Erkrankung des Blutes ist zum Beispiel die Sichelzellanämie. Beispiele für einen Gewebeab- und -umbau bei Krebsprozessen sind das Einwandern von Krebszellen in das gesunde Gewebe (Metastasenbildung) und die Neuausbildung von versorgenden Blutgefäßen (Neo-Angiogenese). Eine andere entzündliche Krankheit, bei denen der A2b-Rezeptor eine Rolle spielt, ist zum Beispiel die Multiple Sklerose.

Die idiopathische Lungenfibrose oder idiopathische pulmonale Fibrose (IPF) ist eine progrediente Lungenerkrankung, die unbehandelt durchschnittlich innerhalb von 2.5 bis 3.5 Jahren nach Diagnosestellung zum Tode führt. Die Patienten sind zum Zeitpunkt der Diagnosestellung meist älter als 60 Jahre, und Männer sind etwas häufiger betroffen als Frauen. Der Krankheitsbeginn der IPF ist schleichend und durch eine zunehmende Atemnot und trockenen Reizhusten gekennzeichnet. IPF gehört zur Gruppe der idiopathischen interstitiellen Pneumonien (IIP), einer heterogenen Gruppe von Lungenerkrankungen, die durch Fibrose und Inflammation unterschiedlichen Grades charakterisiert sind und die mit Hilfe klinischer, bildgebender und feingeweblicher Kriterien unterschieden werden. Innerhalb dieser Gruppe hat die idiopathische pulmonale Fibrose aufgrund ihrer Häufigkeit und des aggressiven Verlaufs eine besondere Bedeutung [Ley et al., Am. J. Respir. Crit. Care Med. 183, 431-440 (2011)]. Die IPF kann entweder sporadisch oder familiär gehäuft auftreten. Die Ursachen sind derzeit nicht geklärt. In den letzten Jahren wurden jedoch zahlreiche Hinweise dafür gefunden, dass eine chronische Schädigung des Alveolarepithels zur Freisetzung von profibrotischen Zytokinen/Mediatoren führt, gefolgt von einer gesteigerten Fibroblastenproliferation und einer vermehrten Kollagenfaserbildung, wodurch es zu einer fleckenförmigen Fibrose und der typischen honigwabenartigen Struktur der Lunge kommt [Strieter et ad.,Chest 136, 1364-1370 (2009)]. Die klinischen Folgen der Fibrosierung sind eine Abnahme der Elastizität des Lungengewebes, eine Verminderung der Diffusionskapazität sowie die Entwicklung einer schweren Hypoxie. Lungenfunktionell kann entsprechend eine Verschlechterung der forcierten Vitalkapazität (FVC) und der Diffusionskapazität (DLCO) festgestellt werden. Wesentliche und prognostisch bedeutende Komorbiditäten der IPF sind die akute Exazerbation und die pulmonale Hypertonie [Beck et al., Pneumologe 10, 105-111 (2013)]. Die Prävalenz der pulmonalen Hypertonie bei interstitiellen Lungenerkrankungen liegt bei 10-40% [Lettieri et al., Chest 129, 746-752 (2006); Behr et al., Eur. Respir. J. 31, 1357-1367 (2008)]. Es gibt gegenwärtig keine kurative Behandlung für die IPF - mit Ausnahme der Lungentransplantation.

Die Pulmonale Hypertonie (PH) ist eine progrediente Lungenerkrankung, die unbehandelt durchschnittlich innerhalb von 2.8 Jahren nach Diagnosestellung zum Tode führt. Definitionsgemäß liegt bei einer chronischen pulmonalen Hypertonie ein pulmonal-arterieller Mitteldruck (mPAP) von > 25 mmHg in Ruhe oder > 30 mmHg unter Belastung vor (Normalwert < 20 mmHg). Die Pathophysiologie der pulmonalen Hypertonie ist gekennzeichnet durch Vasokonstriktion und ein Remodeling der Pulmonalgefäße. Bei der chronischen PH kommt es zu einer Neomuskularisierung primär nicht muskularisierter Lungengefäße, und die Gefäßmuskulatur der bereits muskularisierten Gefäße nimmt an Umfang zu. Durch diese zunehmende Obliteration der Lungenstrombahn kommt es zu einer progredienten Belastung des rechten Herzens, die zu einer verminderten Auswurfleistung des rechten Herzens führt und letztlich in einem Rechtsherzversagen endet [M. Humbert et al., J. Am. Coll. Cardiol. 2004, 43, 13S-24S]. Wenn auch die idiopathische (oder primäre) pulmonal-arterielle Hypertonie (IPAH) eine sehr seltene Erkrankung ist, so ist die sekundäre pulmonale Hypertonie (non-PAH PH, NPAHPH) weit verbreitet, und es wird zur Zeit angenommen, dass PH die dritthäufigste kardiovaskuläre Krankheitsgruppe nach koronarer Herzkrankheit und systemischem Bluthochdruck ist [Naeije, in: A. J. Peacock et al. (Eds.), Pulmonary Circulation. Diseases and their treatment, 3rd edition, Hodder Arnold Publ., 2011, S. 3]. Die Einteilung der pulmonalen Hypertonie in verschiedene Gruppen gemäß der jeweiligen Ätiologie erfolgt seit 2008 nach der Dana Point-Klassifikation [D. Montana und G. Simonneau, in: A. J. Peacock et al. (Eds.), Pulmonary Circulation. Diseases and their treatment, 3rd edition, Hodder Arnold Publ., 2011, S. 197-206].

Trotz aller Fortschritte in der Therapie der PH gibt es bisher keine Aussicht auf Heilung dieser schwerwiegenden Erkrankung. Auf dem Markt befindliche Therapien (z.B. Prostacyclin-Analoga, Endothelinrezeptor-Antagonisten, Phosphodiesterase-Inhibitoren) sind in der Lage, die Lebensqualität, die körperliche Belastbarkeit und die Prognose der Patienten zu verbessern. Hierbei handelt es sich um systemisch applizierte, primär hämodynamisch wirkende Therapieprinzipien, die den Gefäßtonus beeinflussen. Die Anwendbarkeit dieser Medikamente ist durch die z. T. gravierenden Nebenwirkungen und/oder aufwendigen Applikationsformen eingeschränkt. Der Zeitraum, über den unter einer spezifischen Monotherapie die klinische Situation der Patienten stabilisiert oder verbessert werden kann, ist begrenzt (z.B. aufgrund einer Toleranzentwicklung). Es erfolgt schließlich eine Therapieeskalation und somit eine Kombinationstherapie, bei der mehrere Medikamente gleichzeitig gegeben werden müssen. Zur Zeit sind diese Standardtherapeutika nur zur Behandlung der pulmonal-arteriellen Hypertonie (PAH) zugelassen. Bei sekundären Formen der PH, wie z.B. PH-COPD, scheiterten diese Therapieprinzipien (z.B. Sildenafil, Bosentan) in klinischen Studien, da sie infolge einer unselektiven Vasodilatation zu einer Absenkung (Entsättigung) des arteriellen Sauerstoffgehalts bei den Patienten führten. Ursache hierfür ist wahrscheinlich eine ungünstige Beeinflussung der Ventilations-Perfusions-Anpassung innerhalb der Lunge bei heterogenen Lungenerkrankungen aufgrund der systemischen Gabe unselektiver Vasodilatatoren [I. Blanco et al., Am. J. Respir. Crit. Care Med. 2010, 181, 270-278; D. Stolz et al., Eur. Respir. J. 2008, 32, 619-628].

Neue Kombinationstherapien sind eine der aussichtsreichsten zukünftigen Therapieoptionen zur Behandlung der pulmonalen Hypertonie. In diesem Zusammenhang ist die Erkundung neuer pharmakologischer Mechanismen zur Behandlung der PH von besonderem Interesse [Ghofrani et al., Herz 2005, 30, 296-302; E. B. Rosenzweig, Expert Opin. Emerging Drugs 2006, 11, 609-619; T. Ito et al., Curr. Med. Chem. 2007, 14, 719-733]. Vor allem solche neuen Therapieansätze, die mit den bereits auf dem Markt befindlichen Therapiekonzepten kombinierbar sind, könnten Grundlage einer effizienteren Behandlung sein und somit einen großen Vorteil für die Patienten bringen.

Im Sinne der vorliegenden Erfindung schließt der Begriff pulmonale Hypertonie sowohl primäre als auch sekundäre Unterformen (NPAHPH) ein, wie sie nach der Dana Point-Klassifikation gemäß ihrer jeweiligen Ätiologie definiert worden sind [D. Montana und G. Simonneau, in: A. J. Peacock et al. (Eds.), Pulmonary Circulation. Diseases and their treatment, 3rd edition, Hodder Arnold Publ., 2011, S. 197-206; Hoeper et al., J. Am. Coll. Cardiol., 2009, 54 (1), Suppl. S, S85-S96]. Hierzu gehört insbesondere in Gruppe 1 die pulmonal-arterielle Hypertonie (PAH), zu der unter anderem die idiopathischen und die familiären Formen zählen (IPAH bzw. FPAH). Des weiteren umfasst PAH auch die persistierende pulmonale Hypertonie bei Neugeborenen sowie die assoziierte pulmonal-arterielle Hypertonie (APAH), welche assoziiert ist mit Kollagenosen, kongenitalen systemisch-pulmonalen Shuntvitien, portaler Hypertension, HIV-Infektionen, der Einnahme bestimmter Drogen und Medikamente (z.B. von Appetitzüglern), mit Erkrankungen mit einer signifikanten venösen/kapillären Beteiligung wie der pulmonal-venookklusiven Erkrankung und der pulmonal-kapillären Hämangiomatose, oder mit anderen Erkrankungen wie Schilddrüsenerkrankungen, Glykogenspeicherkrankheiten, Morbus Gaucher, hereditärer Teleangiektasie, Hämoglobinopathien, myeloproliferativen Erkrankungen und Splenektomie. In Gruppe 2 der Dana Point-Klassifikation werden PH-Patienten mit einer ursächlichen Linksherzerkrankung, wie ventrikulären, atrialen oder valvulären Erkrankungen, zusammengefasst. Gruppe 3 umfasst Formen der pulmonalen Hypertonie, die mit einer Lungenerkrankung, wie z.B. chronisch-obstruktiver Lungenerkrankung (COPD), interstitieller Lungenkrankheit (ILD), Lungenfibrose (IPF), und/oder einer Hypoxämie (z.B. Schlafapnoe-Syndrom, alveoläre Hypoventilation, chronische Höhenkrankheit, anlagebedingte Fehlbildungen) assoziiert sind. Zur Gruppe 4 zählen PH-Patienten mit chronischthrombotischen und/oder embolischen Erkrankungen, z.B. bei thromboembolischer Obstruktion von proximalen und distalen Lungenarterien (CTEPH) oder bei nicht-thrombotischen Embolisierungen (z.B. infolge von Tumorerkrankungen, Parasiten, Fremdkörpern). Seltenere Formen der pulmonalen Hypertonie, wie z.B. bei Patienten mit Sarkoidose, Histiozytose X oder Lymphangiomatose, sind in der Gruppe 5 zusammengefasst.

Beim Bronchiolitis obliterans-Syndrom (BOS) handelt es sich um eine chronische Abstoßungsreaktion nach erfolgter Lungentransplantation. Innerhalb der ersten fünf Jahre nach Lungentransplantation sind ca. 50-60% aller Patienten, innerhalb der ersten neun Jahre über 90% der Patienten betroffen [Estenne et al., Am. J. Respir. Crit. Care Med. 166, 440-444 (2003)]. Die Ursache der Erkrankung ist nicht geklärt. Trotz vieler Fortschritte bei der Behandlung von Transplantationspatienten haben sich die BOS-Fallzahlen in den vergangenen Jahren kaum verändert. Das BOS ist die wichtigste langfristige Komplikation bei Lungentransplantationen und gilt als Hauptgrund dafür, dass die Überlebensraten nach wie vor deutlich unter denen anderer Organtransplantationen liegen. Beim BOS handelt es sich um ein entzündliches Geschehen, das mit Veränderungen des Lungengewebes einhergeht, die vor allem die kleinen Atemwege betreffen. Die Schädigung und entzündlichen Veränderungen der Epithelzellen sowie der subepithelialen Strukturen der kleineren Atemwege führen aufgrund einer nicht effektiven Regeneration des Epithels und einer aberrierenden Gewebereparation zu einer exzessiven Fibroproliferation. Es kommt zur Vernarbung und schließlich Zerstörung der Bronchiolen sowie zu Pfröpfen von Granulationsgewebe in den kleinen Atemwegen und Alveolen, gelegentlich auch mit vaskulärer Beteiligung. Die Diagnose wird aufgrund der Lungenfunktion gestellt. Beim BOS kommt es zu einer Verschlechterung des FEV1 im Vergleich zum Durchschnitt der zwei besten postoperativ gemessenen Werte. Gegenwärtig gibt es keine kurative Behandlung für BOS. Ein Teil der Patienten verbessert sich unter intensivierter Immunsuppression, bei den nicht darauf ansprechenden Patienten kommt es zu einer anhaltenden Verschlechterung, so dass eine erneute Transplantation (Retransplantation) indiziert ist.

Die chronisch-obstruktive Lungenerkrankung (COPD) ist eine langsam fortschreitende Lungenerkrankung, die durch eine Behinderung der Atemströmung charakterisiert ist, welche durch ein Lungenemphysem und/oder eine chronische Bronchitis hervorgerufen wird. Die ersten Symptome der Erkrankung zeigen sich in der Regel ab dem vierten bis fünften Lebensjahrzehnt. In den darauffolgenden Lebensjahren verschlimmert sich häufig die Kurzatmigkeit und es manifestiert sich Husten, verbunden mit einem ausgiebigen und stellenweise eitrigen Auswurf und einer Stenose-Atmung bis hin zu einer Atemnot (Dyspnoe). COPD ist in erster Linie eine Krankheit von Rauchern: Rauchen ist verantwortlich für 90% aller COPD-Fälle und 80-90% aller COPD-Todesfälle. COPD ist ein großes medizinisches Problem und stellt weltweit die sechsthäufigste Todesursache dar. Von den über 45-jährigen Menschen sind ca. 4-6% betroffen. Obwohl die Behinderung der Atemströmung nur partiell und zeitlich befristet sein kann, ist COPD nicht heilbar. Behandlungsziel ist folglich eine Verbesserung der Lebensqualität, die Linderung der Symptome, die Verhinderung akuter Verschlechterungen und die Verlangsamung der fortschreitenden Beeinträchtigung der Lungenfunktion. Bestehende Pharmakotherapien, die sich seit den letzten zwei bis drei Jahrzehnten kaum geändert haben, sind das Verwenden von Bronchodilatoren, um blockierte Atemwege zu öffnen, und in bestimmten Situationen Kortikosteroide, um die Entzündung der Lunge einzudämmen [P. J. Barnes, N. Engl. J. Med. 343, 269-280 (2000)]. Die chronische Entzündung der Lunge, hervorgerufen durch Zigarettenrauch oder andere Reizstoffe, ist die treibende Kraft der Krankheitsentwicklung. Der zugrunde liegende Mechanismus beinhaltet Immunzellen, die im Zuge der inflammatorischen Reaktion der Lunge Proteasen und verschiedene Zytokine ausschütten, die zu einem Lungenemphysem und Remodeling der Bronchien führen. Die Aufgabe der vorliegenden Erfindung liegt in der Bereitstellung neuer Substanzen, die als potente und selektive Antagonisten des Adenosin-A2b-Rezeptors wirken und sich als solche zur Behandlung und/oder Prävention insbesondere von Erkrankungen der Lunge und des Herz-Kreislauf-Systems eignen.

Aus WO 98/54190-A1 und WO 00/12514-A1 sind Thieno[2,3-d]pyrimidindione (Thienouracile) mit immunsuppressiver Aktivität bekannt. In WO 02/064598-A1 und WO 2004/014916-A1 werden bicyclische Pyrimidin-Derivate als Inhibitoren von Matrix-Metalloproteinasen (MMPs), insbesondere von MMP-13, beschrieben. Weitere bicyclische Pyrimidin-Derivate werden in WO 2005/ 117890-A2 als CCR2-Antagonisten zur Behandlung insbesondere von entzündlichen Erkrankungen offenbart, und in EP 1 847 541-A1 werden bicyclische Pyrimidin-Derivate als GnRH-Antagonisten zur Behandlung Hormon-abhängiger Erkrankungen beansprucht. In US 7 928 111-B2 werden Thienopyrimidinon-Derivate mit geschmacksverstärkenden Eigenschaften beschrieben. Aus WO 2007/103776-A2 sind Thieno[2,3-d]pyrimidindione als Antagonisten des Adenosin A2a-Rezeptors bekannt, welche insbesondere zur Behandlung von ZNS- und Sucht-Erkrankungen geeignet sind. WO 2009/037468 offenbart 2-Amino-4-carboxamido-thieno[3,2-d]pyrimidine als Adenosin A2b-Rezeptor-Antagonisten zur Behandlung von COPD. In WO 2013/071169-A1 werden Thieno[2,3-d]pyrimidindione als ACC-Inhibitoren zur Behandlung von Infektionen und metabolischen Erkrankungen beansprucht.

Gegenstand der vorliegenden Erfindung sind Verbindungen der allgemeinen Formel (I) 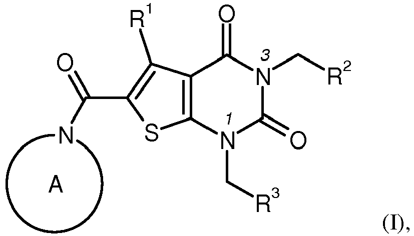 in welcher
- R¹: für Wasserstoff, Methyl oder Ethyl steht, wobei Methyl und Ethyl bis zu dreifach mit Fluor substituiert sein können,
- R²: für Methyl, Ethyl, *n*-Propyl, Isopropyl, Cyclopropyl, Cyclopropylmethyl, 2,2-Difluorvinyl, 3,3-Difluorallyl oder Propargyl oder für eine Gruppe der Formel 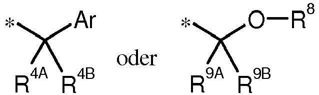 steht,
worin * die Verknüpfung zur CH₂-Gruppe markiert,
Ar Phenyl oder 5- oder 6-gliedriges Heteroaryl mit bis zu zwei Ring-Stickstoffatomen bedeutet,
wobei Phenyl und Heteroaryl ein- oder zweifach, gleich oder verschieden, mit einem Rest ausgewählt aus der Reihe Fluor, Chlor, Methyl, Trifluormethyl, Methoxy und Trifluormethoxy substituiert sein können,
R^{4A} Wasserstoff, Fluor oder Methyl bedeutet,
R^{4B} Wasserstoff, Fluor, Methyl, Trifluormethyl, Hydroxy oder Methoxy bedeutet,
oder
R^{4A} und R^{4B} miteinander verknüpft sind und gemeinsam mit dem Kohlenstoffatom, an das sie gebunden sind, einen Cyclopropyl- oder Cyclobutyl-Ring bilden,
R⁸ Methyl oder Trifluormethyl bedeutet
und
R^{9A} und R^{9B} unabhängig voneinander Wasserstoff, Methyl oder Trifluormethyl bedeuten,
R³ für (C₁-C₆)-Alkyl, (C₂-C₆)-Alkenyl, (C₃-C₇)-Cycloalkyl oder [(C₃-C₇)-Cycloalkyl]methyl steht,
wobei Alkyl und Alkenyl bis zu dreifach und Cycloalkyl bis zu zweifach mit Fluor substituiert sein können
und
wobei in Alkyl und Cycloalkyl bis zu zwei CH₂-Gruppen gegen -O- oder -S- ausgetauscht sein können, mit der Maßgabe, dass sich zwischen solchen Heteroatomen, einschließlich des Uracil-N¹-Atoms, mindestens zwei Kohlenstoffatome befinden,
und
der Ring A für einen mono- oder bicyclischen Aza-Heterocyclus der Formel 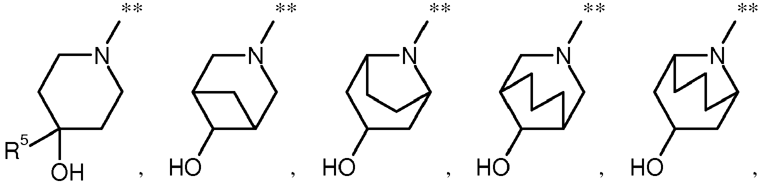 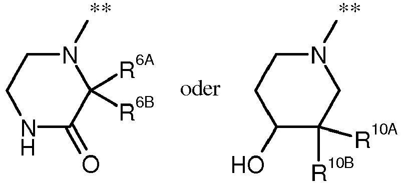 steht, worin ** die Verknüpfung zur Carbonylgruppe markiert,
- R⁵: Wasserstoff, Methyl, Trifluormethyl, Hydroxymethyl oder Ethyl bedeutet,
- R^{6A} und R^{6B}: unabhängig voneinander Wasserstoff, Methyl oder Ethyl bedeuten,
- R^{10A}: Methyl, Ethyl, Hydroxy oder Methoxy bedeutet
und
- R^{10B}: Wasserstoff, Methyl oder Ethyl bedeutet,
sowie ihre Salze, Solvate und Solvate der Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung, Reinigung oder Lagerung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Benzolsulfonsäure, Toluolsulfonsäure, Naphthalindisulfonsäure, Ameisensäure, Essigsäure, Trifluoressigsäure, Propionsäure, Bernsteinsäure, Fumarsäure, Maleinsäure, Milchsäure, Weinsäure, Äpfelsäure, Zitronensäure, Gluconsäure, Benzoesäure und Embonsäure.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomere und Diastereomere und ihre jeweiligen Mischungen. Aus solchen Mischungen von Enantiomeren und/ oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler bzw. chiraler Phase.

Sofern die erfindungsgemäßen Verbindungen in tautomeren Formen vorkommen können, umfasst die vorliegende Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod, wie ²H (Deuterium), ³H (Tritium), ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁹I und ¹³¹I. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoff-Verteilung im Körper; aufgrund der vergleichsweise leichten Herstell- und Detektierbarkeit sind hierfür insbesondere mit ³H- oder ¹⁴C-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise zu einer Verlängerung der Halbwertszeit im Körper oder zu einer Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach allgemein gebräuchlichen, dem Fachmann bekannten Verfahren hergestellt werden, so beispielsweise nach den weiter unten beschriebenen Methoden und den bei den Ausführungsbeispielen wiedergegebenen Vorschriften, indem hierbei entsprechende isotopische Modifikationen der jeweiligen Reagentien und/oder Ausgangsverbindungen eingesetzt werden.

Außerdem umfasst die vorliegende Erfindung auch Prodrugs der erfindungsgemäßen Verbindungen. Der Begriff "Prodrugs" bezeichnet hierbei Verbindungen, welche selbst biologisch aktiv oder inaktiv sein können, jedoch während ihrer Verweilzeit im Körper auf beispielsweise metabolischem oder hydrolytischem Wege zu erfindungsgemäßen Verbindungen umgesetzt werden.

Im Rahmen der vorliegenden Erfindung haben die Substituenten und Reste, soweit nicht anders spezifiziert, die folgende Bedeutung:
(C₁-C₆)-Alkyl und (C₂-C₄)-Alkyl stehen im Rahmen der Erfindung für einen geradkettigen oder verzweigten Alkylrest mit 1 bis 6 bzw. 2 bis 4 Kohlenstoffatomen. Bevorzugt ist ein geradkettiger oder verzweigter Alkylrest mit 2 bis 4 Kohlenstoffatomen. Beispielhaft und vorzugsweise seien genannt: Methyl, Ethyl, *n*-Propyl, Isopropyl, *n*-Butyl, Isobutyl, *sec.*-Butyl, *tert.*-Butyl, *n*-Pentyl, 2-Pentyl, 3-Pentyl, Neopentyl, *n*-Hexyl, 2-Hexyl und 3-Hexyl.
(C₂-C₆)-Alkenyl und (C₂-C₄)-Alkenyl stehen im Rahmen der Erfindung für einen geradkettigen oder verzweigten Alkenylrest mit einer Doppelbindung und 2 bis 6 bzw. 2 bis 4 Kohlenstoffatomen. Bevorzugt ist ein geradkettiger oder verzweigter Alkenylrest mit 2 bis 4 Kohlenstoffatomen. Beispielhaft und vorzugsweise seien genannt: Vinyl, Prop-1-en-1-yl, Prop-2-en-1-yl (Allyl), Prop-1-en-2-yl (Isopropenyl), 2-Methylprop-2-en-1-yl, But-1-en-1-yl, But-1-en-2-yl, But-2-en-1-yl, But-2-en-2-yl, But-3-en-1-yl, Pent-2-en-1-yl, Pent-3-en-1-yl, Pent-4-en-1-yl, 3-Methylbut-2-en-1-yl und 4-Methylpent-3-en-1-yl.
(C₃-C₇)-Cycloalkyl und (C₃-C₆)-Cycloalkyl stehen im Rahmen der Erfindung für eine monocyclische, gesättigte Cycloalkylgruppe mit 3 bis 7 bzw. 3 bis 6 Kohlenstoffatomen. Bevorzugt ist ein Cycloalkylrest mit 3 bis 6 Kohlenstoffatomen. Beispielhaft und vorzugsweise seien genannt: Cyclopropyl, Cyclobutyl, Cyclopentyl, Cyclohexyl und Cycloheptyl.
5-gliedriges Heteroaryl in der Definition der Gruppe Ar steht für einen aromatischen Heterocyclus (Heteroaromaten) mit insgesamt 5 Ringatomen, der ein oder zwei Ring-Stickstoffatome enthält und über ein Ring-Kohlenstoffatom oder ein Ring-Stickstoffatom verknüpft ist. Beispielhaft seien genannt: Pyrrolyl, Pyrazolyl und Imidazolyl. Bevorzugt ist Imidazolyl.
6-gliedriges Heteroaryl in der Definition der Gruppe Ar steht für einen aromatischen Heterocyclus (Heteroaromaten) mit insgesamt 6 Ringatomen, der ein oder zwei Ring-Stickstoffatome enthält und über ein Ring-Kohlenstoffatom verknüpft ist. Beispielhaft seien genannt: Pyridyl, Pyrimidinyl, Pyridazinyl und Pyrazinyl. Bevorzugt ist Pyridyl.

Im Rahmen der vorliegenden Erfindung gilt, dass für alle Reste, die mehrfach auftreten, deren Bedeutung unabhängig voneinander ist. Wenn Reste in den erfindungsgemäßen Verbindungen substituiert sind, können die Reste, soweit nicht anders spezifiziert, ein- oder mehrfach substituiert sein. Eine Substitution mit einem oder mit zwei gleichen oder verschiedenen Substituenten ist bevorzugt. Besonders bevorzugt ist die Substitution mit einem Substituenten.

In einer bestimmten Ausführungsform umfasst die vorliegende Erfindung Verbindungen der Formel (I), in welcher
- R¹: für Wasserstoff, Methyl, Difluormethyl, Trifluormethyl oder Ethyl steht,
- R²: für Methyl, Ethyl, *n*-Propyl, Isopropyl, Cyclopropyl, Cyclopropylmethyl oder Propargyl oder für eine Gruppe der Formel 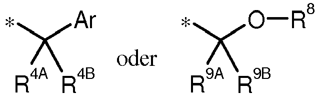 steht, worin * die Verknüpfung zur CH₂-Gruppe markiert,
Ar Phenyl oder 5- oder 6-gliedriges Heteroaryl mit bis zu zwei Ring-Stickstoffatomen bedeutet,
wobei Phenyl und Heteroaryl ein- oder zweifach, gleich oder verschieden, mit einem Rest ausgewählt aus der Reihe Fluor, Chlor, Methyl, Trifluormethyl, Methoxy und Trifluormethoxy substituiert sein können,
R^{4A} Wasserstoff, Fluor oder Methyl bedeutet,
R^{4B} Wasserstoff, Fluor, Methyl, Trifluormethyl, Hydroxy oder Methoxy bedeutet,
oder
R^{4A} und R^{4B} miteinander verknüpft sind und gemeinsam mit dem Kohlenstoffatom, an das sie gebunden sind, einen Cyclopropyl- oder Cyclobutyl-Ring bilden,
R⁸ Methyl oder Trifluormethyl bedeutet
und
R^{9A} und R^{9B} unabhängig voneinander Wasserstoff, Methyl oder Trifluormethyl bedeuten,
- R³: für (C₁-C₆)-Alkyl, (C₂-C₆)-Alkenyl, (C₃-C₇)-Cycloalkyl oder [(C₃-C₇)-Cycloalkyl]methyl steht,
wobei Alkyl und Alkenyl bis zu dreifach und Cycloalkyl bis zu zweifach mit Fluor substituiert sein können
und
wobei in Alkyl und Cycloalkyl bis zu zwei CH₂-Gruppen gegen -O- oder -S- ausgetauscht sein können, mit der Maßgabe, dass sich zwischen solchen Heteroatomen, einschließlich des Uracil-N¹-Atoms, mindestens zwei Kohlenstoffatome befinden,
und der Ring A für einen mono- oder bicyclischen Aza-Heterocyclus der Formel 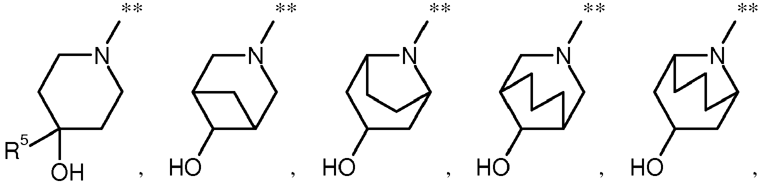 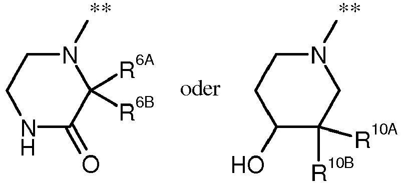 steht, worin ** die Verknüpfung zur Carbonylgruppe markiert,
- R⁵: Wasserstoff, Methyl, Trifluormethyl, Hydroxymethyl oder Ethyl bedeutet,
- R^{6A} und R^{6B}: unabhängig voneinander Wasserstoff, Methyl oder Ethyl bedeuten,
- R^{10A}: Methyl oder Ethyl bedeutet
und
- R^{10B}: Wasserstoff, Methyl oder Ethyl bedeutet,
sowie ihre Salze, Solvate und Solvate der Salze.

In einer weiteren Ausführungsform umfasst die vorliegende Erfindung Verbindungen der Formel (I), in welcher
- R¹: für Wasserstoff, Methyl, Difluormethyl oder Trifluormethyl steht,
- R²: für Methyl, Ethyl, n-Propyl oder Isopropyl oder für eine Gruppe der Formel 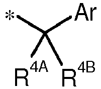 steht, worin * die Verknüpfung zur CH₂-Gruppe markiert,
Ar Phenyl oder 6-gliedriges Heteroaryl mit bis zu zwei Ring-Stickstoffatomen bedeutet,
wobei Phenyl und Heteroaryl ein- oder zweifach, gleich oder verschieden, mit einem Rest ausgewählt aus der Reihe Fluor, Chlor, Methyl, Trifluormethyl, Methoxy und Trifluormethoxy substituiert sein können,
R^{4A} Wasserstoff, Fluor oder Methyl bedeutet,
R^{4B} Wasserstoff, Fluor, Methyl oder Methoxy bedeutet,
oder
R^{4A} und R^{4B} miteinander verknüpft sind und gemeinsam mit dem Kohlenstoffatom, an das sie gebunden sind, einen Cyclopropyl- oder Cyclobutyl-Ring bilden,
- R³: für (C₁-C₆)-Alkyl, (C₂-C₆)-Alkenyl, (C₃-C₇)-Cycloalkyl oder [(C₃-C₇)-Cycloalkyl]methyl steht,
wobei Alkyl und Alkenyl bis zu dreifach und Cycloalkyl bis zu zweifach mit Fluor substituiert sein können
und
wobei in Alkyl und Cycloalkyl bis zu zwei CH₂-Gruppen gegen -O- oder -S- ausgetauscht sein können, mit der Maßgabe, dass sich zwischen solchen Heteroatomen, einschließlich des Uracil-N¹-Atoms, mindestens zwei Kohlenstoffatome befinden,
und
der Ring A für einen mono- oder bicyclischen Aza-Heterocyclus der Formel 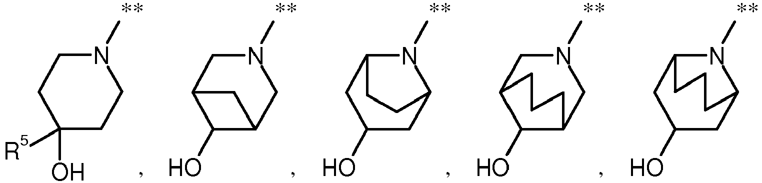 oder 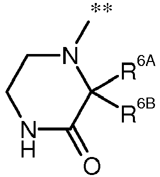 steht, worin ** die Verknüpfung zur Carbonylgruppe markiert,
- R⁵: Wasserstoff, Methyl, Trifluormethyl oder Hydroxymethyl bedeutet
und
- R^{6A} und R^{6B}: unabhängig voneinander Wasserstoff, Methyl oder Ethyl bedeuten,
sowie ihre Salze, Solvate und Solvate der Salze.

Bevorzugt im Rahmen der vorliegenden Erfindung sind Verbindungen der Formel (I), in welcher
- R¹: für Methyl, Difluormethyl, Trifluormethyl, Ethyl oder 1-Fluorethyl steht,
- R²: für Methyl, Ethyl, n-Propyl, Isopropyl, 3,3-Difluorallyl oder Propargyl oder für eine Gruppe der Formel 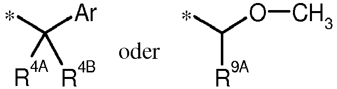 steht, worin * die Verknüpfung zur CH₂-Gruppe markiert,
Ar Phenyl oder Pyridyl bedeutet,
wobei Phenyl und Pyridyl mit Fluor, Chlor, Methyl oder Methoxy substituiert sein können,
R^{4A} Wasserstoff, Fluor oder Methyl bedeutet,
R^{4B} Wasserstoff, Fluor, Methyl, Hydroxy oder Methoxy bedeutet,
oder
R^{4A} und R^{4B} miteinander verknüpft sind und gemeinsam mit dem Kohlenstoffatom, an das sie gebunden sind, einen Cyclopropyl-Ring bilden,
und
R^{9A} Wasserstoff, Methyl oder Trifluormethyl bedeutet,
- R³: für (C₂-C₄)-Alkyl, (C₂-C₄)-Alkenyl, (C₃-C₆)-Cycloalkyl oder [(C₃-C₆)-Cycloalkyl]methyl steht,
wobei Alkyl und Alkenyl bis zu dreifach und Cycloalkyl bis zu zweifach mit Fluor substituiert sein können
und
wobei in Alkyl und Cycloalkyl eine CH₂-Gruppe gegen -O- oder -S- ausgetauscht sein kann, mit der Maßgabe, dass sich zwischen einem solchen Heteroatom und dem Uracil-N¹-Atom mindestens zwei Kohlenstoffatome befinden,
und
der Ring A für einen Aza-Heterocyclus der Formel 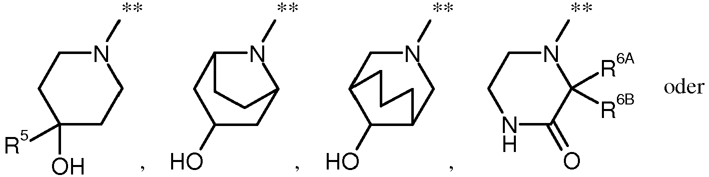 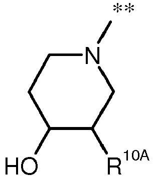 steht, worin ** die Verknüpfung zur Carbonylgruppe markiert,
- R⁵: Wasserstoff, Methyl, Trifluormethyl, Hydroxymethyl oder Ethyl bedeutet,
- R^{6A} und R^{6B}: unabhängig voneinander Wasserstoff, Methyl oder Ethyl bedeuten
und
- R^{10A}: Methyl oder Ethyl bedeutet,
sowie ihre Salze, Solvate und Solvate der Salze.

In einer weiteren bevorzugten Ausführungsform umfasst die vorliegende Erfindung Verbindungen der Formel (I), in welcher
- R¹: für Methyl, Difluormethyl, Trifluormethyl oder Ethyl steht,
- R²: für Methyl, Ethyl, *n*-Propyl, Isopropyl oder Propargyl oder für eine Gruppe der Formel 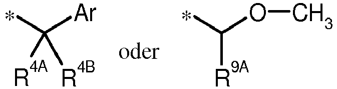 steht, worin * die Verknüpfung zur CH₂-Gruppe markiert,
Ar Phenyl oder Pyridyl bedeutet,
wobei Phenyl und Pyridyl mit Fluor, Chlor, Methyl oder Methoxy substituiert sein können,
R^{4A} Wasserstoff, Fluor oder Methyl bedeutet,
R^{4B} Wasserstoff, Fluor, Methyl, Hydroxy oder Methoxy bedeutet,
oder
R^{4A} und R^{4B} miteinander verknüpft sind und gemeinsam mit dem Kohlenstoffatom, an das sie gebunden sind, einen Cyclopropyl-Ring bilden,
und
R^{9A} Wasserstoff, Methyl oder Trifluormethyl bedeutet,
- R³: für (C₂-C₄)-Alkyl, (C₂-C₄)-Alkenyl, (C₃-C₆)-Cycloalkyl oder [(C₃-C₆)-Cycloalkyl]methyl steht,
wobei Alkyl und Alkenyl bis zu dreifach und Cycloalkyl bis zu zweifach mit Fluor substituiert sein können
und
wobei in Alkyl und Cycloalkyl eine CH₂-Gruppe gegen -O- oder -S- ausgetauscht sein kann, mit der Maßgabe, dass sich zwischen einem solchen Heteroatom und dem Uracil-N¹-Atom mindestens zwei Kohlenstoffatome befinden,
und
der Ring A für einen Aza-Heterocyclus der Formel 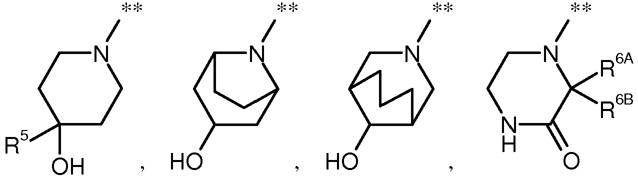 oder 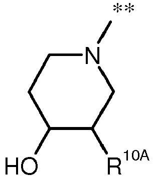 steht, worin ** die Verknüpfung zur Carbonylgruppe markiert,
- R⁵: Wasserstoff, Methyl, Trifluormethyl, Hydroxymethyl oder Ethyl bedeutet,
- R^{6A} und R^{6B}: unabhängig voneinander Wasserstoff, Methyl oder Ethyl bedeuten
und
- R^{10A}: Methyl oder Ethyl bedeutet,
sowie ihre Salze, Solvate und Solvate der Salze.

In einer weiteren bevorzugten Ausführungsform umfasst die vorliegende Erfindung Verbindungen der Formel (I), in welcher
- R¹: für Methyl oder Trifluormethyl steht,
- R²: für Methyl, Ethyl, *n*-Propyl oder Isopropyl oder für eine Gruppe der Formel 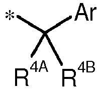 steht, worin * die Verknüpfung zur CH₂-Gruppe markiert,
Ar Phenyl oder Pyridyl bedeutet,
wobei Phenyl und Pyridyl mit Fluor, Chlor, Methyl oder Methoxy substituiert sein können,
R^{4A} Wasserstoff, Fluor oder Methyl bedeutet,
R^{4B} Wasserstoff, Fluor, Methyl oder Methoxy bedeutet,
oder
R^{4A} und R^{4B} miteinander verknüpft sind und gemeinsam mit dem Kohlenstoffatom, an das sie gebunden sind, einen Cyclopropyl-Ring bilden,
- R³: für (C₂-C₄)-Alkyl, (C₂-C₄)-Alkenyl, (C₃-C₆)-Cycloalkyl oder [(C₃-C₆)-Cycloalkyl]methyl steht,
wobei Alkyl und Alkenyl bis zu dreifach und Cycloalkyl bis zu zweifach mit Fluor substituiert sein können
und
wobei in Alkyl und Cycloalkyl eine CH₂-Gruppe gegen -O- oder -S- ausgetauscht sein kann, mit der Maßgabe, dass sich zwischen einem solchen Heteroatom und dem Uracil-N¹-Atom mindestens zwei Kohlenstoffatome befinden,
und
der Ring A für einen Aza-Heterocyclus der Formel 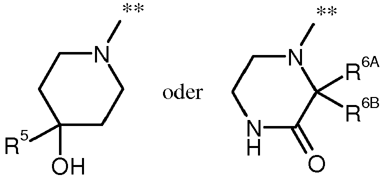 steht, worin ** die Verknüpfung zur Carbonylgruppe markiert,
- R⁵: Wasserstoff, Methyl, Trifluormethyl oder Hydroxymethyl bedeutet
und
R⁶¹ und R^{6B} unabhängig voneinander Wasserstoff, Methyl oder Ethyl bedeuten,
sowie ihre Salze, Solvate und Solvate der Salze.

Eine besondere Ausführungsform der vorliegenden Erfindung betrifft Verbindungen der Formel (I), in welcher
- R¹: für Methyl oder Ethyl steht,
sowie ihre Salze, Solvate und Solvate der Salze.

Eine weitere besondere Ausführungsform der vorliegenden Erfindung betrifft Verbindungen der Formel (I), in welcher
- R¹: für Difluormethyl oder Trifluormethyl steht,
sowie ihre Salze, Solvate und Solvate der Salze.

Eine weitere besondere Ausführungsform der vorliegenden Erfindung betrifft Verbindungen der Formel (I), in welcher
- R²: für Methyl, Ethyl oder Isopropyl steht,
sowie ihre Salze, Solvate und Solvate der Salze.

Eine weitere besondere Ausführungsform der vorliegenden Erfindung betrifft Verbindungen der Formel (I), in welcher
- R²: für Propargyl steht,
sowie ihre Salze, Solvate und Solvate der Salze.

Eine weitere besondere Ausführungsform der vorliegenden Erfindung betrifft Verbindungen der Formel (I), in welcher
- R²: für eine Gruppe der Formel 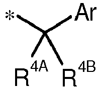 steht, worin * die Verknüpfung zur CH₂-Gruppe markiert,
- Ar: Phenyl bedeutet, das mit Fluor, Chlor, Methyl oder Methoxy substituiert sein kann,
- R^{4A}: Wasserstoff bedeutet
und
- R^{4B}: Wasserstoff, Fluor, Methyl oder Methoxy bedeutet,
sowie ihre Salze, Solvate und Solvate der Salze.

Eine weitere besondere Ausführungsform der vorliegenden Erfindung betrifft Verbindungen der Formel (I), in welcher
- R²: für eine Gruppe der Formel 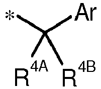 steht, worin * die Verknüpfung zur CH₂-Gruppe markiert,
Ar Phenyl bedeutet, das mit Fluor, Chlor, Methyl oder Methoxy substituiert sein kann,
R^{4A} Wasserstoff oder Methyl bedeutet
und
R^{4B} Hydroxy bedeutet,
sowie ihre Salze, Solvate und Solvate der Salze.

Eine weitere besondere Ausführungsform der vorliegenden Erfindung betrifft Verbindungen der Formel (I), in welcher
- R²: für eine Gruppe der Formel 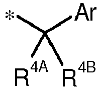 steht, worin * die Verknüpfung zur CH₂-Gruppe markiert,
Ar Phenyl bedeutet, das mit Fluor, Chlor, Methyl oder Methoxy substituiert sein kann,
und
R^{4A} und R^{4B} jeweils Fluor bedeuten oder miteinander verknüpft sind und gemeinsam mit dem Kohlenstoffatom, an das sie gebunden sind, einen Cyclopropyl-Ring bilden,
sowie ihre Salze, Solvate und Solvate der Salze.

Eine weitere besondere Ausführungsform der vorliegenden Erfindung betrifft Verbindungen der Formel (I), in welcher
- R²: für eine Gruppe der Formel 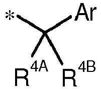 steht, worin * die Verknüpfung zur CH₂-Gruppe markiert,
Ar Pyridyl bedeutet
und
R^{4A} und R^{4B} jeweils Wasserstoff bedeuten,
sowie ihre Salze, Solvate und Solvate der Salze.

Eine weitere besondere Ausführungsform der vorliegenden Erfindung betrifft Verbindungen der Formel (I), in welcher
- R²: für eine Gruppe der Formel 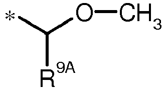 steht, worin * die Verknüpfung zur CH₂-Gruppe markiert und
R^{9A} Wasserstoff, Methyl oder Trifluormethyl bedeutet,
sowie ihre Salze, Solvate und Solvate der Salze.

Eine weitere besondere Ausführungsform der vorliegenden Erfindung betrifft Verbindungen der Formel (I), in welcher
- R³: für (C₂-C₄)-Alkyl, (C₂-C₄)-Alkenyl oder (C₃-C₆)-Cycloalkyl steht,
wobei Alkyl und Alkenyl bis zu dreifach mit Fluor substituiert sein können
und
wobei in Alkyl und Cycloalkyl eine CH₂-Gruppe gegen -O- oder -S- ausgetauscht sein kann, mit der Maßgabe, dass sich zwischen einem solchen Heteroatom und dem Uracil-N¹-Atom mindestens zwei Kohlenstoffatome befinden,
sowie ihre Salze, Solvate und Solvate der Salze.

Eine weitere besondere Ausführungsform der vorliegenden Erfindung betrifft Verbindungen der Formel (I), in welcher
- R³: für 2,2,2-Trifluorethyl oder (Trifluormethoxy)methyl steht,
sowie ihre Salze, Solvate und Solvate der Salze.

Eine weitere besondere Ausführungsform der vorliegenden Erfindung betrifft Verbindungen der Formel (I), in welcher
der Ring A für einen Aza-Heterocyclus der Formel 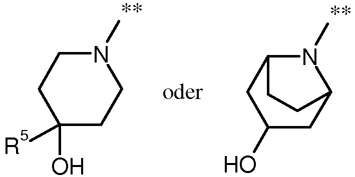 steht, worin ** die Verknüpfung zur Carbonylgruppe markiert
und
- R⁵: Wasserstoff, Methyl, Trifluormethyl, Hydroxymethyl oder Ethyl bedeutet,
sowie ihre Salze, Solvate und Solvate der Salze.

Eine weitere besondere Ausführungsform der vorliegenden Erfindung betrifft Verbindungen der Formel (I), in welcher
der Ring A für einen Aza-Heterocyclus der Formel 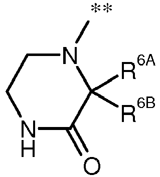 steht, worin ** die Verknüpfung zur Carbonylgruppe markiert
und
R^{6A} und R^{6B} unabhängig voneinander Wasserstoff, Methyl oder Ethyl bedeuten,
sowie ihre Salze, Solvate und Solvate der Salze.

Besonders bevorzugt im Rahmen der vorliegenden Erfindung sind Verbindungen der Formel (I), in welcher
- R¹: für Methyl, Difluormethyl, Trifluormethyl oder Ethyl steht,
- R²: für Methyl, Ethyl, Isopropyl oder Propargyl oder für eine Gruppe der Formel 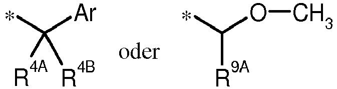 steht, worin * die Verknüpfung zur CH₂-Gruppe markiert,
Ar Phenyl, 3-Pyridyl oder 4-Pyridyl bedeutet,
wobei Phenyl in *meta-* oder *para*-Position mit Fluor oder in *ortho*-Position mit Fluor, Chlor oder Methyl substituiert sein kann,
R^{4A} Wasserstoff, Fluor oder Methyl bedeutet,
R^{4B} Wasserstoff, Fluor, Methyl, Hydroxy oder Methoxy bedeutet,
oder
R^{4A} und R^{4B} miteinander verknüpft sind und gemeinsam mit dem Kohlenstoffatom, an das sie gebunden sind, einen Cyclopropyl-Ring bilden,
und
R^{9A} Methyl oder Trifluormethyl bedeutet,
- R³: für 2,2,2-Trifluorethyl, 3,3-Difluorprop-2-en-1-yl, Methoxymethyl, (Trifluormethoxy)-methyl oder [(Trifluormethyl)sulfanyl]methyl steht,
und
der Ring A für einen Aza-Heterocyclus der Formel 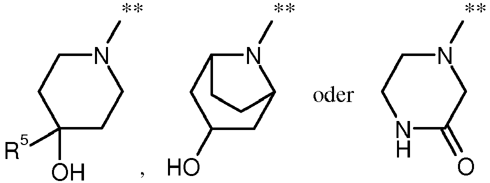 steht, worin ** die Verknüpfung zur Carbonylgruppe markiert
und
- R⁵: Wasserstoff, Methyl, Trifluormethyl, Hydroxymethyl oder Ethyl bedeutet,
sowie ihre Salze, Solvate und Solvate der Salze.

In einer weiteren besonders bevorzugten Ausführungsform umfasst die vorliegende Erfindung Verbindungen der Formel (I), in welcher
- R¹: für Methyl, Difluormethyl, Trifluormethyl oder Ethyl steht,
- R²: für Methyl, Ethyl, Isopropyl oder Propargyl oder für eine Gruppe der Formel 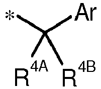 steht, worin * die Verknüpfung zur CH₂-Gruppe markiert,
Ar Phenyl, 3-Pyridyl oder 4-Pyridyl bedeutet,
wobei Phenyl in *meta-* oder *para*-Position mit Fluor oder in *ortho*-Position mit Fluor, Chlor oder Methyl substituiert sein kann,
R^{4A} Wasserstoff oder Fluor bedeutet,
R^{4B} Wasserstoff, Fluor, Methyl, Hydroxy oder Methoxy bedeutet,
oder
R^{4A} und R^{4B} miteinander verknüpft sind und gemeinsam mit dem Kohlenstoffatom, an das sie gebunden sind, einen Cyclopropyl-Ring bilden,
- R³: für 2,2,2-Trifluorethyl, 3,3-Difluorprop-2-en-1-yl, Methoxymethyl, (Trifluormethoxy)-methyl oder [(Trifluormethyl)sulfanyl]methyl steht,
und
der Ring A für einen Aza-Heterocyclus der Formel 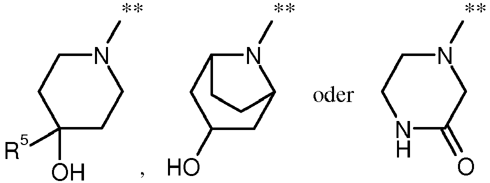 steht, worin ** die Verknüpfung zur Carbonylgruppe markiert
und
- R⁵: Wasserstoff, Methyl, Trifluormethyl, Hydroxymethyl oder Ethyl bedeutet,
sowie ihre Salze, Solvate und Solvate der Salze.

In einer weiteren besonders bevorzugten Ausführungsform umfasst die vorliegende Erfindung Verbindungen der Formel (I), in welcher
- R¹: für Methyl steht,
- R²: für Methyl, Ethyl oder Isopropyl oder für eine Gruppe der Formel 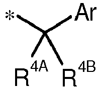 steht, worin * die Verknüpfung zur CH₂-Gruppe markiert,
Ar Phenyl oder 3-Pyridyl bedeutet,
wobei Phenyl in *ortho*-Position mit Fluor, Chlor oder Methyl substituiert sein kann,
R^{4A} Wasserstoff bedeutet
und
R^{4B} Wasserstoff, Methyl oder Methoxy bedeutet,
- R³: für 2,2,2-Trifluorethyl, 3,3-Difluorprop-2-en-1-yl, Methoxymethyl oder (Trifluormethoxy)-methyl steht,
und
der Ring A für einen Aza-Heterocyclus der Formel 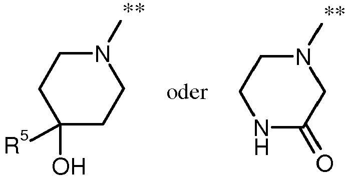 steht, worin ** die Verknüpfung zur Carbonylgruppe markiert
und
- R⁵: Wasserstoff, Methyl oder Hydroxymethyl bedeutet,
sowie ihre Salze, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Ganz besonders bevorzugt sind Kombinationen von zwei oder mehreren der oben genannten Vorzugsbereiche.

Weiterer Gegenstand der Erfindung ist ein Verfahren zur Herstellung der erfindungsgemäßen Verbindungen der Formel (I), dadurch gekennzeichnet, dass man entweder

### [A-1] eine Verbindung der Formel (II)

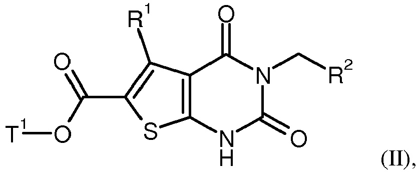

in welcher R¹ und R² die oben angegebenen Bedeutungen haben
und
- T¹: für (C₁-C₄)-Alkyl oder Benzyl steht,
in Gegenwart einer Base mit einer Verbindung der Formel (III) 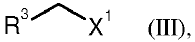 in welcher R³ die oben angegebene Bedeutung hat
und
- X¹: für eine Abgangsgruppe wie beispielsweise Chlor, Brom, Iod, Mesylat, Triflat oder Tosylat steht,
zu einer Verbindung der Formel (IV) 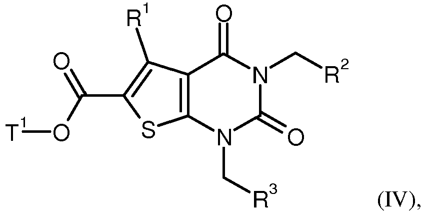 in welcher R¹, R², R³ und T¹ die oben angegebenen Bedeutungen haben,
alkyliert, anschließend den Ester-Rest T¹ abspaltet und die so erhaltene Carbonsäure der Formel (V) 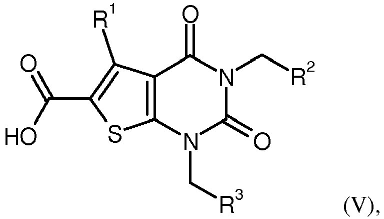 in welcher R¹, R² und R³ die oben angegebenen Bedeutungen haben,
dann unter Aktivierung der Carboxyl-Funktion mit einem Amin der Formel (VI) 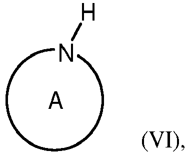 in welcher der Ring A die oben angegebene Bedeutung hat,
kuppelt
oder - in veränderter Abfolge der obigen Reaktionsschritte -

### [A-2] die Verbindung der Formel (II)

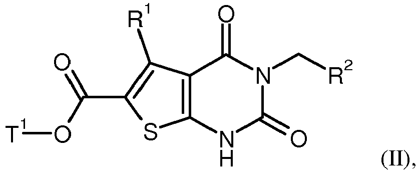

in welcher R¹, R² und T¹ die oben angegebenen Bedeutungen haben,
zunächst durch Abspaltung des Ester-Restes T¹ in die Carbonsäure der Formel (VII) 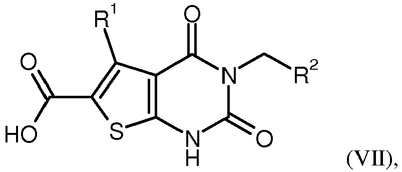 in welcher R¹ und R² die oben angegebenen Bedeutungen haben,
überführt, anschließend unter Aktivierung der Carboxyl-Funktion mit einem Amin der Formel (VI) 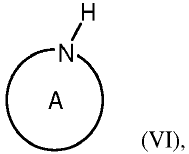 in welcher der Ring A die oben angegebene Bedeutung hat,
zu einer Verbindung der Formel (VIII) 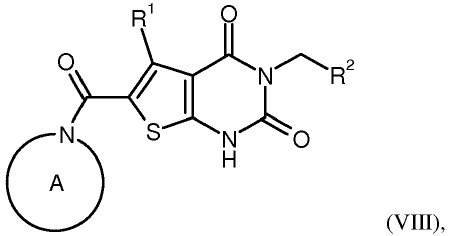 in welcher R¹, R² und der Ring A die oben angegebenen Bedeutungen haben,
kuppelt und diese dann in Gegenwart einer Base mit einer Verbindung der Formel (III) 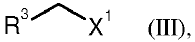 in welcher R³ und X¹ die oben angegebenen Bedeutungen haben,
alkyliert
oder

### [B] zunächst eine N³-geschützte Verbindung der Formel (IX)

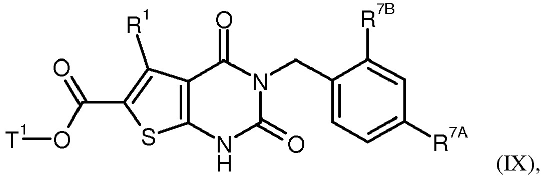

in welcher R¹ und T¹ die oben angegebenen Bedeutungen haben
und
R^{7A} und R^{7B} unabhängig voneinander für Wasserstoff oder Methoxy stehen,
in Gegenwart einer Base mit einer Verbindung der Formel (III) 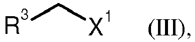 in welcher R³ und X¹ die oben angegebenen Bedeutungen haben,
zu einer Verbindung der Formel (X) 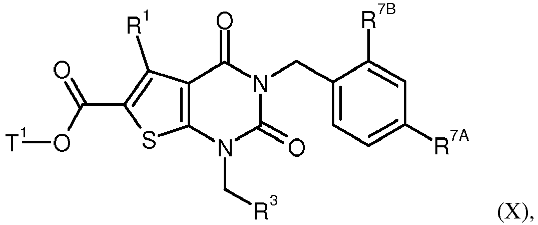 in welcher R¹, R³, R^{7A} , R^{7B} und T¹ die oben angegebenen Bedeutungen haben,
alkyliert, danach durch Behandlung mit einer starken Lewis-Säure wie Aluminiumtrichlorid simultan die *N³*-Benzylgruppe und den Ester-Rest T¹ abspaltet, die so erhaltene Carbonsäure der Formel (XI) 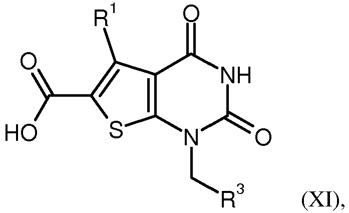 in welcher R¹ und R³ die oben angegebenen Bedeutungen haben,
anschließend unter Aktivierung der Carboxyl-Funktion mit einem Amin der Formel (VI) 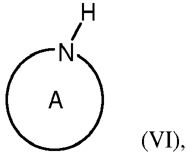 in welcher der Ring A die oben angegebene Bedeutung hat,
zu einer Verbindung der Formel (XII) 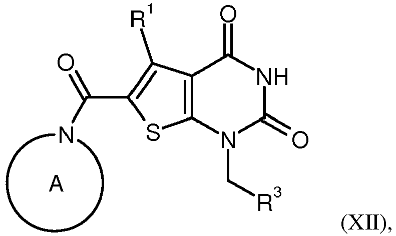 in welcher R¹, R³ und der Ring A die oben angegebenen Bedeutungen haben,
kuppelt und letztere dann entweder (*a*) in Gegenwart einer Base mit einer Verbindung der Formel (XIII) 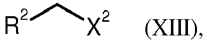 in welcher R² die oben angegebene Bedeutung hat
und
- X²: für eine Abgangsgruppe wie beispielsweise Chlor, Brom, Iod, Mesylat, Triflat oder Tosylat steht, oder (*b*) in Gegenwart eines geeigneten Phosphins und eines Azodicarboxylats mit einer Verbindung der Formel (XIV) 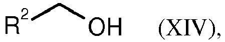
in welcher R² die oben angegebene Bedeutung hat,
umsetzt
und die so hergestellten Verbindungen der Formel (I) gegebenenfalls mit den entsprechenden (*i*) Lösungsmitteln und/oder (*ii*) Säuren in ihre Solvate, Salze und/oder Solvate der Salze überführt.

Bei den zuvor beschriebenen Verfahren kann es gegebenenfalls erforderlich oder von Vorteil sein, eine in der Amin-Verbindung der Formel (VI) vorhandene Hydroxy-Gruppe bei der Kupplung dieses Amins [Reaktionen (V) + (VI) → (I), (VII) + (VI) → (VIII) bzw. (XI) + (VI) → (XII)] und/ oder bei nachfolgenden Alkylierungsreaktionen [(VIII) + (III) → (I) bzw. (XII) + (XIII) oder (XIV) → (I)] temporär geschützt zu halten und erst am Ende der Reaktionssequenz wieder freizusetzen. Für diesen Zweck eignen sich gängige Hydroxy-Schutzgruppen wie Acetyl, Pivaloyl, Benzoyl, *tert*.-Butyl, 2-(Trimethylsilyl)ethyl, Benzyl, Triphenylmethyl, Methoxymethyl, 2-(Trimethylsilyl)-ethoxymethyl, Tetrahydropyran-2-yl, Trimethylsilyl, Triisopropylsilyl oder *tert.*-Butyldimethyl-silyl. Bevorzugt wird Acetyl als Hydroxy-Schutzgruppe verwendet.

Ebenso kann es zweckmäßig sein, eine in der Verbindung (VI) vorhandene amidische NH-Gruppe vorübergehend zu blockieren, um Selektivitätsprobleme bei nachfolgenden Alkylierungsreaktionen im Uracil-Teil des Moleküls zu vermeiden [siehe Umsetzungen (VIII) + (III) → (I) bzw. (XII) + (XIII) oder (XIV) → (I)]. Hierfür sind bekannte Amid-Schutzgruppen wie Allyl, 2-(Trimethylsilyl)ethyl, 4-Methoxybenzyl, 2,4-Dimethoxybenzyl, Methoxymethyl oder Benzyloxymethyl geeignet.

Einführung und Entfernung der genannten Schutzgruppen erfolgen nach allgemein üblichen Methoden [siehe z.B. T.W. Greene und P.G.M. Wuts, Protective Groups in Organic Synthesis, Wiley, New York, 1999].

Als inertes Lösungsmittel für die Alkylierungsreaktionen (II) + (III) → (IV), (VIII) + (III) → (I), (IX) + (III) → (X) und (XII) + (XIII) → (I) eignen sich insbesondere Ether wie Diethylether, Diisopropylether, Methyl-*tert*.-butylether, Tetrahydrofuran, 1,4-Dioxan, 1,2-Dimethoxyethan oder Bis(2-methoxyethyl)ether, oder polar-aprotische Lösungsmittel wie Acetonitril, Butyronitril, Aceton, Methylethylketon, Ethylacetat, *N,N*-Dimethylformamid (DMF), *N,N*-Dimethylacetamid (DMA), Dimethylsulfoxid (DMSO), *N,N*'-Dimethylpropylenharnstoff (DMPU) oder *N*-Methylpyrrolidinon (NMP). Ebenso ist es möglich, Gemische solcher Lösungsmittel einzusetzen. Bevorzugt wird *N,N*-Dimethylformamid verwendet.

Als Base sind für diese Alkylierungsreaktionen insbesondere übliche anorganische Basen geeignet. Hierzu gehören Alkalihydroxide wie Lithium-, Natrium- oder Kaliumhydroxid, Alkalihydrogencarbonate wie Natrium- oder Kaliumhydrogencarbonat, Alkalicarbonate wie Lithium-, Natrium-, Kalium- oder Cäsiumcarbonat, oder Alkalihydride wie Natrium- oder Kaliumhydrid. Bevorzugt wird Cäsiumcarbonat eingesetzt.

Die genannten Umsetzungen werden im Allgemeinen in einem Temperaturbereich von +20°C bis +100°C durchgeführt.

Die Abspaltung der Ester-Gruppe T¹ in den Verfahrensschritten (IV) → (V) und (II) → (VII) wird nach üblichen Methoden durchgeführt, indem man den Ester in einem inerten Lösungsmittel mit einer Säure oder Base behandelt, wobei bei letzterer Variante das zunächst entstehende Salz der Carbonsäure durch nachfolgende Behandlung mit Säure in die freie Carbonsäure überführt wird. Im Falle der *tert*.-Butylester erfolgt die Esterspaltung vorzugsweise mit einer Säure. Methyl- und Ethylester werden bevorzugt mittels einer Base gespalten. Benzylester können alternativ auch durch Hydrierung (Hydrogenolyse) in Gegenwart eines geeigneten Katalysators, wie beispielsweise Palladium auf Aktivkohle, abgespalten werden.

Als inerte Lösungsmittel eignen sich für diese Reaktionen Wasser und die für eine Esterspaltung üblichen organischen Lösungsmittel. Hierzu zählen insbesondere Alkohole wie Methanol, Ethanol, n-Propanol, Isopropanol, *n*-Butanol oder *tert*.-Butanol, Ether wie Diethylether, Tetrahydrofuran, 1,4-Dioxan oder 1,2-Dimethoxyethan, oder andere Lösungsmittel wie Dichlormethan, Acetonitril, *N,N*-Dimethylformamid oder Dimethylsulfoxid. Ebenso ist es möglich, Gemische dieser Lösungsmittel einzusetzen. Im Falle einer basischen Ester-Hydrolyse werden bevorzugt Gemische von Wasser mit Tetrahydrofuran, 1,4-Dioxan, Methanol und/oder Ethanol verwendet. Im Falle der Umsetzung mit Trifluoressigsäure wird bevorzugt Dichlormethan und im Falle der Umsetzung mit Chlorwasserstoff bevorzugt 1,4-Dioxan, jeweils unter wasserfreien Bedingungen, verwendet.

Als Basen sind die für eine Hydrolyse-Reaktion üblichen anorganischen Basen geeignet. Hierzu gehören insbesondere Alkali- oder Erdalkalihydroxide wie beispielsweise Lithium-, Natrium-, Kalium- oder Bariumhydroxid, oder Alkali- oder Erdalkalicarbonate wie Natrium-, Kalium- oder Calciumcarbonat. Bevorzugt wird Lithiumhydroxid eingesetzt.

Als Säuren eignen sich für die Esterspaltung im Allgemeinen Schwefelsäure, Chlorwasserstoff/ Salzsäure, Bromwasserstoff/Bromwasserstoffsäure, Phosphorsäure, Essigsäure, Trifluoressigsäure, Toluolsulfonsäure, Methansulfonsäure oder Trifluormethansulfonsäure oder deren Gemische gegebenenfalls unter Zusatz von Wasser. Bevorzugt werden Chlorwasserstoff oder Trifluoressigsäure verwendet.

Die Esterspaltung erfolgt in der Regel in einem Temperaturbereich von -20°C bis +100°C, bevorzugt bei 0°C bis +50°C.

Die Kupplungsreaktionen (V) + (VI) → (I), (VII) + (VI) → (VIII) und (XI) + (VI) → (XII) [Amid-Bildung] können mit Hilfe eines Kondensations- oder Aktivierungsmittels durchgeführt werden oder über die Zwischenstufe der entsprechenden Carbonsäurechloride erfolgen.

Als solche Kondensations- oder Aktivierungsmittel eignen sich beispielsweise Carbodiimide wie *N,N*'-Diethyl-, *N,N'*-Dipropyl-, *N,N'*-Diisopropyl-, *N,N'*-Dicyclohexylcarbodiimid (DCC) oder *N*-(3-Dimethylaminopropyl)-*N'*-ethylcarbodiimid-Hydrochlorid (EDC), Phosgen-Derivate wie *N,N'*-Carbonyldiimidazol (CDI) oder Isobutylchlorformiat, 1,2-Oxazolium-Verbindungen wie 2-Ethyl-5-phenyl-1,2-oxazolium-3-sulfat oder 2-*tert*.-Butyl-5-methylisoxazolium-perchlorat, Acylamino-Verbindungen wie 2-Ethoxy-1-ethoxycarbonyl-1,2-dihydrochinolin, α-Chlorenamine wie 1-Chlor-2-methyl-1-dimethylamino-1-propen, Phosphor-Verbindungen wie Propanphosphonsäureanhydrid, Cyanophosphonsäurediethylester, Bis-(2-oxo-3-oxazolidinyl)-phosphorylchlorid, Benzotriazol-1-yloxy-tris(dimethylamino)phosphonium-hexafluorophosphat oder Benzotriazol-1-yloxy-tris(pyrrolidino)phosphonium-hexafluorophosphat (PyBOP), oder Uronium-Verbindungen wie *O*-(Benzotriazol-1-yl)-*N,N,N',N'*-tetramethyluronium-tetrafluoroborat (TBTU), *O*-(Benzotriazol-1-yl)*-N,N,N',N'*-tetramethyluronium-hexafluorophosphat (HBTU), 2-(2-Oxo-1-(2*H*)-pyridyl)-1,1,3,3-tetramethyluronium-tetrafluoroborat (TPTU), *O*-(7-Azabenzotriazol-1-yl)-*N,N,N,N'*-tetramethyl-uronium-hexafluorophosphat (HATU) oder O-(1*H*-6-Chlorbenzotriazol-1-yl)-1,1,3,3-tetramethyl-uronium-tetrafluoroborat (TCTU), gegebenenfalls in Kombination mit weiteren Hilfsstoffen wie 1-Hydroxybenzotriazol (HOBt) oder *N*-Hydroxysuccinimid (HOSu), sowie als Basen Alkalicarbonate, z.B. Natrium- oder Kaliumcarbonat, oder tertiäre Aminbasen wie Triethylamin, *N*-Methylmorpholin (NMM), *N*-Methylpiperidin (NMP), *N,N*-Diisopropylethylamin, Pyridin oder 4-*N,N*-Dimethylaminopyridin (DMAP). Bevorzugt eingesetzt wird *O*-(7-Azabenzotriazol-1-yl)-*N,N,N',N'-*tetramethyluronium-hexafluorophosphat (HATU) in Kombination mit *N,N*-Diisopropylethylamin, oder *N*-(3-Dimethylaminopropyl)-*N'*-ethylcarbodiimid-Hydrochlorid (EDC) in Verbindung mit 1-Hydroxybenzotriazol (HOBt) und Triethylamin.

Bei zweistufiger Reaktionsführung über die entsprechenden Carbonsäurechloride wird die Kupplung mit der Amin-Komponente (VI) in Gegenwart einer üblichen Hilfsbase wie Natriumcarbonat, Kaliumcarbonat, Triethylamin, *N*-Methylmorpholin (NMM), *N*-Methylpiperidin (NMP), *N,N-*Diisopropylethylamin, Pyridin, 2,6-Dimethylpyridin, 4-*N,N*-Dimethylaminopyridin (DMAP), 1,8-Diazabicyclo[5.4.0]undec-7-en (DBU) oder 1,5-Diazabicyclo[4.3.0]non-5-en (DBN) durchgeführt; bevorzugt wird *N,N*-Diisopropylethylamin verwendet. Die Herstellung der Carbonsäurechloride selbst geschieht auf übliche Weise durch Behandlung der Carbonsäure (V), (VII) bzw. (XI) mit Thionylchlorid oder Oxalylchlorid in einem inerten Lösungsmittel wie Dichlormethan.

Inerte Lösungsmittel für die genannten Kupplungsreaktionen sind beispielsweise Ether wie Diethylether, Diisopropylether, Methyl-*tert*.-butylether, Tetrahydrofuran, 1,4-Dioxan, 1,2-Dimethoxyethan oder Bis(2-methoxyethyl)ether, Kohlenwasserstoffe wie Benzol, Toluol, Xylol, Pentan, Hexan oder Cyclohexan, Halogenkohlenwasserstoffe wie Dichlormethan, Trichlormethan, Tetrachlormethan, 1,2-Dichlorethan, Trichlorethylen oder Chlorbenzol, oder polar-aprotische Lösungsmittel wie Aceton, Methylethylketon, Acetonitril, Butyronitril, Ethylacetat, Pyridin, Dimethylsulfoxid (DMSO), *N,N*-Dimethylformamid (DMF), *N,N*'-Dimethylpropylenharnstoff (DMPU) oder *N-*Methylpyrrolidinon (NMP). Auch können Gemische solcher Lösungsmittel eingesetzt werden. Bevorzugt werden Dichlormethan, Tetrahydrofuran, *N,N*-Dimethylformamid oder Gemische hiervon verwendet.

Die Kupplungen werden in der Regel in einem Temperaturbereich von -20°C bis +60°C, bevorzugt bei 0°C bis +40°C durchgeführt.

Die simultane Abspaltung der *N³*-Benzyl-Schutzgruppe und des Ester-Restes T¹ im Verfahrensschritt (X) → (XI) erfolgt zweckmäßigerweise durch Behandlung von (X) mit einer starken Lewis-Säure, wie insbesondere überschüssigem Aluminiumtrichlorid, in einem inerten Lösungsmittel wie Toluol, Xylol oder Chlorbenzol in einem Temperaturbereich von +20°C bis +120°C, bevorzugt im Temperaturbereich von +40°C bis +70°C.

Die Umsetzung (XII) + (XIV) → (I) wird unter den üblichen Bedingungen einer "Mitsunobu-Reaktion" in Gegenwart von Triphenylphosphin und einem Azodicarboxylat, wie Diethylazodicarboxylat (DEAD) oder Diisopropylazodicarboxylat (DIAD), durchgeführt [siehe z.B. D. L. Hughes, Org. Reactions 42, 335 (1992); D. L. Hughes, Org. Prep. Proced. Int. 28 (2), 127 (1996)]. Als inertes Lösungsmittel für diese Reaktion wird vorzugsweise Tetrahydrofuran verwendet.

Die zuvor beschriebenen Verfahrensschritte können bei normalem, bei erhöhtem oder bei erniedrigtem Druck durchgeführt werden (z.B. im Bereich von 0.5 bis 5 bar); im Allgemeinen arbeitet man jeweils bei Normaldruck.

Verbindungen der Formel (II), in welcher R¹ für Methyl oder Ethyl steht, können dadurch hergestellt werden, dass man eine Verbindung der Formel (XV) 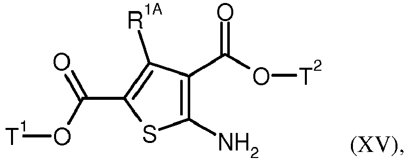 in welcher T¹ die oben angegebene Bedeutung hat,
- R^{1A}: für Methyl oder Ethyl steht
und
- T²: für Methyl oder Ethyl steht,
in Gegenwart einer Base entweder (*a*) zunächst mit einem Phosgen-Äquivalent wie *N,N*'-Carbonyldiimidazol und dann mit einem Amin der Formel (XVI) 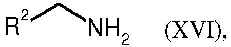 in welcher R² die oben angegebene Bedeutung hat,
oder (*b*) mit einem Isocyanat der Formel (XVII) 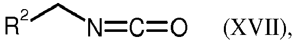 in welcher R² die oben angegebene Bedeutung hat,
zu einer Verbindung der Formel (XVIII) 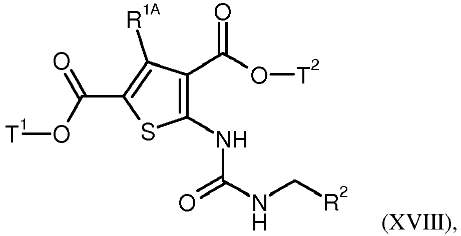 in welcher R^{1A}, R², T¹ und T² die oben angegebenen Bedeutungen haben,
umsetzt und diese dann durch Behandlung mit einer Base zu der Verbindung der Formel (II-A) 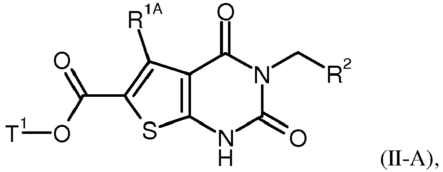 in welcher R^{1A}, R² und T¹ die oben angegebenen Bedeutungen haben,
cyclisiert.

Inerte Lösungsmittel für die Transformation des Aminothiophens (XV) in das Harnstoff-Derivat (XVIII) mit Hilfe von *N,N'*-Carbonyldiimidazol und dem Amin (XVI) oder mittels des Isocyanats (XVII) sind beispielsweise Ether wie Diethylether, Diisopropylether, Methyl-*tert*.-butylether, Tetrahydrofuran, 1,4-Dioxan, 1,2-Dimethoxyethan oder Bis(2-methoxyethyl)ether, Kohlenwasserstoffe wie Benzol, Toluol, Xylol, Pentan, Hexan oder Cyclohexan, Halogenkohlenwasserstoffe wie Dichlormethan, Trichlormethan, Tetrachlormethan, 1,2-Dichlorethan, Trichlorethylen oder Chlorbenzol, oder polar-aprotische Lösungsmittel wie Aceton, Methylethylketon, Acetonitril, Butyronitril, Ethylacetat, Pyridin, Dimethylsulfoxid (DMSO), *N,N*-Dimethylformamid (DMF), *N,N-*Dimethylacetamid (DMA), *N,N*'-Dimethylpropylenharnstoff (DMPU) oder *N*-Methylpyrrolidinon (NMP). Auch können Gemische dieser Lösungsmittel eingesetzt werden. Bevorzugt wird Dichlormethan oder Tetrahydrofuran verwendet.

Als Hilfsbase werden für diese Reaktionen vorzugsweise tertiäre Aminbasen wie Triethylamin, *N*-Methylmorpholin (NMM), *N*-Methylpiperidin (NMP), *N,N*-Diisopropylethylamin, Pyridin oder 4-*N,N*-Dimethylaminopyridin (DMAP) verwendet; bevorzugt ist Triethylamin.

Als Phosgen-Äquivalente können neben *N,N'*-Carbonyldiimidazol alternativ auch Trichlormethylchlorformiat ("Diphosgen"), Bis(trichlormethyl)carbonat ("Triphosgen") oder ähnliche Reagentien in Betracht kommen.

Die Umsetzungen werden im Allgemeinen in einem Temperaturbereich von 0°C bis +100°C, bevorzugt bei +20°C bis +60°C durchgeführt.

Die baseninduzierte Cyclisierungsreaktion (XVIII) → (II-A) erfolgt vorzugsweise mit Hilfe eines Alkalialkoholats wie Natrium- oder Kaliummethanolat, Natrium- oder Kaliumethanolat oder Natrium- oder Kalium-*tert*.-butylat in dem betreffenden Alkohol als Lösungsmittel oder mit Hilfe eines Alkalihydrids wie Natrium- oder Kaliumhydrid in Tetrahydrofuran oder *N,N*-Dimethylformamid als inertem Lösungsmittel; bevorzugt wird Natriumethanolat in Ethanol verwendet. Die Umsetzung wird in der Regel in einem Temperaturbereich von 0°C bis +100°C, bevorzugt bei +20°C bis +60°C durchgeführt [zur Synthesesequenz (XV) → (XVIII) → (II-A) allgemein vgl. z.B. auch die in EP 1 847 541-A1 zur Herstellung anderer Thienouracil-Derivate beschriebenen Verfahren].

Die Verbindungen der Formel (XV) ihrerseits lassen sich auf literaturbekanntem Wege über die 3-Komponenten-Reaktion eines Acetessigsäure- oder β-Ketovaleriansäureesters (für R^{1A} = Methyl bzw. Ethyl) mit einem α-Cyanoessigsäureester und elementarem Schwefel erhalten ["Gewald-Reaktion"; siehe z.B. B. P. McKibben et al., Tetrahedron Lett. 40, 5471-5474 (1999) und dort zitierte weitere Literatur].

Verbindungen der Formel (II), in welcher R¹ für Wasserstoff steht, können dadurch hergestellt werden, dass man eine Verbindung der Formel (XIX) 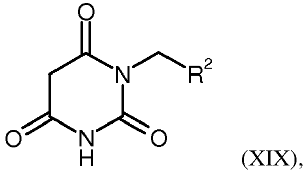 in welcher R² die oben angegebene Bedeutung hat,
mit einer Mischung aus Phosphoroxychlorid und *N,N*-Dimethylformamid in eine Verbindung der Formel (XX) 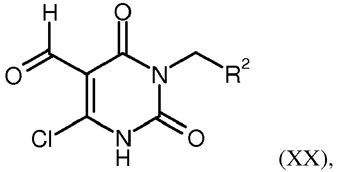 in welcher R² die oben angegebene Bedeutung hat,
überführt und diese dann in Gegenwart einer Base mit einem α-Mercaptoessigsäureester der Formel (XXI) 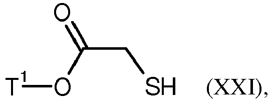 in welcher T¹ die oben angegebene Bedeutung hat,
zur Verbindung der Formel (II-B) 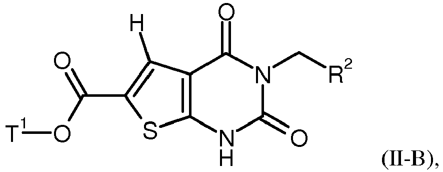 in welcher R² und T¹ die oben angegebenen Bedeutungen haben,
kondensiert.

Die Umwandlung des Barbitursäure-Derivats (XIX) in das 6-Chlor-5-formylpyrimidindion (XX) erfolgt über eine regioselektive Vilsmaier-Haack-Reaktion durch Behandlung von (XIX) mit einer vorgebildeten Mischung aus Phosphoroxychlorid und *N,N*-Dimethylformamid, welche im großen Überschuss eingesetzt wird und gleichzeitig auch als Lösungsmittel dient [vgl. z.B. K. Tanaka et al., Chem. Pharm. Bull. 35 (4), 1397-1404 (1987)]. Die Umsetzung wird in einem Temperaturbereich von +20°C bis +120°C durchgeführt.

Für die nachfolgende Kondensationsreaktion (XX) + (XXI) → (II-B) geeignete Basen sind insbesondere Alkalicarbonate wie Lithium-, Natrium-, Kalium- oder Cäsiumcarbonat, Alkalialkoholate wie Natrium- oder Kaliummethanolat, Natrium- oder Kaliumethanolat oder Natrium- oder Kalium-*tert*.-butylat, oder Alkalihydride wie Natrium- oder Kaliumhydrid; bevorzugt wird Natrium- oder Kaliumcarbonat verwendet [vgl. auch K. Hirota et al., J. Heterocycl. Chem. 27 (3), 717-721 (1990)].

Die Umsetzung wird vorzugsweise in einem alkoholischen Lösungsmittel, wie Methanol, Ethanol, Isopropanol oder *tert*.-Butanol, oder in einem inerten polar-aprotischen Lösungsmittel, wie *N,N-*Dimethylformamid (DMF), *N,N*'-Dimethylpropylenharnstoff (DMPU) oder *N*-Methylpyrrolidinon (NMP), in einem Temperaturbereich von +20°C bis +150°C durchgeführt, wobei sich eine Reaktionsführung unter Mikrowellenbestrahlung als vorteilhaft erwiesen hat. Bevorzugt wird Ethanol als Lösungsmittel verwendet.

Die Verbindungen der Formel (XIX) ihrerseits können nach einem geläufigen Verfahren durch baseninduzierte Kondensation eines Malonsäureesters der Formel (XXII) 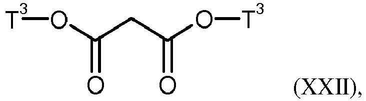 in welcher
- T³: für Methyl oder Ethyl steht,
mit einem Harnstoff-Derivat der Formel (XXIII) 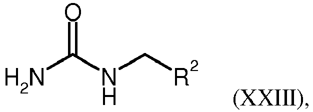 in welcher R² die oben angegebene Bedeutung hat,
hergestellt werden.

Die Kondensation (XXII) + (XXIII) → (XIX) erfolgt üblicherweise mit Hilfe eines Alkalialkoholats wie Natrium- oder Kaliummethanolat, Natrium- oder Kaliumethanolat oder Natrium- oder Kalium-*tert*.-butylat in dem betreffenden Alkohol als Lösungsmittel oder mit Hilfe eines Alkalihydrids wie Natrium- oder Kaliumhydrid in Tetrahydrofuran oder *N,N*-Dimethylformamid als inertem Lösungsmittel; bevorzugt wird Natriumethanolat in Ethanol verwendet. Die Reaktion wird im Allgemeinen in einem Temperaturbereich von +20°C bis +100°C durchgeführt.

Verbindungen der Formel (II), in welcher R¹ für Difluormethyl oder Trifluormethyl steht, lassen sich nach den zuvor (für R¹ = Methyl, Ethyl oder Wasserstoff) beschriebenen Verfahren nicht oder nur in schlechten Ausbeuten (< 10%) erhalten. Weiterer Gegenstand der vorliegenden Erfindung ist daher ein neues Verfahren zur Herstellung von Verbindungen der Formel (II-C) 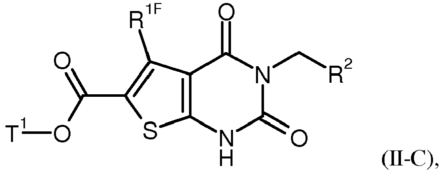 in welcher R² die oben angegebene Bedeutung hat,
- R^{1F}: für Difluormethyl oder Trifluormethyl steht
und
- T¹: für (C₁-C₄)-Alkyl oder Benzyl steht,
dadurch gekennzeichnet, dass man eine Verbindung der Formel (XIX) 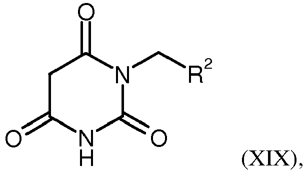 in welcher R² die oben angegebene Bedeutung hat,
mit Phosphoroxychlorid in eine Verbindung der Formel (XXIV) 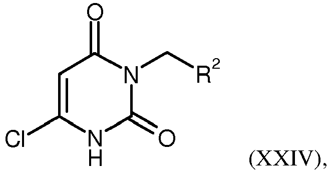 in welcher R² die oben angegebene Bedeutung hat,
überführt, dann in Gegenwart von überschüssigem Pyridin mit einem Anhydrid der Formel (XXV) 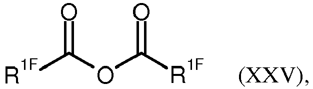 in welcher R^{1F} die oben angegebene Bedeutung hat,
zu einer Betain-Verbindung der Formel (XXVI) 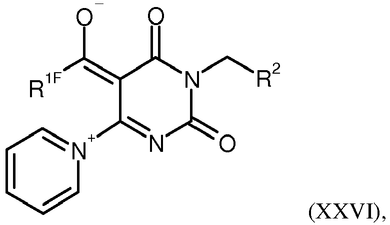 in welcher R^{1F} und R² die oben angegebenen Bedeutungen haben,
umsetzt und diese in Gegenwart einer Base mit einem α-Mercaptoessigsäureester der Formel (XXI) 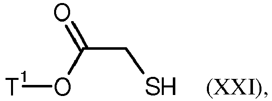 in welcher T¹ die oben angegebene Bedeutung hat,
zur Verbindung (II-C) kondensiert.

Die Umwandlung des Barbitursäure-Derivats (XIX) in das 6-Chlorpyrimidindion (XXIV) wird mit Hilfe von überschüssigem Phosphoroxychlorid in einem wässrigen Alkohol, wie Methanol oder Ethanol, als Lösungsmittel in einem Temperaturbereich von 0°C bis +100°C durchgeführt.

Die nachfolgende Überführung in das Pyridinium-Enolat-Betain (XXVI) erfolgt in Anlehnung an eine in der Literatur beschriebene Methode zur Synthese von 3-substituierten Chromon-Derivaten [I. Yokoe et al., Chem. Pharm. Bull. 42 (8), 1697-1699 (1994)], indem das 6-Chlorpyrimidindion (XXIV) in Gegenwart eines größeren Überschusses von Pyridin (ca. zehnfach) mit dem Anhydrid (XXV) behandelt wird. Die Reaktion wird in der Regel in einem Temperaturbereich von 0°C bis +40°C durchgeführt, und als inertes Lösungsmittel wird vorzugsweise Acetonitril verwendet.

Die Kondensation des Betains (XXVI) mit dem Mercaptoessigsäureester (XXI) zur Zielverbindung (II-C) schließlich wird auf analoge Weise durchgeführt wie zuvor für die Umsetzung (XX) + (XXI) → (II-B) beschrieben, wobei auch hier eine Reaktionsführung unter Mikrowellenbestrahlung von Vorteil ist.

Die am Uracil-N³-Atom temporär geschützten Intermediate der Formel (IX) aus Verfahren [B] können auf analoge Weise mit Hilfe der zuvor beschriebenen Reaktionssequenzen erhalten werden, indem dort anstelle der Verbindungen (XVI), (XVII) oder (XXIII) die entsprechenden Benzyl-Derivate eingesetzt werden, wie beispielsweise Verbindungen der Formel (XXVII) bzw. (XXVIII) 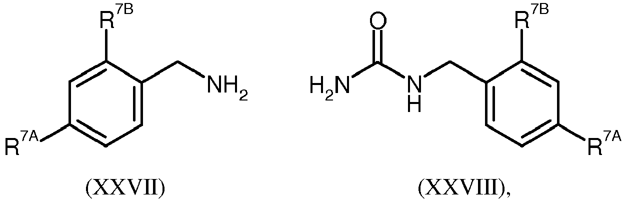 in welchen R^{7A} und R^{7B} die oben angegebenen Bedeutungen haben.

Verbindungen der Formel (IV), in welcher R¹ für Difluormethyl steht, können auch dadurch erhalten werden, dass man ausgehend von einer Verbindung der Formel (IV-A) 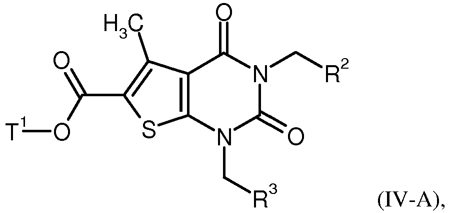 in welcher R², R³ und T¹ die oben angegebenen Bedeutungen haben,
zunächst durch Behandlung mit *N*-Bromsuccinimid (NBS) in Gegenwart von katalytischem 2,2'-Azobis(2-methylpropionitril) (AIBN) die Brommethyl-Verbindung (XXIX) 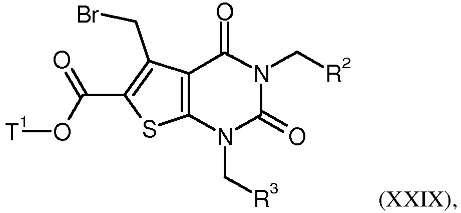 in welcher R², R³ und T¹ die oben angegebenen Bedeutungen haben,
herstellt, diese anschließend nach bekannten Methoden zur Formyl-Verbindung (XXX) 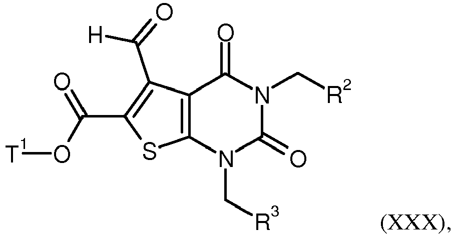 in welcher R², R³ und T¹ die oben angegebenen Bedeutungen haben,
oxidiert und dann zur Difluormethyl-Verbindung (IV-B) 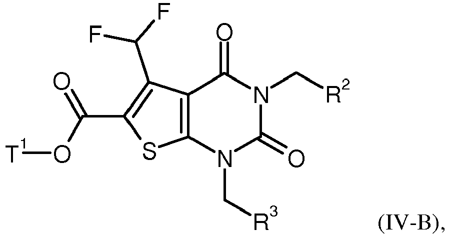 in welcher R², R³ und T¹ die oben angegebenen Bedeutungen haben,
fluoriert.

Die Oxidation im Reaktionsschritt (XXIX) → (XXX) wird vorzugsweise mit Hilfe von N-Methylmorpholin-N-oxid (NMO) in Gegenwart von Molekularsieb durchgeführt [vgl. z.B. D. R. Levine et al., J. Am. Chem. Soc. 2014, 136 (19), 7132-7139]. Die Umsetzung erfolgt im Allgemeinen in einem Temperaturbereich von -20°C bis +25°C in einem inerten Lösungsmittel wie beispielsweise Acetonitril.

Zur Deoxy-Fluorierung im Verfahrensschritt (XXX) → (IV-B) eignen sich bekannte Agentien wie Diethylaminoschwefeltrifluorid (DAST), Morpholinoschwefeltrifluorid (Morpho-DAST) oder Bis-(2-methoxyethyl)aminoschwefeltrifluorid (Deoxo-Fluor®). Die Umsetzung wird üblicherweise in einem Temperaturbereich von -10°C bis +25°C in einem inerten Lösungsmittel wie beispielsweise Dichlormethan oder Tetrahydrofuran durchgeführt.

Verbindungen der Formel (IV), in welcher R¹ für Fluormethyl steht, d.h. Verbindungen der Formel (IV-C) 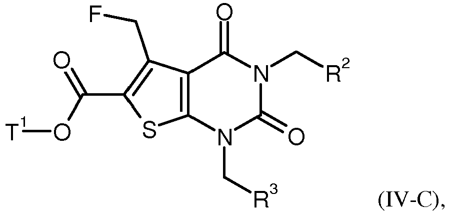 in welcher R², R³ und T¹ die oben angegebenen Bedeutungen haben,
können auf einfache Weise durch Substitutionsreaktion der oben beschriebenen Brommethyl-Verbindung (XXIX) mit einem Fluorid, wie beispielsweise Kaliumfluorid, Cäsiumfluorid oder Tetran-butylammoniumfluorid (TBAF), hergestellt werden. Die Umsetzung erfolgt in der Regel in einem Temperaturbereich von 0°C bis +60°C in einem inerten Lösungsmittel wie Aceton, Acetonitril, *N,N*-Dimethylformamid (DMF) oder Tetrahydrofuran oder Gemischen hiervon.

Verbindungen der Formel (IV), in welcher R¹ für 1-Fluorethyl steht, d.h. Verbindungen der Formel (IV-D) 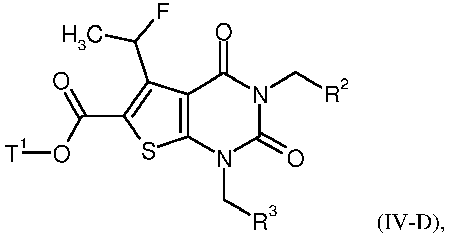 in welcher R², R³ und T¹ die oben angegebenen Bedeutungen haben,
lassen sich erhalten, indem man die oben beschriebene Formyl-Verbindung (XXX) mit Methylmagnesiumbromid unter üblichen Bedingungen bei ca. 0°C in die 1-Hydroxyethyl-Verbindung (XXXI) 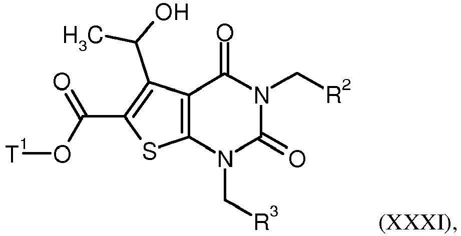 in welcher R², R³ und T¹ die oben angegebenen Bedeutungen haben,
überführt und diese dann auf analoge Weise, wie zuvor für die Transformation (XXX) → (IV-B) beschrieben, mit einem Fluorierungsagens wie Diethylaminoschwefeltrifluorid (DAST) oder Bis-(2-methoxyethyl)aminoschwefeltrifluorid (Deoxo-Fluor®) behandelt.

Die so erhältlichen Intermediate der Formel (IV-B), (IV-C) bzw. (IV-D) werden dann gemäß der zuvor unter [A-1] beschriebenen Reaktionssequenz (IV) → (V) und (V) + (VI) → (I) weiter zu den entsprechenden erfindungsgemäßen Verbindungen der Formel (I) umgesetzt.

Eine alternatives Verfahren zur Herstellung von Verbindungen der Formel (V-A) 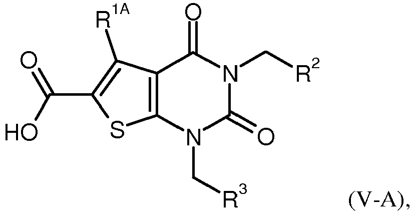 in welcher R² und R³ die oben angegebenen Bedeutungen haben
und
- R^{1A}: für Methyl oder Ethyl steht,
besteht darin, dass man eine Verbindung der Formel (XXXII) 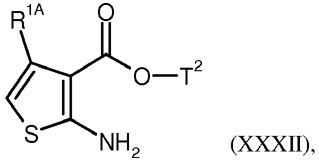 in welcher R^{1A} die oben angegebene Bedeutung hat
und
- T²: für Methyl oder Ethyl steht,
zunächst in Analogie zur oben beschriebenen Reaktionssequenz (XV) → (XVIII) → (II-A) und (II) → (IV) in eine Verbindung der Formel (XXXIII) 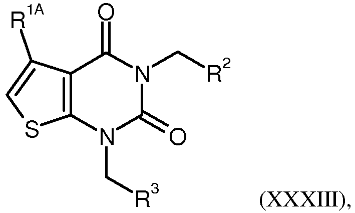 in welcher R^{1A}, R² und R³ die oben angegebenen Bedeutungen haben,
überführt, diese dann mit einer Mischung aus *N,N*-Dimethylformamid und Phosphoroxychlorid zu einer Verbindung der Formel (XXXIV) 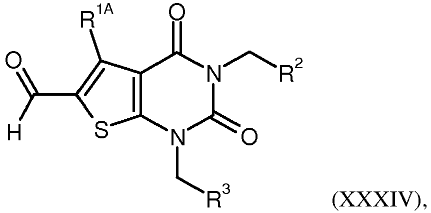 in welcher R^{1A}, R² und R³ die oben angegebenen Bedeutungen haben,
formyliert und anschließend zur Carboxyl-Verbindung der Formel (V-A) oxidiert.

Die Transformation (XXXIII) → (XXXIV) erfolgt unter den üblichen Bedingungen einer Vilsmaier-Haack-Reaktion mit einer Mischung aus *N,N*-Dimethylformamid und Phosphoroxychlorid, welche im großen Überschuss eingesetzt wird und gleichzeitig auch als Lösungsmittel dient. Die Umsetzung wird im Allgemeinen in einem Temperaturbereich von 0°C bis +120°C durchgeführt.

Als Oxidationsmittel für die Transformation (XXXIV) → (V-A) wird vorzugsweise Natriumchlorit in Gegenwart von Hydrogenphosphat-gepufferter Sulfaminsäure verwendet [vgl. z.B. WO 2008/115912-A1, Example 2; M. O. Anderson et al., J. Med. Chem. 2012, 55 (12), 5942-5950]. Die Umsetzung erfolgt üblicherweise in einem Temperaturbereich von -10°C bis +20°C in einem Gemisch von Wasser mit Aceton oder 1,4-Dioxan als inertem Lösungsmittel.

Die Verbindungen der Formeln (III), (VI), (XIII), (XIV), (XVI), (XVII), (XXI), (XXII), (XXIII), (XXV), (XXVII), (XXVIII) und (XXXII) sind entweder kommerziell erhältlich oder als solche in der Literatur beschrieben, oder sie können, ausgehend von anderen kommerziell erhältlichen Verbindungen, nach dem Fachmann geläufigen, literaturbekannten Methoden hergestellt werden. Zahlreiche detaillierte Vorschriften und weitere Literaturangaben befinden sich auch im Experimentellen Teil im Abschnitt zur Herstellung der Ausgangsverbindungen und Intermediate.

Die Herstellung der erfindungsgemäßen Verbindungen kann durch die folgenden Reaktionsschemata beispielhaft veranschaulicht werden:

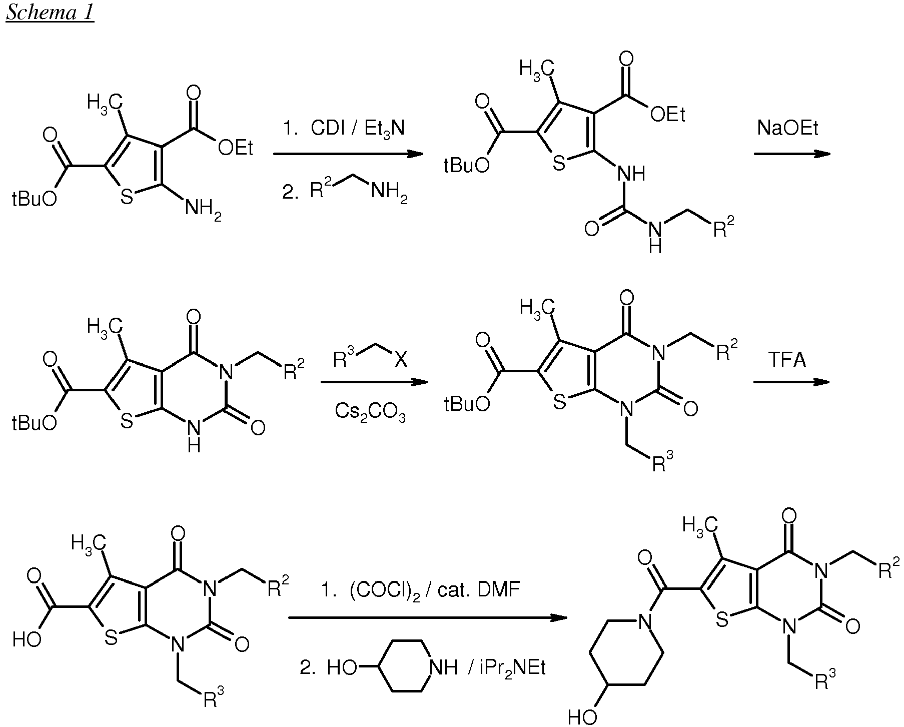

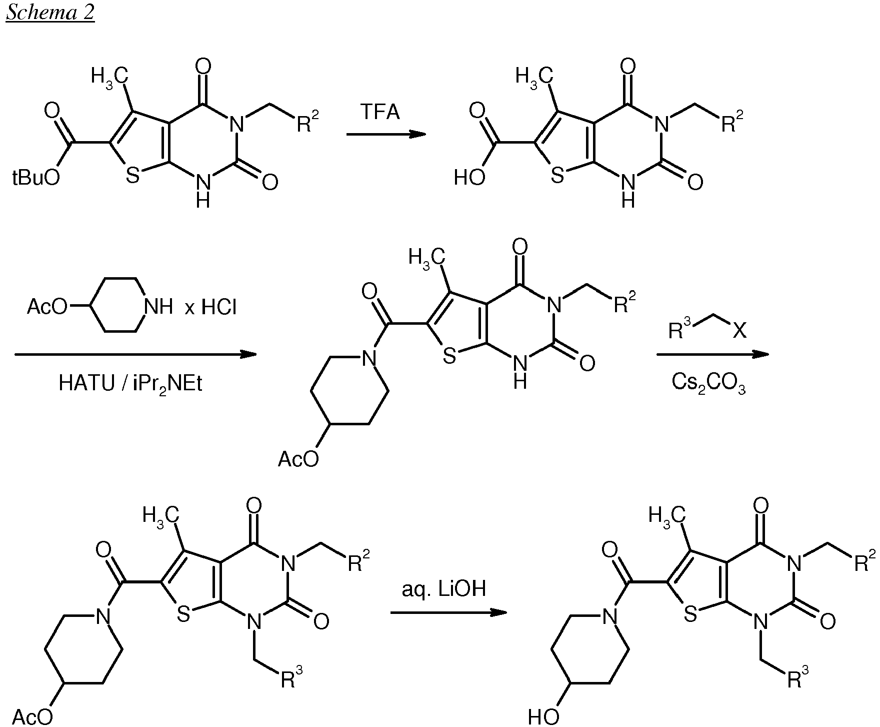

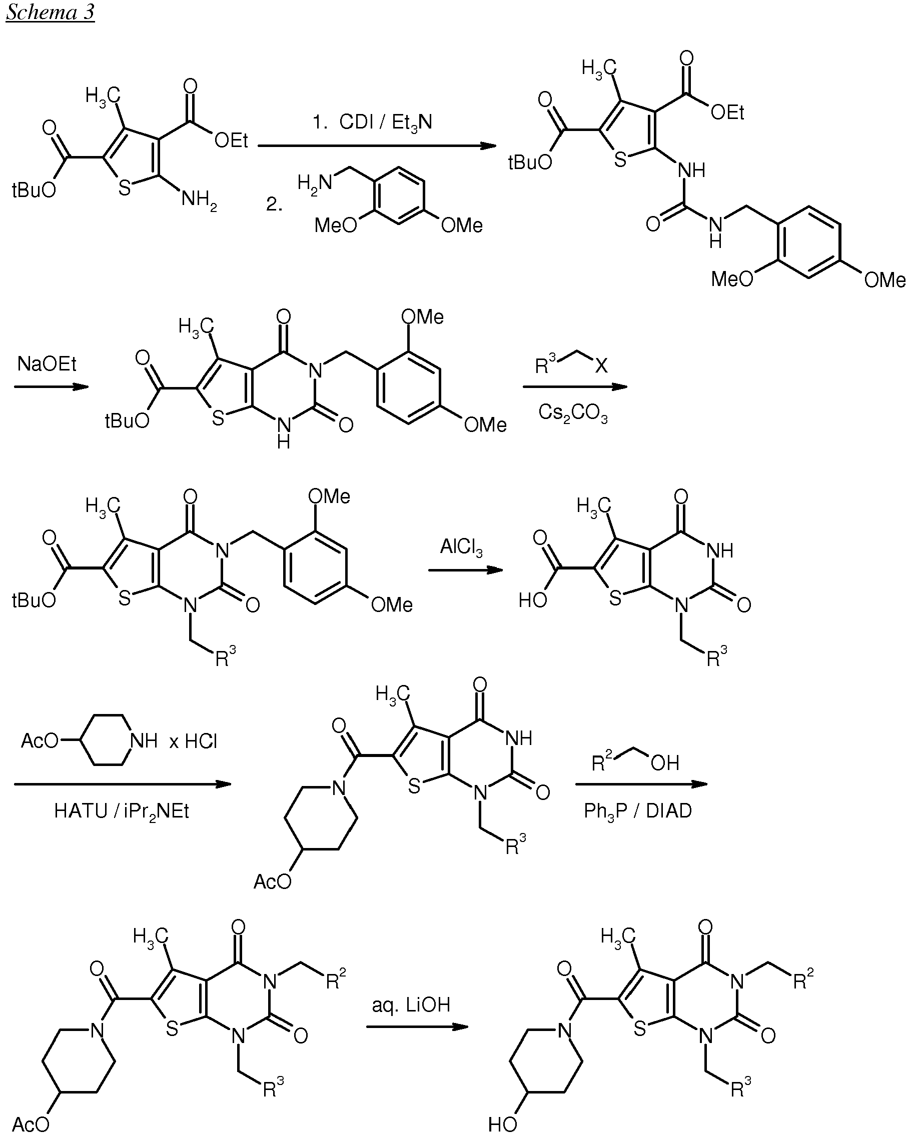

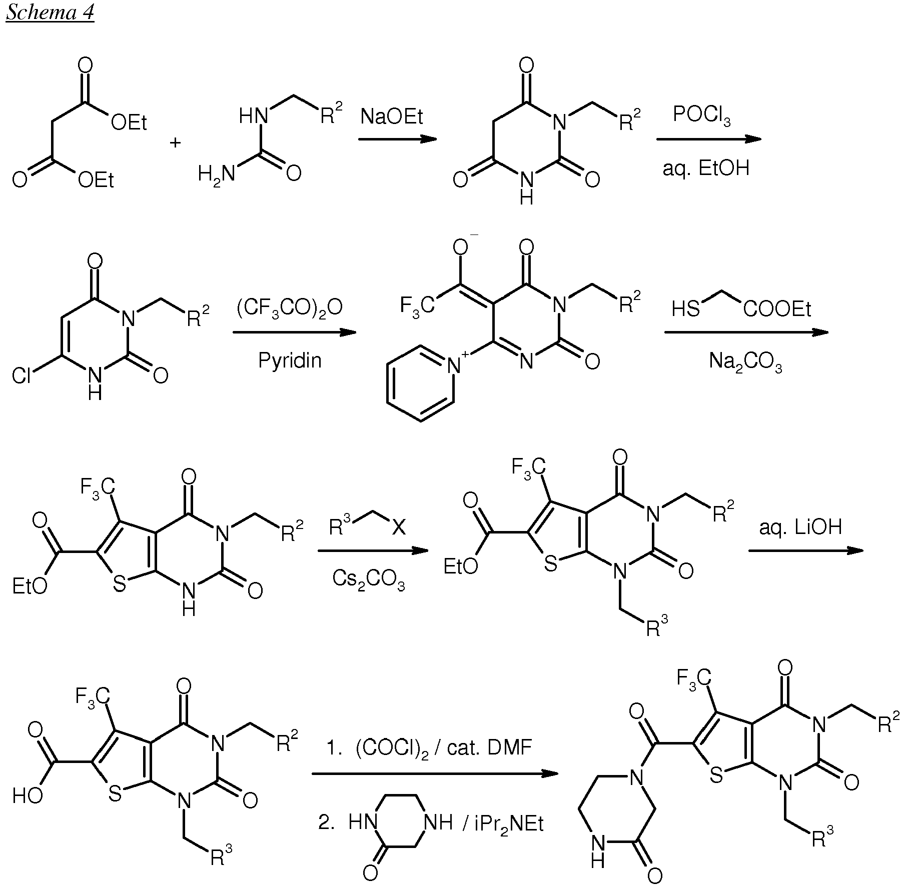

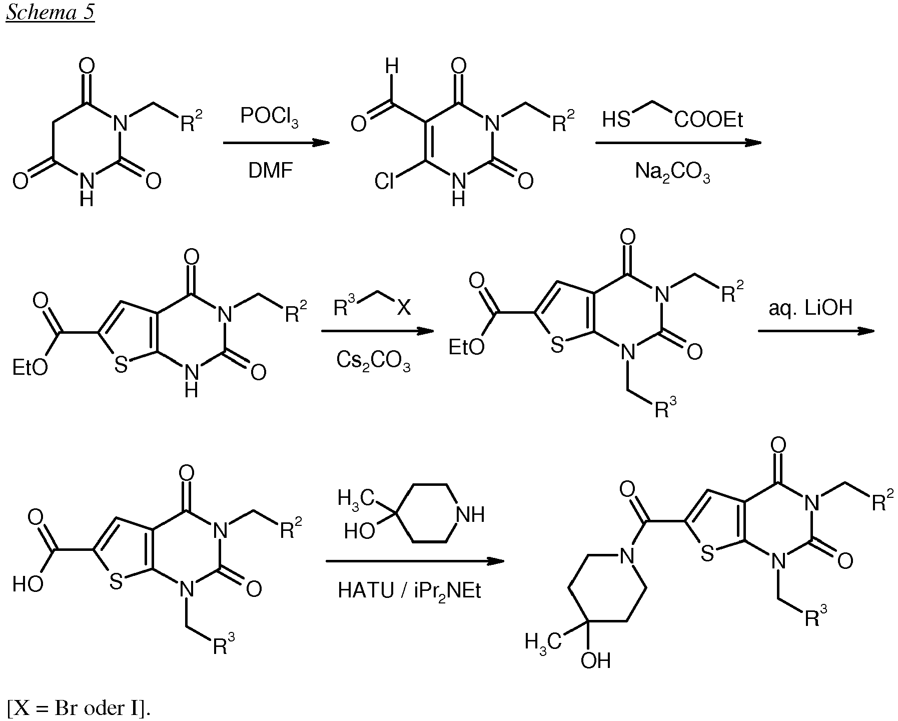

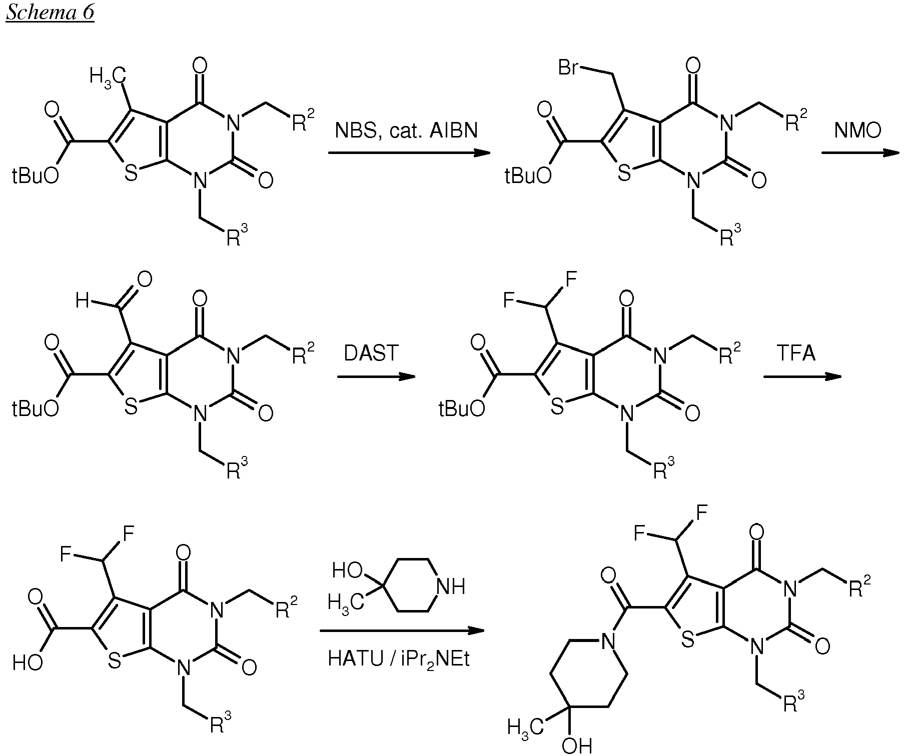

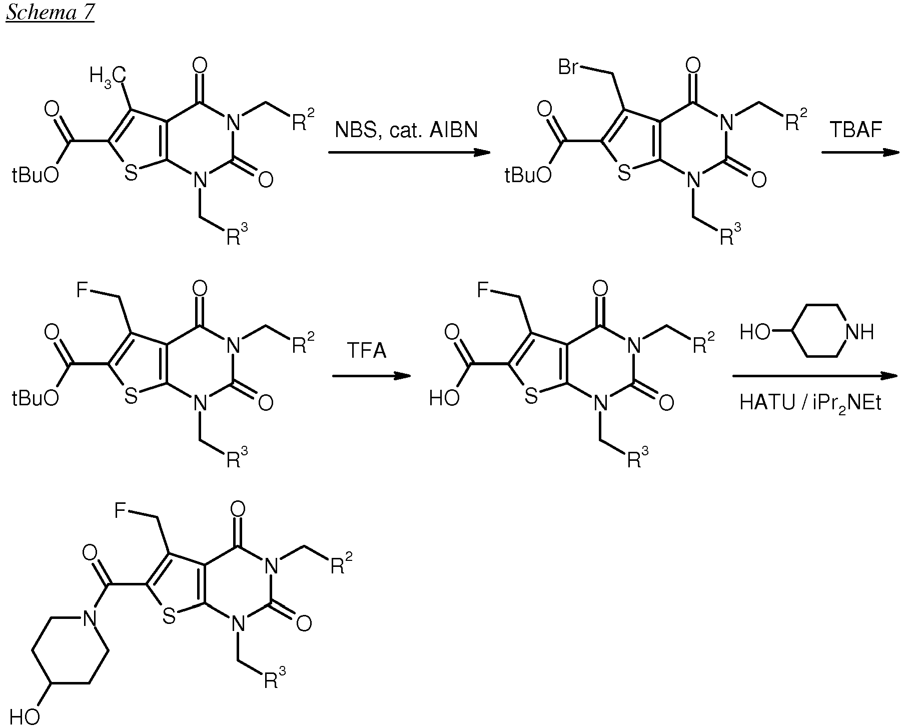

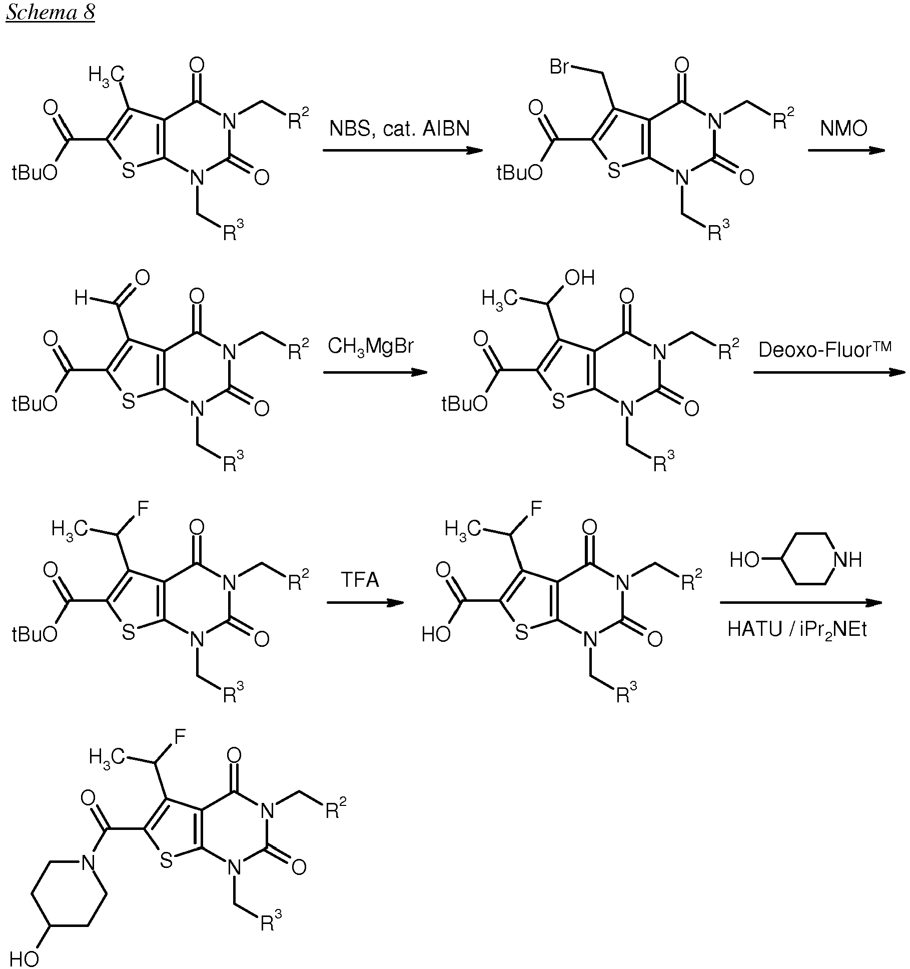

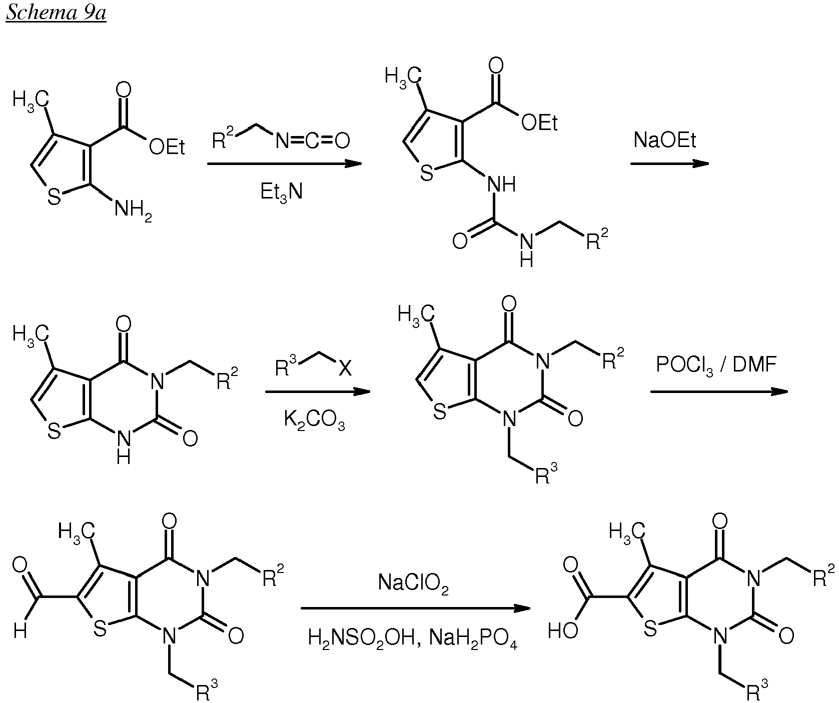

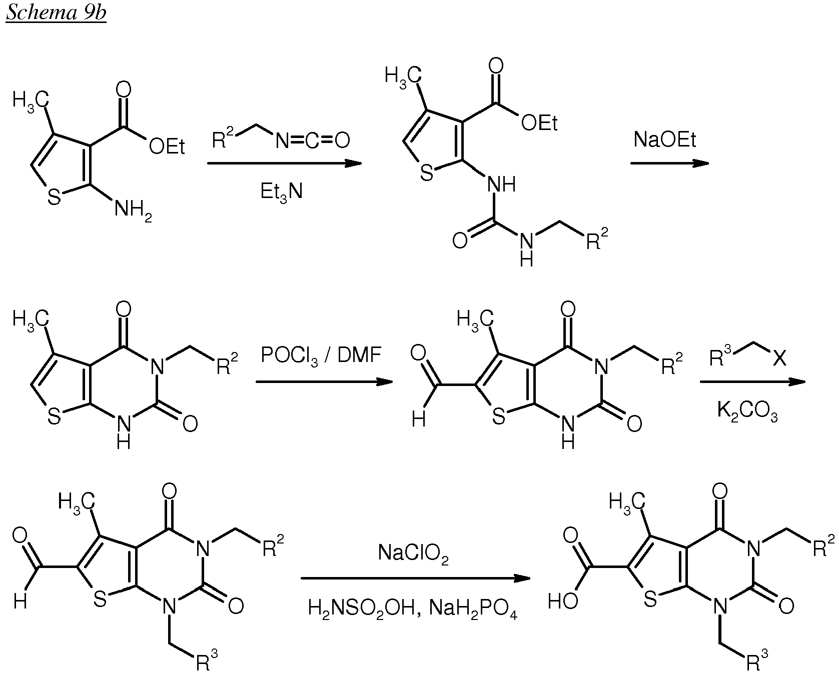

Die erfindungsgemäßen Verbindungen besitzen wertvolle pharmakologische Eigenschaften und können zur Vorbeugung und Behandlung von Erkrankungen bei Menschen und Tieren verwendet werden.

Die erfindungsgemäßen Verbindungen stellen potente und selektive Antagonisten des Adenosin-A2b-Rezeptors dar und eignen sich daher zur Behandlung und/oder Prävention von Erkrankungen und pathologischen Prozessen, insbesondere solcher, bei denen im Zuge eines Entzündungsgeschehens und/oder eines Gewebe- oder Gefäßumbaus der A2b-Rezeptor involviert ist.

Dazu zählen im Sinne der vorliegenden Erfindung insbesondere Erkrankungen wie die Gruppe der interstitiellen idiopathischen Pneumonien, zu denen die idiopathische pulmonale Fibrose (IPF), die akute interstitielle Pneumonie, nicht-spezifische interstitielle Pneumonien, lymphoide interstitielle Pneumonien, respiratorische Bronchiolitis mit interstitieller Lungenerkrankung, kryptogene organisierende Pneumonien, desquamative interstitielle Pneumonien und nicht-klassifizierbare idiopathische interstitielle Pneumonien gehören, ferner granulomatöse interstitielle Lungenerkrankungen, interstitielle Lungenerkrankungen bekannter Ursache und andere interstitielle Lungenerkrankungen unbekannter Ursache, die pulmonale arterielle Hypertonie (PAH) und andere Formen der pulmonalen Hypertonie (PH), das Bronchiolitis obliterans-Syndrom (BOS), die chronisch-obstruktive Lungenerkrankung (COPD), das akute Atemwegssyndrom (ARDS), akute Lungenschädigung (ALI), alpha-1-Antitrypsin-Defizienz (AATD), Lungenemphysem (z.B. durch Zigarettenrauch induziertes Lungenemphysem), zystische Fibrose (CF), entzündliche und fibrotische Erkrankungen der Niere, chronische Darmentzündungen (IBD, Morbus Crohn, Colitis ulcerosa), Peritonitis, Peritonealfibrose, rheumatoide Erkrankungen, multiple Sklerose, entzündliche und fibrotische Hauterkrankungen, Sichelzellanämie sowie entzündliche und fibrotische Augenerkrankungen.

Die erfindungsgemäßen Verbindungen können weiterhin verwendet werden zur Behandlung und/ oder Prävention von asthmatischen Erkrankungen unterschiedlicher Schweregrade mit intermittierendem oder persistierendem Verlauf (refraktives Asthma, bronchiales Asthma, allergisches Asthma, intrinsisches Asthma, extrinsisches Asthma, durch Medikamente oder durch Staub induziertes Asthma), von verschiedenen Formen der Bronchitis (chronische Bronchitis, infektiöse Bronchitis, eosinophile Bronchitis), von Bronchiektasien, Pneumonie, Farmerlunge und verwandten Krankheiten, Husten- und Erkältungskrankheiten (chronischer entzündlicher Husten, iatrogener Husten), Nasenschleimhautentzündungen (einschließlich medikamentöse Rhinitis, vasomotorische Rhinitis und jahreszeitabhängige, allergische Rhinitis, z.B. Heuschnupfen) und von Polypen.

Die erfindungsgemäßen Verbindungen können darüber hinaus zur Behandlung und/oder Prävention von kardiovaskulären Erkrankungen eingesetzt werden, wie beispielsweise Bluthochdruck (Hypertonie), Herzinsuffizienz, koronare Herzerkrankung, stabile und instabile Angina pectoris, renale Hypertonie, periphere und kardiale Gefäßerkrankungen, Arrhythmien, Rhythmusstörungen der Vorhöfe und der Kammern sowie Überleitungsstörungen wie beispielsweise atrio-ventrikuläre Blockaden des Grades I-III, supraventrikuläre Tachyarrhythmie, Vorhofflimmern, Vorhofflattern, Kammerflimmern, Kammerflattern, ventrikuläre Tachyarrhythmie, Torsade de pointes-Tachykardie, Extrasystolen des Vorhofs und des Ventrikels, AV-junktionale Extrasystolen, Sick-Sinus-Syndrom, Synkopen, AV-Knoten-Reentry-Tachykardie, Wolff-Parkinson-White-Syndrom, akutes Koronarsyndrom (ACS), autoimmune Herzerkrankungen (Perikarditis, Endokarditis, Valvolitis, Aortitis, Kardiomyopathien), Boxerkardiomyopathie, Aneurysmen, Schock wie kardiogener Schock, septischer Schock und anaphylaktischer Schock, ferner zur Behandlung und/oder Prävention von thromboembolischen Erkrankungen und Ischämien, wie myokardiale Ischämie, Myokardinfarkt, Hirnschlag, Herzhypertrophie, transistorische und ischämische Attacken, Präeklampsie, entzündliche kardiovaskuläre Erkrankungen, Spasmen der Koronararterien und peripherer Arterien, Ödembildung wie beispielsweise pulmonales Ödem, Hirnödem, renales Ödem oder Herzinsuffizienz-bedingtes Ödem, periphere Durchblutungsstörungen, Reperfusionsschäden, arterielle und venöse Thrombosen, Mikroalbuminurie, Herzmuskelschwäche, endotheliale Dysfunktion, mikro- und makrovaskuläre Schädigungen (Vaskulitis), sowie zur Verhinderung von Restenosen beispielsweise nach Thrombolyse-Therapien, percutan-transluminalen Angioplastien (PTA), percutan-transluminalen Koronarangioplastien (PTCA), Herztransplantationen und Bypass-Operationen.

Im Sinne der vorliegenden Erfindung umfasst der Begriff Herzinsuffizienz sowohl akute als auch chronische Erscheinungsformen der Herzinsuffizienz wie auch spezifische oder verwandte Krankheitsformen hiervon, wie akute dekompensierte Herzinsuffizienz, Rechtsherzinsuffizienz, Linksherzinsuffizienz, Globalinsuffizienz, ischämische Kardiomyopathie, dilatative Kardiomyopathie, hypertrophe Kardiomyopathie, idiopathische Kardiomyopathie, angeborene Herzfehler, Herzklappenfehler, Herzinsuffizienz bei Herzklappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen sowie diastolische und systolische Herzinsuffizienz.

Die erfindungsgemäßen Verbindungen eignen sich außerdem zur Behandlung und/oder Prävention von Nierenerkrankungen, insbesondere von Niereninsuffizienz und Nierenversagen. Im Sinne der vorliegenden Erfindung umfassen die Begriffe Niereninsuffizienz und Nierenversagen sowohl akute als auch chronische Erscheinungsformen hiervon wie auch diesen zugrundeliegende oder verwandte Nierenerkrankungen, wie renale Hypoperfusion, intradialytische Hypotonie, obstruktive Uropathie, Glomerulopathien, Glomerulonephritis, akute Glomerulonephritis, Glomerulosklerose, tubulointerstitielle Erkrankungen, nephropathische Erkrankungen wie primäre und angeborene Nierenerkrankung, Nierenentzündung, immunologische Nierenerkrankungen wie Nierentransplantat-Abstoßung und Immunkomplex-induzierte Nierenerkrankungen, durch toxische Substanzen induzierte Nephropathie, Kontrastmittel-induzierte Nephropathie, diabetische und nicht-diabetische Nephropathie, Pyelonephritis, Nierenzysten, Nephrosklerose, hypertensive Nephrosklerose und nephrotisches Syndrom, welche diagnostisch beispielsweise durch abnorm verminderte Kreatinin- und/oder Wasser-Ausscheidung, abnorm erhöhte Blutkonzentrationen von Harnstoff, Stickstoff, Kalium und/oder Kreatinin, veränderte Aktivität von Nierenenzymen wie z.B. Glutamylsynthetase, veränderte Urinosmolarität oder Urinmenge, erhöhte Mikroalbuminurie, Makroalbuminurie, Läsionen an Glomerula und Arteriolen, tubuläre Dilatation, Hyperphosphatämie und/oder die Notwendigkeit zur Dialyse charakterisiert werden können. Die vorliegende Erfindung umfasst auch die Verwendung der erfindungsgemäßen Verbindungen zur Behandlung und/oder Prävention von Folgeerscheinungen einer Niereninsuffizienz, wie beispielsweise Hypertonie, Lungenödem, Herzinsuffizienz, Urämie, Anämie, Elektrolytstörungen (z.B. Hyperkalämie, Hyponaträmie) und Störungen im Knochen- und Kohlenhydrat-Metabolismus.

Darüber hinaus sind die erfindungsgemäßen Verbindungen zur Behandlung und/oder Prävention von Erkrankungen des Urogenitalsystems geeignet, wie beispielsweise benignes Prostata-Syndrom (BPS), benigne Prostatahyperplasie (BPH), benigne Prostatavergrößerung (BPE), Blasenentleerungsstörungen (BOO), untere Harnwegssyndrome (LUTS), neurogene überaktive Blase (OAB), Inkontinenz wie beispielsweise Misch-, Drang-, Stress- oder Überlauf-Inkontinenz (MUI, UUI, SUI, OUI), Beckenschmerzen sowie erektile Dysfunktion und weibliche sexuelle Dysfunktion.

Zudem besitzen die erfindungsgemäßen Verbindungen anti-inflammatorische Wirkung und können daher als entzündungshemmende Mittel zur Behandlung und/oder Prävention von Sepsis (SIRS), multiplem Organversagen (MODS, MOF), entzündlichen Erkrankungen der Niere, chronischen Darmentzündungen (IBD, Morbus Crohn, Colitis ulcerosa), Pankreatitis, Peritonitis, Cystitis, Urethritis, Prostatitis, Epidimytitis, Oophoritis, Salpingitis, Vulvovaginitis, rheumatoiden Erkrankungen, entzündlichen Erkrankungen des Zentralnervensystems, multipler Sklerose, entzündlichen Hauterkrankungen und entzündlichen Augenerkrankungen eingesetzt werden.

Die erfindungsgemäßen Verbindungen sind ferner zur Behandlung und/oder Prävention von fibrotischen Erkrankungen der inneren Organe, wie beispielsweise der Lunge, des Herzens, der Niere, des Knochenmarks und insbesondere der Leber, sowie von dermatologischen Fibrosen und fibrotischen Erkrankungen des Auges geeignet. Im Sinne der vorliegenden Erfindung umfasst der Begriff fibrotische Erkrankungen insbesondere solche Erkrankungen wie Leberfibrose, Leberzirrhose, Lungenfibrose, Endomyokardfibrose, Nephropathie, Glomerulonephritis, interstitielle Nierenfibrose, fibrotische Schäden in Folge von Diabetes, Knochenmarksfibrose, Peritonealfibrose und ähnliche fibrotische Erkrankungen, Sklerodermie, Morphaea, Keloide, hypertrophe Narbenbildung, Naevi, diabetische Retinopathie, proliferative Vitroretinopathie und Erkrankungen des Bindegewebes (z.B. Sarkoidose). Die erfindungsgemäßen Verbindungen können ebenso verwendet werden zur Förderung der Wundheilung, zur Bekämpfung postoperativer Narbenbildung, z.B. nach Glaukom-Operationen, und zu kosmetischen Zwecken bei alternder oder verhornender Haut.

Auch können die erfindungsgemäßen Verbindungen zur Behandlung und/oder Prävention von Anämien verwendet werden, wie hämolytischen Anämien, insbesondere Hämoglobinopathien wie Sichelzellanämie und Thalassämien, megaloblastären Anämien, Eisenmangel-Anämien, Anämien durch akuten Blutverlust, Verdrängungsanämien und aplastischen Anämien.

Die erfindungsgemäßen Verbindungen sind zudem zur Behandlung von Krebserkrankungen geeignet, wie beispielsweise von Hautkrebs, Hirntumoren, Brustkrebs, Knochenmarktumoren, Leukämien, Liposarcomen, Karzinomen des Magen-Darm-Traktes, der Leber, Bauchspeicheldrüse, Lunge, Niere, Harnleiter, Prostata und des Genitaltraktes sowie von bösartigen Tumoren des lymphoproliferativen Systems, wie z.B. Hodgkin's und Non-Hodgkin's Lymphom.

Darüber hinaus können die erfindungsgemäßen Verbindungen eingesetzt werden zur Behandlung und/oder Prävention von Arteriosklerose, Lipidstoffwechselstörungen und Dyslipidämien (Hypolipoproteinämie, Hypertriglyceridämie, Hyperlipidämie, kombinierte Hyperlipidämien, Hypercholesterolämie, Abetalipoproteinämie, Sitosterolämie), Xanthomatose, Tangier-Krankheit, Fettsucht (Adipositas), Fettleibigkeit (Obesitas), metabolischen Erkrankungen (Metabolisches Syndrom, Hyperglykämie, Insulin-abhängiger Diabetes, nicht-Insulin-abhängiger Diabetes, Gestationsdiabetes, Hyperinsulinämie, Insulinresistenz, Glukose-Intoleranz und diabetische Spätfolgen wie Retinopathie, Nephropathie und Neuropathie), von Erkrankungen des Gastrointestinaltrakts und des Abdomen (Glossitis, Gingivitis, Periodontitis, Oesophagitis, eosinophile Gastroenteritis, Mastocytose, Morbus Crohn, Colitis, Proctitis, Pruritis ani, Diarrhöe, Zöliakie, Hepatitis, Leberfibrose, Leberzirrhose, Pankreatitis und Cholecystitis), von Erkrankungen des Zentralen Nervensystems und von neurodegenerativen Störungen (Schlaganfall, Alzheimer'sche Krankheit, Parkinson'sche Krankheit, Demenz, Epilepsie, Depressionen, Multiple Sklerose), Immunerkrankungen, Schilddrüsenerkrankungen (Hyperthyreose), Hauterkrankungen (Psoriasis, Akne, Ekzeme, Neurodermitis, vielfältige Formen der Dermatitis wie z.B. Dermatitis abacribus, Dermatitis actinica, Dermatitis allergica, Dermatitis ammoniacalis, Dermatitis artefacta, Dermatitis autogenica, Dermatitis atrophicans, Dermatitis calorica, Dermatitis combustionis, Dermatitis congelationis, Dermatitis cosmetica, Dermatitis escharotica, Dermatitis exfoliativa, Dermatitis gangraenose, Dermatitis haemostatica, Dermatitis herpetiformis, Dermatitis lichenoides, Dermatitis linearis, Dermatitis maligna, Dermatitis medimencatosa, Dermatitis palmaris et plantaris, Dermatitis parasitaria, Dermatitis photoallergica, Dermatitis phototoxica, Dermatitis pustularis, Dermatitis seborrhoica, Dermatitis solaris, Dermatitis toxica, Dermatitis ulcerosa, Dermatitis veneata, infektiöse Dermatitis, pyogene Dermatitis und Rosazea-artige Dermatitis, sowie Keratitis, Bullosis, Vasculitis, Cellulitis, Panniculitis, Lupus erythematodes, Erythema, Lymphome, Hautkrebs, Sweet-Syndrom, Weber-Christian-Syndrom, Narbenbildung, Warzenbildung, Frostbeulen), von entzündlichen Augenerkrankungen (Saccoidosis, Blepharitis, Conjunctivitis, Iritis, Uveitis, Chorioiditis, Ophthalmitis), viralen Erkrankungen (durch Influenza-, Adeno- und Coronaviren, wie z.B. HPV, HCMV, HIV, SARS), von Erkrankungen des Skelettknochens und der Gelenke sowie der Skelettmuskel (vielfältige Formen der Arthritis wie z.B. Arthritis alcaptonurica, Arthritis ankylosans, Arthritis dysenterica, Arthritis exsudativa, Arthritis fungosa, Arthritis gonorrhoica, Arthritis mutilans, Arthritis psoriatica, Arthritis purulenta, Arthritis rheumatica, Arthritis serosa, Arthritis syphilitica, Arthritis tuberculosa, Arthritis urica, Arthritis villonodularis pigmentosa, atypische Arthritis, hämophile Arthritis, juvenile chronische Arthritis, rheumatoide Arthritis und metastatische Arthritis, des weiteren das Still-Syndrom, Felty-Syndrom, Sjörgen-Syndrom, Clutton-Syndrom, Poncet-Syndrom, Pott-Syndrom und Reiter-Syndrom, vielfältige Formen der Arthropathien wie z.B. Arthropathie deformans, Arthropathie neuropathica, Arthropathie ovaripriva, Arthropathie psoriatica und Arthropathie tabica, systemische Sklerosen, vielfältige Formen der entzündlichen Myopathien wie z.B. Myopathie epidemica, Myopathie fibrosa, Myopathie myoglobinurica, Myopathie ossificans, Myopathie ossificans neurotica, Myopathie ossificans progressiva multiplex, Myopathie purulenta, Myopathie rheumatica, Myopathie trichinosa, Myopathie tropica und Myopathie typhosa, sowie das Günther-Syndrom und das Münchmeyer-Syndrom), von entzündlichen Arterienveränderungen (vielfältige Formen der Arteritis wie z.B. Endarteritis, Mesarteritis, Periarteritis, Panarteritis, Arteritis rheumatica, Arteritis deformans, Arteritis temporalis, Arteritis cranialis, Arteritis gigantocellularis und Arteritis granulomatosa, sowie das Horton-Syndrom, Churg-Strauss-Syndrom und die Takayasu-Arteritis), des Muckle-Well-Syndroms, der Kikuchi-Krankheit, von Polychondritis, Sklerodermia sowie von weiteren Erkrankungen mit einer entzündlichen oder immunologischen Komponente, wie beispielsweise Katarakt, Kachexie, Osteoporose, Gicht, Inkontinenz, Lepra, Sezary-Syndrom und paraneoplastisches Syndrom, bei Abstossungsreaktionen nach Organtransplantationen und zur Wundheilung und Angiogenese insbesondere bei chronischen Wunden.

Aufgrund ihres Eigenschaftsprofils eignen sich die erfindungsgemäßen Verbindungen insbesondere zur Behandlung und/oder Prävention von interstitiellen Lungenerkrankungen, vor allem der idiopathischen Lungenfibrose (IPF), sowie von pulmonaler Hypertonie (PH), Bronchiolitis obliterans-Syndrom (BOS), chronisch-obstruktiven Lungenerkrankungen (COPD), Asthma, zystischer Fibrose (CF), Myokardinfarkt, Herzinsuffizienz und Hämoglobinopathien, insbesondere Sichelzellanämie.

Die zuvor genannten, gut charakterisierten Krankheiten des Menschen können mit vergleichbarer Ätiologie auch in anderen Säugetieren vorkommen und dort ebenfalls mit den Verbindungen der vorliegenden Erfindung behandelt werden.

Im Sinne der vorliegenden Erfindung umfasst der Begriff "Behandlung" oder "behandeln" ein Hemmen, Verzögern, Aufhalten, Lindern, Abschwächen, Einschränken, Verringern, Unterdrücken, Zurückdrängen oder Heilen einer Krankheit, eines Leidens, einer Erkrankung, einer Verletzung oder einer gesundheitlichen Störung, der Entfaltung, des Verlaufs oder des Fortschreitens solcher Zustände und/oder der Symptome solcher Zustände. Der Begriff "Therapie" wird hierbei als synonym mit dem Begriff "Behandlung" verstanden.

Die Begriffe "Prävention", "Prophylaxe" oder "Vorbeugung" werden im Rahmen der vorliegenden Erfindung synonym verwendet und bezeichnen das Vermeiden oder Vermindern des Risikos, eine Krankheit, ein Leiden, eine Erkrankung, eine Verletzung oder eine gesundheitliche Störung, eine Entfaltung oder ein Fortschreiten solcher Zustände und/oder die Symptome solcher Zustände zu bekommen, zu erfahren, zu erleiden oder zu haben.

Die Behandlung oder die Prävention einer Krankheit, eines Leidens, einer Erkrankung, einer Verletzung oder einer gesundheitlichen Störung können teilweise oder vollständig erfolgen.

Weiterer Gegenstand der vorliegenden Erfindung ist somit die Verwendung der erfindungsgemäßen Verbindungen zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Weiterer Gegenstand der vorliegenden Erfindung ist ein Arzneimittel, enthaltend mindestens eine der erfindungsgemäßen Verbindungen, zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen in einem Verfahren zur Behandlung und/oder Prävention von Erkrankungen, insbesondere der zuvor genannten Erkrankungen.

Weiterer Gegenstand der vorliegenden Erfindung ist ein Verfahren zur Behandlung und/oder Prävention von Erkrankungen, insbesondere der zuvor genannten Erkrankungen, unter Verwendung einer wirksamen Menge von mindestens einer der erfindungsgemäßen Verbindungen.

Die erfindungsgemäßen Verbindungen können allein oder bei Bedarf in Kombination mit einer oder mehreren anderen pharmakologisch wirksamen Substanzen eingesetzt werden, solange diese Kombination nicht zu unerwünschten und inakzeptablen Nebenwirkungen führt. Weiterer Gegenstand der vorliegenden Erfindung sind daher Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen. Als hierfür geeignete Kombinationswirkstoffe seien beispielhaft und vorzugsweise genannt:
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- Verbindungen, die den Abbau von cyclischem Guanosinmonophosphat (cGMP) und/oder cyclischem Adenosinmonophosphat (cAMP) inhibieren, wie beispielsweise Inhibitoren der Phosphodiesterasen (PDE) 1, 2, 3, 4 und/oder 5, insbesondere PDE 5-Inhibitoren wie Sildenafil, Vardenafil, Tadalafil, Udenafil, Dasantafil, Avanafil, Mirodenafil oder Lodenafil;
- NO- und Häm-unabhängige Aktivatoren der löslichen Guanylatcyclase (sGC), wie insbesondere die in WO 01/19355, WO 01/19776, WO 01/19778, WO 01/19780, WO 02/070462 und WO 02/070510 beschriebenen Verbindungen;
- NO-unabhängige, jedoch Häm-abhängige Stimulatoren der löslichen Guanylatcyclase (sGC), wie insbesondere Riociguat sowie die in WO 00/06568, WO 00/06569, WO 02/42301, WO 03/095451, WO 2011/147809, WO 2012/004258, WO 2012/028647 und WO 2012/059549 beschriebenen Verbindungen;
- Prostacyclin-Analoga und IP-Rezeptor-Agonisten, wie beispielhaft und vorzugsweise Iloprost, Beraprost, Treprostinil, Epoprostenol oder NS-304;
- Endothelin-Rezeptor-Antagonisten, wie beispielhaft und vorzugsweise Bosentan, Darusentan, Ambrisentan oder Sitaxsentan;
- Verbindungen, die die humane neutrophile Elastase (HNE) inhibieren, wie beispielhaft und vorzugsweise Sivelestat oder DX-890 (Reltran);
- die Signaltransduktionskaskade inhibierende Verbindungen, beispielhaft und vorzugsweise aus der Gruppe der Kinase-Inhibitoren, insbesondere aus der Gruppe der Tyrosinkinase- und/oder Serin/Threoninkinase-Inhibitoren, wie beispielhaft und vorzugsweise Nintedanib, Dasatinib, Nilotinib, Bosutinib, Regorafenib, Sorafenib, Sunitinib, Cediranib, Axitinib, Telatinib, Imatinib, Brivanib, Pazopanib, Vatalanib, Gefitinib, Erlotinib, Lapatinib, Canertinib, Lestaurtinib, Pelitinib, Semaxanib oder Tandutinib;
- Verbindungen, die den Ab- und Umbau der Extrazellulärmatrix inhibieren, beispielhaft und vorzugsweise Inhibitoren der Matrix-Metalloproteasen (MMPs), insbesondere Inhibitoren von Stromelysin, Kollagenasen, Gelatinasen und Aggrecanasen (hierbei vor allem von MMP-1, MMP-3, MMP-8, MMP-9, MMP-10, MMP-11 und MMP-13) sowie der Metallo-Elastase (MMP-12);
- Verbindungen, die die Bindung von Serotonin an dessen Rezeptor blockieren, beispielhaft und vorzugsweise Antagonisten des 5-HT_{2B}-Rezeptors wie PRX-08066;
- Antagonisten von Wachstumsfaktoren, Zytokinen und Chemokinen, beispielhaft und vorzugsweise Antagonisten von THF-β, CTGF, IL-1, IL-4, IL-5, IL-6, IL-8, IL-13 und Integrinen;
- die Rho-Kinase inhibierende Verbindungen, wie beispielhaft und vorzugsweise Fasudil, Y-27632, SLx-2119, BF-66851, BF-66852, BF-66853, KI-23095 oder BA-1049;
- Verbindungen, die die lösliche Epoxidhydrolase (sEH) inhibieren, wie beispielsweise *N,N'*-Dicyclohexylharnstoff, 12-(3-Adamantan-1-yl-ureido)-dodecansäure oder 1-Adamantan-1-yl-3-{5-[2-(2-ethoxyethoxy)ethoxy]pentyl} -harnstoff;
- den Energiestoffwechsel des Herzens beeinflussende Verbindungen, wie beispielhaft und vorzugsweise Etomoxir, Dichloracetat, Ranolazin oder Trimetazidin;
- anti-obstruktiv wirkende Mittel, wie sie z.B. zur Therapie der chronisch-obstruktiven Lungenerkrankung (COPD) oder eines Asthma bronchiale eingesetzt werden, beispielhaft und vorzugsweise aus der Gruppe der inhalativ oder systemisch angewendeten beta-adrenergen Rezeptor-Agonisten (beta-Mimetika) und der inhalativ angewendeten anti-muscarinergen Substanzen;
- entzündungshemmende, immunmodulierende, immunsuppressive und/oder zytotoxische Mittel, beispielhaft und vorzugsweise aus der Gruppe der systemisch oder inhalativ angewendeten Corticosteroide sowie Acetylcystein, Montelukast, Azathioprin, Cyclophosphamid, Hydroxycarbamid, Azithromycin, IFN-γ, Pirfenidon oder Etanercept;
- antifibrotisch wirkende Mittel, wie beispielhaft und vorzugsweise Lysophosphatidsäure-Rezeptor 1 (LPA-1)-Antagonisten, Lysyloxidase (LOX)-Inhibitoren, Lysyloxidase-like-2-Inhibitoren, Vasoaktives intestinales Peptid (VIP), VIP-Analoga, αᵥβ₆-Integrin-Antagonisten, Cholchicin, IFN-β, D-Penicillamin, Inhibitoren des WNT-Signalwegs oder CCR2-Antagonisten;
- antithrombotisch wirkende Mittel, beispielhaft und vorzugsweise aus der Gruppe der Thrombozytenaggregationshemmer, der Antikoagulantien und der profibrinolytischen Substanzen;
- den Blutdruck senkende Wirkstoffe, beispielhaft und vorzugsweise aus der Gruppe der Calcium-Antagonisten, Angiotensin AII-Antagonisten, ACE-Hemmer, Vasopeptidase-Inhibitoren, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika;
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten; und/oder
- Chemotherapeutika, wie sie z.B. zur Therapie von Neubildungen (Neoplasien) der Lunge oder anderer Organe eingesetzt werden.

Bei einer bevorzugten Ausführungsform der Erfindung werden die erfindungsgemäßen Verbindungen in Kombination mit einem beta-adrenergen Rezeptor-Agonisten, wie beispielhaft und vorzugsweise Albuterol, Isoproterenol, Metaproterenol, Terbutalin, Fenoterol, Formoterol, Reproterol, Salbutamol oder Salmeterol, verabreicht.

Bei einer bevorzugten Ausführungsform der Erfindung werden die erfindungsgemäßen Verbindungen in Kombination mit einer anti-muscarinergen Substanz, wie beispielhaft und vorzugsweise Ipratropiumbromid, Tiotropiumbromid oder Oxitropiumbromid, verabreicht.

Bei einer bevorzugten Ausführungsform der Erfindung werden die erfindungsgemäßen Verbindungen in Kombination mit einem Corticosteroid, wie beispielhaft und vorzugsweise Prednison, Prednisolon, Methylprednisolon, Triamcinolon, Dexamethason, Beclomethason, Betamethason, Flunisolid, Budesonid oder Fluticason, verabreicht.

Unter antithrombotisch wirkenden Mittel werden vorzugsweise Verbindungen aus der Gruppe der Thrombozytenaggregationshemmer, der Antikoagulantien und der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Melagatran, Dabigatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban, Apixaban, Fidexaban, Razaxaban, Fondaparinux, Idraparinux, DU-176b, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embursatan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton oder Eplerenon, verabreicht.

Bei einer bevorzugten Ausführungsform der Erfindung werden die erfindungsgemäßen Verbindungen in Kombination mit einem Diuretikum, wie beispielhaft und vorzugsweise Furosemid, Bumetanid, Torsemid, Bendroflumethiazid, Chlorthiazid, Hydrochlorthiazid, Hydroflumethiazid, Methyclothiazid, Polythiazid, Trichlormethiazid, Chlorthalidon, Indapamid, Metolazon, Quinethazon, Acetazolamid, Dichlorphenamid, Methazolamid, Glycerin, Isosorbid, Mannitol, Amilorid oder Triamteren, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Torcetrapib (CP-529 414), JJT-705 oder CETP-vaccine (Avant), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW 501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Besonders bevorzugt sind Kombinationen der erfindungsgemäßen Verbindungen mit einem oder mehreren weiteren Wirkstoffen ausgewählt aus der Gruppe der PDE 5-Inhibitoren, sGC-Aktivatoren, sGC-Stimulatoren, Prostacyclin-Analoga, Endothelin-Antagonisten, der antifibrotisch wirkenden Mittel, der entzündungshemmend, immunmodulierend, immunsuppressiv und/oder zytotoxisch wirkenden Mittel und/oder der die Signaltransduktionskaskade inhibierenden Verbindungen.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophilisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. inhalativ, intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer, Dosieraerosole), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale und die parenterale Applikation, insbesondere die orale, die intravenöse und die intrapulmonale (inhalative) Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht. Bei intrapulmonaler Applikation beträgt die Menge im Allgemeinen etwa 0.1 bis 50 mg je Inhalation.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

### A. Beispiele

### Abkürzungen und Akronyme:

- abs.: absolut
- Ac: Acetyl
- AIBN: 2,2'-Azobis(2-methylpropionitril)
- aq.: wässrig, wässrige Lösung
- br.: breit (bei NMR-Signal)
- Bsp.: Beispiel
- Bu: Butyl
- c: Konzentration
- ca.: *circa,* ungefähr
- cat.: katalytisch
- CDI: *N,N'*-Carbonyldiimidazol
- CI: chemische Ionisation (bei MS)
- d: Dublett (bei NMR)
- d: Tag(e)
- DAST: *N,N*-Diethylaminoschwefeltrifluorid
- DC: Dünnschichtchromatographie
- DCI: direkte chemische Ionisation (bei MS)
- dd: Dublett von Dublett (bei NMR)
- Deoxo-Fluor®/™: Bis(2-methoxyethyl)aminoschwefeltrifluorid
- DIAD: Diisopropylazodicarboxylat
- DME: 1,2-Dimethoxyethan
- DMF: *N,N*-Dimethylformamid
- DMSO: Dimethylsulfoxid
- dq: Dublett von Quartett (bei NMR)
- dt: Dublett von Triplett (bei NMR)
- d. Th.: der Theorie (bei chemischer Ausbeute)
- EDC: *N'*-(3-Dimethylaminopropyl)-*N*-ethylcarbodiimid-Hydrochlorid
- ee: Enantiomerenüberschuss
- EI: Elektronenstoß-Ionisation (bei MS)
- eq.: Äquivalent(e)
- ESI: Elektrospray-Ionisation (bei MS)
- Et: Ethyl
- GC: Gaschromatographie
- GC/MS: Gaschromatographie-gekoppelte Massenspektrometrie
- h: Stunde(n)
- HATU: *O*-(7-Azabenzotriazol-1-yl)-*N,N,N,N'*-tetramethyluronium-hexafluorophosphat
- HOBt: 1-Hydroxy-1*H*-benzotriazol-Hydrat
- HPLC: Hochdruck-, Hochleistungsflüssigchromatographie
- iPr: Isopropyl
- konz.: konzentriert (bei Lösung)
- LC: Flüssigchromatographie
- LC/MS: Flüssigchromatographie-gekoppelte Massenspektrometrie
- Lit.: Literatur(stelle)
- m: Multiplett (bei NMR)
- Me: Methyl
- min: Minute(n)
- MPLC: Mitteldruckflüssigchromatographie (über Kieselgel; auch "flash-Chromatographie" genannt)
- MS: Massenspektrometrie
- NBS: *N*-Bromsuccinimid
- NMM: *N*-Methylmorpholin
- NMO: *N*-Methylmorpholin-*N*-oxid
- NMP: *N*-Methyl-2-pyrrolidinon
- NMR: Kernresonanzspektrometrie
- Pd/C: Palladium auf Aktivkohle
- PEG: Polyethylenglykol
- Ph: Phenyl
- Pr: Propyl
- quant.: quantitativ (bei chemischer Ausbeute)
- quart: Quartett (bei NMR)
- quint: Quintett (bei NMR)
- R_{f}: Retentionsindex (bei DC)
- RP: reverse phase (Umkehrphase, bei HPLC)
- RT: Raumtemperatur
- Rₜ: Retentionszeit (bei HPLC, LC/MS)
- s: Singulett (bei NMR)
- sept: Septett (bei NMR)
- SFC: superkritische Flüssigchromatographie
- t: Triplett (bei NMR)
- TBAF: Tetra-*n*-butylammoniumfluorid
- TBME: *tert*.-Butyl-methylether
- TBTU: *N*-[(1*H*-Benzotriazol-1-yloxy)(dimethylamino)methylen]-*N*-methylmethanaminium-Tetrafluoroborat
- tBu: *tert.*-Butyl
- TFA: Trifluoressigsäure
- THF: Tetrahydrofuran
- TMS: Tetramethylsilan
- UV: Ultraviolett-Spektrometrie
- v/v: Volumen zu Volumen-Verhältnis (einer Lösung)
- zus.: zusammen

### HPLC-, LC/MS- und GC/MS-Methoden:

### Methode 1 (LC/MS):

Instrument: Waters ACQUITY SQD UPLC System; Säule: Waters Acquity UPLC HSS T3 1.8 µm, 50 mm x 1 mm; Eluent A: 11 Wasser + 0.25 ml 99%-ige Ameisensäure, Eluent B: 11 Acetonitril + 0.25 ml 99%-ige Ameisensäure; Gradient: 0.0 min 90% A → 1.2 min 5% A → 2.0 min 5% A; Temperatur: 50°C; Fluss: 0.40 ml/min; UV-Detektion: 210-400 nm.

### Methode 2 (LC/MS):

Instrument: Micromass Quattro Premier mit Waters UPLC Acquity; Säule: Thermo Hypersil GOLD 1.9 µm, 50 mm x 1 mm; Eluent A: 11 Wasser + 0.5 ml 50%-ige Ameisensäure, Eluent B: 11 Acetonitril + 0.5 ml 50%-ige Ameisensäure; Gradient: 0.0 min 97% A → 0.5 min 97% A → 3.2 min 5% A → 4.0 min 5% A; Temperatur: 50°C; Fluss: 0.30 ml/min; UV-Detektion: 210 nm.

### Methode 3 (LC/MS):

Instrument: Waters SQD mit Waters UPLC; Säule: Zorbax SB-Aq (Agilent) 1.8 µm, 50 mm x 2.1 mm; Eluent A: Wasser + 0.025% Ameisensäure, Eluent B: Acetonitril + 0.025% Ameisensäure; Gradient: 0.0 min 98% A → 0.9 min 25% A → 1.0 min 5% A → 1.4 min 5% A → 1.41 min 98% A → 1.5 min 98% A; Temperatur: 40°C; Fluss: 0.60 ml/min; UV-Detektion: DAD, 210 nm.

### Methode 4 (GC/MS):

Instrument: Thermo DFS, Trace GC Ultra; Säule: Restek RTX-35, 15 m x 200 µm x 0.33 µm; konstanter Fluss mit Helium: 1.20 ml/min; Ofen: 60°C; Einlass: 220°C; Gradient: 60°C, 30°C/min → 300°C (3.33 min halten).

### Methode 5 (präparative HPLC):

Säule: Chromatorex C18, 10 µm, 125 mm x 30 mm; Eluent: Acetonitril / Wasser mit 0.1% Ameisensäure; Gradient: 20:80 → 95:5 innerhalb von 20 min.

### Methode 6 (präparative HPLC):

Säule: Chromatorex C18, 10 µm, 125 mm x 30 mm; Eluent: Acetonitril / Wasser mit 0.1% Ameisensäure; Gradient: 30:70 → 95:5 innerhalb von 20 min.

### Methode 7 (präparative HPLC):

Säule: Chromatorex C18, 10 µm, 125 mm x 30 mm; Eluent: Methanol / Wasser mit 0.1% Ameisensäure; Gradient: 20:80 → 95:5 innerhalb von 20 min.

### Methode 8 (präparative HPLC mit Massendetektion):

Instrument: Waters; Säule: Phenomenex Luna 5 µm C18(2) 100A, AXIA Tech., 50 mm x 21.2 mm; Eluent A: Wasser + 0.05% Ameisensäure, Eluent B: Acetonitril + 0.05% Ameisensäure; Gradient: 0.0 min 95% A → 0.15 min 95% A → 8.0 min 5% A → 9.0 min 5% A; Fluss: 40 ml/ min; UV-Detektion: DAD, 210-400 nm.

### Methode 9 (LC/MS):

Instrument: Agilent MS Quad 6150 mit HPLC Agilent 1290; Säule: Waters Acquity UPLC HSS T3 1.8 µm, 50 mm x 2.1 mm; Eluent A: 11 Wasser + 0.25 ml 99%-ige Ameisensäure, Eluent B: 11 Acetonitril + 0.25 ml 99%-ige Ameisensäure; Gradient: 0.0 min 90% A → 0.3 min 90% A → 1.7 min 5% A → 3.0 min 5% A; Fluss: 1.20 ml/min; Temperatur: 50°C; UV-Detektion: 205-305 nm.

### Methode 10 (LC/MS):

Instrument: Waters Synapt G2S mit UPLC Waters Acquity I-CLASS; Säule: Waters HSS T3 C18 1.8 µm, 50 mm x 2.1 mm; Eluent A: 11 Wasser + 0.01% Ameisensäure, Eluent B: 11 Acetonitril + 0.01% Ameisensäure; Gradient: 0.0 min 10% B → 0.3 min 10% B → 1.7 min 95% B → 2.5 min 95% B; Fluss: 1.20 ml/min; Temperatur: 50°C; UV-Detektion: 210 nm.

### Methode 11 (präparative HPLC):

Säule: Chromatorex C18, 10 µm, 125 mm x 30 mm; Eluent: Methanol / Wasser mit 0.1% Ameisensäure; Gradient: 30:70 → 95:5 innerhalb von 20 min.

### Methode 12 (präparative HPLC):

Säule: Chromatorex C18, 10 µm, 125 mm x 30 mm; Eluent A: Wasser + 0.05% Trifluoressigsäure, Eluent B: Acetonitril + 0.05% Trifluoressigsäure; Gradient: 0.0 min 75% A → 5.0 min 75% A → 8.5 min 53% A - 15 min 53% A.

### Methode 13 (präparative HPLC):

Säule: Chromatorex C18, 10 µm, 125 mm x 30 mm; Eluent A: Wasser + 0.05% Trifluoressigsäure, Eluent B: Acetonitril + 0.05% Trifluoressigsäure; Gradient: 0.0 min 75% A → 5.0 min 75% A → 7.0 min 60% A - 16 min 45% A - 18 min 45% A.

### Methode 14 (präparative HPLC):

Säule: Chromatorex C18, 10 µm, 125 mm x 30 mm; Eluent A: Wasser + 0.05% Trifluoressigsäure, Eluent B: Acetonitril + 0.05% Trifluoressigsäure; Gradient: 0.0 min 80% A → 5.0 min 80% A → 8.0 min 55% A - 15 min 55% A.

### Methode 15 (präparative HPLC):

Säule: Chromatorex C18, 10 µm, 125 mm x 30 mm; Eluent: Acetonitril / Wasser mit 0.1% Ameisensäure; Gradient: 40:60 → 95:5 innerhalb von 20 min.

### Methode 16 (präparative HPLC):

Säule: Chromatorex C18, 10 µm, 125 mm x 30 mm; Eluent: Acetonitril / Wasser mit 0.1% Ameisensäure; Gradient: 15:85 → 95:5 innerhalb von 20 min.

### Methode 17 (LC/MS):

Instrument: Waters Acquity SQD UPLC System; Säule: Waters Acquity UPLC HSS T3 1.8 µm, 50 mm x 1 mm; Eluent A: 11 Wasser + 0.25 ml 99%-ige Ameisensäure, Eluent B: 11 Acetonitril + 0.25 ml 99%-ige Ameisensäure; Gradient: 0.0 min 95% A → 6.0 min 5% A → 7.5 min 5% A; Ofen: 50°C; Fluss: 0.35 ml/min; UV-Detektion: 210-400 nm.

### Methode 18 (präparative HPLC):

Säule: Reprosil-Pur C18, 10 µm, 125 mm x 30 mm; Eluent: Acetonitril / Wasser mit 0.05% Trifluoressigsäure; Gradient: 50:50 → 100:0 innerhalb von 12 min.

### Methode 19 (präparative HPLC):

Säule: Reprosil-Pur C18, 10 µm, 125 mm x 30 mm; Eluent: Acetonitril / Wasser mit 0.05% Trifluoressigsäure; Gradient: 10:90 → 100:0 innerhalb von 12 min.

### Weitere Angaben:

Die nachfolgenden Beschreibungen der Kopplungsmuster von ¹H-NMR-Signalen orientieren sich an dem optischen Erscheinungsbild der betreffenden Signale und entsprechen nicht notwendigerweise einer strengen, physikalisch korrekten Interpretation. In der Regel bezieht sich die Angabe zur chemischen Verschiebung auf das Zentrum des betreffenden Signals; bei breiten Multipletts erfolgt in der Regel die Angabe eines Intervalls.

Schmelzpunkte und Schmelzbereiche, soweit angegeben, sind nicht korrigiert.

In den Fällen, in denen Reaktionsprodukte durch Ausrühren, Verrühren oder Umkristallisieren gewonnen wurden, war es oft möglich, weitere Produktmengen aus der jeweiligen Mutterlauge durch Chromatographie zu isolieren. Auf die Beschreibung dieser Chromatographie wird im Folgenden jedoch verzichtet, es denn, ein großer Teil der Gesamtausbeute konnte erst in diesem Schritt isoliert werden.

Für alle Reaktanden oder Reagenzien, deren Herstellung im Folgenden nicht explizit beschrieben ist, gilt, dass sie von allgemein zugänglichen Quellen kommerziell bezogen wurden. Für alle übrigen Reaktanden oder Reagenzien, deren Herstellung im Folgenden ebenfalls nicht beschrieben ist, und die nicht kommerziell erhältlich waren, oder von Quellen bezogen wurden, die nicht allgemein zugänglich sind, ist ein Verweis auf die veröffentlichte Literatur angegeben, in der ihre Herstellung beschrieben ist.

### Ausgangsverbindungen und Intermediate:

### Beispiel 1A

### 2-tert.-Butyl-4-ethyl-5-amino-3-methylthiophen-2,4-dicarboxylat

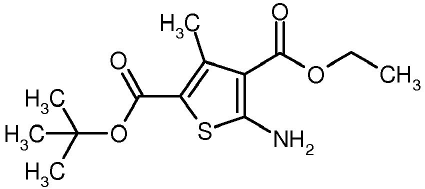

10.0 g (63.2 mmol) Acetessigsäure-tert.-butylester, 7.15 g (63.2 mmol) Cyanessigsäureethylester und 2.23 g (69.5 mmol) Schwefel wurden in 15 ml Ethanol vorgelegt und auf 45°C erwärmt. Zu diesem Gemisch wurden 7.5 ml (72.7 mmol) Diethylamin hinzugetropft. Anschließend wurde das Reaktionsgemisch 8 h bei 65°C gerührt. Danach wurden alle flüchtigen Bestandteile am Rotationsverdampfer entfernt. Der verbliebene Rückstand wurde mit ca. 500 ml Wasser versetzt und dreimal mit je ca. 200 ml Ethylacetat extrahiert. Die vereinigten organischen Extrakte wurden mit ca. 200 ml gesättigter Kochsalz-Lösung gewaschen und über wasserfreiem Magnesiumsulfat getrocknet. Nach Filtration wurde zur Trockene eingedampft. Das erhaltene Rohprodukt wurde mittels MPLC gereinigt (ca. 300 g Kieselgel, Cyclohexan/Ethylacetat 10:1). Nach Vereinigen der Produktfraktionen, Eindampfen und Trocknen des Rückstands im Hochvakuum wurden 9.72 g (52% d. Th.) der Titelverbindung erhalten.

¹H-NMR (400 MHz, CDCl₃, δ/ppm): 6.44 (br. s, 2H), 4.31 (quart, 2H), 2.66 (s, 3H), 1.54 (s, 9H), 1.37 (t, 3H).

LC/MS (Methode 1, ESIpos): Rₜ = 1.20 min, m/z = 286 [M+H]⁺.

### Beispiel 2A

### 2-tert. -Butyl-4-ethyl-3-methyl-5-{[(2-phenylethyl)carbamoyl]amino}thiophen-2,4-dicarboxylat

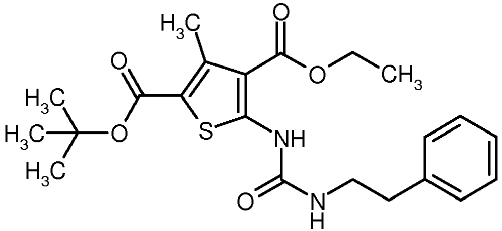

10.0 g (35.0 mmol) 2-*tert*.-Butyl-4-ethyl-5-amino-3-methylthiophen-2,4-dicarboxylat (Beispiel 1A) wurden in 500 ml Dichlormethan gelöst und mit 11.4 g (70.1 mmol) *N,N'*-Carbonyldiimidazol (CDI) und 19.6 ml (140 mmol) Triethylamin versetzt. Das Reaktionsgemisch wurde 2 Tage bei RT gerührt, bevor 8.8 ml (70.1 mmol) 2-Phenethylamin hinzugefügt wurden. Nach weiteren 2 h Rühren bei RT wurde das Gemisch am Rotationsverdampfer zur Trockene eingedampft. Der verbliebene Rückstand wurde mittels MPLC gereinigt (Kieselgel, Cyclohexan/Ethylacetat 20:1 → 10:1). Nach Eindampfen der Produktfraktionen und Trocknen des Rückstands im Hochvakuum wurden 14.4 g (95% d. Th.) der Titelverbindung erhalten.

¹H-NMR (400 MHz, DMSO-d₆, δ/ppm): 10.54 (s, 1H), 8.17 (t, 1H), 7.33-7.29 (m, 2H), 7.26-7.19 (m, 3H), 4.30 (quart, 2H), 3.36 (quart, 2H), 2.77 (t, 2H), 2.62 (s, 3H), 1.50 (s, 9H), 1.32 (t, 3H).

LC/MS (Methode 1, ESIpos): Rₜ = 1.43 min, m/z = 433 [M+H]⁺.

### Beispiel 3A

### 2-tert.-Butyl-4-ethyl-5-({[2-(2-fluorphenyl)ethyl]carbamoyl}amino)-3-methylthiophen-2,4-di-carboxylat

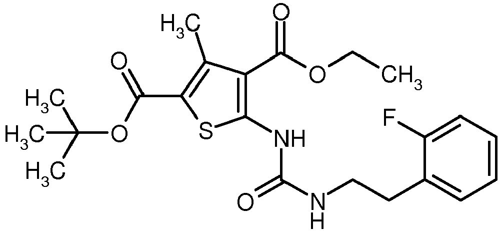

Analog zu dem unter Bsp. 2A beschriebenen Verfahren wurden aus 2.0 g (7.01 mmol) 2-*tert.-*Butyl-4-ethyl-5-amino-3-methylthiophen-2,4-dicarboxylat (Beispiel 1A), 2.27 g (14.0 mmol) CDI, 3.9 ml (28.0 mmol) Triethylamin und 3.5 ml (14.0 mmol) 2-(2-Fluorphenyl)ethylamin 1.82 g (55% d. Th., 95% Reinheit) der Titelverbindung erhalten. Abweichend erfolgte die MPLC-Reinigung hier mit Cyclohexan/Ethylacetat 10:1 als Laufmittel.

¹H-NMR (400 MHz, CDCl₃, δ/ppm): 10.90 (br. s, 1H), 7.24-7.19 (m, 2H), 7.10-7.01 (m, 2H), 5.18 (t, 1H), 4.32 (quart, 2H), 3.58 (quart, 2H), 2.94 (t, 2H), 2.71 (s, 3H), 1.55 (s, 9H), 1.39 (t, 3H).

LC/MS (Methode 1, ESIpos): Rₜ = 1.38 min, m/z = 451 [M+H]⁺.

### Beispiel 4A

### 2-tert.-Butyl-4-ethyl-5-({[2-(2-chlorphenyl)ethyl]carbamoyl}amino)-3-methylthiophen-2,4-di-carboxylat

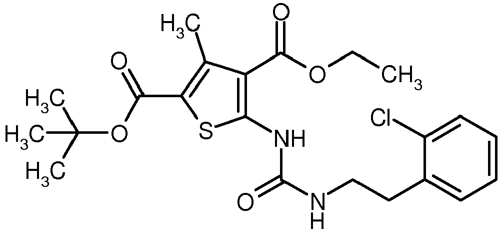

Analog zu dem unter Bsp. 2A beschriebenen Verfahren wurden aus 2.0 g (7.01 mmol) 2-*tert.-*Butyl-4-ethyl-5-amino-3-methylthiophen-2,4-dicarboxylat (Beispiel 1A), 2.27 g (14.0 mmol) CDI, 3.9 ml (28.0 mmol) Triethylamin und 2.18 g (14.0 mmol) 2-(2-Chlorphenyl)ethylamin 2.49 g (72% d. Th., 95% Reinheit) der Titelverbindung erhalten. Abweichend erfolgte die MPLC-Reinigung hier mit Cyclohexan/Ethylacetat 10:1 als Laufmittel.

¹H-NMR (400 MHz, CDCl₃, δ/ppm): 10.91 (br. s, 1H), 7.38-7.35 (m, 1H), 7.26-7.16 (m, 3H), 5.14 (t, 1H), 4.33 (quart, 2H), 3.60 (quart, 2H), 3.04 (t, 2H), 2.71 (s, 3H), 1.55 (s, 9H), 1.39 (t, 3H).

LC/MS (Methode 1, ESIpos): Rₜ = 1.42 min, m/z = 467/469 [M+H]⁺.

### Beispiel 5A

### 2-tert.-Butyl-4-ethyl-3-methyl-5-({[2-(pyridin-3-yl)ethyl]carbamoyl}amino)thiophen-2,4-di-carboxylat

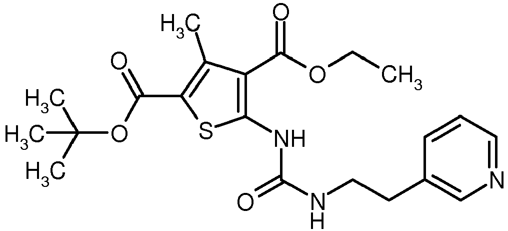

Analog zu dem unter Bsp. 2A beschriebenen Verfahren wurden aus 2.0 g (7.01 mmol) 2-*tert.-*Butyl-4-ethyl-5-amino-3-methylthiophen-2,4-dicarboxylat (Beispiel 1A), 2.27 g (14.0 mmol) CDI, 3.9 ml (28.0 mmol) Triethylamin und 1.71 g (14.0 mmol) 2-(Pyridin-3-yl)ethanamin 2.46 g (75% d. Th., 93% Reinheit) der Titelverbindung erhalten. Abweichend erfolgte die MPLC-Reinigung hier mit Cyclohexan/Ethylacetat 1:2 als Laufmittel.

¹H-NMR (400 MHz, CDCl₃, δ/ppm): 10.94 (br. s, 1H), 8.50 (m, 1H), 8.46 (m, 1H), 7.56 (m, 1H), 7.24 (m, 1H), 5.42 (br. s, 1H), 4.31 (quart, 2H), 3.59 (quart, 2H), 2.92 (t, 2H), 2.70 (s, 3H), 1.55 (s, 9H), 1.39 (t, 3H).

LC/MS (Methode 1, ESIpos): Rₜ = 0.99 min, m/z = 434 [M+H]⁺.

### Beispiel 6A

### 2-tert.-Butyl-4-ethyl-5-[(ethylcarbamoyl)amino]-3-methylthiophen-2,4-dicarboxylat

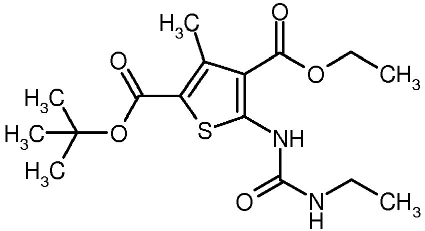

6.0 g (21.0 mmol) 2-*tert*.-Butyl-4-ethyl-5-amino-3-methylthiophen-2,4-dicarboxylat (Beispiel 1A) wurden in 300 ml Dichlormethan gelöst und mit 6.82 g (42.1 mmol) CDI und 11.7 ml (84.1 mmol) Triethylamin versetzt. Das Reaktionsgemisch wurde 2 Tage bei RT gerührt, bevor 42 ml (84.1 mmol) einer 2 M Lösung von Ethylamin in THF hinzugefügt wurden. Nach weiteren 2 h Rühren bei RT wurde das Gemisch am Rotationsverdampfer zur Trockene eingedampft. Der verbliebene Rückstand wurde mittels MPLC gereinigt (Biotage-Kartusche mit 340 g Kieselgel, Cyclohexan/ Ethylacetat 10:1 → 5:1). Nach Eindampfen der Produktfraktionen und Trocknen des Rückstands im Hochvakuum wurden 6.98 g (93% d. Th.) der Titelverbindung erhalten.

¹H-NMR (400 MHz, DMSO-d₆, δ/ppm): 10.52 (br. s, 1H), 8.06 (br. t, 1H), 4.31 (quart, 2H), 3.13 (dq, 2H), 2.77 (t, 2H), 2.62 (s, 3H), 1.50 (s, 9H), 1.32 (t, 3H), 1.07 (t, 3H).

LC/MS (Methode 1, ESIpos): Rₜ = 1.26 min, m/z = 357 [M+H]⁺.

### Beispiel 7A

### 2-tert.-Butyl-4-ethyl-5-{[(2,4-dimethoxybenzyl)carbamoyl]amino}-3-methylthiophen-2,4-di-carboxylat

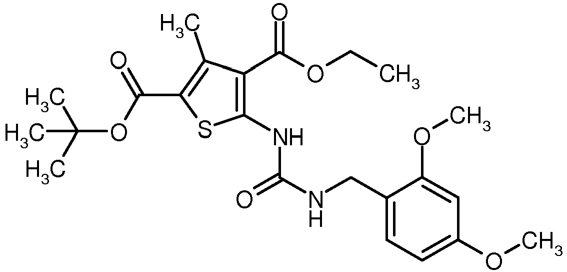

8.78 g (30.8 mmol) 2-*tert*.-Butyl-4-ethyl-5-amino-3-methylthiophen-2,4-dicarboxylat (Beispiel 1A) wurden in 300 ml Dichlormethan gelöst und mit 9.98 g (61.5 mmol) CDI und 17.2 ml (123 mmol) Triethylamin versetzt. Das Reaktionsgemisch wurde 2 Tage bei RT gerührt, bevor 9.3 ml (61.5 mmol) 2,4-Dimethoxybenzylamin hinzugefügt wurden. Nach weiteren 2 h Rühren bei RT wurde das Gemisch mit 200 ml Dichlormethan verdünnt und nacheinander mit je ca. 200 ml Wasser und gesättigter Natriumchlorid-Lösung gewaschen. Nach Trocknen über wasserfreiem Magnesiumsulfat wurde filtriert und eingedampft. Der verbliebene Rückstand wurde in Dichlormethan aufgenommen, wobei ein Teil ungelöst blieb, der durch Filtration abgetrennt wurde. Das Filtrat wurde auf Kieselgel aufgezogen und an Kieselgel mit Cyclohexan/Ethylacetat 2:1 → 1:1 als Laufmittel chromatographiert. Nach Vereinigen der Produktfraktionen, Eindampfen und Trocknen des Rückstands im Hochvakuum wurden 12.8 g (87% d. Th.) der Titelverbindung erhalten.

¹H-NMR (400 MHz, DMSO-d₆, δ/ppm): 10.56 (s, 1H), 8.32 (t, 1H), 7.13 (d, 1H), 6.57 (d, 1H), 6.49 (dd, 1H), 4.30 (quart, 2H), 4.19 (d, 2H), 3.80 (s, 3H), 3.75 (s, 3H), 2.62 (s, 3H), 1.50 (s, 9H), 1.32 (t, 3H).

LC/MS (Methode 1, ESIpos): Rₜ = 1.40 min, m/z = 479 [M+H]⁺.

### Beispiel 8A

### tert.-Butyl-5-methyl-2,4-dioxo-3-(2-phenylethyl)-1,2,3,4-tetrahydrothieno[2,3-d]pyrimidin-6-carboxylat

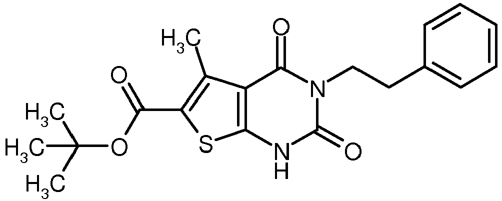

7.34 g (17.0 mmol) der Verbindung aus Bsp. 2A wurden in 145 ml Ethanol gelöst und mit 9.5 ml (25.4 mmol) einer 20%-igen Lösung von Natriumethanolat in Ethanol versetzt. Das Reaktionsgemisch wurde 2 h bei RT gerührt. Danach wurde es in ca. 400 ml Wasser gegossen und mit 5 M Essigsäure auf einen pH-Wert von ca. 5 gebracht. Dabei fiel das Produkt aus. Es wurde abgesaugt, mit Wasser neutral gewaschen und im Hochvakuum getrocknet. Es wurden 5.89 g (91% d. Th.) der Titelverbindung erhalten.

¹H-NMR (400 MHz, DMSO-d₆, δ/ppm): 12.44 (s, 1H), 7.33-7.29 (m, 2H), 7.25-7.20 (m, 3H), 4.01 (m, 2H), 2.83 (m, 2H), 2.72 (s, 3H), 1.52 (s, 9H).

LC/MS (Methode 1, ESIpos): Rₜ = 1.28 min, m/z = 387 [M+H]⁺.

### Beispiel 9A

### tert.-Butyl-3-[2-(2-fluorphenyl)ethyl]-5-methyl-2,4-dioxo-1,2,3,4-tetrahydrothieno[2,3-d]pyrimidin-6-carboxylat

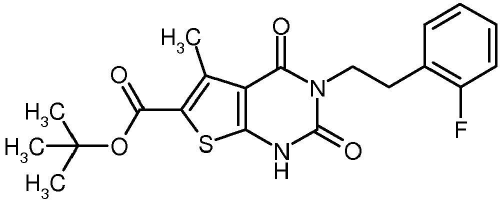

Analog zu dem unter Bsp. 8A beschriebenen Verfahren wurden aus 1.76 g (3.90 mmol) der Verbindung aus Bsp. 3A 1.55 g (98% d. Th.) der Titelverbindung erhalten. Abweichend zu dem zuvor beschriebenen Verfahren wurde hier das ausgefallene Produkt nach dem Absaugen in Ethylacetat gelöst und nochmals mit Wasser gewaschen. Anschließend wurde die organische Phase über wasserfreiem Magnesiumsulfat getrocknet, filtriert und eingeengt und der Rückstand schließlich im Hochvakuum getrocknet.

¹H-NMR (400 MHz, CDCl₃, δ/ppm): 7.28-7.24 (m, 1H, teilweise überdeckt vom CHCl₃-Signal), 7.22-7.17 (m, 1H), 7.08-6.99 (m, 2H), 4.25 (t, 2H), 3.03 (t, 2H), 2.82 (s, 3H), 1.58 (s, 9H).

LC/MS (Methode 1, ESIpos): Rₜ = 1.25 min, m/z = 405 [M+H]⁺.

### Beispiel 10A

### tert.-Butyl-3-[2-(2-chlorphenyl)ethyl]-5-methyl-2,4-dioxo-1,2,3,4-tetrahydrothieno[2,3-d]pyrimidin-6-carboxylat

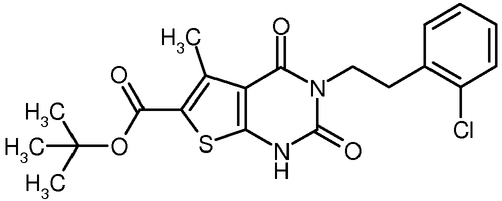

Analog zu dem unter Bsp. 8A beschriebenen Verfahren wurden aus 2.42 g (5.17 mmol) der Verbindung aus Bsp. 4A 1.71 g (76% d. Th., 97% Reinheit) der Titelverbindung erhalten. Abweichend zu dem zuvor beschriebenen Verfahren wurde hier das ausgefallene Produkt nach dem Absaugen in Ethylacetat gelöst und nochmals mit Wasser gewaschen. Anschließend wurde die organische Phase über wasserfreiem Magnesiumsulfat getrocknet, filtriert und eingeengt und der Rückstand schließlich im Hochvakuum getrocknet.

¹H-NMR (400 MHz, CDCl₃, δ/ppm): 7.35 (dd, 1H), 7.30 (dd, 1H), 7.20-7.14 (m, 2H), 4.26 (t, 2H), 3.13 (t, 2H), 2.83 (s, 3H), 1.58 (s, 9H).

LC/MS (Methode 1, ESIpos): Rₜ = 1.30 min, m/z = 421/422 [M+H]⁺.

### Beispiel 11A

### tert.-Butyl-5-methyl-2,4-dioxo-3-[2-(pyridin-3-yl)ethyl]-1,2,3,4-tetrahydrothieno[2,3-d]pyrimidin-6-carboxylat

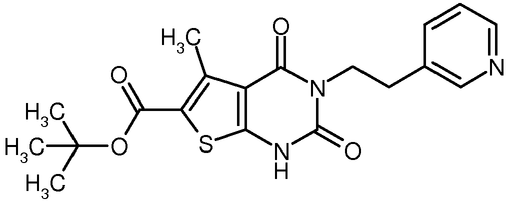

Analog zu dem unter Bsp. 8A beschriebenen Verfahren wurden aus 2.41 g (5.56 mmol) der Verbindung aus Bsp. 5A 1.89 g (87% d. Th.) der Titelverbindung erhalten. Abweichend zu dem zuvor beschriebenen Verfahren wurde hier das ausgefallene Produkt nach dem Absaugen in Ethylacetat gelöst und nochmals mit Wasser und gesättigter Natriumhydrogencarbonat-Lösung gewaschen. Anschließend wurde die organische Phase über wasserfreiem Magnesiumsulfat getrocknet, filtriert und eingeengt und der Rückstand schließlich im Hochvakuum getrocknet.

¹H-NMR (400 MHz, DMSO-d₆, δ/ppm): 8.42-8.40 (m, 2H), 7.64 (dt, 1H), 7.31 (dd, 1H), 4.01 (t, 2H), 2.81 (t, 2H), 2.67 (s, 3H), 1.49 (s, 9H).

LC/MS (Methode 1, ESIpos): Rₜ = 0.82 min, m/z = 388 [M+H]⁺.

### Beispiel 12A

### tert.-Butyl-3-ethyl-5-methyl-2,4-dioxo-1,2,3,4-tetrahydrothieno[2,3-d]pyrimidin-6-carboxylat

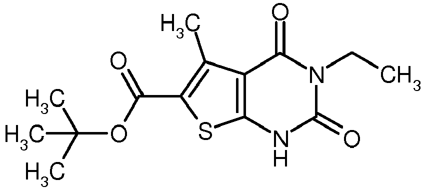

6.98 g (19.6 mmol) der Verbindung aus Bsp. 6A wurden in 130 ml Ethanol gelöst und mit 11 ml (29.4 mmol) einer 20%-igen Lösung von Natriumethanolat in Ethanol versetzt. Das Reaktionsgemisch wurde 2 h bei RT gerührt. Danach wurde es in ca. 400 ml Wasser gegossen und mit 5 M Essigsäure auf einen pH-Wert von ca. 5 gebracht. Dabei fiel das Produkt aus. Es wurde abgesaugt, mit Wasser neutral gewaschen und im Hochvakuum getrocknet. Es wurden 5.89 g (97% d. Th.) der Titelverbindung erhalten.

¹H-NMR (400 MHz, DMSO-d₆, δ/ppm): 12.39 (s, 1H), 3.85 (quart, 2H), 2.71 (s, 3H), 1.51 (s, 9H), 1.11 (t, 3H).

LC/MS (Methode 1, ESIpos): Rₜ = 1.06 min, m/z = 311 [M+H]⁺.

### Beispiel 13A

### tert.-Butyl-3-(2,4-dimethoxybenzyl)-5-methyl-2,4-dioxo-1,2,3,4-tetrahydrothieno[2,3-d]pyrimidin-6-carboxylat

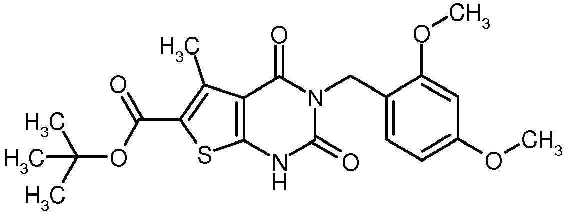

12.8 g (26.8 mmol) der Verbindung aus Bsp. 7A wurden in 250 ml Ethanol gelöst und mit 15 ml (40.2 mmol) einer 20%-igen Lösung von Natriumethanolat in Ethanol versetzt. Das Reaktionsgemisch wurde ca. 16 h bei RT gerührt. Danach wurde es in ca. 1.5 Liter Wasser gegossen und mit Essigsäure auf einen pH-Wert von ca. 5 gebracht. Dabei fiel das Produkt aus. Es wurde abgesaugt, mit Wasser neutral gewaschen und im Hochvakuum getrocknet. Es wurden 11.3 g (97% d. Th.) der Titelverbindung erhalten.

¹H-NMR (400 MHz, DMSO-d₆, δ/ppm): 12.50 (s, 1H), 6.72 (d, 1H), 6.56 (d, 1H), 6.39 (dd, 1H), 4.89 (s, 2H), 3.82 (s, 3H), 3.72 (s, 3H), 2.69 (s, 3H), 1.52 (s, 9H).

LC/MS (Methode 1, ESIpos): Rₜ = 1.20 min, m/z = 433 [M+H]⁺.

### Beispiel 14A

### 1-(2-Phenylethyl)pyrimidin-2,4,6(1H,3H,5H)-trion

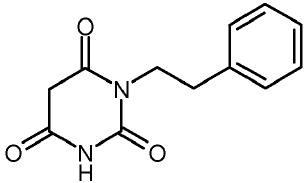

20.0 g (122 mmol) 2-Phenethylharnstoff [kommerziell erhältlich; Lit. z.B.: L. De Luca, A. Porcheddu, G. Giacomelli, I. Murgia, Synlett 2010 (16), 2439-2442] und 18.5 ml (122 mmol) Malonsäurediethylester wurden in 70 ml Ethanol gelöst und mit 45.5 ml (122 mmol) einer 20%-igen Lösung von Natriumethanolat in Ethanol versetzt. Das Gemisch wurde 16 h unter Rückfluss erhitzt. Danach wurde der Großteil des Lösungsmittels am Rotationsverdampfer entfernt und der verbliebene Rückstand mit ca. 100 ml Wasser versetzt. Vom Ungelösten wurde abfiltriert, und das Filtrat wurde mit konzentrierter Salzsäure auf pH 3-4 angesäuert. Dabei fiel das Produkt aus, das abgesaugt und zunächst mit Wasser und dann mit Hexan/Diethylether 1:1 gewaschen wurde. Nach Trocknen im Hochvakuum wurden 20.9 g (72% d. Th.) der Titelverbindung erhalten.

¹H-NMR (400 MHz, DMSO-d₆, δ/ppm): 11.36 (s, 1H), 7.33-7.29 (m, 2H), 7.25-7.20 (m, 3H), 3.86 (dd, 2H), 3.62 (s, 2H), 2.77 (dd, 2H).

LC/MS (Methode 1, ESIpos): Rₜ = 0.76 min, m/z = 233 [M+H]⁺.

### Beispiel 15A

### 1-Ethylpyrimidin-2,4,6(1H,3H,5H)-trion

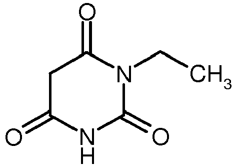

25.0 g (284 mmol) Ethylharnstoff und 43 ml (284 mmol) Malonsäurediethylester wurden in 150 ml Ethanol gelöst und mit 106 ml (284 mmol) einer 20%-igen Lösung von Natriumethanolat in Ethanol versetzt. Das Gemisch wurde 1 h unter Rückfluss erhitzt, wobei ein Niederschlag ausfiel. Nach dem Abkühlen auf RT wurde der Niederschlag abgetrennt und das Filtrat am Rotationsverdampfer vom Großteil des Lösungsmittels befreit. Der verbliebene Rückstand wurde mit ca. 500 ml Wasser versetzt und mit 5 M Salzsäure auf pH 3-4 angesäuert. Dann wurde die wässrige Lösung dreimal mit je ca. 100 ml Ethylacetat extrahiert. Nach Trocknen über wasserfreiem Magnesiumsulfat, Filtration und Eindampfen der vereinigten organischen Extrakte wurde eine erste Fraktion der Titelverbindung erhalten (14.1 g, 31% d. Th.). Die zuvor verbliebene wässrige Phase wurde bis auf ein Volumen von ca. 250 ml aufkonzentriert, mit 5 M Salzsäure auf pH 1 eingestellt und bis zur Sättigung mit festem Natriumchlorid versetzt. Es wurde erneut mit Ethylacetat extrahiert und die organische Phase über Magnesiumsulfat getrocknet, filtriert und eingedampft. Das so gewonnene Produkt wurde bei RT mit 200 ml Diethylether verrührt. Anschließend wurde filtriert und der Rückstand im Hochvakuum getrocknet. Auf diese Weise wurde eine zweite Fraktion der Titelverbindung gewonnen (6.0 g, 13% d. Th.). Insgesamt wurden so 20.1 g (45% d. Th.) der Titelverbindung erhalten.

¹H-NMR (400 MHz, DMSO-d₆, δ/ppm): 11.30 (s, 1H), 3.70 (quart, 2H), 3.59 (s, 2H), 1.06 (t, 3H).

GC/MS (Methode 4, Elpos): Rₜ = 4.28 min, m/z = 156 [M]⁺.

### Beispiel 16A

### 6-Chlor-3-(2-phenylethyl)pyrimidin-2,4(1H,3H)-dion

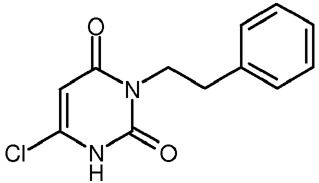

Bei einer Temperatur von 0°C wurden 36.5 ml (392 mmol) Phosphoroxychlorid vorsichtig zu 8.2 ml 50%-igem wässrigen Ethanol gegeben. Dann wurde, ebenfalls bei 0°C, portionsweise mit 10.2 g (43.9 mmol) der Verbindung aus Bsp. 14A versetzt. Nach beendeter Zugabe wurde das Reaktionsgemisch zunächst 30 min auf 50°C und dann 90 min auf 100°C erwärmt. Nachdem das Gemisch auf RT abgekühlt war, wurde es in ca. 100 ml Eiswasser gegossen und 1 h gerührt. Anschließend wurde der ausgefallene Feststoff abgesaugt und zunächst mit Wasser und dann mit Hexan gewaschen. Danach wurde der Feststoff mit wenig Dichlormethan bei RT verrührt, erneut abgesaugt und schließlich im Hochvakuum getrocknet. Es wurden 3.16 g (26% d. Th., 93% Reinheit) der Titelverbindung erhalten.

¹H-NMR (400 MHz, DMSO-d₆, δ/ppm): 12.38 (s, 1H), 7.32-7.28 (m, 2H), 7.23-7.19 (m, 3H), 5.91 (s, ca. 1H), 3.93 (dd, 2H), 2.80 (dd, 2H).

LC/MS (Methode 1, ESIpos): Rₜ = 0.79 min, m/z = 251/253 [M+H]⁺.

### Beispiel 17A

### 6-Chlor-3-ethylpyrimidin-2,4(1H,3H)-dion

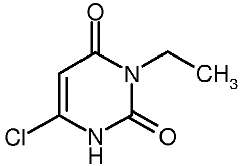

Bei einer Temperatur von 0°C wurden 28.8 ml (309 mmol) Phosphoroxychlorid vorsichtig zu 6.6 ml 50%-igem wässrigen Ethanol gegeben. Dann wurde, ebenfalls bei 0°C, portionsweise mit 5.4 g (34.6 mmol) der Verbindung aus Bsp. 15A versetzt. Nach beendeter Zugabe wurde das Reaktionsgemisch zunächst 30 min auf 50°C und dann 2 h auf 100°C erwärmt. Nachdem das Gemisch auf RT abgekühlt war, wurde es in ca. 100 ml Eiswasser gegossen. Der dabei ausgefallene Feststoff wurde abgesaugt und mit Wasser gewaschen. Nach Trocknen im Hochvakuum wurden 2.78 g (46% d. Th.) der Titelverbindung erhalten.

¹H-NMR (400 MHz, DMSO-d₆, δ/ppm): 12.34 (s, 1H), 5.89 (s, ca. 1H), 3.76 (quart, 2H), 1.07 (t, 3H).

LC/MS (Methode 1, ESIpos): Rₜ = 0.42 min, m/z = 175/177 [M+H]⁺.

### Beispiel 18A

### 1-[2,6-Dioxo-1-(2-phenylethyl)-4-(pyridinium-1-yl)-1,6-dihydropyrimidin-5(2H)-yliden]-2,2,2-tri-fluorethanolat

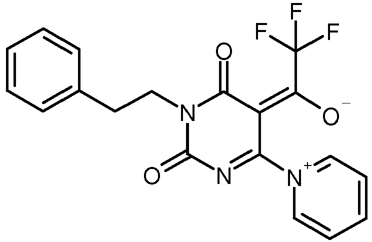

Eine Suspension von 1.0 g (3.99 mmol) der Verbindung aus Bsp. 16A in 10 ml Acetonitril wurde bei RT mit 3.2 ml (39.9 mmol) Pyridin versetzt. Dann wurden langsam 2.3 ml (16.0 mmol) Trifluoressigsäureanhydrid hinzugetropft. Nach beendeter Zugabe wurde das Gemisch noch 1 h bei RT gerührt. Anschließend wurde mit ca. 100 ml Wasser versetzt und mit ca. 100 ml Ethylacetat extrahiert. Der organische Extrakt wurde mit gesättigter wässriger Kochsalz-Lösung gewaschen. Dann wurde der im Extrakt fein suspendierte Feststoff abgesaugt und mit wenig Ethylacetat nachgewaschen. Der Feststoff wurde im Hochvakuum getrocknet und ergab so eine erste Fraktion der Titelverbindung (659 mg, 42% d. Th.). Das erhaltene Filtrat wurde über wasserfreiem Magnesiumsulfat getrocknet, filtriert und zur Trockene eingedampft. Der verbliebene Feststoff wurde in einem Gemisch aus 25 ml Diisopropylether und 11 ml Ethylacetat 30 min lang bei 40°C verrührt. Nach dem Abkühlen auf RT wurde der Feststoff abgesaugt und mit wenig Diethylether nachgewaschen. Nach Trocknen im Hochvakuum wurde so eine zweite Fraktion der Titelverbindung erhalten (499 mg, 32% d. Th.). Insgesamt wurden auf diese Weise 1.16 g (74% d. Th.) der Titelverbindung gewonnen.

¹H-NMR (400 MHz, DMSO-d₆, δ/ppm): 9.28 (d, 2H), 8.81 (t, 1H), 8.28 (t, 2H), 7.36-7.28 (m, 4H), 7.25-7.21 (m, 1H), 4.03 (dd, 2H), 2.83 (dd, 2H).

LC/MS (Methode 1, ESIpos): Rₜ = 0.85 min, m/z = 390 [M+H]⁺.

### Beispiel 19A

### 1-[1-Ethyl-2,6-dioxo-4-(pyridinium-1-yl)-1,6-dihydropyrimidin-5(2H)-yliden]-2,2,2-trifluor-ethanolat

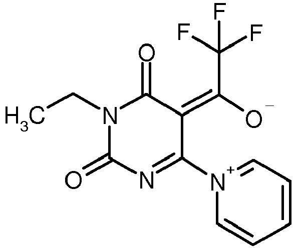

Eine Suspension von 1.0 g (5.73 mmol) der Verbindung aus Bsp. 17A in 15 ml Acetonitril wurde bei RT mit 4.6 ml (57.3 mmol) Pyridin versetzt. Dann wurden langsam 3.2 ml (22.9 mmol) Trifluoressigsäureanhydrid hinzugetropft. Nach beendeter Zugabe wurde das Gemisch noch 1 h bei RT gerührt. Anschließend wurde mit ca. 100 ml Wasser versetzt und zweimal mit je ca. 100 ml Ethylacetat extrahiert. Der organische Extrakt wurde mit gesättigter wässriger Kochsalz-Lösung gewaschen und anschließend zur Trockene eingedampft. Der verbliebene Feststoff wurde in einem Gemisch aus 25 ml Diisopropylether und 5 ml Ethylacetat 30 min lang bei 40°C verrührt. Nach dem Abkühlen auf RT wurde der Feststoff abgesaugt und mit wenig Pentan nachgewaschen. Nach Trocknen im Hochvakuum wurden 1.54 g (86% d. Th.) der Titelverbindung erhalten.

¹H-NMR (400 MHz, DMSO-d₆, δ/ppm): 9.28 (d, 2H), 8.80 (t, 1H), 8.26 (t, 2H), 3.87 (quart, 2H), 1.13 (t, 3H).

LC/MS (Methode 1, ESIpos): Rₜ = 0.56 min, m/z = 314 [M+H]⁺.

### Beispiel 20A

### Ethyl-2,4-dioxo-3-(2-phenylethyl)-5-(trifluormethyl)-1,2,3,4-tetrahydrothieno[2,3-d]pyrimidin-6-carboxylat

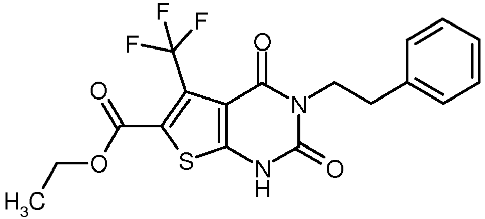

In einem Mikrowellenofen (Biotage Initiator mit dynamischer Steuerung der Einstrahlleistung) wurde ein Gemisch aus 950 mg (2.44 mmol) der Verbindung aus Bsp. 18A, 569 mg (5.37 mmol) Natriumcarbonat und 535 µl (4.88 mmol) Mercaptoessigsäureethylester in 26 ml Ethanol 1 h lang auf 120°C erhitzt. Anschließend wurde am Rotationsverdampfer zur Trockene eingedampft. Der verbliebene Rückstand wurde in ca. 700 ml Ethylacetat aufgenommen und nacheinander mit je ca. 300 ml halbgesättigter wässriger Ammoniumchlorid-Lösung, Wasser und gesättigter Kochsalz-Lösung gewaschen. Nach Trocknen über wasserfreiem Magnesiumsulfat wurde filtriert und eingedampft. Das Rohprodukt wurde mittels präparativer HPLC gereinigt (Methode 5). Nach Vereinigen und Eindampfen der Produktfraktionen und Trocknen des Rückstands im Hochvakuum wurden 728 mg (72% d. Th.) der Titelverbindung erhalten.

¹H-NMR (400 MHz, DMSO-d₆, δ/ppm): 12.61 (s, 1H), 7.33-7.29 (m, 2H), 7.26-7.20 (m, 3H), 4.33 (quart, 2H), 4.02 (m, 2H), 2.83 (m, 2H), 1.29 (t, 3H).

LC/MS (Methode 1, ESIpos): Rₜ = 1.13 min, m/z = 413 [M+H]⁺.

### Beispiel 21A

### Ethyl-3-ethyl-2,4-dioxo-5-(trifluormethyl)-1,2,3,4-tetrahydrothieno[2,3-d]pyrimidin-6-carboxylat

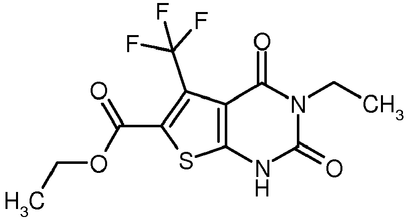

In einem Mikrowellenofen (Biotage Initiator mit dynamischer Steuerung der Einstrahlleistung) wurde ein Gemisch aus 4.75 g (15.2 mmol) der Verbindung aus Bsp. 19A, 3.54 g (33.4 mmol) Natriumcarbonat und 3.3 ml (30.3 mmol) Mercaptoessigsäureethylester in 39 ml Ethanol 1 h lang auf 120°C erhitzt. Anschließend wurde am Rotationsverdampfer zur Trockene eingedampft. Der verbliebene Rückstand wurde mit ca. 200 ml Wasser versetzt und mit Essigsäure schwach sauer gestellt (ca. pH 4). Es wurde dreimal mit je ca. 100 ml Dichlormethan extrahiert. Nach Waschen der vereinigten organischen Phasen mit gesättigter Natriumchlorid-Lösung und Trocknen über wasserfreiem Magnesiumsulfat wurde filtriert und eingedampft. Das Rohprodukt wurde mittels MPLC gereinigt (Puriflash-Kartusche mit 25 g Kieselgel, Cyclohexan/Ethylacetat 3:1 → 1:1). Nach Vereinigen und Eindampfen der Produktfraktionen und Trocknen des Rückstands im Hochvakuum wurden 2.78 g (54% d. Th.) der Titelverbindung erhalten.

¹H-NMR (400 MHz, CDCl₃, δ/ppm): 11.38 (s, 1H), 4.40 (quart, 2H), 4.10 (quart, 2H), 1.39 (t, 3H), 1.29 (t, 3H).

LC/MS (Methode 2, ESIpos): Rₜ = 2.06 min, m/z = 337 [M+H]⁺.

### Beispiel 22A

### 1-[2,6-Dioxo-1-(2-phenylethyl)-4-(pyridinium-1-yl)-1,6-dihydropyrimidin-5(2H)-yliden]-2,2-difluorethanolat

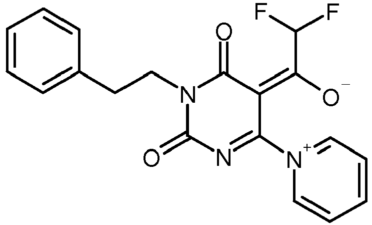

Eine Suspension von 5.0 g (19.9 mmol) der Verbindung aus Bsp. 16A in 50 ml Acetonitril wurde bei RT mit 16.1 ml (199 mmol) Pyridin versetzt. Dann wurden langsam 9.9 ml (79.8 mmol) Difluoressigsäureanhydrid hinzugetropft. Nach beendeter Zugabe wurde noch 1 h bei RT nachgerührt. Anschließend wurde mit ca. 500 ml Wasser versetzt und zweimal mit je ca. 500 ml Ethylacetat extrahiert. Der organische Extrakt wurde mit gesättigter wässriger Kochsalz-Lösung gewaschen. Der im Extrakt fein suspendierte Feststoff wurde danach abgesaugt und mit wenig Ethylacetat nachgewaschen. Der Feststoff wurde im Hochvakuum getrocknet und ergab so eine erste Fraktion der Titelverbindung (2.8 g, 37% d. Th.). Das Filtrat wurde über wasserfreiem Magnesiumsulfat getrocknet, filtriert und zur Trockene eingeengt. Der verbliebene Rückstand wurde zunächst in einem Gemisch aus 20 ml Diisopropylether und 8 ml Ethylacetat bei RT verrührt. Da der nach Absaugen erhaltene Feststoff immer noch etwas verunreinigt war, wurde ein zweites Mal mit einem Gemisch aus 30 ml Diethylether und 10 ml Ethylacetat verrührt. Nach neuerlichem Absaugen und Trocknen im Hochvakuum wurde auf diese Weise eine zweite Fraktion der Titelverbindung gewonnen (2.3 g, 31% d. Th.). Insgesamt wurden somit 5.1 g (68% d. Th.) der Titelverbindung erhalten.

¹H-NMR (400 MHz, DMSO-d₆, δ/ppm): 9.22 (d, 2H), 8.81 (t, 1H), 8.27 (t, 2H), 7.36-7.28 (m, 4H), 7.26-7.22 (m, 1H), 6.97 (t, 1H), 4.04 (m, 2H), 2.84 (m, 2H).

LC/MS (Methode 1, ESIpos): Rₜ = 0.82 min, m/z = 372 [M+H]⁺.

### Beispiel 23A

### Ethyl-2,4-dioxo-3-(2-phenylethyl)-5-(difluormethyl)-1,2,3,4-tetrahydrothieno[2,3-d]pyrimidin-6-carboxylat

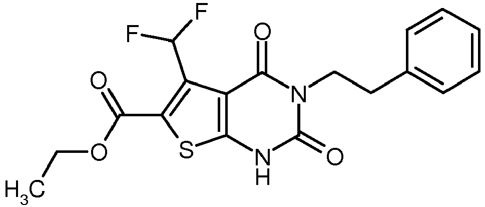

In einem Mikrowellenofen (Biotage Initiator mit dynamischer Steuerung der Einstrahlleistung) wurde ein Gemisch aus 4.0 g (10.8 mmol) der Verbindung aus Bsp. 22A, 2.51 g (23.7 mmol) Natriumcarbonat und 2.36 ml (21.5 mmol) Mercaptoessigsäureethylester in 22.4 ml Ethanol 2 h lang auf 120°C erhitzt. Anschließend wurde das Reaktionsgemisch am Rotationsverdampfer zur Trockene eingeengt. Der verbliebene Rückstand wurde in ca. 400 ml Wasser aufgenommen, durch Zugabe von Essigsäure schwach sauer gestellt und dreimal mit je ca. 400 ml Dichlormethan extrahiert. Der in den vereinigten organischen Extrakten fein suspendierte Feststoff wurde abgesaugt und mit einem Gemisch aus 25 ml Pentan und 25 ml Dichlormethan für 30 min bei RT verrührt. Nach erneutem Absaugen und Trocknen im Hochvakuum wurde so eine erste Fraktion der Titelverbindung erhalten (1.76 g, 41% d. Th.). Das Filtrat aus der Filtration der vereinigten organischen Extrakte wurde mit gesättigter wässriger Natriumchlorid-Lösung gewaschen, über wasserfreiem Magnesiumsulfat getrocknet, filtriert und eingeengt. Der dabei verbliebene Feststoff wurde mit einem Gemisch aus 20 ml Pentan und 20 ml Dichlormethan für 30 min bei RT verrührt. Nach Absaugen und Trocknen im Hochvakuum wurde auf diese Weise eine zweite Fraktion der Titelverbindung gewonnen (1.14 g, 26% d. Th.). Insgesamt wurden somit 2.9 g (68% d. Th.) der Titelverbindung erhalten.

¹H-NMR (400 MHz, DMSO-d₆, δ/ppm): 12.67 (s, 1H), 7.72 (t, 1H), 7.34-7.29 (m, 2H), 7.26-7.20 (m, 3H), 4.33 (quart, 2H), 4.02 (m, 2H), 2.84 (m, 2H), 1.31 (t, 3H).

LC/MS (Methode 1, ESIpos): Rₜ = 1.06 min, m/z = 395 [M+H]⁺.

### Beispiel 24A

### 6-Chlor-2,4-dioxo-3-(2-phenylethyl)-1,2,3,4-tetrahydropyrimidin-5-carbaldehyd

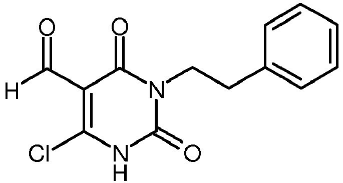

Bei einer Temperatur von 0°C wurden 31.7 ml (340 mmol) Phosphoroxychlorid tropfenweise zu 7.9 ml DMF hinzugefügt. Nach beendeter Zugabe wurde das Kältebad entfernt und das Gemisch bei RT portionsweise mit 3.95 g (17.0 mmol) der Verbindung aus Bsp. 14A versetzt. Anschließend wurde 2 h unter Rückfluss erhitzt. Nach Abkühlen wurde überschüssiges Phosphoroxychlorid am Rotationsverdampfer entfernt. Der verbliebene Rückstand wurde unter starkem Rühren vorsichtig in Eiswasser gegossen. Der dabei ausgefallene Niederschlag wurde abgesaugt und anschließend in Ethylacetat gelöst. Es wurde nacheinander mit Wasser und gesättigter Natriumchlorid-Lösung ausgeschüttelt. Die vereinigten wässrigen Waschphasen wurde mit Dichlormethan re-extrahiert, und die Dichlormethan-Phase wurde mit der Ethylacetat-Phase vereinigt. Nach Eindampfen der vereinigten organischen Phasen wurde das Rohprodukt in ca. 70 ml Ethylacetat suspendiert und für 30 min bei RT verrührt. Nach Absaugen des Feststoffs und Trocknen im Hochvakuum wurde so eine erste Fraktion der Titelverbindung erhalten (1.08 g, 91% Reinheit, 20% d. Th.). Das Filtrat wurde aufkonzentriert und anschließend durch Saugfiltration über Kieselgel mit Ethylacetat als Laufmittel grob gereinigt. Nach neuerlichem Eindampfen wurde der verbliebene Feststoff in einem Gemisch aus 10 ml Ethylacetat und 3 ml Pentan für 3 h bei RT verrührt. Nach Absaugen und Trocknen im Hochvakuum wurde auf diese Weise eine zweite Fraktion der Titelverbindung gewonnen (0.91 g, 63% Reinheit, 12% d. Th.). Beide Fraktionen konnten ohne weitere Aufreinigung in Folgereaktionen eingesetzt werden. Insgesamt wurden somit 1.99 g (78% Reinheit, 33% d. Th.) der Titelverbindung erhalten.

¹H-NMR (500 MHz, CDCl₃, δ/ppm): 10.14 (s, 1H), 9.91 (br. s, 1H), 7.33-7.29 (m, 2H), 7.28-7.22 (m, 3H), 4.19 (m, 2H), 2.96 (m, 2H).

LC/MS (Methode 1, ESIpos): Rₜ = 0.72 min, m/z = 279/281 [M+H]⁺.

### Beispiel 25A

### Ethyl-2,4-dioxo-3-(2-phenylethyl)-1,2,3,4-tetrahydrothieno[2,3-d]pyrimidin-6-carboxylat

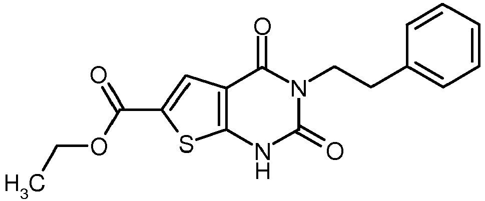

In einem Mikrowellenofen (Biotage Initiator mit dynamischer Steuerung der Einstrahlleistung) wurde ein Gemisch aus 1.08 g (3.88 mmol) der Verbindung aus Bsp. 24A, 411 mg (3.88 mmol) Natriumcarbonat und 425 µl (3.88 mmol) Mercaptoessigsäureethylester in 15 ml Ethanol 90 min lang auf 120°C erhitzt. Anschließend wurde das Reaktionsgemisch am Rotationsverdampfer zur Trockene eingeengt. Der verbliebene Rückstand wurde in Ethylacetat aufgenommen und zweimal mit Wasser und einmal mit gesättigter wässriger Natriumchlorid-Lösung gewaschen. Nach Trocknen über wasserfreiem Magnesiumsulfat wurde filtriert und eingeengt. Der verbliebene Rückstand wurde in wenig Dichlormethan gelöst, mit ein paar Tropfen Ethanol versetzt und anschließend über eine Biotage-Kartusche gereinigt (50 g Kieselgel, Laufmittelgradient: Cyclohexan/Ethylacetat 5:1 → 1:2). Nach Eindampfen der Produktfraktionen und Trocknen des Rückstands im Hochvakuum wurden 901 mg (67% d. Th.) der Titelverbindung erhalten.

¹H-NMR (400 MHz, DMSO-d₆, δ/ppm): 12.54 (s, 1H), 7.77 (s, 1H), 7.33-7.28 (m, 2H), 7.24-7.20 (m, 3H), 4.29 (quart, 2H), 4.03 (m, 2H), 2.84 (m, 2H), 1.30 (t, 3H).

LC/MS (Methode 1, ESIpos): Rₜ = 1.00 min, m/z = 345 [M+H]⁺.

### Beispiel 26A

### tert.-Butyl-5-methyl-2,4-dioxo-3-(2-phenylethyl)-1-(3,3,3-trifluorpropyl)-1,2,3,4-tetrahydrothieno-[2,3-d]pyrimidin-6-carboxylat

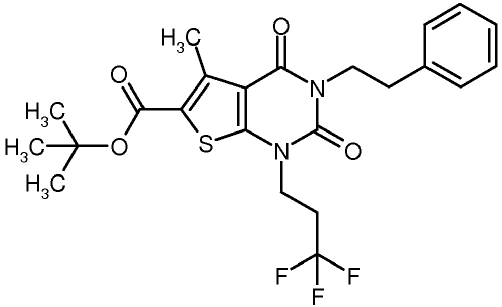

Eine Lösung von 5.98 g (15.5 mmol) der Verbindung aus Bsp. 8A in 150 ml DMF wurde mit 5.55 g (17.0 mmol) Cäsiumcarbonat versetzt und 15 min bei RT gerührt. Dann wurden 4.16 g (18.6 mmol) 3,3,3-Trifluor-1-iodpropan hinzugefügt, und das Gemisch wurde 3 h auf 100°C erhitzt. Danach wurde am Rotationsverdampfer zur Trockene eingedampft. Der verbliebene Rückstand wurde in Ethylacetat aufgenommen und nacheinander mit Wasser und gesättigter Natriumchlorid-Lösung gewaschen. Nach Trocknen über wasserfreiem Magnesiumsulfat wurde filtriert und eingedampft. Das Rohprodukt wurde mittels MPLC gereinigt (Biotage-Kartusche mit 340 g Kieselgel, Cyclohexan/Ethylacetat 10:1). Die Produktfraktionen wurden vereinigt und eingedampft. Abschließend wurde der Rückstand bei RT in 50 ml Pentan/Dichlormethan (100:5) verrührt. Nach neuerlicher Filtration, Eindampfen und Trocknen im Hochvakuum wurden 5.33 g (71% d. Th.) der Titelverbindung erhalten.

¹H-NMR (400 MHz, DMSO-d₆, δ/ppm): 7.33-7.28 (m, 2H), 7.25-7.20 (m, 3H), 4.14 (t, 2H), 4.06 (m, 2H), 2.84 (m, 2H), 2.79-2.72 (m, 2H), 2.76 (s, 3H), 1.54 (s, 9H).

LC/MS (Methode 1, ESIpos): Rₜ = 1.49 min, m/z = 483 [M+H]⁺.

### Beispiel 27A

### tert.-Butyl-1-(4,4-difluorbut-3-en-1-yl)-5-methyl-2,4-dioxo-3-(2-phenylethyl)-1,2,3,4-tetrahydro-thieno[2,3-d]pyrimidin-6-carboxylat

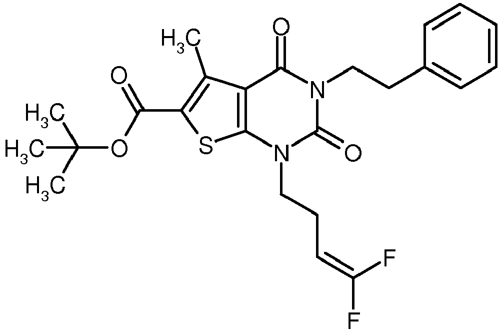

Analog zu dem unter Bsp. 26A beschriebenen Verfahren wurden aus 1.0 g (2.59 mmol) der Verbindung aus Bsp. 8A, 1.27 g (3.88 mmol) Cäsiumcarbonat und 664 mg (3.88 mmol) 4-Brom-1,1-difluorbut-1-en 468 mg (37% d. Th.) der Titelverbindung erhalten. Abweichend von dem zuvor beschriebenen Verfahren konnte hier auf die MPLC-Reinigung verzichtet werden. Das Rohprodukt wurde durch Verrühren bei RT in einem Gemisch aus 20 ml Pentan und 0.5 ml Dichlormethan gereinigt.

¹H-NMR (400 MHz, CDCl₃, δ/ppm): 7.32-7.29 (m, 4H), 7.26-7.20 (m, 1H), 4.28-4.24 (m, 1H), 4.24-4.18 (m, 2H), 3.97 (t, 2H), 2.96-2.92 (m, 2H), 2.86 (s, 3H), 2.47 (quart, 2H), 1.59 (s, 9H).

LC/MS (Methode 1, ESIpos): Rₜ = 1.52 min, m/z = 477 [M+H]⁺.

### Beispiel 28A

### tert.-Butyl-1-(2-methoxyethyl)-5-methyl-2,4-dioxo-3-(2-phenylethyl)-1,2,3,4-tetrahydrothieno-[2,3-d]pyrimidin-6-carboxylat

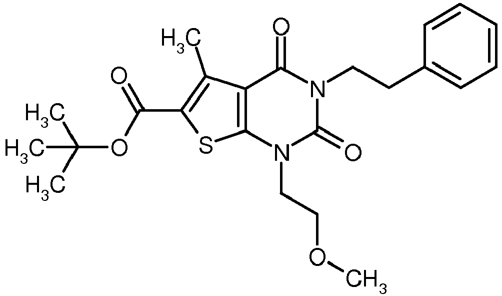

Eine Lösung von 2.0 g (5.18 mmol) der Verbindung aus Bsp. 8A in 60 ml DMF wurde mit 1.85 g (5.69 mmol) Cäsiumcarbonat versetzt und 10 min bei RT gerührt. Dann wurden 863 mg (6.21 mmol) 2-Bromethyl-methylether hinzugefügt, und das Gemisch wurde 30 min auf 100°C erhitzt. Danach wurde am Rotationsverdampfer zur Trockene eingedampft. Der verbliebene Rückstand wurde in Ethylacetat aufgenommen und nacheinander mit Wasser und gesättigter Natriumchlorid-Lösung gewaschen. Nach Trocknen über wasserfreiem Magnesiumsulfat wurde filtriert und eingedampft. Das Rohprodukt wurde mittels MPLC gereinigt (Puriflash-Säule mit 80 g Kieselgel, Cyclohexan/Ethylacetat 10:1). Nach Filtration, Eindampfen und Trocknen des Rückstands im Hochvakuum wurden 2.22 g (96% d. Th.) der Titelverbindung erhalten.

¹H-NMR (400 MHz, DMSO-d₆, δ/ppm): 7.32-7.28 (m, 2H), 7.24-7.20 (m, 3H), 4.09-4.04 (m, 4H), 3.61 (t, 2H), 3.24 (s, 3H), 2.84 (dd, 2H), 2.74 (s, 3H), 1.53 (s, 9H).

LC/MS (Methode 1, ESIpos): Rₜ = 1.40 min, m/z = 445 [M+H]⁺.

### Beispiel 29A

### tert.-Butyl-3-[2-(2-fluorphenyl)ethyl]-5-methyl-2,4-dioxo-1-(3,3,3-trifluorpropyl)-1,2,3,4-tetra-hydrothieno[2,3-d]pyrimidin-6-carboxylat

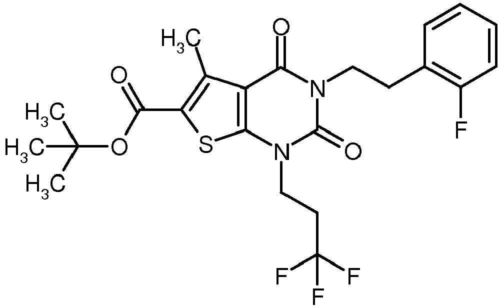

Eine Lösung von 750 mg (1.85 mmol) der Verbindung aus Bsp. 9A in 20 ml DMF wurde mit 665 mg (2.04 mmol) Cäsiumcarbonat versetzt und 15 min bei RT gerührt. Dann wurden 498 mg (2.23 mmol) 3,3,3-Trifluor-1-iodpropan hinzugefügt. Das Gemisch wurde insgesamt 17 h auf 100°C erhitzt, wobei nach 5 h Reaktionszeit noch einmal 498 mg (2.23 mmol) und nach 8 h Reaktionszeit weitere 249 mg (1.12 mmol) 3,3,3-Trifluor-1-iodpropan hinzugefügt wurden. Nach beendeter Reaktion wurde am Rotationsverdampfer zur Trockene eingedampft. Der verbliebene Rückstand wurde in Ethylacetat aufgenommen und nacheinander mit Wasser und gesättigter Natriumchlorid-Lösung gewaschen. Nach Trocknen über wasserfreiem Magnesiumsulfat wurde filtriert und eingedampft. Das Rohprodukt wurde mittels MPLC gereinigt (Puriflash-Säule mit 200 g Kieselgel, Cyclohexan/Ethylacetat 5:1). Die Produktfraktionen wurden vereinigt und eingedampft. Abschließend wurde der Rückstand bei RT in einem Gemisch aus 30 ml Pentan und 1 ml Dichlormethan verrührt. Nach neuerlicher Filtration, Eindampfen und Trocknen im Hochvakuum wurden 430 mg (44% d. Th., 95% Reinheit) der Titelverbindung erhalten.

¹H-NMR (400 MHz, DMSO-d₆, δ/ppm): 7.29-7.24 (m, 2H), 7.15-7.09 (m, 2H), 4.12 (t, 2H), 4.10 (t, 2H), 2.91 (t, 2H), 2.73 (s, 3H), 2.71 (t, 2H), 1.53 (s, 9H).

LC/MS (Methode 1, ESIpos): Rₜ = 1.42 min, m/z = 501 [M+H]⁺.

### Beispiel 30A

### tert.-Butyl-1-(4,4-difluorbut-3-en-1-yl)-3-[2-(2-fluorphenyl)ethyl]-5-methyl-2,4-dioxo-1,2,3,4-tetrahydrothieno[2,3-d]pyrimidin-6-carboxylat

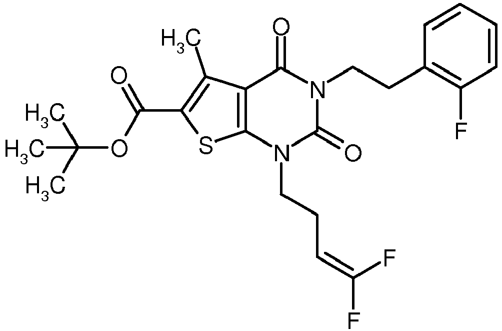

Analog zu dem unter Bsp. 29A beschriebenen Verfahren wurden aus 750 mg (1.85 mmol) der Verbindung aus Bsp. 9A, 906 mg (2.78 mmol) Cäsiumcarbonat und 476 mg (2.78 mmol) 4-Brom-1,1-difluorbut-1-en 480 mg (52% d. Th.) der Titelverbindung erhalten. Abweichend von dem zuvor beschriebenen Verfahren betrug die Reaktionszeit hier nur 60 min, und während dieser Zeit erfolgte keine weitere Zugabe von Alkylierungsagens. Außerdem konnte auf die MPLC-Reinigung verzichtet werden. Das Rohprodukt wurde durch Verrühren in 20 ml Pentan bei RT gereinigt.

¹H-NMR (400 MHz, DMSO-d₆, δ/ppm): 7.29-7.23 (m, 2H), 7.15-7.09 (m, 2H), 4.60 (d von t von d, 1H), 4.10 (t, 2H), 3.94 (t, 2H), 2.90 (t, 2H), 2.72 (s, 3H), 2.38-2.32 (m, 2H), 1.53 (s, 9H).

LC/MS (Methode 1, ESIpos): Rₜ = 1.46 min, m/z = 495 [M+H]⁺.

### Beispiel 31A

### tert.-Butyl-3-[2-(2-chlorphenyl)ethyl]-5-methyl-2,4-dioxo-1-(3,3,3-trifluorpropyl)-1,2,3,4-tetra-hydrothieno[2,3-d]pyrimidin-6-carboxylat

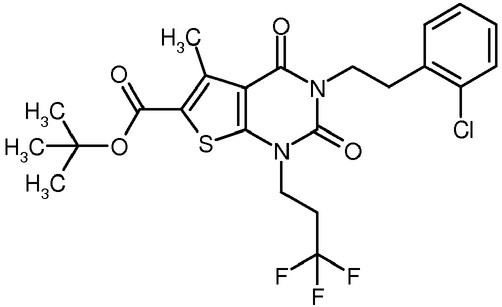

Analog zu dem unter Bsp. 29A beschriebenen Verfahren wurden aus 750 mg (1.78 mmol) der Verbindung aus Bsp. 10A, 639 mg (1.69 mmol) Cäsiumcarbonat und zunächst 479 mg (2.14 mmol) 3,3,3-Trifluor-1-iodpropan 440 mg (48% d. Th.) der Titelverbindung erhalten. Abweichend von dem zuvor beschriebenen Verfahren betrug die Reaktionszeit hier insgesamt 3 Tage, und die Zugabe von weiterem Alkylierungsagens erfolgte nach 6 h und 16 h (jeweils 240 mg, 1.07 mmol).

¹H-NMR (400 MHz, CDCl₃, δ/ppm): 7.36-7.33 (m, 1H), 7.27-7.23 (m, 1H, teilweise überdeckt vom CHCl₃-Signal), 7.19-7.14 (m, 2H), 4.28 (t, 2H), 4.13 (t, 2H), 3.11 (t, 2H), 2.84 (s, 3H), 2.59-2.47 (m, 2H), 1.59 (s, 9H).

LC/MS (Methode 1, ESIpos): Rₜ = 1.46 min, m/z = 517/519 [M+H]⁺.

### Beispiel 32A

### tert.-Butyl-3-[2-(2-chlorphenyl)ethyl]-1-(4,4-difluorbut-3-en-1-yl)-5-methyl-2,4-dioxo-1,2,3,4-tetrahydrothieno[2,3-d]pyrimidin-6-carboxylat

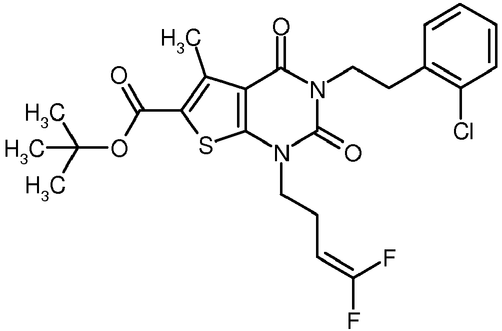

Analog zu dem unter Bsp. 29A beschriebenen Verfahren wurden aus 750 mg (1.85 mmol) der Verbindung aus Bsp. 10A, 871 mg (2.67 mmol) Cäsiumcarbonat und 457 mg (2.67 mmol) 4-Brom-1,1-difluorbut-1-en 543 mg (57% d. Th.) der Titelverbindung erhalten. Abweichend von dem zuvor beschriebenen Verfahren betrug die Reaktionszeit hier nur 60 min, und während dieser Zeit erfolgte keine weitere Zugabe von Alkylierungsagens. Außerdem konnte auf die MPLC-Reinigung verzichtet werden. Das Rohprodukt wurde durch Verrühren in 20 ml Pentan bei RT gereinigt.

¹H-NMR (400 MHz, DMSO-d₆, δ/ppm): 7.42-7.38 (m, 1H), 7.30-7.22 (m, 3H), 4.60 (d von t von d, 1H), 4.12 (t, 2H), 3.93 (t, 2H), 2.99 (t, 2H), 2.72 (s, 3H), 2.37-2.31 (m, 2H), 1.53 (s, 9H).

LC/MS (Methode 1, ESIpos): Rₜ = 1.50 min, m/z = 511/513 [M+H]⁺.

### Beispiel 33A

### tert.-Butyl-5-methyl-2,4-dioxo-3-[2-(pyridin-3-yl)ethyl]-1-(3,3,3-trifluorpropyl)-1,2,3,4-tetrahydro-thieno[2,3-d]pyrimidin-6-carboxylat

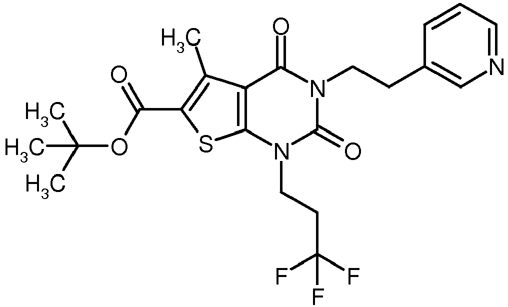

Eine Lösung von 900 mg (2.32 mmol) der Verbindung aus Bsp. 11A in 25 ml DMF wurde mit 1.14 g (3.48 mmol) Cäsiumcarbonat versetzt und 15 min bei RT gerührt. Dann wurden 780 mg (3.48 mmol) 3,3,3-Trifluor-1-iodpropan hinzugefügt, und das Gemisch wurde 6 h auf 90°C erhitzt. Danach wurde am Rotationsverdampfer zur Trockene eingedampft. Der verbliebene Rückstand wurde in Ethylacetat aufgenommen und nacheinander mit Wasser und gesättigter Natriumchlorid-Lösung gewaschen. Nach Trocknen über wasserfreiem Magnesiumsulfat wurde filtriert und eingedampft. Das Rohprodukt wurde mittels MPLC gereinigt (Biotage-Kartusche mit 50 g Kieselgel, Cyclohexan/Ethylacetat 5:1 → 1:1). Die Produktfraktionen wurden vereinigt und eingedampft. Der Rückstand wurde dann in einem Gemisch aus 20 ml Cyclohexan und 1 ml Dichlormethan 2 h lang bei RT verrührt. Nach neuerlicher Filtration, Eindampfen und Trocknen im Hochvakuum wurde eine erste Fraktion der Titelverbindung erhalten (117 mg, 10% d. Th.). Das Filtrat wurde nach Eindampfen mittels präparativer HPLC (Methode 5) weiter aufgereinigt. Nach Vereinigen und Eindampfen der Produktfraktionen wurde auf diese Weise eine zweite Fraktion der Titelverbindung erhalten (381 mg, 33% d. Th.). Insgesamt wurden so 498 mg (44% d. Th.) der Titelverbindung gewonnen.

¹H-NMR (400 MHz, DMSO-d₆, δ/ppm): 8.43-8.41 (m, 2H), 7.67-7.63 (m, 1H), 7.31 (dd, 1H), 4.13 (t, 2H), 4.10 (t, 2H), 2.89 (t, 2H), 2.80-2.69 (m, 2H), 2.73 (s, 3H), 1.53 (s, 9H).

LC/MS (Methode 1, ESIpos): Rₜ = 1.07 min, m/z = 484 [M+H]⁺.

### Beispiel 34A

### tert.-Butyl-1-(4,4-difluorbut-3-en-1-yl)-5-methyl-2,4-dioxo-3-[2-(pyridin-3-yl)ethyl]-1,2,3,4-tetra-hydrothieno[2,3-d]pyrimidin-6-carboxylat

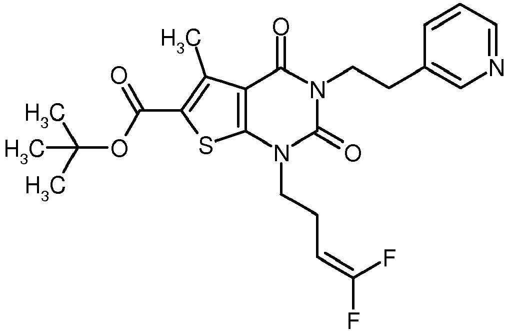

Analog zu dem unter Bsp. 33A beschriebenen Verfahren wurden aus 900 mg (2.32 mmol) der Verbindung aus Bsp. 11A, 1.14 g (3.48 mmol) Cäsiumcarbonat und 596 mg (3.48 mmol) 4-Brom-1,1-difluorbut-1-en insgesamt 610 mg (55% d. Th.) der Titelverbindung erhalten.

¹H-NMR (400 MHz, DMSO-d₆, δ/ppm): 8.43-8.40 (m, 2H), 7.67-7.64 (m, 1H), 7.31 (dd, 1H), 4.61 (d von t von d, 1H), 4.10 (t, 2H), 3.94 (t, 2H), 2.88 (t, 2H), 2.73 (s, 3H), 2.39-2.33 (m, 2H), 1.53 (s, 9H).

LC/MS (Methode 1, ESIpos): Rₜ = 1.10 min, m/z = 478 [M+H]⁺.

### Beispiel 35A

### tert.-Butyl-3-ethyl-5-methyl-2,4-dioxo-1-(3,3,3-trifluorpropyl)-1,2,3,4-tetrahydrothieno[2,3-d]-pyrimidin-6-carboxylat

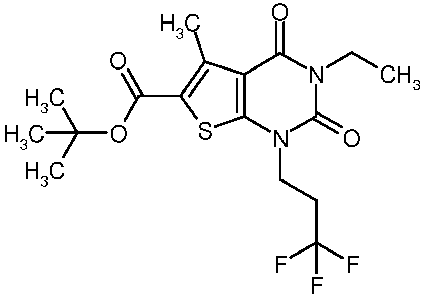

Eine Lösung von 3.0 g (9.67 mmol) der Verbindung aus Bsp. 12A in 100 ml DMF wurde mit 4.72 g (14.5 mmol) Cäsiumcarbonat versetzt und 20 min bei RT gerührt. Dann wurden 2.57 g (14.5 mmol) 3,3,3-Trifluor-1-iodpropan hinzugefügt, und das Gemisch wurde 5 h auf 100°C erhitzt. Danach wurde am Rotationsverdampfer zur Trockene eingedampft. Der verbliebene Rückstand wurde in Ethylacetat aufgenommen und nacheinander mit Wasser und gesättigter Natriumchlorid-Lösung gewaschen. Nach Trocknen über wasserfreiem Magnesiumsulfat wurde filtriert und eingedampft. Das Rohprodukt wurde mittels MPLC gereinigt (Puriflash-Säule mit 120 g Kieselgel, Cyclohexan/ Ethylacetat 10:1). Die Produktfraktionen wurden vereinigt, eingedampft und der Rückstand im Hochvakuum getrocknet. Es wurden 3.24 g (76% d. Th., 93% Reinheit) der Titelverbindung erhalten.

¹H-NMR (400 MHz, DMSO-d₆, δ/ppm): 4.15 (t, 2H), 3.90 (quart, 2H), 2.84-2.74 (m, 2H), 2.76 (s, 3H), 1.53 (s, 9H), 1.13 (t, 3H).

LC/MS (Methode 1, ESIpos): Rₜ = 1.36 min, m/z = 407 [M+H]⁺.

### Beispiel 36A

### tert.-Butyl-1-(4,4-difluorbut-3-en-1-yl)-3-ethyl-5-methyl-2,4-dioxo-1,2,3,4-tetrahydrothieno[2,3-d]-pyrimidin-6-carboxylat

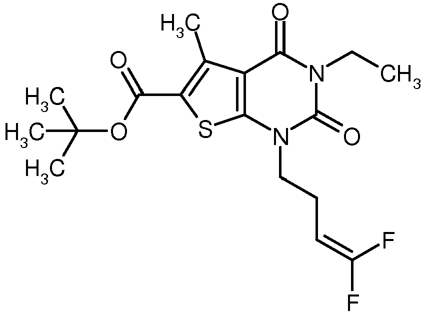

Analog zu dem unter Bsp. 35A beschriebenen Verfahren wurden aus 800 mg (2.58 mmol) der Verbindung aus Bsp. 12A, 1.26 g (3.87 mmol) Cäsiumcarbonat und 661 mg (3.87 mmol) 4-Brom-1,1-difluorbut-1-en 778 mg (76% d. Th.) der Titelverbindung erhalten. Abweichend von dem zuvor beschriebenen Verfahren betrug die Reaktionszeit hier nur 1 h. Ebenfalls abweichend wurde für die MPLC-Reinigung eine 40 g Kieselgel enthaltende Puriflash-Kartusche verwendet, und das Produkt wurde abschließend durch Verrühren in Pentan/Dichlormethan (30:1) gereinigt.

¹H-NMR (400 MHz, CDCl₃, δ/ppm): 4.25 (d von t von d, 1H), 4.06 (quart, 2H), 3.98 (t, 2H), 2.85 (s, 3H), 2.51 (*pseudo-*quart, 2H), 1.59 (s, 9H), 1.25 (t, 3H).

LC/MS (Methode 1, ESIpos): Rₜ = 1.39 min, m/z = 401 [M+H]⁺.

### Beispiel 37A

### tert.-Butyl-3-ethyl-1-(2-methoxyethyl)-5-methyl-2,4-dioxo-1,2,3,4-tetrahydrothieno[2,3-d]pyrimidin-6-carboxylat

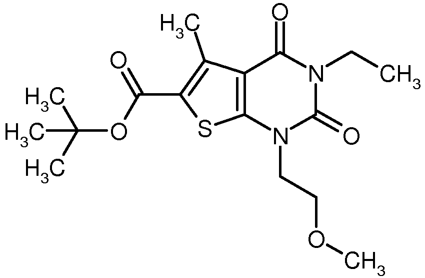

Analog zu dem unter Bsp. 35A beschriebenen Verfahren wurden aus 800 mg (2.58 mmol) der Verbindung aus Bsp. 12A, 1.26 g (3.87 mmol) Cäsiumcarbonat und 537 mg (3.87 mmol) 2-Bromethyl-methylether 680 mg (71% d. Th.) der Titelverbindung erhalten. Abweichend von dem zuvor beschriebenen Verfahren wurde hier für die MPLC-Reinigung eine 50 g Kieselgel enthaltende Biotage-Kartusche verwendet, und das Produkt wurde abschließend durch Verrühren in 31 ml Pentan/Dichlormethan (30:1) gereinigt.

¹H-NMR (400 MHz, CDCl₃, δ/ppm): 4.13 (t, 2H), 4.07 (quart, 2H), 3.74 (t, 2H), 3.36 (s, 3H), 2.84 (s, 3H), 1.58 (s, 9H), 1.26 (t, 3H).

LC/MS (Methode 1, ESIpos): Rₜ = 1.24 min, m/z = 369 [M+H]⁺.

### Beispiel 38A

### Ethyl-2,4-dioxo-3-(2-phenylethyl)-5-(trifluormethyl)-1-(3,3,3-trifluorpropyl)-1,2,3,4-tetrahydro-thieno[2,3-d]pyrimidin-6-carboxylat

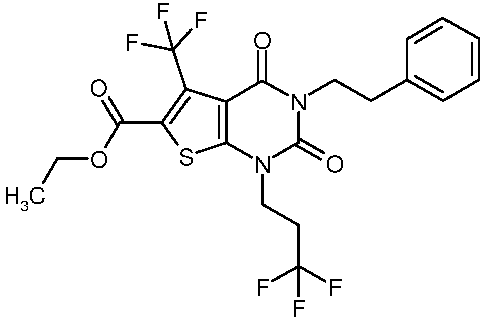

Eine Lösung von 700 mg (1.70 mmol) der Verbindung aus Bsp. 20A in 20 ml DMF wurde mit 719 mg (2.21 mmol) Cäsiumcarbonat versetzt und 15 min bei RT gerührt. Dann wurden 570 mg (2.55 mmol) 3,3,3-Trifluor-1-iodpropan hinzugefügt, und das Gemisch wurde 60 min auf 100°C erhitzt. Danach wurde mit 400 ml Wasser versetzt und dreimal mit je ca. 100 ml Diethylether extrahiert. Die vereinigten organischen Extrakte wurden mit gesättigter Natriumchlorid-Lösung gewaschen. Nach Trocknen über wasserfreiem Magnesiumsulfat wurde filtriert und eingedampft. Das Rohprodukt wurde in einem Gemisch aus 25 ml Pentan und 0.5 ml Diethylether 1 h lang bei RT verrührt. Es wurde filtriert und der Rückstand im Hochvakuum getrocknet. Auf diese Weise wurde eine erste Fraktion der Titelverbindung erhalten (497 mg, 57% d. Th.). Eine zweite Fraktion wurde durch präparative HPLC (Methode 6) aus der Mutterlauge des Verrührens isoliert (138 mg, 16% d. Th.). Insgesamt wurden so 635 mg (73% d. Th.) der Titelverbindung gewonnen.

¹H-NMR (400 MHz, CDCl₃, δ/ppm): 7.32-7.20 (m, 5H, teilweise überdeckt vom CHCl₃-Signal), 4.42 (quart, 2H), 4.26-4.22 (m, 2H), 4.16 (t, 2H), 2.98-2.94 (m, 2H), 2.64-2.53 (m, 2H), 1.41 (t, 3H).

LC/MS (Methode 1, ESIpos): Rₜ = 1.30 min, m/z = 509 [M+H]⁺.

### Beispiel 39A

### Ethyl-3-ethyl-2,4-dioxo-5-(trifluormethyl)-1-(3,3,3-trifluorpropyl)-1,2,3,4-tetrahydrothieno[2,3-d]-pyrimidin-6-carboxylat

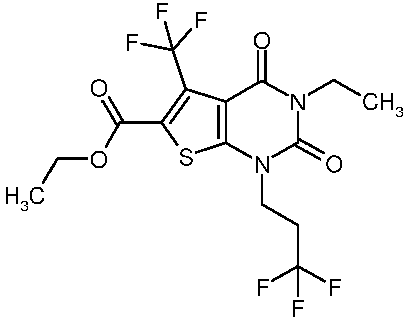

Eine Lösung von 2.0 g (5.95 mmol) der Verbindung aus Bsp. 21A in 40 ml DMF wurde mit 1.39 g (13.1 mmol) Cäsiumcarbonat versetzt und 15 min bei RT gerührt. Dann wurden 2.66 g (11.9 mmol) 3,3,3-Trifluor-1-iodpropan hinzugefügt, und das Gemisch wurde auf 60°C erwärmt. Nach 1 h wurden weitere 2.66 g (11.9 mmol) 3,3,3-Trifluor-1-iodpropan hinzugefügt. Das Rühren bei 60°C wurde 16 h lang fortgesetzt. Nach dem Abkühlen auf RT wurde mit ca. 160 ml Wasser versetzt und dreimal mit je ca. 80 ml Diethylether extrahiert. Die vereinigten organischen Extrakte wurden mit gesättigter Natriumchlorid-Lösung gewaschen. Nach Trocknen über wasserfreiem Magnesiumsulfat wurde filtriert und eingedampft. Das Rohprodukt wurde mittels MPLC über eine Puriflash-Kartusche gereinigt (100 g Kieselgel, Cyclohexan/Ethylacetat 7:1 → 1:1). Die Produktfraktionen wurden vereinigt, eingedampft und der Rückstand im Hochvakuum getrocknet. Es wurden 1.86 g (72% d. Th.) der Titelverbindung erhalten.

¹H-NMR (400 MHz, CDCl₃, δ/ppm): 4.41 (quart, 2H), 4.20 (t, 2H), 4.08 (quart, 2H), 2.73-2.61 (m, 2H), 1.40 (t, 3H), 1.26 (t, 3H).

LC/MS (Methode 1, ESIpos): Rₜ = 1.15 min, m/z = 433 [M+H]⁺.

### Beispiel 40A

### Ethyl-5-(difluormethyl)-2,4-dioxo-3-(2-phenylethyl)-1-(3,3,3-trifluorpropyl)-1,2,3,4-tetrahydro-thieno[2,3-d]pyrimidin-6-carboxylat

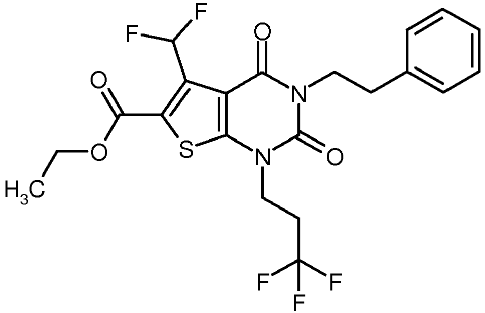

Analog zu dem unter Bsp. 38A beschriebenen Verfahren wurden aus 2.90 g (7.35 mmol) der Verbindung aus Bsp. 23A und 1.72 ml (14.7 mmol) 3,3,3-Trifluor-1-iodpropan 2.22 g (61% d. Th.) der Titelverbindung erhalten. Das Rohprodukt wurde hier zur Reinigung dreimal hintereinander mit folgenden Lösungsmittelgemischen bei RT verrührt: Beim ersten und zweiten Mal jeweils mit 100 ml Pentan und 5 ml Diethylether, beim dritten Mal mit 50 ml Pentan und 5 ml Diethylether.

¹H-NMR (400 MHz, DMSO-d₆, δ/ppm): 7.74 (t, 1H), 7.33-7.29 (m, 2H), 7.26-7.21 (m, 3H), 4.36 (quart, 2H), 4.20 (t, 2H), 4.08 (m, 2H), 2.87-2.75 (m, 4H), 1.32 (t, 3H).

LC/MS (Methode 1, ESIpos): Rₜ = 1.27 min, m/z = 491 [M+H]⁺.

### Beispiel 41A

### Ethyl-2,4-dioxo-3-(2-phenylethyl)-1-(3,3,3-trifluorpropyl)-1,2,3,4-tetrahydrothieno[2,3-d]-pyrimidin-6-carboxylat

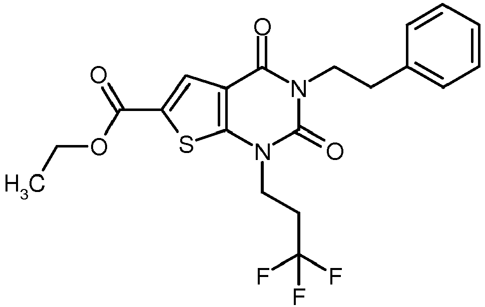

Eine Lösung von 900 mg (2.61 mmol) der Verbindung aus Bsp. 25A in 10 ml DMF wurde mit 1.28 g (3.92 mmol) Cäsiumcarbonat versetzt und 20 min bei RT gerührt. Dann wurden 878 mg (3.92 mmol) 3,3,3-Trifluor-1-iodpropan hinzugefügt, und das Gemisch wurde in einem Mikrowellenofen (Biotage Initiator mit dynamischer Steuerung der Einstrahlleistung) für 2 h 45 min bei 100°C gerührt. Nach dem Abkühlen auf RT wurde mit ca. 60 ml Wasser versetzt und mit Ethylacetat extrahiert. Der organische Extrakt wurde mit Wasser und gesättigter Natriumchlorid-Lösung gewaschen. Nach Trocknen über wasserfreiem Magnesiumsulfat wurde filtriert und eingeengt. Der erhaltene Rückstand wurde mit einem Gemisch aus 20 ml Pentan und 1 ml Diethylether für ca. 16 h bei RT verrührt. Nach Absaugen und Trocknen im Hochvakuum wurden 895 mg (77% d. Th.) der Titelverbindung erhalten.

¹H-NMR (400 MHz, DMSO-d₆, δ/ppm): 7.87 (s, 1H), 7.32-7.28 (m, 2H), 7.24-7.19 (m, 3H), 4.32 (quart, 2H), 4.17 (t, 2H), 4.09 (m, 2H), 2.89-2.73 (m, 4H), 1.31 (t, 3H).

LC/MS (Methode 1, ESIpos): Rₜ = 1.24 min, m/z = 441 [M+H]⁺.

### Beispiel 42A

### tert.-Butyl-3-(2,4-dimethoxybenzyl)-5-methyl-2,4-dioxo-1-(3,3,3-trifluorpropyl)-1,2,3,4-tetra-hydrothieno[2,3-d]pyrimidin-6-carboxylat

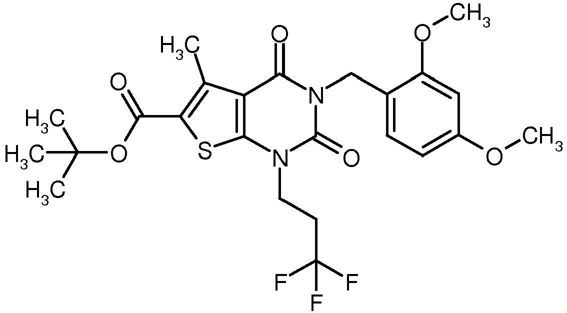

Eine Lösung von 11.3 g (26.2 mmol) der Verbindung aus Bsp. 13A in 250 ml DMF wurde mit 6.10 g (57.6 mmol) Cäsiumcarbonat versetzt und 15 min bei RT gerührt. Dann wurden 11.7 g (52.3 mmol) 3,3,3-Trifluor-1-iodpropan hinzugefügt, und das Gemisch wurde auf 70°C erwärmt. Nach 6 h wurden weitere 11.7 g (52.3 mmol) 3,3,3-Trifluor-1-iodpropan hinzugefügt. Das Rühren bei 70°C wurde 7 h lang fortgesetzt. Nach dem Abkühlen auf RT wurde der größte Teil der flüchtigen Komponenten (einschließlich DMF) am Rotationsverdampfer entfernt. Der Rückstand wurde mit ca. 400 ml Ethylacetat versetzt und nacheinander mit Wasser und gesättigter Natriumchlorid-Lösung gewaschen. Nach Trocknen der organischen Phase über wasserfreiem Magnesiumsulfat wurde filtriert und eingedampft. Das Rohprodukt wurde mittels MPLC über eine Biotage-Kartusche gereinigt (340 g Kieselgel, Cyclohexan/Ethylacetat 5:1). Die Produktfraktionen wurden vereinigt und eingedampft. Nach Verrühren des Rückstands mit Pentan/Dichlormethan (25:1), neuerlichem Filtrieren und Trocknen im Hochvakuum wurden 8.78 g (63% d. Th.) der Titelverbindung erhalten.

¹H-NMR (400 MHz, DMSO-d₆, δ/ppm): 6.75 (d, 1H), 6.57 (d, 1H), 6.39 (dd, 1H), 4.95 (s, 2H), 4.17 (t, 2H), 3.81 (s, 3H), 3.72 (s, 3H), 2.87-2.74 (m, 2H), 2.74 (s, 3H), 1.54 (s, 9H).

LC/MS (Methode 1, ESIpos): Rₜ = 1.38 min, m/z = 529 [M+H]⁺.

### Beispiel 43A

### 5-Methyl-2,4-dioxo-3-(2-phenylethyl)-1-(3,3,3-trifluorpropyl)-1,2,3,4-tetrahydrothieno[2,3-d]-pyrimidin-6-carbonsäure

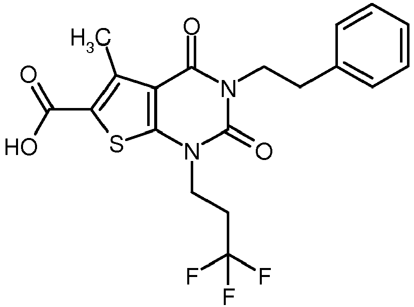

Eine Lösung von 5.69 g (11.8 mmol) der Verbindung aus Bsp. 26A in 240 ml Dichlormethan wurde mit 80 ml Trifluoressigsäure versetzt und 2 h bei RT gerührt. Danach wurde das Reaktionsgemisch am Rotationsverdampfer zur Trockene eingedampft. Der verbliebene Rückstand wurde in Diethylether verrührt, abgesaugt und der Feststoff im Hochvakuum getrocknet. Es wurden 4.89 g (97% d. Th.) der Titelverbindung erhalten.

¹H-NMR (400 MHz, DMSO-d₆, δ/ppm): 13.47 (br. s, 1H), 7.33-7.28 (m, 2H), 7.26-7.20 (m, 3H), 4.14 (t, 2H), 4.07 (m, 2H), 2.84 (m, 2H), 2.80-2.70 (m, 2H), 2.77 (s, 3H).

LC/MS (Methode 1, ESIpos): Rₜ = 1.11 min, m/z = 427 [M+H]⁺.

### Beispiel 44A

### 1-(4,4-Difluorbut-3-en-1-yl)-5-methyl-2,4-dioxo-3-(2-phenylethyl)-1,2,3,4-tetrahydrothieno[2,3-d]-pyrimidin-6-carbonsäure

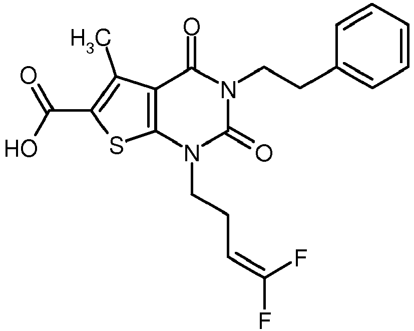

Analog zu dem unter Bsp. 43A beschriebenen Verfahren wurden aus 520 mg (1.09 mmol) der Verbindung aus Bsp. 27A und 5 ml Trifluoressigsäure in 10 ml Dichlormethan 328 mg (71% d. Th.) der Titelverbindung erhalten.

¹H-NMR (400 MHz, DMSO-d₆, δ/ppm): 13.42 (br. s, 1H), 7.33-7.28 (m, 2H), 7.25-7.20 (m, 3H), 4.63 (d von t von d, 1H), 4.07 (m, 2H), 3.96 (t, 2H), 2.83 (m, 2H), 2.76 (s, 3H), 2.38 (m, 2H).

LC/MS (Methode 1, ESIpos): Rₜ = 1.15 min, m/z = 421 [M+H]⁺.

### Beispiel 45A

### 1-(2-Methoxyethyl)-5-methyl-2,4-dioxo-3-(2-phenylethyl)-1,2,3,4-tetrahydrothieno[2,3-d]pyrimidin-6-carbonsäure

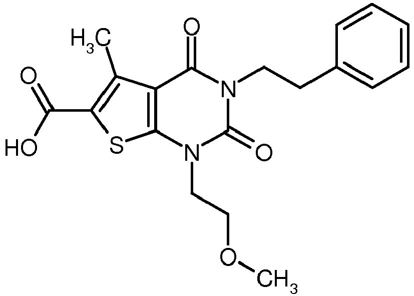

Eine Lösung von 5.0 g (11.2 mmol) der Verbindung aus Bsp. 28A in 225 ml Dichlormethan wurde mit 75 ml Trifluoressigsäure versetzt und 2 h bei RT gerührt. Danach wurde das Reaktionsgemisch am Rotationsverdampfer zur Trockene eingedampft. Der verbliebene Rückstand wurde in Diethylether verrührt, abgesaugt und der Feststoff im Hochvakuum getrocknet. Es wurden 4.1 g (92% d. Th.) der Titelverbindung erhalten.

¹H-NMR (400 MHz, DMSO-d₆, δ/ppm): 13.37 (br. s, 1H), 7.33-7.29 (m, 2H), 7.25-7.20 (m, 3H), 4.09-4.04 (m, 4H), 3.62 (t, 2H), 3.25 (s, 3H), 2.84 (dd, 2H), 2.75 (s, 3H).

LC/MS (Methode 1, ESIpos): Rₜ = 1.03 min, m/z = 389 [M+H]⁺.

### Beispiel 46A

### 3-[2-(2-Fluorphenyl)ethyl]-5-methyl-2,4-dioxo-1-(3,3,3-trifluorpropyl)-1,2,3,4-tetrahydrothieno-[2,3-d]pyrimidin-6-carbonsäure

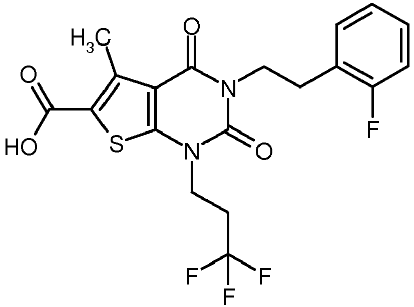

Eine Lösung von 420 mg (0.84 mmol) der Verbindung aus Bsp. 29A in 20 ml Dichlormethan wurde mit 10 ml Trifluoressigsäure versetzt und 1 h bei RT gerührt. Danach wurde das Reaktionsgemisch am Rotationsverdampfer zur Trockene eingedampft und der Rückstand anschließend im Hochvakuum getrocknet. Es wurden 300 mg (79% d. Th.) der Titelverbindung erhalten.

¹H-NMR (400 MHz, DMSO-d₆, δ/ppm): 13.47 (br. s, 1H), 7.29-7.24 (m, 2H), 7.16-7.09 (m, 2H), 4.12 (t, 2H), 4.10 (t, 2H), 2.91 (t, 2H), 2.74 (s, 3H), 2.70 (t, 2H).

LC/MS (Methode 1, ESIpos): Rₜ = 1.11 min, m/z = 445 [M+H]⁺.

### Beispiel 47A

### 1-(4,4-Difluorbut-3-en-1-yl)-3-[2-(2-fluorphenyl)ethyl]-5-methyl-2,4-dioxo-1,2,3,4-tetrahydro-thieno[2,3-d]pyrimidin-6-carbonsäure

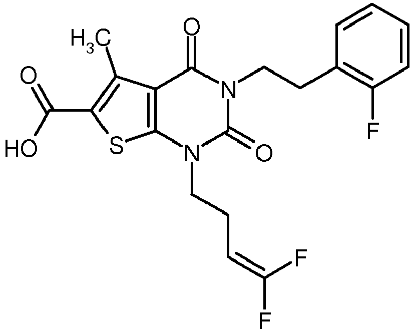

Eine Lösung von 750 mg (1.52 mmol) der Verbindung aus Bsp. 30A in 30 ml Dichlormethan wurde mit 15 ml Trifluoressigsäure versetzt und 1 h bei RT gerührt. Danach wurde das Reaktionsgemisch am Rotationsverdampfer zur Trockene eingedampft. Der verbliebene Rückstand wurde in Diethylether verrührt, abgesaugt und der Feststoff im Hochvakuum getrocknet. Es wurden so 320 mg (47% d. Th.) der Titelverbindung erhalten. Da die Mutterlauge außer der Zielverbindung keine weiteren Verunreinigungen enthielt, wurde sie vollständig eingedampft und der verbliebene Rückstand ebenfalls im Hochvakuum getrocknet (290 mg, 42% d. Th.). Insgesamt wurden auf diese Weise 610 mg (90% d. Th.) der Titelverbindung erhalten.

¹H-NMR (400 MHz, DMSO-d₆, δ/ppm): 13.42 (br. s, 1H), 7.30-7.24 (m, 2H), 7.16-7.10 (m, 2H), 4.61 (d von t von d, 1H), 4.10 (t, 2H), 3.93 (t, 2H), 2.90 (t, 2H), 2.73 (s, 3H), 2.38-2.32 (m, 2H).

LC/MS (Methode 1, ESIpos): Rₜ = 1.15 min, m/z = 439 [M+H]⁺.

### Beispiel 48A

### 3-[2-(2-Chlorphenyl)ethyl]-5-methyl-2,4-dioxo-1-(3,3,3-trifluorpropyl)-1,2,3,4-tetrahydrothieno-[2,3-d]pyrimidin-6-carbonsäure

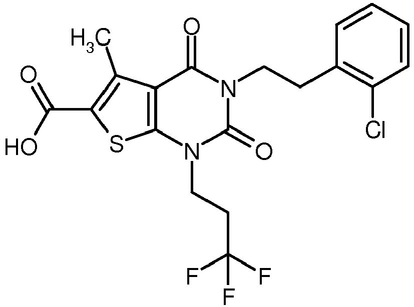

Eine Lösung von 420 mg (0.81 mmol) der Verbindung aus Bsp. 31A in 15 ml Dichlormethan wurde mit 7 ml Trifluoressigsäure versetzt und 2 h bei RT gerührt. Danach wurde das Reaktionsgemisch am Rotationsverdampfer zur Trockene eingedampft. Der verbliebene Rückstand wurde in Diethylether verrührt, abgesaugt und der Feststoff im Hochvakuum getrocknet. Es wurden 295 mg (79% d. Th.) der Titelverbindung erhalten.

¹H-NMR (400 MHz, DMSO-d₆, δ/ppm): 13.46 (br. s, 1H), 7.43-7.39 (m, 1H), 7.31-7.24 (m, 3H), 4.15-4.10 (m, 4H), 3.00 (t, 2H), 2.76-2.67 (m, 2H), 2.74 (s, 3H).

LC/MS (Methode 1, ESIpos): Rₜ = 1.17 min, m/z = 461/463 [M+H]⁺.

### Beispiel 49A

### 3-[2-(2-Chlorphenyl)ethyl]-1-(4,4-difluorbut-3-en-1-yl)-5-methyl-2,4-dioxo-1,2,3,4-tetrahydro-thieno[2,3-d]pyrimidin-6-carbonsäure

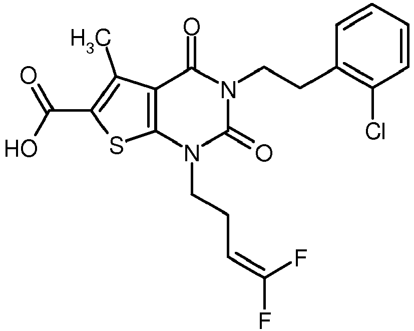

Analog zu dem unter Bsp. 48A beschriebenen Verfahren wurden aus 525 mg (1.03 mmol) der Verbindung aus Bsp. 32A und 7 ml Trifluoressigsäure in 15 ml Dichlormethan 345 mg (73% d. Th.) der Titelverbindung erhalten.

¹H-NMR (400 MHz, DMSO-d₆, δ/ppm): 13.41 (br. s, 1H), 7.42-7.39 (m, 1H), 7.31-7.24 (m, 3H), 4.61 (d von t von d, 1H), 4.12 (t, 2H), 3.93 (t, 2H), 2.99 (t, 2H), 2.73 (s, 3H), 2.37-2.31 (m, 2H).

LC/MS (Methode 1, ESIpos): Rₜ = 1.19 min, m/z = 455/457 [M+H]⁺.

### Beispiel 50A

### 5-Methyl-2,4-dioxo-3-[2-(pyridin-3-yl)ethyl]-1-(3,3,3-trifluorpropyl)-1,2,3,4-tetrahydrothieno-[2,3-d]pyrimidin-6-carbonsäure-Hydrochlorid

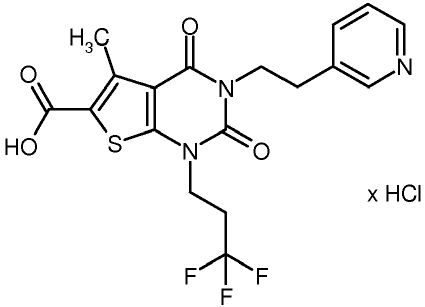

450 mg (0.93 mmol) der Verbindung aus Bsp. 33A wurden in 9.3 ml einer 4 M Lösung von Chlorwasserstoff in Dioxan versetzt und ca. 16 h bei RT gerührt. Danach wurde das Reaktionsgemisch am Rotationsverdampfer zur Trockene eingedampft. Der verbliebene Rückstand wurde in 10 ml Diethylether 30 min lang bei RT verrührt, abgesaugt und der Feststoff im Hochvakuum getrocknet. Es wurden 463 mg (91% d. Th.) der Titelverbindung erhalten.

¹H-NMR (400 MHz, DMSO-d₆, δ/ppm): 13.50 (br. s, 1H), 8.82 (s, 1H), 8.74 (d, 1H), 8.36 (d, 1H), 7.88 (t, 1H), 4.19 (t, 2H), 4.12 (t, 2H), 3.09 (t, 2H), 2.80-2.68 (m, 2H), 2.72 (s, 3H).

LC/MS (Methode 1, ESIpos): Rₜ = 0.67 min, m/z = 428 [M+H]⁺.

### Beispiel 51A

### 1-(4,4-Difluorbut-3-en-1-yl)-5-methyl-2,4-dioxo-3-[2-(pyridin-3-yl)ethyl]-1,2,3,4-tetrahydrothieno-[2,3-d]pyrimidin-6-carbonsäure-Hydrochlorid

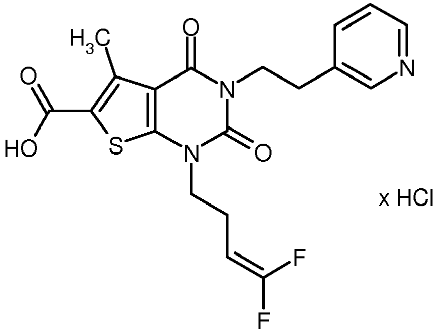

Analog zu dem unter Bsp. 50A beschriebenen Verfahren wurden aus 450 mg (0.94 mmol) der Verbindung aus Bsp. 34A 365 mg (82% d. Th.) der Titelverbindung erhalten.

¹H-NMR (400 MHz, DMSO-d₆, δ/ppm): 13.45 (br. s, 1H), 8.82 (s, 1H), 8.73 (d, 1H), 8.35 (d, 1H), 7.87 (t, 1H), 4.61 (d von t von d, 1H), 4.19 (t, 2H), 3.92 (t, 2H), 3.08 (t, 2H), 2.72 (s, 3H), 2.37-2.30 (m, 2H).

LC/MS (Methode 1, ESIpos): Rₜ = 0.70 min, m/z = 422 [M+H]⁺.

### Beispiel 52A

### 3-Ethyl-5-methyl-2,4-dioxo-1-(3,3,3-trifluorpropyl)-1,2,3,4-tetrahydrothieno[2,3-d]pyrimidin-6-carbonsäure

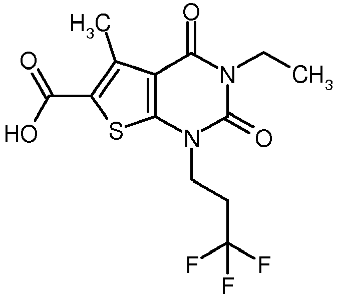

### Methode A:

Eine Lösung von 6.89 g (16.9 mmol) der Verbindung aus Bsp. 35A in 240 ml Dichlormethan wurde mit 80 ml Trifluoressigsäure versetzt und 2 h bei RT gerührt. Danach wurde das Reaktionsgemisch am Rotationsverdampfer zur Trockene eingedampft. Der verbliebene Rückstand wurde in Diethylether verrührt, abgesaugt und der Feststoff im Hochvakuum getrocknet. Es wurden 5.13 g (86% d. Th.) der Titelverbindung erhalten.

¹H-NMR (400 MHz, DMSO-d₆, δ/ppm): 13.46 (br. s, 1H), 4.15 (t, 2H), 3.91 (quart, 2H), 2.85-2.73 (m, 2H), 2.76 (s, 3H), 1.13 (t, 3H).

LC/MS (Methode 1, ESIpos): Rₜ = 0.94 min, m/z = 351 [M+H]⁺.

### Methode B:

Eine Lösung von 2.9 g (8.67 mmol) der Verbindung aus Bsp. 167A in einem Gemisch aus 22 ml Aceton und 10 ml Wasser wurde bei ca. 2°C mit 926 mg (9.54 mmol) Sulfaminsäure und 1.56 g (13.0 mmol) Natriumdihydrogenphosphat versetzt. Anschließend wurde eine Lösung von 1.03 g (9.11 mmol, 80% Gehalt) Natriumchlorit in 4 ml Wasser so langsam zugetropft, dass die Temperatur des Reaktionsgemisches nicht über 6°C stieg. Nach 30 min wurde das Kältebad entfernt und das Gemisch über Nacht bei RT weiter gerührt. Danach wurde mit 100 ml Wasser verdünnt und zweimal mit je 150 ml Ethylacetat extrahiert. Der organische Extrakt wurde über wasserfreiem Natriumsulfat getrocknet, anschließend filtriert und das Filtrat zur Trockene eingeengt. Das Rohprodukt wurde durch Chromatographie gereinigt (40 g Kieselgel, Laufmittel: Dichlormethan/ Methanol 100:0 → 90:10). Nach Einengen der Produktfraktionen und Trocknen des Rückstandes wurden 2.60 g (85% d. Th.) der Titelverbindung erhalten.

### Beispiel 53A

### 1-(4,4-Difluorbut-3-en-1-yl)-3-ethyl-5-methyl-2,4-dioxo-1,2,3,4-tetrahydrothieno[2,3-d]pyrimidin-6-carbonsäure

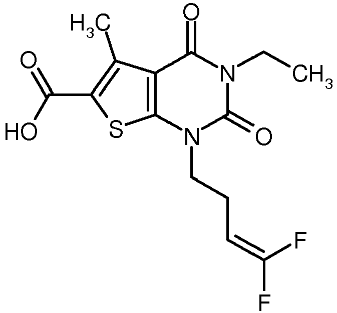

Analog zu dem unter Bsp. 52A beschriebenen Verfahren wurden aus 750 mg (1.87 mmol) der Verbindung aus Bsp. 36A 528 mg (81% d. Th.) der Titelverbindung erhalten.

¹H-NMR (400 MHz, DMSO-d₆, δ/ppm): 13.40 (br. s, 1H), 4.64 (d von t von d, 1H), 3.96 (t, 2H), 3.90 (quart, 2H), 2.76 (s, 3H), 2.40 (*pseudo-*quart, 2H), 1.12 (t, 3H).

LC/MS (Methode 1, ESIpos): Rₜ = 0.94 min, m/z = 345 [M+H]⁺.

### Beispiel 54A

### 3-Ethyl-1-(2-methoxyethyl)-5-methyl-2,4-dioxo-1,2,3,4-tetrahydrothieno[2,3-d]pyrimidin-6-carbonsäure

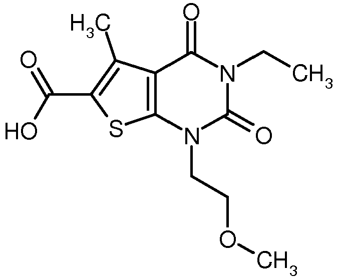

Analog zu dem unter Bsp. 52A beschriebenen Verfahren wurden aus 650 mg (1.76 mmol) der Verbindung aus Bsp. 37A 469 mg (85% d. Th.) der Titelverbindung erhalten.

¹H-NMR (400 MHz, DMSO-d₆, δ/ppm): 13.36 (br. s, 1H), 4.07 (t, 2H), 3.90 (quart, 2H), 3.65 (t, 2H), 3.25 (s, 3H), 2.75 (s, 3H), 1.13 (t, 3H).

LC/MS (Methode 1, ESIpos): Rₜ = 0.78 min, m/z = 313 [M+H]⁺.

### Beispiel 55A

### 2,4-Dioxo-3-(2-phenylethyl)-5-(trifluormethyl)-1-(3,3,3-trifluorpropyl)-1,2,3,4-tetrahydrothieno-[2,3-d]pyrimidin-6-carbonsäure

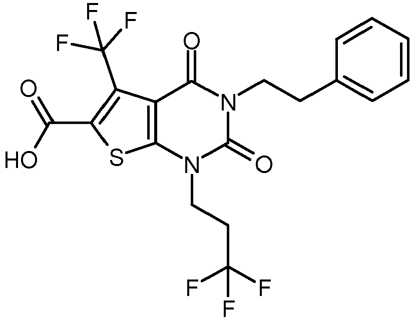

Eine Lösung von 495 mg (0.970 mmol) der Verbindung aus Bsp. 38A in 20 ml Ethanol wurde mit einer Lösung von 35 mg (1.46 mmol) Lithiumhydroxid in 10 ml Wasser versetzt und ca. 16 h bei RT gerührt. Anschließend wurden alle flüchtigen Bestandteile am Rotationsverdampfer entfernt. Der verbliebene Rückstand wurde in Wasser aufgenommen und mit 2 ml 1 M Salzäure angesäuert. Dabei fiel das Produkt aus, das abgesaugt, mit Wasser gewaschen und im Hochvakuum getrocknet wurde. Es wurden 460 mg (95% d. Th.) der Titelverbindung erhalten.

¹H-NMR (400 MHz, DMSO-d₆, δ/ppm): 14.50 (br. s, 1H), 7.33-7.28 (m, 2H), 7.26-7.20 (m, 3H), 4.18 (t, 2H), 4.07 (m, 2H), 2.74-2.68 (m, 4H).

LC/MS (Methode 2, ESIpos): Rₜ = 2.34 min, m/z = 481 [M+H]⁺.

### Beispiel 56A

### 3-Ethyl-2,4-dioxo-5-(trifluormethyl)-1-(3,3,3-trifluorpropyl)-1,2,3,4-tetrahydrothieno[2,3-d]-pyrimidin-6-carbonsäure

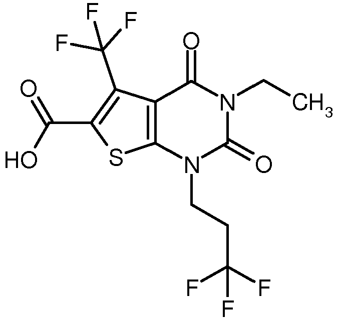

Analog zu dem unter Bsp. 55A beschriebenen Verfahren wurden aus 231 mg (0.540 mmol) der Verbindung aus Bsp. 39A 178 mg (82% d. Th.) der Titelverbindung erhalten.

¹H-NMR (400 MHz, DMSO-d₆, δ/ppm): 14.51 (br. s, 1H), 4.18 (t, 2H), 3.91 (quart, 2H), 2.87-2.75 (m, 2H), 1.13 (t, 3H).

LC/MS (Methode 1, ESIpos): Rₜ = 0.76 min, m/z = 405 [M+H]⁺.

### Beispiel 57A

### 5-(Difluormethyl)-2,4-dioxo-3-(2-phenylethyl)-1-(3,3,3-trifluorpropyl)-1,2,3,4-tetrahydrothieno-[2,3-d]pyrimidin-6-carbonsäure

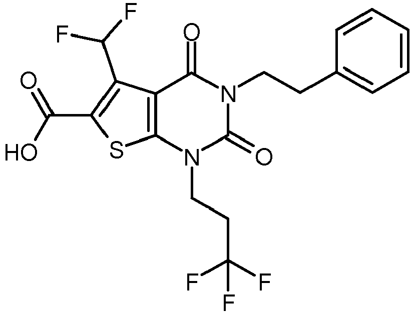

Eine Lösung von 800 mg (1.63 mmol) der Verbindung aus Bsp. 40A in 33 ml Ethanol wurde mit einer Lösung von 59 mg (2.45 mmol) Lithiumhydroxid in 17 ml Wasser versetzt und 3 h bei RT gerührt. Anschließend wurden alle flüchtigen Bestandteile am Rotationsverdampfer entfernt. Der verbliebene Rückstand wurde in Wasser aufgenommen und mit 2 ml 1 M Salzäure angesäuert. Es wurde dreimal mit je ca. 100 ml Dichlormethan extrahiert. Die vereinigten organischen Extrakte wurden einmal mit gesättigter Natriumchlorid-Lösung gewaschen, über wasserfreiem Magnesiumsulfat getrocknet, filtriert und eingeengt. Der verbliebene Rückstand wurde mit einem Gemisch aus 30 ml Pentan und 3 ml Diethylether bei RT verrührt. Nach Absaugen und Trocknen im Hochvakuum wurden 482 mg (63% d. Th.) der Titelverbindung erhalten.

¹H-NMR (400 MHz, DMSO-d₆, δ/ppm): 7.79 (t, 1H), 7.34-7.29 (m, 2H), 7.26-7.20 (m, 3H), 4.19 (t, 2H), 4.07 (m, 2H), 2.87-2.73 (m, 4H).

LC/MS (Methode 1, ESIpos): Rₜ = 1.01 min, m/z = 463 [M+H]⁺.

### Beispiel 58A

### 2,4-Dioxo-3-(2-phenylethyl)-1-(3,3,3-trifluorpropyl)-1,2,3,4-tetrahydrothieno[2,3-d]pyrimidin-6-carbonsäure

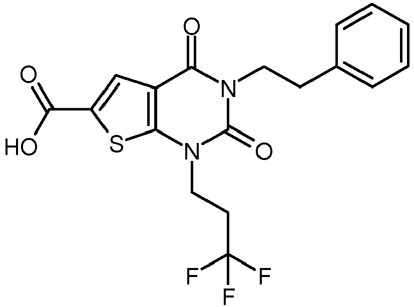

Eine Lösung von 800 mg (1.82 mmol) der Verbindung aus Bsp. 41A in 24 ml Ethanol wurde mit einer Lösung von 65 mg (2.73 mmol) Lithiumhydroxid in 8 ml Wasser versetzt. Anschließend wurden noch 2 ml THF hinzugefügt. Das Reaktionsgemisch wurde 3 h bei RT gerührt. Danach wurden alle flüchtigen Bestandteile am Rotationsverdampfer entfernt. Der verbliebene Rückstand wurde in ca. 200 ml Wasser aufgenommen und mit 3 ml 1 M Salzäure angesäuert. Dabei fiel das Produkt aus, das abgesaugt, mit Wasser neutral gewaschen und im Hochvakuum getrocknet wurde. Es wurden 639 mg (83% d. Th., 97% Reinheit) der Titelverbindung erhalten.

¹H-NMR (400 MHz, DMSO-d₆, δ/ppm): 13.63 (br. s, 1H), 7.79 (s, 1H), 7.32-7.28 (m, 2H), 7.24-7.20 (m, 3H), 4.16 (t, 2H), 4.08 (m, 2H), 2.85 (m, 2H), 2.78 (m, 2H).

LC/MS (Methode 1, ESIpos): Rₜ = 1.04 min, m/z = 413 [M+H]⁺.

### Beispiel 59A

### 5-Methyl-2,4-dioxo-1-(3,3,3-trifluorpropyl)-1,2,3,4-tetrahydrothieno[2,3-d]pyrimidin-6-carbonsäure

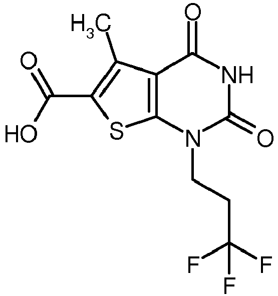

3.32 g (6.27 mmol) der Verbindung aus Bsp. 42A wurden in 130 ml Toluol gelöst und bei RT mit 5.02 g (37.6 mmol) festem Aluminiumtrichlorid versetzt. Anschließend wurde das Reaktionsgemisch 90 min bei 50°C gerührt. Nach dem Abkühlen auf RT wurden 70 ml Wasser und 300 ml Ethylacetat hinzugefügt. Nach Phasentrennung wurde die organische Phase nacheinander mit Wasser und gesättigter Natriumchlorid-Lösung gewaschen. Nach Trocknen über wasserfreiem Magnesiumsulfat wurde filtriert und eingedampft. Das Rohprodukt wurde bei RT mit 50 ml Pentan/Dichlormethan (20:1) verrührt. Nach Absaugen und Trocknen des Feststoffs im Hochvakuum wurden 1.93 g (87% d. Th., ca. 92% Reinheit) der Titelverbindung erhalten.

¹H-NMR (400 MHz, DMSO-d₆, δ/ppm): 13.40 (breit, 1H), 11.63 (s, 1H), 4.09 (t, 2H), 2.83-2.69 (m, 2H), 2.72 (s, 3H).

LC/MS (Methode 1, ESIpos): Rₜ = 0.65 min, m/z = 323 [M+H]⁺.

### Beispiel 60A

### 1-{[5-Methyl-2,4-dioxo-1-(3,3,3-trifluorpropyl)-1,2,3,4-tetrahydrothieno[2,3-d]pyrimidin-6-yl]-carbonyl}piperidin-4-ylacetat

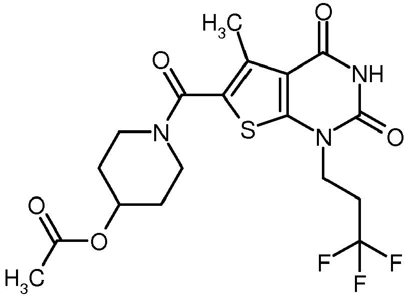

1.93 g (6.0 mmol) der Verbindung aus Bsp. 59A wurden in 40 ml DMF gelöst und nacheinander mit 2.74 g (7.20 mmol) HATU, 1.29 g (7.20 mmol) Piperidin-4-ylacetat-Hydrochlorid [Lit.: T. Kikuchi et al., J. Med. Chem. 2005, 48 (7), 2577-2583] und 3.1 ml (18.0 mmol) *N*,*N*-Diisopropylethylamin versetzt. Nachdem das Reaktionsgemisch ca. 16 h bei RT gerührt worden war, wurde es auf Wasser gegossen, wobei das Produkt ausfiel. Dieses wurde abgesaugt, mit wenig Wasser nachgewaschen und im Hochvakuum getrocknet. Nach Verrühren mit Diethylether wurden 2.35 g (80% d. Th., ca. 92% Reinheit) der Titelverbindung erhalten.

¹H-NMR (400 MHz, CDCl₃, δ/ppm): 8.38 (s, 1H), 5.04 (m, 1H), 4.15 (t, 2H), 3.88-3.80 (m, 2H), 3.55-3.48 (m, 2H), 2.71-2.59 (m, 2H), 2.49 (s, 3H), 2.09 (s, 3H), 1.98-1.91 (m, 2H), 1.77-1.69 (m, 2H).

LC/MS (Methode 1, ESIpos): Rₜ = 0.81 min, m/z = 448 [M+H]⁺.

### Beispiel 61A

### 3-Ethyl-5-methyl-2,4-dioxo-1,2,3,4-tetrahydrothieno[2,3-d]pyrimidin-6-carbonsäure

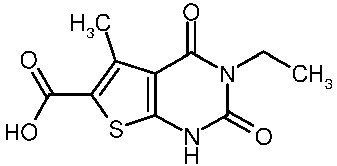

Eine Lösung von 30.0 g (96.7 mmol) der Verbindung aus Bsp. 12A in 300 ml Dichlormethan wurde mit 149 ml Trifluoressigsäure versetzt und 12 h bei RT gerührt. Danach wurde das Reaktionsgemisch am Rotationsverdampfer zur Trockene eingedampft. Der verbliebene Rückstand wurde in Diisopropylether verrührt, abgesaugt und der Feststoff im Hochvakuum getrocknet. Es wurden 22.80 g (92% d. Th.) der Titelverbindung erhalten.

LC/MS (Methode 1, ESIpos): Rₜ = 0.61 min, m/z = 255 [M+H]⁺.

### Beispiel 62A

### 1-[(3-Ethyl-5-methyl-2,4-dioxo-1,2,3,4-tetrahydrothieno[2,3-d]pyrimidin-6-yl)carbonyl]piperidin-4-ylacetat

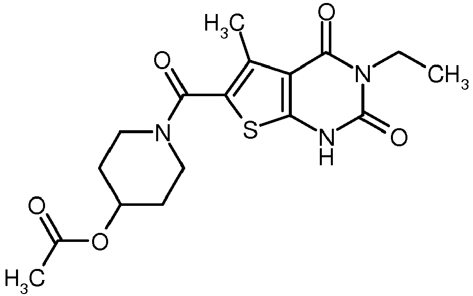

19.50 g (76.7 mmol) der Verbindung aus Bsp. 61A und 15.16 g (84.4 mmol) Piperidin-4-ylacetat-Hydrochlorid [Lit.: T. Kikuchi et al., J. Med. Chem. 2005, 48 (7), 2577-2583] wurden in 390 ml DMF gelöst und bei 0°C nacheinander mit 43.7 g (115 mmol) HATU und 47 ml (268 mmol) *N,N-*Diisopropylethylamin versetzt. Nachdem das Reaktionsgemisch ca. 16 h bei RT gerührt worden war, wurde es mit ca. 400 ml Ethylacetat verdünnt und nacheinander mit Wasser, 1 M Salzsäure, Wasser und gesättigter wässriger Natriumchlorid-Lösung gewaschen. Nach Trocknen über wasserfreiem Magnesiumsulfat wurde filtriert und anschließend zur Trockene eingedampft. Der erhaltene Rückstand wurde mittels Saugfiltration über Kieselgel mit einem Cyclohexan/Ethylacetat-Gradienten gereinigt. Nach Eindampfen der Produktfraktion und Trocknen des Rückstands im Hochvakuum wurden 8.80 g (30% d. Th.) der Titelverbindung erhalten.

¹H-NMR (400 MHz, DMSO-d₆, δ/ppm): 12.23 (s, 1H), 4.90 (m, 1H), 3.86 (quart, 2H), 3.77-3.69 (m, 2H), 3.41-3.33 (m, 2H, teilweise überdeckt vom Wassersignal), 2.35 (s, 3H), 2.02 (s, 3H), 1.90-1.83 (m, 2H), 1.58-1.49 (m, 2H), 1.11 (t, 3H).

LC/MS (Methode 1, ESIpos): Rₜ = 0.72 min, m/z = 380 [M+H]⁺.

### Beispiel 63A

### 1-({5-Methyl-3-[2-(2-methylphenyl)ethyl]-2,4-dioxo-1-(3,3,3-trifluorpropyl)-1,2,3,4-tetrahydro-thieno[2,3-d]pyrimidin-6-yl}carbonyl)piperidin-4-ylacetat

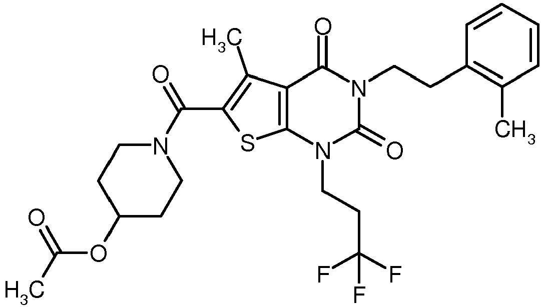

70 mg (0.156 mmol) der Verbindung aus Bsp. 60A und 31.5 µl (0.235 mmol) 2-(2-Methylphenyl)-ethylalkohol wurden in 2 ml wasserfreiem THF gelöst und nacheinander mit 62 mg (0.235 mmol) Triphenylphosphin und 46.5 µl (0.235 mmol) Diisopropylazodicarboxylat (DIAD) versetzt. Nach ca. 16 h Rühren bei RT wurde das Reaktionsgemisch in zwei Portionen mittels präparativer HPLC in seine Komponenten aufgetrennt (Methode 5). Die Produktfraktionen wurden vereinigt, eingedampft und der Rückstand im Hochvakuum getrocknet. Es wurden 66 mg (72% d. Th.) der Titelverbindung erhalten.

¹H-NMR (400 MHz, CDCl₃, δ/ppm): 7.22-7.11 (m, 4H), 5.04 (m, 1H), 4.18-4.13 (m, 4H), 3.89-3.81 (m, 2H), 3.55-3.48 (m, 2H), 2.95 (m, 2H), 2.64-2.55 (m, 2H), 2.53 (s, 3H), 2.47 (s, 3H), 2.09 (s, 3H), 1.99-1.91 (m, 2H), 1.78-1.69 (m, 2H).

LC/MS (Methode 1, ESIpos): Rₜ = 1.24 min, m/z = 566 [M+H]⁺.

### Beispiel 64A

### 1-({3-[2-(3-Fluorphenyl)ethyl]-5-methyl-2,4-dioxo-1-(3,3,3-trifluorpropyl)-1,2,3,4-tetrahydro-thieno[2,3-d]pyrimidin-6-yl}carbonyl)piperidin-4-ylacetat

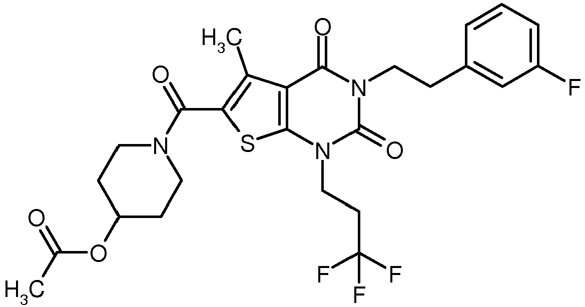

Analog zu dem unter Bsp. 63A beschriebenen Verfahren wurden aus 70 mg (0.156 mmol) der Verbindung aus Bsp. 60A und 29.3 µl (0.235 mmol) 2-(3-Fluorphenyl)ethylalkohol 65 mg (73% d. Th.) der Titelverbindung erhalten.

¹H-NMR (400 MHz, CDCl₃, δ/ppm): 7.07 (d, 1H), 7.00 (d, 1H), 6.92 (dt, 1H), 5.04 (m, 1H), 4.23-4.13 (m, 4H), 3.89-3.81 (m, 2H), 3.56-3.48 (m, 2H), 2.94 (m, 2H), 2.66-2.55 (m, 2H), 2.51 (s, 3H), 2.09 (s, 3H), 1.99-1.91 (m, 2H), 1.78-1.69 (m, 2H).

LC/MS (Methode 1, ESIpos): Rₜ = 1.19 min, m/z = 570 [M+H]⁺.

### Beispiel 65A

### 1-({3-[2-(3-Chlorphenyl)ethyl]-5-methyl-2,4-dioxo-1-(3,3,3-trifluorpropyl)-1,2,3,4-tetrahydro-thieno[2,3-d]pyrimidin-6-yl}carbonyl)piperidin-4-ylacetat

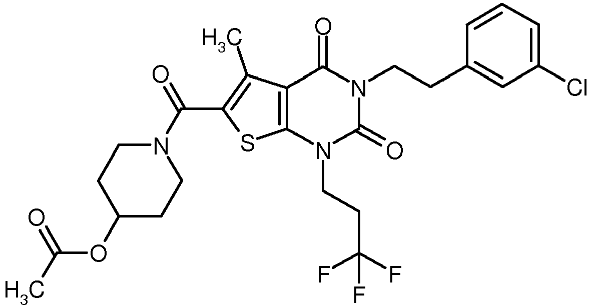

Analog zu dem unter Bsp. 63A beschriebenen Verfahren wurden aus 70 mg (0.156 mmol) der Verbindung aus Bsp. 60A und 37 mg (0.235 mmol) 2-(3-Chlorphenyl)ethylalkohol 74 mg (79% d. Th.) der Titelverbindung erhalten.

¹H-NMR (400 MHz, CDCl₃, δ/ppm): 7.28-7.17 (m, 4H, teilweise überdeckt vom CHCl₃-Signal), 5.04 (m, 1H), 4.22-4.13 (m, 4H), 3.88-3.81 (m, 2H), 3.56-3.48 (m, 2H), 2.92 (m, 2H), 2.66-2.55 (m, 2H), 2.52 (s, 3H), 2.09 (s, 3H), 1.99-1.91 (m, 2H), 1.78-1.69 (m, 2H).

LC/MS (Methode 1, ESIpos): Rₜ = 1.24 min, m/z = 586/588 [M+H]⁺.

### Beispiel 66A

### 1-({5-Methyl-3-[2-(3-methylphenyl)ethyl]-2,4-dioxo-1-(3,3,3-trifluorpropyl)-1,2,3,4-tetrahydro-thieno[2,3-d]pyrimidin-6-yl}carbonyl)piperidin-4-ylacetat

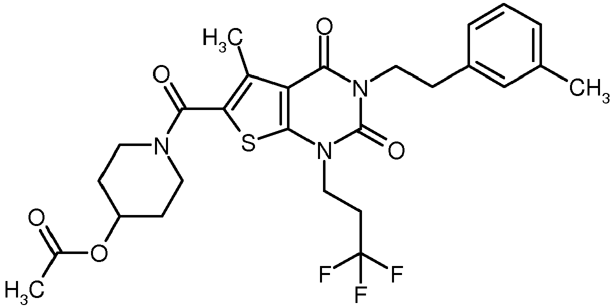

Analog zu dem unter Bsp. 63A beschriebenen Verfahren wurden aus 70 mg (0.156 mmol) der Verbindung aus Bsp. 60A und 37 mg (0.235 mmol) 2-(3-Methylphenyl)ethylalkohol 61 mg (66% d. Th.) der Titelverbindung erhalten.

¹H-NMR (400 MHz, CDCl₃, δ/ppm): 7.20 (t, 1H), 7.13 (s, 1H), 7.10 (d, 1H), 7.05 (d, 1H), 5.04 (m, 1H), 4.21-4.14 (m, 4H), 3.89-3.81 (m, 2H), 3.56-3.48 (m, 2H), 2.89 (m, 2H), 2.67-2.55 (m, 2H), 2.53 (s, 3H), 2.34 (s, 3H), 2.09 (s, 3H), 1.99-1.91 (m, 2H), 1.78-1.69 (m, 2H).

LC/MS (Methode 1, ESIpos): Rₜ = 1.23 min, m/z = 566 [M+H]⁺.

### Beispiel 67A

### 1-({3-[2-(4-Fluorphenyl)ethyl]-5-methyl-2,4-dioxo-1-(3,3,3-trifluorpropyl)-1,2,3,4-tetrahydro-thieno[2,3-d]pyrimidin-6-yl}carbonyl)piperidin-4-ylacetat

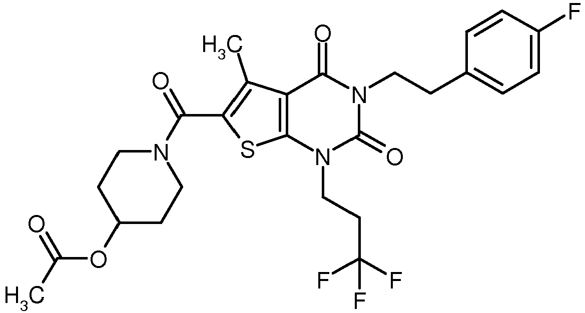

Analog zu dem unter Bsp. 63A beschriebenen Verfahren wurden aus 42 mg (0.094 mmol) der Verbindung aus Bsp. 60A und 20 mg (0.141 mmol) 2-(4-Fluorphenyl)ethylalkohol 39 mg (72% d. Th.) der Titelverbindung erhalten.

¹H-NMR (400 MHz, CDCl₃, δ/ppm): 7.27-7.23 (m, 2H, teilweise überdeckt vom CHCl₃-Signal), 6.99 (t, 2H), 5.04 (m, 1H), 4.20-4.13 (m, 4H), 3.88-3.81 (m, 2H), 3.56-3.48 (m, 2H), 2.91 (m, 2H), 2.66-2.55 (m, 2H), 2.51 (s, 3H), 2.09 (s, 3H), 1.99-1.91 (m, 2H), 1.77-1.69 (m, 2H).

LC/MS (Methode 1, ESIpos): Rₜ = 1.20 min, m/z = 570 [M+H]⁺.

### Beispiel 68A

### 1-({3-[2-(4-Chlorphenyl)ethyl]-5-methyl-2,4-dioxo-1-(3,3,3-trifluorpropyl)-1,2,3,4-tetrahydro-thieno[2,3-d]pyrimidin-6-yl}carbonyl)piperidin-4-ylacetat

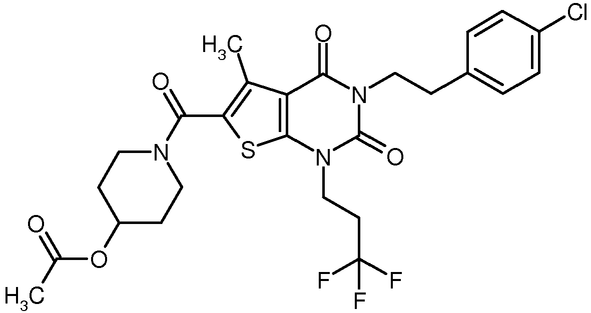

Analog zu dem unter Bsp. 63A beschriebenen Verfahren wurden aus 70 mg (0.156 mmol) der Verbindung aus Bsp. 60A und 37 mg (0.235 mmol) 2-(4-Chlorphenyl)ethylalkohol 78 mg (85% d. Th.) der Titelverbindung erhalten.

¹H-NMR (400 MHz, CDCl₃, δ/ppm): 7.27 (d, 2H), 7.22 (d, 2H), 5.04 (m, 1H), 4.20-4.13 (m, 4H), 3.89-3.80 (m, 2H), 3.56-3.48 (m, 2H), 2.91 (m, 2H), 2.65-2.53 (m, 2H), 2.51 (s, 3H), 2.09 (s, 3H), 1.99-1.91 (m, 2H), 1.78-1.69 (m, 2H).

LC/MS (Methode 1, ESIpos): Rₜ = 1.23 min, m/z = 586/588 [M+H]⁺.

### Beispiel 69A

### 1-({3-[2-(4-Methoxyphenyl)ethyl]-5-methyl-2,4-dioxo-1-(3,3,3-trifluorpropyl)-1,2,3,4-tetrahydro-thieno[2,3-d]pyrimidin-6-yl}carbonyl)piperidin-4-ylacetat

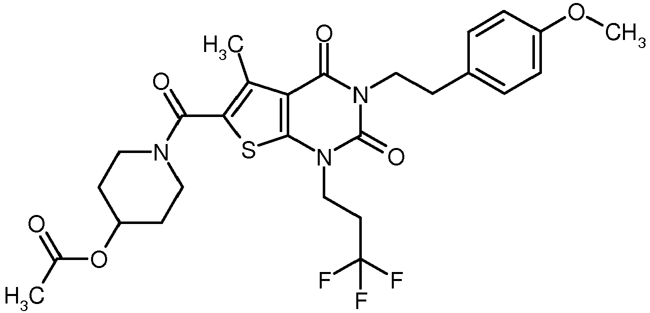

Analog zu dem unter Bsp. 63A beschriebenen Verfahren wurden aus 70 mg (0.156 mmol) der Verbindung aus Bsp. 60A und 36 mg (0.235 mmol) 2-(4-Methoxyphenyl)ethylalkohol 72 mg (79% d. Th.) der Titelverbindung erhalten.

¹H-NMR (400 MHz, CDCl₃, δ/ppm): 7.20 (d, 2H), 6.84 (d, 2H), 5.04 (m, 1H), 4.19-4.13 (m, 4H), 3.89-3.80 (m, 2H), 3.79 (s, 3H), 3.55-3.48 (m, 2H), 2.88 (m, 2H), 2.64-2.55 (m, 2H), 2.52 (s, 3H), 2.09 (s, 3H), 1.99-1.91 (m, 2H), 1.77-1.69 (m, 2H).

LC/MS (Methode 1, ESIpos): Rₜ = 1.14 min, m/z = 582 [M+H]⁺.

### Beispiel 70A

### 1-({5-Methyl-2,4-dioxo-3-[2-(pyrazin-2-yl)ethyl]-1-(3,3,3-trifluorpropyl)-1,2,3,4-tetrahydrothieno-[2,3-d]pyrimidin-6-yl}carbonyl)piperidin-4-ylacetat

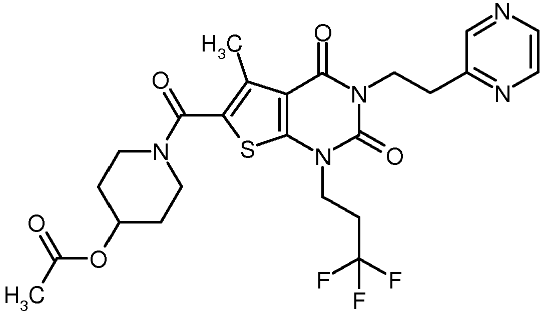

Analog zu dem unter Bsp. 63A beschriebenen Verfahren wurden aus 70 mg (0.156 mmol) der Verbindung aus Bsp. 60A und 29 mg (0.235 mmol) 2-(2-Hydroxyethyl)pyrazin [kommerziell erhältlich; Lit. z.B.: US-Patent US 5 344 830, Beispiel 18 / Stufe A] zwei Fraktionen der Titelverbindung erhalten: 14 mg (16% d. Th., rein) sowie 93 mg eines Gemisches der Titelverbindung mit Triphenylphosphinoxid. Abweichend zum Verfahren in Bsp. 63A erfolgte die präparative HPLC-Reinigung hier nach Methode 7.

¹H-NMR (400 MHz, CDCl₃, δ/ppm): 8.51-8.42 (m, 3H), 5.04 (m, 1H), 4.41 (t, 2H), 4.13 (t, 2H), 3.89-3.80 (m, 2H), 3.55-3.47 (m, 2H), 3.18 (t, 2H), 2.65-2.54 (m, 2H), 2.48 (s, 3H), 2.09 (s, 3H), 1.99-1.91 (m, 2H), 1.78-1.68 (m, 2H).

LC/MS (Methode 1, ESIpos): Rₜ = 0.93 min, m/z = 554 [M+H]⁺.

### Beispiel 71A

### 1-({5-Methyl-2,4-dioxo-3-[2-phenylpropyl]-1-(3,3,3-trifluorpropyl)-1,2,3,4-tetrahydrothieno-[2,3-d]pyrimidin-6-yl}carbonyl)piperidin-4-ylacetat (Racemat)

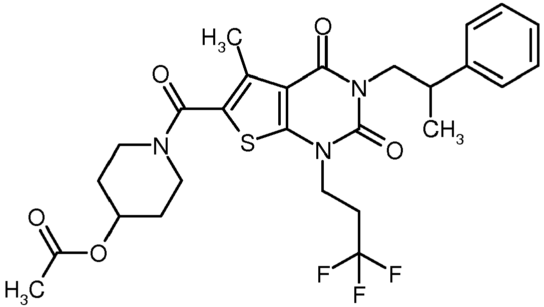

Analog zu dem unter Bsp. 63A beschriebenen Verfahren wurden aus 200 mg (0.447 mmol) der Verbindung aus Bsp. 60A und 91 mg (0.670 mmol) racemischem 2-Phenylpropan-1-ol 238 mg (94% d. Th.) der Titelverbindung erhalten.

¹H-NMR (400 MHz, DMSO-d₆, δ/ppm): 7.34-7.24 (m, 4H), 7.22-7.17 (m, 1H), 4.92 (m, 1H), 4.12-4.05 (m, 3H), 3.94 (m, 1H), 3.79-3.71 (m, 2H), 3.43-3.36 (m, 2H), 3.27-3.19 (m, 1H), 2.78-2.66 (m, 2H), 2.38 (s, 3H), 2.03 (s, 3H), 1.92-1.85 (m, 2H), 1.60-1.51 (m, 2H), 1.19 (d, 3H).

LC/MS (Methode 1, ESIpos): Rₜ = 1.20 min, m/z = 566 [M+H]⁺.

### Beispiel 72A

### 1-{[3-(2-Methoxy-2-phenylethyl)-5-methyl-2,4-dioxo-1-(3,3,3-trifluorpropyl)-1,2,3,4-tetrahydro-thieno[2,3-d]pyrimidin-6-yl]carbonyl}piperidin-4-ylacetat (Racemat)

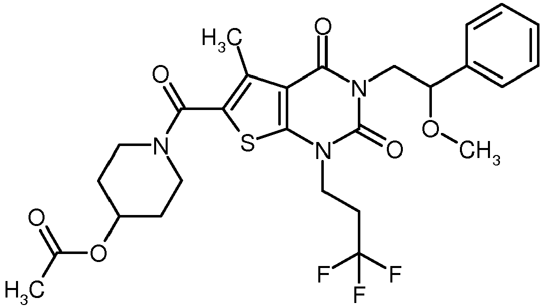

Analog zu dem unter Bsp. 63A beschriebenen Verfahren wurden aus 200 mg (0.447 mmol) der Verbindung aus Bsp. 60A und 102 mg (0.670 mmol) racemischem 2-Methoxy-2-phenylethanol 256 mg (93% d. Th., 95% Reinheit) der Titelverbindung erhalten.

¹H-NMR (400 MHz, DMSO-d₆, δ/ppm): 7.41-7.36 (m, 2H), 7.34-7.30 (m, 3H), 4.92 (m, 1H), 4.57 (dd, 1H), 4.35 (dd, 1H), 4.19-4.04 (m, 2H), 3.81 (dd, 1H), 3.79-3.72 (m, 2H), 3.44-3.37 (m, 2H), 3.07 (s, 3H), 2.79-2.67 (m, 2H), 2.39 (s, 3H), 2.03 (s, 3H), 1.93-1.84 (m, 2H), 1.61-1.52 (m, 2H).

LC/MS (Methode 1, ESIpos): Rₜ = 1.15 min, m/z = 582 [M+H]⁺.

### Beispiel 73A

### 1-({5-Methyl-2,4-dioxo-3-[(1-phenylcyclopropyl)methyl]-1-(3,3,3-trifluorpropyl)-1,2,3,4-tetra-hydrothieno[2,3-d]pyrimidin-6-yl}carbonyl)piperidin-4-ylacetat

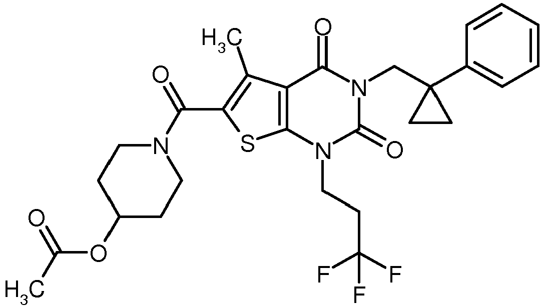

Analog zu dem unter Bsp. 63A beschriebenen Verfahren wurden aus 100 mg (0.223 mmol) der Verbindung aus Bsp. 60A und 50 mg (0.335 mmol) (1-Phenylcyclopropyl)methanol [Lit.: L.K. Sydnes, P.F.F. Pereira, M. Sandberg, H.H. Oevreboe, J. Chem. Res. Miniprint 2001 (4), 464-474] 57 mg (42% d. Th., 96% Reinheit) der Titelverbindung erhalten.

¹H-NMR (400 MHz, DMSO-d₆, δ/ppm): 7.26-7.19 (m, 4H), 7.17-7.13 (m, 1H), 4.91 (m, 1H), 4.16 (s, 2H), 4.00 (t, 2H), 3.78-3.70 (m, 2H), 3.43-3.35 (m, 2H), 2.63-2.51 (m, 2H, teilweise überdeckt vom DMSO-Signal), 2.32 (s, 3H), 2.03 (s, 3H), 1.91-1.85 (m, 2H), 1.60-1.51 (m, 2H), 0.95 (m, 2H), 0.72 (m, 2H).

LC/MS (Methode 1, ESIpos): Rₜ = 1.22 min, m/z = 578 [M+H]⁺.

### Beispiel 74A

### 1-{[5-Methyl-3-(2-methyl-2-phenylpropyl)-2,4-dioxo-1-(3,3,3-trifluorpropyl)-1,2,3,4-tetrahydro-thieno[2,3-d]pyrimidin-6-yl]carbonyl}piperidin-4-ylacetat

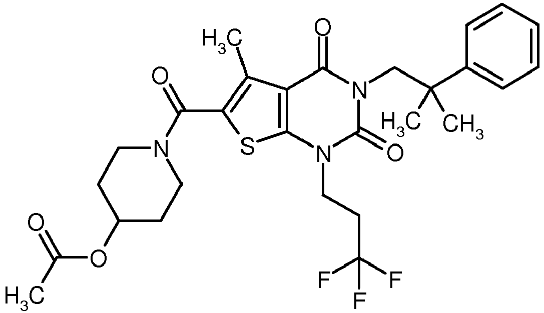

Analog zu dem unter Bsp. 63A beschriebenen Verfahren wurden aus 150 mg (0.335 mmol) der Verbindung aus Bsp. 60A und 76 mg (0.503 mmol) 2-Methyl-2-phenylpropan-1-ol [Lit.: D. Coletta, B. di Giacomo, B. Natalini, M.-H. Ni, R. Pellicciari, Farmaco 1999, 54 (9), 600-610] 104 mg (51% d. Th., 95% Reinheit) der Titelverbindung erhalten.

¹H-NMR (400 MHz, DMSO-d₆, δ/ppm): 7.43 (d, 2H), 7.32 (t, 2H), 7.21 (t, 1H), 4.92 (m, 1H), 4.10-4.05 (m, 4H), 3.79-3.71 (m, 2H), 3.44-3.36 (m, 2H), 2.77-2.65 (m, 2H), 2.35 (s, 3H), 2.03 (s, 3H), 1.92-1.85 (m, 2H), 1.61-1.52 (m, 2H), 1.28 (s, 6H).

LC/MS (Methode 1, ESIpos): Rₜ = 1.29 min, m/z = 580 [M+H]⁺.

### Beispiel 75A

### 1-({5-Methyl-2,4-dioxo-3-[2-(pyridin-2-yl)ethyl]-1-(3,3,3-trifluorpropyl)-1,2,3,4-tetrahydrothieno-[2,3-d]pyrimidin-6-yl}carbonyl)piperidin-4-ylacetat (Ameisensäure-Salz)

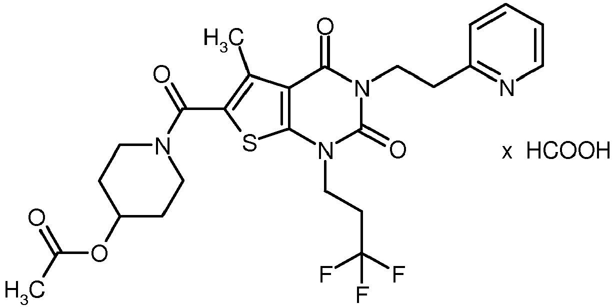

Analog zu dem unter Bsp. 63A beschriebenen Verfahren wurden aus 70 mg (0.156 mmol) der Verbindung aus Bsp. 60A und 29 mg (0.235 mmol) 2-(2-Hydroxyethyl)pyridin 112 mg (95% d. Th., 80% Reinheit) der Titelverbindung erhalten.

¹H-NMR (400 MHz, DMSO-d₆, δ/ppm): 8.88 (s, 1H), 8.48 (d, 1H), 7.71 (dt, 1H), 7.28 (d, 1H), 7.23 (dd, 1H), 4.92 (m, 1H), 4.22 (t, 1H), 4.11 (t, 1H), 3.79-3.71 (m, 2H), 3.44-3.36 (m, 2H), 2.99 (t, 2H), 2.81-2.69 (m, 2H), 2.39 (s, 3H), 2.03 (s, 3H), 1.92-1.85 (m, 2H), 1.61-1.51 (m, 2H).

LC/MS (Methode 1, ESIpos): Rₜ = 0.79 min, m/z = 553 [M+H]⁺.

### Beispiel 76A

### 1-({5-Methyl-2,4-dioxo-3-[2-(pyridin-4-yl)ethyl]-1-(3,3,3-trifluorpropyl)-1,2,3,4-tetrahydrothieno-[2,3-d]pyrimidin-6-yl}carbonyl)piperidin-4-ylacetat (Ameisensäure-Salz)

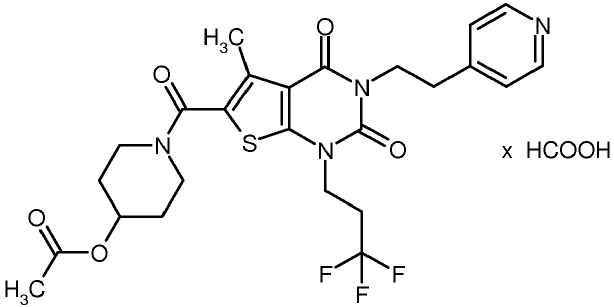

Analog zu dem unter Bsp. 63A beschriebenen Verfahren wurden aus 70 mg (0.156 mmol) der Verbindung aus Bsp. 60A und 29 mg (0.235 mmol) 4-(2-Hydroxyethyl)pyridin 54 mg (50% d. Th., 80% Reinheit) der Titelverbindung erhalten.

LC/MS (Methode 1, ESIpos): Rₜ = 0.76 min, m/z = 553 [M+H]⁺.

### Beispiel 77A

### 1-({3-[2-(1H-Imidazol-1-yl)ethyl]-5-methyl-2,4-dioxo-1-(3,3,3-trifluorpropyl)-1,2,3,4-tetrahydro-thieno[2,3-d]pyrimidin-6-yl}carbonyl)piperidin-4-ylacetat

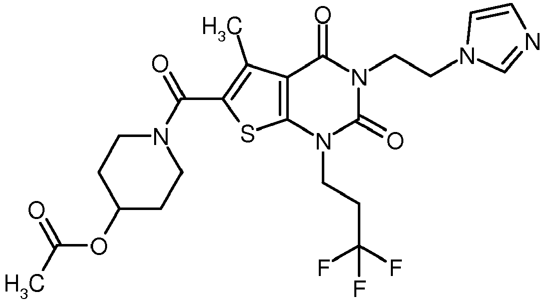

Eine Lösung von 100 mg (0.223 mmol) der Verbindung aus Bsp. 60A in 3 ml DMF wurde mit 182 mg (0.559 mmol) Cäsiumcarbonat versetzt und 10 min bei RT gerührt. Dann wurden 45 mg (0.268 mmol) 1-(2-Chlorethyl)-1*H*-imidazol-Hydrochlorid [Lit.: M.L. Burdeinyi, S.V. Popkov, M. V. Kharchevnikowa, Russ. Chem. Bull. 2009, 58 (5), 936-939] hinzugefügt. Nach Rühren über Nacht bei RT war der Umsatz nicht vollständig. Daher wurde das Gemisch noch 1 h auf 70°C erwärmt. Nach dem Abkühlen auf RT wurde das Reaktionsgemisch direkt mittels präparativer HPLC in seine Komponenten aufgetrennt (Methode 5). Die Produktfraktionen wurden vereinigt und eingeengt. Der Rückstand wurde in etwas Methanol gelöst und über eine Hydrogencarbonat-Kartusche gegeben (Fa. Polymerlabs, Stratospheres SPE, PL-HCO₃ MP SPE, Kapazität 0.9 mmol). Nach anschließendem Eindampfen und Trocknen des Rückstands im Hochvakuum wurden 106 mg (87% d. Th.) der Titelverbindung als freie Base erhalten.

¹H-NMR (400 MHz, DMSO-d₆, δ/ppm): 7.58 (s, 1H), 7.12 (s, 1H), 6.85 (s, 1H), 4.92 (m, 1H), 4.23-4.17 (m, 4H), 4.09 (t, 2H), 3.79-3.71 (m, 2H), 3.43-3.36 (m, 2H), 2.80-2.68 (m, 2H), 2.36 (s, 3H), 2.02 (s, 3H), 1.92-1.84 (m, 2H), 1.60-1.52 (m, 2H).

LC/MS (Methode 1, ESIpos): Rₜ = 0.67 min, m/z = 542 [M+H]⁺.

### Beispiel 78A

### 1-{[3-(But-3-in-1-yl)-5-methyl-2,4-dioxo-1-(3,3,3-trifluorpropyl)-1,2,3,4-tetrahydrothieno[2,3-d]-pyrimidin-6-yl]carbonyl}piperidin-4-ylacetat

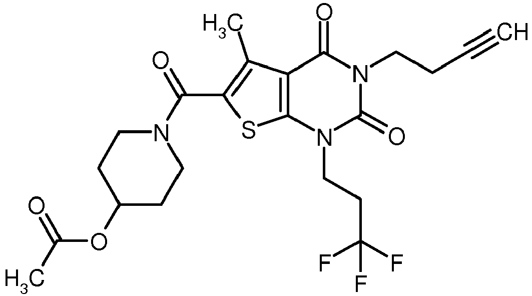

Eine Lösung von 100 mg (0.223 mmol) der Verbindung aus Bsp. 60A in 3 ml DMF wurde mit 80 mg (0.246 mmol) Cäsiumcarbonat versetzt und 10 min bei RT gerührt. Dann wurden 37 mg (0.268 mmol) 4-Brombutin hinzugefügt. Das Reaktionsgemisch wurde ca. 16 h bei RT gerührt und dann direkt mittels präparativer HPLC in seine Komponenten aufgetrennt (Methode 5). Die Produktfraktionen wurden vereinigt, eingeengt und der Rückstand im Hochvakuum getrocknet. Es wurden 90 mg (80% d. Th.) der Titelverbindung erhalten.

¹H-NMR (400 MHz, DMSO-d₆, δ/ppm): 4.92 (m, 1H), 4.13 (t, 2H), 4.01 (t, 2H), 3.79-3.71 (m, 2H), 3.43-3.36 (m, 2H), 2.88 (t, 1H), 2.85-2.73 (m, 2H), 2.49-2.44 (m, 2H), 2.39 (s, 3H), 2.02 (s, 3H), 1.92-1.84 (m, 2H), 1.60-1.51 (m, 2H).

LC/MS (Methode 1, ESIpos): Rₜ = 0.99 min, m/z = 500 [M+H]⁺.

### Beispiel 79A

### 1-{[3-(2-Methoxyethyl)-5-methyl-2,4-dioxo-1-(3,3,3-trifluorpropyl)-1,2,3,4-tetrahydrothieno-[2,3-d]pyrimidin-6-yl]carbonyl}piperidin-4-ylacetat

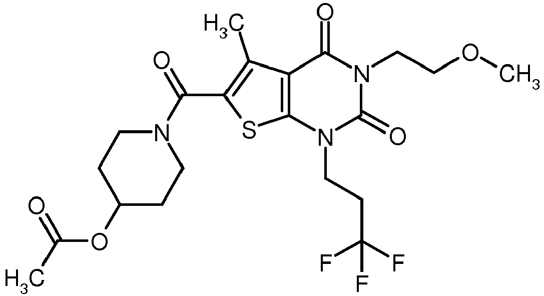

Analog zu dem unter Bsp. 78A beschriebenen Verfahren wurden aus 100 mg (0.223 mmol) der Verbindung aus Bsp. 60A und 37 mg (0.268 mmol) 2-Bromethyl-methylether 94 mg (83% d. Th.) der Titelverbindung erhalten.

¹H-NMR (400 MHz, DMSO-d₆, δ/ppm): 4.92 (m, 1H), 4.13 (t, 2H), 4.06 (t, 2H), 3.79-3.71 (m, 2H), 3.50 (t, 2H), 3.44-3.36 (m, 2H), 3.24 (s, 3H), 2.85-2.73 (m, 2H), 2.39 (s, 3H), 2.02 (s, 3H), 1.91-1.84 (m, 2H), 1.60-1.51 (m, 2H).

LC/MS (Methode 1, ESIpos): Rₜ = 0.93 min, m/z = 506 [M+H]⁺.

### Beispiel 80A

### 1-{[3-(2-Cyclopropylethyl)-5-methyl-2,4-dioxo-1-(3,3,3-trifluorpropyl)-1,2,3,4-tetrahydrothieno-[2,3-d]pyrimidin-6-yl]carbonyl}piperidin-4-ylacetat

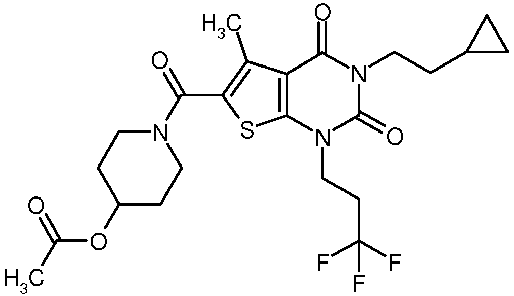

Analog zu dem unter Bsp. 63A beschriebenen Verfahren wurden aus 100 mg (0.223 mmol) der Verbindung aus Bsp. 60A und 29 mg (0.335 mmol) 2-Cyclopropylethanol 52 mg (45% d. Th.) der Titelverbindung erhalten.

¹H-NMR (400 MHz, DMSO-d₆, δ/ppm): 4.91 (m, 1H), 4.12 (t, 2H), 3.95 (t, 2H), 3.78-3.71 (m, 2H), 3.43-3.34 (m, 2H, teilweise überdeckt vom Wassersignal), 2.85-2.73 (m, 2H), 2.39 (s, 3H), 2.02 (s, 3H), 1.91-1.84 (m, 2H), 1.60-1.51 (m, 2H), 1.44 (quart, 2H), 0.72-0.65 (m, 1H), 0.41-0.37 (m, 2H), 0.02 (m, 2H, teilweise überdeckt vom TMS-Signal).

LC/MS (Methode 1, ESIpos): Rₜ = 1.12 min, m/z = 516 [M+H]⁺.

### Beispiel 81A

### 1-{[3-Isobutyl-5-methyl-2,4-dioxo-1-(3,3,3-trifluorpropyl)-1,2,3,4-tetrahydrothieno[2,3-d]-pyrimidin-6-yl]carbonyl}piperidin-4-ylacetat

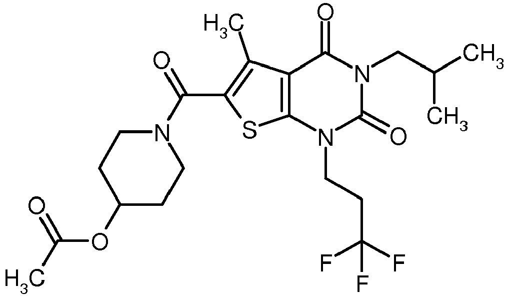

Analog zu dem unter Bsp. 63A beschriebenen Verfahren wurden aus 100 mg (0.223 mmol) der Verbindung aus Bsp. 60A und 25 mg (0.335 mmol) 2-Methylpropanol 95 mg (84% d. Th.) der Titelverbindung erhalten.

¹H-NMR (400 MHz, DMSO-d₆, δ/ppm): 4.92 (m, 1H), 4.13 (t, 2H), 3.79-3.70 (m, 4H), 3.44-3.36 (m, 2H), 2.85-2.73 (m, 2H), 2.39 (s, 3H), 2.09-1.99 (m, 1H), 2.02 (s, 3H), 1.92-1.84 (m, 2H), 1.60-1.51 (m, 2H), 0.86 (d, 6H).

LC/MS (Methode 1, ESIpos): Rₜ = 1.11 min, m/z = 504 [M+H]⁺.

### Beispiel 82A

### 1-{[3-(2-Methoxypropyl)-5-methyl-2,4-dioxo-1-(3,3,3-trifluorpropyl)-1,2,3,4-tetrahydrothieno-[2,3-d]pyrimidin-6-yl]carbonyl}piperidin-4-ylacetat (Racemat)

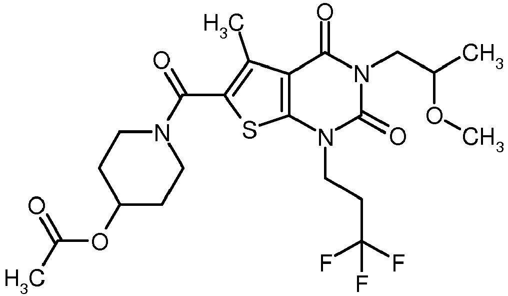

Analog zu dem unter Bsp. 63A beschriebenen Verfahren wurden aus 80 mg (0.179 mmol) der Verbindung aus Bsp. 60A und 24 mg (0.268 mmol) racemischem 2-Methoxypropanol 53 mg (57% d. Th.) der Titelverbindung erhalten. Abweichend zu dem zuvor beschriebenen Verfahren wurde hier das nach der präparativen HPLC erhaltene Produkt noch einmal über Kieselgel chromatographiert (Laufmittel: Cyclohexan/Ethylacetat 2:1).

¹H-NMR (400 MHz, DMSO-d₆, δ/ppm): 4.92 (m, 1H), 4.13 (quart, 2H), 3.79-3.72 (m, 4H), 3.64 (m, 1H), 3.44-3.36 (m, 2H), 3.22 (s, 3H), 2.85-2.73 (m, 2H), 2.39 (s, 3H), 2.02 (s, 3H), 1.92-1.85 (m, 2H), 1.60-1.51 (m, 2H), 1.06 (d, 3H).

LC/MS (Methode 1, ESIpos): Rₜ = 0.99 min, m/z = 520 [M+H]⁺.

### Beispiel 83A

### 1-({3-Ethyl-5-methyl-2,4-dioxo-1-[2-(trifluormethoxy)ethyl]-1,2,3,4-tetrahydrothieno[2,3-d]-pyrimidin-6-yl}carbonyl)piperidin-4-ylacetat

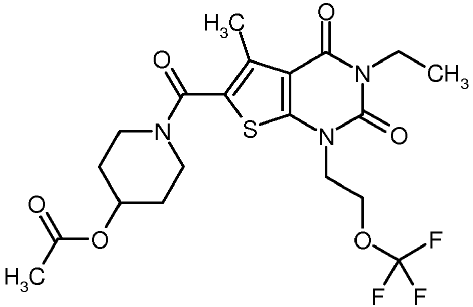

Eine Lösung von 75 mg (0.198 mmol) der Verbindung aus Bsp. 62A in 2 ml DMF wurde mit 97 mg (0.296 mmol) Cäsiumcarbonat versetzt und 20 min bei RT gerührt. Dann wurden 57 mg (0.296 mmol) 1-Brom-2-(trifluormethoxy)ethan [kommerziell erhältlich; Lit.: P.E. Aldrich, W.A. Sheppard, J. Org. Chem. 1964, 29 (1), 11-15] hinzugefügt. Das Reaktionsgemisch wurde 15 min in einem Mikrowellenofen (Biotage Initiator Sixty) auf 100°C erhitzt. Nach dem Abkühlen auf RT wurde das Gemisch direkt mittels präparativer HPLC in seine Komponenten aufgetrennt (Methode 13). Die Produktfraktionen wurden vereinigt, eingeengt und der Rückstand im Hochvakuum getrocknet. Es wurden 66 mg (68% d. Th.) der Titelverbindung erhalten.

¹H-NMR (400 MHz, DMSO-d₆, δ/ppm): 4.91 (m, 1H), 4.42 (t, 2H), 4.22 (t, 2H), 3.91 (quart, 2H), 3.78-3.70 (m, 2H), 3.43-3.38 (m, 2H, teilweise überdeckt vom Wassersignal), 2.39 (s, 3H), 2.02 (s, 3H), 1.91-1.84 (m, 2H), 1.59-1.50 (m, 2H), 1.12 (t, 3H).

LC/MS (Methode 1, ESIpos): Rₜ = 1.01 min, m/z = 492 [M+H]⁺.

### Beispiel 84A

### 1-[(3-Ethyl-5-methyl-2,4-dioxo-1-{2-[(trifluormethyl)sulfanyl]ethyl}-1,2,3,4-tetrahydrothieno-[2,3-d]pyrimidin-6-yl)carbonyl]piperidin-4-ylacetat

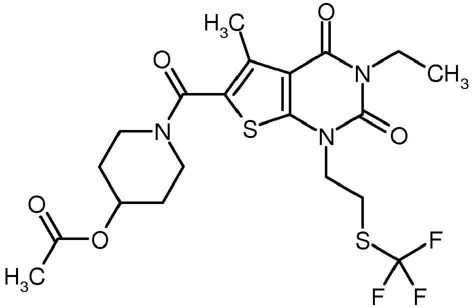

Analog zu dem unter Bsp. 83A beschriebenen Verfahren wurden aus 75 mg (0.198 mmol) der Verbindung aus Bsp. 62A und 62 mg (0.296 mmol) 2-Bromethyl-trifluormethylsulfid 74 mg (74% d. Th.) der Titelverbindung erhalten.

¹H-NMR (400 MHz, DMSO-d₆, δ/ppm): 4.91 (m, 1H), 4.16 (t, 2H), 3.91 (quart, 2H), 3.78-3.71 (m, 2H), 3.43-3.34 (m, 4H, teilweise überdeckt vom Wassersignal), 2.39 (s, 3H), 2.02 (s, 3H), 1.92-1.84 (m, 2H), 1.59-1.51 (m, 2H), 1.12 (t, 3H).

LC/MS (Methode 1, ESIpos): Rₜ = 1.07 min, m/z = 508 [M+H]⁺.

### Beispiel 85A

### 1-({3-Ethyl-5-methyl-2,4-dioxo-1-[2-(trifluormethyl)prop-2-en-1-yl]-1,2,3,4-tetrahydrothieno-[2,3-d]pyrimidin-6-yl}carbonyl)piperidin-4-ylacetat

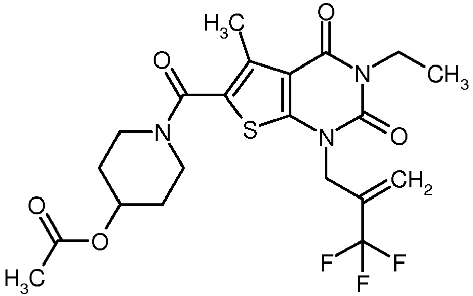

Analog zu dem unter Bsp. 83A beschriebenen Verfahren wurden aus 75 mg (0.198 mmol) der Verbindung aus Bsp. 62A und 56 mg (0.296 mmol) 2-(Brommethyl)-3,3,3-trifluorpropen 77 mg (73% d. Th., 91% Reinheit) der Titelverbindung erhalten.

¹H-NMR (400 MHz, DMSO-d₆, δ/ppm): 6.02 (s, 1H), 5.83 (s, 1H), 4.91 (m, 1H), 4.79 (s, 2H), 3.92 (quart, 2H), 3.77-3.69 (m, 2H), 3.43-3.37 (m, 2H, teilweise überdeckt vom Wassersignal), 2.40 (s, 3H), 2.02 (s, 3H), 1.91-1.83 (m, 2H), 1.59-1.50 (m, 2H), 1.13 (t, 3H).

LC/MS (Methode 1, ESIpos): Rₜ = 1.03 min, m/z = 488 [M+H]⁺.

### Beispiel 86A

### 1-({1-[(2,2-Difluorcyclopropyl)methyl]-3-ethyl-5-methyl-2,4-dioxo-1,2,3,4-tetrahydrothieno-[2,3-d]pyrimidin-6-yl}carbonyl)piperidin-4-ylacetat (Racemat)

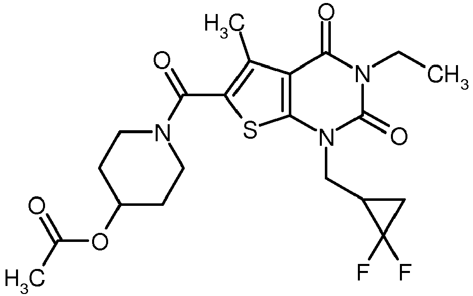

Analog zu dem unter Bsp. 83A beschriebenen Verfahren wurden aus 75 mg (0.198 mmol) der Verbindung aus Bsp. 62A und 51 mg (0.296 mmol) racemischem 2-(Bronunethyl)-1,1-difluorcyclo-propan 68 mg (72% d. Th.) der Titelverbindung erhalten.

¹H-NMR (400 MHz, DMSO-d₆, δ/ppm): 4.91 (m, 1H), 4.19-4.11 (m, 1H), 4.00-3.90 (m, 1H), 3.91 (quart, 2H), 3.79-3.71 (m, 2H), 3.43-3.36 (m, 2H, teilweise überdeckt vom Wassersignal), 2.40 (s, 3H), 2.29-2.17 (m, 1H), 2.02 (s, 3H), 1.91-1.84 (m, 2H), 1.77-1.67 (m, 1H), 1.60-1.45 (m, 3H), 1.13 (t, 3H).

LC/MS (Methode 1, ESIpos): Rₜ = 1.08 min, m/z = 470 [M+H]⁺.

### Beispiel 87A

### 2-tert.-Butyl-4-ethyl-5-{[(2,2-difluor-2-phenylethyl)carbamoyl]amino}-3-methylthiophen-2,4-dicarboxylat

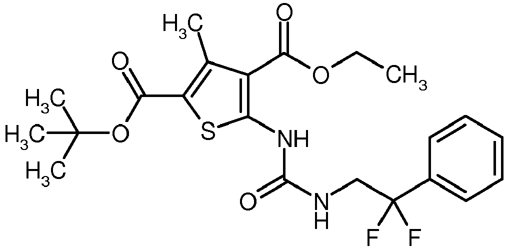

Analog zu dem unter Bsp. 2A beschriebenen Verfahren wurden aus 1.63 g (5.69 mmol) 2-*tert.-*Butyl-4-ethyl-5-amino-3-methylthiophen-2,4-dicarboxylat (Beispiel 1A), 1.85 g (11.4 mmol) *N,N'-*Carbonyldiimidazol, 3.2 ml (22.8 mmol) Triethylamin und 1.79 g (11.4 mmol) 2,2-Difluor-2-phenylethanamin [Lit.: K.D. Kreuter, M.R. Player et al., Bioorg. Med. Chem. Lett. 2008, 18 (9), 2865-2870] 2.0 g (71% d. Th., 95% Reinheit) der Titelverbindung erhalten. Abweichend erfolgte die MPLC-Reinigung hier mit Cyclohexan/Ethylacetat 5:1 als Laufmittel.

¹H-NMR (400 MHz, DMSO-d₆, δ/ppm): 10.64 (s, 1H), 8.59 (t, 1H), 8.58-7.49 (m, 5H), 4.32 (quart, 2H), 3.92 (dt, 2H), 2.62 (s, 3H), 1.49 (s, 9H), 1.33 (t, 3H).

LC/MS (Methode 1, ESIpos): Rₜ = 1.37 min, m/z = 469 [M+H]⁺.

### Beispiel 88A

### 2-tert.-Butyl-4-ethyl-3-methyl-5-[(propylcarbamoyl)amino]thiophen-2,4-dicarboxylat

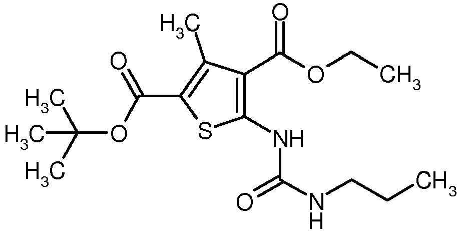

Analog zu dem unter Bsp. 2A beschriebenen Verfahren wurden aus 15.0 g (52.6 mmol) 2-*tert.-*Butyl-4-ethyl-5-amino-3-methylthiophen-2,4-dicarboxylat (Beispiel 1A), 17.0 g (105 mmol) *N,N'-*Carbonyldiimidazol, 29 ml (210 mmol) Triethylamin und 17.3 ml (210 mmol) Propylamin 12.4 g (63% d. Th.) der Titelverbindung erhalten. Abweichend erfolgte die MPLC-Reinigung hier mit Cyclohexan/Ethylacetat 10:1 als Laufmittel.

¹H-NMR (400 MHz, DMSO-d₆, δ/ppm): 10.54 (s, 1H), 8.05 (t, 1H), 4.31 (quart, 2H), 3.07 (quart, 2H), 2.62 (s, 3H), 1.50 (s, 9H), 1.50-1.41 (m, 2H), 1.32 (t, 3H), 0.88 (t, 3H).

LC/MS (Methode 1, ESIpos): Rₜ = 1.30 min, m/z = 371 [M+H]⁺.

### Beispiel 89A

### 2-tert.-Butyl-4-ethyl-3-methyl-5-{[(3,3,3-trifluor-2-methoxypropyl)carbamoyl]amino}thiophen-2,4-dicarboxylat (Racemat)

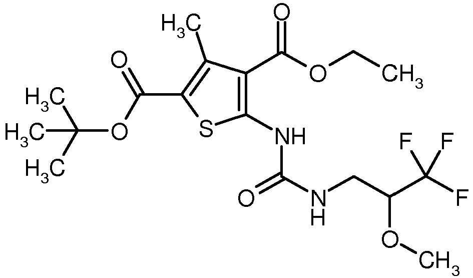

Analog zu dem unter Bsp. 2A beschriebenen Verfahren wurden aus 0.99 g (3.48 mmol) 2-*tert.-*Butyl-4-ethyl-5-amino-3-methylthiophen-2,4-dicarboxylat (Beispiel 1A), 1.13 g (6.96 mmol) *N,N'-*Carbonyldiimidazol, 1.9 ml (13.9 mmol) Triethylamin und 1.25 g (6.96 mmol) racemischem 3,3,3-Trifluor-2-methoxypropan-1-amin-Hydrochlorid 1.39 g (87% d. Th.) der Titelverbindung erhalten. Abweichend erfolgte die MPLC-Reinigung hier mit einem Cyclohexan/Ethylacetat-Gradienten 10:1 → 5:1 als Laufmittel.

¹H-NMR (400 MHz, DMSO-d₆, δ/ppm): 10.64 (s, 1H), 8.39 (t, 1H), 4.32 (quart, 2H), 4.03-3.96 (m, 1H), 3.57-3.50 (m, 1H), 3.51 (s, 3H), 3.33-3.26 (m, 1H, teilweise überdeckt vom Wassersignal), 2.63 (s, 3H), 1.50 (s, 9H), 1.33 (t, 3H).

LC/MS (Methode 1, ESIpos): Rₜ = 1.35 min, m/z = 455 [M+H]⁺.

### Beispiel 90A

### tert.-Butyl-3-(2,2-difluor-2-phenylethyl)-5-methyl-2,4-dioxo-1,2,3,4-tetrahydrothieno[2,3-d]-pyrimidin-6-carboxylat

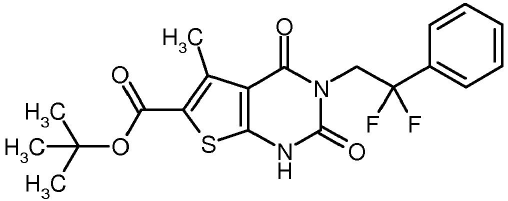

Analog zu dem unter Bsp. 8A beschriebenen Verfahren wurden aus 1.96 g (4.18 mmol) der Verbindung aus Bsp. 87A 1.64 g (92% d. Th.) der Titelverbindung erhalten.

¹H-NMR (400 MHz, DMSO-d₆, δ/ppm): 12.57 (s, 1H), 7.56-7.49 (m, 5H), 4.56 (t, 2H), 2.69 (s, 3H), 1.52 (s, 9H).

LC/MS (Methode 1, ESIneg): Rₜ = 1.20 min, m/z = 421 [M-H]⁻.

### Beispiel 91A

### tert.-Butyl-5-methyl-2,4-dioxo-3-propyl-1,2,3,4-tetrahydrothieno[2,3-d]pyrimidin-6-carboxylat

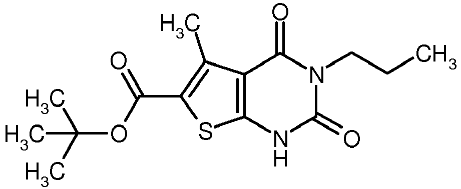

Analog zu dem unter Bsp. 8A beschriebenen Verfahren wurden aus 17.5 g (47.1 mmol) der Verbindung aus Bsp. 88A 13.9 g (90% d. Th.) der Titelverbindung erhalten.

¹H-NMR (400 MHz, DMSO-d₆, δ/ppm): 12.38 (s, 1H), 3.77 (dd, 2H), 2.71 (s, 3H), 1.60-1.49 (m, 2H), 1.52 (s, 9H), 0.87 (t, 3H).

LC/MS (Methode 1, ESIpos): Rₜ = 1.11 min, m/z = 325 [M+H]⁺.

### Beispiel 92A

### tert.-Butyl-5-methyl-2,4-dioxo-3-(3,3,3-trifluor-2-methoxypropyl)-1,2,3,4-tetrahydrothieno[2,3-d]-pyrimidin-6-carboxylat (Racemat)

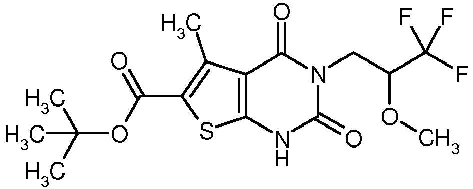

Analog zu dem unter Bsp. 8A beschriebenen Verfahren wurden aus 1.38 g (3.04 mmol) der Verbindung aus Bsp. 89A 1.19 g (96% d. Th.) der Titelverbindung erhalten.

¹H-NMR (400 MHz, DMSO-d₆, δ/ppm): 12.56 (s, 1H), 4.24-4.16 (m, 2H), 4.00 (quart, 1H), 3.43 (s, 3H), 2.72 (s, 3H), 1.52 (s, 9H).

LC/MS (Methode 9, ESIpos): Rₜ = 1.43 min, m/z = 409 [M+H]⁺.

### Beispiel 93A

### tert.-Butyl-3-(2,2-difluor-2-phenylethyl)-5-methyl-2,4-dioxo-1-(3,3,3-trifluorpropyl)-1,2,3,4-tetra-hydrothieno[2,3-d]pyrimidin-6-carboxylat

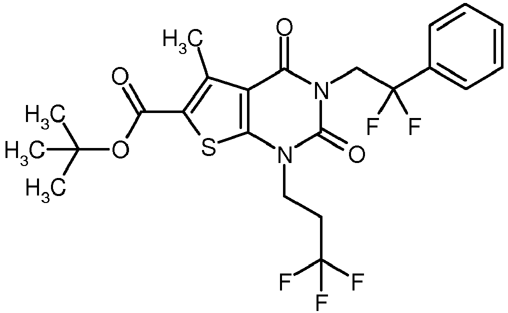

Eine Lösung von 800 mg (1.89 mmol) der Verbindung aus Bsp. 90A in 20 ml DMF wurde mit 926 mg (2.84 mmol) Cäsiumcarbonat versetzt und 20 min bei RT gerührt. Dann wurden 636 mg (2.84 mmol) 3,3,3-Trifluor-1-iodpropan hinzugefügt und das Gemisch 5 h bei 60°C gerührt. Nach dem Abkühlen auf RT wurde mit Ethylacetat verdünnt und nacheinander mit Wasser und gesättigter Natriumchlorid-Lösung gewaschen. Nach Trocknen über wasserfreiem Magnesiumsulfat wurde filtriert und eingeengt. Das Rohprodukt wurde mittels MPLC gereinigt (Biotage-Kartusche mit 50 g Kieselgel, Laufmittel Cyclohexan/Ethylacetat 10:1). Die Produktfraktionen wurden vereinigt und eingeengt. Nach Trocknen des Rückstands im Hochvakuum wurden 625 mg (63% d. Th.) der Titelverbindung erhalten.

¹H-NMR (400 MHz, DMSO-d₆, δ/ppm): 7.56-7.49 (m, 5H), 4.62 (t, 2H), 4.15 (t, 2H), 2.79-2.67 (m, 2H), 2.73 (s, 3H), 1.54 (s, 9H).

LC/MS (Methode 1, ESIpos): Rₜ = 1.39 min, m/z = 519 [M+H]⁺.

### Beispiel 94A

### tert.-Butyl-3-(2,2-difluor-2-phenylethyl)-1-(2-methoxyethyl)-5-methyl-2,4-dioxo-1,2,3,4-tetra-hydrothieno[2,3-d]pyrimidin-6-carboxylat

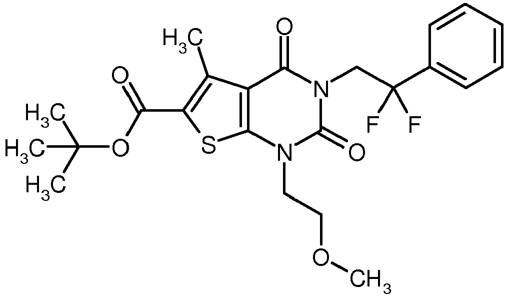

Eine Lösung von 800 mg (1.89 mmol) der Verbindung aus Bsp. 90A in 20 ml DMF wurde mit 926 mg (2.84 mmol) Cäsiumcarbonat versetzt und 10 min bei RT gerührt. Dann wurden 395 mg (2.84 mmol) 2-Bromethyl-methylether hinzugefügt und das Gemisch 4 h bei 65°C gerührt. Nach dem Abkühlen auf RT wurde mit Ethylacetat verdünnt und nacheinander mit Wasser und gesättigter Natriumchlorid-Lösung gewaschen. Nach Trocknen über wasserfreiem Magnesiumsulfat wurde filtriert und eingeengt. Das Rohprodukt wurde mittels MPLC gereinigt (Puriflash-Kartusche mit 50 g Kieselgel, Laufmittel Cyclohexan/Ethylacetat 10:1). Die Produktfraktionen wurden vereinigt und eingeengt. Der Rückstand wurde zur weiteren Aufreinigung mit 20 ml eines Gemisches aus Pentan und Dichlormethan (20:1) bei RT verrührt. Nach neuerlicher Filtration und Trocknen im Hochvakuum wurden 685 mg (75% d. Th.) der Titelverbindung erhalten.

¹H-NMR (400 MHz, DMSO-d₆, δ/ppm): 7.55-7.49 (m, 5H), 4.62 (t, 2H), 4.06 (t, 2H), 3.58 (t, 2H), 3.24 (s, 3H), 2.72 (s, 3H), 1.53 (s, 9H).

LC/MS (Methode 1, ESIpos): Rₜ = 1.37 min, m/z = 481 [M+H]⁺.

### Beispiel 95A

### tert.-Butyl-5-methyl-2,4-dioxo-3-propyl-1-[2-(trifluormethoxy)ethyl]-1,2,3,4-tetrahydrothieno-[2,3-d]pyrimidin-6-carboxylat

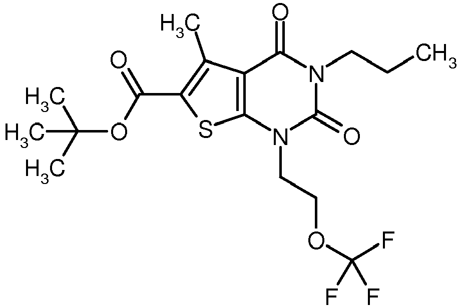

Eine Lösung von 1.12 g (3.46 mmol) der Verbindung aus Bsp. 91A in 30 ml DMF wurde mit 1.67 g (5.18 mmol) Cäsiumcarbonat versetzt und 20 min bei RT gerührt. Dann wurden 1.0 g (5.18 mmol) 1-Brom-2-trifluormethoxyethan hinzugefügt und das Gemisch 5 h bei 80°C gerührt. Anschließend wurde zur Trockene eingeengt und der Rückstand mit Ethylacetat aufgenommen. Es wurde nacheinander mit Wasser und gesättigter Natriumchlorid-Lösung gewaschen. Nach Trocknen über wasserfreiem Magnesiumsulfat wurde filtriert und eingeengt. Das Rohprodukt wurde mittels MPLC gereinigt (Biotage-Kartusche mit 100 g Kieselgel, Laufmittel Cyclohexan/Ethylacetat 10:1 → 5:1). Die Produktfraktionen wurden vereinigt und eingeengt. Nach Trocknen des Rückstands im Hochvakuum wurden 1.28 g (82% d. Th., 97% Reinheit) der Titelverbindung erhalten.

¹H-NMR (400 MHz, DMSO-d₆, δ/ppm): 4.42 (t, 2H), 4.25 (t, 2H), 3.83 (t, 2H), 2.75 (s, 3H), 1.61-1.51 (m, 2H), 1.53 (s, 9H), 0.86 (t, 3H).

LC/MS (Methode 1, ESIpos): Rₜ = 1.37 min; keine Ionisierung.

### Beispiel 96A

### tert.-Butyl-5-methyl-2,4-dioxo-3-(3,3,3-trifluor-2-methoxypropyl)-1-(3,3,3-trifluorpropyl)-1,2,3,4-tetrahydrothieno[2,3-d]pyrimidin-6-carboxylat (Racemat)

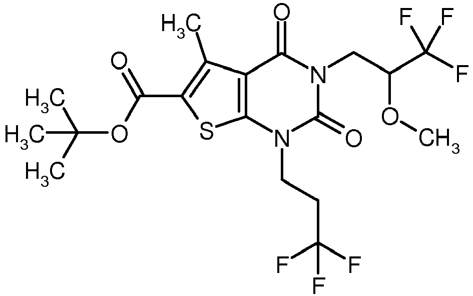

Eine Lösung von 500 mg (1.22 mmol) der Verbindung aus Bsp. 92A in 5 ml DMF wurde mit 598 mg (1.84 mmol) Cäsiumcarbonat versetzt und 20 min bei RT gerührt. Dann wurden 411 mg (1.84 mmol) 3,3,3-Trifluor-1-iodpropan hinzugefügt. Das Gemisch wurde in einem Mikrowellenofen (Biotage Initiator mit dynamischer Steuerung der Einstrahlleistung) 2 h bei 100°C gerührt. Nach dem Abkühlen auf RT wurde mit ca. 50 ml Wasser versetzt und mit Ethylacetat extrahiert. Der organische Extrakt wurde mit Wasser und gesättigter Natriumchlorid-Lösung gewaschen. Nach Trocknen über wasserfreiem Magnesiumsulfat wurde filtriert und eingeengt. Der erhaltene Rückstand wurde mittels präparativer HPLC gereinigt (Methode 6). Nach Vereinigen der Produktfraktionen, Einengen und Trocknen im Hochvakuum wurden 495 mg (80% d. Th.) der Titelverbindung erhalten.

¹H-NMR (400 MHz, DMSO-d₆, δ/ppm): 4.29-4.17 (m, 4H), 4.05 (dd, 1H), 3.42 (s, 3H), 2.87-2.76 (m, 2H), 2.76 (s, 3H), 1.54 (s, 9H).

LC/MS (Methode 9, ESIpos): Rₜ = 1.38 min, m/z = 505 [M+H]⁺.

### Beispiel 97A

### tert.-Butyl-3-ethyl-5-methyl-2,4-dioxo-1-[2-(trifluormethoxy)ethyl]-1,2,3,4-tetrahydrothieno-[2,3-d]pyrimidin-6-carboxylat

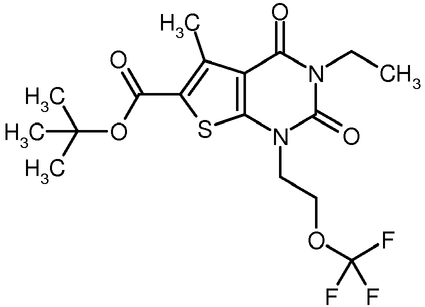

Analog zu dem unter Bsp. 26A beschriebenen Verfahren wurden aus 2.50 g (8.06 mmol) der Verbindung aus Bsp. 12A und 2.33 g (12.1 mmol) 1-Brom-2-trifluormethoxyethan 1.93 g (56% d. Th.) der Titelverbindung erhalten. Abweichend zum zuvor beschriebenen Verfahren betrug die Reaktionszeit bei 100°C hier 5 h. Die MPLC-Reinigung erfolgte über eine 100 g Kieselgel enthaltende Biotage-Kartusche mit einem Laufmittelgradienten Cyclohexan/Ethylacetat 50:1 → 10:1. Das abschließende Verrühren des so erhaltenen Produkts wurde mit einem Gemisch aus 30 ml Pentan und 0.5 ml Dichlormethan durchgeführt.

¹H-NMR (400 MHz, DMSO-d₆, δ/ppm): 4.42 (t, 2H), 4.25 (t, 2H), 3.91 (quart, 2H), 2.75 (s, 3H), 1.53 (s, 9H), 1.13 (t, 3H).

LC/MS (Methode 1, ESIpos): Rₜ = 1.30 min; schlechte Ionisierung.

### Beispiel 98A

### tert.-Butyl-5-methyl-2,4-dioxo-3-propyl-1-(3,3,3-trifluorpropyl)-1,2,3,4-tetrahydrothieno[2,3-d]-pyrimidin-6-carboxylat

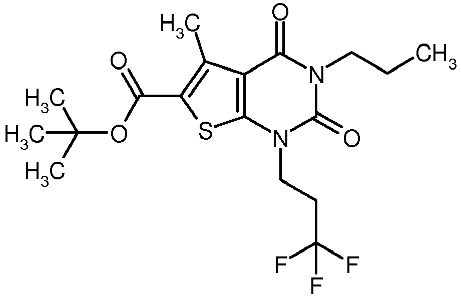

Eine Lösung von 4.0 g (12.3 mmol) der Verbindung aus Bsp. 91A in 120 ml DMF wurde mit 6.03 g (18.5 mmol) Cäsiumcarbonat versetzt und 20 min bei RT gerührt. Dann wurden 4.14 g (18.5 mmol) 3,3,3-Trifluor-1-iodpropan hinzugefügt und das Gemisch 5 h bei 70°C gerührt. Nach dieser Zeit wurden weitere 4.02 g (12.3 mmol) Cäsiumcarbonat und 2.76 g (12.3 mmol) 3,3,3-Trifluor-1-iodpropan hinzugefügt und das Gemisch nochmals 4 h bei 70°C gerührt. Nach dem Abkühlen auf RT wurde mit Ethylacetat verdünnt und nacheinander mit Wasser und gesättigter Natriumchlorid-Lösung gewaschen. Nach Trocknen über wasserfreiem Magnesiumsulfat wurde filtriert und eingeengt. Das Rohprodukt wurde mittels MPLC gereinigt (Biotage-Kartusche mit 100 g Kieselgel, Laufmittel Cyclohexan/Ethylacetat 10:1). Die Produktfraktionen wurden vereinigt und eingeengt. Da das so erhaltene Produkt noch verunreinigt war, erfolgte eine weitere Aufreinigung mittels präparativer HPLC (Methode 6). Nach neuerlichem Eindampfen und Trocknen im Hochvakuum wurden 4.23 g (81% d. Th.) der Titelverbindung erhalten.

¹H-NMR (400 MHz, DMSO-d₆, δ/ppm): 4.15 (t, 2H), 3.83 (t, 2H), 2.85-2.73 (m, 2H), 2.75 (s, 3H), 1.62-1.51 (m, 2H), 1.53 (s, 9H), 0.87 (t, 3H).

LC/MS (Methode 1, ESIpos): Rₜ = 1.37 min, m/z = 421 [M+H]⁺.

### Beispiel 99A

### tert.-Butyl-1-(2-methoxyethyl)-5-methyl-2,4-dioxo-3-propyl-1,2,3,4-tetrahydrothieno[2,3-d]-pyrimidin-6-carboxylat

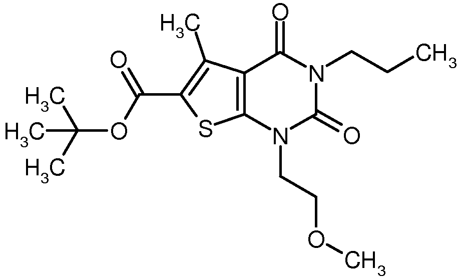

Analog zu dem unter Bsp. 26A beschriebenen Verfahren wurden aus 4.0 g (12.3 mmol) der Verbindung aus Bsp. 91A und 2.57 g (18.5 mmol) 1-Brom-2-methoxyethan 3.59 g (76% d. Th.) der Titelverbindung erhalten. Abweichend zum zuvor beschriebenen Verfahren betrug die Reaktionszeit bei 100°C hier 5 h. Die MPLC-Reinigung erfolgte über eine 100 g Kieselgel enthaltende Biotage-Kartusche mit einem Laufmittelgradienten Cyclohexan/Ethylacetat 10:1 → 5:1. Das abschließende Verrühren des so erhaltenen Produkts wurde mit einem Gemisch aus 60 ml Pentan und 1.5 ml Dichlormethan durchgeführt.

¹H-NMR (400 MHz, DMSO-d₆, δ/ppm): 4.07 (t, 2H), 3.82 (t, 2H), 3.65 (t, 2H), 3.24 (s, 3H), 2.74 (s, 3H), 1.61-1.50 (m, 2H), 1.53 (s, 9H), 0.87 (t, 3H).

LC/MS (Methode 1, ESIpos): Rₜ = 1.29 min, m/z = 383 [M+H]⁺.

### Beispiel 100A

### 3-(2,2-Difluor-2-phenylethyl)-5-methyl-2,4-dioxo-1-(3,3,3-trifluorpropyl)-1,2,3,4-tetrahydro-thieno[2,3-d]pyrimidin-6-carbonsäure

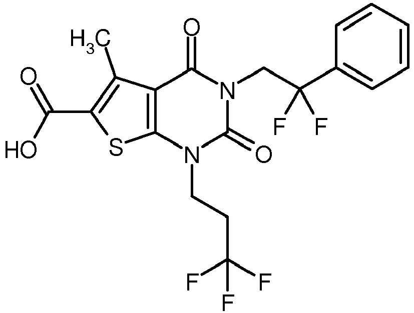

Analog zu dem unter Bsp. 43A beschriebenen Verfahren wurden aus 614 mg (1.18 mmol) der Verbindung aus Bsp. 93A und 10 ml Trifluoressigsäure in 20 ml Dichlormethan 543 mg (99% d. Th.) der Titelverbindung erhalten.

¹H-NMR (400 MHz, DMSO-d₆, δ/ppm): 13.52 (s, breit, 1H), 7.56-7.49 (m, 5H), 4.63 (t, 2H), 4.15 (t, 2H), 2.79-2.67 (m, 2H), 2.74 (s, 3H).

LC/MS (Methode 1, ESIneg): Rₜ = 1.09 min, m/z = 461 [M-H]⁻.

### Beispiel 101A

### 3-(2,2-Difluor-2-phenylethyl)-1-(2-methoxyethyl)-5-methyl-2,4-dioxo-1,2,3,4-tetrahydrothieno-[2,3-d]pyrimidin-6-carbonsäure

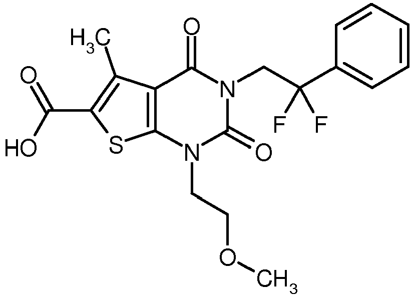

Analog zu dem unter Bsp. 43A beschriebenen Verfahren wurden aus 673 mg (1.40 mmol) der Verbindung aus Bsp. 94A und 10 ml Trifluoressigsäure in 20 ml Dichlormethan 419 mg (67% d. Th., 96% Reinheit) der Titelverbindung erhalten.

¹H-NMR (400 MHz, DMSO-d₆, δ/ppm): 13.41 (s, breit, 1H), 7.56-7.49 (m, 5H), 4.62 (t, 2H), 4.06 (t, 2H), 3.59 (t, 2H), 3.24 (s, 3H), 2.73 (s, 3H).

LC/MS (Methode 1, ESIpos): Rₜ = 0.98 min, m/z = 425 [M+H]⁺.

### Beispiel 102A

### 5-Methyl-2,4-dioxo-3-propyl-1-[2-(trifluormethoxy)ethyl]-1,2,3,4-tetrahydrothieno[2,3-d]-pyrimidin-6-carbonsäure

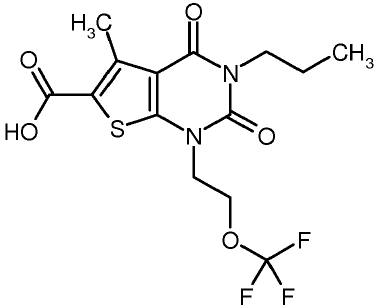

Analog zu dem unter Bsp. 43A beschriebenen Verfahren wurden aus 1.25 g (2.86 mmol) der Verbindung aus Bsp. 95A und 15 ml Trifluoressigsäure in 30 ml Dichlormethan 1.06 g (95% d. Th., 97% Reinheit) der Titelverbindung erhalten.

¹H-NMR (400 MHz, DMSO-d₆, δ/ppm): 13.40 (s, breit, 1H), 4.41 (t, 2H), 4.25 (t, 2H), 3.83 (t, 2H), 2.75 (s, 3H), 1.57 (m, 2H), 0.87 (t, 3H).

LC/MS (Methode 9, ESIpos): Rₜ = 1.25 min, m/z = 381 [M+H]⁺.

### Beispiel 103A

### 5-Methyl-2,4-dioxo-3-(3,3,3-trifluor-2-methoxypropyl)-1-(3,3,3-trifluorpropyl)-1,2,3,4-tetrahydro-thieno[2,3-d]pyrimidin-6-carbonsäure (Racemat)

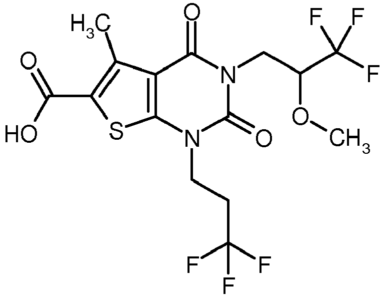

Analog zu dem unter Bsp. 43A beschriebenen Verfahren wurden aus 488 mg (0.967 mmol) der Verbindung aus Bsp. 96A und 8 ml Trifluoressigsäure in 16 ml Dichlormethan 432 mg (99% d. Th.) der Titelverbindung erhalten.

¹H-NMR (400 MHz, DMSO-d₆, δ/ppm): 13.50 (s, breit, 1H), 4.29-4.16 (m, 4H), 4.05 (dd, 1H), 3.43 (s, 3H), 2.87-2.75 (m, 2H), 2.76 (s, 3H).

LC/MS (Methode 1, ESIpos): Rₜ = 1.01 min, m/z = 449 [M+H]⁺.

### Beispiel 104A

### 3-Ethyl-5-methyl-2,4-dioxo-1-[2-(trifluormethoxy)ethyl]-1,2,3,4-tetrahydrothieno[2,3-d]pyrimidin-6-carbonsäure

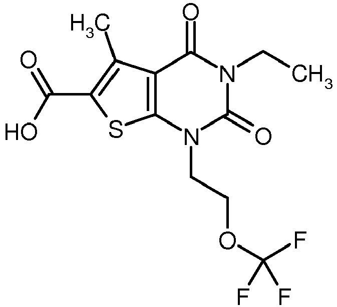

Eine Lösung von 2.31 g (5.47 mmol) der Verbindung aus Bsp. 97A in 60 ml Dichlormethan wurde mit 30 ml Trifluoressigsäure versetzt und 1 h bei RT gerührt. Danach wurde das Reaktionsgemisch am Rotationsverdampfer bis zur Trockene eingeengt. Der verbliebene Rückstand wurde in ca. 400 ml Ethylacetat gelöst und zweimal mit Wasser und einmal mit gesättigter Natriumchlorid-Lösung gewaschen. Nach Trocknen über wasserfreiem Magnesiumsulfat wurde filtriert und eingeengt und der Rückstand im Hochvakuum getrocknet. Es wurden 2.04 g (99% d. Th.) der Titelverbindung erhalten.

¹H-NMR (400 MHz, DMSO-d₆, δ/ppm): 13.40 (s, breit, 1H), 4.42 (t, 2H), 4.25 (t, 2H), 3.91 (quart, 2H), 2.76 (s, 3H), 1.13 (t, 3H).

LC/MS (Methode 1, ESIpos): Rₜ = 0.91 min, m/z = 367 [M+H]⁺.

### Beispiel 105A

### 5-Methyl-2,4-dioxo-3-propyl-1-(3,3,3-trifluorpropyl)-1,2,3,4-tetrahydrothieno[2,3-d]pyrimidin-6-carbonsäure

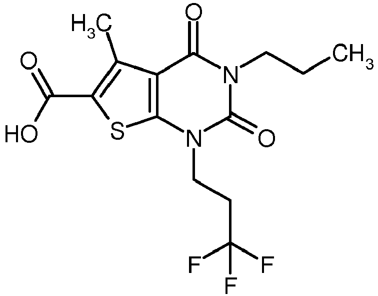

Analog zu dem unter Bsp. 43A beschriebenen Verfahren wurden aus 4.20 g (9.99 mmol) der Verbindung aus Bsp. 98A und 60 ml Trifluoressigsäure in 120 ml Dichlormethan 2.61 g (71% d. Th.) der Titelverbindung erhalten.

¹H-NMR (400 MHz, DMSO-d₆, δ/ppm): 13.35 (s, breit, 1H), 4.15 (t, 2H), 3.83 (t, 2H), 2.84-2.74 (m, 2H), 2.76 (s, 3H), 1.57 (m, 2H), 0.87 (t, 3H).

LC/MS (Methode 1, ESIpos): Rₜ = 0.98 min, m/z = 365 [M+H]⁺.

### Beispiel 106A

### 1-(2-Methoxyethyl)-5-methyl-2,4-dioxo-3-propyl-1,2,3,4-tetrahydrothieno[2,3-d]pyrimidin-6-carbonsäure

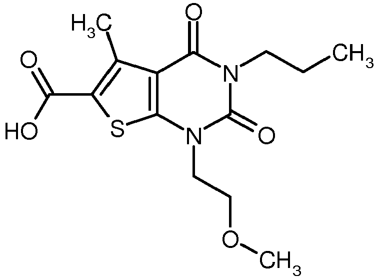

Analog zu dem unter Bsp. 43A beschriebenen Verfahren wurden aus 4.15 g (10.8 mmol) der Verbindung aus Bsp. 99A und 60 ml Trifluoressigsäure in 120 ml Dichlormethan 3.44 g (97% d. Th.) der Titelverbindung erhalten.

¹H-NMR (400 MHz, DMSO-d₆, δ/ppm): 13.32 (s, breit, 1H), 4.07 (t, 2H), 3.82 (dd, 2H), 3.65 (t, 2H), 3.24 (s, 3H), 2.75 (s, 3H), 1.57 (m, 2H), 0.87 (t, 3H).

LC/MS (Methode 1, ESIpos): Rₜ = 0.85 min, m/z = 327 [M+H]⁺.

### Beispiel 107A

### 2-tert.-Butyl-4-ethyl-5-amino-3-ethylthiophen-2,4-dicarboxylat

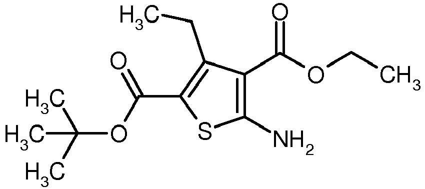

6.5 g (37.7 mmol) *tert*.-Butyl-3-oxopentanoat [Lit.: S. Luedeke, M. Mueller, M. Richter, Adv. Synth. Catal. 2009, 351 (1-2), 253-259], 4.27 g (37.7 mmol) Cyanessigsäureethylester und 1.33 g (41.5 mmol) Schwefel wurden in 10 ml Ethanol vorgelegt und auf 45°C erwärmt. Zu diesem Gemisch wurden 3.8 ml (43.3 mmol) Morpholin hinzugetropft. Danach wurde das Reaktionsgemisch 5 h bei 60°C gerührt. Anschließend wurden alle flüchtigen Bestandteile am Rotationsverdampfer entfernt. Der verbliebene Rückstand wurde mit ca. 250 ml Wasser versetzt und dreimal mit je ca. 200 ml Ethylacetat extrahiert. Die vereinigten organischen Extrakte wurden mit ca. 200 ml gesättigter Kochsalz-Lösung gewaschen und dann über wasserfreiem Magnesiumsulfat getrocknet. Nach Filtration wurde zur Trockene eingeengt. Das so erhaltene Rohprodukt wurde mittels MPLC gereinigt (Biotage-Kartusche, 340 g Kieselgel, Laufmittel Cyclohexan/Ethylacetat 20:1 → 10:1). Nach Vereinigen der Produktfraktionen, Einengen und Trocknen im Hochvakuum wurden 4.06 g (35% d. Th.) der Titelverbindung erhalten.

¹H-NMR (400 MHz, CDCl₃, δ/ppm): 6.45 (s, breit, 2H), 4.32 (quart, 2H), 3.22 (quart, 2H), 1.54 (s, 9H), 1.37 (t, 3H), 1.16 (t, 3H).

LC/MS (Methode 1, ESIpos): Rₜ = 1.23 min, m/z = 300 [M+H]⁺.

### Beispiel 108A

### 2-tert.-Butyl-4-ethyl-3-ethyl-5-[(ethylcarbamoyl)amino]thiophen-2,4-dicarboxylat

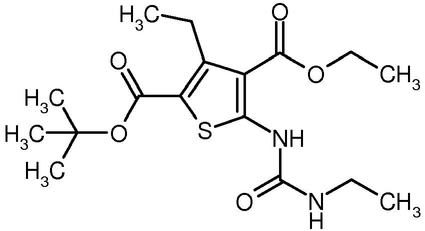

Analog zu dem unter Bsp. 2A beschriebenen Verfahren wurden aus 1.44 g (4.81 mmol) der Verbindung aus Bsp. 107A, 1.56 g (9.62 mmol) *N*,*N'*-Carbonyldiimidazol (CDI), 2.7 ml (19.2 mmol) Triethylamin und 9.6 ml (19.2 mmol) einer 2 M Lösung von Ethylamin in THF 1.68 g (89% d. Th., 95% Reinheit) der Titelverbindung erhalten. Die Reaktionszeit für die Umsetzung mit CDI betrug hier 4 Tage, und die MPLC-Reinigung erfolgte mit Cyclohexan/Ethylacetat 10:1 → 5:1 als Laufmittel.

¹H-NMR (400 MHz, CDCl₃, δ/ppm): 10.98 (s, breit, 1H), 4.85 (t, 1H), 4.35 (quart, 2H), 3.37 (m, 2H), 3.27 (quart, 2H), 1.55 (s, 9H), 1.40 (t, 3H), 1.22 (t, 3H), 1.17 (t, 3H).

LC/MS (Methode 1, ESIpos): Rₜ = 1.24 min, m/z = 371 [M+H]⁺.

### Beispiel 109A

### tert.-Butyl-3,5-diethyl-2,4-dioxo-1,2,3,4-tetrahydrothieno[2,3-d]pyrimidin-6-carboxylat

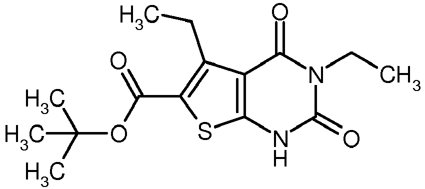

Analog zu dem unter Bsp. 8A beschriebenen Verfahren wurden aus 1.60 g (4.32 mmol) der Verbindung aus Bsp. 108A 1.34 g (88% d. Th., 92% Reinheit) der Titelverbindung erhalten.

¹H-NMR (400 MHz, CDCl₃, δ/ppm): 10.49 (s, breit, 1H), 4.08 (quart, 2H), 3.37 (quart, 2H), 1.58 (s, 9H), 1.29 (t, 3H), 1.23 (t, 3H).

LC/MS (Methode 1, ESIpos): Rₜ = 1.07 min, m/z = 325 [M+H]⁺.

### Beispiel 110A

### tert.-Butyl-3,5-diethyl-2,4-dioxo-1-(3,3,3-trifluorpropyl)-1,2,3,4-tetrahydrothieno[2,3-d]pyrimidin-6-carboxylat

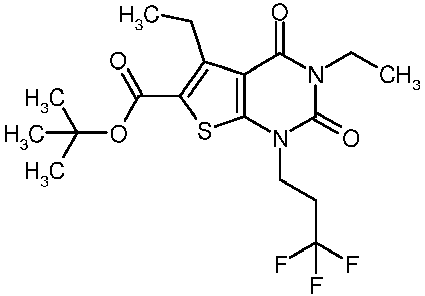

Eine Lösung von 980 mg (3.02 mmol) der Verbindung aus Bsp. 109A in 15 ml DMF wurde mit 1.48 g (4.53 mmol) Cäsiumcarbonat versetzt und 20 min bei RT gerührt. Dann wurden 1.02 g (4.53 mmol) 3,3,3-Trifluor-1-iodpropan hinzugefügt, und das Gemisch wurde in einem Mikrowellenofen (Biotage Initiator mit dynamischer Steuerung der Einstrahlleistung) 2 h bei 100°C gerührt. Nach dem Abkühlen auf RT wurde mit ca. 75 ml Wasser versetzt und mit Ethylacetat extrahiert. Der organische Extrakt wurde mit Wasser und gesättigter Natriumchlorid-Lösung gewaschen. Nach Trocknen über wasserfreiem Magnesiumsulfat wurde filtriert und eingeengt. Der erhaltene Rückstand wurde zunächst mittels MPLC grob vorgereinigt (Biotage-Kartusche, 50 g Kieselgel, Laufmittel Cyclohexan/Ethylacetat 20:1). Die Produkt enthaltenden Fraktionen wurden vereinigt und zur Trockene eingeengt. Der Rückstand wurde mit einem Gemisch aus 20 ml Pentan und ca. 0.5 ml Dichlormethan 16 h lang bei RT verrührt. Nach Absaugen und Trocknen im Hochvakuum wurde so eine erste Fraktion von 326 mg der Titelverbindung erhalten. Das Filtrat des Verrührens wurde eingeengt und der Rückstand mittels präparativer HPLC gereinigt (Methode 6). Nach Einengen der Produktfraktionen und Trocknen im Hochvakuum wurde auf diese Weise eine zweite Fraktion von 565 mg der Titelverbindung gewonnen. Insgesamt wurden somit 891 mg (70% d. Th.) der Titelverbindung erhalten.

¹H-NMR (400 MHz, CDCl₃, δ/ppm): 4.19 (m, 2H), 4.07 (quart, 2H), 3.38 (quart, 2H), 2.71-2.59 (m, 2H), 1.59 (s, 9H), 1.26 (t, 3H), 1.23 (t, 3H).

LC/MS (Methode 1, ESIpos): Rₜ = 1.39 min, m/z = 421 [M+H]⁺.

### Beispiel 111A

### 3,5-Diethyl-2,4-dioxo-1-(3,3,3-trifluorpropyl)-1,2,3,4-tetrahydrothieno[2,3-d]pyrimidin-6-carbonsäure

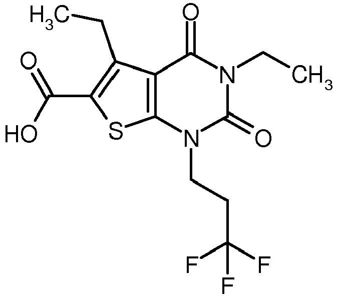

Analog zu dem unter Bsp. 43A beschriebenen Verfahren wurden aus 1.15 g (2.73 mmol) der Verbindung aus Bsp. 110A und 20 ml Trifluoressigsäure in 60 ml Dichlormethan 851 mg (85% d. Th.) der Titelverbindung erhalten.

¹H-NMR (400 MHz, DMSO-d₆, δ/ppm): 13.43 (s, breit, 1H), 4.15 (t, 2H), 3.92 (quart, 2H), 3.35-3.29 (m, 2H, teilweise überdeckt vom Wassersignal), 2.86-2.74 (m, 2H), 1.135 (t, 3H), 1.130 (t, 3H).

LC/MS (Methode 10, ESIpos): Rₜ = 1.26 min, m/z = 365 [M+H]⁺.

### Beispiel 112A

### 3-Ethyl-5-methyl-6-[(3-methyl-4-oxopiperidin-1-yl)carbonyl]-1-(3,3,3-trifluorpropyl)thieno[2,3-d]-pyrimidin-2,4(1H,3H)-dion (Racemat)

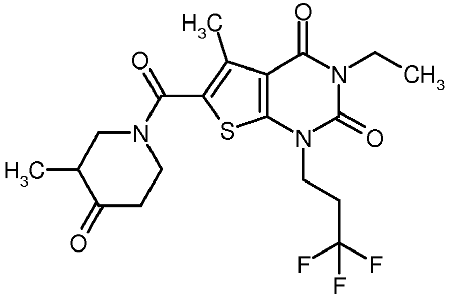

*Darstellung des Säurechlorids:* Eine Lösung von 200 mg (0.571 mmol) der Verbindung aus Bsp. 52A in 4 ml Dichlormethan wurde bei RT zunächst mit 249 µl (2.86 mmol) Oxalylchlorid und dann mit einem kleinen Tropfen DMF versetzt. Nachdem das Reaktionsgemisch 2.5 h bei RT gerührt worden war, wurde es am Rotationsverdampfer bis zur Trockene eingeengt. Der verbliebene Rückstand wurde im Hochvakuum getrocknet und anschließend im nächsten Teilschritt weiter umgesetzt.

*Darstellung des Amids:* Das oben erhaltene Säurechlorid wurde in 1 ml Dichlormethan gelöst und zu einer Lösung von 171 mg (1.14 mmol) 3-Methylpiperidin-4-on-Hydrochlorid und 398 µl (2.28 mmol) *N*,*N*-Diisopropylethylamin in 5 ml Dichlormethan getropft. Das Reaktionsgemisch wurde 2 h bei RT gerührt. Nachdem das Gemisch am Rotationsverdampfer zur Trockene eingeengt worden war, wurde das Rohprodukt mittels präparativer HPLC gereinigt (Methode 6). Nach Vereinigen der Produktfraktionen, Einengen und Trocknen im Hochvakuum wurden 236 mg (88% d. Th., 95% Reinheit) der Titelverbindung erhalten.

¹H-NMR (400 MHz, DMSO-d₆, δ/ppm): 4.18-4.09 (m, 4H), 3.92 (quart, 2H), 3.53-3.45 (m, 1H), 3.19-3.12 (m, 1H), 2.86-2.74 (m, 2H), 2.71-2.63 (m, 1H), 2.61-2.52 (m, 1H, teilweise überdeckt vom DMSO-Signal), 2.44 (s, 3H), 2.35 (dt, 1H), 1.13 (t, 3H), 0.92 (d, 3H).

LC/MS (Methode 1, ESIpos): Rₜ = 0.93 min, m/z = 446 [M+H]⁺.

### Beispiel 113A

### 1-[1-Ethyl-2,6-dioxo-4-(pyridinium-1-yl)-1,6-dihydropyrimidin-5(2H)-yliden]-2,2-difluorethanolat

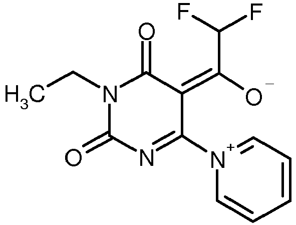

Eine Suspension von 7.5 g (43.0 mmol) der Verbindung aus Bsp. 17A in 110 ml Acetonitril wurde bei RT mit 35 ml (430 mmol) Pyridin versetzt. Dann wurden langsam 21.4 ml (172 mmol) Difluoressigsäureanhydrid hinzugetropft. Nach beendeter Zugabe wurde noch 1 h bei RT gerührt. Anschließend wurde mit ca. 300 ml Wasser versetzt und viermal mit je ca. 100 ml Ethylacetat extrahiert. Der organische Extrakt wurde mit gesättigter wässriger Kochsalz-Lösung gewaschen, über wasserfreiem Magnesiumsulfat getrocknet und anschließend zur Trockene eingeengt. Der verbliebene Feststoff wurde bei RT in einem Gemisch aus 50 ml Diisopropylether und 50 ml Diethylether verrührt. Nach Absaugen und Trocknen im Hochvakuum wurden 6.38 g (50% d. Th.) der Titelverbindung erhalten.

¹H-NMR (400 MHz, DMSO-d₆, δ/ppm): 9.20 (d, 2H), 8.80 (t, 1H), 8.25 (t, 2H), 6.97 (t, 1H), 3.89 (quart, 2H), 1.14 (t, 3H).

LC/MS (Methode 1, ESIpos): Rₜ = 0.46 min, m/z = 296 [M+H]⁺.

### Beispiel 114A

### Ethyl-5-(difluormethyl)-3-ethyl-2,4-dioxo-1,2,3,4-tetrahydrothieno[2,3-d]pyrimidin-6-carboxylat

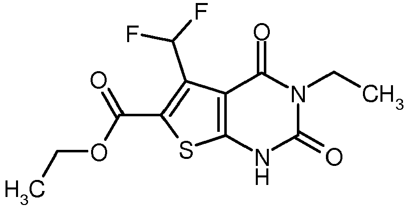

Eine Suspension von 2.04 g (6.92 mmol) der Verbindung aus Bsp. 113A in 15 ml Ethanol wurde mit 1.5 ml (13.8 mmol) Mercaptoessigsäureethylester versetzt und 5 min bei RT gerührt. Dann wurden 1.61 g (15.2 mmol) Natriumcarbonat hinzugefügt, und das Gemisch wurde in einem Mikrowellenofen (Biotage Initiator mit dynamischer Steuerung der Einstrahlleistung) 1 h lang auf 120°C erhitzt. Drei solcher Ansätze wurden vereinigt und am Rotationsverdampfer zur Trockene eingeengt. Der verbliebene Rückstand wurde in ca. 300 ml Wasser aufgenommen, durch Zugabe von Essigsäure schwach sauer gestellt und dreimal mit je ca. 100 ml Dichlormethan extrahiert. Der organische Extrakt wurde mit gesättigter wässriger Natriumchlorid-Lösung gewaschen, über wasserfreiem Magnesiumsulfat getrocknet, filtriert und eingeengt. Der hierbei verbliebene Feststoff wurde über eine Kieselgel-Kartusche chromatographiert (Puriflash, Cyclohexan/Ethylacetat-Gradient 3:1 → 1:1). Nach Eindampfen der Produktfraktionen und Trocknen des Rückstands im Hochvakuum wurden 890 mg (12% d. Th.) der Titelverbindung erhalten.

¹H-NMR (400 MHz, DMSO-d₆, δ/ppm): 12.59 (s, 1H), 7.71 (t, 1H), 4.32 (quart, 2H), 3.87 (quart, 2H), 1.30 (t, 3H), 1.13 (t, 3H).

LC/MS (Methode 1, ESIpos): Rₜ = 0.87 min, m/z = 319 [M+H]⁺.

### Beispiel 115A

### Ethyl-5-(difluormethyl)-3-ethyl-2,4-dioxo-1-(3,3,3-trifluorpropyl)-1,2,3,4-tetrahydrothieno[2,3-d]-pyrimidin-6-carboxylat

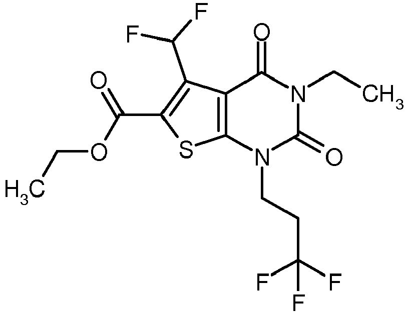

Eine Lösung von 880 mg (2.76 mmol) der Verbindung aus Bsp. 114A in 12 ml DMF wurde mit 1.35 g (4.15 mmol) Cäsiumcarbonat versetzt und 20 min bei RT gerührt. Dann wurden 929 mg (4.15 mmol) 3,3,3-Trifluor-1-iodpropan hinzugefügt, und das Gemisch wurde 2 h auf 80°C erwärmt. Nach dem Abkühlen auf RT wurde mit ca. 100 ml Ethylacetat verdünnt und nacheinander zweimal mit je ca. 100 ml Wasser und einmal mit ca. 100 ml gesättigter Natriumchlorid-Lösung gewaschen. Nach Trocknen über wasserfreiem Magnesiumsulfat wurde filtriert und eingeengt. Das Rohprodukt wurde mittels präparativer HPLC gereinigt (Methode 6). Die Produktfraktionen wurden vereinigt und eingeengt und der Rückstand im Hochvakuum getrocknet. Es wurden 750 mg (63% d. Th.) der Titelverbindung erhalten.

¹H-NMR (400 MHz, DMSO-d₆, δ/ppm): 7.74 (t, 1H), 4.36 (quart, 2H), 4.20 (t, 2H), 3.92 (quart, 2H), 2.88-2.76 (m, 2H), 1.32 (t, 3H), 1.14 (t, 3H).

LC/MS (Methode 1, ESIpos): Rₜ = 1.13 min, m/z = 415 [M+H]⁺.

### Beispiel 116A

### Ethyl-5-(difluormethyl)-3-ethyl-2,4-dioxo-1-[2-(trifluormethoxy)ethyl]-1,2,3,4-tetrahydrothieno-[2,3-d]pyrimidin-6-carboxylat

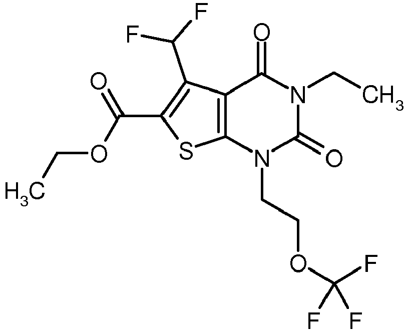

Analog zu dem unter Bsp. 115A beschriebenen Verfahren wurden aus 500 mg (1.57 mmol) der Verbindung aus Bsp. 114A und 455 mg (2.36 mmol) 1-Brom-2-(trifluormethoxy)ethan [kommerziell erhältlich; Lit.: P.E. Aldrich, W.A. Sheppard, J. Org. Chem. 1964, 29 (1), 11-15] 385 mg (56% d. Th.) der Titelverbindung erhalten. Die Reinigung des Rohprodukts über präparative HPLC erfolgte hier nach Methode 5.

¹H-NMR (400 MHz, DMSO-d₆, δ/ppm): 7.73 (t, 1H), 4.43 (t, 2H), 4.35 (quart, 2H), 4.32 (t, 2H), 3.93 (quart, 2H), 1.31 (t, 3H), 1.14 (t, 3H).

LC/MS (Methode 1, ESIpos): Rₜ = 1.12 min, m/z = 431 [M+H]⁺.

### Beispiel 117A

### tert.-Butyl-5-methyl-2,4-dioxo-3-(2-phenylethyl)-1-[2-(trifluormethoxy)ethyl]-1,2,3,4-tetrahydro-thieno[2,3-d]pyrimidin-6-carboxylat

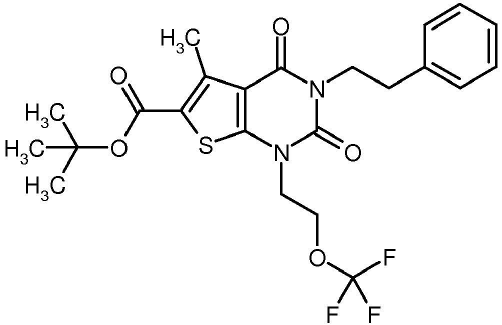

Eine Lösung von 1.0 g (2.59 mmol) der Verbindung aus Bsp. 8A in 15 ml DMF wurde mit 1.26 g (3.88 mmol) Cäsiumcarbonat versetzt und 20 min bei RT gerührt. Dann wurden 749 mg (3.88 mmol) 1-Brom-2-(trifluormethoxy)ethan [kommerziell erhältlich; Lit.: P.E. Aldrich, W.A. Sheppard, J. Org. Chem. 1964, 29 (1), 11-15] hinzugefügt, und das Gemisch wurde 2 h auf 80°C erwärmt. Nach dem Abkühlen auf RT wurde mit ca. 300 ml Wasser versetzt und nacheinander dreimal mit je ca. 75 ml Diethylether extrahiert. Der vereinigte organische Extrakt wurde je einmal mit ca. 100 ml Wasser und ca. 100 ml gesättigter Natriumchlorid-Lösung gewaschen. Nach Trocknen über wasserfreiem Magnesiumsulfat wurde filtriert und eingeengt. Das Rohprodukt wurde bei 0°C mit 30 ml Pentan, dem einige Tropfen Dichlormethan zugesetzt waren, verrührt. Der Feststoff wurde abgesaugt und ergab nach Trocknen im Hochvakuum eine erste Fraktion von 665 mg der Titelverbindung. Aus der eingeengten Mutterlauge wurde nach Reinigung durch präparative HPLC (Methode 15) eine zweite Fraktion von 402 mg der Titelverbindung gewonnen. Insgesamt wurden so 1.07 g (82% d. Th.) der Titelverbindung erhalten.

¹H-NMR (400 MHz, CDCl₃, δ/ppm): 7.30-7.20 (m, 5H, teilweise überdeckt vom CHCl₃-Signal), 4.28 (t, 2H), 4.22 (t, 2H), 4.21 (t, 2H), 2.97-2.93 (m, 2H), 2.85 (s, 3H), 1.59 (s, 9H).

LC/MS (Methode 1, ESIpos): Rₜ = 1.43 min, m/z = 499 [M+H]⁺.

### Beispiel 118A

### 5-Methyl-2,4-dioxo-3-(2-phenylethyl)-1-[2-(trifluormethoxy)ethyl]-1,2,3,4-tetrahydrothieno-[2,3-d]pyrimidin-6-carbonsäure

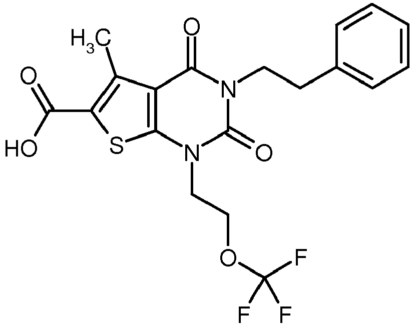

Eine Lösung von 1.0 g (2.01 mmol) der Verbindung aus Bsp. 117A in 25 ml Dichlormethan wurde mit 12.5 ml Trifluoressigsäure versetzt und 1 h bei RT gerührt. Danach wurde das Reaktionsgemisch am Rotationsverdampfer zur Trockene eingeengt. Der verbliebene Rückstand wurde über Nacht bei RT in einem Gemisch aus 40 ml Diethylether und 20 ml Pentan verrührt. Nach Absaugen und Trocknen im Hochvakuum wurde so eine erste Fraktion von 559 mg der Titelverbindung erhalten. Aus der eingeengten Mutterlauge wurde nach Reinigung durch präparative HPLC (Methode 5) eine zweite Fraktion von 266 mg der Titelverbindung gewonnen. Insgesamt wurden so 825 mg (92% d. Th.) der Titelverbindung erhalten.

¹H-NMR (400 MHz, DMSO-d₆, δ/ppm): 13.42 (breit, 1H), 7.32-7.27 (m, 2H), 7.24-7.19 (m, 3H), 4.39 (t, 2H), 4.25 (t, 2H), 4.10-4.06 (m, 2H), 2.87-2.82 (m, 2H), 2.75 (s, 3H).

LC/MS (Methode 1, ESIpos): Rₜ = 1.11 min, m/z = 443 [M+H]⁺.

### Beispiel 119A

### 5-(Difluormethyl)-3-ethyl-2,4-dioxo-1-(3,3,3-trifluorpropyl)-1,2,3,4-tetrahydrothieno[2,3-d]-pyrimidin-6-carbonsäure

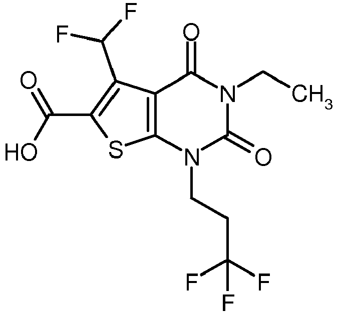

Analog zu dem unter Bsp. 55A beschriebenen Verfahren wurden aus 733 mg (1.77 mmol) der Verbindung aus Bsp. 115A 668 mg (95% d. Th.) der Titelverbindung erhalten.

¹H-NMR (400 MHz, DMSO-d₆, δ/ppm): 7.77 (t, 1H), 4.19 (t, 2H), 3.92 (quart, 2H), 2.88-2.75 (m, 2H), 1.14 (t, 3H).

LC/MS (Methode 1, ESIpos): Rₜ = 0.85 min, m/z = 387 [M+H]⁺.

### Beispiel 120A

### 5-(Difluormethyl)-3-ethyl-2,4-dioxo-1-[2-(trifluormethoxy)ethyl]-1,2,3,4-tetrahydrothieno[2,3-d]-pyrimidin-6-carbonsäure

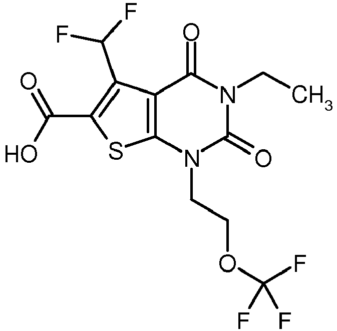

Analog zu dem unter Bsp. 55A beschriebenen Verfahren wurden aus 365 mg (0.848 mmol) der Verbindung aus Bsp. 116A 320 mg (91% d. Th.) der Titelverbindung erhalten. Die Reaktionszeit betrug hier 3 h.

¹H-NMR (400 MHz, DMSO-d₆, δ/ppm): 14.38 (sehr breit, 1H), 7.77 (t, 1H), 4.43 (t, 2H), 4.30 (t, 2H), 3.92 (quart, 2H), 1.14 (t, 3H).

LC/MS (Methode 1, ESIpos): Rₜ = 0.85 min, m/z = 403 [M+H]⁺.

### Beispiel 121A

### 1-{[5-Methyl-2,4-dioxo-3-(2-oxo-2-phenylethyl)-1-(3,3,3-trifluorpropyl)-1,2,3,4-tetrahydrothieno-[2,3-d]pyrimidin-6-yl]carbonyl}piperidin-4-ylacetat

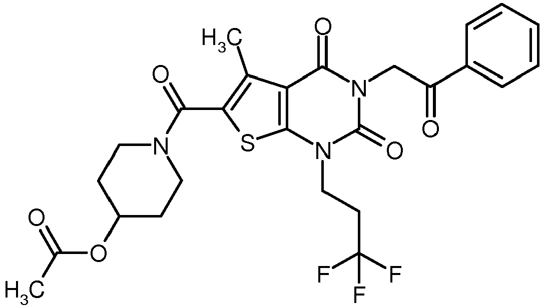

Analog zu dem unter Bsp. 78A beschriebenen Verfahren wurden aus 500 mg (1.12 mmol) der Verbindung aus Bsp. 60A und 190 mg (1.23 mmol) 2-Chloracetophenon 573 mg (88% d. Th.) der Titelverbindung erhalten. Die Reinigung des Produkts erfolgte in diesem Fall mittels MPLC (Biotage-Kartusche, 50 g Kieselgel, Cyclohexan/Ethylacetat-Gradient 2:1 → 1:1).

¹H-NMR (400 MHz, DMSO-d₆, δ/ppm): 8.10 (d, 2H), 7.74 (t, 1H), 7.60 (t, 2H), 5.43 (s, 2H), 4.94-4.90 (m, 1H), 4.19 (t, 2H), 3.80-3.74 (m, 2H), 3.44-3.40 (m, 2H), 2.86-2.78 (m, 2H), 2.38 (s, 3H), 2.03 (s, 3H), 1.92-1.87 (m, 2H), 1.60-1.54 (m, 2H).

LC/MS (Methode 1, ESIpos): Rₜ = 1.06 min, m/z = 566 [M+H]⁺.

### Beispiel 122A

### 1-{[5-Methyl-2,4-dioxo-3-(3,3,3-trifluor-2-phenylpropyl)-1-(3,3,3-trifluorpropyl)-1,2,3,4-tetra-hydrothieno[2,3-d]pyrimidin-6-yl]carbonyl}piperidin-4-ylacetat (Racemat)

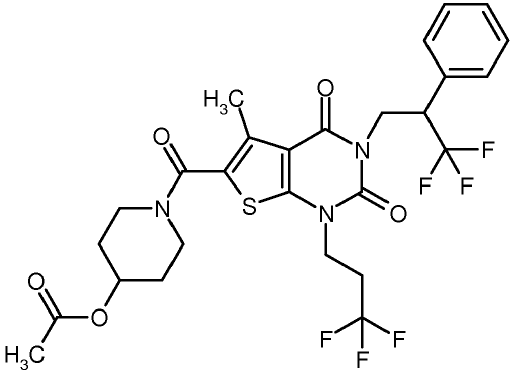

Analog zu dem unter Bsp. 63A beschriebenen Verfahren wurden aus 100 mg (0.223 mmol) der Verbindung aus Bsp. 60A und 64 mg (0.335 mmol) racemischem 3,3,3-Trifluor-2-phenylpropanol [hergestellt analog zu J.Y. Hamilton et al., Synthesis 2013, 45 (13), 1857-1862] 11 mg (7% d. Th.) der Titelverbindung erhalten. Aus einem zweiten Ansatz mit 200 mg (0.447 mmol) der Verbindung aus Bsp. 60A wurden 17 mg (6% d. Th.) der Titelverbindung erhalten.

¹H-NMR (400 MHz, CDCl₃, δ/ppm): 7.37-7.30 (m, 5H), 5.06-5.00 (m, 1H), 4.62-4.50 (m, 2H), 4.16-3.98 (m, 3H), 3.87-3.79 (m, 2H), 3.54-3.46 (m, 2H), 2.52-2.40 (m, 2H), 2.47 (s, 3H), 2.09 (s, 3H), 1.98-1.90 (m, 2H), 1.77-1.68 (m, 2H).

LC/MS (Methode 1, ESIpos): Rₜ = 1.19 min, m/z = 620 [M+H]⁺.

### Beispiel 123A

### 1-{[3-(4,4-Difluorbut-3-en-1-yl)-5-methyl-2,4-dioxo-1-(3,3,3-trifluorpropyl)-1,2,3,4-tetrahydro-thieno[2,3-d]pyrimidin-6-yl]carbonyl}piperidin-4-ylacetat

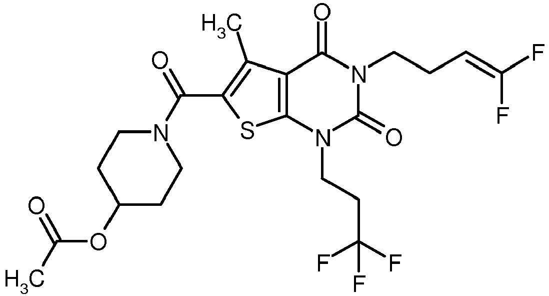

Analog zu dem unter Bsp. 78A beschriebenen Verfahren wurden aus 100 mg (0.223 mmol) der Verbindung aus Bsp. 60A und 46 mg (0.268 mmol) 4-Brom-1,1-difluorbuten 97 mg (80% d. Th.) der Titelverbindung erhalten.

¹H-NMR (400 MHz, DMSO-d₆, δ/ppm): 4.95-4.89 (m, 1H), 4.53 (dtd, 1H), 4.13 (t, 2H), 3.93 (t, 2H), 3.79-3.71 (m, 2H), 3.44-3.37 (m, 2H, teilweise überdeckt vom Wassersignal), 2.85-2.71 (m, 2H), 2.39 (s, 3H), 2.27 (quart, 2H), 2.02 (s, 3H), 1.92-1.85 (m, 2H), 1.60-1.52 (m, 2H).

LC/MS (Methode 1, ESIpos): Rₜ = 1.10 min, m/z = 538 [M+H]⁺.

### Beispiel 124A

### 1-{[3-(2-Hydroxy-2-phenylethyl)-5-methyl-2,4-dioxo-1-(3,3,3-trifluorpropyl)-1,2,3,4-tetrahydro-thieno[2,3-d]pyrimidin-6-yl]carbonyl}piperidin-4-ylacetat (Racemat)

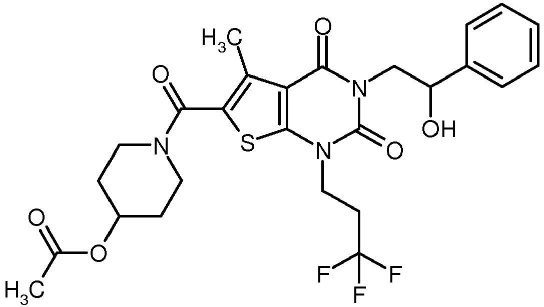

Eine Lösung von 538 mg (0.951 mmol) der Verbindung aus Bsp. 121A in 40 ml Methanol und 1 ml Dichlormethan wurde bei RT portionsweise über einen Zeitraum von ca. 2 h mit insgesamt 18 mg (0.476 mmol) festem Natriumborhydrid versetzt. Nach weiteren 30 min bei RT wurde mit Wasser versetzt und mehrfach mit Ethylacetat extrahiert. Der vereinigte organische Extrakt wurde mit gesättigter Natriumchlorid-Lösung gewaschen, über wasserfreiem Magnesiumsulfat getrocknet, filtriert und eingeengt. Der erhaltene Rückstand wurde mittels präparativer HPLC (Methode 16) gereinigt. Nach Eindampfen der Produktfraktionen und Trocknen des Rückstands im Hochvakuum wurden 435 mg (80% d. Th.) der Titelverbindung erhalten.

¹H-NMR (400 MHz, DMSO-d₆, δ/ppm): 7.38-7.31 (m, 4H), 7.28-7.23 (m, 1H), 5.39 (d, 1H), 4.96-4.89 (m, 2H), 4.23 (dd, 1H), 4.11 (t, 2H), 3.82 (dd, 1H), 3.78-3.72 (m, 2H), 3.44-3.37 (m, 2H), 2.79-2.67 (m, 2H), 2.39 (s, 3H), 2.03 (s, 3H), 1.93-1.85 (m, 2H), 1.61-1.52 (m, 2H).

LC/MS (Methode 1, ESIpos): Rₜ = 0.98 min, m/z = 568 [M+H]⁺.

### Beispiel 125A

### 1-{[3-(2-Hydroxy-2-phenylethyl)-5-methyl-2,4-dioxo-1-(3,3,3-trifluorpropyl)-1,2,3,4-tetrahydro-thieno[2,3-d]pyrimidin-6-yl]carbonyl}piperidin-4-ylacetat (Enantiomer 1)

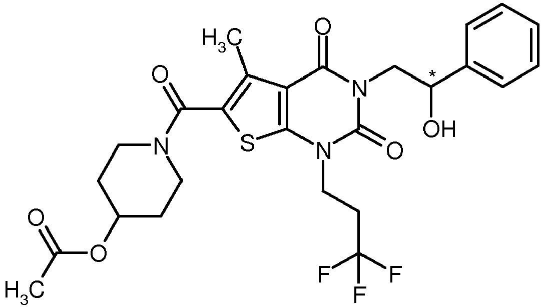

452 mg (0.796 mmol) der racemischen Verbindung aus Bsp. 124A wurden in 20 ml Ethanol/ Acetonitril (1:1) gelöst und in 40 Portionen mittels präparativer SFC-HPLC an chiraler Phase in die Enantiomere getrennt [Säule: Daicel Chiralpak IC, 5 µm, 250 mm x 20 mm; Eluent: Kohlendioxid/Methanol 60:40; Fluss: 80 ml/min; Temperatur: 40°C; Detektion: 210 nm]. Nach Einengen der Produktfraktionen und Trocknen des Rückstands im Hochvakuum wurden 210 mg (92% d. Th.) von Enantiomer 1 erhalten.

¹H-NMR (400 MHz, DMSO-d₆, δ/ppm): 7.38-7.31 (m, 4H), 7.28-7.23 (m, 1H), 5.39 (d, 1H), 4.96-4.89 (m, 2H), 4.23 (dd, 1H), 4.11 (t, 2H), 3.82 (dd, 1H), 3.79-3.71 (m, 2H), 3.47-3.37 (m, 2H), 2.80-2.66 (m, 2H), 2.39 (s, 3H), 2.03 (s, 3H), 1.93-1.85 (m, 2H), 1.61-1.52 (m, 2H).

Chirale analytische SFC [Säule: Daicel Chiralpak IC, 5 µm, 250 mm x 4.6 mm; Eluent: Kohlendioxid/Methanol 60:40; Fluss: 3 ml/min; Temperatur: 40°C; Detektion: 210 nm]: Rt = 6.53 min; 99.9% ee.

### Beispiel 126A

### 1-{[3-(2-Hydroxy-2-phenylethyl)-5-methyl-2,4-dioxo-1-(3,3,3-trifluorpropyl)-1,2,3,4-tetrahydro-thieno[2,3-d]pyrimidin-6-yl]carbonyl}piperidin-4-ylacetat (Enantiomer 2)

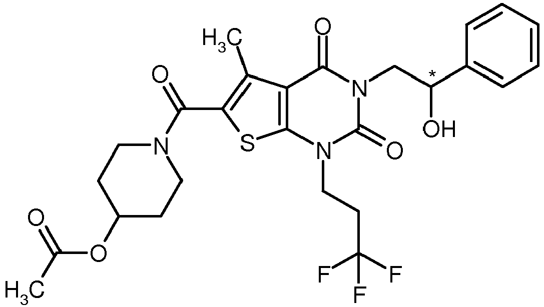

452 mg (0.796 mmol) der racemischen Verbindung aus Bsp. 124A wurden in 20 ml Ethanol/ Acetonitril (1:1) gelöst und in 40 Portionen mittels präparativer SFC-HPLC an chiraler Phase in die Enantiomere getrennt [Säule: Daicel Chiralpak IC, 5 µm, 250 mm x 20 mm; Eluent: Kohlendioxid/Methanol 60:40; Fluss: 80 ml/min; Temperatur: 40°C; Detektion: 210 nm]. Nach Einengen der Produktfraktionen und Trocknen des Rückstands im Hochvakuum wurden 206 mg (91% d. Th.) von Enantiomer 2 erhalten.

¹H-NMR (400 MHz, DMSO-d₆, δ/ppm): 7.38-7.31 (m, 4H), 7.28-7.23 (m, 1H), 5.39 (d, 1H), 4.96-4.89 (m, 2H), 4.23 (dd, 1H), 4.11 (t, 2H), 3.82 (dd, 1H), 3.79-3.71 (m, 2H), 3.47-3.37 (m, 2H), 2.79-2.66 (m, 2H), 2.39 (s, 3H), 2.03 (s, 3H), 1.93-1.85 (m, 2H), 1.61-1.52 (m, 2H).

Chirale analytische SFC [Säule: Daicel Chiralpak IC, 5 µm, 250 mm x 4.6 mm; Eluent: Kohlendioxid/Methanol 60:40; Fluss: 3 ml/min; Temperatur: 40°C; Detektion: 210 nm]: Rₜ = 5.04 min; 99.9% ee.

### Beispiel 127A

### 1-{[3-(2-Fluor-2-phenylethyl)-5-methyl-2,4-dioxo-1-(3,3,3-trifluorpropyl)-1,2,3,4-tetrahydro-thieno[2,3-d]pyrimidin-6-yl]carbonyl}piperidin-4-ylacetat (Enantiomer 1)

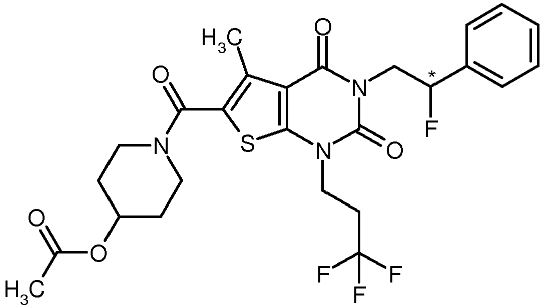

100 mg (0.176 mmol) der Verbindung aus Bsp. 125A wurden in 5 ml Dichlormethan gelöst und bei -20°C tropfenweise mit einer Lösung von 28 µl (0.211 mmol) Diethylaminoschwefeltrifluorid (DAST) in 0.5 ml Dichlormethan versetzt. Das Reaktionsgemisch wurde anschließend 3 h bei RT gerührt. Danach wurde noch einmal die gleiche Menge an DAST in Dichlormethan zugesetzt. Nach weiteren 4 h Rühren wurde mit halbgesättigter wässriger Natriumhydrogencarbonat-Lösung versetzt und mit Dichlormethan extrahiert. Der organische Extrakt wurde über wasserfreiem Magnesiumsulfat getrocknet, filtriert und eingeengt. Der Rückstand wurde mittels präparativer HPLC gereinigt (Methode 5). Nach Einengen der Produktfraktionen und Trocknen des Rückstands im Hochvakuum wurden 84 mg (83% d. Th.) der Titelverbindung erhalten.

¹H-NMR (400 MHz, DMSO-d₆, δ/ppm): 7.49-7.40 (m, 5H), 5.77 (ddd, 1H), 4.92 (m, 1H), 4.63 (m, 1H), 4.16 (t, 2H), 4.00 (ddd, 1H), 3.80-3.72 (m, 2H), 3.45-3.37 (m, 2H), 2.86-2.74 (m, 2H), 2.41 (s, 3H), 2.03 (s, 3H), 1.93-1.85 (m, 2H), 1.61-1.52 (m, 2H).

LC/MS (Methode 1, ESIpos): Rₜ = 1.11 min, m/z = 570 [M+H]⁺.

### Beispiel 128A

### 1-{[3-(2-Fluor-2-phenylethyl)-5-methyl-2,4-dioxo-1-(3,3,3-trifluorpropyl)-1,2,3,4-tetrahydro-thieno[2,3-d]pyrimidin-6-yl]carbonyl}piperidin-4-ylacetat (Enantiomer 2)

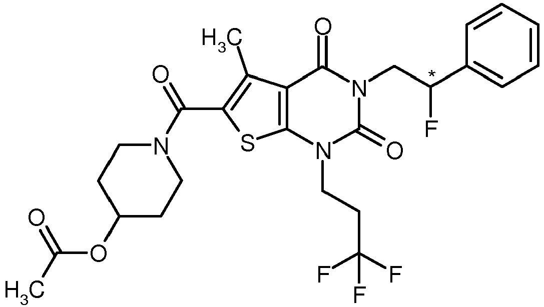

70 mg (0.123 mmol) der Verbindung aus Bsp. 126A wurden in 4 ml Dichlormethan gelöst und bei -20°C tropfenweise mit einer Lösung von 20 µl (0.148 mmol) Diethylaminoschwefeltrifluorid (DAST) in 0.5 ml Dichlormethan versetzt. Das Reaktionsgemisch wurde anschließend 1 h bei -20°C gerührt. Danach wurde noch einmal die gleiche Menge an DAST in Dichlormethan zugesetzt. Nach einer weiteren Stunde bei -20°C wurde das Kältebad entfernt und das Rühren bei RT fortgesetzt. Nach 6 h bei RT wurde mit halbgesättigter wässriger Natriumhydrogencarbonat-Lösung versetzt und mit Dichlormethan extrahiert. Der organische Extrakt wurde über wasserfreiem Magnesiumsulfat getrocknet, filtriert und eingeengt. Der Rückstand wurde mittels präparativer HPLC gereinigt (Methode 5). Nach Einengen der Produktfraktionen und Trocknen des Rückstands im Hochvakuum wurden 62 mg (88% d. Th.) der Titelverbindung erhalten.

¹H-NMR (400 MHz, DMSO-d₆, δ/ppm): 7.49-7.41 (m, 5H), 5.77 (ddd, 1H), 4.92 (m, 1H), 4.63 (m, 1H), 4.16 (t, 2H), 3.99 (ddd, 1H), 3.81-3.72 (m, 2H), 3.45-3.37 (m, 2H), 2.86-2.74 (m, 2H), 2.41 (s, 3H), 2.03 (s, 3H), 1.93-1.85 (m, 2H), 1.61-1.52 (m, 2H).

LC/MS (Methode 1, ESIpos): Rₜ = 1.11 min, m/z = 570 [M+H]⁺.

### Beispiel 129A

### 1-{[3-(2-Hydroxy-2-phenylpropyl)-5-methyl-2,4-dioxo-1-(3,3,3-trifluorpropyl)-1,2,3,4-tetrahydro-thieno[2,3-d]pyrimidin-6-yl]carbonyl}piperidin-4-ylacetat (Racemat)

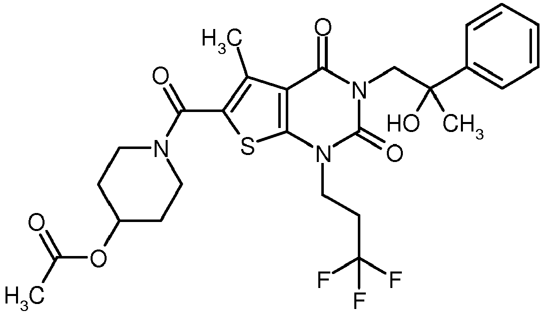

105 mg (0.186 mmol) der Verbindung aus Bsp. 121A wurden in 3 ml wasserfreiem THF gelöst und bei 0°C mit 186 µl (0.186 mmol) einer 1 M Lösung von Methylmagnesiumbromid in THF versetzt. Nach 3 h bei 0°C wurde noch einmal mit der gleichen Menge an Grignard-Reagenz versetzt. Nach weiteren 30 min wurde das Reaktionsgemisch mit ca. 0.5 ml gesättigter wässriger Ammoniumchlorid-Lösung versetzt, dann mit Ethylacetat verdünnt und schließlich mit soviel wasserfreiem Magnesiumsulfat versetzt, bis die wässrige Phase komplett aufgenommen war. Danach wurde abgesaugt und das Filtrat eingedampft. Der Filtrat-Rückstand wurde mittels präparativer HPLC gereinigt (Methode 5). Nach Vereinigen der Produktfraktionen, Einengen und Trocknen im Hochvakuum wurden 55 mg (50% d. Th.) der Titelverbindung erhalten.

¹H-NMR (400 MHz, DMSO-d₆, δ/ppm): 7.48 (d, 2H), 7.30 (t, 2H), 7.21 (t, 1H), 5.04 (s, 1H), 4.96-4.89 (m, 1H), 4.18 (quart, 2H), 4.12-4.03 (m, 2H), 3.79-3.72 (m, 2H), 3.44-3.37 (m, 2H), 2.76-2.64 (m, 2H), 2.36 (s, 3H), 2.03 (s, 3H), 1.93-1.85 (m, 2H), 1.61-1.52 (m, 2H), 1.42 (s, 3H).

LC/MS (Methode 1, ESIpos): Rₜ = 1.10 min, m/z = 582 [M+H]⁺.

### Beispiel 130A

### 1-{[3-Ethyl-5-methyl-1-(3-methylbutyl)-2,4-dioxo-1,2,3,4-tetrahydrothieno[2,3-d]pyrimidin-6-yl]-carbonyl}piperidin-4-yl-acetat

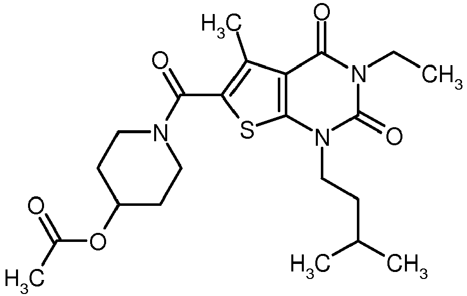

Analog zu dem unter Bsp. 83A beschriebenen Verfahren wurden aus 150 mg (0.395 mmol) der Verbindung aus Bsp. 62A und 117 mg (0.593 mmol) 1-Iod-3-methylbutan 150 mg (84% d. Th.) der Titelverbindung erhalten. Abweichend betrug die Reaktionszeit im Mikrowellenofen 60 min, und die Reinigung über präparative HPLC erfolgte nach Methode 6.

¹H-NMR (400 MHz, DMSO-d₆, δ/ppm): 4.91 (m, 1H), 3.92-3.87 (m, 2H), 3.91 (quart, 2H), 3.78-3.71 (m, 2H), 3.40 (m, 2H), 2.39 (s, 3H), 2.02 (s, 3H), 1.91-1.84 (m, 2H), 1.65 (sept, 1H), 1.60-1.54 (m, 4H), 1.12 (t, 3H), 0.95 (d, 6H).

LC/MS (Methode 1, ESIpos): Rₜ = 1.10 min, m/z = 450 [M+H]⁺.

### Beispiel 131A

### 1-{[3-Ethyl-5-methyl-2,4-dioxo-1-(4,4,4-trifluorbutyl)-1,2,3,4-tetrahydrothieno[2,3-d]pyrimidin-6-yl]carbonyl}piperidin-4-yl-acetat

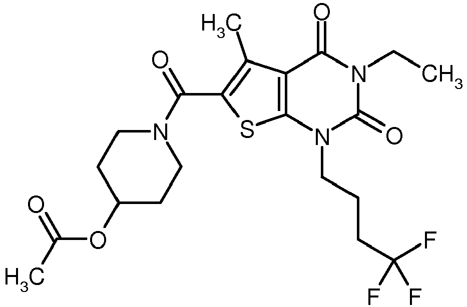

Analog zu dem unter Bsp. 83A beschriebenen Verfahren wurden aus 150 mg (0.395 mmol) der Verbindung aus Bsp. 62A und 141 mg (0.593 mmol) 4,4,4-Trifluor-1-iodbutan 163 mg (84% d. Th.) der Titelverbindung erhalten. Abweichend betrug die Reaktionszeit im Mikrowellenofen 60 min, und die Reinigung über präparative HPLC erfolgte nach Methode 6.

¹H-NMR (400 MHz, DMSO-d₆, δ/ppm): 4.92 (m, 1H), 3.97 (t, 2H), 3.90 (quart, 2H), 3.79-3.71 (m, 2H), 3.40 (m, 2H), 2.47-2.38 (m, 2H), 2.39 (s, 3H), 2.02 (s, 3H), 1.95-1.85 (m, 4H), 1.60-1.51 (m, 2H), 1.13 (t, 3H).

LC/MS (Methode 1, ESIpos): Rₜ = 1.01 min, m/z = 490 [M+H]⁺.

### Beispiel 132A

### 1-{[3-Ethyl-5-methyl-2,4-dioxo-1-(3,4,4-trifluorbut-3-en-1-yl)-1,2,3,4-tetrahydrothieno[2,3-d]-pyrimidin-6-yl]carbonyl}piperidin-4-yl-acetat

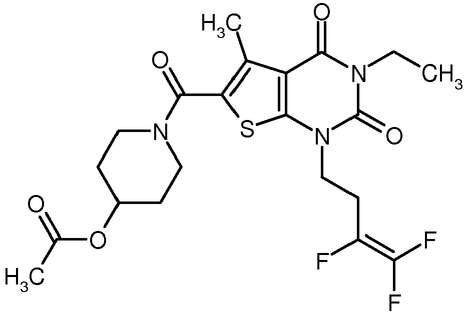

Analog zu dem unter Bsp. 83A beschriebenen Verfahren wurden aus 150 mg (0.395 mmol) der Verbindung aus Bsp. 62A und 140 mg (0.593 mmol) 1,1,2-Trifluor-4-iodbut-1-en 72 mg (37% d. Th.) der Titelverbindung erhalten. Abweichend betrug die Reaktionszeit im Mikrowellenofen 90 min, und die Reinigung über präparative HPLC erfolgte nach Methode 6.

¹H-NMR (400 MHz, DMSO-d₆, δ/ppm): 4.91 (m, 1H), 4.10 (t, 2H), 3.91 (quart, 2H), 3.78-3.70 (m, 2H), 3.39 (m, 2H), 2.82 (m, 2H), 2.39 (s, 3H), 2.02 (s, 3H), 1.91-1.84 (m, 2H), 1.60-1.51 (m, 2H), 1.12 (t, 3H).

LC/MS (Methode 1, ESIpos): Rₜ = 1.00 min, m/z = 488 [M+H]⁺.

### Beispiel 133A

### 6-Chlor-3-isobutylpyrimidin-2,4(1H,3H)-dion

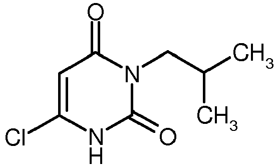

Bei einer Temperatur von 0°C wurden 18.8 ml (201 mmol) Phosphoroxychlorid vorsichtig zu 4 ml 50%-igem wässrigen Ethanol gegeben. Anschließend wurde, ebenfalls bei 0°C, portionsweise mit 4.15 g (22.6 mmol) 1-Isobutylpyrimidin-2,4,6(1*H*,3*H*,5*H*)-trion [Lit.: G. Brückmann, S. D. Isaacs, J. Am. Chem. Soc. 1949, 71 (2), 390-392] versetzt. Nach beendeter Zugabe wurde das Reaktionsgemisch zunächst 30 min auf 50°C und dann 2 h auf 100°C erwärmt. Nach Abkühlen auf RT wurde das Reaktionsgemisch auf ca. 80 ml Eiswasser gegossen. Der dabei ausgefallene Feststoff wurde abgesaugt und mit Wasser gewaschen. Nach Trocknen im Hochvakuum wurden 3.20 g (70% d. Th.) der Titelverbindung erhalten.

¹H-NMR (400 MHz, DMSO-d₆, δ/ppm): 12.33 (br. s, 1H), 5.89 (s, 1H), 3.56 (d, 2H), 1.99 (m, 1H), 0.83 (d, 6H).

LC/MS (Methode 1, ESIpos): Rₜ = 0.64 min, m/z = 203/205 [M+H]⁺.

### Beispiel 134A

### 2,2-Difluor-1-[1-isobutyl-2,6-dioxo-4-(pyridinium-1-yl)-1,6-dihydropyrimidin-5(2H)-yliden]-ethanolat

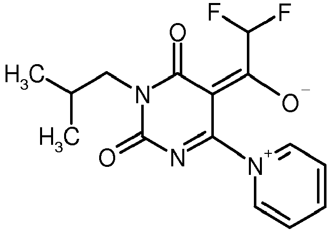

Eine Suspension von 3.2 g (15.8 mmol) der Verbindung aus Bsp. 133A in 35 ml Ethylacetat wurde bei RT mit 13 ml (158 mmol) Pyridin versetzt. Anschließend wurden langsam 7.9 ml (63.2 mmol) Difluoressigsäureanhydrid hinzugetropft. Nach beendetem Zutropfen wurde noch 1 h bei RT gerührt. Danach wurde der ausgefallene Feststoff abgesaugt, mit wenig Ethylacetat und Pentan gewaschen und im Hochvakuum getrocknet. Es wurden 2.30 g (45% d. Th.) der Titelverbindung erhalten.

¹H-NMR (400 MHz, DMSO-d₆, δ/ppm): 9.22 (d, 2H), 8.80 (t, 1H), 8.25 (t, 2H), 6.95 (t, 1H), 3.70 (d, 2H), 2.08 (m, 1H), 0.89 (d, 6H).

LC/MS (Methode 1, ESIpos): Rₜ = 0.66 min, m/z = 324 [M+H]⁺.

### Beispiel 135A

### Ethyl-5-(difluormethyl)-3-isobutyl-2,4-dioxo-1,2,3,4-tetrahydrothieno[2,3-d]pyrimidin-6-carboxylat

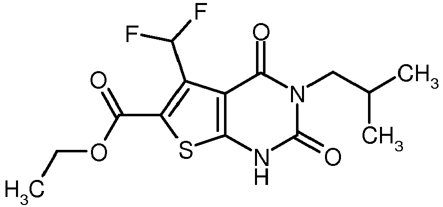

Eine Suspension von 1.11 g (3.45 mmol) der Verbindung aus Bsp. 134A in 14 ml Ethanol wurde mit 757 µl (6.90 mmol) Mercaptoessigsäureethylester versetzt und 5 min bei RT gerührt. Anschließend wurden 804 mg (7.59 mmol) Natriumcarbonat hinzugefügt und das Gemisch in einem Mikrowellenofen (Biotage Initiator mit dynamischer Steuerung der Einstrahlleistung) 2 h auf 125°C erhitzt. Zwei solcher Ansätze wurden danach vereinigt und am Rotationsverdampfer zur Trockene eingeengt. Der verbliebene Rückstand wurde in ca. 300 ml Wasser aufgenommen, durch Zugabe von Essigsäure schwach sauer gestellt und dreimal mit je ca. 100 ml Dichlormethan extrahiert. Der organische Extrakt wurde mit gesättigter wässriger Natriumchlorid-Lösung gewaschen, über wasserfreiem Magnesiumsulfat getrocknet, filtriert und eingeengt. Der Rückstand wurde in wenig Dichlormethan aufgeschlämmt und bei RT verrührt. Danach wurde der Feststoff abgesaugt und im Hochvakuum getrocknet. Auf diese Weise wurde eine erste Fraktion von 1.59 g der Titelverbindung erhalten. Das Filtrat wurde zur Trockene eingeengt und über eine Kieselgel-Kartusche chromatographiert (Biotage, 100 g Kieselgel, Laufmittel Cyclohexan/Ethylacetat 3:1 → 1:1). Nach Eindampfen der Produktfraktionen und Trocknen des Rückstands im Hochvakuum wurden 355 mg einer zweiten Fraktion der Titelverbindung gewonnen. Insgesamt wurden so 1.94 g (81% d. Th.) der Titelverbindung erhalten.

¹H-NMR (400 MHz, DMSO-d₆, δ/ppm): 12.59 (s, 1H), 7.70 (t, 1H), 4.32 (quart, 2H), 3.68 (d, 2H), 2.03 (m, 1H), 1.30 (t, 3H), 0.86 (d, 6H).

LC/MS (Methode 1, ESIpos): Rₜ = 0.98 min, m/z = 347 [M+H]⁺.

### Beispiel 136A

### Ethyl-5-(difluormethyl)-3-isobutyl-2,4-dioxo-1-[2-(trifluormethoxy)ethyl]-1,2,3,4-tetrahydro-thieno[2,3-d]pyrimidin-6-carboxylat

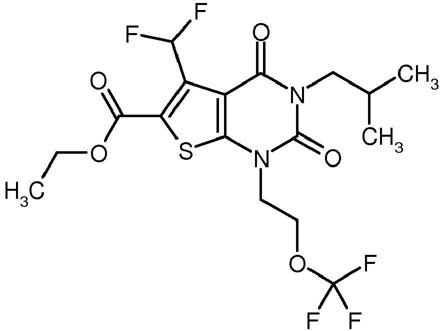

Eine Lösung von 600 mg (1.73 mmol) der Verbindung aus Bsp. 135A in 9 ml DMF wurde mit 847 mg (2.60 mmol) Cäsiumcarbonat versetzt und 20 min bei RT gerührt. Anschließend wurden 501 mg (2.60 mmol) 1-Brom-2-(trifluormethoxy)ethan [kommerziell erhältlich; Lit.: P.E. Aldrich, W.A. Sheppard, J. Org. Chem. 1964, 29 (1), 11-15] hinzugefügt, und das Gemisch wurde in einem Mikrowellenofen (Biotage Initiator mit dynamischer Steuerung der Einstrahlleistung) 1 h lang auf 100°C erhitzt. Nach dem Abkühlen auf RT wurde mit ca. 80 ml Ethylacetat verdünnt und nacheinander zweimal mit je ca. 80 ml Wasser und einmal mit ca. 80 ml gesättigter Natriumchlorid-Lösung gewaschen. Nach Trocknen über wasserfreiem Magnesiumsulfat wurde filtriert und eingeengt. Das Rohprodukt wurde mittels präparativer HPLC gereinigt (Methode 5). Die Produktfraktionen wurden vereinigt und eingeengt und der Rückstand im Hochvakuum getrocknet. Es wurden 553 mg (69% d. Th.) der Titelverbindung erhalten.

¹H-NMR (400 MHz, DMSO-d₆, δ/ppm): 7.72 (t, 1H), 4.42 (t, 2H), 4.35 (quart, 2H), 4.32 (t, 2H), 3.74 (d, 2H), 2.03 (m, 1H), 1.31 (t, 3H), 0.86 (d, 6H).

LC/MS (Methode 1, ESIpos): Rₜ = 1.23 min, m/z = 459 [M+H]⁺.

### Beispiel 137A

### Ethyl-5-(difluormethyl)-3-isobutyl-2,4-dioxo-1-(3,3,3-trifluorpropyl)-1,2,3,4-tetrahydrothieno-[2,3-d]pyrimidin-6-carboxylat

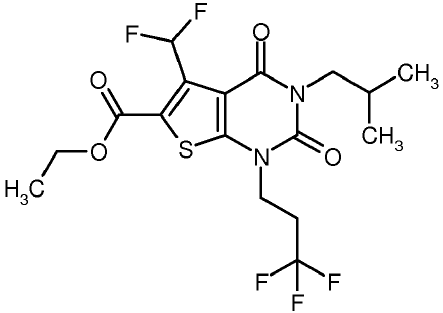

Analog zu dem unter Bsp. 136A beschriebenen Verfahren wurden aus 600 mg (1.73 mmol) der Verbindung aus Bsp. 135A und 582 mg (2.60 mmol) 3,3,3-Trifluor-1-iodpropan 570 mg (74% d. Th.) der Titelverbindung erhalten. Die Reinigung über präparative HPLC erfolgte hier nach Methode 6.

¹H-NMR (400 MHz, DMSO-d₆, δ/ppm): 7.73 (t, 1H), 4.36 (quart, 2H), 4.21 (t, 2H), 3.73 (d, 2H), 2.81 (m, 2H), 2.03 (m, 1H), 1.32 (t, 3H), 0.87 (d, 6H).

LC/MS (Methode 1, ESIpos): Rₜ = 1.22 min, m/z = 443 [M+H]⁺.

### Beispiel 138A

### 5-(Difluormethyl)-3-isobutyl-2,4-dioxo-1-[2-(trifluormethoxy)ethyl]-1,2,3,4-tetrahydrothieno-[2,3-d]pyrimidin-6-carbonsäure

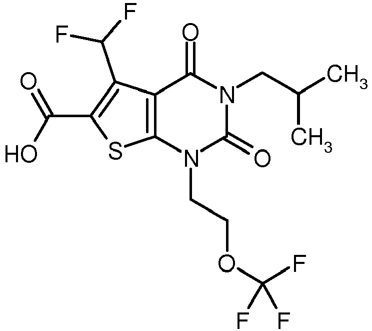

Eine Lösung von 530 mg (1.16 mmol) der Verbindung aus Bsp. 136A in 12 ml Ethanol wurde mit 1.73 ml (1.73 mmol) einer 1 M Lösung von Lithiumhydroxid in Wasser versetzt und 3 h bei RT gerührt. Anschließend wurden alle flüchtigen Bestandteile am Rotationsverdampfer entfernt. Der verbliebene Rückstand wurde in Wasser aufgenommen und mit ca. 1.5 ml 1 M Salzäure angesäuert. Dabei fiel das Produkt aus, das abgesaugt, mit Wasser gewaschen und im Hochvakuum getrocknet wurde. Es wurden 466 mg (93% d. Th.) der Titelverbindung erhalten.

¹H-NMR (400 MHz, DMSO-d₆, δ/ppm): 14.43 (sehr breit, ca. 1H), 7.76 (t, 1H), 4.42 (t, 2H), 4.30 (t, 2H), 3.74 (d, 2H), 2.03 (m, 1H), 0.86 (d, 6H).

LC/MS (Methode 1, ESIpos): Rₜ = 0.99 min, m/z = 431 [M+H]⁺.

### Beispiel 139A

### 5-(Difluormethyl)-3-isobutyl-2,4-dioxo-1-(3,3,3-trifluorpropyl)-1,2,3,4-tetrahydrothieno[2,3-d]-pyrimidin-6-carbonsäure

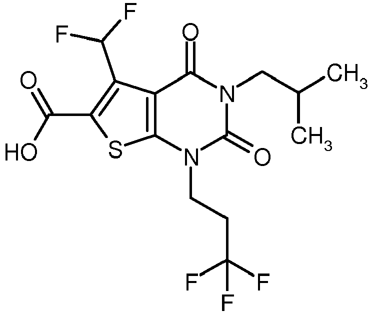

Analog zu dem unter Bsp. 138A beschriebenen Verfahren wurden aus 540 mg (1.22 mmol) der Verbindung aus Bsp. 137A 463 mg (91% d. Th.) der Titelverbindung erhalten.

¹H-NMR (400 MHz, DMSO-d₆, δ/ppm): 14.39 (sehr breit, ca. 1H), 7.77 (t, 1H), 4.19 (t, 2H), 3.73 (d, 2H), 2.81 (m, 2H), 2.03 (m, 1H), 0.86 (d, 6H).

LC/MS (Methode 1, ESIpos): Rₜ = 0.98 min, m/z = 415 [M+H]⁺.

### Beispiel 140A

### tert.-Butyl-5-(brommethyl)-2,4-dioxo-3-propyl-1-(3,3,3-trifluorpropyl)-1,2,3,4-tetrahydrothieno-[2,3-d]pyrimidin-6-carboxylat

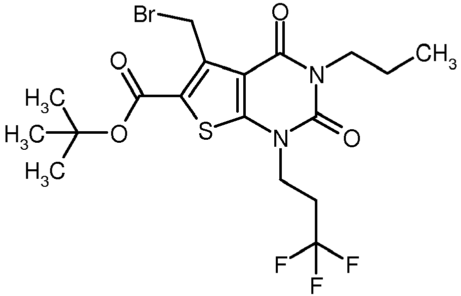

200 mg (0.476 mmol) der Verbindung aus Bsp. 98A, 178 mg (0.999 mmol) *N*-Bromsuccinimid (NBS) und 7.8 mg (0.048 mmol) 2,2'-Azobis(2-methylpropionitril) (AIBN) wurden in 5 ml wasserfreiem Acetonitril ca. 16 h unter Rückfluss erhitzt. Danach wurden alle flüchtigen Bestandteile am Rotationsverdampfer weitestgehend entfernt. Der verbliebene Rückstand wurde mittels Saugfiltration über Kieselgel mit Dichlormethan als Laufmittel gereinigt. Nach Vereinigen der Produktfraktionen, Einengen und Trocknen im Hochvakuum wurden 213 mg (89% d. Th.) der Titelverbindung erhalten.

¹H-NMR (400 MHz, DMSO-d₆, δ/ppm): 5.21 (s, 2H), 4.17 (t, 2H), 3.85 (t, 2H), 2.87-2.75 (m, 2H), 1.63-1.53 (m, 2H), 1.57 (s, 9H), 0.88 (t, 3H).

LC/MS (Methode 1, ESIpos): Rₜ = 1.34 min, m/z = 499/501 [M+H]⁺.

### Beispiel 141A

### tert.-Butyl-5-(brommethyl)-3-ethyl-2,4-dioxo-1-(3,3,3-trifluorpropyl)-1,2,3,4-tetrahydrothieno-[2,3-d]pyrimidin-6-carboxylat

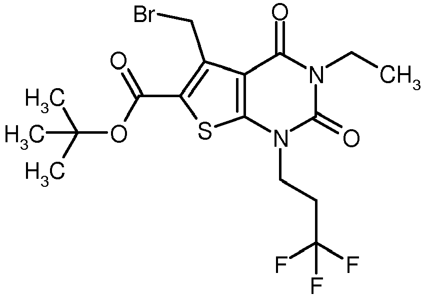

Analog zu dem unter Bsp. 140A beschriebenen Verfahren wurden aus 4.40 g (10.8 mmol) der Verbindung aus Bsp. 35A, 2.02 g (11.4 mmol) *N*-Bromsuccinimid (NBS) und 89 mg (0.541 mmol) 2,2'-Azobis(2-methylpropionitril) (AIBN) 4.56 g (82% d. Th., 95% Reinheit) der Titelverbindung erhalten.

¹H-NMR (400 MHz, DMSO-d₆, δ/ppm): 5.21 (s, 2H), 4.17 (t, 2H), 3.93 (quart, 2H), 2.88-2.76 (m, 2H), 1.57 (s, 9H), 1.15 (t, 3H).

LC/MS (Methode 1, ESIpos): Rₜ = 1.31 min; keine Ionisierung.

### Beispiel 142A

### tert.-Butyl-5-formyl-2,4-dioxo-3-propyl-1-(3,3,3-trifluorpropyl)-1,2,3,4-tetrahydrothieno[2,3-d]-pyrimidin-6-carboxylat

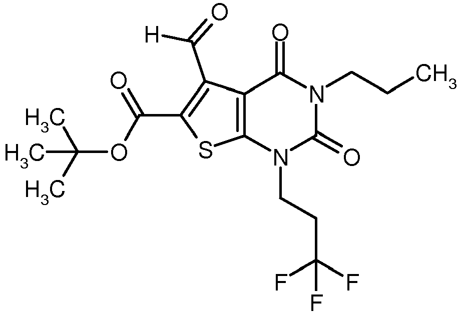

Eine Lösung von 385 mg (0.771 mmol) der Verbindung aus Bsp. 140A in 5 ml wasserfreiem Acetonitril wurde mit 385 mg Molekularsieb (4A) und, bei einer Temperatur von -10°C, portionsweise mit insgesamt 181 mg (1.54 mmol) *N*-Methylmorpholin-*N*-oxid (NMO) versetzt. Das Reaktionsgemisch wurde anschließend 4 h bei RT gerührt. Dann wurde das Gemisch über wenig Kieselgur filtriert und das Filtrat zur Trockene eingeengt. Der erhaltene Rückstand wurde in 20 ml Diisopropylether/Ethylacetat (1:1) aufgenommen und nacheinander zweimal mit je 10 ml wässriger Zitronensäure-Lösung und einmal mit 10 ml gesättigter wässriger Natriumchlorid-Lösung gewaschen. Nach Trocknen über wasserfreiem Magnesiumsulfat wurde filtriert und eingeengt. Trocknen des Rückstands im Hochvakuum ergab 247 mg (73% d. Th.) der Titelverbindung.

¹H-NMR (400 MHz, DMSO-d₆, δ/ppm): 10.36 (s, 1H), 4.19 (t, 2H), 3.81 (t, 2H), 2.88-2.76 (m, 2H), 1.61-1.51 (m, 2H), 1.50 (s, 9H), 0.86 (t, 3H).

LC/MS (Methode 1, ESIpos): Rₜ = 1.22 min, m/z = 435 [M+H]⁺.

### Beispiel 143A

### tert.-Butyl-3-ethyl-5-formyl-2,4-dioxo-1-(3,3,3-trifluorpropyl)-1,2,3,4-tetrahydrothieno[2,3-d]-pyrimidin-6-carboxylat

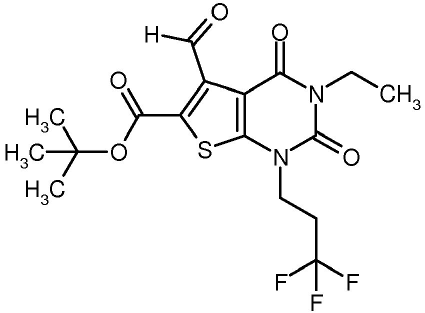

Analog zu dem unter Bsp. 142A beschriebenen Verfahren wurden aus 3.75 g (7.73 mmol) der Verbindung aus Bsp. 141A und 1.81 g (15.4 mmol) *N*-Methylmorpholin-*N*-oxid (NMO) 2.38 g (73% d. Th.) der Titelverbindung erhalten. Die Reaktionszeit betrug in diesem Fall 5 h.

¹H-NMR (400 MHz, DMSO-d₆, δ/ppm): 10.36 (s, 1H), 4.19 (t, 2H), 3.88 (quart, 2H), 2.88-2.76 (m, 2H), 1.50 (s, 9H), 1.12 (t, 3H).

LC/MS (Methode 1, ESIpos): Rₜ = 1.15 min, m/z = 421 [M+H]⁺.

### Beispiel 144A

### tert.-Butyl-5-(difluormethyl)-2,4-dioxo-3-propyl-1-(3,3,3-trifluorpropyl)-1,2,3,4-tetrahydrothieno-[2,3-d]pyrimidin-6-carboxylat

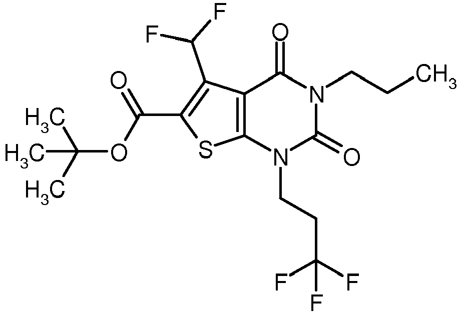

Eine Lösung von 235 mg (0.541 mmol) der Verbindung aus Bsp. 142A in 5 ml Dichlormethan wurde bei 0°C mit 218 mg (1.35 mmol) *N,N*-Diethylaminoschwefeltrifluorid (DAST) versetzt. Anschließend wurde 3 h bei RT gerührt. Danach wurde das Reaktionsgemisch mit 0.5 ml gesättigter wässriger Natriumhydrogencarbonat-Lösung versetzt und wenige Minuten bei RT gerührt. Dann wurde mit 5 ml Ethylacetat verdünnt und anschließend so viel festes wasserfreies Magnesiumsulfat zugesetzt, wie nötig war, um die wässrige Phase komplett aufzunehmen. Es wurde filtriert, der Rückstand wurde mit etwas Ethylacetat nachgewaschen und das Filtrat eingeengt. Der erhaltene Feststoff wurde mittels präparativer HPLC gereinigt (Methode 6). Nach Einengen der Produktfraktionen und Trocknen im Hochvakuum wurden 198 mg (80% d. Th.) der Titelverbindung erhalten.

¹H-NMR (400 MHz, DMSO-d₆, δ/ppm): 7.71 (t, 1H), 4.19 (t, 2H), 3.84 (t, 2H), 2.87-2.75 (m, 2H), 1.62-1.53 (m, 2H), 1.55 (s, 9H), 0.88 (t, 3H).

LC/MS (Methode 1, ESIpos): Rₜ = 1.32 min, m/z = 457 [M+H]⁺.

### Beispiel 145A

### tert.-Butyl-3-ethyl-5-(fluormethyl)-2,4-dioxo-1-(3,3,3-trifluorpropyl)-1,2,3,4-tetrahydrothieno-[2,3-d]pyrimidin-6-carboxylat

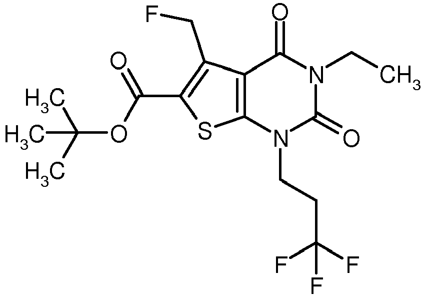

Eine Lösung von 800 mg (1.65 mmol) der Verbindung aus Bsp. 141A in 16 ml wasserfreiem Acetonitril wurde mit 400 mg Molekularsieb (4A) und 2 ml (1.98 mmol) einer 1 M Lösung von Tetra-*n*-butylammoniumfluorid (TBAF) in THF versetzt. Nachdem das Gemisch ca. 16 h bei RT gerührt worden war, wurde filtriert und das Filtrat zur Trockene eingeengt. Der erhaltene Rückstand wurde mittels präparativer HPLC gereinigt (Methode 18). Nach Einengen der Produktfraktionen und Trocknen im Hochvakuum wurden 450 mg (60% d. Th., 94% Reinheit) der Titelverbindung erhalten.

¹H-NMR (400 MHz, DMSO-d₆, δ/ppm): 5.93 (d, 2H), 4.19 (t, 2H), 3.93 (quart, 2H), 2.88-2.76 (m, 2H), 1.55 (s, 9H), 1.14 (t, 3H).

LC/MS (Methode 1, ESIpos): Rₜ = 1.21 min, m/z = 425 [M+H]⁺.

### Beispiel 146A

### tert.-Butyl-3-ethyl-5-(1-hydroxyethyl)-2,4-dioxo-1-(3,3,3-trifluorpropyl)-1,2,3,4-tetrahydrothieno-[2,3-d]pyrimidin-6-carboxylat (Racemat)

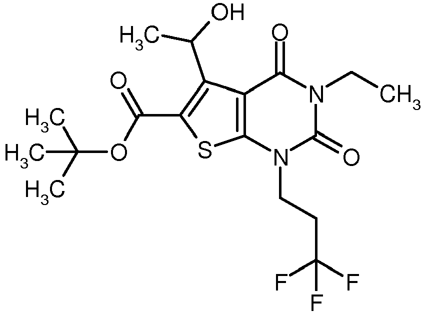

Eine Lösung von 1.0 g (2.38 mmol) der Verbindung aus Bsp. 143A in 24 ml wasserfreiem THF wurde bei 0°C tropfenweise mit 1.9 ml einer 1.4 M Lösung von Methylmagnesiumbromid in einem THF/Toluol-Gemisch versetzt. Nachdem das Reaktionsgemisch 1 h bei 0°C gerührt worden war, wurden bei dieser Temperatur 5 ml Wasser hinzugefügt. Man ließ das Gemisch auf RT kommen und extrahierte dann mit Ethylacetat. Der organische Extrakt wurde nacheinander mit Wasser und gesättigter Natriumchlorid-Lösung gewaschen. Nach Trocknen über wasserfreiem Natriumsulfat wurde filtriert und eingeengt. Der erhaltene Rückstand wurde mittels präparativer HPLC gereinigt (Methode 18). Die Produktfraktionen wurden vereinigt und eingeengt und der Rückstand im Hochvakuum getrocknet. Es wurden 480 mg (46% d. Th.) der Titelverbindung erhalten.

¹H-NMR (400 MHz, DMSO-d₆, δ/ppm): 5.76 (breit, 1H), 4.18 (t, 2H), 3.97 (quart, 2H), 2.88-2.76 (m, 2H), 1.54 (s, 9H), 1.41 (d, 3H), 1.16 (t, 3H).

LC/MS (Methode 1, ESIpos): Rₜ = 1.22 min, m/z = 437 [M+H]⁺.

### Beispiel 147A

### tert.-Butyl-3-ethyl-5-(1-fluorethyl)-2,4-dioxo-1-(3,3,3-trifluorpropyl)-1,2,3,4-tetrahydrothieno-[2,3-d]pyrimidin-6-carboxylat (Racemat)

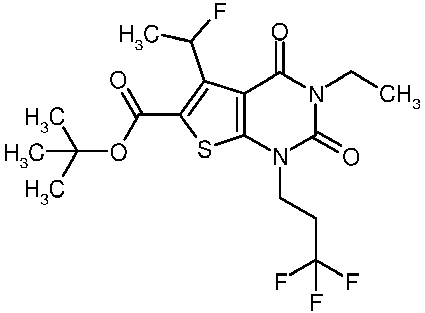

Eine Lösung von 400 mg (0.916 mmol) der Verbindung aus Bsp. 146A in 9.2 ml Dichlormethan wurde bei 0°C mit 405 mg (0.916 mmol) einer 50%-igen Lösung von Bis(2-methoxyethyl)amino-schwefeltrifluorid (Deoxo-Fluor®) in THF versetzt. Nach 30 min Rühren bei RT wurden weitere 203 mg (0.458 mmol) Deoxo-Fluor®-Lösung hinzugefügt. Nach weiteren 30 min wurde mit gesättigter wässriger Natriumhydrogencarbonat-Lösung versetzt und mit Ethylacetat extrahiert. Der organische Extrakt wurde mit Wasser gewaschen und über wasserfreiem Natriumsulfat getrocknet. Nach Filtration, Einengen und Trocknen im Hochvakuum wurden 430 mg (90% d. Th., 85% Reinheit) der Titelverbindung erhalten.

LC/MS (Methode 1, ESIpos): Rₜ = 1.28 min, m/z = 439 [M+H]⁺.

### Beispiel 148A

### 5-(Difluormethyl)-2,4-dioxo-3-propyl-1-(3,3,3-trifluorpropyl)-1,2,3,4-tetrahydrothieno[2,3-d]-pyrimidin-6-carbonsäure

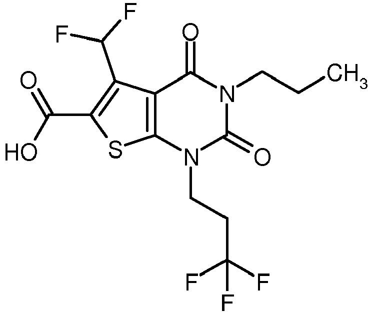

Eine Lösung von 190 mg (0.416 mmol) der Verbindung aus Bsp. 144A in 2 ml Dichlormethan wurde mit 1 ml Trifluoressigsäure versetzt und 3 h bei RT gerührt. Danach wurde das Reaktionsgemisch am Rotationsverdampfer zur Trockene eingeengt. Der verbliebene Rückstand wurde zweimal in je 5 ml Dichlormethan gelöst und jeweils wieder eingeengt. Nach Trocknen im Hochvakuum wurden 166 mg (97% d. Th., 97% Reinheit) der Titelverbindung erhalten.

¹H-NMR (400 MHz, DMSO-d₆, δ/ppm): 14.43 (sehr breit, ca. 1H), 7.76 (t, 1H), 4.19 (t, 2H), 3.84 (t, 2H), 2.87-2.75 (m, 2H), 1.62-1.53 (m, 2H), 0.88 (t, 3H).

LC/MS (Methode 1, ESIpos): Rₜ = 0.91 min, m/z = 401 [M+H]⁺.

### Beispiel 149A

### 3-Ethyl-5-(fluormethyl)-2,4-dioxo-1-(3,3,3-trifluorpropyl)-1,2,3,4-tetrahydrothieno[2,3-d]-pyrimidin-6-carbonsäure

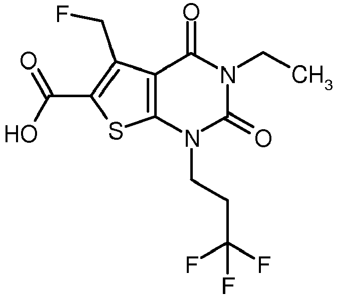

Analog zu dem unter Bsp. 148A beschriebenen Verfahren wurden aus 450 mg (1.06 mmol) der Verbindung aus Bsp. 145A 390 mg (92% d. Th., 93% Reinheit) der Titelverbindung erhalten.

¹H-NMR (400 MHz, DMSO-d₆, δ/ppm): 14.04 (sehr breit, ca. 1H), 5.95 (d, 2H), 4.18 (t, 2H), 3.93 (quart, 2H), 2.87-2.75 (m, 2H), 1.14 (t, 3H).

LC/MS (Methode 1, ESIpos): Rₜ = 0.86 min, m/z = 369 [M+H]⁺.

### Beispiel 150A

### 3-Ethyl-5-(1-fluorethyl)-2,4-dioxo-1-(3,3,3-trifluorpropyl)-1,2,3,4-tetrahydrothieno[2,3-d]-pyrimidin-6-carbonsäure (Racemat)

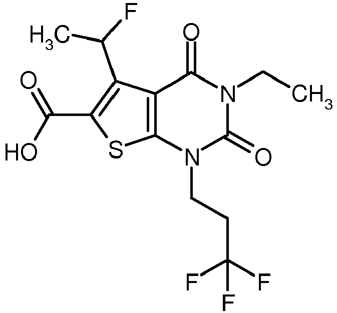

Analog zu dem unter Bsp. 148A beschriebenen Verfahren wurden aus 430 mg (0.834 mmol, 85% Reinheit) der Verbindung aus Bsp. 147A 290 mg (77% d. Th., 85% Reinheit) der Titelverbindung erhalten.

¹H-NMR (400 MHz, DMSO-d₆, δ/ppm): 13.91 (sehr breit, ca. 1H), 6.75 (d von quart, 1H), 4.17 (t, 2H), 3.92 (quart, 2H), 2.87-2.75 (m, 2H), 1.73 (dd, 3H), 1.14 (t, 3H).

LC/MS (Methode 1, ESIpos): Rₜ = 0.89 min, m/z = 383 [M+H]⁺.

### Beispiel 151A

### tert.-Butyl-5-(brommethyl)-2,4-dioxo-3-propyl-1-[2-(trifluormethoxy)ethyl]-1,2,3,4-tetrahydro-thieno[2,3-d]pyrimidin-6-carboxylat

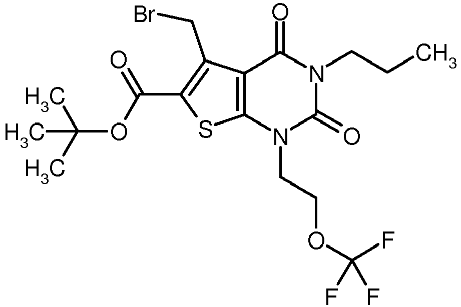

Analog zu dem unter Bsp. 140A beschriebenen Verfahren wurden aus 2.0 g (4.58 mmol) der Verbindung aus Bsp. 95A, 856 mg (4.81 mmol) *N*-Bromsuccinimid (NBS) und 38 mg (0.229 mmol) 2,2'-Azobis(2-methylpropionitril) (AIBN) 1.90 g (80% d. Th.) der Titelverbindung erhalten. Die Reaktionszeit betrug in diesem Fall 1 h.

¹H-NMR (400 MHz, DMSO-d₆, δ/ppm): 5.20 (s, 2H), 4.42 (t, 2H), 4.27 (t, 2H), 3.86 (t, 2H), 1.63-1.54 (m, 2H), 1.56 (s, 9H), 0.88 (t, 3H).

LC/MS (Methode 1, ESIpos): Rₜ = 1.37 min, m/z = 515/517 [M+H]⁺.

### Beispiel 152A

### tert.-Butyl-5-formyl-2,4-dioxo-3-propyl-1-[2-(trifluormethoxy)ethyl]-1,2,3,4-tetrahydrothieno-[2,3-d]pyrimidin-6-carboxylat

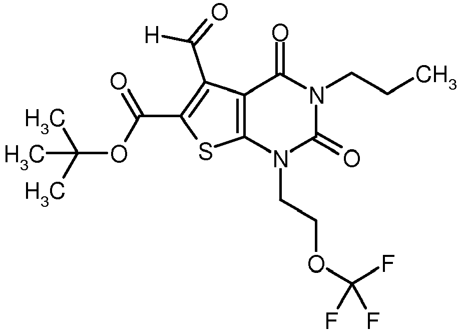

Analog zu dem unter Bsp. 142A beschriebenen Verfahren wurden aus 1.80 g (3.49 mmol) der Verbindung aus Bsp. 151A und 818 mg (6.99 mmol) *N*-Methylmorpholin-*N*-oxid (NMO) 1.17 g (74% d. Th.) der Titelverbindung erhalten. Die Reaktionszeit betrug in diesem Fall 5 h.

¹H-NMR (400 MHz, DMSO-d₆, δ/ppm): 10.35 (s, 1H), 4.43 (t, 2H), 4.29 (t, 2H), 3.81 (t, 2H), 1.61-1.51 (m, 2H), 1.50 (s, 9H), 0.85 (t, 3H).

LC/MS (Methode 1, ESIpos): Rₜ = 1.21 min, m/z = 451 [M+H]⁺.

### Beispiel 153A

### tert.-Butyl-5-(difluormethyl)-2,4-dioxo-3-propyl-1-[2-(trifluormethoxy)ethyl]-1,2,3,4-tetrahydro-thieno[2,3-d]pyrimidin-6-carboxylat

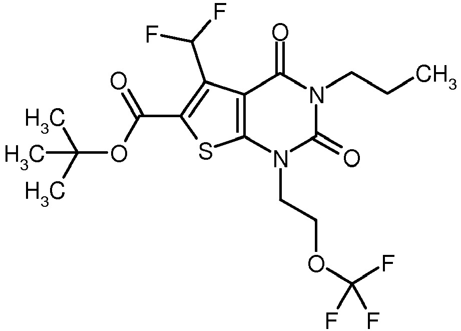

Analog zu dem unter Bsp. 144A beschriebenen Verfahren wurden aus 1.11 g (2.47 mmol) der Verbindung aus Bsp. 152A und 998 mg (6.19 mmol) *N,N*-Diethylaminoschwefeltrifluorid (DAST) 935 mg (79% d. Th.) der Titelverbindung erhalten. Die Reinigung des Produktes erfolgte hier mittels Chromatographie über eine Kieselgel-Kartusche (Biotage, 50 g Kieselgel, Laufmittel: Cyclohexan/Ethylacetat 5:1).

¹H-NMR (400 MHz, DMSO-d₆, δ/ppm): 7.71 (t, 1H), 4.42 (t, 2H), 4.30 (t, 2H), 3.85 (t, 2H), 1.62-1.52 (m, 2H), 1.54 (s, 9H), 0.87 (t, 3H).

LC/MS (Methode 1, ESIpos): Rₜ = 1.29 min, m/z = 473 [M+H]⁺.

### Beispiel 154A

### 5-(Difluormethyl)-2,4-dioxo-3-propyl-1-[2-(trifluormethoxy)ethyl]-1,2,3,4-tetrahydrothieno[2,3-d]-pyrimidin-6-carbonsäure

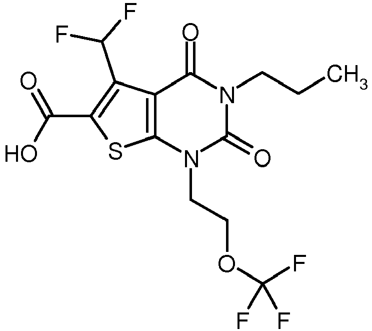

Analog zu dem unter Bsp. 148A beschriebenen Verfahren wurden aus 916 mg (1.94 mmol) der Verbindung aus Bsp. 153A 806 mg (98% d. Th.) der Titelverbindung erhalten.

¹H-NMR (400 MHz, DMSO-d₆, δ/ppm): 14.34 (sehr breit, 1H), 7.76 (t, 1H), 4.42 (t, 2H), 4.30 (t, 2H), 3.85 (t, 2H), 1.62-1.53 (m, 2H), 0.87 (t, 3H).

LC/MS (Methode 1, ESIpos): Rₜ = 0.88 min, m/z = 417 [M+H]⁺.

### Beispiel 155A

### tert.-Butyl-1-ethyl-5-methyl-2,4-dioxo-3-propyl-1,2,3,4-tetrahydrothieno[2,3-d]pyrimidin-6-carboxylat

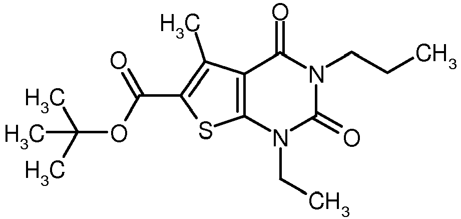

Eine Lösung von 3.0 g (9.25 mmol) der Verbindung aus Bsp. 91A in 36 ml DMF wurde mit 4.52 g (13.9 mmol) Cäsiumcarbonat versetzt und 10 min bei RT gerührt. Anschließend wurden 1.1 ml (13.9 mmol) Iodethan hinzugefügt, und das Gemisch wurde 1 h bei 100°C in einem Mikrowellenofen (Biotage Initiator mit dynamischer Steuerung der Einstrahlleistung) gerührt. Nach dem Abkühlen auf RT wurde mit Ethylacetat verdünnt und nacheinander mit Wasser und gesättigter Natriumchlorid-Lösung gewaschen. Nach Trocknen über wasserfreiem Magnesiumsulfat wurde filtriert und eingeengt. Das Rohprodukt wurde mittels MPLC gereinigt (Biotage-Kartusche mit 100 g Kieselgel, Laufmittel: Cyclohexan/Ethylacetat 20:1). Die Produktfraktionen wurden vereinigt und eingeengt. Nach Trocknen des Rückstands im Hochvakuum wurden 3.06 g (93% d. Th.) der Titelverbindung erhalten.

¹H-NMR (400 MHz, DMSO-d₆, δ/ppm): 3.94 (quart, 2H), 3.82 (m, 2H), 2.75 (s, 3H), 1.61-1.51 (m, 2H), 1.53 (s, 9H), 1.25 (t, 3H), 0.87 (t, 3H).

LC/MS (Methode 1, ESIpos): Rₜ = 1.34 min, m/z = 353 [M+H]⁺.

### Beispiel 156A

### tert.-Butyl-5-(brommethyl)-1-ethyl-2,4-dioxo-3-propyl-1,2,3,4-tetrahydrothieno[2,3-d]pyrimidin-6-carboxylat

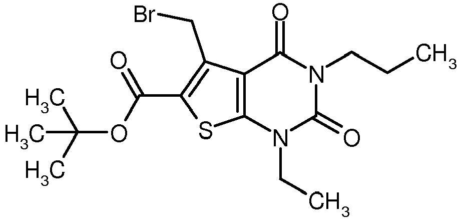

Analog zu dem unter Bsp. 140A beschriebenen Verfahren wurden aus 2.0 g (5.67 mmol) der Verbindung aus Bsp. 155A, 1.06 g (5.96 mmol) *N*-Bromsuccinimid (NBS) und 47 mg (0.284 mmol) 2,2'-Azobis(2-methylpropionitril) (AIBN) 2.22 g (90% d. Th.) der Titelverbindung erhalten. Die Reaktionszeit betrug in diesem Fall 1 h.

¹H-NMR (400 MHz, DMSO-d₆, δ/ppm): 5.21 (s, 2H), 3.96 (quart, 2H), 3.84 (m, 2H), 1.63-1.53 (m, 2H), 1.57 (s, 9H), 1.26 (t, 3H), 0.89 (t, 3H).

LC/MS (Methode 1, ESIpos): Rₜ = 1.38 min, m/z = 431/433 [M+H]⁺.

### Beispiel 157A

### tert.-Butyl-1-ethyl-5-formyl-2,4-dioxo-3-propyl-1,2,3,4-tetrahydrothieno[2,3-d]pyrimidin-6-carboxylat

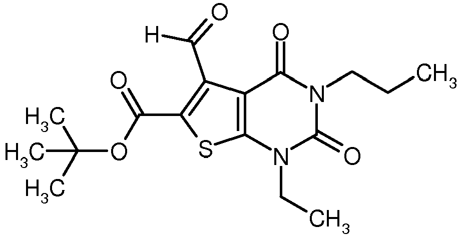

Analog zu dem unter Bsp. 142A beschriebenen Verfahren wurden aus 2.15 g (4.98 mmol) der Verbindung aus Bsp. 156A und 1.17 g (9.97 mmol) *N*-Methylmorpholin-*N*-oxid (NMO) 1.38 g (75% d. Th.) der Titelverbindung erhalten. Die Reaktionszeit betrug hier ca. 16 h.

¹H-NMR (400 MHz, DMSO-d₆, δ/ppm): 10.35 (s, 1H), 3.97 (quart, 2H), 3.80 (m, 2H), 1.62-1.51 (m, 2H), 1.50 (s, 9H), 1.27 (t, 3H), 0.86 (t, 3H).

LC/MS (Methode 1, ESIpos): Rₜ = 1.16 min, m/z = 367 [M+H]⁺.

### Beispiel 158A

### tert.-Butyl-5-(difluormethyl)-1-ethyl-2,4-dioxo-3-propyl-1,2,3,4-tetrahydrothieno[2,3-d]pyrimidin-6-carboxylat

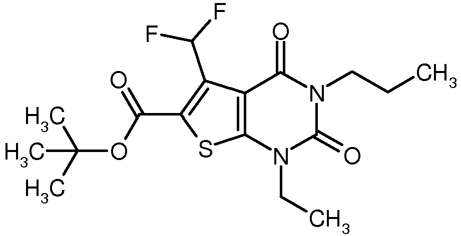

Analog zu dem unter Bsp. 144A beschriebenen Verfahren wurden aus 1.32 g (3.60 mmol) der Verbindung aus Bsp. 157A und 1.45 g (9.01 mmol) *N,N*-Diethylaminoschwefeltrifluorid (DAST) 1.08 g (77% d. Th.) der Titelverbindung erhalten. Die Reinigung des Produkts erfolgte hier mittels Chromatographie über eine Kieselgel-Kartusche (Biotage, 100 g Kieselgel, Laufmittel: Cyclohexan/Ethylacetat 5:1).

¹H-NMR (400 MHz, DMSO-d₆, δ/ppm): 7.72 (t, 1H), 3.98 (quart, 2H), 3.83 (m, 2H), 1.62-1.53 (m, 2H), 1.55 (s, 9H), 1.27 (t, 3H), 0.88 (t, 3H).

LC/MS (Methode 1, ESIpos): Rₜ = 1.25 min, m/z = 389 [M+H]⁺.

### Beispiel 159A

### 5-(Difluormethyl)-1-ethyl-2,4-dioxo-3-propyl-1,2,3,4-tetrahydrothieno[2,3-d]pyrimidin-6-carbonsäure

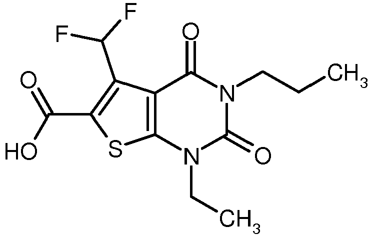

Analog zu dem unter Bsp. 148A beschriebenen Verfahren wurden aus 1.0 g (2.57 mmol) der Verbindung aus Bsp. 158A 855 mg (99% d. Th.) der Titelverbindung erhalten.

¹H-NMR (400 MHz, DMSO-d₆, δ/ppm): 14.28 (sehr breit, ca. 1H), 7.76 (t, 1H), 3.98 (quart, 2H), 3.83 (m, 2H), 1.62-1.53 (m, 2H), 1.27 (t, 3H), 0.88 (t, 3H).

LC/MS (Methode 1, ESIpos): Rₜ = 0.74 min, m/z = 333 [M+H]⁺.

### Beispiel 160A

### 3-Isobutyl-5-methyl-2,4-dioxo-1-(3,3,3-trifluorpropyl)-1,2,3,4-tetrahydrothieno[2,3-d]pyrimidin-6-carbonsäure

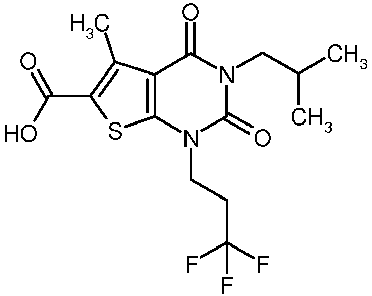

Eine Lösung von 250 mg (0.776 mmol) der Verbindung aus Bsp. 59A in 7.5 ml wasserfreiem DMF wurde mit 758 mg (2.33 mmol) Cäsiumcarbonat versetzt und 10 min bei RT gerührt. Anschließend wurden 211 µl (1.94 mmol) 1-Brom-3-methylpropan hinzugefügt, und das Gemisch wurde zunächst 3 h bei RT und dann ca. 16 h bei 60°C gerührt. Nach dem Abkühlen auf RT wurde das Reaktionsgemisch mit 2 ml 2 M Natronlauge versetzt und weitere 2 h bei RT gerührt. Dann wurde das Gemisch auf ca. 50 ml Wasser gegossen und mit Dichlormethan extrahiert. Der organische Extrakt wurde verworfen, und die wässrige Phase wurde mit 1 M Salzsäure auf einen pH-Wert von ca. 4 gebracht. Es wurde mit Ethylacetat extrahiert. Der organische Extrakt wurde über wasserfreiem Magnesiumsulfat getrocknet, filtriert und eingeengt. Der erhaltene Rückstand wurde mittels präparativer HPLC (Methode 5) gereinigt. Nach Eindampfen der Produktfraktionen und Trocknen des Rückstands im Hochvakuum wurden 132 mg (44% d. Th.) der Titelverbindung erhalten.

¹H-NMR (400 MHz, DMSO-d₆, δ/ppm): 13.42 (breit, 1H), 4.15 (t, 2H), 3.72 (d, 2H), 2.85-2.71 (m, 2H), 2.75 (s, 3H), 2.09-1.98 (m, 1H), 0.86 (d, 6H).

LC/MS (Methode 1, ESIpos): Rₜ = 0.99 min, m/z = 379 [M+H]⁺.

### Beispiel 161A

### 3-(Cyclopropylmethyl)-5-methyl-2,4-dioxo-1-(3,3,3-trifluorpropyl)-1,2,3,4-tetrahydrothieno-[2,3-d]pyrimidin-6-carbonsäure

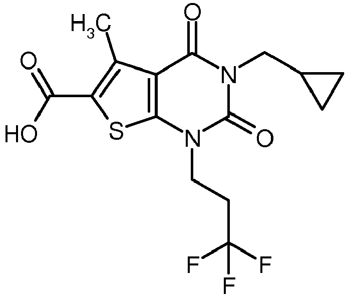

Eine Lösung von 250 mg (0.776 mmol) der Verbindung aus Bsp. 59A in 7.5 ml wasserfreiem DMF wurde mit 758 mg (2.33 mmol) Cäsiumcarbonat versetzt und 10 min bei RT gerührt. Anschließend wurden 262 mg (1.94 mmol) (Brommethyl)cyclopropan hinzugefügt, und das Gemisch wurde ca. 16 h bei RT gerührt. Danach wurde das Reaktionsgemisch mit 2 ml 2 M Natronlauge versetzt und weitere 3 h bei RT gerührt. Dann wurde das Gemisch auf ca. 50 ml Wasser gegossen. Der dabei ausgefallene Feststoff wurde abfiltriert und verworfen. Das Filtrat wurde mit 1 M Salzsäure auf einen pH-Wert von ca. 4-5 gebracht. Das dabei ausgefallene Produkt wurde abgesaugt, mit wenig Wasser gewaschen und im Hochvakuum getrocknet. Es wurden 160 mg (52% d. Th., 95% Reinheit) der Titelverbindung erhalten, die ohne zusätzliche Aufreinigung weiterverwendet wurden.

¹H-NMR (400 MHz, DMSO-d₆, δ/ppm): 13.44 (breit, ca. 1H), 4.16 (t, 2H), 3.77 (d, 2H), 2.86-2.73 (m, 2H), 2.76 (s, 3H), 1.20-1.13 (m, 1H), 0.45-0.40 (m, 2H), 0.36-0.32 (m, 2H).

LC/MS (Methode 1, ESIpos): Rₜ = 0.95 min, m/z = 377 [M+H]⁺.

### Beispiel 162A

### 3-(2-Methoxypropyl)-5-methyl-2,4-dioxo-1-(3,3,3-trifluorpropyl)-1,2,3,4-tetrahydrothieno[2,3-d]-pyrimidin-6-carbonsäure (Racemat)

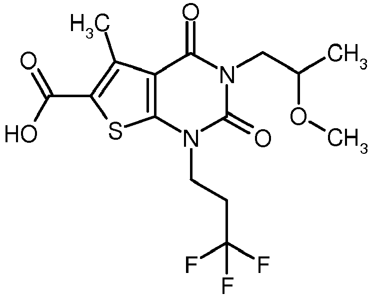

### 1. Schritt: 2-Methoxypropyl-3-(2-methoxypropyl)-5-methyl-2,4-dioxo-1-(3,3,3-trifluorpropyl)-1,2,3,4-tetrahydrothieno[2,3-d]pyrimidin-6-carboxylat

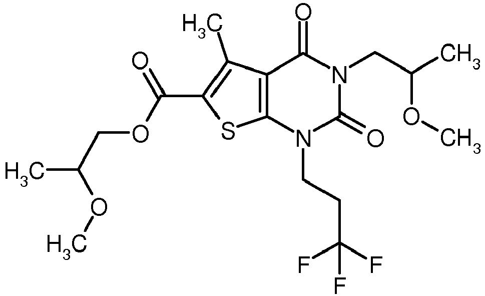

Eine Lösung von 500 mg (1.55 mmol) der Verbindung aus Bsp. 59A in 15 ml wasserfreiem DMF wurde mit 1.52 g (4.66 mmol) Cäsiumcarbonat versetzt und 10 min bei RT gerührt. Dann wurden 948 mg (3.88 mmol) racemisches 2-Methoxypropyl-4-methylbenzolsulfonat [hergestellt analog zu einer publizierten Methode, ausgehend von racemischem Methyl-2-methoxypropionat; Lit.: A. Terfort, H. Brunner, J. Chem. Soc. Perkin Trans. 1, 1996 (12), 1467-1479] hinzugefügt und das Gemisch zunächst ca. 16 h bei RT gerührt. Da die Reaktion noch nicht vollständig war, wurde danach noch ca. 18 h bei 80°C gerührt. Nach dem Abkühlen auf RT wurde mit ca. 100 ml Wasser versetzt und mit Dichlormethan extrahiert. Der organische Extrakt wurde über wasserfreiem Magnesiumsulfat getrocknet, filtriert und eingeengt. Der erhaltene Rückstand wurde mittels präparativer HPLC gereinigt (Methode 6). Nach Einengen der Produktfraktionen wurden 130 mg (18% d. Th.) der Titelverbindung erhalten.

¹H-NMR (400 MHz, DMSO-d₆, δ/ppm): 4.31 (dd, 1H), 4.22-4.13 (m, 3H), 4.06 (dd, 1H), 3.77 (dd, 1H), 3.67-3.59 (m, 2H), 3.29 (s, 3H), 3.22 (s, 3H), 2.86-2.74 (m, 2H), 2.79 (s, 3H), 1.15 (d, 3H), 1.06 (d, 3H).

LC/MS (Methode 1, ESIpos): Rₜ = 1.12 min, m/z = 467 [M+H]⁺.

### 2. Schritt: 3-(2-Methoxypropyl)-5-methyl-2,4-dioxo-1-(3,3,3-trifluorpropyl)-1,2,3,4-tetrahydro-thieno[2,3-d]pyrimidin-6-carbonsäure (Racemat)

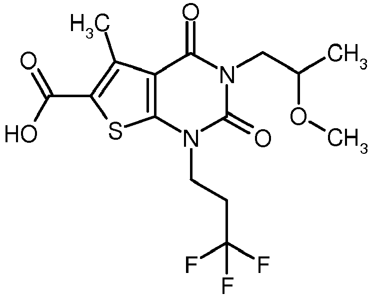

125 mg (0.268 mmol) der Verbindung aus dem vorangegangenen Schritt 1 wurden in 5 ml Ethanol gelöst, mit 1.34 ml (1.34 mmol) einer 1 M Lösung von Lithiumhydroxid in Wasser versetzt und 2 h bei RT gerührt. Anschließend wurde das Ethanol am Rotationsverdampfer entfernt, der Rückstand mit Wasser verdünnt und durch Zusatz von 1 M Salzsäure angesäuert. Es wurde dann mit Ethylacetat extrahiert. Der organische Extrakt wurde über wasserfreiem Magnesiumsulfat getrocknet, filtriert und eingeengt. Es wurden 103 mg (63% d. Th., 66% Reinheit) der Titelverbindung erhalten, die ohne zusätzliche Aufreinigung weiter umgesetzt wurden.

¹H-NMR (400 MHz, DMSO-d₆, δ/ppm): 13.41 (breit, 1H), 4.19-4.13 (m, 2H), 4.05 (dd, 1H), 3.77 (dd, 1H), 3.67-3.59 (m, 1H), 3.22 (s, 3H), 2.85-2.73 (m, 2H), 2.76 (s, 3H), 1.06 (d, 3H).

LC/MS (Methode 1, ESIpos): Rₜ = 0.84 min, m/z = 395 [M+H]⁺.

### Beispiel 163A

### Ethyl-2-[(ethylcarbamoyl)amino]-4-methylthiophen-3-carboxylat

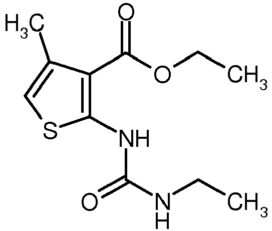

Eine Lösung von 150 g (0.810 mol) Ethyl-2-amino-4-methylthiophen-3-carboxylat und 113 ml (0.810 mol) Triethylamin in 1.5 Liter THF wurde mit 96 ml (1.21 mol) Ethylisocyanat versetzt. Das Reaktionsgemisch wurde 2 Tage unter Rückfluss erhitzt. Nach dem Abkühlen auf RT wurde das Gemisch in ca. 2 Liter Wasser gegossen und viermal mit insgesamt 1.1 Liter Dichlormethan extrahiert. Der organische Extrakt wurde über wasserfreiem Natriumsulfat getrocknet, dann filtriert und zur Trockene eingeengt. Nach Trocknen des Rückstands im Hochvakuum wurden 200 g (89% d. Th, ca. 93% Reinheit) der Titelverbindung erhalten, die ohne weitere Reinigung im nächsten Reaktionsschritt eingesetzt wurde.

¹H-NMR (300 MHz, DMSO-d₆, δ/ppm): 10.28 (s, 1H), 7.83 (breit, 1H), 6.39 (s, 1H), 4.27 (quart, 2H), 3.13 (m, 2H), 2.26 (s, 3H), 1.31 (t, 3H), 1.06 (t, 3H).

LC/MS (Methode 1, ESIpos): Rₜ = 0.92 min, m/z = 257 [M+H]⁺.

### Beispiel 164A

### 3-Ethyl-5-methylthieno[2,3-d]pyrimidin-2,4(1H,3H)-dion

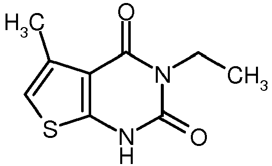

67 g (261 mmol) der Verbindung aus Bsp. 163A wurden in 1.6 Liter Ethanol gelöst und mit 141 ml (392 mmol) einer 21%-igen Lösung von Natriumethylat in Ethanol versetzt. Nachdem das Gemisch ca. 16 h bei RT gerührt worden war, wurde es in ca. 500 ml kaltes Wasser gegossen und durch Zusatz von Eisessig auf einen pH-Wert von ca. 5 gebracht. Der dabei ausgefallene Niederschlag wurde abgesaugt, mit Wasser neutral gewaschen und getrocknet. Es wurden 50 g (91% d. Th.) der Titelverbindung erhalten.

¹H-NMR (300 MHz, DMSO-d₆, δ/ppm): 6.66 (s, 1H), 3.86 (quart, 2H), 2.35 (s, 3H), 1.11 (t, 3H).

LC/MS (Methode 1, ESIpos): Rₜ = 0.67 min, m/z = 211 [M+H]⁺.

### Beispiel 165A

### 3-Ethyl-5-methyl-2,4-dioxo-1,2,3,4-tetrahydrothieno[2,3-d]pyrimidin-6-carbaldehyd

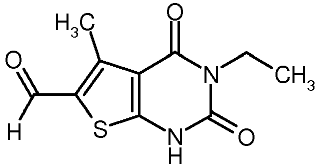

Eine Lösung von 850 mg (4.04 mmol) der Verbindung aus Bsp. 164A in 3.1 ml (40.4 mmol) DMF wurde vorsichtig mit 4.5 ml (48.5 mmol) Phosphoroxychlorid versetzt. Nachdem die stark exotherme Reaktion abgeklungen war, wurde das Gemisch noch weitere 15 min gerührt. Dann wurde das Reaktionsgemisch vorsichtig in 100 ml Eiswasser eingerührt. Nach 1 h Rühren wurde das ausgefallene Produkt abgesaugt, mit Wasser neutral gewaschen und getrocknet. Es wurden 936 mg (97% d. Th.) der Titelverbindung erhalten.

¹H-NMR (400 MHz, DMSO-d₆, δ/ppm): 12.58 (breit, 1H), 10.06 (s, 1H), 3.86 (quart, 2H), 2.76 (s, 3H), 1.12 (t, 3H).

LC/MS (Methode 1, ESIpos): Rₜ = 0.68 min, m/z = 239 [M+H]⁺.

### Beispiel 166A

### 3-Ethyl-5-methyl-1-(3,3,3-trifluorpropyl)thieno[2,3-d]pyrimidin-2,4(1H,3H)-dion

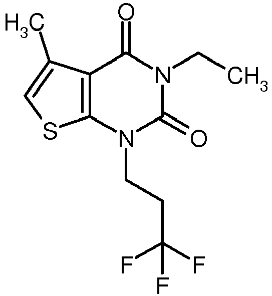

2.0 g (9.51 mmol) der Verbindung aus Bsp. 164A und 3.29 g (23.8 mmol) Kaliumcarbonat wurden in 50 ml wasserfreiem DMF 15 min bei RT gerührt, bevor 3.3 ml (28.5 mmol) 1,1,1-Trifluor-3-iod-propan hinzugefügt wurden. Da der Umsatz nach Rühren über Nacht bei RT nicht vollständig war, wurde mit weiteren 1.31 g (9.51 mmol) Kaliumcarbonat und 1.1 ml (9.51 mmol) 1,1,1-Trifluor-3-iodpropan versetzt und das Gemisch 2 h bei 60°C gerührt. Nach dem Abkühlen auf RT wurde mit Ethylacetat verdünnt und nacheinander zweimal mit Wasser und einmal mit gesättigter Natriumchlorid-Lösung gewaschen. Nach Trocknen über wasserfreiem Magnesiumsulfat wurde filtriert und das Filtrat zur Trockene eingeengt. Das Rohprodukt wurde durch Chromatographie über eine Kieselgel-Kartusche gereinigt (Biotage, 340 g Kieselgel, Laufmittel: Cyclohexan/Ethylacetat 24:1 → 10:1). Nach Einengen und Trocknen der Produktfraktionen wurden 2.06 g (70% d. Th.) der Titelverbindung erhalten.

¹H-NMR (400 MHz, DMSO-d₆, δ/ppm): 6.88 (s, 1H), 4.12 (t, 2H), 3.91 (quart, 2H), 2.84-2.71 (m, 2H), 2.39 (s, 3H), 1.12 (t, 3H).

LC/MS (Methode 1, ESIpos): Rₜ = 1.02 min, m/z = 307 [M+H]⁺.

### Beispiel 167A

### 3-Ethyl-5-methyl-2,4-dioxo-1-(3,3,3-trifluorpropyl)-1,2,3,4-tetrahydrothieno[2,3-d]pyrimidin-6-carbaldehyd

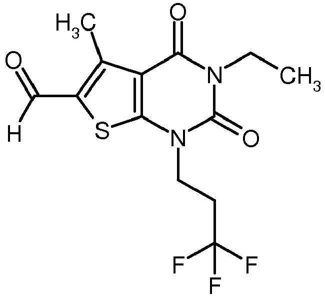

### Methode A:

5.0 g (21.0 mmol) der Verbindung aus Bsp. 165A und 7.25 g (52.5 mmol) Kaliumcarbonat wurden in einem Gemisch aus 95 ml Acetonitril und 15 ml DMF 15 min bei RT gerührt, bevor 14.1 g (63.0 mmol) 1,1,1-Trifluor-3-iodpropan hinzugefügt wurden. Das Reaktionsgemisch wurde ca. 16 h bei einer Temperatur von 78°C gerührt. Nach dem Abkühlen auf RT wurde mit 500 ml Ethylacetat verdünnt und nacheinander zweimal mit je 100 ml Wasser und einmal mit 50 ml gesättigter Natriumchlorid-Lösung gewaschen. Nach Trocknen über wasserfreiem Natriumsulfat wurde filtriert und das Filtrat zur Trockene eingeengt. Das Rohprodukt wurde durch Chromatographie gereinigt (80 g Kieselgel, Laufmittel: Heptan/Ethylacetat 100:0 → 60:40). Nach Einengen der Produktfraktionen und Trocknen des Rückstands wurden 2.99 g (42% d. Th.) der Titelverbindung erhalten.

### Methode B:

Eine Lösung von 5.10 g (16.6 mmol) der Verbindung aus Bsp. 166A in 25.6 ml (333 mmol) DMF wurde vorsichtig mit 7.8 ml (83.2 mmol) Phosphoroxychlorid versetzt. Nachdem die stark exotherme Reaktion fast abgeklungen war, wurde das Gemisch noch weitere 30 min bei 100°C gerührt. Nach dem Abkühlen auf RT wurde das Reaktionsgemisch vorsichtig in 100 ml Eiswasser eingerührt. Nach 1 h Rühren wurde das ausgefallene Produkt abgesaugt, mit Wasser neutral gewaschen und getrocknet. Es wurden 5.08 g (85% d. Th., 94% Reinheit) der Titelverbindung erhalten.

¹H-NMR (400 MHz, DMSO-d₆, δ/ppm): 10.11 (s, 1H), 4.18 (t, 2H), 3.91 (quart, 2H), 2.86-2.74 (m, 2H), 2.80 (s, 3H), 1.14 (t, 3H).

LC/MS (Methode 1, ESIpos): Rₜ = 0.98 min, m/z = 335 [M+H]⁺.

### Ausführungsbeispiele:

### Beispiel 1

### 6-[(4-Hydroxypiperidin-1-yl)carbonyl]-5-methyl-3-(2-phenylethyl)-1-(3,3,3-trifluorpropyl)thieno-[2,3-d]pyrimidin-2,4(1H,3H)-dion

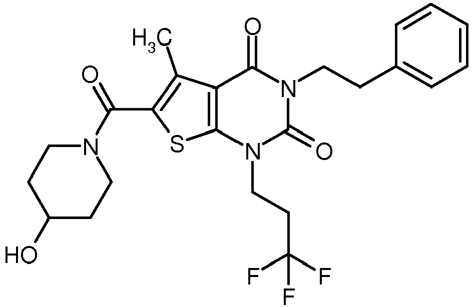

*Darstellung des Säurechlorids:* Eine Lösung von 60 mg (0.140 mmol) der Verbindung aus Bsp. 43A in 2 ml Dichlormethan wurde bei RT zunächst mit 61 µl (0.70 mmol) Oxalylchlorid und dann mit einem kleinen Tropfen DMF versetzt. Nachdem das Reaktionsgemisch 2 h bei RT gerührt worden war, wurde es am Rotationsverdampfer zur Trockene eingedampft. Der verbliebene Rückstand wurde im Hochvakuum getrocknet und anschließend im nächsten Teilschritt weiter umgesetzt.

*Darstellung des Amids:* Das oben erhaltene Säurechlorid wurde in 2 ml wasserfreiem THF gelöst und diese Lösung zu einer Lösung von 17 mg (0.170 mmol) 4-Hydroxypiperidin und 49 µl (0.280 mmol) *N,N*-Diisopropylethylamin in 2 ml wasserfreiem THF getropft. Anschließend wurden noch einige Tropfen Dichlormethan hinzugegeben, und das Reaktionsgemisch wurde 1 h bei RT gerührt. Nachdem das Gemisch am Rotationsverdampfer zur Trockene eingedampft worden war, wurde das Rohprodukt mittels präparativer HPLC gereinigt (Methode 5). Nach Vereinigen der Produktfraktionen, Eindampfen und Trocknen des Rückstands im Hochvakuum wurden 69 mg (96% d. Th.) der Titelverbindung erhalten.

¹H-NMR (400 MHz, CDCl₃, δ/ppm): 7.32-7.21 (m, 5H, teilweise überdeckt vom CHCl₃-Signal), 4.22 (t, 2H), 4.14 (t, 2H), 4.03 (m, 1H), 3.98-3.90 (br. m, 2H), 3.45-3.37 (m, 2H), 2.94 (t, 2H), 2.64-2.52 (m, 2H), 2.51 (s, 3H), 1.98-1.91 (m, 2H), 1.65-1.55 (m, 2H, teilweise überdeckt vom Wassersignal).

LC/MS (Methode 1, ESIpos): Rₜ = 1.04 min, m/z = 510 [M+H]⁺.

### Beispiel 2

### 1-(4,4-Difluorbut-3-en-1-yl)-6-[(4-hydroxypiperidin-1-yl)carbonyl]-5-methyl-3-(2-phenylethyl)-thieno[2,3-d]pyrimidin-2,4(1H,3H)-dion

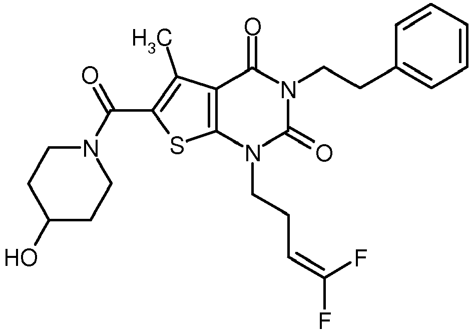

Analog zu dem unter Bsp. 1 beschriebenen Verfahren wurden aus 70 mg (0.170 mmol) der Verbindung aus Bsp. 44A und 20 mg (0.20 mmol) 4-Hydroxypiperidin 42 mg (50% d. Th.) der Titelverbindung erhalten. Abweichend zum zuvor beschriebenen Verfahren betrug die Reaktionszeit im zweiten Teilschritt (Amid-Bildung) nicht 1 h, sondern ca. 16 h.

¹H-NMR (400 MHz, DMSO-d₆, δ/ppm): 7.33-7.29 (m, 2H), 7.27-7.20 (m, 3H), 4.82 (d, 1H), 4.62 (d von t von d, 1H), 4.07 (dd, 2H), 3.93 (t, 2H), 3.82-3.71 (br. m, 3H), 3.24 (m, 2H), 2.83 (dd, 2H), 2.41-2.33 (m, 2H), 2.38 (s, 3H), 1.80-1.72 (m, 2H), 1.40-1.30 (m, 2H).

LC/MS (Methode 1, ESIpos): Rₜ = 1.04 min, m/z = 504 [M+H]⁺.

### Beispiel 3

### 6-[(4-Hydroxypiperidin-1-yl)carbonyl]-1-(2-methoxyethyl)-5-methyl-3-(2-phenylethyl)thieno-[2,3-d]pyrimidin-2,4(1H,3H)-dion

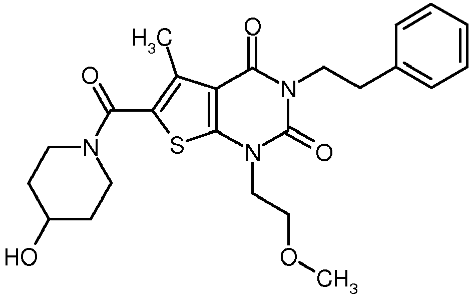

Analog zu dem unter Bsp. 1 beschriebenen Verfahren wurden aus 100 mg (0.260 mmol) der Verbindung aus Bsp. 45A und 31 mg (0.310 mmol) 4-Hydroxypiperidin 102 mg (82% d. Th.) der Titelverbindung erhalten. Abweichend zum zuvor beschriebenen Verfahren betrug die Reaktionszeit im zweiten Teilschritt (Amid-Bildung) nicht 1 h, sondern ca. 16 h. Die präparative HPLC-Reinigung wurde hier nach Methode 6 durchgeführt.

¹H-NMR (400 MHz, DMSO-d₆, δ/ppm): 7.33-7.29 (m, 2H), 7.26-7.20 (m, 3H), 4.82 (d, 1H), 4.09-4.02 (m, 4H), 3.82-3.71 (m, 3H), 3.62 (t, 2H), 3.27-3.19 (m, 2H), 2.25 (s, 3H), 2.84 (m, 2H), 2.37 (s, 3H), 1.80-1.73 (m, 2H), 1.40-1.30 (m, 2H).

LC/MS (Methode 1, ESIpos): Rₜ = 0.93 min, m/z = 472 [M+H]⁺.

### Beispiel 4

### 3-[2-(2-Fluorphenyl)ethyl]-6-[(4-hydroxypiperidin-1-yl)carbonyl]-5-methyl-1-(3,3,3-trifluor-propyl)thieno[2,3-d]pyrimidin-2,4(1H,3H)-dion

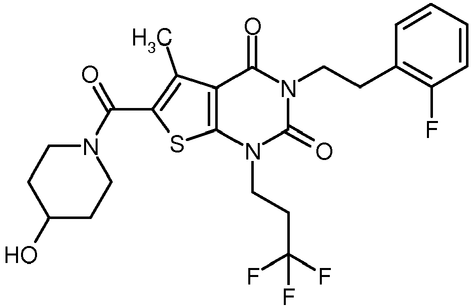

Analog zu dem unter Bsp. 1 beschriebenen Verfahren wurden aus 95 mg (0.210 mmol) der Verbindung aus Bsp. 46A und 29 mg (0.287 mmol) 4-Hydroxypiperidin 112 mg (99% d. Th.) der Titelverbindung erhalten. Abweichend zum zuvor beschriebenen Verfahren betrug die Reaktionszeit im zweiten Teilschritt (Amid-Bildung) nicht 1 h, sondern ca. 16 h.

¹H-NMR (400 MHz, DMSO-d₆, δ/ppm): 7.30-7.24 (m, 2H), 7.17-7.10 (m, 2H), 4.82 (d, 1H), 4.13-4.06 (m, 4H), 3.82-3.71 (m, 3H), 3.24 (m, 2H), 2.90 (t, 2H), 2.78-2.66 (m, 2H), 2.36 (s, 3H), 1.80-1.72 (m, 2H), 1.40-1.31 (m, 2H).

LC/MS (Methode 1, ESIpos): Rₜ = 1.03 min, m/z = 528 [M+H]⁺.

### Beispiel 5

### 1-(4,4-Difluorbut-3-en-1-yl)-3-[2-(2-fluorphenyl)ethyl]-6-[(4-hydroxypiperidin-1-yl)carbonyl]-5-methylthieno[2,3-d]pyrimidin-2,4(1H,3H)-dion

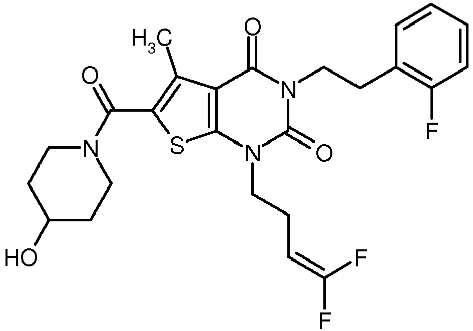

Analog zu dem unter Bsp. 1 beschriebenen Verfahren wurden aus 75 mg (0.170 mmol) der Verbindung aus Bsp. 47A und 41 mg (0.405 mmol) 4-Hydroxypiperidin 88 mg (99% d. Th.) der Titelverbindung erhalten. Abweichend zum zuvor beschriebenen Verfahren betrug die Reaktionszeit im ersten Teilschritt (Säurechlorid-Bildung) 3 h und im zweiten Teilschritt (Amid-Bildung) ca. 16 h.

¹H-NMR (400 MHz, DMSO-d₆, δ/ppm): 7.30-7.24 (m, 2H), 7.16-7.10 (m, 2H), 4.81 (d, 1H), 4.60 (d von t von d, 1H), 4.10 (m, 2H), 3.91 (m, 2H), 3.81-3.71 (m, 3H), 3.23 (m, 2H), 2.90 (m, 2H), 2.36 (s, 3H), 1.80-1.72 (m, 2H), 1.40-1.30 (m, 2H).

LC/MS (Methode 1, ESIpos): Rₜ = 1.04 min, m/z = 522 [M+H]⁺.

### Beispiel 6

### 3-[2-(2-Chlorphenyl)ethyl]-6-[(4-hydroxypiperidin-1-yl)carbonyl]-5-methyl-1-(3,3,3-trifluor-propyl)thieno[2,3-d]pyrimidin-2,4(1H,3H)-dion

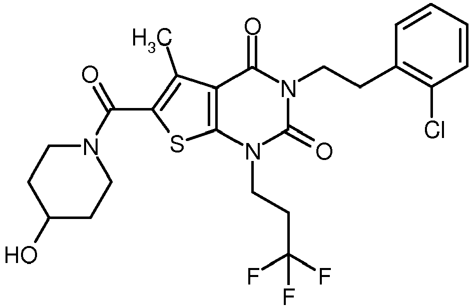

*Darstellung des Säurechlorids:* Eine Lösung von 75 mg (0.160 mmol) der Verbindung aus Bsp. 48A in 2.5 ml Dichlormethan wurde bei RT zunächst mit 71 µl (0.810 mmol) Oxalylchlorid und dann mit einem kleinen Tropfen DMF versetzt. Nachdem das Reaktionsgemisch 2 h bei RT gerührt worden war, wurde es am Rotationsverdampfer zur Trockene eingedampft. Der verbliebene Rückstand wurde im Hochvakuum getrocknet und anschließend im nächsten Teilschritt weiter umgesetzt.

*Darstellung des Amids:* Das oben erhaltene Säurechlorid wurde in 2 ml Dichlormethan gelöst und diese Lösung zu einer Lösung von 25 mg (0.240 mmol) 4-Hydroxypiperidin und 57 µl (0.330 mmol) *N,N*-Diisopropylethylamin in 2.5 ml wasserfreiem THF getropft. Anschließend wurde das Reaktionsgemisch ca. 16 h bei RT gerührt. Nachdem das Gemisch am Rotationsverdampfer zur Trockene eingedampft worden war, wurde das Rohprodukt mittels präparativer HPLC gereinigt (Methode 5). Nach Vereinigen der Produktfraktionen, Eindampfen und Trocknen des Rückstands im Hochvakuum wurden 83 mg (94% d. Th.) der Titelverbindung erhalten.

¹H-NMR (400 MHz, DMSO-d₆, δ/ppm): 7.43-7.40 (m, 1H), 7.31-7.24 (m, 3H), 4.82 (d, 1H), 4.15-4.07 (m, 4H), 3.82-3.71 (m, 3H), 3.24 (m, 2H), 2.99 (t, 2H), 2.78-2.66 (m, 2H), 2.36 (s, 3H), 1.80-1.73 (m, 2H), 1.40-1.31 (m, 2H).

LC/MS (Methode 1, ESIpos): Rₜ = 1.08 min, m/z = 544/546 [M+H]⁺.

### Beispiel 7

### 3-[2-(2-Chlorphenyl)ethyl]-1-(4,4-difluorbut-3-en-1-yl)-6-[(4-hydroxypiperidin-1-yl)carbonyl]-5-methylthieno[2,3-d]pyrimidin-2,4(1H,3H)-dion

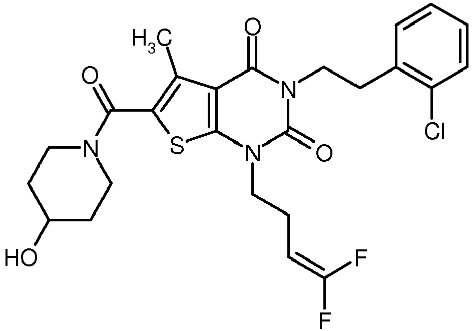

Analog zu dem unter Bsp. 6 beschriebenen Verfahren wurden aus 75 mg (0.160 mmol) der Verbindung aus Bsp. 49A und 25 mg (0.250 mmol) 4-Hydroxypiperidin 85 mg (95% d. Th.) der Titelverbindung erhalten.

¹H-NMR (400 MHz, DMSO-d₆, δ/ppm): 7.43-7.40 (m, 1H), 7.32-7.23 (m, 3H), 4.82 (d, 1H), 4.60 (d von t von d, 1H), 4.13 (m, 2H), 3.91 (t, 2H), 3.81-3.71 (m, 3H), 3.23 (m, 2H), 2.99 (t, 2H), 2.38-2.32 (m, 2H), 2.35 (s, 3H), 1.80-1.73 (m, 2H), 1.40-1.30 (m, 2H).

LC/MS (Methode 1, ESIpos): Rₜ = 1.10 min, m/z = 538/540 [M+H]⁺.

### Beispiel 8

### 6-[(4-Hydroxypiperidin-1-yl)carbonyl]-5-methyl-3-[2-(2-methylphenyl)ethyl]-1-(3,3,3-trifluor-propyl)thieno[2,3-d]pyrimidin-2,4(1H,3H)-dion

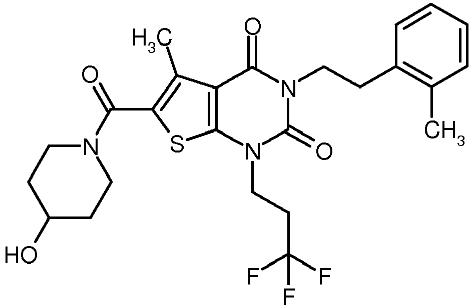

59 mg (0.104 mmol) der Verbindung aus Bsp. 63A wurden in 3 ml Ethanol gelöst und mit 210 µl (0.210 mmol) einer 1 M Lösung von Lithiumhydroxid in Wasser versetzt. Nach 1 h Rühren bei RT wurde das Reaktionsgemisch direkt mittels präparativer HPLC in seine Komponenten aufgetrennt (Methode 5). Die Produktfraktionen wurden vereinigt, eingedampft und der Rückstand im Hochvakuum getrocknet. Es wurden 51 mg (93% d. Th.) der Titelverbindung erhalten.

¹H-NMR (400 MHz, CDCl₃, δ/ppm): 7.22-7.10 (m, 4H), 4.19-4.12 (m, 4H), 4.03 (m, 1H), 3.98-3.90 (m, 2H), 3.44-3.37 (m, 2H), 2.94 (m, 2H), 2.65-2.54 (m, 2H), 2.52 (s, 3H), 2.47 (s, 3H), 1.98-1.90 (m, 2H), 1.65-1.57 (m, 2H, teilweise überdeckt vom Wassersignal).

LC/MS (Methode 1, ESIpos): Rₜ = 1.06 min, m/z = 524 [M+H]⁺.

### Beispiel 9

### 3-[2-(3-Fluorphenyl)ethyl]-6-[(4-hydroxypiperidin-1-yl)carbonyl]-5-methyl-1-(3,3,3-trifluor-propyl)thieno[2,3-d]pyrimidin-2,4(1H,3H)-dion

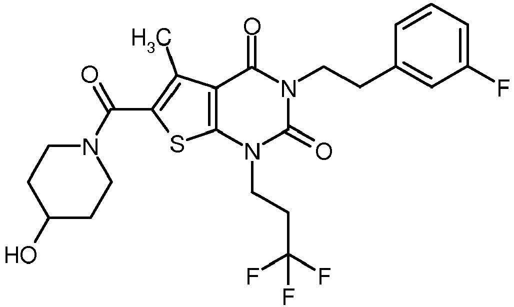

Analog zu dem unter Bsp. 8 beschriebenen Verfahren wurden aus 53 mg (0.093 mmol) der Verbindung aus Bsp. 64A 37 mg (75% d. Th.) der Titelverbindung erhalten. Abweichend zum zuvor beschriebenen Verfahren betrug die Reaktionszeit hier 3 h.

¹H-NMR (400 MHz, CDCl₃, δ/ppm): 7.07 (d, 1H), 7.00 (d, 1H), 6.92 (dt, 1H), 4.23-4.13 (m, 4H), 4.04 (m, 1H), 3.98-3.90 (m, 2H), 3.45-3.37 (m, 2H), 2.94 (m, 2H), 2.66-2.55 (m, 2H), 2.51 (s, 3H), 1.98-1.91 (m, 2H), 1.66-1.57 (m, 2H, teilweise überdeckt vom Wassersignal).

LC/MS (Methode 1, ESIpos): Rₜ = 1.01 min, m/z = 528 [M+H]⁺.

### Beispiel 10

### 3-[2-(3-Chlorphenyl)ethyl]-6-[(4-hydroxypiperidin-1-yl)carbonyl]-5-methyl-1-(3,3,3-trifluor-propyl)thieno[2,3-d]pyrimidin-2,4(1H,3H)-dion

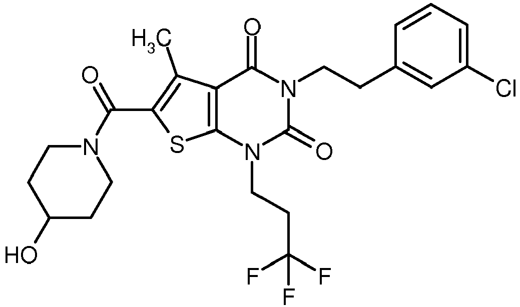

Analog zu dem unter Bsp. 8 beschriebenen Verfahren wurden aus 60 mg (0.10 mmol) der Verbindung aus Bsp. 65A 45 mg (77% d. Th., 95% Reinheit) der Titelverbindung erhalten. Abweichend zum zuvor beschriebenen Verfahren betrug die Reaktionszeit hier 3 h.

¹H-NMR (400 MHz, CDCl₃, δ/ppm): 7.29-7.17 (m, 4H, teilweise überdeckt vom CHCl₃-Signal), 4.22-4.13 (m, 4H), 4.03 (m, 1H), 3.98-3.89 (m, 2H), 3.45-3.37 (m, 2H), 2.92 (m, 2H), 2.67-2.55 (m, 2H), 2.51 (s, 3H), 1.98-1.90 (m, 2H), 1.66-1.56 (m, 2H, teilweise überdeckt vom Wassersignal).

LC/MS (Methode 1, ESIpos): Rₜ = 1.06 min, m/z = 544/546 [M+H]⁺.

### Beispiel 11

### 6-[(4-Hydroxypiperidin-1-yl)carbonyl]-5-methyl-3-[2-(3-methylphenyl)ethyl]-1-(3,3,3-trifluor-propyl)thieno[2,3-d]pyrimidin-2,4(1H,3H)-dion

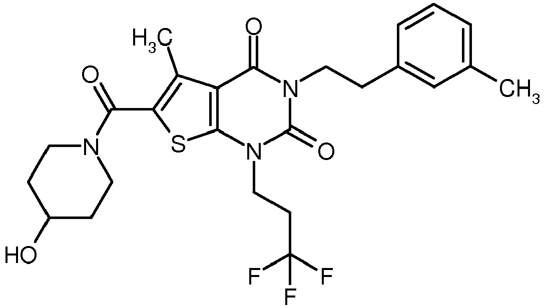

Analog zu dem unter Bsp. 8 beschriebenen Verfahren wurden aus 55 mg (0.10 mmol) der Verbindung aus Bsp. 66A 44 mg (86% d. Th.) der Titelverbindung erhalten.

¹H-NMR (400 MHz, CDCl₃, δ/ppm): 7.20 (t, 1H), 7.14 (s, 1H), 7.10 (d, 1H), 7.04 (d, 1H), 4.21-4.14 (m, 4H), 4.03 (m, 1H), 3.98-3.90 (m, 2H), 3.44-3.37 (m, 2H), 2.89 (m, 2H), 2.66-2.55 (m, 2H), 2.52 (s, 3H), 2.33 (s, 3H), 1.98-1.90 (m, 2H), 1.66-1.56 (m, 2H, teilweise überdeckt vom Wassersignal).

LC/MS (Methode 1, ESIpos): Rₜ = 1.09 min, m/z = 524 [M+H]⁺.

### Beispiel 12

### 3-[2-(4-Fluorphenyl)ethyl]-6-[(4-hydroxypiperidin-1-yl)carbonyl]-5-methyl-1-(3,3,3-trifluor-propyl)thieno[2,3-d]pyrimidin-2,4(1H,3H)-dion

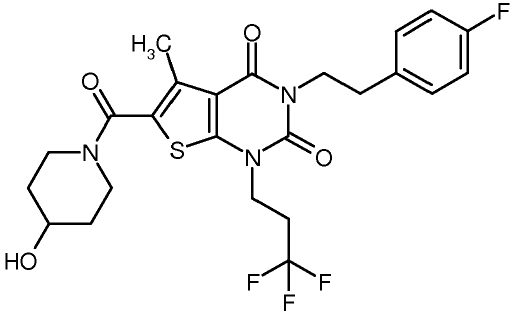

Analog zu dem unter Bsp. 8 beschriebenen Verfahren wurden aus 35 mg (0.061 mmol) der Verbindung aus Bsp. 67A 30 mg (93% d. Th.) der Titelverbindung erhalten. Abweichend zum zuvor beschriebenen Verfahren betrug die Reaktionszeit hier 2 h.

¹H-NMR (400 MHz, CDCl₃, δ/ppm): 7.27-7.23 (m, 2H, teilweise überdeckt vom CHCl₃-Signal), 6.99 (t, 2H), 4.20-4.13 (m, 4H), 4.03 (m, 1H), 3.97-3.90 (m, 2H), 3.45-3.37 (m, 2H), 2.91 (m, 2H), 2.66-2.54 (m, 2H), 2.51 (s, 3H), 1.98-1.90 (m, 2H), 1.66-1.55 (m, 2H, teilweise überdeckt vom Wassersignal).

LC/MS (Methode 1, ESIpos): Rₜ = 1.02 min, m/z = 528 [M+H]⁺.

### Beispiel 13

### 3-[2-(4-Chlorphenyl)ethyl]-6-[(4-hydroxypiperidin-1-yl)carbonyl]-5-methyl-1-(3,3,3-trifluor-propyl)thieno[2,3-d]pyrimidin-2,4(1H,3H)-dion

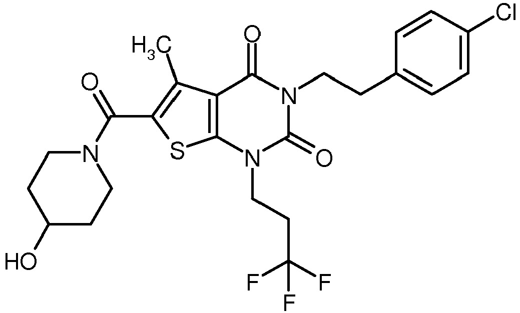

Analog zu dem unter Bsp. 8 beschriebenen Verfahren wurden aus 74 mg (0.126 mmol) der Verbindung aus Bsp. 68A 60 mg (87% d. Th.) der Titelverbindung erhalten. Abweichend zum zuvor beschriebenen Verfahren betrug die Reaktionszeit hier 2 h.

¹H-NMR (400 MHz, CDCl₃, δ/ppm): 7.27 (d, 2H), 7.22 (d, 2H), 4.20-4.13 (m, 4H), 4.04 (m, 1H), 3.97-3.90 (m, 2H), 3.45-3.37 (m, 2H), 2.91 (m, 2H), 2.65-2.53 (m, 2H), 2.51 (s, 3H), 1.98-1.90 (m, 2H), 1.66-1.56 (m, 2H, teilweise überdeckt vom Wassersignal).

LC/MS (Methode 1, ESIpos): Rₜ = 1.10 min, m/z = 544/546 [M+H]⁺.

### Beispiel 14

### 6-[(4-Hydroxypiperidin-1-yl)carbonyl]-3-[2-(4-methoxyphenyl)ethyl]-5-methyl-1-(3,3,3-trifluor-propyl)thieno[2,3-d]pyrimidin-2,4(1H,3H)-dion

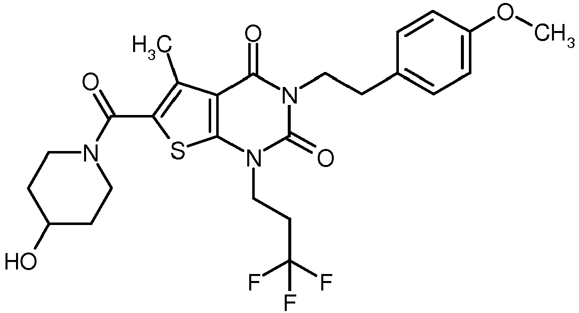

Analog zu dem unter Bsp. 8 beschriebenen Verfahren wurden aus 66 mg (0.11 mmol) der Verbindung aus Bsp. 69A 54 mg (87% d. Th.) der Titelverbindung erhalten. Abweichend zum zuvor beschriebenen Verfahren betrug die Reaktionszeit hier 2 h.

¹H-NMR (400 MHz, CDCl₃, δ/ppm): 7.20 (d, 2H), 6.84 (d, 2H), 4.19-4.12 (m, 4H), 4.03 (m, 1H), 3.98-3.90 (m, 2H), 3.79 (s, 3H), 3.44-3.37 (m, 2H), 2.88 (m, 2H), 2.64-2.54 (m, 2H), 2.51 (s, 3H), 1.98-1.91 (m, 2H), 1.65-1.55 (m, 2H, teilweise überdeckt vom Wassersignal).

LC/MS (Methode 1, ESIpos): Rₜ = 0.98 min, m/z = 540 [M+H]⁺.

### Beispiel 15

### 3-[2-(2-Chlorphenyl)ethyl]-1-(4,4-difluorbut-3-en-1-yl)-6-[(4-hydroxypiperidin-1-yl)carbonyl]-5-methylthieno[2,3-d]pyrimidin-2,4(1H,3H)-dion

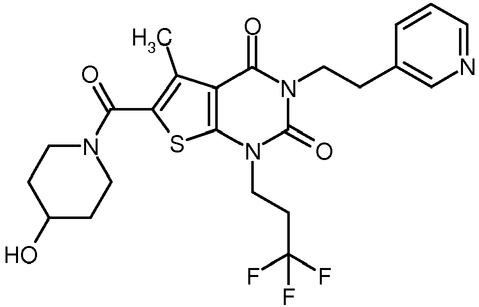

Analog zu dem unter Bsp. 6 beschriebenen Verfahren wurden aus 75 mg (0.160 mmol) der Verbindung aus Bsp. 50A und 25 mg (0.250 mmol) 4-Hydroxypiperidin 62 mg (70% d. Th., 93% Reinheit) der Titelverbindung erhalten. Zusätzlich zu dem zuvor beschriebenen Verfahren wurde hier das nach der präparativen HPLC-Reinigung erhaltene Produkt in etwas Methanol gelöst und die Lösung über eine Hydrogencarbonat-Kartusche gegeben (Fa. Polymerlabs, Stratospheres SPE, PL-HCO₃ MP SPE, Kapazität 0.9 mmol), um nach anschließendem Eindampfen und Trocknen des Rückstands im Hochvakuum die freie Base zu erhalten.

¹H-NMR (400 MHz, DMSO-d₆, δ/ppm): 8.44-8.41 (m, 2H), 7.66 (d, 1H), 7.32 (dd, 1H), 4.82 (d, 1H), 4.13-4.08 (m, 4H), 3.82-3.71 (m, 3H), 3.24 (m, 2H), 2.88 (t, 2H), 2.81-2.68 (m, 2H), 2.36 (s, 3H), 1.80-1.72 (m, 2H), 1.41-1.31 (m, 2H).

LC/MS (Methode 1, ESIpos): Rₜ = 0.60 min, m/z = 511 [M+H]⁺.

### Beispiel 16

### 1-(4,4-Difluorbut-3-en-1-yl)-6-[(4-hydroxypiperidin-1-yl)carbonyl]-5-methyl-3-[2-(pyridin-3-yl)-ethyl]thieno[2,3-d]pyrimidin-2,4(1H,3H)-dion

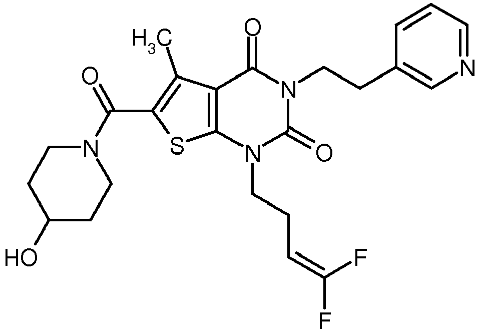

Analog zu dem unter Bsp. 6 beschriebenen Verfahren wurden aus 75 mg (0.160 mmol) der Verbindung aus Bsp. 51A und 25 mg (0.250 mmol) 4-Hydroxypiperidin 50 mg (61% d. Th.) der Titelverbindung erhalten. Zusätzlich zu dem zuvor beschriebenen Verfahren wurde hier das nach der präparativen HPLC-Reinigung erhaltene Produkt in etwas Methanol gelöst und die Lösung über eine Hydrogencarbonat-Kartusche gegeben (Fa. Polymerlabs, Stratospheres SPE, PL-HCO₃ MP SPE, Kapazität 0.9 mmol), um nach anschließendem Eindampfen und Trocknen des Rückstands im Hochvakuum die freie Base zu erhalten.

¹H-NMR (400 MHz, DMSO-d₆, δ/ppm): 8.44-8.41 (m, 2H), 7.66 (d, 1H), 7.32 (dd, 1H), 4.82 (breit, 1H), 4.61 (d von t von d, 1H), 4.10 (m, 2H), 3.92 (t, 2H), 3.81-3.72 (m, 3H), 3.24 (m, 2H), 2.88 (t, 2H), 2.39-2.33 (m, 2H), 2.36 (s, 3H), 1.80-1.72 (m, 2H), 1.40-1.31 (m, 2H).

LC/MS (Methode 1, ESIpos): Rₜ = 0.63 min, m/z = 505 [M+H]⁺.

### Beispiel 17

### 6-[(4-Hydroxypiperidin-1-yl)carbonyl]-5-methyl-3-[2-(pyrazin-2-yl)ethyl]-1-(3,3,3-trifluorpropyl)-thieno[2,3-d]pyrimidin-2,4(1H,3H)-dion

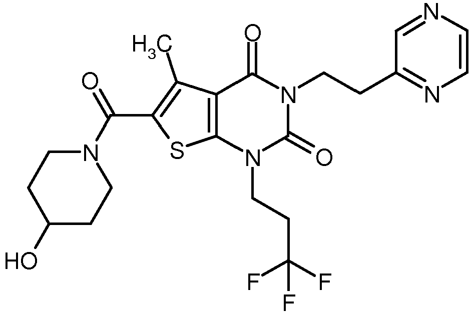

Analog zu dem unter Bsp. 8 beschriebenen Verfahren wurden aus 90 mg (0.08 mmol, Reinheit ca. 50%) der Verbindung aus Bsp. 70A 35 mg (85% d. Th.) der Titelverbindung erhalten. Zusätzlich zu dem zuvor beschriebenen Verfahren wurde hier das nach der präparativen HPLC-Reinigung erhaltene Produkt in etwas Methanol gelöst und die Lösung über eine Hydrogencarbonat-Kartusche gegeben (Fa. Polymerlabs, Stratospheres SPE, PL-HCO₃ MP SPE, Kapazität 0.9 mmol), um nach anschließendem Eindampfen und Trocknen des Rückstands im Hochvakuum die freie Base zu erhalten.

¹H-NMR (400 MHz, DMSO-d₆, δ/ppm): 8.50 (d, 1H), 8.49 (s, 1H), 8.43 (d, 1H), 4.42 (t, 2H), 4.13 (t, 2H), 4.04 (m, 1H), 3.98-3.90 (m, 2H), 3.44-3.37 (m, 2H), 3.18 (t, 2H), 2.65-2.53 (m, 2H), 2.48 (s, 3H), 1.98-1.90 (m, 2H), 1.66-1.56 (m, 2H, überdeckt vom Wassersignal).

LC/MS (Methode 1, ESIpos): Rₜ = 0.77 min, m/z = 512 [M+H]⁺.

### Beispiel 18

### 3-Ethyl-6-[(4-hydroxypiperidin-1-yl)carbonyl]-5-methyl-1-(3,3,3-trifluorpropyl)thieno[2,3-d]-pyrimidin-2,4(1H,3H)-dion

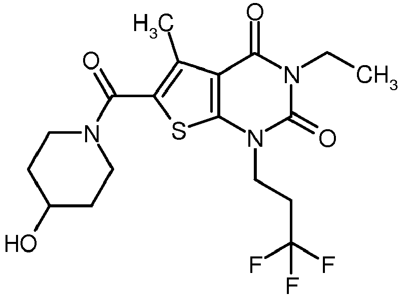

*Darstellung des Säurechlorids:* Eine Lösung von 2.0 g (5.71 mmol) der Verbindung aus Bsp. 52A in 60 ml Dichlormethan wurde bei RT zunächst mit 2.5 ml (28.6 mmol) Oxalylchlorid und dann mit einem Tropfen DMF versetzt. Nachdem das Reaktionsgemisch 2 h bei RT gerührt worden war, wurde es am Rotationsverdampfer zur Trockene eingedampft. Der verbliebene Rückstand wurde im Hochvakuum getrocknet und anschließend im nächsten Teilschritt weiter umgesetzt.

*Darstellung des Amids:* Das oben erhaltene Säurechlorid wurde in 30 ml wasserfreiem THF gelöst und diese Lösung zu einer Lösung von 693 mg (6.85 mmol) 4-Hydroxypiperidin und 2 ml (11.4 mmol) *N,N*-Diisopropylethylamin in 30 ml wasserfreiem THF getropft. Anschließend wurde das Reaktionsgemisch ca. 16 h bei RT gerührt. Nachdem das Gemisch am Rotationsverdampfer zur Trockene eingedampft worden war, wurde der Rückstand mit ca. 100 ml Wasser versetzt und dreimal mit je ca. 150 ml Ethylacetat extrahiert. Der vereinigte organische Extrakt wurde mit gesättigter wässriger Kochsalz-Lösung gewaschen, über wasserfreiem Magnesiumsulfat getrocknet, filtriert und eingedampft. Der so erhaltene Rückstand wurde mit einem Gemisch aus 30 ml Pentan und 0.5 ml Diethylether 2 h lang bei RT verrührt. Anschließend wurde filtriert, mit wenig Pentan nachgewaschen und der Feststoff im Hochvakuum getrocknet. Es wurden 2.32 g (91% d. Th.) der Titelverbindung erhalten.

¹H-NMR (400 MHz, DMSO-d₆, δ/ppm): 4.81 (d, 1H), 4.13 (t, 2H), 3.91 (quart, 2H), 3.82-3.71 (m, 3H), 3.24 (m, 2H), 2.85-2.73 (m, 2H), 2.38 (s, 3H), 1.80-1.72 (m, 2H), 1.39-1.30 (m, 2H), 1.13 (t, 3H).

LC/MS (Methode 1, ESIpos): Rₜ = 0.84 min, m/z = 434 [M+H]⁺.

### Beispiel 19

### 1-(4,4-Difluorbut-3-en-1-yl)-3-ethyl-6-[(4-hydroxypiperidin-1-yl)carbonyl]-5-methylthieno[2,3-d]-pyrimidin-2,4(1H,3H)-dion

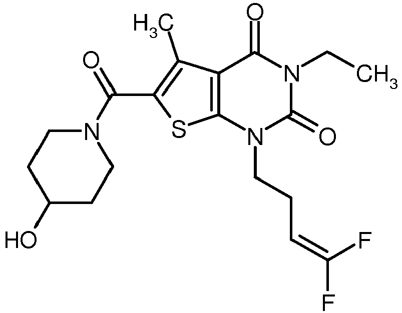

Analog zu dem unter Bsp. 1 beschriebenen Verfahren wurden aus 70 mg (0.20 mmol) der Verbindung aus Bsp. 53A und 25 mg (0.240 mmol) 4-Hydroxypiperidin 70 mg (80% d. Th.) der Titelverbindung erhalten. Abweichend vom zuvor beschriebenen Verfahren betrug die Reaktionszeit im ersten Teilschritt (Säurechlorid-Bildung) nur 1 h und im zweiten Teilschritt (Amid-Bildung) nicht 1 h, sondern ca. 16 h.

¹H-NMR (400 MHz, DMSO-d₆, δ/ppm): 4.81 (d, 1H), 4.63 (d von t von d, 1H), 3.96-3.88 (m, 4H), 3.81-3.71 (m, 3H), 3.23 (m, 2H), 2.42-2.36 (m, 2H), 2.38 (s, 3H), 1.79-1.72 (m, 2H), 1.39-1.30 (m, 2H), 1.12 (t, 3H).

LC/MS (Methode 1, ESIpos): Rₜ = 0.84 min, m/z = 428 [M+H]⁺.

### Beispiel 20

### 3-Ethyl-6-[(4-hydroxypiperidin-1-yl)carbonyl]-1-(2-methoxyethyl)-5-methylthieno[2,3-d]pyrimidin-2,4(1H,3H)-dion

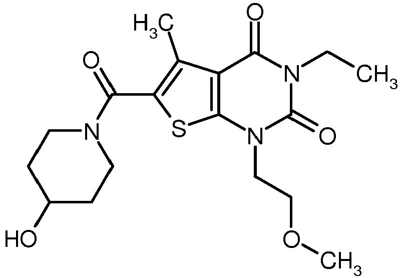

Analog zu dem unter Bsp. 1 beschriebenen Verfahren wurden aus 75 mg (0.220 mmol) der Verbindung aus Bsp. 54A und 27 mg (0.270 mmol) 4-Hydroxypiperidin 57 mg (64% d. Th.) der Titelverbindung erhalten. Abweichend vom zuvor beschriebenen Verfahren betrug die Reaktionszeit im ersten Teilschritt (Säurechlorid-Bildung) nur 1 h.

¹H-NMR (400 MHz, DMSO-d₆, δ/ppm): 4.81 (d, 1H), 4.05 (t, 2H), 3.91 (quart, 2H), 3.82-3.70 (m, 3H), 3.65 (t, 2H), 3.26-3.19 (m, 2H), 3.25 (s, 3H), 2.37 (s, 3H), 1.79-1.72 (m, 2H), 1.39-1.30 (m, 2H), 1.12 (t, 3H).

LC/MS (Methode 1, ESIpos): Rₜ = 0.72 min, m/z = 396 [M+H]⁺.

### Beispiel 21

### 3-Ethyl-6-[(4-hydroxy-4-methylpiperidin-1-yl)carbonyl]-5-methyl-1-(3,3,3-trifluorpropyl)thieno-[2,3-d]pyrimidin-2,4(1H,3H)-dion

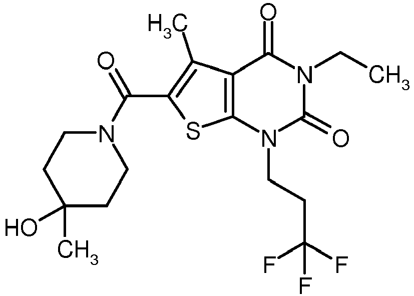

*Darstellung des Säurechlorids:* Eine Lösung von 100 mg (0.290 mmol) der Verbindung aus Bsp. 52A in 3 ml Dichlormethan wurde bei RT zunächst mit 125 µl (1.43 mmol) Oxalylchlorid und dann mit einem Tropfen DMF versetzt. Nachdem das Reaktionsgemisch 1 h bei RT gerührt worden war, wurde es am Rotationsverdampfer zur Trockene eingedampft. Der verbliebene Rückstand wurde im Hochvakuum getrocknet und anschließend im nächsten Teilschritt weiter umgesetzt.

*Darstellung des Amids:* Das oben erhaltene Säurechlorid wurde in 3 ml wasserfreiem THF gelöst und mit 40 mg (0.340 mmol) 4-Methylpiperidin-4-ol [kommerziell erhältlich; Lit. z.B.: J. M. McManus et al., J. Med. Chem. 1965, 8 (6), 766-776] sowie 100 µl (0.570 mmol) *N,N*-Diisopropylethylamin versetzt. Anschließend wurde das Reaktionsgemisch ca. 16 h bei RT gerührt. Nachdem das Gemisch am Rotationsverdampfer zur Trockene eingedampft worden war, wurde das Rohprodukt mittels präparativer HPLC gereinigt (Methode 5). Nach Vereinigen der Produktfraktionen, Eindampfen und Trocknen des Rückstands im Hochvakuum wurden 113 mg (88% d. Th.) der Titelverbindung erhalten.

¹H-NMR (400 MHz, DMSO-d₆, δ/ppm): 4.47 (s, 1H), 4.12 (t, 2H), 3.91 (quart, 2H), 3.72 (breit, 2H), 3.36 (breit, 2H), 2.85-2.73 (m, 2H), 2.38 (s, 3H), 1.53-1.40 (m, 4H), 1.15 (s, 3H), 1.13 (t, 3H).

LC/MS (Methode 1, ESIpos): Rₜ = 0.90 min, m/z = 448 [M+H]⁺.

### Beispiel 22

### 3-Ethyl-6-{[4-hydroxy-4-(trifluormethyl)piperidin-1-yl]carbonyl}-5-methyl-1-(3,3,3-trifluor-propyl)thieno[2,3-d]pyrimidin-2,4(1H,3H)-dion

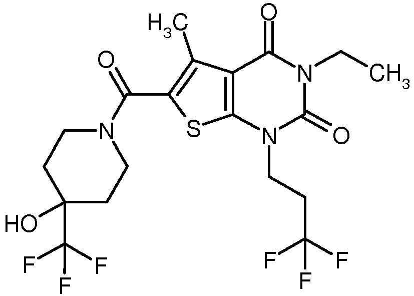

Analog zu dem unter Bsp. 21 beschriebenen Verfahren wurden aus 100 mg (0.290 mmol) der Verbindung aus Bsp. 52A und 70 mg (0.340 mmol) 4-Trifluormethylpiperidin-4-ol [kommerziell erhältlich; Lit. z.B.: WO 2005/103002-A2, Zwischenprodukt 1] 135 mg (94% d. Th.) der Titelverbindung erhalten.

¹H-NMR (400 MHz, DMSO-d₆, δ/ppm): 6.19 (s, 1H), 4.13 (t, 2H), 4.02 (breit, 2H), 3.91 (quart, 2H), 3.22 (br. t, 2H), 2.86-2.73 (m, 2H), 2.40 (s, 3H), 1.76-1.61 (m, 4H), 1.12 (t, 3H).

LC/MS (Methode 1, ESIpos): Rₜ = 0.97 min, m/z = 502 [M+H]⁺.

### Beispiel 23

### 3-Ethyl-6-[(3-hydroxy-8-azabicyclo[3.2.1]oct-8-yl)carbonyl]-5-methyl-1-(3,3,3-trifluorpropyl)-thieno[2,3-d]pyrimidin-2,4(1H,3H)-dion

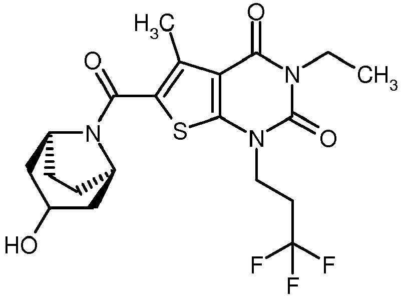

12.7 mg (0.10 mmol) 8-Azabicyclo[3.2.1]octan-3-ol [G. B. Kok et al., J. Org. Chem. 2010, 75 (14), 4806-4811] wurden nacheinander mit Lösungen von 35 mg (0.10 mmol) der Verbindung aus Bsp. 52A und 45.6 mg (0.120 mmol) HATU in jeweils 300 µl DMF sowie mit 35 µl (0.20 mmol) *N,N*-Diisopropylethylamin versetzt. Nach einer Reaktionszeit von ca. 16 h bei RT wurde das Reaktionsgemisch direkt mittels präparativer HPLC in seine Komponenten aufgetrennt (Methode 8). Die Produktfraktionen wurden vereinigt, eingedampft und der Rückstand im Hochvakuum getrocknet. Es wurden 29 mg (52% d. Th., 80% Reinheit) der Titelverbindung erhalten.

LC/MS (Methode 3, ESIpos): Rₜ = 0.98 min, m/z = 460 [M+H]⁺.

### Beispiel 24

### 6-[(4-Hydroxypiperidin-1-yl)carbonyl]-3-(2-phenylethyl)-5-(trifluormethyl)-1-(3,3,3-trifluor-propyl)thieno[2,3-d]pyrimidin-2,4(1H,3H)-dion

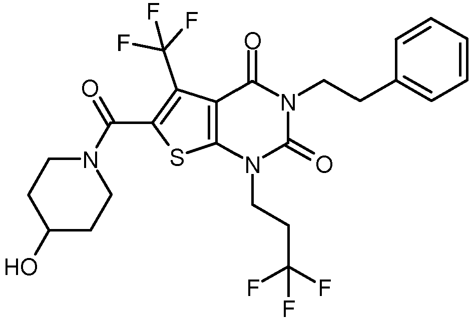

*Darstellung des Säurechlorids:* Eine Lösung von 75 mg (0.150 mmol) der Verbindung aus Bsp. 55A in 2.2 ml Dichlormethan wurde bei RT zunächst mit 67 µl (0.760 mmol) Oxalylchlorid und dann mit einem kleinen Tropfen DMF versetzt. Nachdem das Reaktionsgemisch 2 h bei RT gerührt worden war, wurde es am Rotationsverdampfer zur Trockene eingedampft. Der verbliebene Rückstand wurde im Hochvakuum getrocknet und anschließend im nächsten Teilschritt weiter umgesetzt.

*Darstellung des Amids:* Das oben erhaltene Säurechlorid wurde in 2 ml wasserfreiem THF gelöst und diese Lösung zu einer Lösung von 20 mg (0.20 mmol) 4-Hydroxypiperidin und 53 µl (0.310 mmol) *N,N*-Diisopropylethylamin in 1 ml Dichlormethan getropft. Anschließend wurde das Reaktionsgemisch 1 h bei RT gerührt. Danach wurde das Gemisch am Rotationsverdampfer zur Trockene eingedampft und der Rückstand mittels präparativer HPLC gereinigt (Methode 5). Nach Vereinigen der Produktfraktionen, Eindampfen und Trocknen des Rückstands im Hochvakuum wurden 72 mg (84% d. Th.) der Titelverbindung erhalten.

¹H-NMR (400 MHz, DMSO-d₆, δ/ppm): 7.34-7.29 (m, 2H), 7.27-7.21 (m, 3H), 4.86 (d, 1H), 4.16 (m, 2H), 4.07 (m, 2H), 3.97 (breit, 1H), 3.76 (breit, 1H), 3.45 (breit, 1H), 3.35-3.21 (m, 1H), 3.16 (m, 1H), 2.87-2.73 (m, 4H), 1.84-1.65 (breit, 2H), 1.45-1.28 (breit, 2H).

LC/MS (Methode 1, ESIpos): Rₜ = 1.07 min, m/z = 564 [M+H]⁺.

### Beispiel 25

### 3-Ethyl-6-[(4-hydroxypiperidin-1-yl)carbonyl]-5-(trifluormethyl)-1-(3,3,3-trifluorpropyl)thieno-[2,3-d]pyrimidin-2,4(1H,3H)-dion

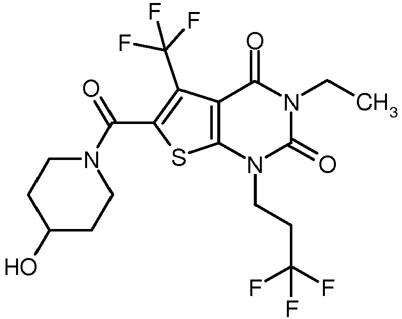

Analog zu dem unter Bsp. 24 beschriebenen Verfahren wurden aus 55 mg (0.140 mmol) der Verbindung aus Bsp. 56A und 18 mg (0.180 mmol) 4-Hydroxypiperidin 64 mg (97% d. Th.) der Titelverbindung erhalten. Abweichend vom zuvor beschriebenen Verfahren betrug die Reaktionszeit im zweiten Teilschritt (Amid-Bildung) ca. 16 h.

¹H-NMR (400 MHz, DMSO-d₆, δ/ppm): 4.85 (d, 1H), 4.16 (t, 2H), 4.02-3.88 (m, 3H), 3.76 (breit, 1H), 3.45 (m, 1H), 3.32-3.22 (br. m, 1H), 3.15 (m, 1H), 2.88-2.76 (m, 2H), 1.83-1.64 (breit, 2H), 1.43-1.25 (breit, 2H), 1.13 (t, 3H).

LC/MS (Methode 1, ESIpos): Rₜ = 0.87 min, m/z = 488 [M+H]⁺.

### Beispiel 26

### 5-Methyl-6-[(3-oxopiperazin-1-yl)carbonyl]-3-(2-phenylethyl)-1-(3,3,3-trifluorpropyl)thieno-[2,3-d]pyrimidin-2,4(1H,3H)-dion

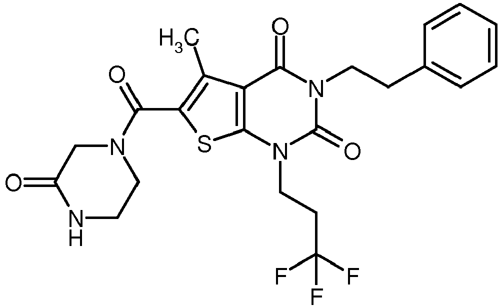

*Darstellung des Säurechlorids:* Eine Lösung von 75 mg (0.180 mmol) der Verbindung aus Bsp. 43A in 2 ml Dichlormethan wurde bei RT zunächst mit 77 µl (0.880 mmol) Oxalylchlorid und dann mit einem kleinen Tropfen DMF versetzt. Nachdem das Reaktionsgemisch 1 h bei RT gerührt worden war, wurde es am Rotationsverdampfer zur Trockene eingedampft. Der verbliebene Rückstand wurde im Hochvakuum getrocknet und anschließend im nächsten Teilschritt weiter umgesetzt.

*Darstellung des Amids:* Das oben erhaltene Säurechlorid wurde in 2 ml wasserfreiem THF gelöst und mit 21 mg (0.210 mmol) Piperazin-2-on und 61 µl (0.350 mmol) *N,N*-Diisopropylethylamin versetzt. Anschließend wurde das Reaktionsgemisch ca. 16 h bei RT gerührt. Nachdem das Gemisch am Rotationsverdampfer zur Trockene eingedampft worden war, wurde das Rohprodukt mittels präparativer HPLC gereinigt (Methode 5). Nach Vereinigen der Produktfraktionen, Eindampfen und Trocknen des Rückstands im Hochvakuum wurden 85 mg (95% d. Th.) der Titelverbindung erhalten.

¹H-NMR (400 MHz, DMSO-d₆, δ/ppm): 8.16 (s, 1H), 7.33-7.28 (m, 2H), 7.26-7.21 (m, 3H), 4.13 (t, 2H), 4.07 (m, 2H), 4.05 (s, 2H), 3.69 (m, 2H), 3.25 (m, 2H), 2.84 (m, 2H), 2.82-2.71 (m, 2H), 2.41 (s, 3H).

LC/MS (Methode 1, ESIpos): Rₜ = 0.97 min, m/z = 509 [M+H]⁺.

### Beispiel 27

### 1-(4,4-Difluorbut-3-en-1-yl)-5-methyl-6-[(3-oxopiperazin-1-yl)carbonyl]-3-(2-phenylethyl)thieno-[2,3-d]pyrimidin-2,4(1H,3H)-dion

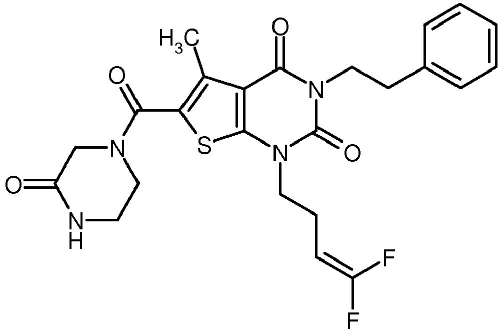

Analog zu dem unter Bsp. 26 beschriebenen Verfahren wurden aus 75 mg (0.180 mmol) der Verbindung aus Bsp. 44A und 21 mg (0.210 mmol) Piperazin-2-on 88 mg (93% d. Th.) der Titelverbindung erhalten.

¹H-NMR (400 MHz, DMSO-d₆, δ/ppm): 8.16 (s, 1H), 7.34-7.29 (m, 2H), 7.26-7.20 (m, 3H), 4.62 (d von t von d, 1H), 4.07 (m, 2H), 4.04 (s, 2H), 3.94 (t, 2H), 3.68 (m, 2H), 3.25 (m, 2H), 2.83 (m, 2H), 2.40 (s, 3H), 2.38 (m, 2H).

LC/MS (Methode 1, ESIpos): Rₜ = 1.00 min, m/z = 503 [M+H]⁺.

### Beispiel 28

### 1-(2-Methoxyethyl)-5-methyl-6-[(3-oxopiperazin-1-yl)carbonyl]-3-(2-phenylethyl)thieno[2,3-d]-pyrimidin-2,4(1H,3H)-dion

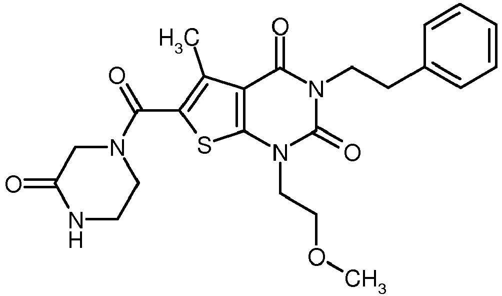

Analog zu dem unter Bsp. 26 beschriebenen Verfahren wurden aus 75 mg (0.190 mmol) der Verbindung aus Bsp. 45A und 23 mg (0.230 mmol) Piperazin-2-on 82 mg (90% d. Th.) der Titelverbindung erhalten.

¹H-NMR (400 MHz, DMSO-d₆, δ/ppm): 8.15 (s, 1H), 7.34-7.29 (m, 2H), 7.26-7.20 (m, 3H), 4.09-4.03 (m, 4H), 4.04 (s, 2H), 3.68 (m, 2H), 3.62 (t, 2H), 3.26-3.22 (m, 2H), 3.25 (s, 3H), 2.84 (m, 2H), 2.39 (s, 3H).

LC/MS (Methode 1, ESIpos): Rₜ = 0.86 min, m/z = 471 [M+H]⁺.

### Beispiel 29

### 3-[2-(2-Fluorphenyl)ethyl]-5-methyl-6-[(3-oxopiperazin-1-yl)carbonyl]-1-(3,3,3-trifluorpropyl)-thieno[2,3-d]pyrimidin-2,4(1H,3H)-dion

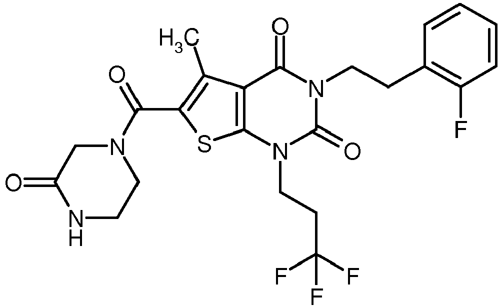

*Garstellung des Säurechlorids:* Eine Lösung von 75 mg (0.170 mmol) der Verbindung aus Bsp. 46A in 1.9 ml Dichlormethan wurde bei RT zunächst mit 74 µl (0.840 mmol) Oxalylchlorid und dann mit einem kleinen Tropfen DMF versetzt. Nachdem das Reaktionsgemisch 1 h bei RT gerührt worden war, wurde es am Rotationsverdampfer zur Trockene eingedampft. Der verbliebene Rückstand wurde im Hochvakuum getrocknet und anschließend im nächsten Teilschritt weiter umgesetzt.

*Darstellung des Amids:* Das oben erhaltene Säurechlorid wurde in 1.9 ml wasserfreiem THF gelöst und mit 20 mg (0.20 mmol) Piperazin-2-on und 59 µl (0.340 mmol) *N,N*-Diisopropylethylamin versetzt. Anschließend wurde das Reaktionsgemisch ca. 16 h bei RT gerührt. Nachdem das Gemisch am Rotationsverdampfer zur Trockene eingedampft worden war, wurde das Rohprodukt mittels präparativer HPLC gereinigt (Methode 5). Nach Vereinigen der Produktfraktionen, Eindampfen und Trocknen des Rückstands im Hochvakuum wurden 65 mg (73% d. Th.) der Titelverbindung erhalten.

¹H-NMR (400 MHz, DMSO-d₆, δ/ppm): 8.16 (s, 1H), 7.29-7.24 (m, 2H), 7.17-7.10 (m, 2H), 4.13-4.07 (m, 4H), 4.04 (s, 2H), 3.68 (m, 2H), 3.25 (m, 2H), 2.91 (t, 2H), 2.78-2.66 (m, 2H), 2.38 (s, 3H).

LC/MS (Methode 1, ESIpos): Rₜ = 0.98 min, m/z = 527 [M+H]⁺.

### Beispiel 30

### 1-(4,4-Difluorbut-3-en-1-yl)-3-[2-(2-fluorphenyl)ethyl]-5-methyl-6-[(3-oxopiperazin-1-yl)-carbonyl]thieno[2,3-d]pyrimidin-2,4(1H,3H)-dion

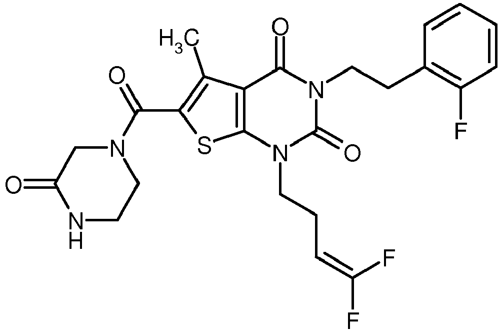

Analog zu dem unter Bsp. 29 beschriebenen Verfahren wurden aus 75 mg (0.170 mmol) der Verbindung aus Bsp. 47A und 21 mg (0.210 mmol) Piperazin-2-on 60 mg (64% d. Th., 95% Reinheit) der Titelverbindung erhalten.

¹H-NMR (400 MHz, DMSO-d₆, δ/ppm): 8.16 (s, 1H), 7.29-7.24 (m, 2H), 7.16-7.10 (m, 2H), 4.60 (d von t von d, 1H), 4.10 (t, 2H), 4.04 (s, 2H), 3.92 (t, 2H), 3.68 (m, 2H), 3.24 (m, 2H), 2.90 (t, 2H), 2.39-2.32 (m, 2H), 2.37 (s, 3H).

LC/MS (Methode 1, ESIpos): Rₜ = 0.98 min, m/z = 521 [M+H]⁺.

### Beispiel 31

### 3-[2-(2-Chlorphenyl)ethyl]-5-methyl-6-[(3-oxopiperazin-1-yl)carbonyl]-1-(3,3,3-trifluorpropyl)-thieno[2,3-d]pyrimidin-2,4(1H,3H)-dion

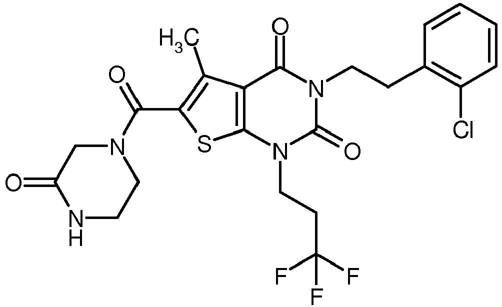

*Garstellung des Säurechlorids:* Eine Lösung von 75 mg (0.160 mmol) der Verbindung aus Bsp. 48A in 2.5 ml Dichlormethan wurde bei RT zunächst mit 71 µl (0.810 mmol) Oxalylchlorid und dann mit einem kleinen Tropfen DMF versetzt. Nachdem das Reaktionsgemisch 2 h bei RT gerührt worden war, wurde es am Rotationsverdampfer zur Trockene eingedampft. Der verbliebene Rückstand wurde im Hochvakuum getrocknet und anschließend im nächsten Teilschritt weiter umgesetzt.

*Garstellung des Amids:* Das oben erhaltene Säurechlorid wurde in 2 ml Dichlormethan gelöst und diese Lösung zu einer Lösung von 24 mg (0.240 mmol) Piperazin-2-on und 57 µl (0.330 mmol) *N,N*-Diisopropylethylamin in 2.5 ml wasserfreiem THF getropft. Anschließend wurde das Reaktionsgemisch ca. 16 h bei RT gerührt. Nachdem das Gemisch am Rotationsverdampfer zur Trockene eingedampft worden war, wurde das Rohprodukt mittels präparativer HPLC gereinigt (Methode 5). Nach Vereinigen der Produktfraktionen, Eindampfen und Trocknen des Rückstands im Hochvakuum wurden 75 mg (84% d. Th.) der Titelverbindung erhalten.

¹H-NMR (400 MHz, DMSO-d₆, δ/ppm): 8.16 (s, 1H), 7.43-7.40 (m, 1H), 7.31-7.24 (m, 3H), 4.15-4.08 (m, 4H), 4.04 (s, 2H), 3.68 (m, 2H), 3.25 (m, 2H), 3.00 (t, 2H), 2.78-2.67 (m, 2H), 2.38 (s, 3H).

LC/MS (Methode 1, ESIpos): Rₜ = 1.01 min, m/z = 543/545 [M+H]⁺.

### Beispiel 32

### 3-[2-(2-Chlorphenyl)ethyl]-1-(4,4-difluorbut-3-en-1-yl)-5-methyl-6-[(3-oxopiperazin-1-yl)-carbonyl]thieno[2,3-d]pyrimidin-2,4(1H,3H)-dion

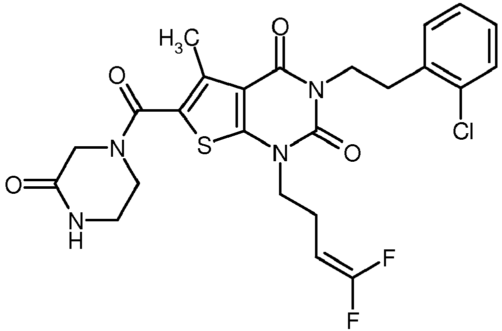

Analog zu dem unter Bsp. 31 beschriebenen Verfahren wurden aus 75 mg (0.170 mmol) der Verbindung aus Bsp. 49A und 25 mg (0.250 mmol) Piperazin-2-on 71 mg (80% d. Th.) der Titelverbindung erhalten.

¹H-NMR (400 MHz, DMSO-d₆, δ/ppm): 8.15 (s, 1H), 7.43-7.39 (m, 1H), 7.32-7.23 (m, 3H), 4.60 (d von t von d, 1H), 4.13 (t, 2H), 4.04 (s, 2H), 3.91 (t, 2H), 3.68 (m, 2H), 3.25 (m, 2H), 2.99 (t, 2H), 2.39-2.32 (m, 2H), 2.37 (s, 3H).

LC/MS (Methode 1, ESIpos): Rₜ = 1.03 min, m/z = 537/539 [M+H]⁺.

### Beispiel 33

### 5-Methyl-6-[(3-oxopiperazin-1-yl)carbonyl]-3-[2-(pyridin-3-yl)ethyl]-1-(3,3,3-trifluorpropyl)-thieno[2,3-d]pyrimidin-2,4(1H,3H)-dion

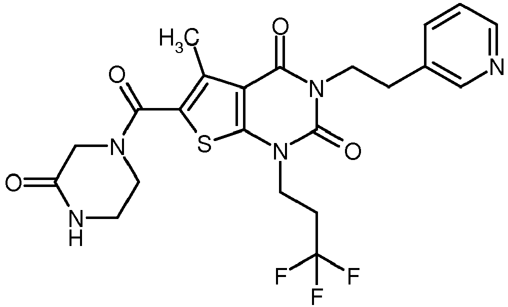

*Garstellung des Säurechlorids:* Eine Lösung von 75 mg (0.160 mmol) der Verbindung aus Bsp. 50A in 2.5 ml Dichlormethan wurde bei RT zunächst mit 71 µl (0.810 mmol) Oxalylchlorid und dann mit einem kleinen Tropfen DMF versetzt. Nachdem das Reaktionsgemisch 2 h bei RT gerührt worden war, wurde es am Rotationsverdampfer zur Trockene eingedampft. Der verbliebene Rückstand wurde im Hochvakuum getrocknet und anschließend im nächsten Teilschritt weiter umgesetzt.

*Garstellung des Amids:* Das oben erhaltene Säurechlorid wurde in 4 ml Dichlormethan gelöst und diese Lösung zu einer Lösung von 24 mg (0.240 mmol) Piperazin-2-on und 84 µl (0.490 mmol) *N,N*-Diisopropylethylamin in 2.5 ml wasserfreiem THF getropft. Anschließend wurde das Reaktionsgemisch ca. 16 h bei RT gerührt. Nachdem das Gemisch am Rotationsverdampfer zur Trockene eingedampft worden war, wurde das Rohprodukt mittels präparativer HPLC gereinigt (Methode 5). Nach Vereinigen und Einengen der Produktfraktionen wurde der Rückstand in etwas Methanol gelöst und die Lösung über eine Hydrogencarbonat-Kartusche gegeben (Fa. Polymerlabs, Stratospheres SPE, PL-HCO₃ MP SPE, Kapazität 0.9 mmol). Nach anschließendem Eindampfen und Trocknen im Hochvakuum wurden 86 mg (100% d. Th.) der Titelverbindung als freie Base erhalten.

¹H-NMR (400 MHz, DMSO-d₆, δ/ppm): 8.44-8.42 (m, 2H), 8.16 (s, 1H), 7.66 (m, 1H), 7.32 (m, 1H), 4.13-4.08 (m, 4H), 4.05 (s, 2H), 3.68 (m, 2H), 3.24 (m, 2H), 2.89 (t, 2H), 2.81-2.69 (m, 2H), 2.38 (s, 3H).

LC/MS (Methode 1, ESIpos): Rₜ = 0.57 min, m/z = 510 [M+H]⁺.

### Beispiel 34

### 1-(4,4-Difluorbut-3-en-1-yl)-5-methyl-6-[(3-oxopiperazin-1-yl)carbonyl]-3-[2-(pyridin-3-yl)ethyl]-thieno[2,3-d]pyrimidin-2,4(1H,3H)-dion

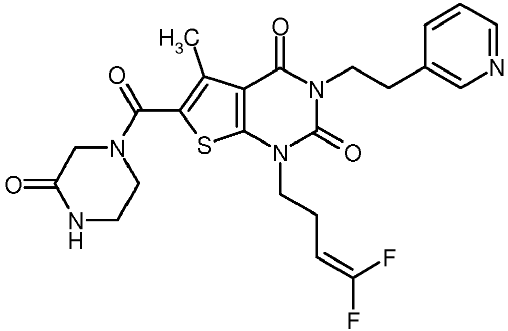

Analog zu dem unter Bsp. 33 beschriebenen Verfahren wurden aus 75 mg (0.160 mmol) der Verbindung aus Bsp. 51A und 25 mg (0.250 mmol) Piperazin-2-on 52 mg (63% d. Th.) der Titelverbindung erhalten.

¹H-NMR (400 MHz, DMSO-d₆, δ/ppm): 8.44-8.41 (m, 2H), 8.16 (s, 1H), 7.66 (m, 1H), 7.32 (m, 1H), 4.61 (d von t von d, 1H), 4.11 (t, 2H), 4.04 (s, 2H), 3.92 (t, 2H), 3.68 (m, 2H), 3.24 (m, 2H), 2.88 (t, 2H), 2.40-2.33 (m, 2H), 2.38 (s, 3H).

LC/MS (Methode 1, ESIpos): Rₜ = 0.59 min, m/z = 504 [M+H]⁺.

### Beispiel 35

### 3-Ethyl-5-methyl-6-[(3-oxopiperazin-1-yl)carbonyl]-1-(3,3,3-trifluorpropyl)thieno[2,3-d]pyrimidin-2,4(1H,3H)-dion

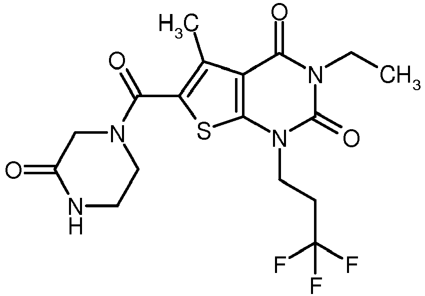

Analog zu dem unter Bsp. 26 beschriebenen Verfahren wurden aus 100 mg (0.290 mmol) der Verbindung aus Bsp. 52A und 34 mg (0.340 mmol) Piperazin-2-on 49 mg (39% d. Th.) der Titelverbindung erhalten.

¹H-NMR (400 MHz, DMSO-d₆, δ/ppm): 8.15 (s, 1H), 4.13 (t, 2H), 4.04 (s, 2H), 3.91 (quart, 2H), 3.68 (m, 2H), 3.24 (m, 2H), 2.85-2.73 (m, 2H), 2.40 (s, 3H), 1.13 (t, 3H).

LC/MS (Methode 1, ESIpos): Rₜ = 0.76 min, m/z = 433 [M+H]⁺.

### Beispiel 36

### 1-(4,4-Difluorbut-3-en-1-yl)-3-ethyl-5-methyl-6-[(3-oxopiperazin-1-yl)carbonyl]thieno[2,3-d]-pyrimidin-2,4(1H,3H)-dion

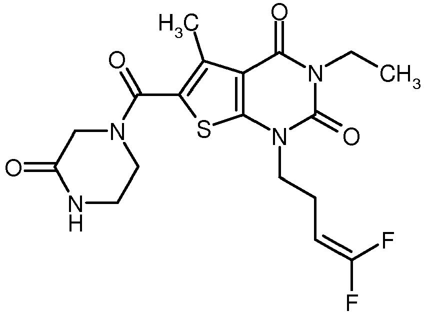

Analog zu dem unter Bsp. 26 beschriebenen Verfahren wurden aus 75 mg (0.220 mmol) der Verbindung aus Bsp. 53A und 26 mg (0.260 mmol) Piperazin-2-on 73 mg (74% d. Th.) der Titelverbindung erhalten.

¹H-NMR (400 MHz, DMSO-d₆, δ/ppm): 8.15 (s, 1H), 4.63 (d von t von d, 1H), 4.04 (s, 2H), 3.96-3.88 (m, 4H), 3.68 (m, 2H), 3.23 (m, 2H), 2.43-2.37 (m, 2H), 2.40 (s, 3H), 1.12 (t, 3H).

LC/MS (Methode 1, ESIpos): Rₜ = 0.81 min, m/z = 427 [M+H]⁺.

### Beispiel 37

### 3-Ethyl-1-(2-methoxyethyl)-5-methyl-6-[(3-oxopiperazin-1-yl)carbonyl]thieno[2,3-d]pyrimidin-2,4(1H,3H)-dion

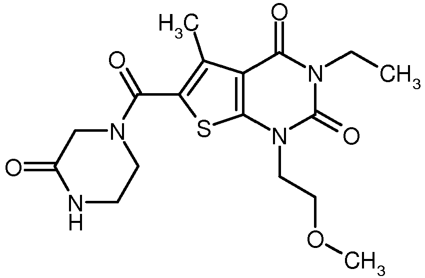

Analog zu dem unter Bsp. 26 beschriebenen Verfahren wurden aus 75 mg (0.240 mmol) der Verbindung aus Bsp. 54A und 29 mg (0.290 mmol) Piperazin-2-on 84 mg (88% d. Th.) der Titelverbindung erhalten.

¹H-NMR (400 MHz, DMSO-d₆, δ/ppm): 8.15 (s, 1H), 4.05 (t, 2H), 4.04 (s, 2H), 3.91 (quart, 2H), 3.69-3.63 (m, 4H), 3.25 (s, 3H), 3.25-3.21 (m, 2H), 2.39 (s, 3H), 1.13 (t, 3H).

LC/MS (Methode 1, ESIpos): Rₜ = 0.63 min, m/z = 395 [M+H]⁺.

### Beispiel 38

### 6-[(2,2-Dimethyl-3-oxopiperazin-1-yl)carbonyl]-3-ethyl-5-methyl-1-(3,3,3-trifluorpropyl)thieno-[2,3-d]pyrimidin-2,4(1H,3H)-dion

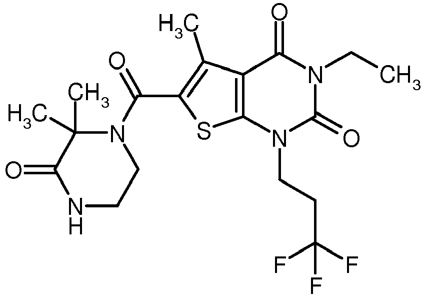

Analog zu dem unter Bsp. 23 beschriebenen Verfahren wurden aus 12.8 mg (0.10 mmol) 3,3-Dimethylpiperazin-2-on [A. Benjahad et al., Tetrahedron Lett. 1994, 35 (51), 9545-9548] und 35 mg (0.10 mmol) der Verbindung aus Bsp. 52A 33 mg (53% d. Th., 75% Reinheit) der Titelverbindung erhalten.

LC/MS (Methode 3, ESIpos): Rₜ = 0.97 min, m/z = 461 [M+H]⁺.

### Beispiel 39

### 3-Ethyl-5-methyl-6-[(2-methyl-3-oxopiperazin-1-yl)carbonyl]-1-(3,3,3-trifluorpropyl)thieno-[2,3-d]pyrimidin-2,4(1H,3H)-dion (Racemat)

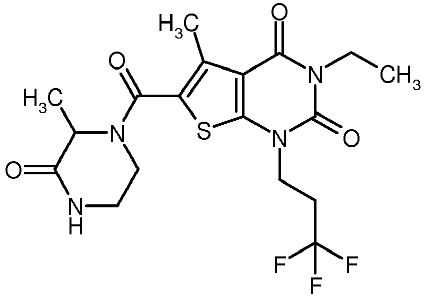

Analog zu dem unter Bsp. 23 beschriebenen Verfahren wurden aus 11.4 mg (0.10 mmol) 3-Methylpiperazin-2-on [K. M. Beck et al., J. Amer. Chem. Soc. 1952, 74 (3), 605-608] und 35 mg (0.10 mmol) der Verbindung aus Bsp. 52A 20 mg (46% d. Th.) der Titelverbindung erhalten.

LC/MS (Methode 3, ESIpos): Rₜ = 0.91 min, m/z = 447 [M+H]⁺.

### Beispiel 40

### 3-Ethyl-6-[(2-ethyl-3-oxopiperazin-1-yl)carbonyl]-5-methyl-1-(3,3,3-trifluorpropyl)thieno[2,3-d]-pyrimidin-2,4(1H,3H)-dion (Racemat)

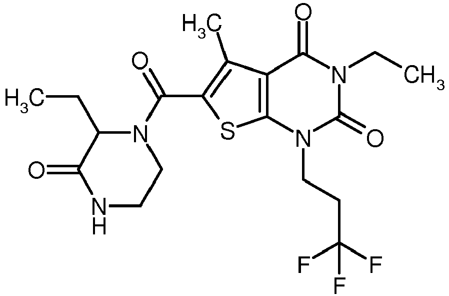

Analog zu dem unter Bsp. 23 beschriebenen Verfahren wurden aus 12.8 mg (0.10 mmol) 3-Ethyl-piperazin-2-on [S. R. Aspinall, J. Amer. Chem. Soc. 1940, 62 (5), 1202-1204] und 35 mg (0.10 mmol) der Verbindung aus Bsp. 52A 15 mg (32% d. Th.) der Titelverbindung erhalten.

LC/MS (Methode 3, ESIpos): Rₜ = 0.94 min, m/z = 461 [M+H]⁺.

### Beispiel 41

### 6-[(3-Oxopiperazin-1-yl)carbonyl]-3-(2-phenylethyl)-5-(trifluormethyl)-1-(3,3,3-trifluorpropyl)-thieno[2,3-d]pyrimidin-2,4(1H,3H)-dion

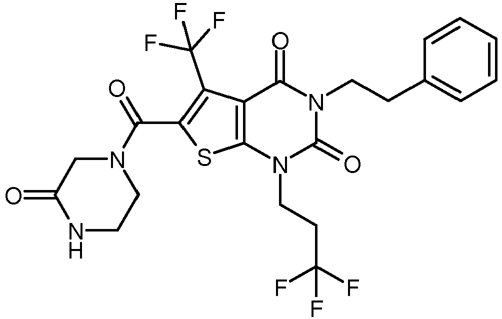

*Garstellung des Säurechlorids:* Eine Lösung von 75 mg (0.150 mmol) der Verbindung aus Bsp. 55A in 2.2 ml Dichlormethan wurde bei RT zunächst mit 66 µl (0.760 mmol) Oxalylchlorid und dann mit einem kleinen Tropfen DMF versetzt. Nachdem das Reaktionsgemisch 2 h bei RT gerührt worden war, wurde es am Rotationsverdampfer zur Trockene eingedampft. Der verbliebene Rückstand wurde im Hochvakuum getrocknet und anschließend im nächsten Teilschritt weiter umgesetzt.

*Garstellung des Amids:* Das oben erhaltene Säurechlorid wurde in 2.2 ml wasserfreiem THF gelöst und diese Lösung zu einer Lösung von 20 mg (0.20 mmol) Piperazin-2-on und 53 µl (0.310 mmol) *N,N*-Diisopropylethylamin in 1 ml Dichlormethan getropft. Anschließend wurde das Reaktionsgemisch ca. 16 h bei RT gerührt. Nachdem das Gemisch am Rotationsverdampfer zur Trockene eingedampft worden war, wurde das Rohprodukt mittels präparativer HPLC gereinigt (Methode 5). Nach Vereinigen der Produktfraktionen, Eindampfen und Trocknen des Rückstands im Hochvakuum wurden 22 mg (24% d. Th.) der Titelverbindung erhalten.

¹H-NMR (400 MHz, DMSO-d₆, δ/ppm): 8.20 (s, 1H), 7.34-7.29 (m, 2H), 7.27-7.21 (m, 3H), 4.17 (m, 2H), 4.11-4.04 (m, 3H), 3.89-3.76 (m, 2H), 3.51 (m, 1H), 3.30-3.12 (m, 2H), 2.88-2.74 (m, 4H).

LC/MS (Methode 1, ESIpos): Rₜ = 1.02 min, m/z = 563 [M+H]⁺.

### Beispiel 42

### 3-Ethyl-6-[(3-oxopiperazin-1-yl)carbonyl]-5-(trifluormethyl)-1-(3,3,3-trifluorpropyl)thieno[2,3-d]-pyrimidin-2,4(1H,3H)-dion

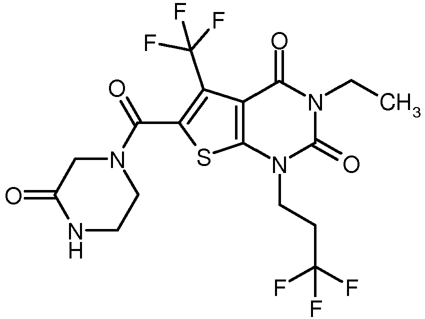

Analog zu dem unter Bsp. 41 beschriebenen Verfahren wurden aus 55 mg (0.140 mmol) der Verbindung aus Bsp. 56A und 18 mg (0.180 mmol) Piperazin-2-on 56 mg (85% d. Th.) der Titelverbindung erhalten.

¹H-NMR (400 MHz, DMSO-d₆, δ/ppm): 8.19 (s, 1H), 4.17 (m, 2H), 4.09 (breit, 1H), 3.92 (quart, 2H), 3.85 (breit, 1H), 3.79 (breit, 1H), 3.50 (m, 1H), 3.26 (breit, 1H), 3.18 (breit, 1H), 2.88-2.76 (m, 2H), 1.14 (t, 3H).

LC/MS (Methode 1, ESIpos): Rₜ = 0.82 min, m/z = 487 [M+H]⁺.

### Beispiel 43

### 6-[(4-Hydroxypiperidin-1-yl)carbonyl]-5-methyl-3-(2-phenylpropyl)-1-(3,3,3-trifluorpropyl)thieno-[2,3-d]pyrimidin-2,4(1H,3H)-dion (Racemat)

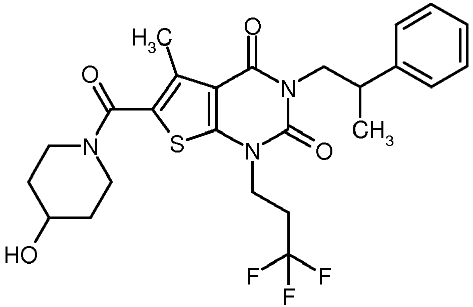

230 mg (0.407 mmol) der Verbindung aus Bsp. 71A wurden in 8 ml Ethanol gelöst und mit 813 µl (0.813 mmol) einer 1 M Lösung von Lithiumhydroxid in Wasser versetzt. Nach 1 h Rühren bei RT wurde das Reaktionsgemisch direkt mittels präparativer HPLC in seine Komponenten aufgetrennt (Methode 5). Die Produktfraktionen wurden vereinigt und eingeengt und der Rückstand im Hochvakuum getrocknet. Es wurden 176 mg (82% d. Th.) der Titelverbindung erhalten.

¹H-NMR (400 MHz, DMSO-d₆, δ/ppm): 7.31-7.24 (m, 4H), 7.22-7.18 (m, 1H), 4.82 (d, 1H), 4.12-4.05 (m, 3H), 3.95 (dd, 1H), 3.82-3.71 (m, 3H), 3.28-3.19 (m, 3H), 2.77-2.65 (m, 2H), 2.36 (s, 3H), 1.80-1.72 (m, 2H), 1.40-1.31 (m, 2H), 1.19 (d, 3H).

LC/MS (Methode 1, ESIpos): Rₜ = 1.06 min, m/z = 524 [M+H]⁺.

### Trennung der Enantiomere:

168 mg (0.321 mmol) der racemischen Verbindung aus Bsp. 43 wurden in 2 ml Ethanol gelöst und in 8 Portionen über präparative HPLC an chiraler Phase in die Enantiomere getrennt (siehe Beispiele 44 und 45) [Säule: Daicel Chiralpak AD-H, 5 µm, 250 mm x 20 mm; Eluent: Isohexan/ Ethanol 1:1; Fluss: 20 ml/min; Temperatur: 23°C; Detektion: 220 nm]:

### Beispiel 44

### 6-[(4-Hydroxypiperidin-1-yl)carbonyl]-5-methyl-3-(2-phenylpropyl)-1-(3,3,3-trifluorpropyl)thieno-[2,3-d]pyrimidin-2,4(1H,3H)-dion (Enantiomer 1)

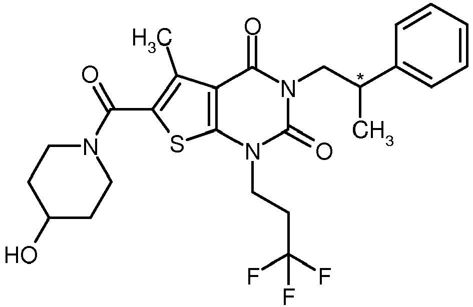

Ausbeute: 54 mg (64% d. Th.)

¹H-NMR (400 MHz, DMSO-d₆, δ/ppm): 7.31-7.24 (m, 4H), 7.22-7.18 (m, 1H), 4.82 (s, breit, 1H), 4.12-4.05 (m, 3H), 3.95 (dd, 1H), 3.82-3.71 (m, 3H), 3.28-3.19 (m, 3H), 2.77-2.65 (m, 2H), 2.36 (s, 3H), 1.80-1.72 (m, 2H), 1.40-1.31 (m, 2H), 1.19 (d, 3H).

Analytische HPLC [Säule: Daicel Chiralpak AD-H, 5 µm, 250 mm x 4.6 mm; Eluent: Isohexan/ Ethanol 1:1; Fluss: 1 ml/min; Temperatur: 30°C; Detektion: 220 nm]: Rₜ = 4.39 min, 99.9% ee.

### Beispiel 45

### 6-[(4-Hydroxypiperidin-1-yl)carbonyl]-5-methyl-3-(2-phenylpropyl)-1-(3,3,3-trifluorpropyl)thieno-[2,3-d]pyrimidin-2,4(1H,3H)-dion (Enantiomer 2)

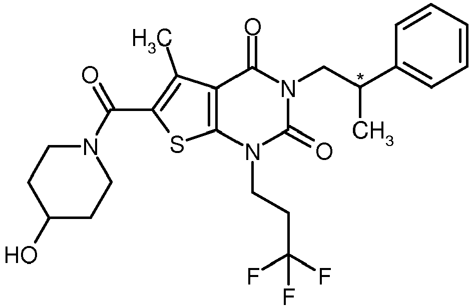

Ausbeute: 58 mg (69% d. Th.)

¹H-NMR (400 MHz, DMSO-d₆, δ/ppm): 7.31-7.24 (m, 4H), 7.22-7.18 (m, 1H), 4.82 (s, breit, 1H), 4.12-4.05 (m, 3H), 3.95 (dd, 1H), 3.82-3.71 (m, 3H), 3.28-3.19 (m, 3H), 2.77-2.65 (m, 2H), 2.36 (s, 3H), 1.80-1.72 (m, 2H), 1.40-1.31 (m, 2H), 1.19 (d, 3H).

Analytische HPLC [Säule: Daicel Chiralpak AD-H, 5 µm, 250 mm x 4.6 mm; Eluent: Isohexan/ Ethanol 1:1; Fluss: 1 ml/min; Temperatur: 30°C; Detektion: 220 nm]: Rₜ = 6.76 min, 99.9% ee.

### Beispiel 46

### 6-[(4-Hydroxypiperidin-1-yl)carbonyl]-3-(2-methoxy-2-phenylethyl)-5-methyl-1-(3,3,3-trifluor-propyl)thieno[2,3-d]pyrimidin-2,4(1H,3H)-dion (Racemat)

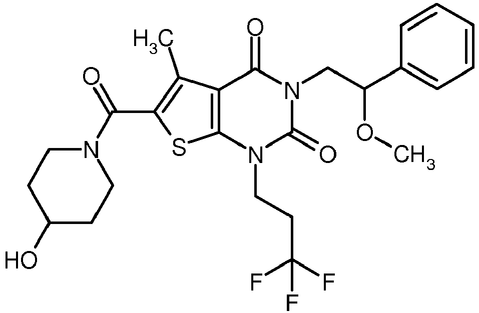

249 mg (0.428 mmol) der Verbindung aus Bsp. 72A wurden in 8 ml Ethanol gelöst und mit 856 µl (0.856 mmol) einer 1 M Lösung von Lithiumhydroxid in Wasser versetzt. Nach 1 h Rühren bei RT wurde das Reaktionsgemisch direkt mittels präparativer HPLC in seine Komponenten aufgetrennt (Methode 5). Die Produktfraktionen wurden vereinigt und eingeengt und der Rückstand im Hochvakuum getrocknet. Es wurden 172 mg (74% d. Th.) der Titelverbindung erhalten.

¹H-NMR (400 MHz, DMSO-d₆, δ/ppm): 7.41-7.37 (m, 2H), 7.34-7.30 (m, 3H), 4.82 (d, 1H), 4.58 (dd, 1H), 4.35 (dd, 1H), 4.18-4.04 (m, 2H), 3.84-3.71 (m, 4H), 3.28-3.20 (m, 2H), 3.07 (s, 3H), 2.79-2.67 (m, 2H), 2.37 (s, 3H), 1.80-1.73 (m, 2H), 1.41-1.31 (m, 2H).

LC/MS (Methode 1, ESIpos): Rₜ = 1.00 min, m/z = 540 [M+H]⁺.

### Trennung der Enantiomere:

160 mg (0.298 mmol) der racemischen Verbindung aus Bsp. 46 wurden in 6 ml Ethanol gelöst und in 6 Portionen über präparative HPLC an chiraler Phase in die Enantiomere getrennt (siehe Beispiele 47 und 48) [Säule: Daicel Chiralcel OZ-H, 5 µm, 250 mm x 20 mm; Eluent: Ethanol; Fluss: 25 ml/min; Temperatur: 50°C; Detektion: 220 nm]:

### Beispiel 47

### 6-[(4-Hydroxypiperidin-1-yl)carbonyl]-3-(2-methoxy-2-phenylethyl)-5-methyl-1-(3,3,3-trifluor-propyl)thieno[2,3-d]pyrimidin-2,4(1H,3H)-dion (Enantiomer 2)

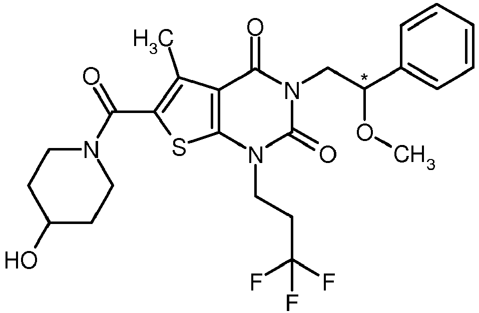

Ausbeute: 68 mg (85% d. Th.)

¹H-NMR (400 MHz, DMSO-d₆, δ/ppm): 7.41-7.37 (m, 2H), 7.34-7.30 (m, 3H), 4.82 (d, 1H), 4.57 (dd, 1H), 4.35 (dd, 1H), 4.18-4.04 (m, 2H), 3.84-3.71 (m, 4H), 3.28-3.20 (m, 2H), 3.07 (s, 3H), 2.79-2.67 (m, 2H), 2.37 (s, 3H), 1.80-1.73 (m, 2H), 1.41-1.31 (m, 2H).

Analytische HPLC [Säule: Daicel Chiralcel OZ-H, 5 µm, 250 mm x 4.6 mm; Eluent: Ethanol; Fluss: 1 ml/min; Temperatur: 50°C; Detektion: 220 nm]: Rₜ = 14.25 min, 99.9% ee.

### Beispiel 48

### 6-[(4-Hydroxypiperidin-1-yl)carbonyl]-3-(2-methoxy-2-phenylethyl)-5-methyl-1-(3,3,3-trifluor-propyl)thieno[2,3-d]pyrimidin-2,4(1H,3H)-dion (Enantiomere 1)

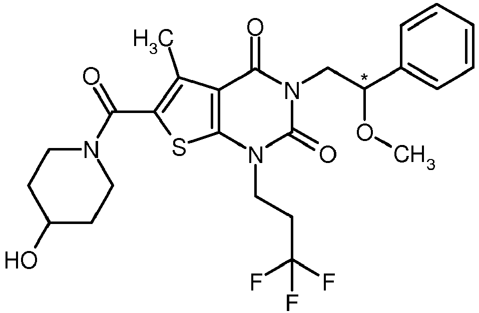

Ausbeute: 70 mg (87% d. Th.)

¹H-NMR (400 MHz, DMSO-d₆, δ/ppm): 7.41-7.37 (m, 2H), 7.34-7.30 (m, 3H), 4.82 (d, 1H), 4.58 (dd, 1H), 4.35 (dd, 1H), 4.18-4.04 (m, 2H), 3.84-3.71 (m, 4H), 3.28-3.20 (m, 2H), 3.07 (s, 3H), 2.79-2.67 (m, 2H), 2.37 (s, 3H), 1.80-1.73 (m, 2H), 1.41-1.31 (m, 2H).

Analytische HPLC [Säule: Daicel Chiralcel OZ-H, 5 µm, 250 mm x 4.6 mm; Eluent: Ethanol; Fluss: 1 ml/min; Temperatur: 50°C; Detektion: 220 nm]: Rₜ = 6.54 min, 99.9% ee.

### Beispiel 49

### 6-[(4-Hydroxypiperidin-1-yl)carbonyl]-5-methyl-3-[(1-phenylcyclopropyl)methyl]-1-(3,3,3-tri-fluorpropyl)thieno[2,3-d]pyrimidin-2,4(1H,3H)-dion

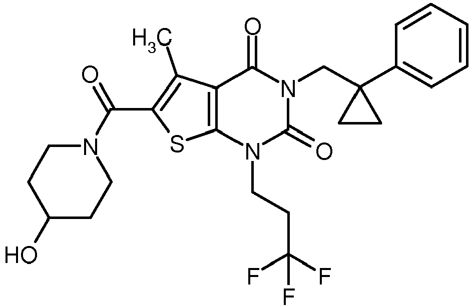

Analog zu dem unter Bsp. 43 beschriebenen Verfahren wurden aus 54 mg (0.094 mmol) der Verbindung aus Bsp. 73A 38 mg (75% d. Th.) der Titelverbindung erhalten.

¹H-NMR (400 MHz, DMSO-d₆, δ/ppm): 7.26-7.19 (m, 4H), 7.17-7.12 (m, 1H), 4.82 (d, 1H), 4.16 (s, 2H), 3.99 (t, 2H), 3.81-3.71 (m, 3H), 3.26-3.20 (m, 2H), 2.63-2.52 (m, 2H, teilweise überdeckt vom DMSO-Signal), 2.31 (s, 3H), 1.80-1.73 (m, 2H), 1.40-1.31 (m, 2H), 0.95 (m, 2H), 0.72 (m, 2H).

LC/MS (Methode 1, ESIpos): Rₜ = 1.08 min, m/z = 536 [M+H]⁺.

### Beispiel 50

### 6-[(4-Hydroxypiperidin-1-yl)carbonyl]-5-methyl-3-(2-methyl-2-phenylpropyl)-1-(3,3,3-trifluor-propyl)thieno[2,3-d]pyrimidin-2,4(1H,3H)-dion

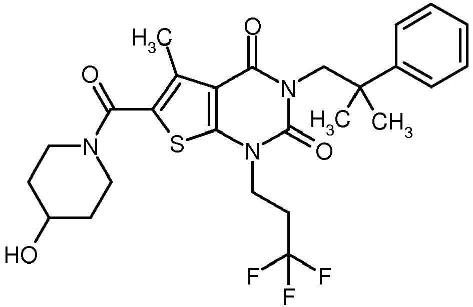

Analog zu dem unter Bsp. 43 beschriebenen Verfahren wurden aus 96 mg (0.166 mmol) der Verbindung aus Bsp. 74A 68 mg (76% d. Th.) der Titelverbindung erhalten.

¹H-NMR (400 MHz, DMSO-d₆, δ/ppm): 7.43 (d, 2H), 7.32 (t, 2H), 7.21 (t, 1H), 4.82 (d, 1H), 4.11-4.04 (m, 4H), 3.82-3.71 (m, 3H), 3.28-3.21 (m, 2H), 2.77-2.65 (m, 2H), 2.34 (s, 3H), 1.80-1.73 (m, 2H), 1.40-1.31 (m, 2H), 1.28 (s, 6H).

LC/MS (Methode 1, ESIpos): Rₜ = 1.08 min, m/z = 538 [M+H]⁺.

### Beispiel 51

### 3-(2,2-Difluor-2-phenylethyl)-6-[(4-hydroxypiperidin-1-yl)carbonyl]-5-methyl-1-(3,3,3-trifluor-propyl)thieno[2,3-d]pyrimidin-2,4(1H,3H)-dion

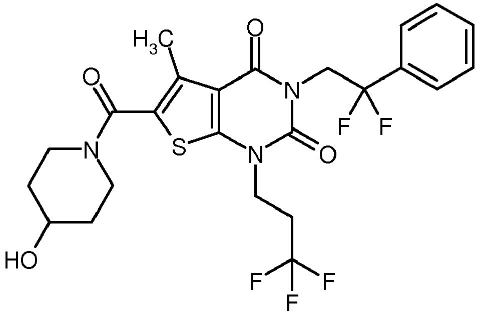

Eine Lösung von 80 mg (0.173 mmol) der Verbindung aus Bsp. 100A in 2.5 ml wasserfreiem DMF wurde nacheinander mit 21 mg (0.208 mmol) 4-Hydroxypiperidin, 39 µl (0.225 mmol) *N,N*-Diisopropylethylamin und 79 mg (0.208 mmol) HATU versetzt. Nach einer Reaktionszeit von ca. 16 h bei RT wurde das Reaktionsgemisch direkt mittels präparativer HPLC in seine Komponenten aufgetrennt (Methode 5). Die Produktfraktionen wurden vereinigt und eingeengt und der Rückstand im Hochvakuum getrocknet. Es wurden 88 mg (93% d. Th.) der Titelverbindung erhalten.

¹H-NMR (400 MHz, DMSO-d₆, δ/ppm): 7.57-7.49 (m, 5H), 4.80 (breit, 1H), 4.62 (t, 2H), 4.12 (t, 2H), 2.82-2.72 (m, 3H), 3.29-3.21 (m, 2H), 2.79-2.67 (m, 2H), 2.36 (s, 3H), 1.81-1.73 (m, 2H), 1.41-1.31 (m, 2H).

LC/MS (Methode 1, ESIpos): Rₜ = 0.98 min, m/z = 546 [M+H]⁺.

### Beispiel 52

### 3-(2,2-Difluor-2-phenylethyl)-6-[(4-hydroxypiperidin-1-yl)carbonyl]-1-(2-methoxyethyl)-5-methyl-thieno[2,3-d]pyrimidin-2,4(1H,3H)-dion

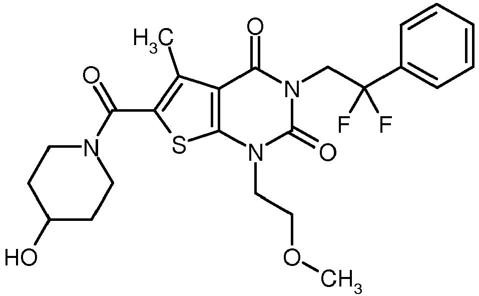

Analog zu dem unter Bsp. 51 beschriebenen Verfahren wurden aus 80 mg (0.188 mmol) der Verbindung aus Bsp. 101A und 23 mg (0.226 mmol) 4-Hydroxypiperidin 90 mg (94% d. Th.) der Titelverbindung erhalten.

¹H-NMR (400 MHz, DMSO-d₆, δ/ppm): 7.57-7.50 (m, 5H), 4.80 (d, 1H), 4.62 (t, 2H), 4.04 (t, 2H), 3.82-3.71 (m, 3H), 3.59 (t, 2H), 3.27-3.21 (m, 2H), 3.25 (s, 3H), 2.35 (s, 3H), 1.81-1.73 (m, 2H), 1.40-1.32 (m, 2H).

LC/MS (Methode 1, ESIpos): Rₜ = 0.90 min, m/z = 508 [M+H]⁺.

### Beispiel 53

### 6-[(4-Hydroxypiperidin-1-yl)carbonyl]-5-methyl-3-[2-(pyridin-2-yl)ethyl]-1-(3,3,3-trifluorpropyl)-thieno[2,3-d]pyrimidin-2,4(1H,3H)-dion

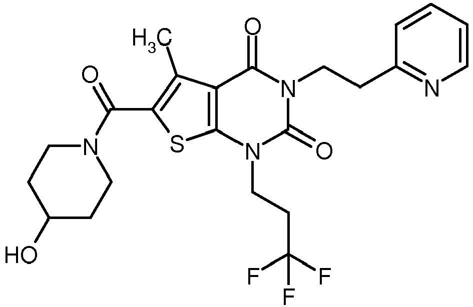

Analog zu dem unter Bsp. 43 beschriebenen Verfahren wurden aus 110 mg (0.199 mmol) der Verbindung aus Bsp. 75A 35 mg (33% d. Th., 96% Reinheit) der Titelverbindung erhalten. Zusätzlich zum zuvor beschriebenen Verfahren wurde hier das Produkt nach der präparativen HPLC nochmals in wenig Methanol gelöst und über eine Hydrogencarbonat-Kartusche (Fa. Polymerlabs, Stratospheres SPE, PL-HCO₃ MP SPE, Kapazität 0.9 mmol) gegeben, um es vom Ameisensäure-Salz in die freie Base zu überführen.

¹H-NMR (400 MHz, DMSO-d₆, δ/ppm): 8.47 (d, 1H), 7.71 (dt, 1H), 7.28 (d, 1H), 7.23 (dd, 1H), 4.82 (d, 1H), 4.22 (t, 1H), 4.11 (t, 1H), 3.82-3.72 (m, 3H), 3.28-3.21 (m, 2H), 2.99 (t, 2H), 2.81-2.69 (m, 2H), 2.38 (s, 3H), 1.80-1.73 (m, 2H), 1.40-1.31 (m, 2H).

LC/MS (Methode 1, ESIpos): Rₜ = 0.63 min, m/z = 511 [M+H]⁺.

### Beispiel 54

### 6-[(4-Hydroxypiperidin-1-yl)carbonyl]-5-methyl-3-[2-(pyridin-4-yl)ethyl]-1-(3,3,3-trifluorpropyl)-thieno[2,3-d]pyrimidin-2,4(1H,3H)-dion

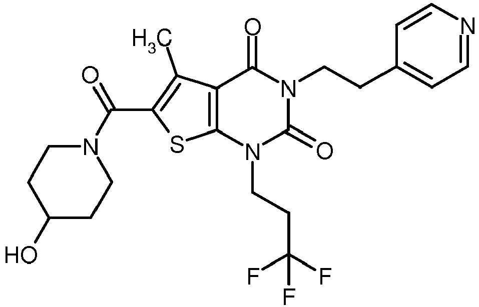

Analog zu dem unter Bsp. 43 beschriebenen Verfahren wurden aus 54 mg (0.098 mmol) der Verbindung aus Bsp. 76A 24 mg (43% d. Th., 90% Reinheit) der Titelverbindung erhalten.

¹H-NMR (400 MHz, DMSO-d₆, δ/ppm): 8.47 (d, 2H), 7.26 (d, 2H), 4.82 (d, 1H), 4.14-4.09 (m, 4H), 3.81-3.71 (m, 3H), 3.28-3.20 (m, 2H), 2.88 (t, 2H), 2.82-2.70 (m, 2H), 2.37 (s, 3H), 1.80-1.73 (m, 2H), 1.40-1.31 (m, 2H).

LC/MS (Methode 1, ESIpos): Rₜ = 0.58 min, m/z = 511 [M+H]⁺.

### Beispiel 55

### 6-[(4-Hydroxypiperidin-1-yl)carbonyl]-3-[2-(1H-imidazol-1-yl)ethyl]-5-methyl-1-(3,3,3-trifluor-propyl)thieno[2,3-d]pyrimidin-2,4(1H,3H)-dion

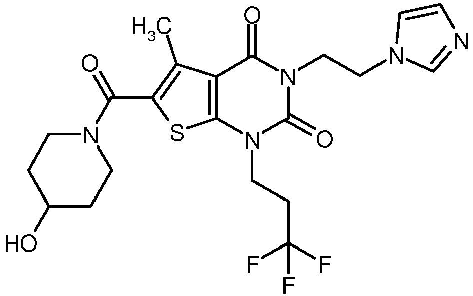

Analog zu dem unter Bsp. 43 beschriebenen Verfahren wurden aus 100 mg (0.185 mmol) der Verbindung aus Bsp. 77A 18 mg (19% d. Th.) der Titelverbindung erhalten. Zusätzlich zum zuvor beschriebenen Verfahren wurde hier das Produkt nach der präparativen HPLC nochmals in wenig Methanol gelöst und über eine Hydrogencarbonat-Kartusche (Fa. Polymerlabs, Stratospheres SPE, PL-HCO₃ MP SPE, Kapazität 0.9 mmol) gegeben, um es vom Ameisensäure-Salz in die freie Base zu überführen.

¹H-NMR (400 MHz, DMSO-d₆, δ/ppm): 7.56 (s, 1H), 7.11 (s, 1H), 6.84 (s, 1H), 4.82 (d, 1H), 4.22-4.17 (m, 4H), 4.08 (t, 2H), 3.81-3.71 (m, 3H), 3.27-3.20 (m, 2H), 2.80-2.68 (m, 2H), 2.34 (s, 3H), 1.80-1.73 (m, 2H), 1.40-1.31 (m, 2H).

LC/MS (Methode 1, ESIpos): Rₜ = 0.49 min, m/z = 500 [M+H]⁺.

### Beispiel 56

### 3-Ethyl-6-[(4-hydroxypiperidin-1-yl)carbonyl]-5-methyl-1-[2-(trifluormethoxy)ethyl]thieno[2,3-d]-pyrimidin-2,4(1H,3H)-dion

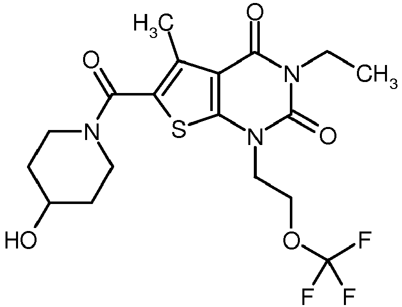

Analog zu dem unter Bsp. 43 beschriebenen Verfahren wurden aus 59 mg (0.121 mmol) der Verbindung aus Bsp. 83A 44 mg (80% d. Th.) der Titelverbindung erhalten. Die Reaktionszeit betrug in diesem Fall 2 h, und die präparative HPLC-Reinigung erfolgte nach Methode 12.

¹H-NMR (400 MHz, DMSO-d₆, δ/ppm): 4.41 (t, 2H), 4.22 (t, 2H), 3.91 (quart, 2H), 3.80-3.71 (m, 3H), 3.26-3.19 (m, 2H), 2.37 (s, 3H), 1.79-1.71 (m, 2H), 1.39-1.29 (m, 2H), 1.12 (t, 3H).

LC/MS (Methode 1, ESIpos): Rₜ = 0.83 min, m/z = 450 [M+H]⁺.

### Beispiel 57

### 3-Ethyl-6-[(4-hydroxypiperidin-1-yl)carbonyl]-5-methyl-1-{2-[(trifluormethyl)sulfanyl]ethyl}-thieno[2,3-d]pyrimidin-2,4(1H,3H)-dion

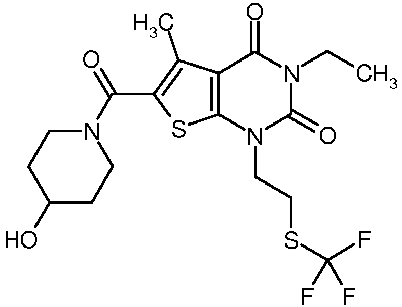

Analog zu dem unter Bsp. 43 beschriebenen Verfahren wurden aus 67 mg (0.133 mmol) der Verbindung aus Bsp. 84A 47 mg (76% d. Th.) der Titelverbindung erhalten. Die Reaktionszeit betrug in diesem Fall 2 h, und die präparative HPLC-Reinigung erfolgte nach Methode 12.

¹H-NMR (400 MHz, DMSO-d₆, δ/ppm): 4.16 (t, 2H), 3.91 (quart, 2H), 3.81-3.71 (m, 3H), 3.37 (t, 2H), 3.26-3.20 (m, 2H), 2.38 (s, 3H), 1.79-1.72 (m, 2H), 1.39-1.30 (m, 2H), 1.12 (t, 3H).

LC/MS (Methode 1, ESIpos): Rₜ = 0.90 min, m/z = 466 [M+H]⁺.

### Beispiel 58

### 3-Ethyl-6-[(4-hydroxypiperidin-1-yl)carbonyl]-5-methyl-1-[2-(trifluormethyl)prop-2-en-1-yl]-thieno[2,3-d]pyrimidin-2,4(1H,3H)-dion

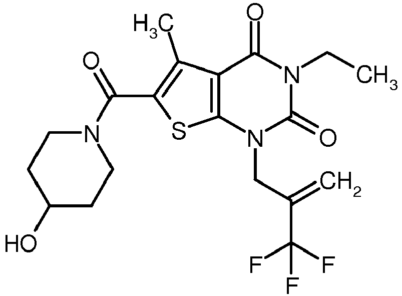

Analog zu dem unter Bsp. 43 beschriebenen Verfahren wurden aus 69 mg (0.142 mmol) der Verbindung aus Bsp. 85A 49 mg (78% d. Th.) der Titelverbindung erhalten. Die Reaktionszeit betrug in diesem Fall 2 h, und die präparative HPLC-Reinigung erfolgte nach Methode 14.

¹H-NMR (400 MHz, DMSO-d₆, δ/ppm): 6.02 (s, 1H), 5.83 (s, 1H), 4.78 (s, 2H), 3.93 (quart, 2H), 3.80-3.70 (m, 3H), 3.26-3.19 (m, 2H), 2.38 (s, 3H), 1.78-1.71 (m, 2H), 1.38-1.29 (m, 2H), 1.13 (t, 3H).

LC/MS (Methode 1, ESIpos): Rₜ = 0.84 min, m/z = 446 [M+H]⁺.

### Beispiel 59

### 1-[(2,2-Difluorcyclopropyl)methyl]-3-ethyl-6-[(4-hydroxypiperidin-1-yl)carbonyl]-5-methylthieno-[2,3-d]pyrimidin-2,4(1H,3H)-dion (Racemat)

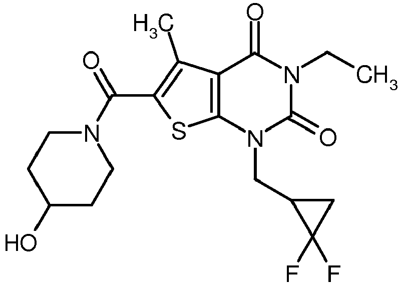

Analog zu dem unter Bsp. 43 beschriebenen Verfahren wurden aus 62 mg (0.132 mmol) der Verbindung aus Bsp. 86A 57 mg (100% d. Th.) der Titelverbindung erhalten. Die Reaktionszeit betrug in diesem Fall 2 h, und die präparative HPLC-Reinigung erfolgte nach Methode 14.

¹H-NMR (400 MHz, DMSO-d₆, δ/ppm): 4.15 (m, 1H), 4.00-3.88 (m, 4H), 3.81-3.70 (m, 3H), 3.27-3.20 (m, 2H), 2.38 (s, 3H), 2.28-2.17 (m, 1H), 1.79-1.67 (m, 3H), 1.53-1.44 (m, 1H), 1.39-1.30 (m, 2H), 1.13 (t, 3H).

LC/MS (Methode 1, ESIpos): Rₜ = 0.80 min, m/z = 428 [M+H]⁺.

### Beispiel 60

### 6-[(4-Hydroxypiperidin-1-yl)carbonyl]-5-methyl-3-propyl-1-[2-(trifluormethoxy)ethyl]thieno-[2,3-d]pyrimidin-2,4(1H,3H)-dion

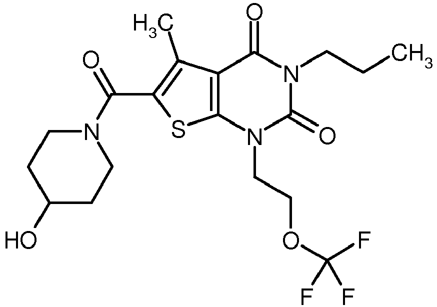

Eine Lösung von 100 mg (0.263 mmol) der Verbindung aus Bsp. 102A in 3 ml wasserfreiem DMF wurde nacheinander mit 32 mg (0.316 mmol) 4-Hydroxypiperidin, 60 µl (0.342 mmol) *N,N*-Diisopropylethylamin und 120 mg (0.316 mmol) HATU versetzt. Nach einer Reaktionszeit von ca. 16 h bei RT wurde das Reaktionsgemisch direkt mittels präparativer HPLC in seine Komponenten aufgetrennt (Methode 5). Die Produktfraktionen wurden vereinigt und eingeengt und der Rückstand im Hochvakuum getrocknet. Es wurden 101 mg (82% d. Th.) der Titelverbindung erhalten.

¹H-NMR (400 MHz, DMSO-d₆, δ/ppm): 4.79 (d, 1H), 4.41 (t, 2H), 4.22 (t, 2H), 3.84 (t, 2H), 3.80-3.70 (m, 3H), 3.26-3.19 (m, 2H), 2.37 (s, 3H), 1.79-1.71 (m, 2H), 1.57 (m, 2H), 1.39-1.30 (m, 2H), 0.87 (t, 3H).

LC/MS (Methode 1, ESIpos): Rₜ = 0.88 min, m/z = 464 [M+H]⁺.

### Beispiel 61

### 3-(But-3-in-1-yl)-6-[(4-hydroxypiperidin-1-yl)carbonyl]-5-methyl-1-(3,3,3-trifluorpropyl)thieno-[2,3-d]pyrimidin-2,4(1H,3H)-dion

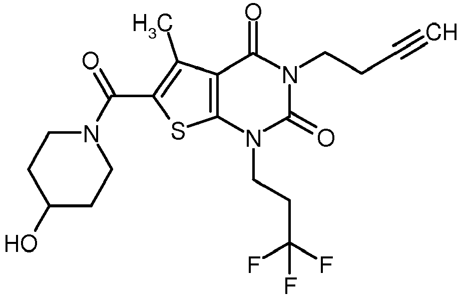

Analog zu dem unter Bsp. 43 beschriebenen Verfahren wurden aus 81 mg (0.162 mmol) der Verbindung aus Bsp. 79A 48 mg (64% d. Th.) der Titelverbindung erhalten.

¹H-NMR (400 MHz, DMSO-d₆, δ/ppm): 4.81 (d, 1H), 4.13 (t, 2H), 4.01 (t, 2H), 3.81-3.71 (m, 3H), 3.27-3.20 (m, 2H), 2.88 (t, 1H), 2.85-2.72 (m, 2H), 2.51-2.44 (m, 2H, teilweise überdeckt vom DMSO-Signal), 2.38 (s, 3H), 1.79-1.72 (m, 2H), 1.39-1.31 (m, 2H).

LC/MS (Methode 1, ESIpos): Rₜ = 0.83 min, m/z = 458 [M+H]⁺.

### Beispiel 62

### 6-[(4-Hydroxypiperidin-1-yl)carbonyl]-3-(2-methoxyethyl)-5-methyl-1-(3,3,3-trifluorpropyl)-thieno[2,3-d]pyrimidin-2,4(1H,3H)-dion

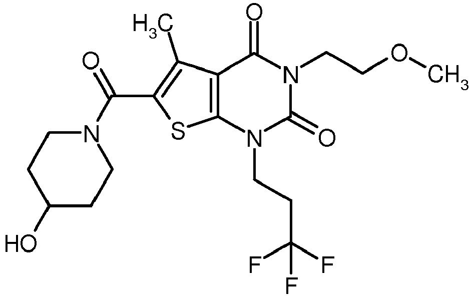

Analog zu dem unter Bsp. 43 beschriebenen Verfahren wurden aus 88 mg (0.174 mmol) der Verbindung aus Bsp. 79A 40 mg (49% d. Th.) der Titelverbindung erhalten. Das nach der präparativen HPLC erhaltene Produkt wurde hier nochmals mit Pentan verrührt.

¹H-NMR (400 MHz, DMSO-d₆, δ/ppm): 4.81 (d, 1H), 4.13 (t, 2H), 4.06 (t, 2H), 3.82-3.71 (m, 3H), 3.50 (t, 2H), 3.27-3.20 (m, 2H), 3.24 (s, 3H), 2.85-2.72 (m, 2H), 2.37 (s, 3H), 1.79-1.72 (m, 2H), 1.40-1.30 (m, 2H).

LC/MS (Methode 1, ESIpos): Rₜ = 0.77 min, m/z = 464 [M+H]⁺.

### Beispiel 63

### 3-(2-Cyclopropylethyl)-6-[(4-hydroxypiperidin-1-yl)carbonyl]-5-methyl-1-(3,3,3-trifluorpropyl)-thieno[2,3-d]pyrimidin-2,4(1H,3H)-dion

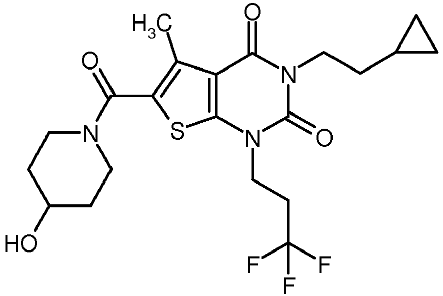

Analog zu dem unter Bsp. 43 beschriebenen Verfahren wurden aus 50 mg (0.097 mmol) der Verbindung aus Bsp. 80A 37 mg (80% d. Th.) der Titelverbindung erhalten.

¹H-NMR (400 MHz, DMSO-d₆, δ/ppm): 4.81 (d, 1H), 4.12 (t, 2H), 3.95 (t, 2H), 3.81-3.71 (m, 3H), 3.27-3.20 (m, 2H), 2.85-2.73 (m, 2H), 2.37 (s, 3H), 1.79-1.72 (m, 2H), 1.44 (quart, 2H), 1.39-1.30 (m, 2H), 0.72-0.65 (m, 1H), 0.41-0.37 (m, 2H), 0.01 (m, 2H, weitgehend überdeckt vom TMS-Signal).

LC/MS (Methode 1, ESIpos): Rₜ = 0.95 min, m/z = 474 [M+H]⁺.

### Beispiel 64

### 6-[(4-Hydroxypiperidin-1-yl)carbonyl]-3-isobutyl-5-methyl-1-(3,3,3-trifluorpropyl)thieno[2,3-d]-pyrimidin-2,4(1H,3H)-dion

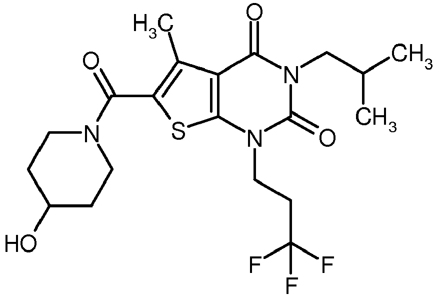

Analog zu dem unter Bsp. 43 beschriebenen Verfahren wurden aus 87 mg (0.173 mmol) der Verbindung aus Bsp. 81A 65 mg (81% d. Th.) der Titelverbindung erhalten.

¹H-NMR (400 MHz, DMSO-d₆, δ/ppm): 4.81 (d, 1H), 4.13 (t, 2H), 3.82-3.70 (m, 5H), 3.27-3.20 (m, 2H), 2.85-2.73 (m, 2H), 2.37 (s, 3H), 2.09-1.99 (m, 1H), 1.80-1.72 (m, 2H), 1.40-1.30 (m, 2H), 0.86 (d, 6H).

LC/MS (Methode 1, ESIpos): Rₜ = 0.96 min, m/z = 462 [M+H]⁺.

### Beispiel 65

### 6-[(4-Hydroxypiperidin-1-yl)carbonyl]-3-(2-methoxypropyl)-5-methyl-1-(3,3,3-trifluorpropyl)-thieno[2,3-d]pyrimidin-2,4(1H,3H)-dion (Racemat)

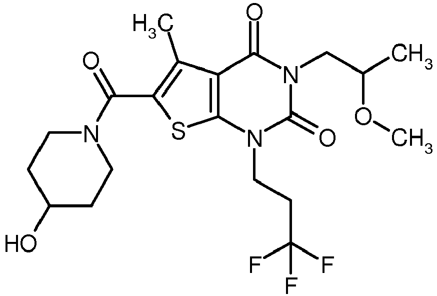

Analog zu dem unter Bsp. 43 beschriebenen Verfahren wurden aus 50 mg (0.096 mmol) der Verbindung aus Bsp. 82A 26 mg (51% d. Th., 90% Reinheit) der Titelverbindung erhalten.

¹H-NMR (400 MHz, DMSO-d₆, δ/ppm): 4.81 (d, 1H), 4.13 (m, 2H), 4.06 (m, 1H), 3.82-3.71 (m, 4H), 3.64 (m, 1H), 3.28-3.23 (m, 2H), 3.22 (s, 3H), 2.85-2.73 (m, 2H), 2.38 (s, 3H), 1.80-1.72 (m, 2H), 1.40-1.30 (m, 2H), 1.06 (d, 3H).

LC/MS (Methode 1, ESIpos): Rₜ = 0.81 min, m/z = 478 [M+H]⁺.

### Beispiel 66

### 6-[(4-Hydroxypiperidin-1-yl)carbonyl]-5-methyl-3-(3,3,3-trifluor-2-methoxypropyl)-1-(3,3,3-tri-fluorpropyl)thieno[2,3-d]pyrimidin-2,4(1H,3H)-dion (Racemat)

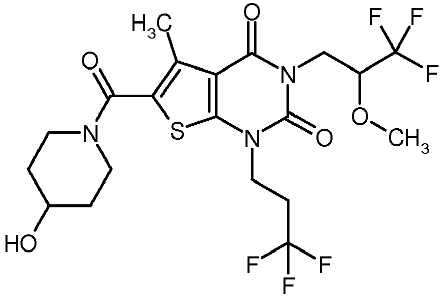

Analog zu dem unter Bsp. 51 beschriebenen Verfahren wurden aus 140 mg (0.312 mmol) der Verbindung aus Bsp. 103A und 38 mg (0.375 mmol) 4-Hydroxypiperidin 162 mg (97% d. Th.) der Titelverbindung erhalten.

¹H-NMR (400 MHz, DMSO-d₆, δ/ppm): 4.81 (d, 1H), 4.29-4.14 (m, 4H), 4.05 (dd, 1H), 3.82-3.71 (m, 3H), 3.43 (s, 3H), 3.28-3.21 (m, 2H), 2.87-2.75 (m, 2H), 2.38 (s, 3H), 1.80-1.73 (m, 2H), 1.41-1.32 (m, 2H).

LC/MS (Methode 1, ESIpos): Rₜ = 0.92 min, m/z = 532 [M+H]⁺.

### Beispiel 67

### 6-[(4-Hydroxy-4-methylpiperidin-1-yl)carbonyl]-5-methyl-3-(2-phenylethyl)-1-(3,3,3-trifluor-propyl)thieno[2,3-d]pyrimidin-2,4(1H,3H)-dion

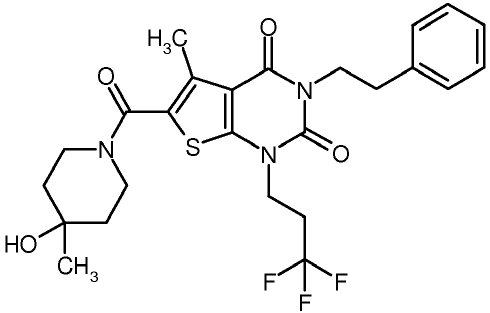

Eine Lösung von 80 mg (0.188 mmol) der Verbindung aus Bsp. 43A in 2 ml wasserfreiem DMF wurde nacheinander mit 26 mg (0.225 mmol) 4-Methylpiperidin-4-ol [kommerziell erhältlich; Lit. z.B.: J. M. McManus et al., J. Med. Chem. 1965, 8 (6), 766-776], 43 µl (0.244 mmol) *N,N*-Diisopropylethylamin und 86 mg (0.225 mmol) HATU versetzt. Nach einer Reaktionszeit von ca. 16 h bei RT wurde das Reaktionsgemisch direkt mittels präparativer HPLC in seine Komponenten aufgetrennt (Methode 6). Die Produktfraktionen wurden vereinigt und eingeengt und der Rückstand im Hochvakuum getrocknet. Es wurden 79 mg (80% d. Th.) der Titelverbindung erhalten.

¹H-NMR (400 MHz, DMSO-d₆, δ/ppm): 7.33-7.29 (m, 2H), 7.26-7.20 (m, 3H), 4.47 (breit, 1H), 4.12 (t, 2H), 4.06 (m, 2H), 3.79-3.65 (m, breit, 2H), 3.37-3.29 (m, breit, 2H, weitgehend überdeckt vom Wassersignal), 2.83 (m, 2H), 2.82-2.70 (m, 2H), 2.38 (s, 3H), 1.54-1.41 (m, 4H), 1.16 (s, 3H).

LC/MS (Methode 1, ESIpos): Rₜ = 1.07 min, m/z = 524 [M+H]⁺.

### Beispiel 68

### 6-{[4-Hydroxy-4-(hydroxymethyl)piperidin-1-yl]carbonyl}-5-methyl-3-(2-phenylethyl)-1-(3,3,3-trifluorpropyl)thieno[2,3-d]pyrimidin-2,4(1H,3H)-dion

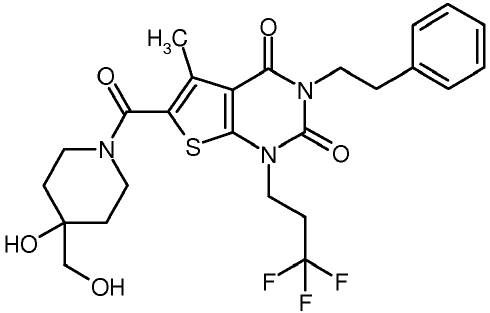

Eine Lösung von 80 mg (0.188 mmol) der Verbindung aus Bsp. 43A in 2 ml wasserfreiem DMF wurde nacheinander mit 38 mg (0.225 mmol) 4-(Hydroxymethyl)piperidin-4-ol-Hydrochlorid [kommerziell erhältlich; Lit. z.B.: WO 2005/103037-A2, Beispiel A3b (freie Base); US 2011/ 288065-A1, Example 105 / Step 1 (Hydrochlorid)], 43 µl (0.244 mmol) *N,N*-Diisopropylethylamin und 86 mg (0.225 mmol) HATU versetzt. Nach einer Reaktionszeit von ca. 16 h bei RT wurde das Reaktionsgemisch direkt mittels präparativer HPLC in seine Komponenten aufgetrennt (Methode 11). Die Produktfraktionen wurden vereinigt und eingeengt und der Rückstand im Hochvakuum getrocknet. Es wurden 84 mg (83% d. Th.) der Titelverbindung erhalten.

¹H-NMR (400 MHz, CDCl₃, δ/ppm): 7.32-7.21 (m, 5H, teilweise überlagert vom CHCl₃-Signal), 4.21 (m, 2H), 4.14 (m, 2H), 4.05 (breit, 2H), 3.52 (d, 2H), 3.49-3.40 (m, 2H), 2.96-2.92 (m, 2H), 2.64-2.52 (m, 2H), 2.51 (s, 3H), 2.15 (s, 1H), 1.86 (t, 1H), 1.76-1.70 (m, 2H), 1.57-1.51 (m, 2H, teilweise überlagert vom Wassersignal).

LC/MS (Methode 1, ESIpos): Rₜ = 0.94 min, m/z = 540 [M+H]⁺.

### Beispiel 69

### 6-{[4-Hydroxy-4-(trifluormethyl)piperidin-1-yl]carbonyl}-5-methyl-3-(2-phenylethyl)-1-(3,3,3-tri-fluorpropyl)thieno[2,3-d]pyrimidin-2,4(1H,3H)-dion

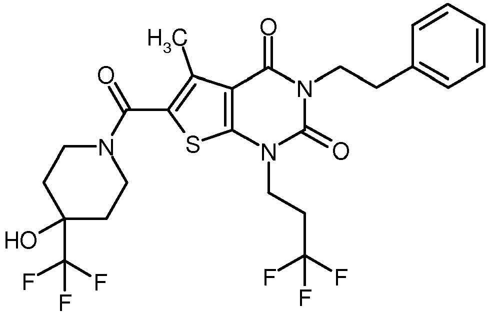

Analog zu dem unter Bsp. 51 beschriebenen Verfahren wurden aus 80 mg (0.188 mmol) der Verbindung aus Bsp. 43A und 46 mg (0.225 mmol) 4-(Trifluormethyl)piperidin-4-ol [kommerziell erhältlich; Lit. z.B.: WO 2005/103002-A2, Zwischenprodukt 1] 90 mg (83% d. Th.) der Titelverbindung erhalten. Für die präparative HPLC wurde in diesem Fall Methode 6 verwendet.

¹H-NMR (400 MHz, DMSO-d₆, δ/ppm): 7.34-7.29 (m, 2H), 7.26-7.21 (m, 3H), 6.20 (s, 1H), 4.13 (t, 2H), 4.07 (m, 2H), 4.00 (breit, 2H), 3.27-3.19 (m, 2H), 2.85-2.81 (m, 2H), 2.82-2.71 (m, 2H), 2.40 (s, 3H), 1.77-1.61 (m, 4H).

LC/MS (Methode 1, ESIpos): Rₜ = 1.14 min, m/z = 577 [M+H]⁺.

### Beispiel 70

### 3-(2,2-Difluor-2-phenylethyl)-6-{[4-hydroxy-4-(hydroxymethyl)piperidin-1-yl]carbonyl}-5-methyl-1-(3,3,3-trifluorpropyl)thieno[2,3-d]pyrimidin-2,4(1H,3H)-dion

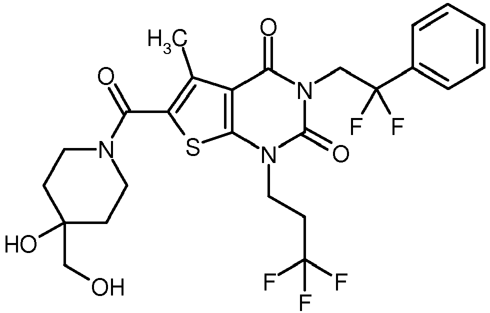

Eine Lösung von 80 mg (0.173 mmol) der Verbindung aus Bsp. 100A in 2.5 ml wasserfreiem DMF wurde nacheinander mit 35 mg (0.208 mmol) 4-(Hydroxymethyl)piperidin-4-ol-Hydrochlorid [kommerziell erhältlich; Lit. z.B.: WO 2005/103037-A2, Beispiel A3b (freie Base); US 2011/ 288065-A1, Example 105 / Step 1 (Hydrochlorid)], 75 µl (0.433 mmol) *N,N*-Diisopropylethylamin und 79 mg (0.208 mmol) HATU versetzt. Nach einer Reaktionszeit von ca. 16 h bei RT wurde das Reaktionsgemisch direkt mittels präparativer HPLC in seine Komponenten aufgetrennt (Methode 5). Die Produktfraktionen wurden vereinigt und eingeengt und der Rückstand im Hochvakuum getrocknet. Es wurden 73 mg (69% d. Th., 95% Reinheit) der Titelverbindung erhalten.

¹H-NMR (400 MHz, DMSO-d₆, δ/ppm): 7.57-7.50 (m, 5H), 4.62 (t, 2H), 4.39 (breit, 1H), 4.12 (t, 2H), 3.92-3.78 (m, breit, 2H), 3.34-3.24 (m, 2H, teilweise überlagert vom Wassersignal), 3.21 (s, 2H), 2.79-2.67 (m, 2H), 2.36 (s, 3H), 1.60-1.51 (m, 2H), 1.44-1.38 (m, 2H).

LC/MS (Methode 1, ESIpos): Rₜ = 0.93 min, m/z = 576 [M+H]⁺.

### Beispiel 71

### 3-(2,2-Difluor-2-phenylethyl)-6-{[4-hydroxy-4-(hydroxymethyl)piperidin-1-yl]carbonyl}-1-(2-methoxyethyl)-5-methylthieno[2,3-d]pyrimidin-2,4(1H,3H)-dion

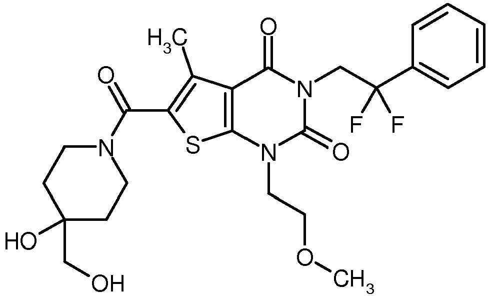

Analog zu dem unter Bsp. 51 beschriebenen Verfahren wurden aus 80 mg (0.188 mmol) der Verbindung aus Bsp. 101A und 38 mg (0.226 mmol) 4-(Hydroxymethyl)piperidin-4-ol-Hydrochlorid [kommerziell erhältlich; Lit. z.B.: WO 2005/103037-A2, Beispiel A3b (freie Base); US 2011/ 288065-A1, Example 105 / Step 1 (Hydrochlorid)] 75 mg (74% d. Th.) der Titelverbindung erhalten.

¹H-NMR (400 MHz, DMSO-d₆, δ/ppm): 7.56-7.51 (m, 5H), 4.62 (t, 2H), 4.36 (breit, 1H), 4.04 (t, 2H), 3.91-3.77 (m, breit, 2H), 3.31-3.25 (m, 2H, teilweise überlagert vom Wassersignal), 3.25 (s, 3H), 3.21 (s, 2H), 2.35 (s, 3H), 1.59-1.51 (m, 2H), 1.44-1.38 (m, 2H).

LC/MS (Methode 1, ESIpos): Rₜ = 0.86 min, m/z = 538 [M+H]⁺.

### Beispiel 72

### 3-Ethyl-6-[(9-hydroxy-3-azabicyclo[3.3.1]non-3-yl)carbonyl]-5-methyl-1-(3,3,3-trifluorpropyl)-thieno[2,3-d]pyrimidin-2,4(1H,3H)-dion (α-Epimer)

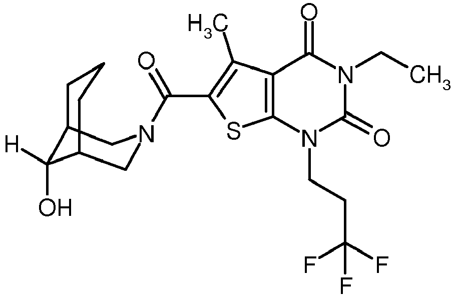

Eine Lösung von 100 mg (0.285 mmol) der Verbindung aus Bsp. 52A in 3 ml wasserfreiem DMF wurde nacheinander mit 48 mg (0.343 mmol) 3-Azabicyclo[3.3.1]nonan-9-ol [Epimerengemisch, kommerziell erhältlich; Lit. z.B.: A. I. Moskalenko, V. I. Boev, Russ. J. Org. Chem. 2010, 46 (10), 1527-1533 (als Hydrochlorid)], 114 µl (0.657 mmol) *N,N*-Diisopropylethylamin und 130 mg (0.343 mmol) HATU versetzt. Nach einer Reaktionszeit von ca. 16 h bei RT wurde das Reaktionsgemisch direkt mittels präparativer HPLC in seine Komponenten aufgetrennt (Methode 5). Es wurden zwei Fraktionen erhalten: 36 mg (26% d. Th.) der Titelverbindung (α-Epimer, wie abgebildet) und 41 mg (29% d. Th.) des korrespondierenden β-Epimers. Die Zuordnung erfolgte analog zu der oben zitierten Literatur über die chemische Verschiebung der epimeren CHOH-Protonen (α: 3.81 ppm, β: 3.62 ppm; in DMSO-d₆).

¹H-NMR (400 MHz, DMSO-d₆, δ/ppm): 4.89 (d, 1H), 4.70-3.60 (sehr breit, 2H), 4.13 (t, 2H), 3.91 (quart, 2H), 3.81 (m, 1H), 3.50-3.00 (sehr breit, 2H, teilweise überdeckt vom Wassersignal), 2.85-2.73 (m, 2H), 2.35 (s, 3H), 2.04-1.93 (m, 2H), 1.77-1.71 (m, 2H), 1.63-1.52 (m, 1H), 1.42-1.33 (m, 3H), 1.13 (t, 3H).

LC/MS (Methode 9, ESIpos): Rₜ = 1.14 min, m/z = 474 [M+H]⁺.

### Beispiel 73

### 3-Ethyl-6-[(4-hydroxy-3-methylpiperidin-1-yl)carbonyl]-5-methyl-1-(3,3,3-trifluorpropyl)thieno-[2,3-d]pyrimidin-2,4(1H,3H)-dion (Enantiomerenpaar 1)

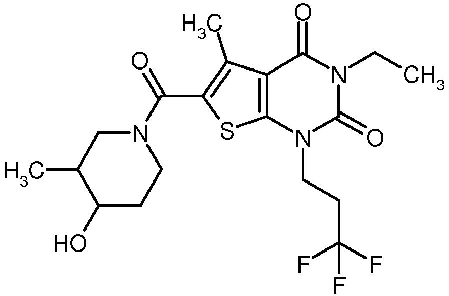

Eine Suspension von 190 mg (0.427 mmol) der racemischen Verbindung aus Bsp. 112A in 20 ml Methanol wurde mit 16 mg (0.427 mmol) Natriumborhydrid versetzt und 2 h bei RT gerührt. Anschließend wurde das Reaktionsgemisch am Rotationsverdampfer bis auf ein kleines Restvolumen eingeengt. Dieser Rückstand wurde dann mittels präparativer HPLC (Methode 5) grob vorgereinigt, so dass alle vier Stereoisomere des Zielprodukts gemeinsam isoliert wurden (185 mg). Die Auftrennung der Stereoisomere erfolgte anschließend mittels präparativer HPLC an chiraler Phase [Säule: Daicel Chiralpak IC, 5 µm, 250 mm x 20 mm; Eluent: Isohexan/Ethanol 7:3; Fluss: 15 ml/ min; Temperatur: 25°C; Detektion: 220 nm]. Dazu wurde das erhaltene Stereoisomerengemisch (185 mg) in 3 ml Ethanol gelöst und in 10 Portionen über die Säule gegeben. Es wurde so eine Auftrennung in die beiden diastereomeren Enantiomerenpaare erreicht. Nach Einengen der jeweiligen Produktfraktionen und Trocknen im Hochvakuum wurden 37 mg (18% d. Th.) der Titelverbindung (Enantiomerenpaar 1) und 62 mg (30% d. Th.) des diastereomeren Enantiomerenpaars 2 erhalten. Die Retentionszeiten (Rₜ) auf einer analytischen HPLC-Säule an chiraler Phase betrugen 8.11 und 8.48 min (Enantiomerenpaar 1) bzw. 9.82 und 10.06 min (Enantiomerenpaar 2) [Säule: Daicel Chiralpak IC, 5 µm, 250 mm x 4.6 mm; Eluent: Isohexan/Ethanol/Diethylamin 70:30:0.2; Fluss: 1 ml/min; Temperatur: 25°C; Detektion: 235 nm].

¹H-NMR (400 MHz, DMSO-d₆, δ/ppm): 4.72 (breit, 1H), 4.12 (t, 2H), 3.91 (quart, 2H), 3.74 (m, 1H), 3.62-3.48 (m, breit, 2H), 3.43 (m, 1H, teilweise überdeckt vom Wassersignal), 3.17 (dd, 1H), 2.85-2.73 (m, 2H), 2.37 (s, 3H), 1.76-1.68 (m, 1H), 1.65-1.54 (m, 2H), 1.13 (t, 3H), 0.83 (d, 3H).

LC/MS (Methode 1, ESIpos): Rₜ = 0.87 min, m/z = 448 [M+H]⁺.

### Beispiel 74

### 3-Ethyl-6-{[4-hydroxy-4-(hydroxymethyl)piperidin-1-yl]carbonyl}-5-methyl-1-(3,3,3-trifluor-propyl)thieno[2,3-d]pyrimidin-2,4(1H,3H)-dion

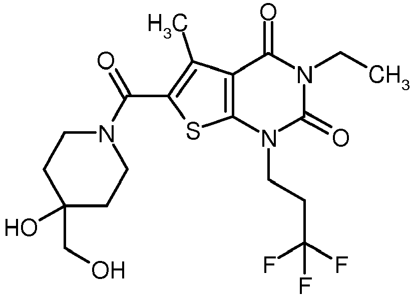

Analog zu dem unter Bsp. 51 beschriebenen Verfahren wurden aus 100 mg (0.285 mmol) der Verbindung aus Bsp. 52A und 57 mg (0.343 mmol) 4-(Hydroxymethyl)piperidin-4-ol-Hydrochlorid [kommerziell erhältlich; Lit. z.B.: WO 2005/103037-A2, Beispiel A3b (freie Base); US 2011/ 288065-A1, Example 105 / Step 1 (Hydrochlorid)] 88 mg (66% d. Th.) der Titelverbindung erhalten. Abweichend zum zuvor beschriebenen Verfahren schloss sich hier an die erste präparative HPLC (nach Methode 5) noch eine zweite HPLC-Reinigung (nach Methode 7) an.

¹H-NMR (400 MHz, DMSO-d₆, δ/ppm): 4.65 (t, 1H), 4.38 (s, 1H), 4.12 (t, 2H), 3.91 (quart, 2H), 3.84 (breit, 2H), 3.27 (breit, 2H, teilweise überlagert vom Wassersignal), 3.20 (d, 2H), 2.85-2.73 (m, 2H), 2.38 (s, 3H), 1.59-1.50 (m, 2H), 1.43-1.37 (m, 2H), 1.12 (t, 3H).

LC/MS (Methode 1, ESIpos): Rₜ = 0.78 min, m/z = 464 [M+H]⁺.

### Beispiel 75

### 3-Ethyl-6-{[4-hydroxy-4-(hydroxymethyl)piperidin-1-yl]carbonyl}-5-methyl-1-[2-(trifluor-methoxy)ethyl]thieno[2,3-d]pyrimidin-2,4(1H,3H)-dion

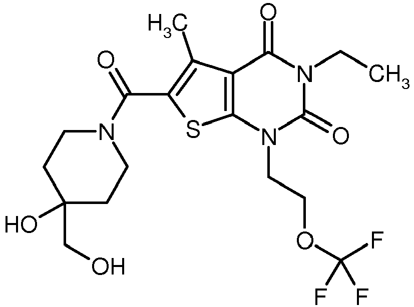

Eine Lösung von 100 mg (0.259 mmol, 95% Reinheit) der Verbindung aus Bsp. 104A in 1.7 ml wasserfreiem DMF wurde nacheinander mit 52 mg (0.311 mmol) 4-(Hydroxymethyl)piperidin-4-ol-Hydrochlorid [kommerziell erhältlich; Lit. z.B.: WO 2005/103037-A2, Beispiel A3b (freie Base); US 2011/288065-A1, Example 105 / Step 1 (Hydrochlorid)], 113 µl (0.648 mmol) *N,N-*Diisopropylethylamin und 118 mg (0.311 mmol) HATU versetzt. Nach einer Reaktionszeit von ca. 16 h bei RT wurde das Reaktionsgemisch direkt mittels präparativer HPLC in seine Komponenten aufgetrennt (Methode 7). Die Produktfraktionen wurden vereinigt und eingeengt und der Rückstand im Hochvakuum getrocknet. Es wurden 103 mg (82% d. Th.) der Titelverbindung erhalten.

¹H-NMR (400 MHz, DMSO-d₆, δ/ppm): 4.63 (t, 1H), 4.41 (t, 2H), 4.36 (s, 1H), 4.22 (t, 2H), 3.91 (quart, 2H), 3.82 (breit, 2H), 3.25 (breit, 2H, teilweise überlagert vom Wassersignal), 3.20 (d, 2H), 2.37 (s, 3H), 1.54 (dt, 2H), 1.43-1.37 (m, 2H), 1.13 (t, 3H).

LC/MS (Methode 9, ESIpos): Rₜ = 0.98 min, m/z = 480 [M+H]⁺.

### Beispiel 76

### 6-[(4-Hydroxy-4-methylpiperidin-1-yl)carbonyl]-5-methyl-3-propyl-1-(3,3,3-trifluorpropyl)thieno-[2,3-d]pyrimidin-2,4(1H,3H)-dion

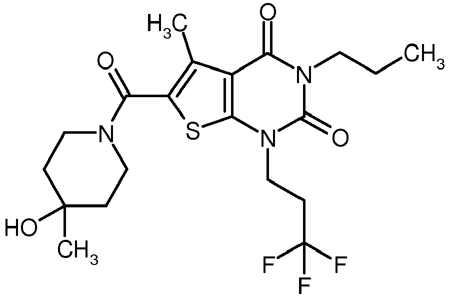

Eine Lösung von 100 mg (0.274 mmol) der Verbindung aus Bsp. 105A in 3 ml wasserfreiem DMF wurde nacheinander mit 38 mg (0.329 mmol) 4-Methylpiperidin-4-ol [kommerziell erhältlich; Lit. z.B.: J. M. McManus et al., J. Med. Chem. 1965, 8 (6), 766-776], 62 µl (0.357 mmol) *N,N*-Diisopropylethylamin und 125 mg (0.329 mmol) HATU versetzt. Nach einer Reaktionszeit von ca. 16 h bei RT wurde das Reaktionsgemisch direkt mittels präparativer HPLC in seine Komponenten aufgetrennt (Methode 5). Die Produktfraktionen wurden vereinigt und eingeengt und der Rückstand im Hochvakuum getrocknet. Es wurden 95 mg (75% d. Th.) der Titelverbindung erhalten.

¹H-NMR (400 MHz, DMSO-d₆, δ/ppm): 4.46 (s, 1H), 4.12 (t, 2H), 3.83 (t, 2H), 3.72 (breit, 2H), 3.34 (m, breit, 2H, teilweise überlagert vom Wassersignal), 2.85-2.72 (m, 2H), 2.68 (s, 3H), 1.61-1.41 (m, 6H), 1.15 (s, 3H), 0.87 (t, 3H).

LC/MS (Methode 1, ESIpos): Rₜ = 0.93 min, m/z = 462 [M+H]⁺.

### Beispiel 77

### 6-{[4-Hydroxy-4-(hydroxymethyl)piperidin-1-yl]carbonyl}-5-methyl-3-propyl-1-(3,3,3-trifluor-propyl)thieno[2,3-d]pyrimidin-2,4(1H,3H)-dion

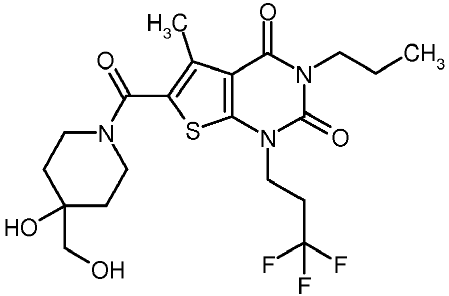

Analog zu dem unter Bsp. 51 beschriebenen Verfahren wurden aus 100 mg (0.274 mmol) der Verbindung aus Bsp. 105A und 55 mg (0.329 mmol) 4-(Hydroxymethyl)piperidin-4-ol-Hydrochlorid [kommerziell erhältlich; Lit. z.B.: WO 2005/103037-A2, Beispiel A3b (freie Base); US 2011/ 288065-A1, Example 105 / Step 1 (Hydrochlorid)] 116 mg (88% d. Th.) der Titelverbindung erhalten.

¹H-NMR (400 MHz, DMSO-d₆, δ/ppm): 4.63 (t, 1H), 4.36 (s, 1H), 4.12 (t, 2H), 3.85 (breit, 2H), 3.83 (t, 2H), 3.28 (breit, 2H, teilweise überlagert vom Wassersignal), 3.21 (d, 2H), 2.85-2.73 (m, 2H), 2.38 (s, 3H), 1.61-1.51 (m, 4H), 1.44-1.37 (m, 2H), 0.87 (t, 3H).

LC/MS (Methode 1, ESIpos): Rₜ = 0.83 min, m/z = 478 [M+H]⁺.

### Beispiel 78

### 6-{[4-Hydroxy-4-(hydroxymethyl)piperidin-1-yl]carbonyl}-1-(2-methoxyethyl)-5-methyl-3-propyl-thieno[2,3-d]pyrimidin-2,4(1H,3H)-dion

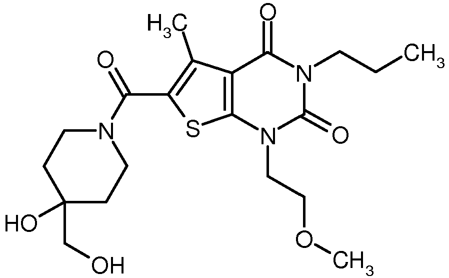

Analog zu dem unter Bsp. 51 beschriebenen Verfahren wurden aus 100 mg (0.306 mmol) der Verbindung aus Bsp. 106A und 62 mg (0.368 mmol) 4-(Hydroxymethyl)piperidin-4-ol-Hydrochlorid [kommerziell erhältlich; Lit. z.B.: WO 2005/103037-A2, Beispiel A3b (freie Base); US 2011/ 288065-A1, Example 105 / Step 1 (Hydrochlorid)] 76 mg (56% d. Th.) der Titelverbindung erhalten. Abweichend zum zuvor beschriebenen Verfahren schloss sich hier an die erste präparative HPLC (nach Methode 5) noch eine zweite HPLC-Reinigung (nach Methode 7) an.

¹H-NMR (400 MHz, DMSO-d₆, δ/ppm): 4.63 (t, 1H), 4.35 (s, 1H), 4.04 (t, 2H), 3.85 (breit, 2H), 3.83 (t, 2H), 3.65 (t, 2H), 3.26 (breit, 2H, teilweise überlagert vom Wassersignal), 3.24 (s, 3H), 3.20 (d, 2H), 2.36 (s, 3H), 1.61-1.50 (m, 4H), 1.43-1.37 (m, 2H), 0.87 (t, 3H).

LC/MS (Methode 1, ESIpos): Rₜ = 0.70 min, m/z = 440 [M+H]⁺.

### Beispiel 79

### 6-[(4-Hydroxy-4-methylpiperidin-1-yl)carbonyl]-5-methyl-3-propyl-1-[2-(trifluormethoxy)ethyl]-thieno[2,3-d]pyrimidin-2,4(1H,3H)-dion

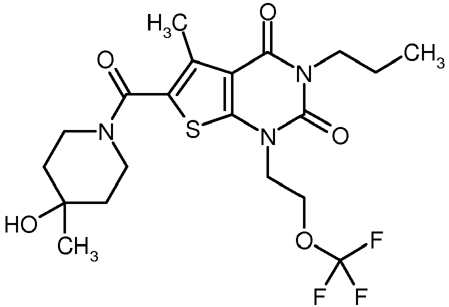

Eine Lösung von 100 mg (0.263 mmol) der Verbindung aus Bsp. 102A in 3 ml wasserfreiem DMF wurde nacheinander mit 36 mg (0.316 mmol) 4-Methylpiperidin-4-ol [kommerziell erhältlich; Lit. z.B.: J. M. McManus et al., J. Med. Chem. 1965, 8 (6), 766-776], 59 µl (0.342 mmol) *N,N*-Diisopropylethylamin und 120 mg (0.316 mmol) HATU versetzt. Nach einer Reaktionszeit von ca. 16 h bei RT wurde das Reaktionsgemisch direkt mittels präparativer HPLC in seine Komponenten aufgetrennt (Methode 5). Die Produktfraktionen wurden vereinigt und eingeengt und der Rückstand im Hochvakuum getrocknet. Es wurden 108 mg (86% d. Th.) der Titelverbindung erhalten.

¹H-NMR (400 MHz, DMSO-d₆, δ/ppm): 4.46 (s, 1H), 4.41 (t, 2H), 4.22 (t, 2H), 3.84 (t, 2H), 3.71 (breit, 2H), 3.34 (m, breit, 2H, teilweise überlagert vom Wassersignal), 2.37 (s, 3H), 1.62-1.40 (m, 6H), 1.15 (s, 3H), 0.87 (t, 3H).

LC/MS (Methode 1, ESIpos): Rₜ = 0.94 min, m/z = 478 [M+H]⁺.

### Beispiel 80

### 6-{[4-Hydroxy-4-(hydroxymethyl)piperidin-1-yl]carbonyl}-5-methyl-3-propyl-1-[2-(trifluor-methoxy)ethyl]thieno[2,3-d]pyrimidin-2,4(1H,3H)-dion

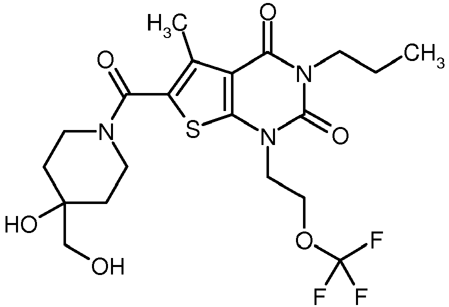

Eine Lösung von 100 mg (0.263 mmol) der Verbindung aus Bsp. 102A in 3 ml wasserfreiem DMF wurde nacheinander mit 53 mg (0.316 mmol) 4-(Hydroxymethyl)piperidin-4-ol-Hydrochlorid [kommerziell erhältlich; Lit. z.B.: WO 2005/103037-A2, Beispiel A3b (freie Base); US 2011/ 288065-A1, Example 105 / Step 1 (Hydrochlorid)], 114 µl (0.657 mmol) *N,N*-Diisopropylethylamin und 120 mg (0.316 mmol) HATU versetzt. Nach einer Reaktionszeit von ca. 16 h bei RT wurde das Reaktionsgemisch direkt mittels präparativer HPLC in seine Komponenten aufgetrennt (Methode 5). Die Produktfraktionen wurden vereinigt und eingeengt und der Rückstand im Hochvakuum getrocknet. Es wurden 78 mg (60% d. Th.) der Titelverbindung erhalten.

¹H-NMR (400 MHz, DMSO-d₆, δ/ppm): 4.63 (t, 1H), 4.41 (t, 2H), 4.36 (s, 1H), 4.22 (t, 2H), 3.84 (t, 2H), 3.82 (breit, 2H), 3.27 (breit, 2H, teilweise überlagert vom Wassersignal), 3.20 (d, 2H), 2.37 (s, 3H), 1.62-1.50 (m, 4H), 1.43-1.37 (m, 2H), 0.87 (t, 3H).

LC/MS (Methode 1, ESIpos): Rₜ = 0.82 min, m/z = 494 [M+H]⁺.

### Beispiel 81

### 6-{[4-Hydroxy-4-(hydroxymethyl)piperidin-1-yl]carbonyl}-5-methyl-3-(3,3,3-trifluor-2-methoxypropyl)-1-(3,3,3-trifluorpropyl)thieno[2,3-d]pyrimidin-2,4(1H,3H)-dion (Racemat)

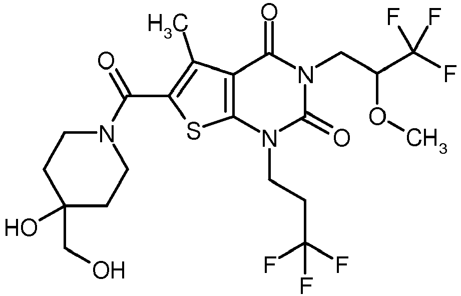

Analog zu dem unter Bsp. 51 beschriebenen Verfahren wurden aus 140 mg (0.312 mmol) der Verbindung aus Bsp. 103A und 63 mg (0.375 mmol) 4-(Hydroxymethyl)piperidin-4-ol-Hydrochlorid [kommerziell erhältlich; Lit. z.B.: WO 2005/103037-A2, Beispiel A3b (freie Base); US 2011/ 288065-A1, Example 105 / Step 1 (Hydrochlorid)] 140 mg (79% d. Th.) der Titelverbindung erhalten.

¹H-NMR (400 MHz, DMSO-d₆, δ/ppm): 4.64 (t, 1H), 4.37 (s, 1H), 4.29-4.14 (m, 4H), 4.05 (dd, 1H), 3.85 (breit, 2H), 3.43 (s, 3H), 3.28 (breit, 2H, teilweise überlagert vom Wassersignal), 3.21 (d, 2H), 2.87-2.75 (m, 2H), 2.38 (s, 3H), 1.56 (dt, 2H), 1.44-1.38 (m, 2H).

LC/MS (Methode 1, ESIpos): Rₜ = 0.87 min, m/z = 562 [M+H]⁺.

### Beispiel 82

### 3-Ethyl-6-[(4-hydroxy-4-methylpiperidin-1-yl)carbonyl]-5-(trifluormethyl)-1-(3,3,3-trifluor-propyl)thieno[2,3-d]pyrimidin-2,4(1H,3H)-dion

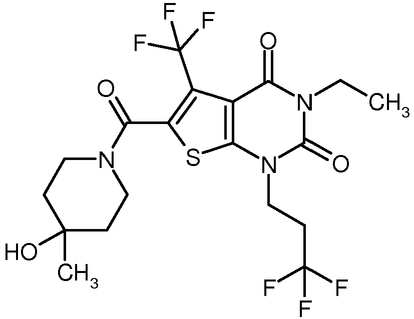

Eine Lösung von 100 mg (0.247 mmol) der Verbindung aus Bsp. 56A in 2 ml wasserfreiem DMF wurde nacheinander mit 31 mg (0.272 mmol) 4-Methylpiperidin-4-ol [kommerziell erhältlich; Lit. z.B.: J. M. McManus et al., J. Med. Chem. 1965, 8 (6), 766-776], 56 µl (0.322 mmol) *N,N*-Diisopropylethylamin und 113 mg (0.297 mmol) HATU versetzt. Nach einer Reaktionszeit von ca. 16 h bei RT wurde das Reaktionsgemisch direkt mittels präparativer HPLC in seine Komponenten aufgetrennt (Methode 5). Die Produktfraktionen wurden vereinigt und eingeeengt und der Rückstand im Hochvakuum getrocknet. Es wurden 96 mg (75% d. Th., 97% Reinheit) der Titelverbindung erhalten.

¹H-NMR (400 MHz, CDCl₃, δ/ppm): 4.38 (m, breit, 1H), 4.24-4.06 (m, 2H), 4.09 (quart, 2H), 3.57-3.43 (m, 1H), 3.41-3.26 (m, 2H), 2.72-2.61 (m, 2H), 1.70-1.48 (m, 4H), 1.33 (s, 3H), 1.27 (t, 3H).

LC/MS (Methode 1, ESIpos): Rₜ = 0.91 min, m/z = 502 [M+H]⁺.

### Beispiel 83

### 3-Ethyl-6-{[4-hydroxy-4-(hydroxymethyl)piperidin-1-yl]carbonyl}-5-(trifluormethyl)-1-(3,3,3-tri-fluorpropyl)thieno[2,3-d]pyrimidin-2,4(1H,3H)-dion

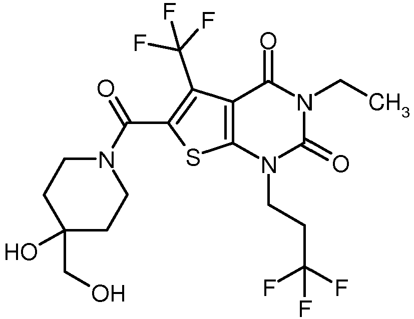

Analog zu dem unter Bsp. 51 beschriebenen Verfahren wurden aus 100 mg (0.247 mmol) der Verbindung aus Bsp. 56A und 46 mg (0.272 mmol) 4-(Hydroxymethyl)piperidin-4-ol-Hydrochlorid [kommerziell erhältlich; Lit. z.B.: WO 2005/103037-A2, Beispiel A3b (freie Base); US 2011/ 288065-A1, Example 105 / Step 1 (Hydrochlorid)] 79 mg (61% d. Th.) der Titelverbindung erhalten.

¹H-NMR (400 MHz, DMSO-d₆, δ/ppm): 4.23 (d, 2H), 4.16 (t, 2H), 3.92 (m, 2H), 3.20 (s, 2H), 3.10 (t, 2H), 2.87-2.75 (m, 2H), 1.60-1.32 (m, 4H), 1.13 (t, 3H).

LC/MS (Methode 1, ESIpos): Rₜ = 0.79 min, m/z = 518 [M+H]⁺.

### Beispiel 84

### 5-(Difluormethyl)-6-[(4-hydroxypiperidin-1-yl)carbonyl]-3-(2-phenylethyl)-1-(3,3,3-trifluor-propyl)thieno[2,3-d]pyrimidin-2,4(1H,3H)-dion

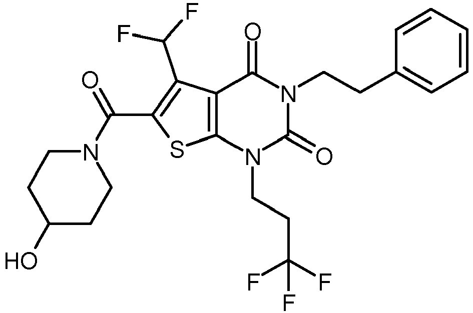

Eine Lösung von 60 mg (0.127 mmol, 97% Reinheit) der Verbindung aus Bsp. 57A in 1.9 ml wasserfreiem DMF wurde nacheinander mit 17 mg (0.165 mmol) 4-Hydroxypiperidin, 29 µl (0.165 mmol) *N,N*-Diisopropylethylamin und 58 mg (0.152 mmol) HATU versetzt. Nach einer Reaktionszeit von ca. 16 h bei RT wurde das Reaktionsgemisch direkt mittels präparativer HPLC in seine Komponenten aufgetrennt (Methode 6). Die Produktfraktionen wurden vereinigt und eingeengt und der Rückstand im Hochvakuum getrocknet. Es wurden 60 mg (88% d. Th.) der Titelverbindung erhalten.

¹H-NMR (400 MHz, DMSO-d₆, δ/ppm): 7.34 (t, 1H), 7.33-7.29 (m, 2H), 7.27-7.23 (m, 3H), 4.84 (d, 1H), 4.15 (t, 2H), 4.07 (m, 2H), 3.96 (breit, 1H), 3.79-3.71 (m, 1H), 3.49 (breit, 1H), 3.22 (breit, 2H), 2.87-2.72 (m, 4H), 1.74 (breit, 2H), 1.41-1.31 (m, 2H).

LC/MS (Methode 2, ESIpos): Rₜ = 2.27 min, m/z = 546 [M+H]⁺.

### Beispiel 85

### 5-(Difluormethyl)-6-[(4-hydroxy-4-methylpiperidin-1-yl)carbonyl]-3-(2-phenylethyl)-1-(3,3,3-tri-fluorpropyl)thieno[2,3-d]pyrimidin-2,4(1H,3H)-dion

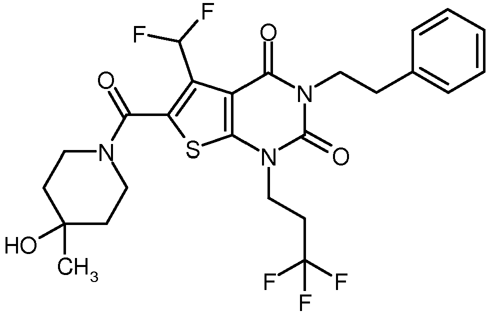

Eine Lösung von 100 mg (0.211 mmol, 97% Reinheit) der Verbindung aus Bsp. 57A in 3.2 ml wasserfreiem DMF wurde nacheinander mit 33 mg (0.274 mmol) 4-Methylpiperidin-4-ol [kommerziell erhältlich; Lit. z.B.: J. M. McManus et al., J. Med. Chem. 1965, 8 (6), 766-776], 48 µl (0.274 mmol) *N,N*-Diisopropylethylamin und 96 mg (0.253 mmol) HATU versetzt. Nach einer Reaktionszeit von ca. 16 h bei RT wurde das Reaktionsgemisch direkt mittels präparativer HPLC in seine Komponenten aufgetrennt (Methode 6). Die Produktfraktionen wurden vereinigt und eingeengt und der Rückstand im Hochvakuum getrocknet. Es wurden 102 mg (86% d. Th.) der Titelverbindung erhalten.

¹H-NMR (400 MHz, DMSO-d₆, δ/ppm): 7.33 (t, 1H), 7.32-7.29 (m, 2H), 7.26-7.21 (m, 3H), 4.48 (s, 1H), 4.15 (t, 2H), 4.08 (m, 2H), 4.08 (breit, 1H), 3.37-3.19 (m, breit, 3H, teilweise überdeckt vom Wassersignal), 2.89-2.72 (m, 4H), 1.56-1.40 (m, breit, 4H), 1.15 (s, 3H).

LC/MS (Methode 1, ESIpos): Rₜ = 1.06 min, m/z = 560 [M+H]⁺.

### Beispiel 86

### 5-(Difluormethyl)-6-{[4-hydroxy-4-(hydroxymethyl)piperidin-1-yl]carbonyl}-3-(2-phenylethyl)-1-(3,3,3-trifluorpropyl)thieno[2,3-d]pyrimidin-2,4(1H,3H)-dion

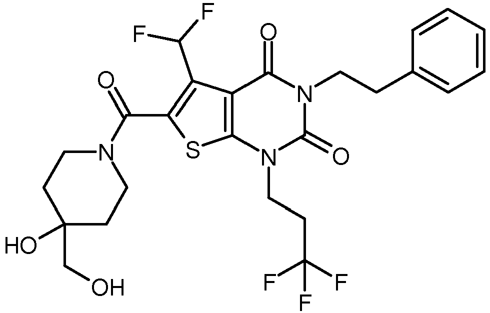

Eine Lösung von 80 mg (0.173 mmol) der Verbindung aus Bsp. 57A in 2.5 ml wasserfreiem DMF wurde nacheinander mit 31 mg (0.225 mmol) 4-(Hydroxymethyl)piperidin-4-ol [kommerziell erhältlich; Lit. z.B.: WO 2005/103037-A2, Beispiel A3b (freie Base); US 2011/288065-A1, Example 105 / Step 1 (Hydrochlorid)], 39 µl (0.225 mmol) *N,N*-Diisopropylethylamin und 79 mg (0.208 mmol) HATU versetzt. Nach einer Reaktionszeit von ca. 16 h bei RT wurde das Reaktionsgemisch direkt mittels präparativer HPLC in seine Komponenten aufgetrennt (Methode 6). Die Produktfraktionen wurden vereinigt und eingeengt und der Rückstand im Hochvakuum getrocknet. Nach Verrühren mit einem Gemisch aus 10 ml Pentan und 1 ml Diisopropylether für 20 min bei RT, Absaugen und neuerlichem Trocknen im Hochvakuum wurden 55 mg (55% d. Th., 96% Reinheit) der Titelverbindung erhalten.

¹H-NMR (400 MHz, DMSO-d₆, δ/ppm): 7.33 (t, 1H), 7.32-7.29 (m, 2H), 7.26-7.21 (m, 3H), 4.67 (t, 1H), 4.39 (s, 1H), 4.22 (breit, 1H), 4.15 (t, 2H), 4.08 (m, 2H), 3.39 (breit, 2H, teilweise überdeckt vom Wassersignal), 3.20 (d, 2H), 3.10 (breit, 1H), 2.88-2.73 (m, 4H), 1.54 (dt, 2H), 1.51-1.31 (breit, 2H).

LC/MS (Methode 1, ESIpos): Rₜ = 0.96 min, m/z = 576 [M+H]⁺.

### Beispiel 87

### 3,5-Diethyl-6-[(4-hydroxypiperidin-1-yl)carbonyl]-1-(3,3,3-trifluorpropyl)thieno[2,3-d]pyrimidin-2,4(1H,3H)-dion

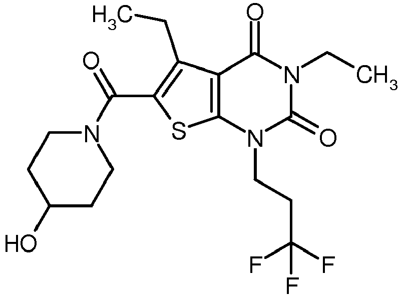

Analog zu dem unter Bsp. 51 beschriebenen Verfahren wurden aus 80 mg (0.220 mmol) der Verbindung aus Bsp. 111A und 24 mg (0.242 mmol) 4-Hydroxypiperidin 96 mg (97% d. Th.) der Titelverbindung erhalten.

¹H-NMR (400 MHz, DMSO-d₆, δ/ppm): 4.80 (d, 1H), 4.12 (t, 2H), 3.92 (quart, 2H), 3.82-3.71 (m, 3H), 3.23 (m, 2H), 2.86-2.74 (m, 4H), 1.79-1.72 (m, 2H), 1.39-1.29 (m, 2H), 1.13 (t, 3H), 1.12 (t, 3H).

LC/MS (Methode 1, ESIpos): Rₜ = 0.86 min, m/z = 448 [M+H]⁺.

### Beispiel 88

### 3,5-Diethyl-6-[(4-hydroxy-4-methylpiperidin-1-yl)carbonyl]-1-(3,3,3-trifluorpropyl)thieno[2,3-d]-pyrimidin-2,4(1H,3H)-dion

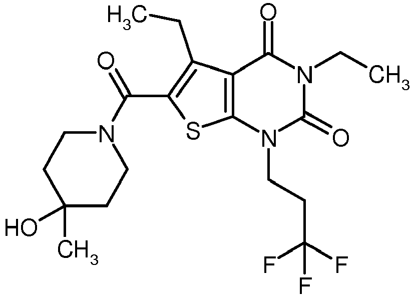

Eine Lösung von 80 mg (0.220 mmol) der Verbindung aus Bsp. 111A in 2 ml wasserfreiem DMF wurde nacheinander mit 28 mg (0.242 mmol) 4-Methylpiperidin-4-ol [kommerziell erhältlich; Lit. z.B.: J. M. McManus et al., J. Med. Chem. 1965, 8 (6), 766-776], 96 µl (0.549 mmol) *N,N*-Diisopropylethylamin und 100 mg (0.263 mmol) HATU versetzt. Nach einer Reaktionszeit von ca. 16 h bei RT wurde das Reaktionsgemisch direkt mittels präparativer HPLC in seine Komponenten aufgetrennt (Methode 5). Die Produktfraktionen wurden vereinigt und eingeengt und der Rückstand im Hochvakuum getrocknet. Es wurden 96 mg (95% d. Th.) der Titelverbindung erhalten.

¹H-NMR (400 MHz, DMSO-d₆, δ/ppm): 4.47 (s, 1H), 4.12 (t, 2H), 3.93 (quart, 2H), 3.72 (breit, 2H), 3.37-3.28 (m, breit, 2H, teilweise überdeckt vom Wassersignal), 2.86-2.74 (m, 4H), 1.53-1.39 (m, 4H), 1.15 (s, 3H), 1.13 (t, 3H), 1.11 (t, 3H).

LC/MS (Methode 1, ESIpos): Rₜ = 0.91 min, m/z = 462 [M+H]⁺.

### Beispiel 89

### 3,5-Diethyl-6-{[4-hydroxy-4-(hydroxymethyl)piperidin-1-yl]carbonyl}-1-(3,3,3-trifluorpropyl)-thieno[2,3-d]pyrimidin-2,4(1H,3H)-dion

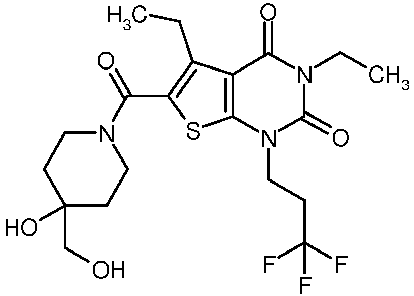

Eine Lösung von 80 mg (0.220 mmol) der Verbindung aus Bsp. 111A in 2 ml wasserfreiem DMF wurde nacheinander mit 40 mg (0.242 mmol) 4-(Hydroxymethyl)piperidin-4-ol-Hydrochlorid [kommerziell erhältlich; Lit. z.B.: WO 2005/103037-A2, Beispiel A3b (freie Base); US 2011/ 288065-A1, Example 105 / Step 1 (Hydrochlorid)], 96 µl (0.549 mmol) *N,N*-Diisopropylethylamin und 100 mg (0.263 mmol) HATU versetzt. Nach einer Reaktionszeit von ca. 16 h bei RT wurde das Reaktionsgemisch direkt mittels präparativer HPLC in seine Komponenten aufgetrennt (Methode 5). Die Produktfraktionen wurden vereinigt und eingeengt und der Rückstand im Hochvakuum getrocknet. Es wurden 89 mg (85% d. Th.) der Titelverbindung erhalten.

¹H-NMR (400 MHz, DMSO-d₆, δ/ppm): 4.64 (t, 1H), 4.37 (s, 1H), 4.12 (t, 2H), 3.92 (quart, 2H), 3.85 (breit, 2H), 3.30-3.21 (m, breit, 2H, teilweise überdeckt vom Wassersignal), 3.21 (d, 2H), 2.86-2.74 (m, 4H), 1.54 (dt, 2H), 1.43-1.37 (m, 2H), 1.13 (t, 3H), 1.12 (t, 3H).

LC/MS (Methode 1, ESIpos): Rₜ = 0.80 min, m/z = 478 [M+H]⁺.

### Beispiel 90

### 6-[(4-Hydroxypiperidin-1-yl)carbonyl]-3-(2-phenylethyl)-1-(3,3,3-trifluorpropyl)thieno[2,3-d]-pyrimidin-2,4(1H,3H)-dion

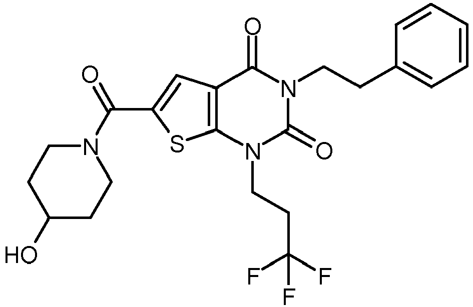

Analog zu dem unter Bsp. 51 beschriebenen Verfahren wurden aus 85 mg (0.196 mmol, 96% Reinheit) der Verbindung aus Bsp. 58A und 22 mg (0.215 mmol) 4-Hydroxypiperidin 79 mg (81% d. Th.) der Titelverbindung erhalten.

¹H-NMR (400 MHz, DMSO-d₆, δ/ppm): 7.55 (s, 1H), 7.32-7.28 (m, 2H), 7.25-7.19 (m, 3H), 4.15 (t, 2H), 4.09 (m, 2H), 4.00-3.92 (m, 2H), 3.39 (breit, 2H), 2.85 (t, 2H), 2.84-2.71 (m, 2H), 1.84-1.77 (m, 2H), 1.45-1.36 (m, 2H).

LC/MS (Methode 1, ESIpos): Rₜ = 0.99 min, m/z = 496 [M+H]⁺.

### Beispiel 91

### 6-[(4-Hydroxy-4-methylpiperidin-1-yl)carbonyl]-3-(2-phenylethyl)-1-(3,3,3-trifluorpropyl)thieno-[2,3-d]pyrimidin-2,4(1H,3H)-dion

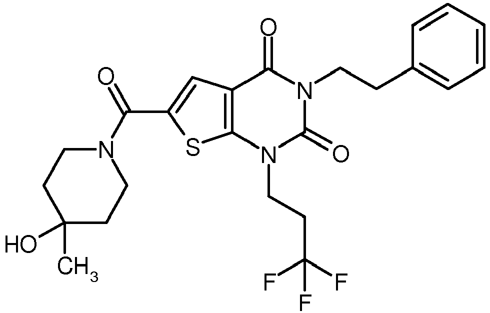

Analog zu dem unter Bsp. 51 beschriebenen Verfahren wurden aus 85 mg (0.196 mmol, 96% Reinheit) der Verbindung aus Bsp. 58A und 22 mg (0.215 mmol) 4-Methylpiperidin-4-ol [kommerziell erhältlich; Lit. z.B.: J. M. McManus et al., J. Med. Chem. 1965, 8 (6), 766-776] 44 mg (44% d. Th.) der Titelverbindung erhalten.

¹H-NMR (400 MHz, DMSO-d₆, δ/ppm): 7.55 (s, 1H), 7.32-7.28 (m, 2H), 7.24-7.19 (m, 3H), 4.15 (t, 2H), 4.09 (m, 2H), 4.00-3.93 (m, 2H), 3.42 (breit, 2H), 2.85 (t, 2H), 2.83-2.71 (m, 2H), 1.57-1.47 (m, 4H), 1.17 (s, 3H).

LC/MS (Methode 1, ESIpos): Rₜ = 1.02 min, m/z = 510 [M+H]⁺.

### Beispiel 92

### 6-{[4-Hydroxy-4-(hydroxymethyl)piperidin-1-yl]carbonyl}-3-(2-phenylethyl)-1-(3,3,3-trifluor-propyl)thieno[2,3-d]pyrimidin-2,4(1H,3H)-dion

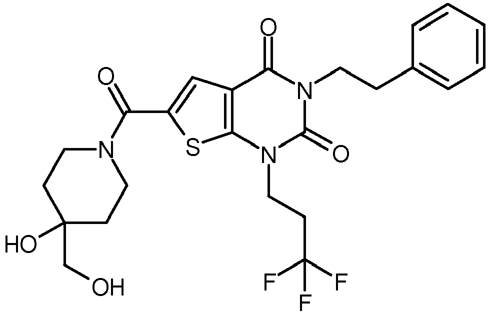

Analog zu dem unter Bsp. 51 beschriebenen Verfahren wurden aus 85 mg (0.196 mmol, 96% Reinheit) der Verbindung aus Bsp. 58A und 36 mg (0.215 mmol) 4-(Hydroxymethyl)piperidin-4-ol-Hydrochlorid [kommerziell erhältlich; Lit. z.B.: WO 2005/103037-A2, Beispiel A3b (freie Base); US 2011/288065-A1, Example 105 / Step 1 (Hydrochlorid)] 86 mg (83% d. Th.) der Titelverbindung erhalten.

¹H-NMR (400 MHz, CDCl₃, δ/ppm): 7.52 (s, 1H), 7.32-7.21 (m, 5H, teilweise überdeckt vom CHCl₃-Signal), 4.29 (breit, 2H), 4.24 (m, 2H), 4.17 (t, 2H), 3.53 (d, 2H), 3.50 (breit, 2H), 2.95 (t, 2H), 2.65-2.54 (m, 2H), 2.21 (breit, 1H), 1.94 (breit, 1H), 1.80-1.74 (m, 2H), 1.61 (m, 2H, teilweise überdeckt vom Wassersignal).

LC/MS (Methode 1, ESIpos): Rₜ = 0.92 min, m/z = 526 [M+H]⁺.

### Beispiel 93

### 3-(2,2-Difluor-2-phenylethyl)-5-methyl-6-[(3-oxopiperazin-1-yl)carbonyl]-1-(3,3,3-trifluorpropyl)-thieno[2,3-d]pyrimidin-2,4(1H,3H)-dion

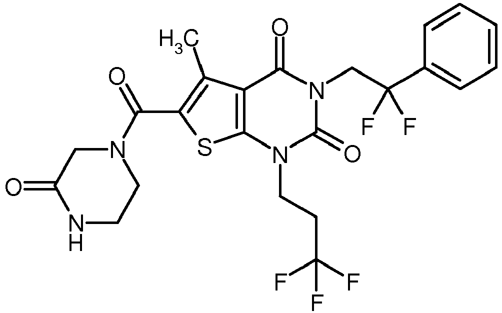

Eine Lösung von 80 mg (0.173 mmol) der Verbindung aus Bsp. 100A in 2.5 ml wasserfreiem DMF wurde nacheinander mit 21 mg (0.208 mmol) 2-Oxopiperazin, 39 µl (0.225 mmol) *N,N*-Diisopropylethylamin und 79 mg (0.208 mmol) HATU versetzt. Nach einer Reaktionszeit von ca. 16 h bei RT wurde das Reaktionsgemisch direkt mittels präparativer HPLC in seine Komponenten aufgetrennt (Methode 5). Die Produktfraktionen wurden vereinigt und eingeengt und der Rückstand im Hochvakuum getrocknet. Es wurden 80 mg (84% d. Th.) der Titelverbindung erhalten.

¹H-NMR (400 MHz, DMSO-d₆, δ/ppm): 8.16 (s, 1H), 7.57-7.49 (m, 5H), 4.63 (t, 2H), 4.13 (t, 2H), 4.05 (s, 2H), 3.69 (t, 2H), 3.25 (m, 2H), 2.79-2.67 (m, 2H), 2.38 (s, 3H).

LC/MS (Methode 1, ESIpos): Rₜ = 0.94 min, m/z = 545 [M+H]⁺.

### Beispiel 94

### 3-(2,2-Difluor-2-phenylethyl)-1-(2-methoxyethyl)-5-methyl-6-[(3-oxopiperazin-1-yl)carbonyl]-thieno[2,3-d]pyrimidin-2,4(1H,3H)-dion

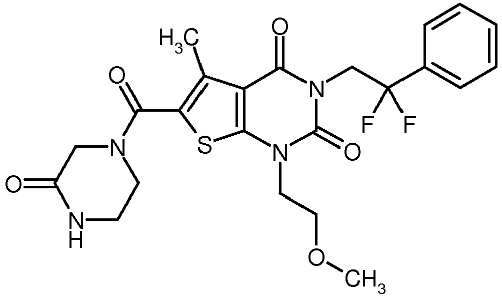

Analog zu dem unter Bsp. 51 beschriebenen Verfahren wurden aus 80 mg (0.188 mmol) der Verbindung aus Bsp. 101A und 23 mg (0.226 mmol) 2-Oxopiperazin 33 mg (32% d. Th., 95% Reinheit) der Titelverbindung erhalten. Das Produkt wurde in diesem Fall durch zweimalige präparative HPLC (jeweils Methode 5) und abschließend durch Verrühren mit Pentan gereinigt.

¹H-NMR (400 MHz, DMSO-d₆, δ/ppm): 8.13 (s, 1H), 7.56-7.50 (m, 5H), 4.63 (t, 2H), 4.05 (s, 2H), 4.04 (t, 2H), 3.69 (t, 2H), 3.59 (t, 2H), 3.24 (s, 3H), 3.24 (m, 2H), 2.37 (s, 3H).

LC/MS (Methode 1, ESIpos): Rₜ = 0.83 min, m/z = 507 [M+H]⁺.

### Beispiel 95

### 5-Methyl-6-[(3-oxopiperazin-1-yl)carbonyl]-3-propyl-1-(3,3,3-trifluorpropyl)thieno[2,3-d]-pyrimidin-2,4(1H,3H)-dion

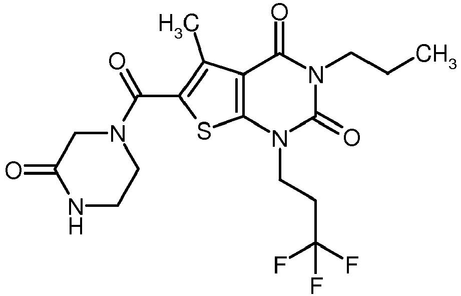

Analog zu dem unter Bsp. 51 beschriebenen Verfahren wurden aus 100 mg (0.274 mmol) der Verbindung aus Bsp. 105A und 33 mg (0.329 mmol) 2-Oxopiperazin 106 mg (86% d. Th.) der Titelverbindung erhalten.

¹H-NMR (400 MHz, DMSO-d₆, δ/ppm): 8.13 (s, 1H), 4.13 (t, 2H), 4.05 (s, 2H), 3.83 (t, 2H), 3.69 (t, 2H), 3.24 (m, 2H), 2.84-2.74 (m, 2H), 2.40 (s, 3H), 1.57 (sext, 2H), 0.87 (t, 3H).

LC/MS (Methode 1, ESIpos): Rₜ = 0.84 min, m/z = 447 [M+H]⁺.

### Beispiel 96

### 5-Methyl-6-[(3-oxopiperazin-1-yl)carbonyl]-3-propyl-1-[2-(trifluormethoxy)ethyl]thieno[2,3-d]-pyrimidin-2,4(1H,3H)-dion

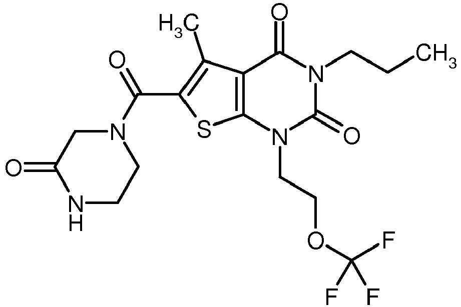

Analog zu dem unter Bsp. 51 beschriebenen Verfahren wurden aus 100 mg (0.263 mmol) der Verbindung aus Bsp. 102A und 32 mg (0.316 mmol) 2-Oxopiperazin 78 mg (64% d. Th.) der Titelverbindung erhalten.

¹H-NMR (400 MHz, DMSO-d₆, δ/ppm): 8.13 (s, 1H), 4.41 (t, 2H), 4.23 (t, 2H), 4.04 (s, 2H), 3.84 (t, 2H), 3.67 (t, 2H), 3.23 (m, 2H), 2.39 (s, 3H), 1.57 (sext, 2H), 0.87 (t, 3H).

LC/MS (Methode 1, ESIpos): Rₜ = 0.83 min, m/z = 463 [M+H]⁺.

### Beispiel 97

### 5-(Difluormethyl)-6-[(3-oxopiperazin-1-yl)carbonyl]-3-(2-phenylethyl)-1-(3,3,3-trifluorpropyl)-thieno[2,3-d]pyrimidin-2,4(1H,3H)-dion

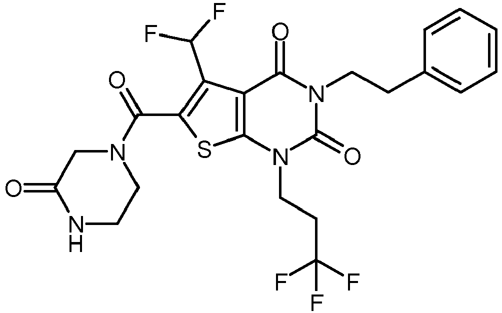

Eine Lösung von 100 mg (0.216 mmol) der Verbindung aus Bsp. 57A in 3.1 ml wasserfreiem DMF wurde nacheinander mit 28 mg (0.281 mmol) 2-Oxopiperazin, 49 µl (0.281 mmol) *N,N*-Diisopropylethylamin und 99 mg (0.260 mmol) HATU versetzt. Nach einer Reaktionszeit von ca. 16 h bei RT wurde das Reaktionsgemisch direkt mittels präparativer HPLC in seine Komponenten aufgetrennt (Methode 6). Die Produktfraktionen wurden vereinigt und eingeengt und der Rückstand im Hochvakuum getrocknet. Nach Verrühren mit einem Gemisch aus 10 ml Pentan und 1 ml Diisopropylether für 20 min bei RT, Absaugen und neuerlichem Trocknen im Hochvakuum wurden 78 mg (66% d. Th.) der Titelverbindung erhalten.

¹H-NMR (400 MHz, DMSO-d₆, δ/ppm): 8.18 (s, 1H), 7.36 (t, 1H), 7.34-7.29 (m, 2H), 7.27-7.21 (m, 3H), 4.15 (t, 2H), 4.08 (m, 2H), 3.90 (breit, 1H), 3.77 (breit, 1H), 3.52 (breit, 2H), 3.22 (breit, 2H), 2.86-2.71 (m, 4H).

LC/MS (Methode 1, ESIpos): Rₜ = 0.98 min, m/z = 545 [M+H]⁺.

### Beispiel 98

### 3,5-Diethyl-6-[(3-oxopiperazin-1-yl)carbonyl]-1-(3,3,3-trifluorpropyl)thieno[2,3-d]pyrimidin-2,4(1H,3H)-dion

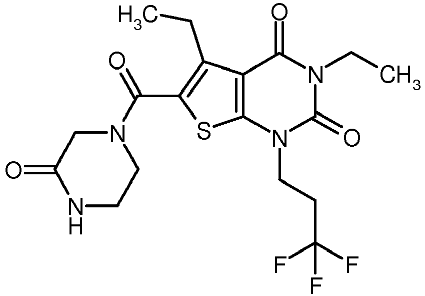

Analog zu dem unter Bsp. 51 beschriebenen Verfahren wurden aus 80 mg (0.220 mmol) der Verbindung aus Bsp. 111A und 24 mg (0.242 mmol) 2-Oxopiperazin 93 mg (95% d. Th.) der Titelverbindung erhalten.

¹H-NMR (400 MHz, DMSO-d₆, δ/ppm): 8.14 (s, 1H), 4.13 (t, 2H), 4.04 (s, 2H), 3.93 (quart, 2H), 3.67 (t, 2H), 3.23 (m, 2H), 2.86-2.74 (m, 4H), 1.13 (t, 3H), 1.11 (t, 3H).

LC/MS (Methode 1, ESIpos): Rₜ = 0.79 min, m/z = 447 [M+H]⁺.

### Beispiel 99

### 6-[(4-Hydroxypiperidin-1-yl)carbonyl]-5-methyl-3-(2-phenylethyl)-1-[2-(trifluormethoxy)ethyl]-thieno[2,3-d]pyrimidin-2,4(1H,3H)-dion

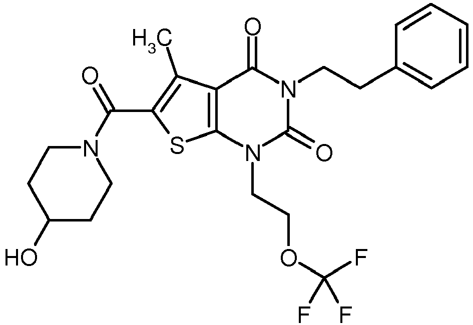

Analog zu dem unter Bsp. 51 beschriebenen Verfahren wurden aus 65 mg (0.147 mmol) der Verbindung aus Bsp. 118A und 17 mg (0.162 mmol) 4-Hydroxypiperidin 72 mg (93% d. Th.) der Titelverbindung erhalten.

¹H-NMR (400 MHz, DMSO-d₆, δ/ppm): 7.32-7.28 (m, 2H), 7.25-7.20 (m, 3H), 4.80 (d, 1H), 4.39 (t, 2H), 4.22 (t, 2H), 4.08 (m, 2H), 3.80-3.71 (m, 3H), 3.23 (m, 2H), 2.84 (m, 2H), 2.37 (s, 3H), 1.79-1.72 (m, 2H), 1.40-1.30 (m, 2H).

LC/MS (Methode 1, ESIpos): Rₜ = 1.05 min, m/z = 526 [M+H]⁺.

### Beispiel 100

### 3-(2-Hydroxy-2-phenylethyl)-6-[(4-hydroxypiperidin-1-yl)carbonyl]-5-methyl-1-(3,3,3-trifluor-propyl)thieno[2,3-d]pyrimidin-2,4(1H,3H)-dion (Enantiomer 1)

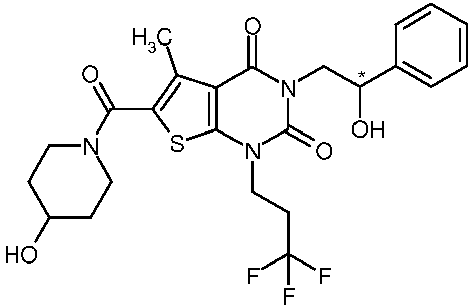

Analog zu dem unter Bsp. 43 beschriebenen Verfahren wurden aus 50 mg (0.088 mmol) der Verbindung aus Bsp. 125A 18 mg (38% d. Th.) der Titelverbindung erhalten.

¹H-NMR (400 MHz, DMSO-d₆, δ/ppm): 7.37-7.31 (m, 4H), 7.28-7.23 (m, 1H), 5.40 (d, 1H), 4.96-4.91 (m, 1H), 4.80 (d, 1H), 4.23 (dd, 1H), 4.11 (t, 2H), 3.85-3.72 (m, 4H), 3.31-3.21 (m, 2H, teilweise überdeckt vom Wassersignal), 2.77-2.66 (m, 2H), 2.38 (s, 3H), 1.80-1.73 (m, 2H), 1.41-1.31 (m, 2H).

LC/MS (Methode 1, ESIpos): Rₜ = 0.86 min, m/z = 526 [M+H]⁺.

Chirale analytische HPLC [Säule: Daicel Chiralcel OD-H, 5 µm, 250 mm x 4.6 mm; Eluent: Isohexan/Ethanol 1:1; Fluss: 1 ml/min; Temperatur: 30°C; Detektion: 220 nm]: Rₜ = 6.65 min; 98% ee.

### Beispiel 101

### 3-(2-Hydroxy-2-phenylethyl)-6-[(4-hydroxypiperidin-1-yl)carbonyl]-5-methyl-1-(3,3,3-trifluor-propyl)thieno[2,3-d]pyrimidin-2,4(1H,3H)-dion (Enantiomer 2)

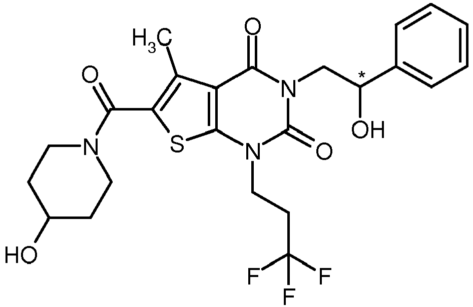

Analog zu dem unter Bsp. 43 beschriebenen Verfahren wurden aus 50 mg (0.088 mmol) der Verbindung aus Bsp. 126A 13 mg (29% d. Th.) der Titelverbindung erhalten.

¹H-NMR (400 MHz, DMSO-d₆, δ/ppm): 7.38-7.32 (m, 4H), 7.28-7.23 (m, 1H), 5.40 (d, 1H), 4.96-4.91 (m, 1H), 4.80 (d, 1H), 4.23 (dd, 1H), 4.11 (t, 2H), 3.85-3.72 (m, 4H), 3.31-3.21 (m, 2H, teilweise überdeckt vom Wassersignal), 2.77-2.66 (m, 2H), 2.38 (s, 3H), 1.80-1.73 (m, 2H), 1.40-1.31 (m, 2H).

LC/MS (Methode 1, ESIpos): Rₜ = 0.86 min, m/z = 526 [M+H]⁺.

Chirale analytische HPLC [Säule: Daicel Chiralcel OD-H, 5 µm, 250 mm x 4.6 mm; Eluent: Isohexan/Ethanol 1:1; Fluss: 1 ml/min; Temperatur: 30°C; Detektion: 220 nm]: Rₜ = 4.98 min; >99% ee.

### Beispiel 102

### 3-(2-Fluor-2-phenylethyl)-6-[(4-hydroxypiperidin-1-yl)carbonyl]-5-methyl-1-(3,3,3-trifluorpropyl)-thieno[2,3-d]pyrimidin-2,4(1H,3H)-dion (Enantiomer 1)

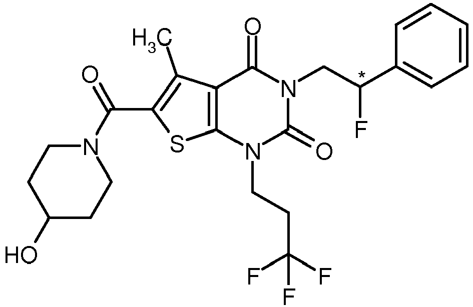

Analog zu dem unter Bsp. 43 beschriebenen Verfahren wurden aus 75 mg (0.133 mmol) der Verbindung aus Bsp. 127A 57 mg (81% d. Th.) der Titelverbindung erhalten. Die Reaktionszeit betrug in diesem Fall 30 min.

¹H-NMR (400 MHz, DMSO-d₆, δ/ppm): 7.49-7.40 (m, 5H), 5.77 (ddd, 1H), 4.80 (d, 1H), 4.63 (m, 1H), 4.15 (t, 2H), 4.00 (ddd, 1H), 3.83-3.72 (m, 3H), 3.29-3.22 (m, 2H), 2.86-2.74 (m, 2H), 2.40 (s, 3H), 1.81-1.73 (m, 2H), 1.41-1.32 (m, 2H).

LC/MS (Methode 1, ESIpos): Rₜ = 0.98 min, m/z = 528 [M+H]⁺.

### Beispiel 103

### 3-(2-Fluor-2-phenylethyl)-6-[(4-hydroxypiperidin-1-yl)carbonyl]-5-methyl-1-(3,3,3-trifluorpropyl)-thieno[2,3-d]pyrimidin-2,4(1H,3H)-dion (Enantiomer 2)

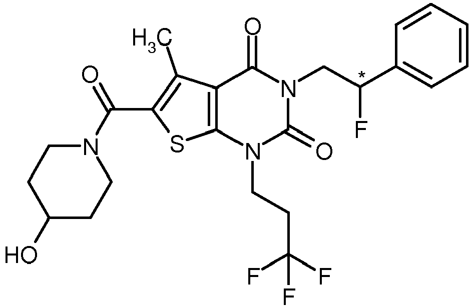

Analog zu dem unter Bsp. 43 beschriebenen Verfahren wurden aus 58 mg (0.102 mmol) der Verbindung aus Bsp. 128A 41 mg (76% d. Th.) der Titelverbindung erhalten. Die Reaktionszeit betrug in diesem Fall 30 min.

¹H-NMR (400 MHz, DMSO-d₆, δ/ppm): 7.49-7.40 (m, 5H), 5.77 (ddd, 1H), 4.82 (d, 1H), 4.63 (m, 1H), 4.16 (t, 2H), 3.99 (ddd, 1H), 3.82-3.71 (m, 3H), 3.29-3.21 (m, 2H), 2.86-2.74 (m, 2H), 2.40 (s, 3H), 1.80-1.73 (m, 2H), 1.41-1.32 (m, 2H).

LC/MS (Methode 1, ESIpos): Rₜ = 0.98 min, m/z = 528 [M+H]⁺.

### Beispiel 104

### 6-[(4-Hydroxypiperidin-1-yl)carbonyl]-5-methyl-3-(3,3,3-trifluor-2-phenylpropyl)-1-(3,3,3-tri-fluorpropyl)thieno[2,3-d]pyrimidin-2,4(1H,3H)-dion (Racemat)

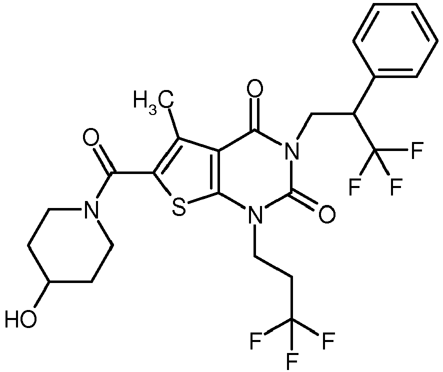

Analog zu dem unter Bsp. 43 beschriebenen Verfahren wurden aus 25 mg (0.040 mmol) der Verbindung aus Bsp. 122A 20 mg (81% d. Th., 95% Reinheit) der Titelverbindung erhalten.

¹H-NMR (400 MHz, CDCl₃, δ/ppm): 7.37-7.31 (m, 5H), 4.62-4.50 (m, 2H), 4.16-4.08 (m, 2H), 4.06-3.98 (m, 2H), 3.95-3.88 (m, 2H), 3.43-3.36 (m, 2H), 2.51-2.40 (m, 2H), 2.47 (s, 3H), 1.97-1.90 (m, 2H), 1.65-1.56 (m, 2H), 1.51 (breit, 1H).

LC/MS (Methode 1, ESIpos): Rₜ = 1.04 min, m/z = 578 [M+H]⁺.

### Beispiel 105

### 3-(2-Hydroxy-2-phenylpropyl)-6-[(4-hydroxypiperidin-1-yl)carbonyl]-5-methyl-1-(3,3,3-trifluor-propyl)thieno[2,3-d]pyrimidin-2,4(1H,3H)-dion (Racemat)

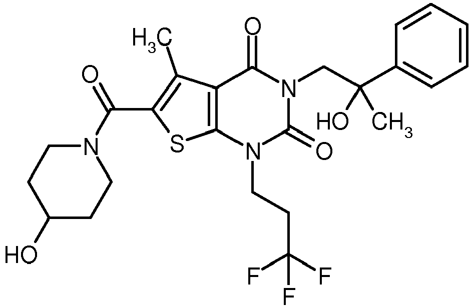

Analog zu dem unter Bsp. 43 beschriebenen Verfahren wurden aus 67 mg (0.115 mmol) der Verbindung aus Bsp. 129A 17 mg (27% d. Th.) der Titelverbindung erhalten.

¹H-NMR (400 MHz, DMSO-d₆, δ/ppm): 7.48 (d, 2H), 7.30 (t, 2H), 7.21 (t, 1H), 5.05 (s, 1H), 4.81 (d, 1H), 4.18 (quart, 2H), 4.12-4.03 (m, 2H), 3.81-3.72 (m, 3H), 3.28-3.21 (m, 2H), 2.75-2.63 (m, 2H), 2.34 (s, 3H), 1.80-1.74 (m, 2H), 1.42 (s, 3H), 1.41-1.32 (m, 2H).

LC/MS (Methode 1, ESIpos): Rₜ = 0.93 min, m/z = 540 [M+H]⁺.

### Beispiel 106

### 3-(4,4-Difluorbut-3-en-1-yl)-6-[(4-hydroxypiperidin-1-yl)carbonyl]-5-methyl-1-(3,3,3-trifluor-propyl)thieno[2,3-d]pyrimidin-2,4(1H,3H)-dion

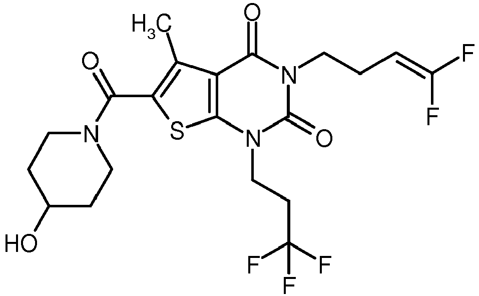

Analog zu dem unter Bsp. 43 beschriebenen Verfahren wurden aus 90 mg (0.167 mmol) der Verbindung aus Bsp. 123A 66 mg (79% d. Th.) der Titelverbindung erhalten.

¹H-NMR (400 MHz, DMSO-d₆, δ/ppm): 4.80 (d, 1H), 4.53 (dtd, 1H), 4.13 (t, 2H), 3.92 (t, 2H), 3.81-3.71 (m, 3H), 3.27-3.21 (m, 2H), 2.84-2.71 (m, 2H), 2.38 (s, 3H), 2.32-2.22 (m, 2H), 1.80-1.72 (m, 2H), 1.40-1.30 (m, 2H).

LC/MS (Methode 1, ESIpos): Rₜ = 0.96 min, m/z = 496 [M+H]⁺.

### Beispiel 107

### 6-[(4-Hydroxypiperidin-1-yl)carbonyl]-5-methyl-3-(3,3,3-trifluor-2-methoxypropyl)-1-(3,3,3-tri-fluorpropyl)thieno[2,3-d]pyrimidin-2,4(1H,3H)-dion (Enantiomer 1)

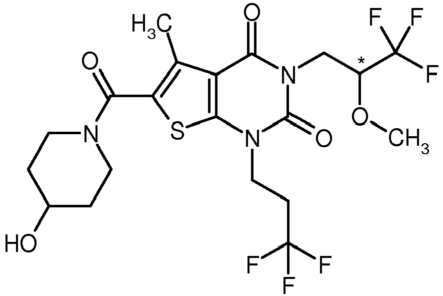

134 mg (0.252 mmol) der racemischen Verbindung aus Bsp. 66 wurden in 4 ml Isopropanol gelöst und in 13 Portionen mittels präparativer HPLC an chiraler Phase in die Enantiomere getrennt [Säule: Daicel Chiralcel OX-H, 5 µm, 250 mm x 20 mm; Eluent: Isohexan/Isopropanol 65:35; Fluss: 15 ml/min; Temperatur: 40°C; Detektion: 220 nm]. Nach Einengen der Produktfraktionen und Trocknen des Rückstands im Hochvakuum wurden 32 mg (47% d. Th.) von Enantiomer 1 erhalten.

¹H-NMR (400 MHz, DMSO-d₆, δ/ppm): 4.81 (d, 1H), 4.29-4.14 (m, 4H), 4.05 (dd, 1H), 3.82-3.71 (m, 3H), 3.43 (s, 3H), 3.28-3.21 (m, 2H), 2.87-2.75 (m, 2H), 2.38 (s, 3H), 1.80-1.73 (m, 2H), 1.40-1.31 (m, 2H).

LC/MS (Methode 1, ESIpos): Rₜ = 0.92 min, m/z = 532 [M+H]⁺.

Chirale analytische HPLC [Säule: LUX Cellulose 4, 5 µm, 250 mm x 4.6 mm; Eluent: Isohexan/ Isopropanol 60:40; Fluss: 1 ml/min; Temperatur: 40°C; Detektion: 220 nm]: Rₜ = 7.47 min; 99.9% ee.

### Beispiel 108

### 6-[(4-Hydroxypiperidin-1-yl)carbonyl]-5-methyl-3-(3,3,3-trifluor-2-methoxypropyl)-1-(3,3,3-tri-fluorpropyl)thieno[2,3-d]pyrimidin-2,4(1H,3H)-dion (Enantiomer 2)

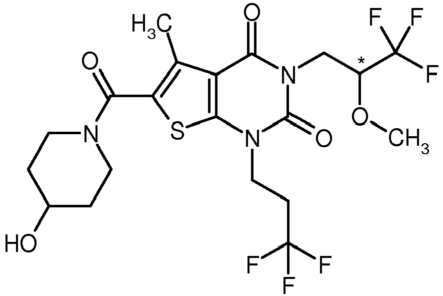

134 mg (0.252 mmol) der racemischen Verbindung aus Bsp. 66 wurden in 4 ml Isopropanol gelöst und in 13 Portionen mittels präparativer HPLC an chiraler Phase in die Enantiomere getrennt [Säule: Daicel Chiralcel OX-H, 5 µm, 250 mm x 20 mm; Eluent: Isohexan/Isopropanol 65:35; Fluss: 15 ml/min; Temperatur: 40°C; Detektion: 220 nm]. Nach Einengen der Produktfraktionen und Trocknen des Rückstands im Hochvakuum wurden 53 mg (79% d. Th.) von Enantiomer 2 erhalten.

¹H-NMR (400 MHz, DMSO-d₆, δ/ppm): 4.81 (d, 1H), 4.29-4.14 (m, 4H), 4.05 (dd, 1H), 3.82-3.71 (m, 3H), 3.43 (s, 3H), 3.28-3.21 (m, 2H), 2.87-2.75 (m, 2H), 2.38 (s, 3H), 1.80-1.73 (m, 2H), 1.40-1.31 (m, 2H).

LC/MS (Methode 1, ESIpos): Rₜ = 0.92 min, m/z = 532 [M+H]⁺.

Chirale analytische HPLC [Säule: LUX Cellulose 4, 5 µm, 250 mm x 4.6 mm; Eluent: Isohexan/ Isopropanol 60:40; Fluss: 1 ml/min; Temperatur: 40°C; Detektion: 220 nm]: Rₜ = 9.67 min; 99.9% ee.

### Beispiel 109

### 6-[(4-Hydroxy-4-methylpiperidin-1-yl)carbonyl]-5-methyl-3-(2-phenylethyl)-1-[2-(trifluor-methoxy)ethyl]thieno[2,3-d]pyrimidin-2,4(1H,3H)-dion

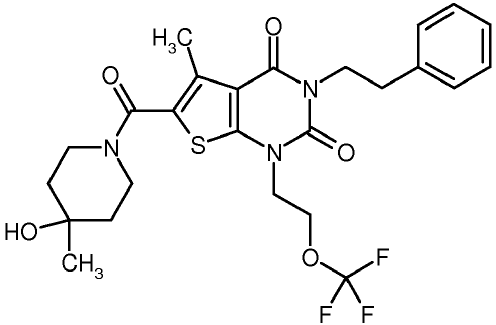

Analog zu dem unter Bsp. 51 beschriebenen Verfahren wurden aus 65 mg (0.147 mmol) der Verbindung aus Bsp. 118A und 19 mg (0.162 mmol) 4-Methylpiperidin-4-ol [kommerziell erhältlich; Lit. z.B.: J. M. McManus et al., J. Med. Chem. 1965, 8 (6), 766-776] 65 mg (79% d. Th.) der Titelverbindung erhalten.

¹H-NMR (400 MHz, DMSO-d₆, δ/ppm): 7.33-7.28 (m, 2H), 7.25-7.20 (m, 3H), 4.46 (s, 1H), 4.39 (t, 2H), 4.22 (t, 2H), 4.08 (m, 2H), 3.76-3.65 (breit, 2H), 3.37-3.29 (m, 2H, teilweise überlagert vom Wassersignal), 2.84 (m, 2H), 2.37 (s, 3H), 1.54-1.40 (m, 4H), 1.15 (s, 3H).

LC/MS (Methode 1, ESIpos): Rₜ = 1.10 min, m/z = 540 [M+H]⁺.

### Beispiel 110

### 6-{[4-Hydroxy-4-(hydroxymethyl)piperidin-1-yl]carbonyl}-5-methyl-3-(2-phenylethyl)-1-[2-(tri-fluormethoxy)ethyl]thieno[2,3-d]pyrimidin-2,4(1H,3H)-dion

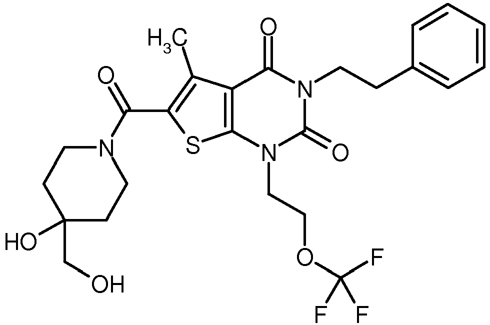

Analog zu dem unter Bsp. 51 beschriebenen Verfahren wurden aus 65 mg (0.147 mmol) der Verbindung aus Bsp. 118A und 27 mg (0.162 mmol) 4-(Hydroxymethyl)piperidin-4-ol-Hydrochlorid [kommerziell erhältlich; Lit. z.B.: WO 2005/103037-A2, Beispiel A3b (freie Base); US 2011/ 288065-A1, Example 105 / Step 1 (Hydrochlorid)] 73 mg (90% d. Th.) der Titelverbindung erhalten.

¹H-NMR (400 MHz, DMSO-d₆, δ/ppm): 7.32-7.28 (m, 2H), 7.25-7.20 (m, 3H), 4.63 (t, 1H), 4.39 (t, 2H), 4.36 (s, 1H), 4.22 (t, 2H), 4.08 (m, 2H), 3.89-3.77 (breit, 2H), 3.31-3.22 (m, 2H, teilweise überdeckt vom Wassersignal), 3.21 (d, 2H), 2.84 (m, 2H), 2.38 (s, 3H), 1.59-1.51 (m, 2H), 1.43-1.37 (m, 2H).

LC/MS (Methode 1, ESIpos): Rₜ = 0.99 min, m/z = 556 [M+H]⁺.

### Beispiel 111

### 3-Ethyl-6-[(4-hydroxy-4-methylpiperidin-1-yl)carbonyl]-5-methyl-1-[2-(trifluormethoxy)ethyl]-thieno[2,3-d]pyrimidin-2,4(1H,3H)-dion

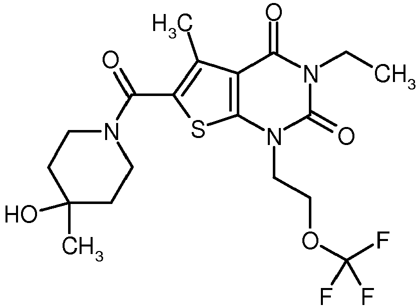

Analog zu dem unter Bsp. 51 beschriebenen Verfahren wurden aus 100 mg (0.273 mmol) der Verbindung aus Bsp. 104A und 38 mg (0.328 mmol) 4-Methylpiperidin-4-ol [kommerziell erhältlich; Lit. z.B.: J. M. McManus et al., J. Med. Chem. 1965, 8 (6), 766-776] 98 mg (77% d. Th.) der Titelverbindung erhalten. Die Reaktionszeit betrug in diesem Fall 1 h.

¹H-NMR (400 MHz, DMSO-d₆, δ/ppm): 4.45 (s, 1H), 4.41 (t, 2H), 4.22 (t, 2H), 3.91 (quart, 2H), 3.75-3.65 (breit, 2H), 3.37-3.29 (m, 2H, teilweise überlagert vom Wassersignal), 2.37 (s, 3H), 1.53-1.40 (m, 4H), 1.15 (s, 3H), 1.13 (t, 3H).

LC/MS (Methode 1, ESIpos): Rₜ = 0.88 min, m/z = 464 [M+H]⁺.

### Beispiel 112

### 3-Ethyl-6-[(4-ethyl-4-hydroxypiperidin-1-yl)carbonyl]-5-methyl-1-[2-(trifluormethoxy)ethyl]-thieno[2,3-d]pyrimidin-2,4(1H,3H)-dion

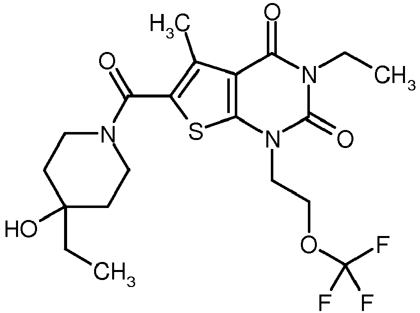

Analog zu dem unter Bsp. 51 beschriebenen Verfahren wurden aus 80 mg (0.218 mmol) der Verbindung aus Bsp. 104A und 44 mg (0.262 mmol) 4-Ethylpiperidin-4-ol-Hydrochlorid [kommerziell erhältlich; Lit. z.B.: US 2004/0067931-A1, Example 3.232 (freie Base)] 97 mg (93% d. Th.) der Titelverbindung erhalten.

¹H-NMR (400 MHz, DMSO-d₆, δ/ppm): 4.41 (t, 2H), 4.26 (s, 1H), 4.22 (t, 2H), 3.91 (quart, 2H), 3.82-3.72 (breit, 2H), 3.33-3.25 (m, 2H, teilweise überlagert vom Wassersignal), 2.37 (s, 3H), 1.51-1.45 (m, 2H), 1.42-1.34 (m, 2H), 1.40 (quart, 2H), 1.13 (t, 3H), 0.83 (t, 3H).

LC/MS (Methode 1, ESIpos): Rₜ = 0.94 min, m/z = 478 [M+H]⁺.

### Beispiel 113

### 3-Ethyl-6-[(4-ethyl-4-hydroxypiperidin-1-yl)carbonyl]-5-methyl-1-(3,3,3-trifluorpropyl)thieno-[2,3-d]pyrimidin-2,4(1H,3H)-dion

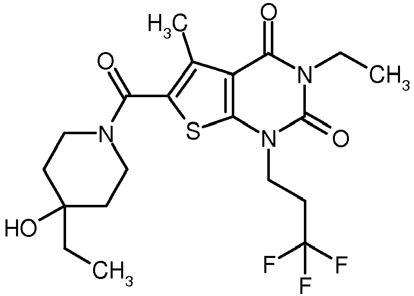

Analog zu dem unter Bsp. 51 beschriebenen Verfahren wurden aus 80 mg (0.228 mmol) der Verbindung aus Bsp. 52A und 45 mg (0.274 mmol) 4-Ethylpiperidin-4-ol-Hydrochlorid [kommerziell erhältlich; Lit. z.B.: US 2004/0067931-A1, Example 3.232 (freie Base)] 94 mg (89% d. Th.) der Titelverbindung erhalten.

¹H-NMR (400 MHz, DMSO-d₆, δ/ppm): 4.26 (s, 1H), 4.12 (t, 2H), 3.91 (quart, 2H), 3.84-3.73 (breit, 2H), 3.33-3.25 (m, 2H, teilweise überlagert vom Wassersignal), 2.85-2.73 (m, 2H), 2.38 (s, 3H), 1.52-1.46 (m, 2H), 1.41 (quart, 2H), 1.41-1.35 (m, 2H), 1.13 (t, 3H), 0.84 (t, 3H).

LC/MS (Methode 1, ESIpos): Rₜ = 0.90 min, m/z = 462 [M+H]⁺.

### Beispiel 114

### 6-{[4-Hydroxy-4-(hydroxymethyl)piperidin-1-yl]carbonyl}-5-methyl-3-(3,3,3-trifluor-2-methoxypropyl)-1-(3,3,3-trifluorpropyl)thieno[2,3-d]pyrimidin-2,4(1H,3H)-dion (Enantiomer 1)

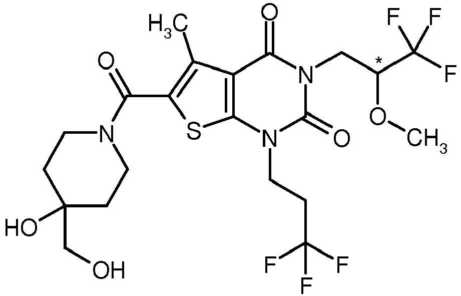

120 mg (0.214 mmol) der racemischen Verbindung aus Bsp. 81 wurden in 4 ml Isopropanol gelöst und in 13 Portionen mittels präparativer HPLC an chiraler Phase in die Enantiomere getrennt [Säule: Daicel Chiralcel OX-H, 5 µm, 250 mm x 20 mm; Eluent: Isohexan/Isopropanol 60:40; Fluss: 15 ml/min; Temperatur: 30°C; Detektion: 220 nm]. Nach Einengen der Produktfraktionen und Trocknen des Rückstands im Hochvakuum wurden 54 mg (86% d. Th., 95% chem. Reinheit, >99% ee) von Enantiomer 1 erhalten. Eine Nachreinigung über präparative HPLC (Methode 5) lieferte 44 mg (73% d. Th.) der Titelverbindung in reiner Form.

¹H-NMR (400 MHz, CDCl₃, δ/ppm): 4.46 (dd, 1H), 4.21-3.97 (m, 6H), 3.51 (s, 5H), 3.45 (br. t, 2H), 2.71-2.60 (m, 2H), 2.50 (s, 3H), 2.21 (breit, 1H), 1.95 (breit, 1H), 1.76-1.70 (m, 2H), 1.58-1.51 (m, 2H, teilweise überlagert vom Wassersignal).

LC/MS (Methode 1, ESIpos): Rₜ = 0.88 min, m/z = 562 [M+H]⁺.

Chirale analytische HPLC [Säule: LUX Cellulose 4, 5 µm, 250 mm x 4.6 mm; Eluent: Isohexan/ Isopropanol 60:40; Fluss: 1 ml/min; Temperatur: 40°C; Detektion: 220 nm]: Rₜ = 8.11 min.

### Beispiel 115

### 6-{[4-Hydroxy-4-(hydroxymethyl)piperidin-1-yl]carbonyl}-5-methyl-3-(3,3,3-trifluor-2-methoxypropyl)-1-(3,3,3-trifluorpropyl)thieno[2,3-d]pyrimidin-2,4(1H,3H)-dion (Enantiomer 2)

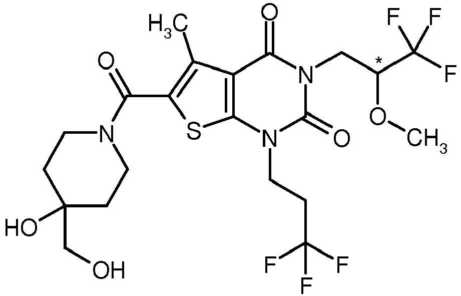

120 mg (0.214 mmol) der racemischen Verbindung aus Bsp. 81 wurden in 4 ml Isopropanol gelöst und in 13 Portionen mittels präparativer HPLC an chiraler Phase in die Enantiomere getrennt [Säule: Daicel Chiralcel OX-H, 5 µm, 250 mm x 20 mm; Eluent: Isohexan/Isopropanol 60:40; Fluss: 15 ml/min; Temperatur: 30°C; Detektion: 220 nm]. Nach Einengen der Produktfraktionen und Trocknen des Rückstands im Hochvakuum wurden 58 mg (92% d. Th., 95% chem. Reinheit, >99% ee) von Enantiomer 2 erhalten. Eine Nachreinigung über präparative HPLC (Methode 5) lieferte 45 mg (75% d. Th.) der Titelverbindung in reiner Form.

¹H-NMR (400 MHz, CDCl₃, δ/ppm): 4.46 (dd, 1H), 4.21-3.97 (m, 6H), 3.51 (s, 5H), 3.45 (br. t, 2H), 2.71-2.60 (m, 2H), 2.50 (s, 3H), 2.20 (breit, 1H), 1.94 (breit, 1H), 1.76-1.70 (m, 2H), 1.57-1.51 (m, 2H, teilweise überlagert vom Wassersignal).

LC/MS (Methode 1, ESIpos): Rₜ = 0.88 min, m/z = 562 [M+H]⁺.

Chirale analytische HPLC [Säule: LUX Cellulose 4, 5 µm, 250 mm x 4.6 mm; Eluent: Isohexan/ Isopropanol 60:40; Fluss: 1 ml/min; Temperatur: 40°C; Detektion: 220 nm]: Rₜ = 10.96 min.

### Beispiel 116

### 6-[(3,4-Dihydroxypiperidin-1-yl)carbonyl]-3-ethyl-5-methyl-1-(3,3,3-trifluorpropyl)thieno[2,3-d]-pyrimidin-2,4(1H,3H)-dion (cis-Racemat)

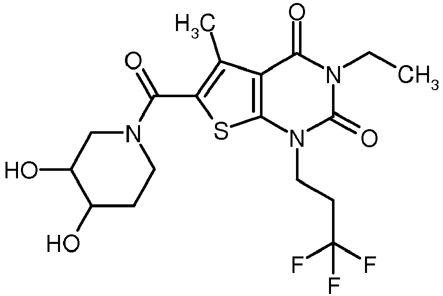

Analog zu dem unter Bsp. 51 beschriebenen Verfahren wurden aus 200 mg (0.571 mmol) der Verbindung aus Bsp. 52A und 105 mg (0.685 mmol) racemischem *cis*-3,4-Dihydroxypiperidin-Hydrochlorid [kommerziell erhältlich; Lit. z.B.: H. H. Jensen et al., Chemistry Eur. J. 2002, 8 (5), 1218-1226] 160 mg (62% d. Th.) der Titelverbindung erhalten. Die Aufreinigung erfolgte durch zweimalige präparative HPLC nach Methode 5.

¹H-NMR (400 MHz, CDCl₃, δ/ppm): 4.23-4.11 (m, 2H), 4.07 (quart, 2H), 3.97 (dt, 1H), 3.89-3.82 (m, 2H), 3.81 (t, 1H), 3.61-3.55 (m, 1H), 3.42-3.36 (m, 1H), 2.70-2.59 (m, 2H), 2.51 (s, 3H), 1.98-1.89 (m, 1H), 1.79-1.72 (m, 1H), 1.25 (t, 3H).

LC/MS (Methode 1, ESIpos): Rₜ = 0.76 min, m/z = 450 [M+H]⁺.

### Beispiel 117

### 6-[(3,4-Dihydroxypiperidin-1-yl)carbonyl]-3-ethyl-5-methyl-1-(3,3,3-trifluorpropyl)thieno[2,3-d]-pyrimidin-2,4(1H,3H)-dion (cis-Enantiomer 1)

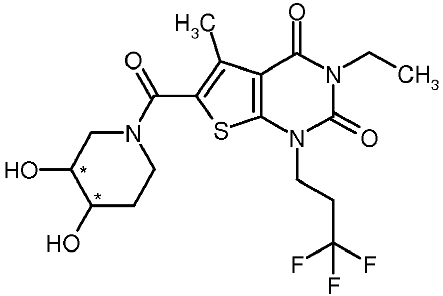

149 mg (0.332 mmol) der racemischen Verbindung aus Bsp. 116 wurden in einem Gemisch aus 1 ml Methanol und 0.5 ml Isopropanol gelöst und in 6 Portionen mittels präparativer HPLC an chiraler Phase in die Enantiomere getrennt [Säule: Daicel Chiralpak IC, 5 µm, 250 mm x 20 mm; Eluent: Isohexan/Ethanol 50:50; Fluss: 20 ml/min; Temperatur: 23°C; Detektion: 220 nm]. Nach Einengen der Produktfraktionen und Trocknen des Rückstands im Hochvakuum wurden 49 mg (63% d. Th., 97% chem. Reinheit, 95% ee) von Enantiomer 1 erhalten. Anschließendes Verrühren mit Pentan ergab 38 mg (51% d. Th.) der Titelverbindung in reiner Form.

¹H-NMR (400 MHz, CDCl₃, δ/ppm): 4.23-4.11 (m, 2H), 4.07 (quart, 2H), 3.99-3.94 (m, 1H), 3.89-3.82 (m, 2H), 3.81 (t, 1H), 3.61-3.55 (m, 1H), 3.42-3.36 (m, 1H), 2.70-2.59 (m, 2H), 2.51 (s, 3H), 2.26 (d, 1H), 2.11 (d, 1H), 1.98-1.89 (m, 1H), 1.79-1.72 (m, 1H), 1.25 (t, 3H).

Chirale analytische HPLC [Säule: Daicel Chiralpak IE-3, 3 µm, 50 mm x 4.6 mm; Eluent: Isohexan/Ethanol 50:50; Fluss: 1 ml/min; Temperatur: 40°C; Detektion: 220 nm]: Rₜ = 1.96 min.

### Beispiel 118

### 6-[(3,4-Dihydroxypiperidin-1-yl)carbonyl]-3-ethyl-5-methyl-1-(3,3,3-trifluorpropyl)thieno[2,3-d]-pyrimidin-2,4(1H,3H)-dion (cis-Enantiomer 2)

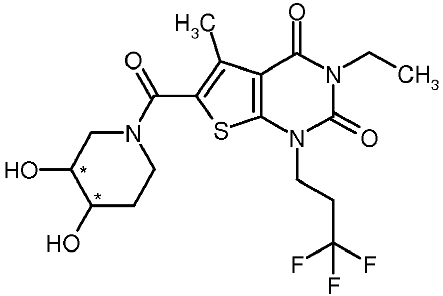

149 mg (0.332 mmol) der racemischen Verbindung aus Bsp. 116 wurden in einem Gemisch aus 1 ml Methanol und 0.5 ml Isopropanol gelöst und in 6 Portionen mittels präparativer HPLC an chiraler Phase in die Enantiomere getrennt [Säule: Daicel Chiralpak IC, 5 µm, 250 mm x 20 mm; Eluent: Isohexan/Ethanol 50:50; Fluss: 20 ml/min; Temperatur: 23°C; Detektion: 220 nm]. Nach Einengen der Produktfraktionen und Trocknen des Rückstands im Hochvakuum wurden 56 mg (74% d. Th., 97% chem. Reinheit, 94% ee) von Enantiomer 2 erhalten. Anschließendes Verrühren mit Pentan ergab 50 mg (67% d. Th.) der Titelverbindung in reiner Form.

¹H-NMR (400 MHz, CDCl₃, δ/ppm): 4.23-4.11 (m, 2H), 4.07 (quart, 2H), 3.99-3.94 (m, 1H), 3.89-3.82 (m, 2H), 3.81 (t, 1H), 3.61-3.55 (m, 1H), 3.42-3.36 (m, 1H), 2.70-2.59 (m, 2H), 2.51 (s, 3H), 2.31 (breit, 1H), 2.14 (breit, 1H), 1.98-1.89 (m, 1H), 1.79-1.72 (m, 1H), 1.25 (t, 3H).

Chirale analytische HPLC [Säule: Daicel Chiralpak IE-3, 3 µm, 50 mm x 4.6 mm; Eluent: Isohexan/Ethanol 50:50; Fluss: 1 ml/min; Temperatur: 40°C; Detektion: 220 nm]: Rₜ = 1.62 min.

### Beispiel 119

### 6-[(3,4-Dihydroxypiperidin-1-yl)carbonyl]-3-ethyl-5-methyl-1-(3,3,3-trifluorpropyl)thieno[2,3-d]-pyrimidin-2,4(1H,3H)-dion (trans-Racemat)

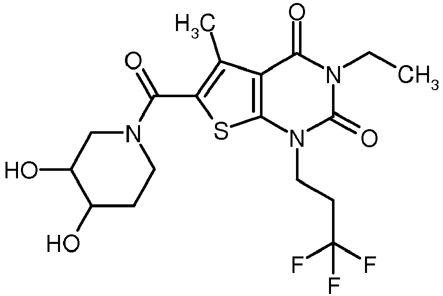

Analog zu dem unter Bsp. 51 beschriebenen Verfahren wurden aus 200 mg (0.571 mmol) der Verbindung aus Bsp. 52A und 105 mg (0.685 mmol) racemischem *trans*-3,4-Dihydroxypiperidin-Hydrochlorid [kommerziell erhältlich; Lit. z.B.: H. H. Jensen et al., Chemistry Eur. J. 2002, 8 (5), 1218-1226] 161 mg (62% d. Th.) der Titelverbindung erhalten. Die Aufreinigung erfolgte durch zweimalige präparative HPLC nach Methode 5.

¹H-NMR (400 MHz, CDCl₃, δ/ppm): 4.24-4.13 (m, 3H), 4.11-4.02 (m, 3H), 3.74-3.66 (m, 1H), 3.60-3.53 (m, 1H), 3.22-3.05 (m, 2H), 2.70-2.59 (m, 2H), 2.51 (s, 3H), 2.11-2.03 (m, 1H), 1.62-1.51 (m, 1H), 1.26 (t, 3H).

LC/MS (Methode 1, ESIpos): Rₜ = 0.76 min, m/z = 450 [M+H]⁺.

### Beispiel 120

### 6-[(3,4-Dihydroxypiperidin-1-yl)carbonyl]-3-ethyl-5-methyl-1-(3,3,3-trifluorpropyl)thieno[2,3-d]-pyrimidin-2,4(1H,3H)-dion (trans-Enantiomer 1)

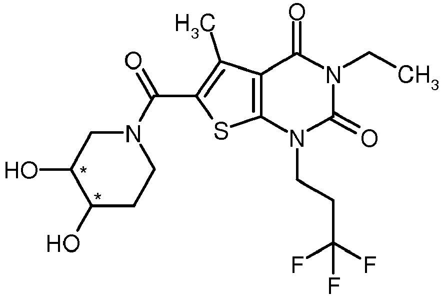

146 mg (0.325 mmol) der racemischen Verbindung aus Bsp. 119 wurden in 3 ml Isopropanol gelöst und in 4 Portionen mittels präparativer HPLC an chiraler Phase in die Enantiomere getrennt [Säule: Daicel Chiralpak IC, 5 µm, 250 mm x 20 mm; Eluent: Isohexan/Isopropanol 40:60; Fluss: 15 ml/min; Temperatur: 35°C; Detektion: 220 nm]. Nach Einengen der Produktfraktionen und Trocknen des Rückstands im Hochvakuum wurden 62 mg (84% d. Th.) von Enantiomer 1 erhalten.

¹H-NMR (400 MHz, CDCl₃, δ/ppm): 4.20 (breit, 1H), 4.17 (m, 2H), 4.08 (breit, 1H), 4.07 (quart, 2H), 3.73-3.67 (m, 1H), 3.59-3.53 (m, 1H), 3.17 (t, 1H), 3.09 (dd, 1H), 2.70-2.59 (m, 2H), 2.51 (s, 3H), 2.48 (breit, 1H), 2.25 (breit, 1H), 2.10-2.04 (m, 1H), 1.61-1.51 (m, 1H, teilweise überdeckt vom Wassersignal), 1.25 (t, 3H).

Chirale analytische HPLC [Säule: Daicel Chiralpak IC, 5 µm, 250 mm x 4.6 mm; Eluent: 40% Isohexan / 60% Isopropanol mit 0.2% TFA und 1% Wasser; Fluss: 1 ml/min; Temperatur: 40°C; Detektion: 220 nm]: Rₜ = 6.69 min; >99% ee.

### Beispiel 121

### 6-[(3,4-Dihydroxypiperidin-1-yl)carbonyl]-3-ethyl-5-methyl-1-(3,3,3-trifluorpropyl)thieno[2,3-d]-pyrimidin-2,4(1H,3H)-dion (trans-Enantiomer 2)

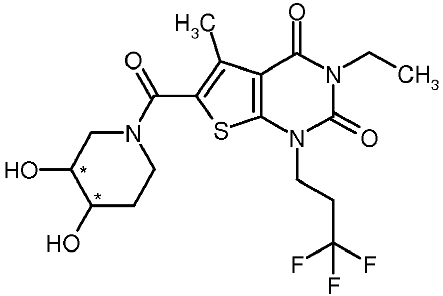

146 mg (0.325 mmol) der racemischen Verbindung aus Bsp. 119 wurden in 3 ml Isopropanol gelöst und in 4 Portionen mittels präparativer HPLC an chiraler Phase in die Enantiomere getrennt [Säule: Daicel Chiralpak IC, 5 µm, 250 mm x 20 mm; Eluent: Isohexan/Isopropanol 40:60; Fluss: 15 ml/min; Temperatur: 35°C; Detektion: 220 nm]. Nach Einengen der Produktfraktionen und Trocknen des Rückstands im Hochvakuum wurden 62 mg (84% d. Th.) von Enantiomer 2 erhalten.

¹H-NMR (400 MHz, CDCl₃, δ/ppm): 4.20 (breit, 1H), 4.17 (m, 2H), 4.08 (breit, 1H), 4.07 (quart, 2H), 3.73-3.67 (m, 1H), 3.59-3.53 (m, 1H), 3.17 (t, 1H), 3.09 (dd, 1H), 2.70-2.59 (m, 2H), 2.51 (br. s, 4H), 2.27 (breit, 1H), 2.10-2.04 (m, 1H), 1.61-1.51 (m, 1H, teilweise überdeckt vom Wassersignal), 1.26 (t, 3H).

Chirale analytische HPLC [Säule: Daicel Chiralpak IC, 5 µm, 250 mm x 4.6 mm; Eluent: 40% Isohexan / 60% Isopropanol mit 0.2% TFA und 1% Wasser; Fluss: 1 ml/min; Temperatur: 40°C; Detektion: 220 nm]: Rₜ = 5.74 min; >99% ee.

### Beispiel 122

### 5-(Difluormethyl)-3-ethyl-6-[(4-hydroxypiperidin-1-yl)carbonyl]-1-(3,3,3-trifluorpropyl)thieno-[2,3-d]pyrimidin-2,4(1H,3H)-dion

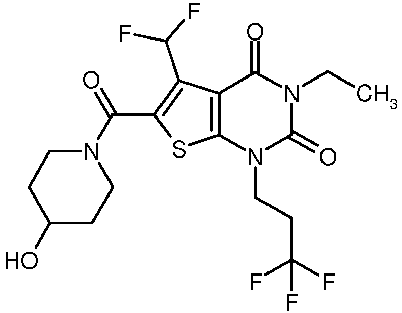

Eine Lösung von 80 mg (0.207 mmol) der Verbindung aus Bsp. 119A in 3 ml wasserfreiem DMF wurde nacheinander mit 23 mg (0.228 mmol) 4-Hydroxypiperidin, 95 mg (0.249 mmol) HATU und 54 µl (0.311 mmol) *N*,*N*-Diisopropylethylamin versetzt. Nach einer Reaktionszeit von ca. 16 h bei RT wurde das Reaktionsgemisch direkt mittels präparativer HPLC (Methode 5) in seine Komponenten aufgetrennt. Die Produktfraktionen wurden vereinigt und eingeengt und der Rückstand im Hochvakuum getrocknet. Es wurden 86 mg (88% d. Th.) der Titelverbindung erhalten.

¹H-NMR (400 MHz, DMSO-d₆, δ/ppm): 7.33 (t, 1H), 4.81 (breit, 1H), 4.15 (t, 2H), 3.94 (breit, 1H), 3.92 (quart, 2H), 3.75 (m, 1H), 3.50 (breit, 1H), 3.21 (breit, 2H), 2.89-2.73 (m, 2H), 1.73 (breit, 2H), 1.36 (breit, 2H), 1.14 (t, 3H).

LC/MS (Methode 1, ESIpos): Rₜ = 0.83 min, m/z = 470 [M+H]⁺.

### Beispiel 123

### 5-(Difluormethyl)-3-ethyl-6-[(4-hydroxypiperidin-1-yl)carbonyl]-1-[2-(trifluormethoxy)ethyl]-thieno[2,3-d]pyrimidin-2,4(1H,3H)-dion

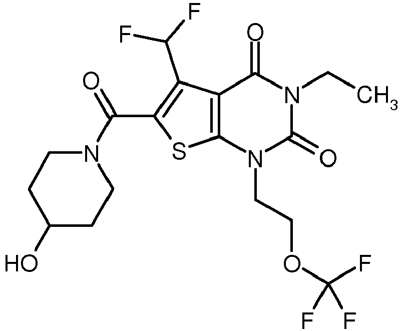

Analog zu dem unter Bsp. 122 beschriebenen Verfahren wurden aus 100 mg (0.249 mmol) der Verbindung aus Bsp. 120A und 30 mg (0.298 mmol) 4-Hydroxypiperidin 70 mg (58% d. Th.) der Titelverbindung erhalten.

¹H-NMR (400 MHz, DMSO-d₆, δ/ppm): 7.33 (t, 1H), 4.81 (breit, 1H), 4.43 (t, 2H), 4.26 (t, 2H), 3.96 (breit, 1H), 3.93 (quart, 2H), 3.74 (m, 1H), 3.46 (breit, 1H), 3.20 (breit, 2H), 1.72 (breit, 2H), 1.35 (breit, 2H), 1.14 (t, 3H).

LC/MS (Methode 1, ESIpos): Rₜ = 0.84 min, m/z = 486 [M+H]⁺.

### Beispiel 124

### 5-(Difluormethyl)-3-ethyl-6-[(4-hydroxy-4-methylpiperidin-1-yl)carbonyl]-1-(3,3,3-trifluorpropyl)-thieno[2,3-d]pyrimidin-2,4(1H,3H)-dion

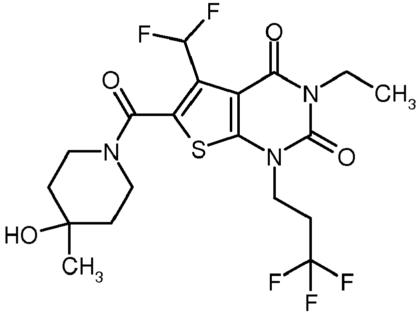

Analog zu dem unter Bsp. 122 beschriebenen Verfahren wurden aus 80 mg (0.207 mmol) der Verbindung aus Bsp. 119A und 26 mg (0.228 mmol) 4-Methylpiperidin-4-ol [kommerziell erhältlich; Lit. z.B.: J. M. McManus et al., J. Med. Chem. 1965, 8 (6), 766-776] 87 mg (83% d. Th., 96% Reinheit) der Titelverbindung erhalten.

¹H-NMR (400 MHz, DMSO-d₆, δ/ppm): 7.33 (t, 1H), 4.47 (breit, 1H), 4.15 (t, 2H), 4.06 (breit, 1H), 3.92 (quart, 2H), 3.24 (breit, 3H), 2.89-2.73 (m, 2H), 1.56-1.40 (breit, 4H), 1.15 (s, 3H), 1.14 (t, 3H).

LC/MS (Methode 1, ESIpos): Rₜ = 0.88 min, m/z = 484 [M+H]⁺.

### Beispiel 125

### 5-(Difluormethyl)-3-ethyl-6-[(4-ethyl-4-hydroxypiperidin-1-yl)carbonyl]-1-(3,3,3-trifluorpropyl)-thieno[2,3-d]pyrimidin-2,4(1H,3H)-dion

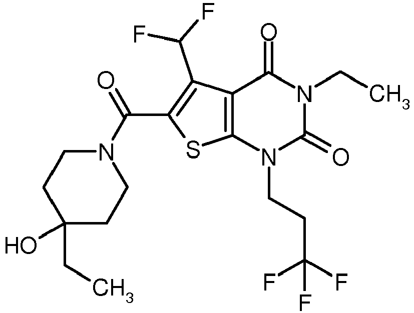

Analog zu dem unter Bsp. 122 beschriebenen Verfahren wurden aus 80 mg (0.207 mmol) der Verbindung aus Bsp. 119A und 41 mg (0.249 mmol) 4-Ethylpiperidin-4-ol-Hydrochlorid [kommerziell erhältlich; Lit. z.B.: US 2004/0067931-A1, Example 3.232 (freie Base)] 86 mg (83% d. Th.) der Titelverbindung erhalten.

¹H-NMR (400 MHz, DMSO-d₆, δ/ppm): 7.33 (t, 1H), 4.27 (s, 1H), 4.16 (breit, 1H), 4.15 (t, 2H), 3.92 (quart, 2H), 3.36 (breit, 2H), 3.13 (breit, 1H), 2.87-2.75 (m, 2H), 1.50 (breit, 2H), 1.40 (quart, 2H), 1.39 (breit, 2H), 1.14 (t, 3H), 0.83 (t, 3H).

LC/MS (Methode 1, ESIpos): Rₜ = 0.92 min, m/z = 498 [M+H]⁺.

### Beispiel 126

### 5-(Difluormethyl)-3-ethyl-6-{[4-hydroxy-4-(hydroxymethyl)piperidin-1-yl]carbonyl}-1-(3,3,3-tri-fluorpropyl)thieno[2,3-d]pyrimidin-2,4(1H,3H)-dion

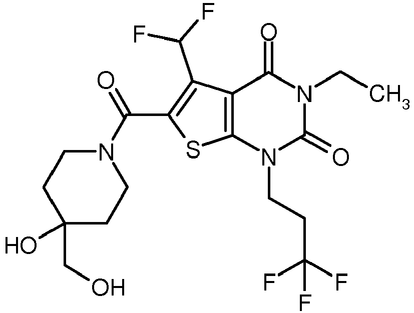

Analog zu dem unter Bsp. 122 beschriebenen Verfahren wurden aus 80 mg (0.207 mmol) der Verbindung aus Bsp. 119A und 38 mg (0.228 mmol) 4-(Hydroxymethyl)piperidin-4-ol-Hydrochlorid [kommerziell erhältlich; Lit. z.B.: WO 2005/103037-A2, Beispiel A3b (freie Base); US 2011/ 288065-A1, Example 105 / Step 1 (Hydrochlorid)] 88 mg (85% d. Th.) der Titelverbindung erhalten.

¹H-NMR (400 MHz, DMSO-d₆, δ/ppm): 7.32 (t, 1H), 4.63 (breit, 1H), 4.36 (breit, 1H), 4.21 (breit, 1H), 4.15 (t, 2H), 3.92 (quart, 2H), 3.37 (breit, 2H), 3.20 (s, 2H), 3.10 (breit, 1H), 2.89-2.73 (m, 2H), 1.53 (dt, 2H), 1.40 (breit, 2H), 1.14 (t, 3H).

LC/MS (Methode 1, ESIpos): Rₜ = 0.77 min, m/z = 500 [M+H]⁺.

### Beispiel 127

### 5-(Difluormethyl)-3-ethyl-6-[(4-hydroxy-4-methylpiperidin-1-yl)carbonyl]-1-[2-(trifluormethoxy)-ethyl]thieno[2,3-d]pyrimidin-2,4(1H,3H)-dion

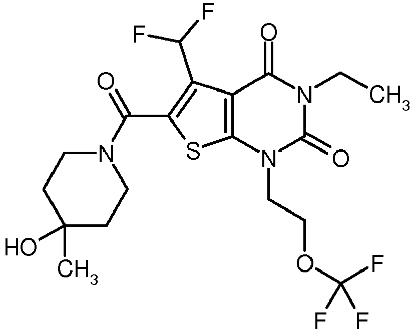

Analog zu dem unter Bsp. 122 beschriebenen Verfahren wurden aus 100 mg (0.249 mmol) der Verbindung aus Bsp. 120A und 34 mg (0.298 mmol) 4-Methylpiperidin-4-ol [kommerziell erhältlich; Lit. z.B.: J. M. McManus et al., J. Med. Chem. 1965, 8 (6), 766-776] 82 mg (66% d. Th.) der Titelverbindung erhalten.

¹H-NMR (400 MHz, DMSO-d₆, δ/ppm): 7.32 (t, 1H), 4.47 (s, 1H), 4.42 (t, 2H), 4.25 (t, 2H), 4.05 (breit, 1H), 3.92 (quart, 2H), 3.25 (breit, 3H), 1.57-1.38 (breit, 4H), 1.14 (s, 3H), 1.14 (t, 3H).

LC/MS (Methode 1, ESIpos): Rₜ = 0.88 min, m/z = 500 [M+H]⁺.

### Beispiel 128

### 3,5-Diethyl-6-[(4-ethyl-4-hydroxypiperidin-1-yl)carbonyl]-1-(3,3,3-trifluorpropyl)thieno[2,3-d]-pyrimidin-2,4(1H,3H)-dion

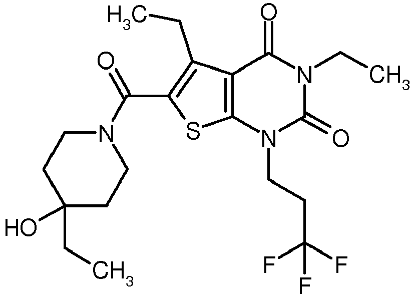

Analog zu dem unter Bsp. 122 beschriebenen Verfahren wurden aus 80 mg (0.220 mmol) der Verbindung aus Bsp. 111A und 44 mg (0.263 mmol) 4-Ethylpiperidin-4-ol-Hydrochlorid [kommerziell erhältlich; Lit. z.B.: US 2004/0067931-A1, Example 3.232 (freie Base)] 100 mg (95% d. Th.) der Titelverbindung erhalten.

¹H-NMR (400 MHz, DMSO-d₆, δ/ppm): 4.27 (s, 1H), 4.12 (t, 2H), 3.92 (quart, 2H), 3.80 (breit, 2H), 3.27 (breit, 2H, teilweise überdeckt vom Wassersignal), 2.86-2.74 (m, 2H), 2.79 (quart, 2H), 1.49 (breit, 2H), 1.41 (quart, 2H), 1.38 (breit, 2H), 1.13 (t, 3H), 1.11 (t, 3H), 0.83 (t, 3H).

LC/MS (Methode 1, ESIpos): Rₜ = 0.96 min, m/z = 476 [M+H]⁺.

### Beispiel 129

### 5-Methyl-6-[(3-oxopiperazin-1-yl)carbonyl]-3-(2-phenylethyl)-1-[2-(trifluormethoxy)ethyl]thieno-[2,3-d]pyrimidin-2,4(1H,3H)-dion

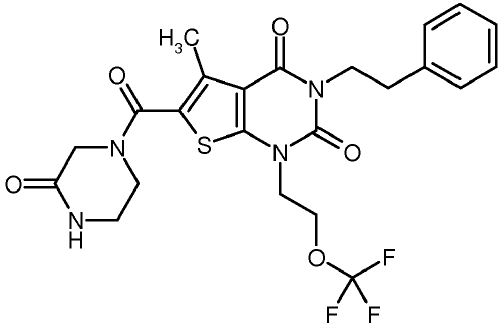

Eine Lösung von 65 mg (0.147 mmol) der Verbindung aus Bsp. 118A in 2 ml wasserfreiem DMF wurde nacheinander mit 16 mg (0.162 mmol) Piperazin-2-on, 67 mg (0.176 mmol) HATU und 64 µl (0.367 mmol) *N*,*N*-Diisopropylethylamin versetzt. Nach einer Reaktionszeit von ca. 16 h bei RT wurde das Reaktionsgemisch direkt mittels präparativer HPLC (Methode 5) in seine Komponenten aufgetrennt. Die Produktfraktionen wurden vereinigt und eingeengt und der Rückstand im Hochvakuum getrocknet. Es wurden 69 mg (77% d. Th.) der Titelverbindung erhalten.

¹H-NMR (400 MHz, DMSO-d₆, δ/ppm): 8.14 (br. s, 1H), 7.32-7.28 (m, 2H), 7.25-7.20 (m, 3H), 4.39 (t, 2H), 4.23 (t, 2H), 4.08 (m, 2H), 4.04 (s, 2H), 3.67 (t, 2H), 3.26-3.22 (m, 2H), 2.84 (m, 2H), 2.39 (s, 3H).

LC/MS (Methode 1, ESIpos): Rₜ = 0.99 min, m/z = 525 [M+H]⁺.

### Beispiel 130

### 3-Ethyl-5-methyl-6-[(3-oxopiperazin-1-yl)carbonyl]-1-[2-(trifluormethoxy)ethyl]thieno[2,3-d]-pyrimidin-2,4(1H,3H)-dion

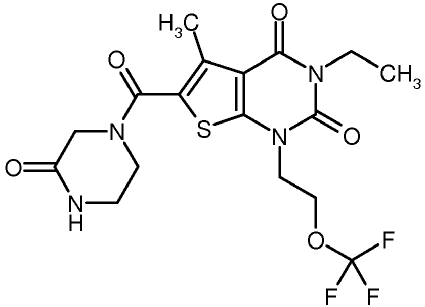

Eine Lösung von 100 mg (0.273 mmol) der Verbindung aus Bsp. 104A in 3 ml wasserfreiem DMF wurde nacheinander mit 33 mg (0.328 mmol) Piperazin-2-on, 125 mg (0.328 mmol) HATU und 62 µl (0.355 mmol) *N*,*N*-Diisopropylethylamin versetzt. Nach einer Reaktionszeit von 1 h bei RT wurde das Reaktionsgemisch direkt mittels präparativer HPLC (Methode 5) in seine Komponenten aufgetrennt. Die Produktfraktionen wurden vereinigt und eingeengt und der Rückstand im Hochvakuum getrocknet. Es wurden 95 mg (77% d. Th.) der Titelverbindung erhalten.

¹H-NMR (400 MHz, DMSO-d₆, δ/ppm): 8.13 (br. s, 1H), 4.42 (t, 2H), 4.23 (t, 2H), 4.03 (s, 2H), 3.92 (quart, 2H), 3.67 (t, 2H), 3.25-3.21 (m, 2H), 2.40 (s, 3H), 1.13 (t, 3H).

LC/MS (Methode 1, ESIpos): Rₜ = 0.74 min, m/z = 449 [M+H]⁺.

### Beispiel 131

### 5-(Difluormethyl)-3-ethyl-6-[(3-oxopiperazin-1-yl)carbonyl]-1-(3,3,3-trifluorpropyl)thieno[2,3-d]-pyrimidin-2,4(1H,3H)-dion

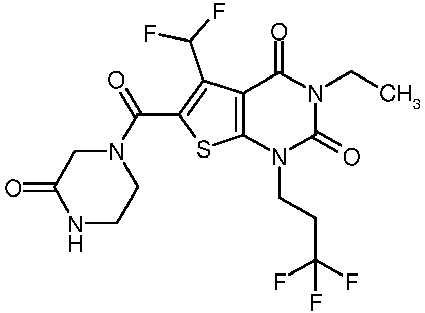

Analog zu dem unter Bsp. 122 beschriebenen Verfahren wurden aus 80 mg (0.207 mmol) der Verbindung aus Bsp. 119A und 23 mg (0.228 mmol) Piperazin-2-on 49 mg (50% d. Th.) der Titelverbindung erhalten. Die Aufreinigung erfolgte durch zweimalige präparative HPLC nach Methode 5.

¹H-NMR (400 MHz, DMSO-d₆, δ/ppm): 8.15 (br. s, 1H), 7.35 (t, 1H), 4.16 (t, 2H), 4.05 (breit, 2H), 3.93 (quart, 2H), 3.75 (breit, 1H), 3.53 (breit, 1H), 3.21 (breit, 2H), 2.88-2.76 (m, 2H), 1.14 (t, 3H).

LC/MS (Methode 1, ESIpos): Rₜ = 0.77 min, m/z = 469 [M+H]⁺.

### Beispiel 132

### 3-(2-Hydroxy-2-phenylpropyl)-6-[(4-hydroxypiperidin-1-yl)carbonyl]-5-methyl-1-(3,3,3-trifluor-propyl)thieno[2,3-d]pyrimidin-2,4(1H,3H)-dion (Enantiomer 1)

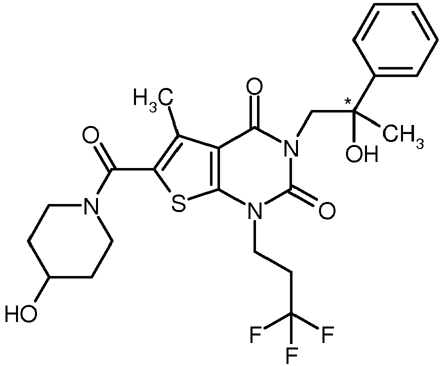

35 mg (0.065 mmol) der racemischen Verbindung aus Bsp. 105 wurden in 6 ml Isohexan/Ethanol (1:1) gelöst und in 3 Portionen mittels präparativer HPLC an chiraler Phase in die Enantiomere getrennt [Säule: Daicel Chiralpak ID, 5 µm, 250 mm x 20 mm; Eluent: Isohexan/Ethanol 50:50; Fluss: 20 ml/min; Temperatur: 35°C; Detektion: 220 nm]. Nach Einengen der Produktfraktionen und Trocknen des Rückstands im Hochvakuum wurden 11 mg (62% d. Th.) von Enantiomer 1 erhalten.

¹H-NMR (400 MHz, DMSO-d₆, δ/ppm): 7.48 (d, 2H), 7.30 (t, 2H), 7.21 (t, 1H), 5.05 (s, 1H), 4.81 (d, 1H), 4.18 (quart, 2H), 4.12-4.03 (m, 2H), 3.81-3.72 (m, 3H), 3.28-3.21 (m, 2H), 2.76-2.63 (m, 2H), 2.34 (s, 3H), 1.80-1.73 (m, 2H), 1.42 (s, 3H), 1.41-1.32 (m, 2H).

Chirale analytische HPLC [Säule: Daicel Chiralpak IB-3, 3 µm, 50 mm x 4.6 mm; Eluent: Isohexan/Ethanol 50:50; Fluss: 1 ml/min; Temperatur: 40°C; Detektion: 220 nm]: Rₜ = 1.23 min; >99% ee.

### Beispiel 133

### 3-(2-Hydroxy-2-phenylpropyl)-6-[(4-hydroxypiperidin-1-yl)carbonyl]-5-methyl-1-(3,3,3-trifluor-propyl)thieno[2,3-d]pyrimidin-2,4(1H,3H)-dion (Enantiomer 2)

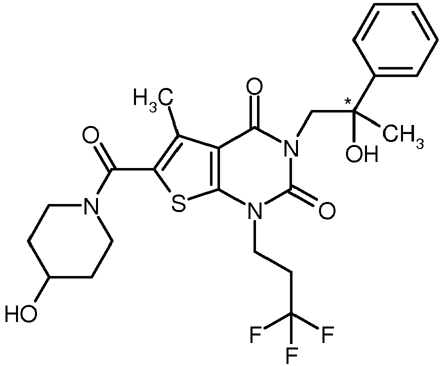

35 mg (0.065 mmol) der racemischen Verbindung aus Bsp. 105 wurden in 6 ml Isohexan/Ethanol (1:1) gelöst und in 3 Portionen mittels präparativer HPLC an chiraler Phase in die Enantiomere getrennt [Säule: Daicel Chiralpak ID, 5 µm, 250 mm x 20 mm; Eluent: Isohexan/Ethanol 50:50; Fluss: 20 ml/min; Temperatur: 35°C; Detektion: 220 nm]. Nach Einengen der Produktfraktionen und Trocknen des Rückstands im Hochvakuum wurden 10 mg (57% d. Th.) von Enantiomer 2 erhalten.

¹H-NMR (400 MHz, DMSO-d₆, δ/ppm): 7.48 (d, 2H), 7.30 (t, 2H), 7.21 (t, 1H), 5.05 (s, 1H), 4.81 (d, 1H), 4.18 (quart, 2H), 4.12-4.03 (m, 2H), 3.82-3.72 (m, 3H), 3.28-3.21 (m, 2H), 2.76-2.63 (m, 2H), 2.34 (s, 3H), 1.81-1.73 (m, 2H), 1.42 (s, 3H), 1.41-1.32 (m, 2H).

Chirale analytische HPLC [Säule: Daicel Chiralpak IB-3, 3 µm, 50 mm x 4.6 mm; Eluent: Isohexan/Ethanol 50:50; Fluss: 1 ml/min; Temperatur: 40°C; Detektion: 220 nm]: Rₜ = 1.10 min; >99% ee.

### Beispiel 134

### 3-Ethyl-6-[(4-hydroxypiperidin-1-yl)carbonyl]-5-methyl-1-(3-methylbutyl)thieno[2,3-d]pyrimidin-2,4(1H,3H)-dion

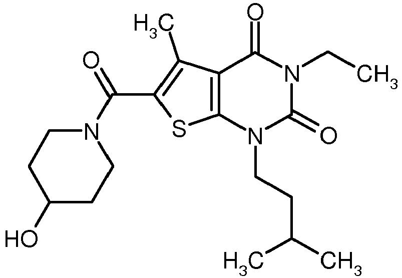

135 mg (0.300 mmol) der Verbindung aus Bsp. 130A wurden in 5 ml Ethanol/THF (1:1) gelöst und mit 360 µl (0.360 mmol) einer 1 M Lösung von Lithiumhydroxid in Wasser versetzt. Nach ca. 16 h Rühren bei RT wurde das Reaktionsgemisch mit 250 µl Eisessig versetzt und mit ca. 200 ml Ethylacetat verdünnt. Es wurde nacheinander zweimal mit Wasser und einmal mit gesättigter Natriumchlorid-Lösung gewaschen. Nach Trocknen über wasserfreiem Magnesiumsulfat wurde filtriert und zur Trockene eingeengt. Es wurden 115 mg (94% d. Th.) der Titelverbindung erhalten.

¹H-NMR (400 MHz, DMSO-d₆, δ/ppm): 4.79 (d, 1H), 3.92-3.88 (m, 2H), 3.91 (quart, 2H), 3.81-3.70 (m, 3H), 3.23 (m, 2H), 2.37 (s, 3H), 1.79-1.72 (m, 2H), 1.65 (sept, 1H), 1.59-1.54 (m, 2H), 1.39-1.31 (m, 2H), 1.12 (t, 3H), 0.94 (d, 6H).

LC/MS (Methode 1, ESIpos): Rₜ = 0.91 min, m/z = 408 [M+H]⁺.

### Beispiel 135

### 3-Ethyl-6-[(4-hydroxypiperidin-1-yl)carbonyl]-5-methyl-1-(4,4,4-trifluorbutyl)thieno[2,3-d]-pyrimidin-2,4(1H,3H)-dion

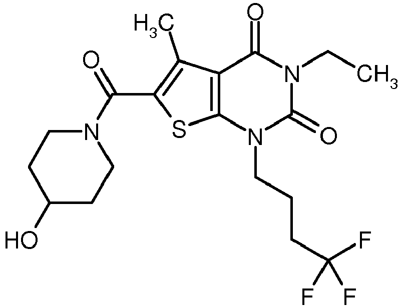

Analog zu dem unter Bsp. 134 beschriebenen Verfahren wurden aus 150 mg (0.306 mmol) der Verbindung aus Bsp. 131A 127 mg (92% d. Th.) der Titelverbindung erhalten. Das Produkt wurde hier abschließend noch mit einem Gemisch aus 10 ml Pentan und 2 ml Diethylether für 10 min bei RT verrührt.

¹H-NMR (400 MHz, DMSO-d₆, δ/ppm): 4.79 (d, 1H), 3.97 (t, 2H), 3.90 (quart, 2H), 3.81-3.71 (m, 3H), 3.23 (m, 2H), 2.46-2.40 (m, 2H), 2.38 (s, 3H), 1.91 (quint, 2H), 1.79-1.72 (m, 2H), 1.39-1.31 (m, 2H), 1.12 (t, 3H).

LC/MS (Methode 1, ESIpos): Rₜ = 0.83 min, m/z = 448 [M+H]⁺.

### Beispiel 136

### 3-Ethyl-6-[(4-hydroxypiperidin-1-yl)carbonyl]-5-methyl-1-(3,4,4-trifluorbut-3-en-1-yl)thieno-[2,3-d]pyrimidin-2,4(1H,3H)-dion

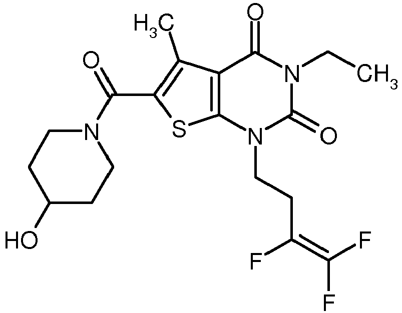

69 mg (0.143 mmol) der Verbindung aus Bsp. 132A wurden in 2.5 ml Ethanol/THF (1:1) gelöst und mit 158 µl (0.158 mmol) einer 1 M Lösung von Lithiumhydroxid in Wasser versetzt. Nach 2 h Rühren bei RT wurde das Reaktionsgemisch bis zur Trockene eingeengt. Der Rückstand wurde in 3 ml Dichlormethan und 1 ml Wasser aufgenommen und intensiv verrührt. Dann wurde die wässrige Phase über eine Extrelut®-NT3-Kartusche abgetrennt. Nach Einengen der organischen Phase und Trocknen des Rückstands im Hochvakuum wurden 57 mg (89% d. Th., 98% Reinheit) der Titelverbindung erhalten.

¹H-NMR (400 MHz, DMSO-d₆, δ/ppm): 4.80 (d, 1H), 4.10 (t, 2H), 3.91 (quart, 2H), 3.80-3.71 (m, 3H), 3.23 (m, 2H), 2.81 (m, 2H), 2.38 (s, 3H), 1.79-1.72 (m, 2H), 1.39-1.31 (m, 2H), 1.12 (t, 3H).

LC/MS (Methode 1, ESIpos): Rₜ = 0.81 min, m/z = 446 [M+H]⁺.

### Beispiel 137

### 5-(Difluormethyl)-6-[(4-hydroxypiperidin-1-yl)carbonyl]-3-isobutyl-1-[2-(trifluormethoxy)ethyl]-thieno[2,3-d]pyrimidin-2,4(1H,3H)-dion

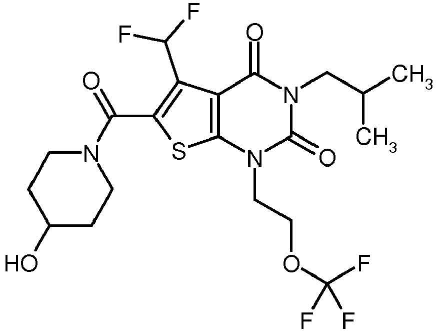

Eine Lösung von 100 mg (0.232 mmol) der Verbindung aus Bsp. 138A in 3 ml wasserfreiem DMF wurde nacheinander mit 28 mg (0.279 mmol) 4-Hydroxypiperidin, 106 mg (0.279 mmol) HATU und 53 µl (0.302 mmol) *N*,*N*-Diisopropylethylamin versetzt. Nach einer Reaktionszeit von ca. 16 h bei RT wurde das Reaktionsgemisch direkt mittels präparativer HPLC (Methode 5) in seine Komponenten aufgetrennt. Die Produktfraktionen wurden vereinigt und eingeengt und der Rückstand im Hochvakuum getrocknet. Es wurden 102 mg (85% d. Th.) der Titelverbindung erhalten.

¹H-NMR (400 MHz, DMSO-d₆, δ/ppm): 7.32 (t, 1H), 4.81 (d, 1H), 4.42 (t, 2H), 4.26 (t, 2H), 3.95 (breit, 1H), 3.75 (m, 1H), 3.73 (d, 2H), 3.48 (breit, 1H), 3.20 (breit, 2H), 2.04 (m, 1H), 1.72 (breit, 2H), 1.35 (breit, 2H), 0.86 (d, 6H).

LC/MS (Methode 1, ESIpos): Rₜ = 0.96 min, m/z = 514 [M+H]⁺.

### Beispiel 138

### 5-(Difluormethyl)-6-[(4-hydroxy-4-methylpiperidin-1-yl)carbonyl]-3-isobutyl-1-[2-(trifluor-methoxy)ethyl]thieno[2,3-d]pyrimidin-2,4(1H,3H)-dion

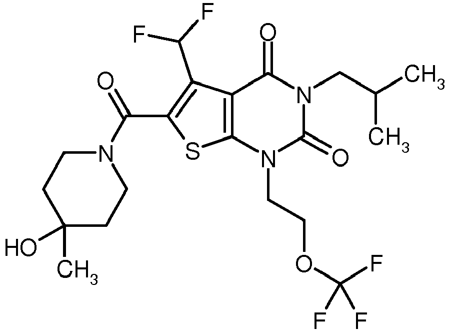

Analog zu dem unter Bsp. 137 beschriebenen Verfahren wurden aus 100 mg (0.232 mmol) der Verbindung aus Bsp. 138A und 32 mg (0.279 mmol) 4-Methylpiperidin-4-ol [kommerziell erhältlich; Lit. z.B.: J. M. McManus et al., J. Med. Chem. 1965, 8 (6), 766-776] 55 mg (44% d. Th.) der Titelverbindung erhalten. Abweichend vom zuvor beschriebenen Verfahren schloss sich hier an die erste präparative HPLC-Aufreinigung (nach Methode 5) noch eine zweite HPLC-Aufreinigung an [Säule: Kinetix C18, 5 µm, 100 mm x 21.5 mm; Laufmittel: 50% Wasser, 45% Acetonitril, 5% Ameisensäure (1% in Wasser); Fluss: 25 ml/min; Temperatur: 25°C; Detektion: 210 nm].

¹H-NMR (400 MHz, DMSO-d₆, δ/ppm): 7.31 (t, 1H), 4.47 (s, 1H), 4.42 (t, 2H), 4.25 (t, 2H), 4.06 (breit, 1H), 3.73 (d, 2H), 3.22 (breit, 3H), 2.04 (m, 1H), 1.52 (breit, 1H), 1.43 (breit, 3H), 1.14 (s, 3H), 0.86 (d, 6H).

LC/MS (Methode 1, ESIpos): Rₜ = 1.01 min, m/z = 528 [M+H]⁺.

### Beispiel 139

### 5-(Difluormethyl)-6-[(4-hydroxypiperidin-1-yl)carbonyl]-3-isobutyl-1-(3,3,3-trifluorpropyl)thieno-[2,3-d]pyrimidin-2,4(1H,3H)-dion

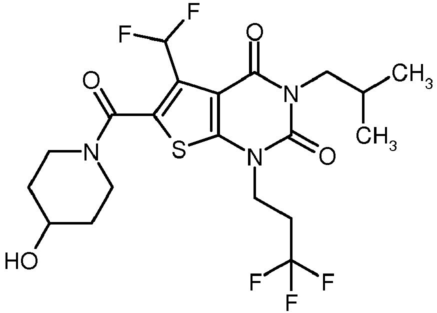

Analog zu dem unter Bsp. 137 beschriebenen Verfahren wurden aus 100 mg (0.241 mmol) der Verbindung aus Bsp. 139A und 29 mg (0.290 mmol) 4-Hydroxypiperidin 109 mg (90% d. Th.) der Titelverbindung erhalten.

¹H-NMR (400 MHz, DMSO-d₆, δ/ppm): 7.32 (t, 1H), 4.81 (d, 1H), 4.15 (t, 2H), 3.96 (breit, 1H), 3.75 (m, 1H), 3.73 (d, 2H), 3.51 (breit, 1H), 3.21 (breit, 2H), 2.81 (m, 2H), 2.04 (m, 1H), 1.73 (breit, 2H), 1.36 (breit, 2H), 0.87 (d, 6H).

LC/MS (Methode 1, ESIpos): Rₜ = 0.94 min, m/z = 498 [M+H]⁺.

### Beispiel 140

### 5-(Difluormethyl)-6-[(4-hydroxy-4-methylpiperidin-1-yl)carbonyl]-3-isobutyl-1-(3,3,3-trifluor-propyl)thieno[2,3-d]pyrimidin-2,4(1H,3H)-dion

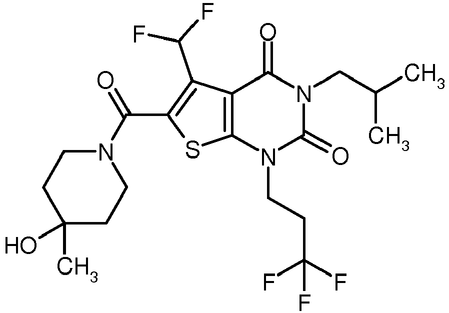

Analog zu dem unter Bsp. 138 beschriebenen Verfahren wurden aus 100 mg (0.241 mmol) der Verbindung aus Bsp. 139A und 33 mg (0.279 mmol) 4-Methylpiperidin-4-ol [kommerziell erhältlich; Lit. z.B.: J. M. McManus et al., J. Med. Chem. 1965, 8 (6), 766-776] 47 mg (38% d. Th.) der Titelverbindung erhalten.

¹H-NMR (400 MHz, DMSO-d₆, δ/ppm): 7.31 (t, 1H), 4.47 (s, 1H), 4.15 (t, 2H), 4.06 (breit, 1H), 3.73 (d, 2H), 3.35 (breit, 2H), 3.22 (breit, 1H), 2.81 (m, 2H), 2.04 (m, 1H), 1.52 (breit, 1H), 1.44 (breit, 3H), 1.15 (s, 3H), 0.87 (d, 6H).

LC/MS (Methode 1, ESIpos): Rₜ = 1.00 min, m/z = 512 [M+H]⁺.

### Beispiel 141

### 5-(Difluormethyl)-6-[(4-hydroxypiperidin-1-yl)carbonyl]-3-propyl-1-(3,3,3-trifluorpropyl)thieno-[2,3-d]pyrimidin-2,4(1H,3H)-dion

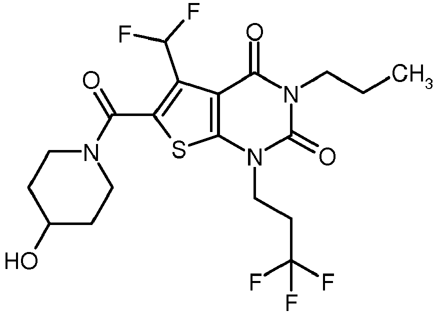

Analog zu dem unter Bsp. 137 beschriebenen Verfahren wurden aus 55 mg (0.137 mmol) der Verbindung aus Bsp. 148A und 17 mg (0.165 mmol) 4-Hydroxypiperidin 54 mg (81% d. Th.) der Titelverbindung erhalten.

¹H-NMR (400 MHz, DMSO-d₆, δ/ppm): 7.32 (t, 1H), 4.81 (breit, 1H), 4.15 (t, 2H), 3.95 (breit, 1H), 3.84 (t, 2H), 3.75 (breit, 1H), 3.50 (breit, 1H), 3.21 (breit, 2H), 2.89-2.75 (m, 2H), 1.73 (breit, 2H), 1.58 (m, 2H), 1.36 (breit, 2H), 0.88 (t, 3H).

LC/MS (Methode 1, ESIpos): Rₜ = 0.88 min, m/z = 484 [M+H]⁺.

### Beispiel 142

### 5-(Difluormethyl)-6-[(4-hydroxy-4-methylpiperidin-1-yl)carbonyl]-3-propyl-1-(3,3,3-trifluor-propyl)thieno[2,3-d]pyrimidin-2,4(1H,3H)-dion

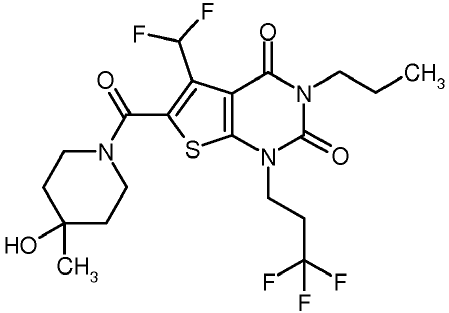

Analog zu dem unter Bsp. 137 beschriebenen Verfahren wurden aus 55 mg (0.137 mmol) der Verbindung aus Bsp. 148A und 19 mg (0.165 mmol) 4-Methylpiperidin-4-ol [kommerziell erhältlich; Lit. z.B.: J. M. McManus et al., J. Med. Chem. 1965, 8 (6), 766-776] 56 mg (78% d. Th., 95% Reinheit) der Titelverbindung erhalten.

¹H-NMR (400 MHz, DMSO-d₆, δ/ppm): 7.32 (t, 1H), 4.47 (breit, 1H), 4.15 (t, 2H), 4.05 (breit, 1H), 3.84 (t, 2H), 3.25 (breit, 3H), 2.87-2.75 (m, 2H), 1.58 (m, 2H), 1.55 (breit, 2H), 1.44 (breit, 2H), 1.15 (s, 3H), 0.88 (t, 3H).

LC/MS (Methode 1, ESIpos): Rₜ = 0.94 min, m/z = 498 [M+H]⁺.

### Beispiel 143

### 3-Ethyl-5-(fluormethyl)-6-[(4-hydroxypiperidin-1-yl)carbonyl]-1-(3,3,3-trifluorpropyl)thieno-[2,3-d]pyrimidin-2,4(1H,3H)-dion

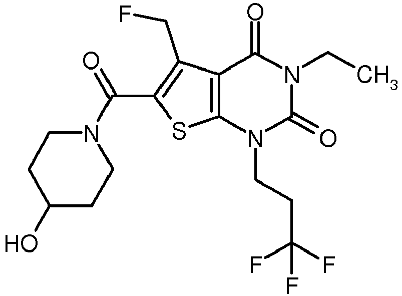

Analog zu dem unter Bsp. 137 beschriebenen Verfahren wurden aus 40 mg (0.109 mmol) der Verbindung aus Bsp. 149A und 13 mg (0.130 mmol) 4-Hydroxypiperidin 46 mg (93% d. Th.) der Titelverbindung erhalten. Die Reaktionszeit betrug in diesem Fall 1 h, und die präparative HPLC-Aufreinigung erfolgte nach Methode 19.

¹H-NMR (400 MHz, DMSO-d₆, δ/ppm): 5.57 (d, 2H), 4.15 (t, 2H), 3.92 (quart, 2H), 3.87-3.58 (breit und m, zus. 4H), 3.28-3.20 (m, 2H), 2.87-2.75 (m, 2H), 1.78-1.71 (m, 2H), 1.41-1.31 (m, 2H), 1.14 (t, 3H).

LC/MS (Methode 1, ESIpos): Rₜ = 0.81 min, m/z = 452 [M+H]⁺.

### Beispiel 144

### 3-Ethyl-5-(fluormethyl)-6-[(4-hydroxy-4-methylpiperidin-1-yl)carbonyl]-1-(3,3,3-trifluorpropyl)-thieno[2,3-d]pyrimidin-2,4(1H,3H)-dion

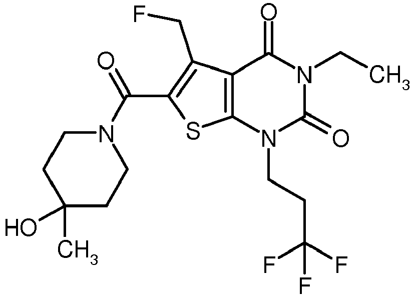

Analog zu dem unter Bsp. 137 beschriebenen Verfahren wurden aus 60 mg (0.163 mmol) der Verbindung aus Bsp. 149A und 30 mg (0.195 mmol) 4-Methylpiperidin-4-ol [kommerziell erhältlich; Lit. z.B.: J. M. McManus et al., J. Med. Chem. 1965, 8 (6), 766-776] 53 mg (69% d. Th.) der Titelverbindung erhalten. Die Reaktionszeit betrug in diesem Fall 1 h, und die präparative HPLC-Aufreinigung erfolgte nach Methode 19.

¹H-NMR (400 MHz, DMSO-d₆, δ/ppm): 5.57 (d, 2H), 4.15 (t, 2H), 3.92 (quart, 2H), 3.36-3.27 (m, 2H), 2.87-2.75 (m, 2H), 1.52-1.40 (m, 4H), 1.15 (s, 3H), 1.14 (t, 3H).

LC/MS (Methode 1, ESIpos): Rₜ = 0.84 min, m/z = 466 [M+H]⁺.

### Beispiel 145

### 3-Ethyl-5-(1-fluorethyl)-6-[(4-hydroxypiperidin-1-yl)carbonyl]-1-(3,3,3-trifluorpropyl)thieno-[2,3-d]pyrimidin-2,4(1H,3H)-dion (Racemat)

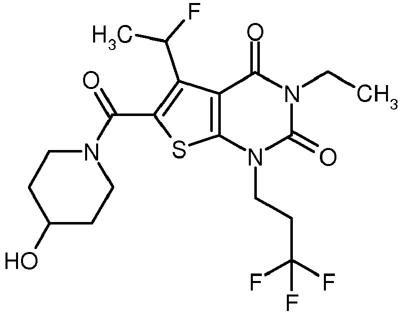

Analog zu dem unter Bsp. 137 beschriebenen Verfahren wurden aus 11 mg (0.029 mmol) der Verbindung aus Bsp. 150A und 3.5 mg (0.035 mmol) 4-Hydroxypiperidin 13 mg (97% d. Th.) der Titelverbindung erhalten. Die Reaktionszeit betrug in diesem Fall 1 h, und die präparative HPLC-Aufreinigung erfolgte nach Methode 19.

¹H-NMR (400 MHz, DMSO-d₆, δ/ppm): 6.20 (d von quart, 1H), 4.19-4.06 (m, 4H), 3.91 (quart, 2H), 3.76-3.70 (m, 1H), 3.25-3.11 (m, 2H), 2.86-2.74 (m, 2H), 1.76-1.66 (m, 2H), 1.61 (dd, 3H), 1.39-1.29 (m, 2H), 1.13 (t, 3H).

LC/MS (Methode 1, ESIpos): Rₜ = 0.81 min, m/z = 466 [M+H]⁺.

### Beispiel 146

### 3-Ethyl-5-(1-fluorethyl)-6-[(4-hydroxy-4-methylpiperidin-1-yl)carbonyl]-1-(3,3,3-trifluorpropyl)-thieno[2,3-d]pyrimidin-2,4(1H,3H)-dion (Racemat)

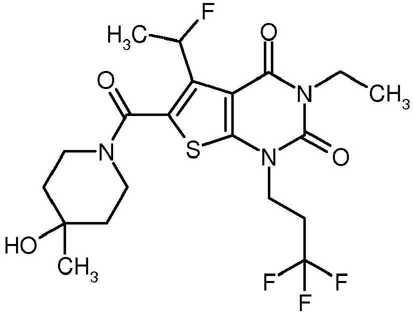

Analog zu dem unter Bsp. 137 beschriebenen Verfahren wurden aus 80 mg (0.178 mmol, 85% Reinheit) der Verbindung aus Bsp. 150A und 32 mg (0.213 mmol) 4-Methylpiperidin-4-ol-Hydrochlorid [kommerziell erhältlich; Lit. z.B.: J. M. McManus et al., J. Med. Chem. 1965, 8 (6), 766-776] 46 mg (53% d. Th.) der Titelverbindung erhalten. Die Reaktionszeit betrug in diesem Fall 1 h, und die präparative HPLC-Aufreinigung erfolgte nach Methode 19.

¹H-NMR (400 MHz, DMSO-d₆, δ/ppm): 6.20 (d von quart, 1H), 4.44 (breit, 1H), 4.19-4.05 (m, 2H), 3.91 (quart, 2H), 3.24 (breit, 2H), 2.86-2.74 (m, 2H), 1.61 (dd, 3H), 1.52-1.37 (m, 4H), 1.14 (s, 3H), 1.13 (t, 3H).

LC/MS (Methode 1, ESIpos): Rₜ = 0.89 min, m/z = 480 [M+H]⁺.

### Beispiel 147

### 3-Ethyl-5-(1-fluorethyl)-6-[(3-oxopiperazin-1-yl)carbonyl]-1-(3,3,3-trifluorpropyl)thieno[2,3-d]-pyrimidin-2,4(1H,3H)-dion (Racemat)

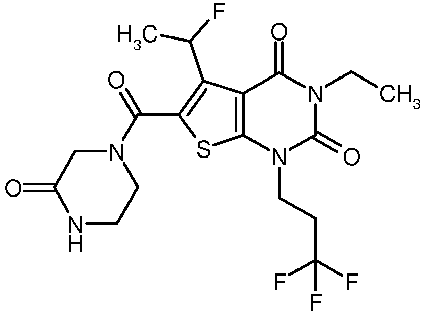

Analog zu dem unter Bsp. 137 beschriebenen Verfahren wurden aus 100 mg (0.222 mmol, 85% Reinheit) der Verbindung aus Bsp. 150A und 27 mg (0.267 mmol) 2-Oxopiperazin 72 mg (66% d. Th., 95% Reinheit) der Titelverbindung erhalten. Die Reaktionszeit betrug in diesem Fall 1 h, und die präparative HPLC-Aufreinigung erfolgte nach Methode 19.

¹H-NMR (400 MHz, DMSO-d₆, δ/ppm): 8.12 (s, 1H), 6.23 (d von quart, 1H), 4.19-4.07 (m, 2H), 3.91 (quart, 2H), 3.60-3.48 (breit, 4H), 3.22-3.18 (breit, 2H), 2.87-2.75 (m, 2H), 1.61 (dd, 3H), 1.13 (t, 3H).

LC/MS (Methode 17, ESIpos): Rₜ = 2.09 min, m/z = 465 [M+H]⁺.

### Beispiel 148

### 3-Ethyl-5-(1-fluorethyl)-6-[(3-oxopiperazin-1-yl)carbonyl]-1-(3,3,3-trifluorpropyl)thieno[2,3-d]-pyrimidin-2,4(1H,3H)-dion (Enantiomer 1)

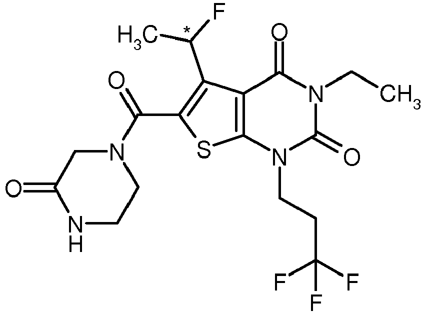

60 mg (0.123 mmol) der racemischen Verbindung aus Bsp. 147 wurden in 7 ml Methanol/ Ethanol/Acetonitril (1:1:1) gelöst und in 4 Portionen mittels präparativer SFC-HPLC an chiraler Phase in die Enantiomere getrennt [Säule: Daicel Chiralpak IC, 5 µm, 250 mm x 20 mm; Eluent: Kohlendioxid/Ethanol 75:25; Fluss: 60 ml/min; Temperatur: 40°C; Detektion: 210 nm]. Nach Einengen der Produktfraktionen und Trocknen des Rückstands im Hochvakuum wurden 12 mg (42% d. Th.) von Enantiomer 1 erhalten.

¹H-NMR (400 MHz, DMSO-d₆, δ/ppm): 8.12 (s, 1H), 6.23 (d von quart, 1H), 4.19-4.08 (m, 2H), 3.97 (breit, 2H), 3.91 (quart, 2H), 3.56 (breit, 2H), 3.20 (breit, 2H), 2.86-2.76 (m, 2H), 1.61 (dd, 3H), 1.13 (t, 3H).

LC/MS (Methode 1, ESIpos): Rₜ = 0.78 min, m/z = 465 [M+H]⁺.

Chirale analytische SFC [Säule: Daicel Chiralpak IC, 3 µm, 250 mm x 4.6 mm; Eluent: Kohlendioxid/Methanol 95:5 → 50:50; Fluss: 3 ml/min; Temperatur: 40°C; Detektion: 210 nm]: Rₜ = 2.91 min; >99% ee.

### Beispiel 149

### 3-Ethyl-5-(1-fluorethyl)-6-[(3-oxopiperazin-1-yl)carbonyl]-1-(3,3,3-trifluorpropyl)thieno[2,3-d]-pyrimidin-2,4(1H,3H)-dion (Enantiomer 2)

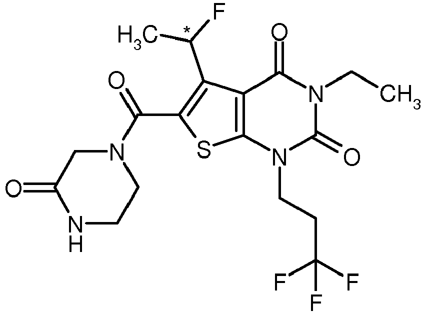

60 mg (0.123 mmol) der racemischen Verbindung aus Bsp. 147 wurden in 7 ml Methanol/ Ethanol/Acetonitril (1:1:1) gelöst und in 4 Portionen mittels präparativer SFC-HPLC an chiraler Phase in die Enantiomere getrennt [Säule: Daicel Chiralpak IC, 5 µm, 250 mm x 20 mm; Eluent: Kohlendioxid/Ethanol 75:25; Fluss: 60 ml/min; Temperatur: 40°C; Detektion: 210 nm]. Nach Einengen der Produktfraktionen und Trocknen des Rückstands im Hochvakuum wurden 12 mg (42% d. Th.) von Enantiomer 2 erhalten.

¹H-NMR (400 MHz, DMSO-d₆, δ/ppm): 8.12 (s, 1H), 6.23 (d von quart, 1H), 4.18-4.08 (m, 2H), 3.96 (breit, 2H), 3.91 (quart, 2H), 3.57 (breit, 2H), 3.20 (breit, 2H), 2.86-2.76 (m, 2H), 1.61 (dd, 3H), 1.13 (t, 3H).

LC/MS (Methode 1, ESIpos): Rₜ = 0.78 min, m/z = 465 [M+H]⁺.

Chirale analytische SFC [Säule: Daicel Chiralpak IC, 3 µm, 250 mm x 4.6 mm; Eluent: Kohlendioxid/Methanol 95:5 → 50:50; Fluss: 3 ml/min; Temperatur: 40°C; Detektion: 210 nm]: Rₜ = 3.48 min; >99% ee.

### Beispiel 150

### 5-(Difluormethyl)-6-[(4-hydroxypiperidin-1-yl)carbonyl]-3-propyl-1-[2-(trifluormethoxy)ethyl]-thieno[2,3-d]pyrimidin-2,4(1H,3H)-dion

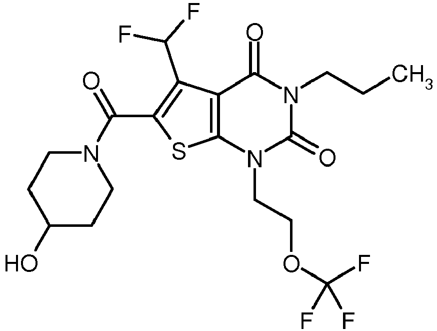

Analog zu dem unter Bsp. 137 beschriebenen Verfahren wurden aus 100 mg (0.240 mmol) der Verbindung aus Bsp. 154A und 29 mg (0.288 mmol) 4-Hydroxypiperidin 93 mg (77% d. Th.) der Titelverbindung erhalten.

¹H-NMR (400 MHz, DMSO-d₆, δ/ppm): 7.32 (t, 1H), 4.81 (d, 1H), 4.42 (t, 2H), 4.25 (t, 2H), 3.95 (breit, 1H), 3.85 (t, 2H), 3.74 (m, 1H), 3.47 (breit, 1H), 3.20 (breit, 2H), 1.72 (breit, 2H), 1.58 (m, 2H), 1.35 (breit, 2H), 0.87 (t, 3H).

LC/MS (Methode 1, ESIpos): Rₜ = 0.89 min, m/z = 500 [M+H]⁺.

### Beispiel 151

### 5-(Difluormethyl)-6-[(4-hydroxy-4-methylpiperidin-1-yl)carbonyl]-3-propyl-1-[2-(trifluor-methoxy)ethyl]thieno[2,3-d]pyrimidin-2,4(1H,3H)-dion

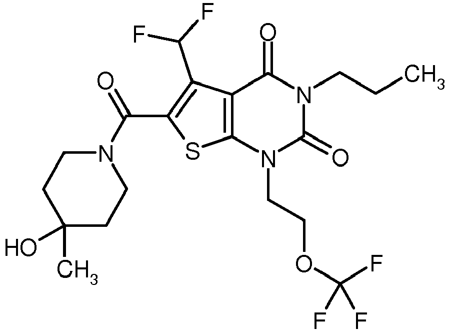

Analog zu dem unter Bsp. 137 beschriebenen Verfahren wurden aus 100 mg (0.240 mmol) der Verbindung aus Bsp. 154A und 33 mg (0.165 mmol) 4-Methylpiperidin-4-ol [kommerziell erhältlich; Lit. z.B.: J. M. McManus et al., J. Med. Chem. 1965, 8 (6), 766-776] 65 mg (52% d. Th.) der Titelverbindung erhalten. Abweichend vom zuvor beschriebenen Verfahren schloss sich hier an die erste präparative HPLC-Aufreinigung (nach Methode 5) noch eine zweite HPLC-Aufreinigung an [Säule: XBridge C18, 5 µm, 100 mm x 30 mm; Laufmittel: Wasser/Acetonitril/1% aq. Ammoniak 55:40:5; Fluss: 75 ml/min; Temperatur: 40°C; Detektion: 210 nm].

¹H-NMR (400 MHz, DMSO-d₆, δ/ppm): 7.31 (t, 1H), 4.48 (s, 1H), 4.42 (t, 2H), 4.25 (t, 2H), 4.05 (breit, 1H), 3.85 (t, 2H), 3.30 (breit, 1H), 3.21 (breit, 2H), 1.58 (m, 2H), 1.53 (breit, 2H), 1.43 (breit, 2H), 1.14 (s, 3H), 0.87 (t, 3H).

LC/MS (Methode 1, ESIpos): Rₜ = 0.94 min, m/z = 514 [M+H]⁺.

### Beispiel 152

### 6-{[4-Hydroxy-4-(hydroxymethyl)piperidin-1-yl]carbonyl}-3-isobutyl-5-methyl-1-(3,3,3-trifluor-propyl)thieno[2,3-d]pyrimidin-2,4(1H,3H)-dion

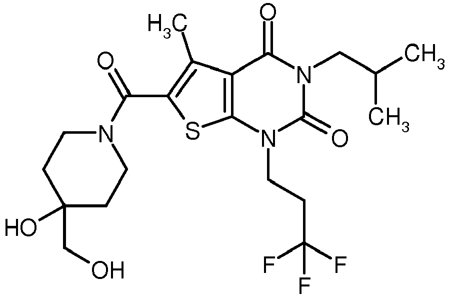

Analog zu dem unter Bsp. 122 beschriebenen Verfahren wurden aus 100 mg (0.264 mmol) der Verbindung aus Bsp. 160A und 53 mg (0.317 mmol) 4-(Hydroxymethyl)piperidin-4-ol-Hydrochlorid [kommerziell erhältlich; Lit. z.B.: WO 2005/103037-A2, Beispiel A3b (freie Base); US 2011/288065-A1, Example 105 / Step 1 (Hydrochlorid)] 106 mg (81% d. Th.) der Titelverbindung erhalten. Die Reaktionszeit betrug in diesem Fall 30 min.

¹H-NMR (400 MHz, DMSO-d₆, δ/ppm): 4.64 (t, 1H), 4.36 (s, 1H), 4.13 (t, 2H), 3.85 (breit, 2H), 3.72 (d, 2H), 3.28 (br. t, 2H), 3.21 (d, 2H), 2.85-2.73 (m, 2H), 2.37 (s, 3H), 2.04 (m, 1H), 1.55 (br. dt, 2H), 1.40 (br. d, 2H), 0.86 (d, 6H).

LC/MS (Methode 1, ESIpos): Rₜ = 0.82 min, m/z = 492 [M+H]⁺.

### Beispiel 153

### 3-(Cyclopropylmethyl)-6-{[4-hydroxy-4-(hydroxymethyl)piperidin-1-yl]carbonyl}-5-methyl-1-(3,3,3-trifluorpropyl)thieno[2,3-d]pyrimidin-2,4(1H,3H)-dion

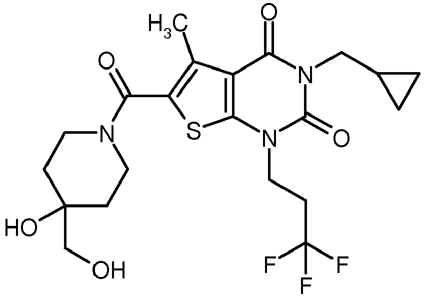

Analog zu dem unter Bsp. 122 beschriebenen Verfahren wurden aus 100 mg (0.266 mmol) der Verbindung aus Bsp. 161A und 53 mg (0.319 mmol) 4-(Hydroxymethyl)piperidin-4-ol-Hydrochlorid [kommerziell erhältlich; Lit. z.B.: WO 2005/103037-A2, Beispiel A3b (freie Base); US 2011/288065-A1, Example 105 / Step 1 (Hydrochlorid)] 45 mg (34% d. Th.) der Titelverbindung erhalten. Die Reaktionszeit betrug in diesem Fall 30 min.

¹H-NMR (400 MHz, DMSO-d₆, δ/ppm): 4.64 (breit, 1H), 4.36 (br. s, 1H), 4.14 (t, 2H), 3.85 (breit, 2H), 3.77 (d, 2H), 3.27 (br. t, 2H), 3.21 (breit, 2H), 2.86-2.74 (m, 2H), 2.38 (s, 3H), 1.55 (br. dt, 2H), 1.40 (br. d, 2H), 1.17 (m, 1H), 0.45-0.40 (m, 2H), 0.36-0.32 (m, 2H).

LC/MS (Methode 1, ESIpos): Rₜ = 0.78 min, m/z = 490 [M+H]⁺.

### Beispiel 154

### 6-{[4-Hydroxy-4-(hydroxymethyl)piperidin-1-yl]carbonyl}-3-(2-methoxypropyl)-5-methyl-1-(3,3,3-trifluorpropyl)thieno[2,3-d]pyrimidin-2,4(1H,3H)-dion (Racemat)

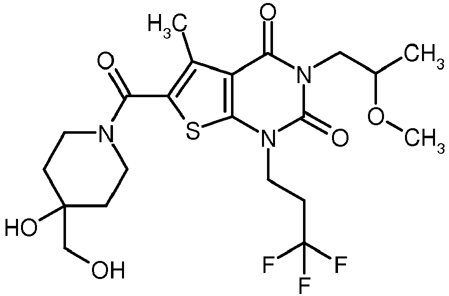

Analog zu dem unter Bsp. 122 beschriebenen Verfahren wurden aus 99 mg (0.166 mmol, 66% Reinheit) der Verbindung aus Bsp. 162A und 33 mg (0.199 mmol) 4-(Hydroxymethyl)piperidin-4-ol-Hydrochlorid [kommerziell erhältlich; Lit. z.B.: WO 2005/103037-A2, Beispiel A3b (freie Base); US 2011/288065-A1, Example 105 / Step 1 (Hydrochlorid)] 37 mg (44% d. Th.) der Titelverbindung erhalten. Die Reaktionszeit betrug hier 30 min.

¹H-NMR (400 MHz, DMSO-d₆, δ/ppm): 4.64 (t, 1H), 4.37 (s, 1H), 4.15-4.11 (m, 2H), 4.06 (dd, 1H), 3.85 (breit, 2H), 3.77 (dd, 1H), 3.68-3.60 (m, 1H), 3.28 (breit, 2H), 3.22 (s, 3H), 3.21 (d, 2H), 2.85-2.73 (m, 2H), 2.38 (s, 3H), 1.55 (br. dt, 2H), 1.40 (br. d, 2H), 1.06 (d, 3H).

LC/MS (Methode 1, ESIpos): Rₜ = 0.75 min, m/z = 508 [M+H]⁺.

### B. Bewertung der pharmakologischen Wirksamkeit

Die pharmakologische Aktivität der erfindungsgemäßen Verbindungen kann durch *in vitro-* und *in* vivo-Untersuchungen, wie sie dem Fachmann bekannt sind, nachgewiesen werden. Die nachfolgenden Anwendungsbeispiele beschreiben die biologische Wirkung der erfindungsgemäßen Verbindungen, ohne die Erfindung auf diese Beispiele zu beschränken.

### B-1. Zelluläre in vitro-Tests zur Bestimmung der A2b-Rezeptor-Aktivität und der Adenosin-rezeptor-Selektivität

Die Identifizierung von selektiven Antagonisten des humanen Adenosin A2b-Rezeptors sowie die Quantifizierung der Wirksamkeit und Selektivität der erfindungsgemäßen Verbindungen erfolgte mit Hilfe von rekombinanten Zelllinien für die humanen Adenosin-Rezeptoren A1, A2a, A2b und A3. Diese Zelllinien leiten sich ursprünglich von einer Ovarepithelzelle des Hamsters ab (Chinese Hamster Ovary, CHO-K1, American Type Culture Collection, Manassas, VA 20108, USA). Die Zelllinien zur Testung der Wirksamkeit an den A1-, A2a- und A2b-Rezeptoren enthalten neben dem jeweils rekombinant exprimierten Adenosin-Rezeptor ein Reportergen-Konstrukt, bei dem die Expression der Leuchtkäfer (*Photinus pyradis*)-Luziferase unter der Kontrolle eines Promotors steht, der über intrazelluläre Signalkaskaden durch Stimulation der Rezeptoren mit dem (nicht Subtyp-selektiven) Adenosinrezeptor-Agonisten NECA (5'-*N*-Ethylcarboxamidoadenosin) aktiviert werden kann [S.J. Hill, J.G. Baker, S. Rhees, Curr. Opin. Pharmacol. 1, 526-532 (2001)].

Im Falle der A2a- und A2b-Zelllinien handelt es sich um einen Minimalpromotor mit mehreren cAMP-responsiblen Elementen (CRE). Stimulation der Gₛ-gekoppelten A2b- oder A2a-Rezeptoren durch NECA führt über die Bildung von cAMP letztlich zur CRE-abhängigen Induktion der Luziferase-Expression, die 3 Stunden nach Beginn der Inkubation mit NECA mit einer Detektionslösung in einem geeigneten Luminometer nachgewiesen wird. Zur Testung der Antagonisten wird zunächst in einem Vorversuch die Konzentration von NECA bestimmt, die am jeweiligen Versuchstag zur halbmaximalen Stimulation der Luziferase-Expression führt (EC₅₀-Konzentration). Durch gemeinsame Inkubation dieser EC₅₀-Konzentration von NECA mit den zu testenden Substanzen kann dann deren antagonistische Wirkung bestimmt werden.

Die Zelllinie zur Testung des Gᵢ-gekoppelten A1-Rezeptors enthält ein anderes Reportergen-Konstrukt, bei dem die Expression der Leuchtkäfer-Luziferase unter der Kontrolle eines NFAT (nuclear factor of activated T-cells)-Promoters steht. Diese Zelllinie wurde neben dem A1-Rezeptor und dem NFAT-Reportergen auch noch mit einem weiteren Gen, das für das promiskuitive Gα₁₆-Protein kodiert [T.T. Amatruda, D.A. Steele, V.Z. Slepak, M.I. Simon, Proc. Natl. Acad. Sci. USA 88, 5587-5591 (1991)], entweder unabhängig oder als Fusionsgen stabil transfiziert. Die daraus resultierenden Testzellen reagieren auf Stimulation des normalerweise Gᵢ-gekoppelten A1-Rezeptors mit einer Erhöhung der intrazellulären Calcium-Konzentration, die dann zu einer NFAT-abhängigen Luziferase-Expression führt. Die Versuchsdurchführung zur Testung der Antagonisten auf dem A1-Rezeptor entspricht dem Vorgehen für die Testung mit den A2a- und A2b-Zelllinien.

Bei der Erzeugung der A3-Rezeptor-Zelllinie wurde ebenfalls eine Ko-Transfektion von A3-Rezeptor und dem promiskuitiven Gα₁₆-Protein durchgeführt, so dass auch hier die Stimulation des Rezeptors zu einer Erhöhung der intrazellulären Calcium-Konzentration führt. Diese Calcium-Erhöhung wird im A3-Rezeptor-Test allerdings direkt über das Calcium-sensitive Photoprotein Photina® [S. Bovolenta, M. Foti, S. Lohmer, S. Corazza, J. Biomol. Screen. 12, 694-704 (2007)] gemessen. Nach Bestimmung der EC₅₀-Konzentration von NECA erfolgt die Messung der Substanzwirkungen nach 5-10 Minuten Vorinkubation mit Substanz durch Zugabe dieser EC₅₀-Konzentration in Messposition in einem geeigneten dispensierfähigen Luminometer.

In der folgenden Tabelle 1 sind für individuelle Ausführungsbeispiele die IC₅₀-Werte aus dem A2b-Rezeptor-Assay aufgeführt (zum Teil als Mittelwerte aus mehreren unabhängigen Einzelbestimmungen und gerundet auf zwei signifikante Stellen):

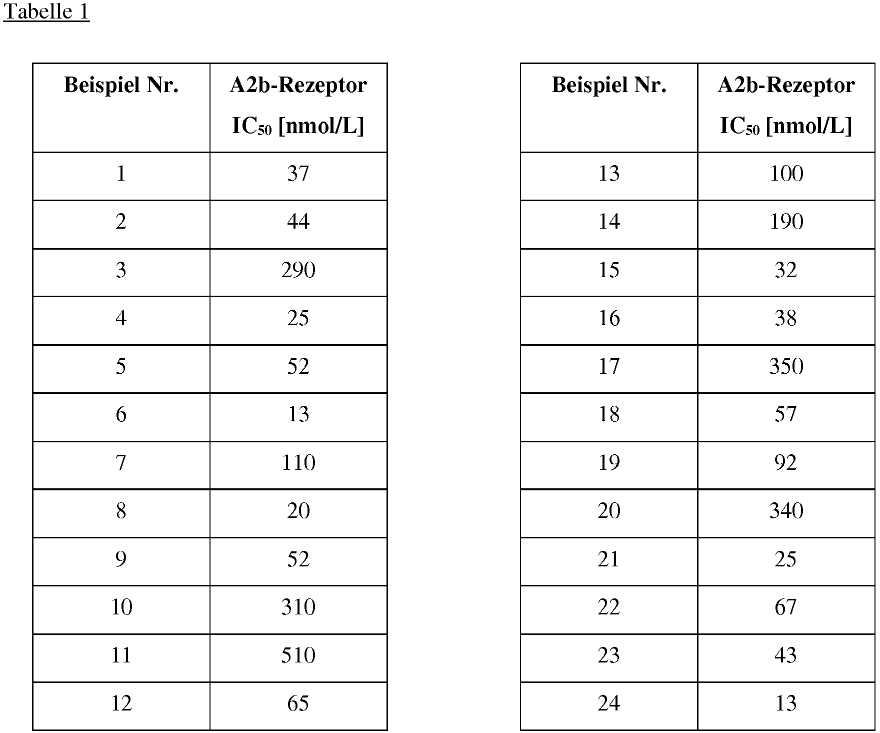

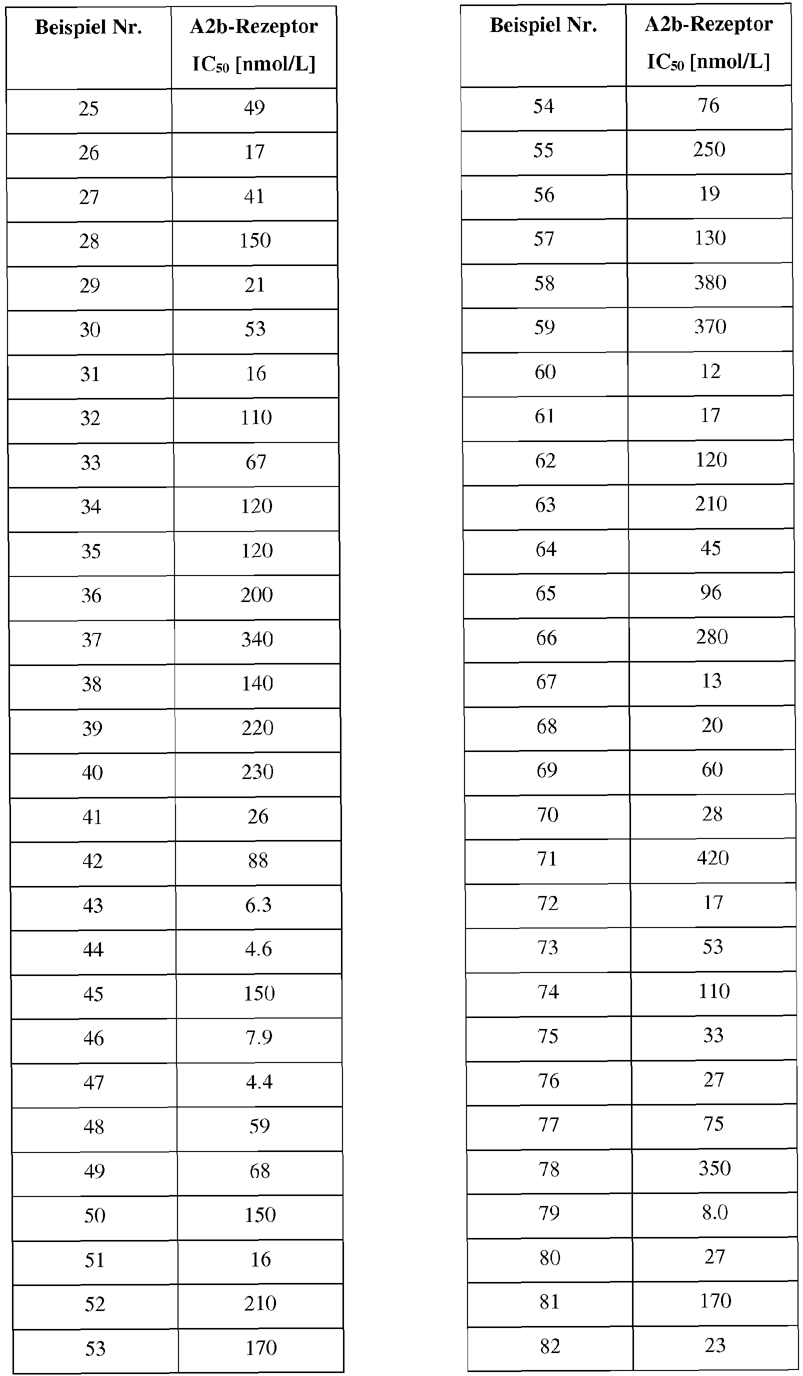

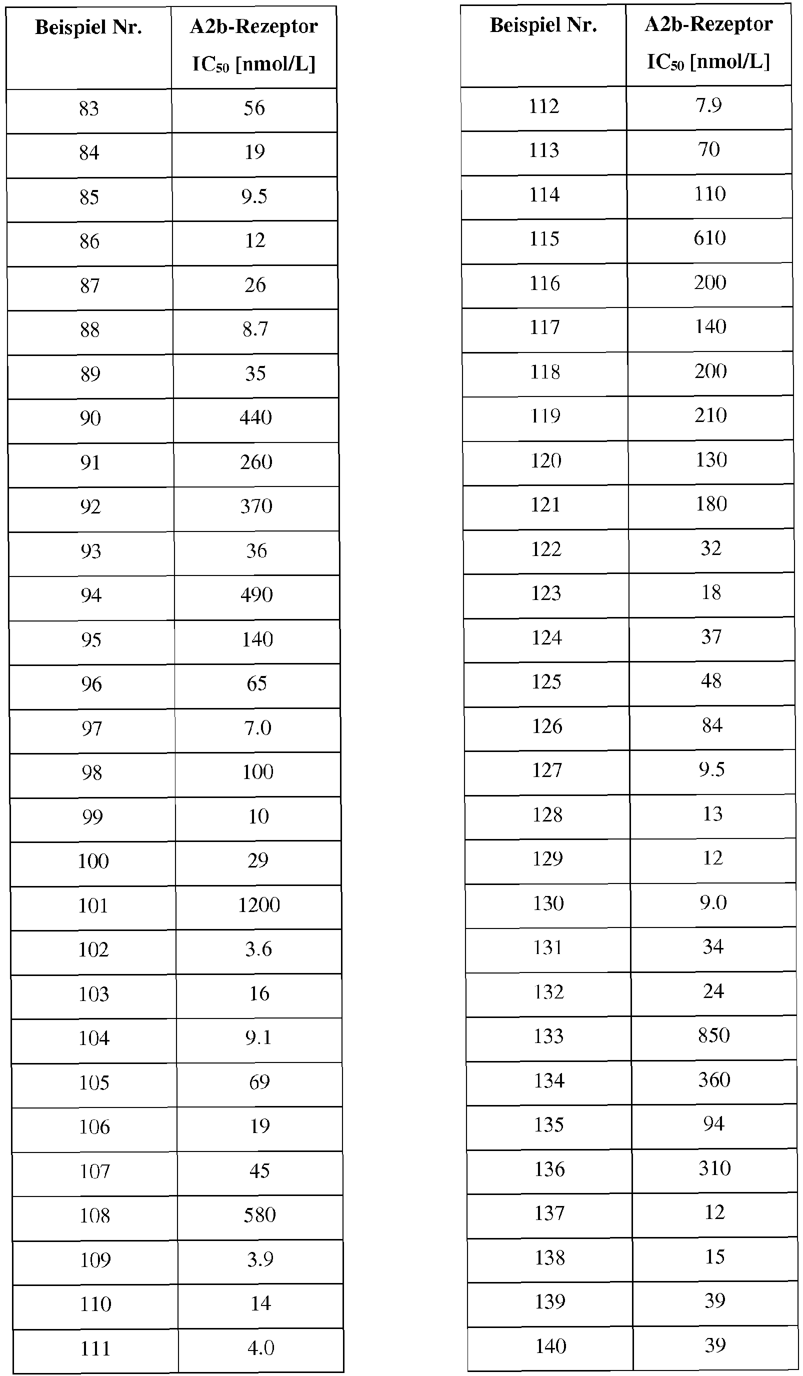

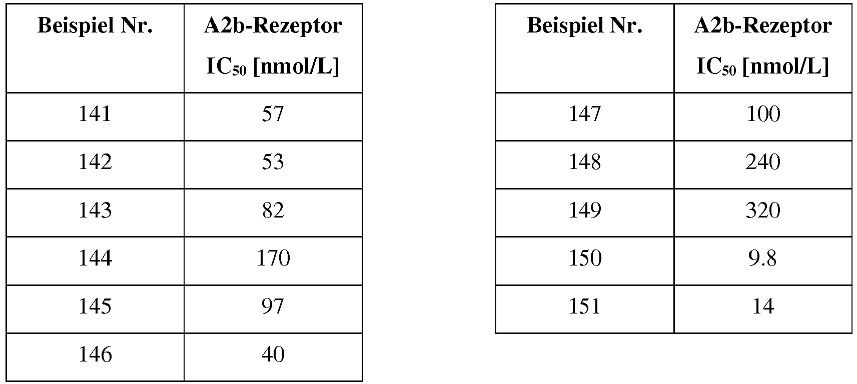

### B-2. Messung der NECA-induzierten IL-6-Freisetzung von LL29-Fibroblasten

Die Stimulation von Fibroblasten mit Adenosin oder dem Adenosin-Analogon 5'-*N*-Ethylcarboxamidoadenosin (NECA) führt zu einer Freisetzung des pro-inflammatorischen und pro-fibrotischen Zytokins IL-6, die durch Hemmung des A2b-Rezeptors verhindert werden kann.

Es werden daher konfluente Zellen der humanen Fibroblasten-Zelllinie LL29 mit den Testsubstanzen behandelt und mit NECA (10 µM) stimuliert. Nach einer Inkubationszeit von 24 Stunden wird der Zellüberstand abgenommen und humanes IL-6 mittels ELISA (Quantikine® IL6 ELISA, R&D Systems, Minneapolis, USA) im Zellüberstand bestimmt.

In der folgenden Tabelle 2 sind für repräsentative Ausführungsbeispiele die IC₅₀-Werte aus diesem Assay aufgeführt (zum Teil als Mittelwerte aus mehreren unabhängigen Einzelbestimmungen und gerundet auf zwei signifikante Stellen):

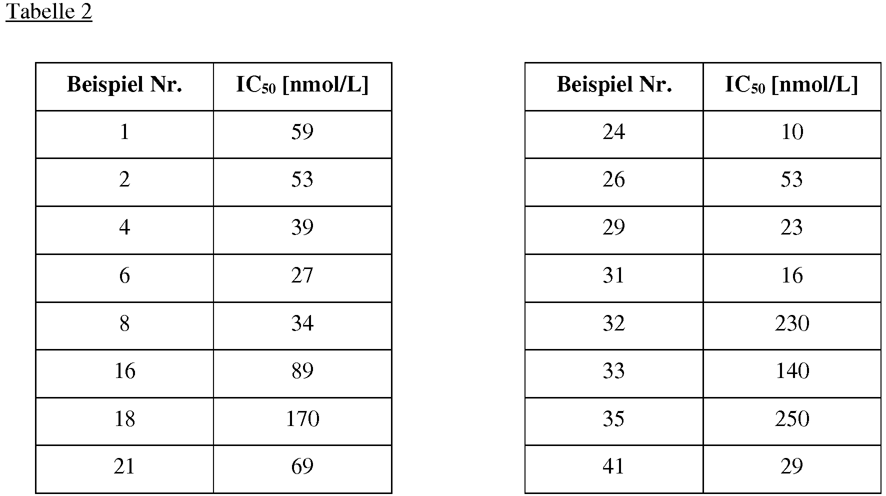

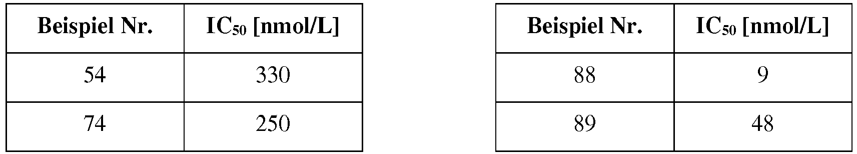

### B-3. Tiermodell der Monocrotalin-induzierten pulmonalen Hypertonie

Die Monocrotalin-induzierte pulmonale Hypertonie der Ratte ist ein weit verbreitetes Tiermodell für die pulmonale Hypertonie. Das Pyrrolizidin-Alkaloid Monocrotalin wird nach subkutaner Injektion in der Leber zum toxischen Monocrotalinpyrrol metabolisiert und führt innerhalb weniger Tage zu einer Endothelschädigung im Lungenkreislauf, gefolgt von einem Remodeling der kleinen pulmonalen Arterien (Mediahypertrophie, *de* novo-Muskularisierung). Eine einmalige subkutane Injektion ist ausreichend, um bei Ratten innerhalb von 4 Wochen eine ausgeprägte pulmonale Hypertonie zu induzieren [Cowan et al., Nature Med. 6, 698-702 (2000)].

Für das Modell werden männliche Sprague-Dawley-Ratten verwendet. An Tag 0 erhalten die Tiere eine subkutane Injektion von 60 mg/kg Monocrotalin. Die Behandlung der Tiere mit der Testsubstanz (per gavage, durch Zusatz im Futter oder Trinkwasser, per osmotischer Minipumpe, per subkutaner oder intraperitonealer Injektion oder per Inhalation) beginnt erst frühestens 14 Tage nach der Monocrotalin-Injektion und erstreckt sich über einen Zeitraum von mindestens 14 Tagen. Am Studienende erfolgen hämodynamische Untersuchungen der Tiere. Für die hämodynamische Messung werden die Ratten initial mit Pentobarbital (60 mg/kg) anästhesiert. Anschließend werden die Tiere tracheotomiert und künstlich beatmet (Frequenz: 60 Atemzüge/min; Verhältnis Inspiration zu Exspiration: 50:50; positiver end-exspiratorischer Druck: 1 cm H₂O; Atemzugvolumen: 10 ml/kg Körpergewicht; FIO₂: 0.5). Die Narkose wird durch Isofluran-Inhalationsnarkose aufrecht erhalten. Der systemische Blutdruck wird in der linken *A. carotis* mittels eines Millar-Microtip-Katheters ermittelt. Ein Polyethylenkatheter wird über die rechte *V. jugularis* in den rechten Ventrikel vorgeschoben zur Bestimmung des rechten Ventrikeldrucks. Im Anschluss an die Hämodynamik wird das Herz entnommen, das Verhältnis rechter zu linker Ventrikel inklusive Septum bestimmt und das Gewebe für Expressionsanalysen tiefgefroren. Die Lunge wird ebenfalls entnommen, die linke Lungenhälfte wird in Formalin zur histopathologischen Untersuchung fixiert und die rechte Lungenhälfte wird für Expressionsanalysen tiefgefroren. Weiterhin werden Plasmaproben zur Bestimmung von Biomarkern (zum Beispiel proBNP) und Plasma-Substanzspiegeln gewonnen.

### B-4. Tiermodell der SU5416/Hypoxie-induzierten pulmonalen Hypertonie

Die SU5416/Hypoxie-induzierte pulmonale Hypertonie der Ratte ist ein weit verbreitetes Tiermodell für die pulmonale Hypertonie. Durch die Injektion des VEGF-Rezeptor-Antagonisten SU5416 in Kombination mit Hypoxie kann die Auswirkung des reduzierten Sauerstoffgehalts verstärkt werden und zu Endothelveränderungen in Form von plexiformen Läsionen führen. Eine einmalige subkutane Injektion, in der Regel von 20 mg/kg, ist ausreichend, um in Kombination mit Hypoxie, das heißt, erhöhten vaskulären Scherkräften durch Vasokonstriktion, eine schwere pulmonale Hypertonie zu induzieren [Oka et al., Circ. Res. 100, 923-929 (2007)].

Für das Modell werden männliche Sprague-Dawley-Ratten oder Dahl-Salz-Ratten verwendet. An Tag 0 erhalten die Tiere eine subkutane Injektion von SU5416 und werden in kontrollierter hypoxischer Atmosphäre (10% Sauerstoff) gehalten. Entsprechende Kontrollratten erhalten eine Injektion von Vehikel und werden unter normoxischen Bedingungen gehalten. Chronische Hypoxie von mindestens 14 Tagen mit anschließender Normoxie von mindestens 28 Tagen führt zur Entwicklung einer funktionell und morphologisch nachweisbaren pulmonalen Hypertonie. Die Behandlung der Tiere mit der Testsubstanz (per gavage, durch Zusatz im Futter oder Trinkwasser, per osmotischer Minipumpe, per subkutaner oder intraperitonealer Injektion oder per Inhalation) beginnt frühestens 14 Tage nach SU5416-Injektion und Beginn der Haltung in kontrollierter hypoxischer Atmosphäre und erstreckt sich über einen Zeitraum von mindestens 14-28 Tagen.

Am Studienende erfolgen hämodynamische Untersuchungen der Tiere. Für die hämodynamische Messung werden die Ratten initial mit Pentobarbital (60 mg/kg) anästhesiert. Anschließend werden die Tiere tracheotomiert und künstlich beatmet (Frequenz: 60 Atemzüge/min; Verhältnis Inspiration zu Exspiration: 50:50; positiver end-exspiratorischer Druck: 1 cm H₂O; Atemzugvolumen: 10 ml/kg Körpergewicht; FIO₂: 0.5). Die Narkose wird durch Isofluran-Inhalationsnarkose aufrecht erhalten. Der systemische Blutdruck wird in der linken *A. carotis* mittels eines Millar-Microtip-Katheters ermittelt. Ein Polyethylenkatheter wird über die rechte *V. jugularis* in den rechten Ventrikel vorgeschoben zur Bestimmung des rechten Ventrikeldrucks. Im Anschluss an die Hämodynamik wird das Herz entnommen, das Verhältnis rechter zu linker Ventrikel inklusive Septum bestimmt und das Gewebe für Expressionsanalysen tiefgefroren. Die Lunge wird ebenfalls entnommen, die linke Lungenhälfte wird in Formalin zur histopathologischen Untersuchung fixiert und die rechte Lungenhälfte wird für Expressionsanalysen tiefgefroren. Weiterhin werden Plasmaproben zur Bestimmung von Biomarkern (zum Beispiel proBNP) und Plasma-Substanzspiegeln gewonnen.

### B-5. Tiermodell der Bleomycin-induzierten pulmonalen Fibrose

Die Bleomycin-induzierte Lungenfibrose bei der Maus oder Ratte ist ein weit verbreitetes Tiermodell für die Lungenfibrose. Bleomycin ist ein Glykopeptid-Antibiotikum, das in der Onkologie zur Therapie von Hodentumoren, Hodgkin- und Non-Hodgkin-Tumoren eingesetzt wird. Es wird renal eliminiert, besitzt eine Halbwertszeit von ca. 3 Stunden und beeinflusst als Zytostatikum verschiedene Phasen des Teilungszyklus [Lazo et al., Cancer Chemother. Biol. Response Modif. 15, 44-50 (1994)]. Sein anti-neoplastischer Effekt beruht auf einer oxidativ-schädigenden Wirkung auf DNA [Hay et al., Arch. Toxicol. 65, 81-94 (1991)]. Das Lungengewebe ist gegenüber Bleomycin in besonderer Weise gefährdet, da hier sog. Cysteinhydrolasen, welche in anderen Geweben zu einer Inaktivierung von Bleomycin führen, nur in geringer Anzahl vorhanden sind. Nach Gabe von Bleomycin kommt es bei den Tieren zu einem "acute respiratory distress syndrome" (ARDS) mit anschliessender Entwicklung einer Lungenfibrose.

Die Verabreichung des Bleomycins kann in einfacher oder mehrfacher Gabe intratracheal, inhalativ, intravenös oder intraperitoneal erfolgen. Die Behandlung der Tiere mit der Testsubstanz (per gavage, durch Zusatz im Futter oder Trinkwasser, per osmotischer Minipumpe, per subkutaner oder intraperitonealer Injektion oder per Inhalation) beginnt am Tag der ersten Applikation des Bleomycins oder therapeutisch 3-14 Tage später und erstreckt sich über einen Zeitraum von 2-6 Wochen. Am Studienende werden eine bronchio-alveoläre Lavage zur Bestimmung des Zellgehaltes und der pro-inflammatorischen und pro-fibrotischen Marker sowie eine histologische Beurteilung der Lungenfibrose durchgeführt.

### B-6. Tiermodell der DQ12-Quarz-induzierten pulmonalen Fibrose

DQ12-Quarz-induzierte Lungenfibrose an Maus und Ratte ist ein weit verbreitetes Tiermodell für Lungenfibrose [Shimbori et al., Exp. Lung Res. 36, 292-301 (2010)]. DQ12-Quarz ist ein durch Brechen beziehungsweise Mahlen hochaktiver Quarz. Die intratracheale oder inhalative Applikation von DQ12-Quarz führt bei Mäusen und Ratten zu einer Alveolarproteinose gefolgt von einer interstitiellen Lungenfibrose. Die Tiere erhalten eine einfache oder mehrfache intratracheale oder inhalative Instillation von DQ12-Quarz. Die Behandlung der Tiere mit der Testsubstanz (per gavage, durch Zusatz im Futter oder Trinkwasser, per osmotischer Minipumpe, per subkutaner oder intraperitonealer Injektion oder per Inhalation) beginnt am Tag der ersten Instillation des Silikats oder therapeutisch 3-14 Tage später und erstreckt sich über einen Zeitraum von 3-12 Wochen. Am Studienende werden eine bronchio-alveoläre Lavage zur Bestimmung des Zellgehalts und der pro-inflammatorischen und pro-fibrotischen Marker sowie eine histologische Beurteilung der Lungenfibrose durchgeführt.

### B-7. Tiermodell der DQ12-Quarz- oder FITC-induzierten pulmonalen Inflammation

Eine intratracheale Gabe von DQ12-Quarz oder Fluoresceinisothiocyanat (FITC) bei Maus und Ratte führt zu einer Inflammation in der Lunge [Shimbori et al., Exp. Lung Res. 36, 292-301 (2010)]. Die Tiere werden am Tag der Instillation von DQ12-Quarz oder FITC oder einen Tag später für eine Dauer von 24 h bis zu 7 Tagen mit der Testsubstanz behandelt (per gavage, durch Zusatz im Futter oder Trinkwasser, per osmotischer Minipumpe, per subkutaner oder intraperitonealer Injektion oder per Inhalation). Am Versuchsende wird eine bronchio-alveoläre Lavage zur Bestimmung des Zellgehaltes und der pro-inflammatorischen und pro-fibrotischen Marker durchgeführt.

### B-8. Tiermodell des Elastase-induzierten Lungenemphysems

Das Elastase-induzierte Lungenemphysem bei Maus, Ratte oder Hamster ist ein weit verbreitetes Tiermodell für Lungenemphysem [Sawada et al., Exp. Lung Res. 33, 277-288 (2007)]. Die Tiere erhalten eine orotracheale Instillation porciner Pankreas-Elastase. Die Behandlung der Tiere beginnt am Tag der Instillation der porcinen Pankreas-Elastase und erstreckt sich über einen Zeitraum von 3 Wochen. Am Studienende wird eine Alveolarmorphometrie durchgeführt.

### B-9. Tiermodell der permanenten Koronarligatur an Maus und Ratte

Mäuse bzw. Ratten werden mit 5% Isofluran im Narkosekäfig narkotisiert, intubiert, an eine Beatmungspumpe angeschlossen und mit 2% Isofluran/N₂O/O₂ beatmet. Die Körpertemperatur wird durch eine Wärmematte auf 37-38°C gehalten. Als Schmerzmittel wird Temgesic® gegeben. Der Brustkorb wird zwischen der dritten und vierten Rippe seitlich geöffnet und das Herz freigelegt. Die Herzkranzarterie des linken Ventrikels (LAD) wird mit einem Okklusionsfaden kurz unterhalb ihres Ursprungs (unterhalb des linken Atriums) unterstochen und permanent abgebunden. Der Thorax wird wieder geschlossen und die Muskelschichten und die Oberhaut zugenäht. Die Tiere werden vom Tag der Operation an oder bis zu einer Woche später für einen Zeitraum von 4-8 Wochen mit der Testsubstanz behandelt (per gavage, durch Zusatz der Testsubstanz im Futter oder Trinkwasser, per osmotischer Minipumpe, per subkutaner oder intraperitonealer Injektion oder per Inhalation). Als weitere Kontrolle wird eine "sham"-Gruppe mitgeführt, bei der nur der Operationsvorgang, nicht aber die LAD-Okklusion durchgeführt wurde.

Am Versuchsende werden die Tiere erneut narkotisiert [1.5% Isofluran (Maus), 2% Isofluran (Ratte)/N₂O/Luft], und ein Druckkatheter wird über die *A*. *carotis* in den linken Ventrikel eingeführt. Dort werden Herzfrequenz, linksventrikulärer Druck (LVP), linksventrikulärer end-diastolischer Druck (LVEDP), Kontraktilität (dp/dt) und Relaxationsgeschwindigkeit (tau) mit Hilfe des Powerlab-Systems (AD Instruments, ADI-PWLB-4SP) und der Chart5-Software (SN 425-0586) erfasst und ausgewertet. Anschließend wird eine Blutprobe zur Bestimmung der Substanz-Plasmaspiegel und Plasmabiomarker entnommen und die Tiere abgetötet. Herz (Herzkammern, linker Ventrikel plus Septum, rechter Ventrikel), Leber, Lunge und Niere werden entnommen und gewogen.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

### Tablettengewicht 212 mg. Durchmesser 8 mm, Wölbungsradius 12 mm

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel® (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 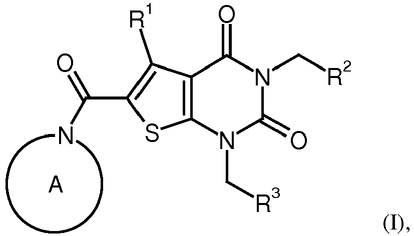 in welcher
R¹ für Wasserstoff, Methyl oder Ethyl steht, wobei Methyl und Ethyl bis zu dreifach mit Fluor substituiert sein können,
R² für Methyl, Ethyl, n-Propyl, Isopropyl, Cyclopropyl, Cyclopropylmethyl, 2,2-Difluorvinyl, 3,3-Difluorallyl oder Propargyl oder für eine Gruppe der Formel 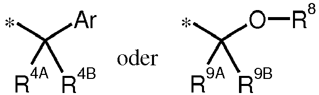 steht, worin * die Verknüpfung zur CH₂-Gruppe markiert,
Ar Phenyl oder 5- oder 6-gliedriges Heteroaryl mit bis zu zwei Ring-Stickstoffatomen bedeutet,
wobei Phenyl und Heteroaryl ein- oder zweifach, gleich oder verschieden, mit einem Rest ausgewählt aus der Reihe Fluor, Chlor, Methyl, Trifluormethyl, Methoxy und Trifluormethoxy substituiert sein können,
R^{4A} Wasserstoff, Fluor oder Methyl bedeutet,
R^{4B} Wasserstoff, Fluor, Methyl, Trifluormethyl, Hydroxy oder Methoxy bedeutet,
oder
R^{4A} und R^{4B} miteinander verknüpft sind und gemeinsam mit dem Kohlenstoffatom, an das sie gebunden sind, einen Cyclopropyl- oder Cyclobutyl-Ring bilden,
R⁸ Methyl oder Trifluormethyl bedeutet
und
R^{9A} und R^{9B} unabhängig voneinander Wasserstoff, Methyl oder Trifluormethyl bedeuten,
R³ für (C₁-C₆)-Alkyl, (C₂-C₆)-Alkenyl, (C₃-C₇)-Cycloalkyl oder [(C₃-C₇)-Cycloalkyl]methyl steht,
wobei Alkyl und Alkenyl bis zu dreifach und Cycloalkyl bis zu zweifach mit Fluor substituiert sein können
und
wobei in Alkyl und Cycloalkyl bis zu zwei CH₂-Gruppen gegen -O- oder -S- ausgetauscht sein können, mit der Maßgabe, dass sich zwischen solchen Heteroatomen, einschließlich des Uracil-N¹-Atoms, mindestens zwei Kohlenstoffatome befinden,
und
der Ring A für einen mono- oder bicyclischen Aza-Heterocyclus der Formel 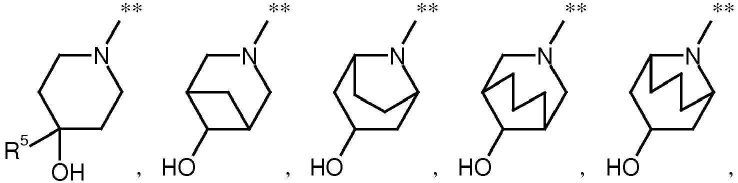 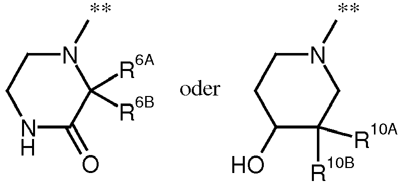 steht, worin ** die Verknüpfung zur Carbonylgruppe markiert,
R⁵ Wasserstoff, Methyl, Trifluormethyl, Hydroxymethyl oder Ethyl bedeutet,
R^{6A} und R^{6B} unabhängig voneinander Wasserstoff, Methyl oder Ethyl bedeuten,
R^{10A} Methyl, Ethyl, Hydroxy oder Methoxy bedeutet
und
R^{10B} Wasserstoff, Methyl oder Ethyl bedeutet,
sowie ihre Salze, Solvate und Solvate der Salze.

2. Verbindung der Formel (I) nach Anspruch 1, in welcher
R¹ für Methyl, Difluormethyl, Trifluormethyl, Ethyl oder 1-Fluorethyl steht,
R² für Methyl, Ethyl, n-Propyl, Isopropyl, 3,3-Difluorallyl oder Propargyl oder für eine Gruppe der Formel 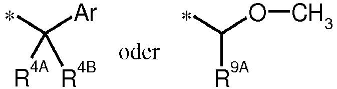 steht, worin * die Verknüpfung zur CH₂-Gruppe markiert,
Ar Phenyl oder Pyridyl bedeutet,
wobei Phenyl und Pyridyl mit Fluor, Chlor, Methyl oder Methoxy substituiert sein können,
R^{4A} Wasserstoff, Fluor oder Methyl bedeutet,
R^{4B} Wasserstoff, Fluor, Methyl, Hydroxy oder Methoxy bedeutet,
oder
R^{4A} und R^{4B} miteinander verknüpft sind und gemeinsam mit dem Kohlenstoffatom, an das sie gebunden sind, einen Cyclopropyl-Ring bilden,
und
R^{9A} Wasserstoff, Methyl oder Trifluormethyl bedeutet,
R³ für (C₂-C₄)-Alkyl, (C₂-C₄)-Alkenyl, (C₃-C₆)-Cycloalkyl oder [(C₃-C₆)-Cycloalkyl]methyl steht,
wobei Alkyl und Alkenyl bis zu dreifach und Cycloalkyl bis zu zweifach mit Fluor substituiert sein können
und
wobei in Alkyl und Cycloalkyl eine CH₂-Gruppe gegen -O- oder -S- ausgetauscht sein kann, mit der Maßgabe, dass sich zwischen einem solchen Heteroatom und dem Uracil-N¹-Atom mindestens zwei Kohlenstoffatome befinden,
und
der Ring A für einen Aza-Heterocyclus der Formel 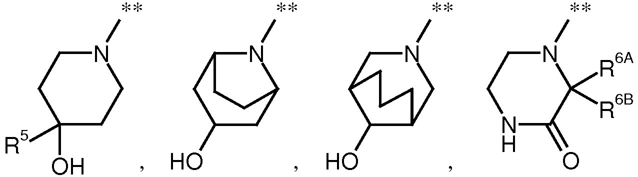 oder 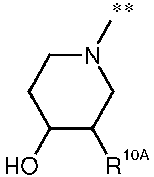 steht, worin ** die Verknüpfung zur Carbonylgruppe markiert,
R⁵ Wasserstoff, Methyl, Trifluormethyl, Hydroxymethyl oder Ethyl bedeutet,
R^{6A} und R^{6B} unabhängig voneinander Wasserstoff, Methyl oder Ethyl bedeuten und
R^{10A} Methyl oder Ethyl bedeutet,
sowie ihre Salze, Solvate und Solvate der Salze.

3. Verbindung der Formel (I) nach Anspruch 1 oder 2, in welcher
R¹ für Methyl, Difluormethyl, Trifluormethyl oder Ethyl steht,
R² für Methyl, Ethyl, Isopropyl oder Propargyl oder für eine Gruppe der Formel 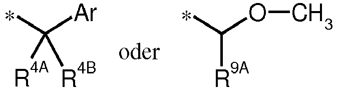 steht, worin * die Verknüpfung zur CH₂-Gruppe markiert,
Ar Phenyl, 3-Pyridyl oder 4-Pyridyl bedeutet,
wobei Phenyl in *meta-* oder *para*-Position mit Fluor oder in *ortho*-Position mit Fluor, Chlor oder Methyl substituiert sein kann,
R^{4A} Wasserstoff, Fluor oder Methyl bedeutet,
R^{4B} Wasserstoff, Fluor, Methyl, Hydroxy oder Methoxy bedeutet,
oder
R^{4A} und R^{4B} miteinander verknüpft sind und gemeinsam mit dem Kohlenstoffatom, an das sie gebunden sind, einen Cyclopropyl-Ring bilden,
und
R^{9A} Methyl oder Trifluormethyl bedeutet,
R³ für 2,2,2-Trifluorethyl, 3,3-Difluorprop-2-en-1-yl, Methoxymethyl, (Trifluormethoxy)methyl oder [(Trifluormethyl)sulfanyl]methyl steht,
und
der Ring A für einen Aza-Heterocyclus der Formel 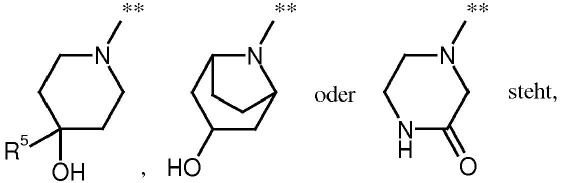 worin ** die Verknüpfung zur Carbonylgruppe markiert und
R⁵ Wasserstoff, Methyl, Trifluormethyl, Hydroxymethyl oder Ethyl bedeutet,
sowie ihre Salze, Solvate und Solvate der Salze.

4. Verbindung wie in Anspruch 1, wobei diese Verbindung folgende Struktur hat 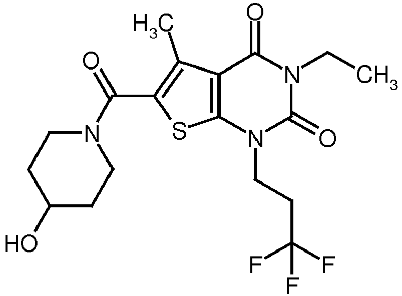 3-Ethyl-6-[(4-hydroxypiperidin-1-yl)carbonyl]-5-methyl-1-(3,3,3-trifluorpropyl)thieno[2,3-d]pyrimidin-2,4(1*H*,3*H*)-dion sowie deren Salze, Solvate und Solvate der Salze.

5. Verbindung wie in Anspruch 1, wobei diese Verbindung folgende Struktur hat 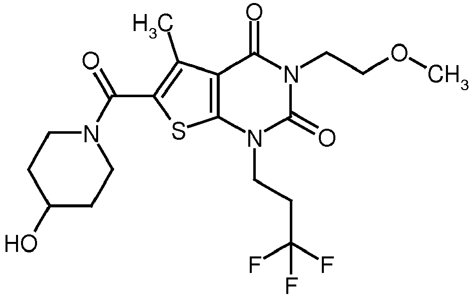 6-[(4-Hydroxypiperidin-1-yl)carbonyl]-3-(2-methoxyethyl)-5-methyl-1-(3,3,3-trifluorpropyl)thieno[2,3-d]pyrimidin-2,4(1*H*,3*H*)-dion sowie deren Salze, Solvate und Solvate der Salze.

6. Verbindung wie in Anspruch 1, wobei diese Verbindung folgende Struktur hat 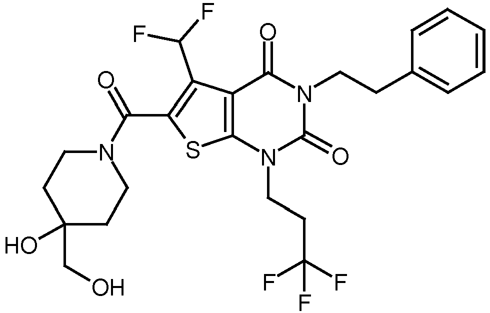 5-(Difluormethyl)-6-{[4-hydroxy-4-(hydroxymethyl)piperidin-1-yl]carbonyl}-3-(2-phenylethyl)-1-(3,3,3-trifluorpropyl)thieno[2,3-d]pyrimidin-2,4(1*H*,3*H*)-dion sowie deren Salze, Solvate und Solvate der Salze.

7. Verbindung wie in Anspruch 1, wobei diese Verbindung folgende Struktur hat 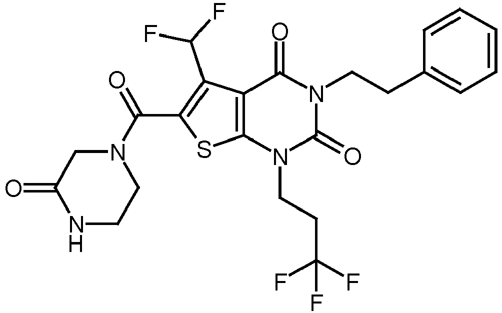 5-(Difluormethyl)-6-[(3-oxopiperazin-1-yl)carbonyl]-3-(2-phenylethyl)-1-(3,3,3-trifluorpropyl)thieno[2,3-d]pyrimidin-2,4(1*H*,3*H*)-dion sowie deren Salze, Solvate und Solvate der Salze.

8. Verbindung wie in Anspruch 1, wobei diese Verbindung folgende Struktur hat 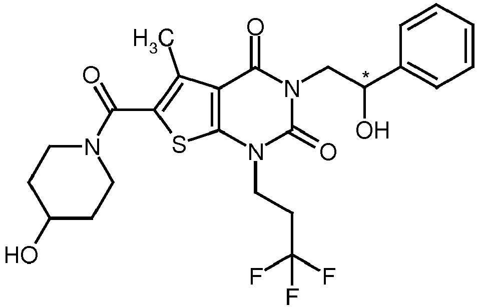 3-(2-Hydroxy-2-phenylethyl)-6-[(4-hydroxypiperidin-1-yl)carbonyl]-5-methyl-1-(3,3,3-trifluorpropyl)thieno[2,3-d]pyrimidin-2,4(1*H*,3*H*)-dion (*Enantiomer 1*) sowie deren Salze, Solvate und Solvate der Salze.

9. Verbindung wie in Anspruch 1, wobei diese Verbindung folgende Struktur hat 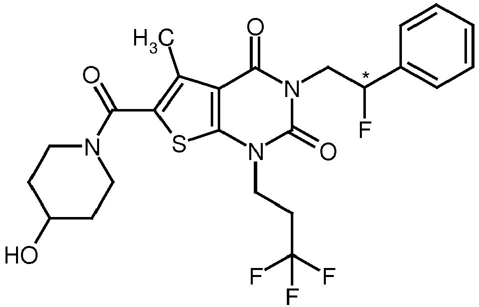 3-(2-Fluor-2-phenylethyl)-6-[(4-hydroxypiperidin-1-yl)carbonyl]-5-methyl-1-(3,3,3-trifluorpropyl)thieno[2,3-d]pyrimidin-2,4(1*H*,3*H*)-dion (*Enantiomer 1*) sowie deren Salze, Solvate und Solvate der Salze.

10. Verbindung wie in Anspruch 1, wobei diese Verbindung folgende Struktur hat 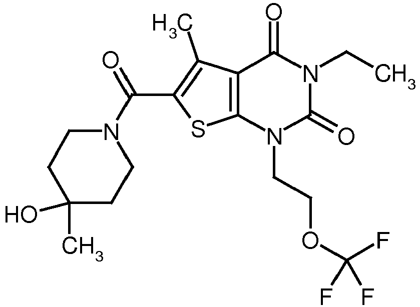 3-Ethyl-6-[(4-hydroxy-4-methylpiperidin-1-yl)carbonyl]-5-methyl-1-[2-(trifluormethoxy)ethyl]thieno[2,3-d]pyrimidin-2,4(1*H*,3*H*)-dion sowie deren Salze, Solvate und Solvate der Salze.

11. Verbindung wie in Anspruch 1, wobei diese Verbindung folgende Struktur hat 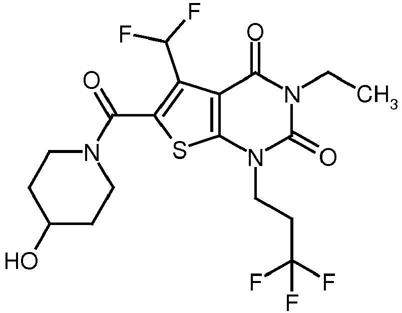 5-(Difluormethyl)-3-ethyl-6-[(4-hydroxypiperidin-1-yl)carbonyl]-1-(3,3,3-trifluorpropyl)-thieno[2,3-d]pyrimidin-2,4(1*H*,3*H*)-dion sowie deren Salze, Solvate und Solvate der Salze.

12. Verbindung wie in Anspruch 1, wobei diese Verbindung folgende Struktur hat 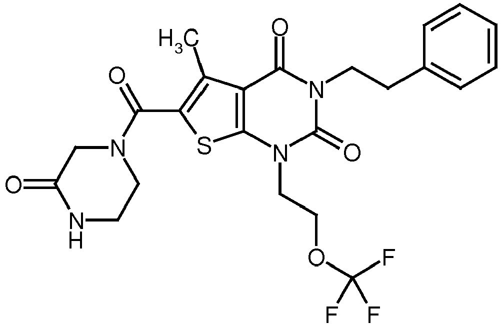 5-Methyl-6-[(3-oxopiperazin-1-yl)carbonyl] -3-(2-phenylethyl)-1-[2-(trifluormethoxy)ethyl]thieno[2,3-d]pyrimidin-2,4(1*H*,3*H*)-dion sowie deren Salze, Solvate und Solvate der Salze.

13. Verfahren zur Herstellung einer Verbindung der Formel (I), wie in den Ansprüchen 1 bis 12 definiert, **dadurch gekennzeichnet, dass** man entweder
[A-1] eine Verbindung der Formel (II) 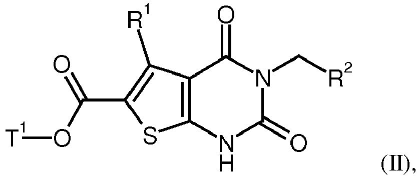 in welcher R¹ und R² die in den Ansprüchen 1 bis 3 angegebenen Bedeutungen haben
und
T¹ für (C₁-C₄)-Alkyl oder Benzyl steht,
in Gegenwart einer Base mit einer Verbindung der Formel (III) 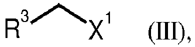 in welcher R³ die in den Ansprüchen 1 bis 3 angegebene Bedeutung hat
und
X¹ für eine Abgangsgruppe wie beispielsweise Chlor, Brom, Iod, Mesylat, Triflat oder Tosylat steht,
zu einer Verbindung der Formel (IV) 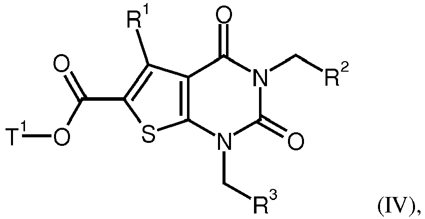 in welcher R¹, R², R³ und T¹ die oben angegebenen Bedeutungen haben,
alkyliert, anschließend den Ester-Rest T¹ abspaltet und die so erhaltene Carbonsäure der Formel (V) 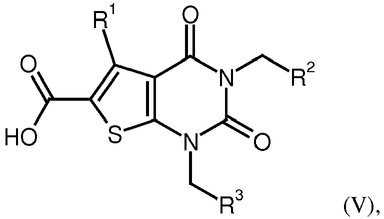 in welcher R¹, R² und R³ die oben angegebenen Bedeutungen haben,
dann unter Aktivierung der Carboxyl-Funktion mit einem Amin der Formel (VI) 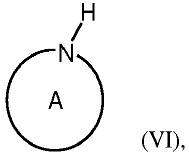 in welcher der Ring A die in den Ansprüchen 1 bis 3 angegebene Bedeutung hat, kuppelt
oder
[A-2] die Verbindung der Formel (II) 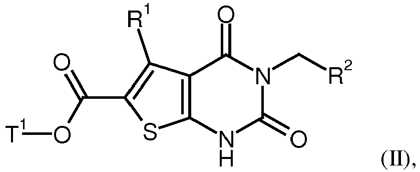 in welcher R¹, R² und T¹ die oben angegebenen Bedeutungen haben,
zunächst durch Abspaltung des Ester-Restes T¹ in die Carbonsäure der Formel (VII) 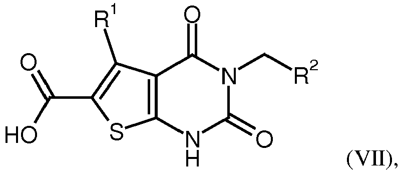 in welcher R¹ und R² die oben angegebenen Bedeutungen haben,
überführt, anschließend unter Aktivierung der Carboxyl-Funktion mit einem Amin der Formel (VI) 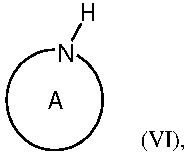 in welcher der Ring A die oben angegebene Bedeutung hat,
zu einer Verbindung der Formel (VIII) 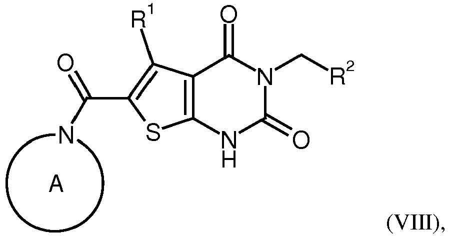 in welcher R¹, R² und der Ring A die oben angegebenen Bedeutungen haben,
kuppelt und diese dann in Gegenwart einer Base mit einer Verbindung der Formel (III) 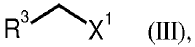 in welcher R³ und X¹ die oben angegebenen Bedeutungen haben, alkyliert
oder
[B] zunächst eine *N*³-geschützte Verbindung der Formel (IX) 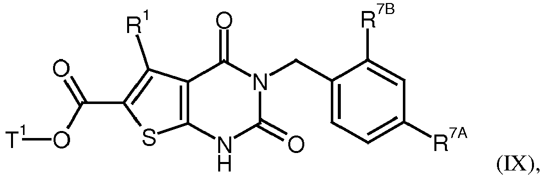 in welcher R¹ und T¹ die oben angegebenen Bedeutungen haben
und
R^{7A} und R^{7B} unabhängig voneinander für Wasserstoff oder Methoxy stehen,
in Gegenwart einer Base mit einer Verbindung der Formel (III) 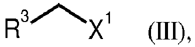 in welcher R³ und X¹ die oben angegebenen Bedeutungen haben,
zu einer Verbindung der Formel (X) 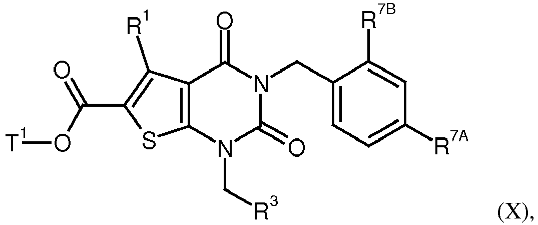 in welcher R¹, R³, R^{7A} , R^{7B} und T¹ die oben angegebenen Bedeutungen haben,
alkyliert, danach durch Behandlung mit einer starken Lewis-Säure wie Aluminiumtrichlorid simultan die *N³*-Benzylgruppe und den Ester-Rest T¹ abspaltet, die so erhaltene Carbonsäure der Formel (XI) 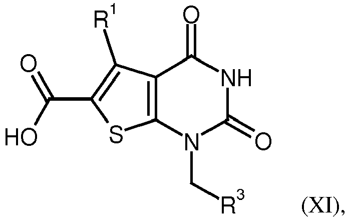 in welcher R¹ und R³ die oben angegebenen Bedeutungen haben,
anschließend unter Aktivierung der Carboxyl-Funktion mit einem Amin der Formel (VI) 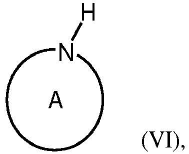 in welcher der Ring A die oben angegebene Bedeutung hat,
zu einer Verbindung der Formel (XII) 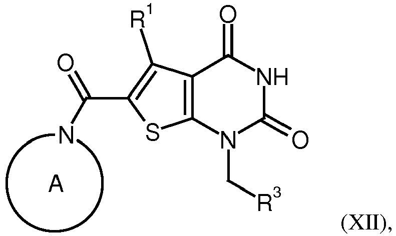 in welcher R¹, R³ und der Ring A die oben angegebenen Bedeutungen haben,
kuppelt und letztere dann entweder (*a*) in Gegenwart einer Base mit einer Verbindung der Formel (XIII) 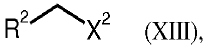 in welcher R² die oben angegebene Bedeutung hat
und
X² für eine Abgangsgruppe wie beispielsweise Chlor, Brom, Iod, Mesylat, Triflat oder Tosylat steht,
oder (b) in Gegenwart eines geeigneten Phosphins und eines Azodicarboxylats mit einer Verbindung der Formel (XIV) 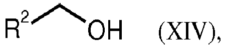 in welcher R² die oben angegebene Bedeutung hat,
umsetzt
und die so hergestellten Verbindungen der Formel (I) gegebenenfalls mit den entsprechenden (i) Lösungsmitteln und/oder (*ii*) Säuren in ihre Solvate, Salze und/oder Solvate der Salze überführt.

14. Verfahren zur Herstellung von Verbindungen der Formel (II-C) 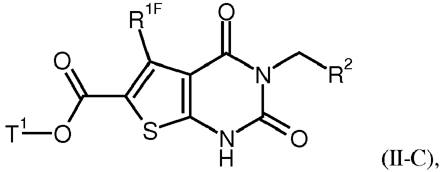 in welcher R² die in den Ansprüchen 1 bis 3 angegebene Bedeutung hat,
R^{1F} für Difluormethyl oder Trifluormethyl steht
und
T¹ für (C₁-C₄)-Alkyl oder Benzyl steht,
**dadurch gekennzeichnet, dass** man eine Verbindung der Formel (XIX) 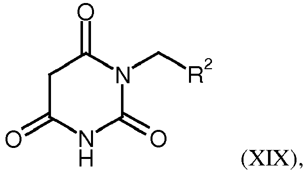 in welcher R² die oben angegebene Bedeutung hat,
mit Phosphoroxychlorid in eine Verbindung der Formel (XXIV) 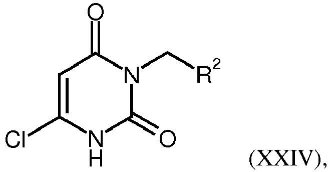 in welcher R² die oben angegebene Bedeutung hat,
überführt, dann in Gegenwart von überschüssigem Pyridin mit einem Anhydrid der Formel (XXV) 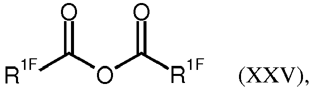 in welcher R^{1F} die oben angegebene Bedeutung hat, zu einer Betain-Verbindung der Formel (XXVI) 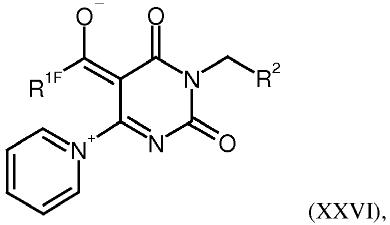 in welcher R^{1F} und R² die oben angegebenen Bedeutungen haben,
umsetzt und diese in Gegenwart einer Base mit einem α-Mercaptoessigsäureester der Formel (XXI) 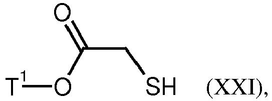 in welcher T¹ die oben angegebene Bedeutung hat,
zur Verbindung (II-C) kondensiert.

15. Verbindung, wie in einem der Ansprüche 1 bis 12 definiert, zur Verwendung in einem Verfahren zur Behandlung und/oder Prävention von Krankheiten.

16. Verbindung, wie in einem der Ansprüche 1 bis 12 definiert, zur Verwendung in einem Verfahren zur Behandlung und/oder Prävention von idiopathischer Lungenfibrose, pulmonaler Hypertonie, Bronchiolitis obliterans-Syndrom, chronisch-obstruktiver Lungenerkrankung, Asthma, zystischer Fibrose, Myokardinfarkt, Herzinsuffizienz und Sichelzellanämie.

17. Verwendung einer Verbindung, wie in einem der Ansprüche 1 bis 12 definiert, zur Herstellung eines Arzneimittels zur Behandlung und/oder Prävention von idiopathischer Lungenfibrose, pulmonaler Hypertonie, Bronchiolitis obliterans-Syndrom, chronisch-obstruktiver Lungenerkrankung, Asthma, zystischer Fibrose, Myokardinfarkt, Herzinsuffizienz und Sichelzellanämie.

18. Arzneimittel enthaltend eine Verbindung, wie in einem der Ansprüche 1 bis 12 definiert, in Kombination mit einem oder mehreren inerten, nicht-toxischen, pharmazeutisch geeigneten Hilfsstoffen.

19. Arzneimittel enthaltend eine Verbindung, wie in einem der Ansprüche 1 bis 12 definiert, in Kombination mit einem oder mehreren weiteren Wirkstoffen ausgewählt aus der Gruppe der PDE 5-Inhibitoren, sGC-Aktivatoren, sGC-Stimulatoren, Prostacyclin-Analoga, Endothelin-Antagonisten, der antifibrotisch wirkenden Mittel, der entzündungshemmend, immunmodulierend, immunsuppressiv und/oder zytotoxisch wirkenden Mittel und/oder der die Signaltransduktionskaskade inhibierenden Verbindungen.

20. Arzneimittel nach Anspruch 18 oder 19 zur Verwendung in einem Verfahren zur Behandlung und/oder Prävention von idiopathischer Lungenfibrose, pulmonaler Hypertonie, Bronchiolitis obliterans-Syndrom, chronisch-obstruktiver Lungenerkrankung, Asthma, zystischer Fibrose, Myokardinfarkt, Herzinsuffizienz und Sichelzellanämie.

## Claims

1. Compound of the formula (I) 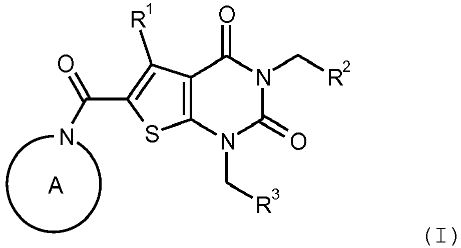 in which
R¹ represents hydrogen, methyl or ethyl, where methyl and ethyl may be substituted up to three times by fluorine,
R² represents methyl, ethyl, *n*-propyl, isopropyl, cyclopropyl, cyclopropylmethyl, 2,2-difluorovinyl, 3,3-difluoroallyl or propargyl or represents a group of the formula 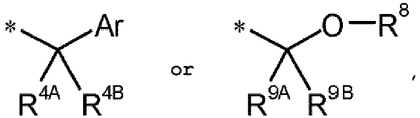 in which * denotes the point of attachment to the CH₂ group,
Ar represents phenyl or 5- or 6-membered heteroaryl having up to two ring nitrogen atoms,
where phenyl and heteroaryl may be mono- or disubstituted by identical or different radicals selected from the group consisting of fluorine, chlorine, methyl, trifluoromethyl, methoxy and trifluoromethoxy,
R^{4A} represents hydrogen, fluorine or methyl,
R^{4B} represents hydrogen, fluorine, methyl, trifluoromethyl, hydroxy or methoxy,
or
R^{4A} and R^{4B} are attached to one another and together with the carbon atom to which they are attached form a cyclopropyl or cyclobutyl ring,
R⁸ represents methyl or trifluoromethyl
and
R^{9A} and R^{9B} independently of one another represent hydrogen, methyl or trifluoromethyl,
R³ represents (C₁-C₆)-alkyl, (C₂-C₆)-alkenyl, (C₃-C₇)-cycloalkyl or [(C₃-C₇-cycloalkyl]methyl,
where alkyl and alkenyl may be substituted up to three times and cycloalkyl may be substituted up to two times by fluorine
and
where in alkyl and cycloalkyl up to two CH₂ groups may be replaced by -O- or -S-, with the proviso that there are at least two carbon atoms between such heteroatoms including the uracil N¹-atom,
and
the ring A represents a mono- or bicyclic aza heterocycle of the formula 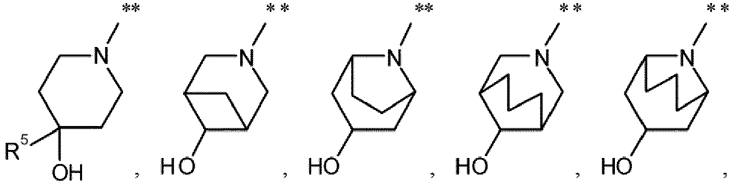 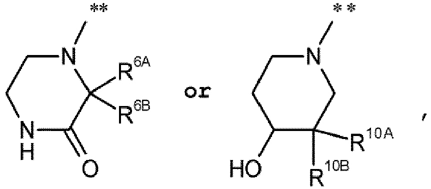 in which ** denotes the point of attachment to the carbonyl group,
R⁵ represents hydrogen, methyl, trifluoromethyl, hydroxymethyl or ethyl,
R^{6A} and R^{6B} each independently of one another represent hydrogen, methyl or ethyl,
R^{10A} represents methyl, ethyl, hydroxy or methoxy,
and
R^{10B} represents hydrogen, methyl or ethyl,
and their salts, solvates and solvates of the salts.

2. Compound of the formula (I) according to Claim 1 in which
R¹ represents methyl, difluoromethyl, trifluoromethyl, ethyl or 1-fluoroethyl,
R² represents methyl, ethyl, *n*-propyl, isopropyl, 3,3-difluoroallyl or propargyl or represents a group of the formula 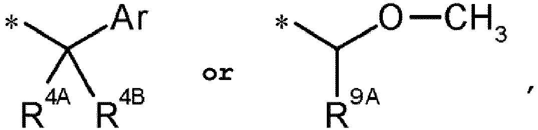
in which * denotes the point of attachment to the CH₂ group,
Ar represents phenyl or pyridyl,
where phenyl and pyridyl may be substituted by fluorine, chlorine, methyl or methoxy,
R^{4A} represents hydrogen, fluorine or methyl,
R^{4B} represents hydrogen, fluorine, methyl, hydroxy or methoxy,
or
R^{4A} and R^{4B} are attached to one another and together with the carbon atom to which they are attached form a cyclopropyl ring,
and
R^{9A} represents hydrogen, methyl or trifluoromethyl,
R³ represents (C₂-C₄) -alkyl, (C₂-C₄) -alkenyl, (C₃-C₆)-cycloalkyl or [(C₃-C₆)-cycloalkyl]methyl,
where alkyl and alkenyl may be substituted up to three times and cycloalkyl may be substituted up to two times by fluorine
and
where in alkyl and cycloalkyl one CH₂ group may be replaced by -O- or -S-, with the proviso that there are at least two carbon atoms between such a heteroatom and the uracil N¹-atom,
and
the ring A represents an aza heterocycle of the formula 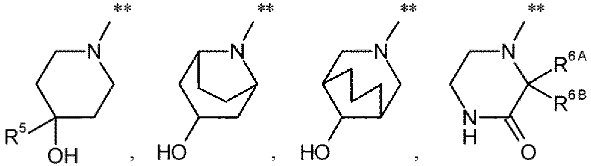 or 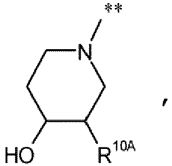
in which ** denotes the point of attachment to the carbonyl group,
R⁵ represents hydrogen, methyl, trifluoromethyl, hydroxymethyl or ethyl,
R^{6A} and R^{6B} independently of one another represent hydrogen, methyl or ethyl,
and
R^{10A} represents methyl or ethyl,
and their salts, solvates and solvates of the salts.

3. Compound of the formula (I) according to Claim 1 or 2 in which
R¹ represents methyl, difluoromethyl, trifluoromethyl or ethyl,
R² represents methyl, ethyl, isopropyl or propargyl or represents a group of the formula 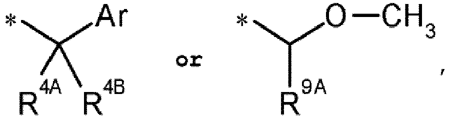
in which * denotes the point of attachment to the CH₂ group,
Ar represents phenyl, 3-pyridyl or 4-pyridyl,
where phenyl may be substituted in the *meta-* or *para*-position by fluorine or in the *ortho*-position by fluorine, chlorine or methyl,
R^{4A} represents hydrogen, fluorine or methyl,
R^{4B} represents hydrogen, fluorine, methyl, hydroxy or methoxy,
or
R^{4A} and R^{4B} are attached to one another and together with the carbon atom to which they are attached form a cyclopropyl ring,
and
R^{9A} represents methyl or trifluoromethyl,
R³ represents 2,2,2-trifluoroethyl, 3,3-difluoroprop-2-en-1-yl, methoxymethyl, (trifluoromethoxy)methyl or [(trifluoromethyl)sulphanyl]methyl,
and
the ring A represents an aza heterocycle of the formula 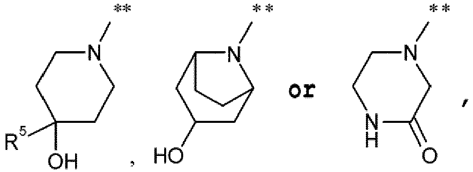
in which ** denotes the point of attachment to the carbonyl group,
and
R⁵ represents hydrogen, methyl, trifluoromethyl, hydroxymethyl or ethyl, and their salts, solvates and solvates of the salts.

4. Compound according to Claim 1, where this compound has the following structure: 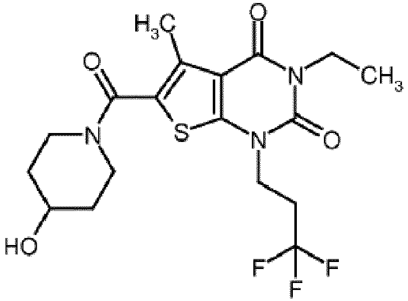 3-ethyl-6-[(4-hydroxypiperidin-1-yl)carbonyl]-5-methyl-1-(3,3,3-trifluoropropyl)thieno[2,3-d]pyrimidine-2,4(1*H*, 3*H*)-dione, and its salts, solvates and solvates of the salts.

5. Compound according to Claim 1, where this compound has the following structure: 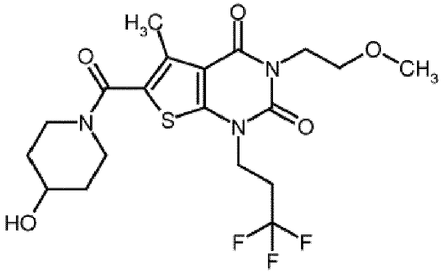 6-[(4-hydroxypiperidin-1-yl)carbonyl-3-(2-methoxyethyl)-5-methyl-1-(3,3,3-trifluoropropyl)thieno[2,3-d]pyrimidine-2,4-(1*H*, 3*H*)-dione, and its salts, solvates and solvates of the salts.

6. Compound according to Claim 1, where this compound has the following structure: 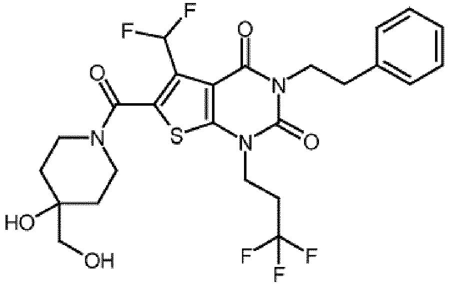 5-(difluoromethyl)-6-{[4-hydroxy-4-(hydroxymethyl)piperidin-1-yl]carbonyl}-3-(2-phenylethyl)-1-(3,3,3-trifluoropropyl)thieno[2,3-d]pyrimidine-2,4(1*H*, 3*H*)-dione, and its salts, solvates and solvates of the salts.

7. Compound according to Claim 1, where this compound has the following structure: 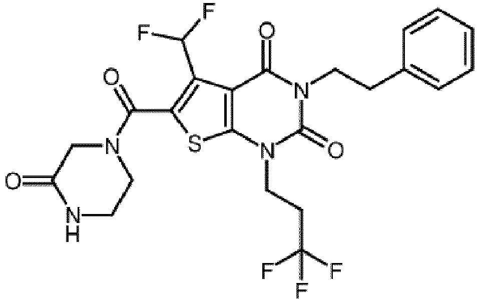 5-(difluoromethyl)-6-[(3-oxopiperazin-1-yl)carbonyl]-3-(2-phenylethyl)-1-(3,3,3-trifluoropropyl)thieno[2,3-d]pyrimidine-2,4(1*H*, 3*H*)-dione, and its salts, solvates and solvates of the salts.

8. Compound according to Claim 1, where this compound has the following structure: 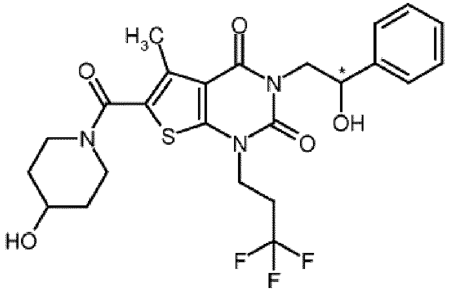 3-(2-hydroxy-2-phenylethyl)-6-[(4-hydroxypiperidin-1-yl)carbonyl]-5-methyl-1-(3,3,3-trifluoropropyl)thieno[2,3-d]pyrimidine-2,4(1*H*, 3*H*)-dione (*enantiomer 1*)*,* and its salts, solvates and solvates of the salts.

9. Compound according to Claim 1, where this compound has the following structure: 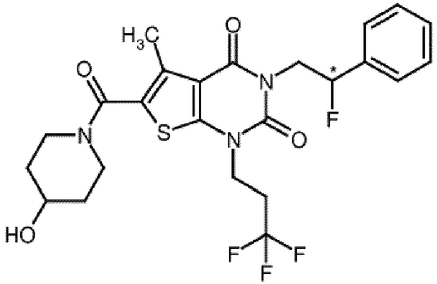 3-(2-fluoro-2-phenylethyl)-6-[(4-hydroxypiperidin-1-yl)carbonyl]-5-methyl-1-(3,3,3-trifluoropropyl)thieno[2,3-d]pyrimidine-2,4(1*H*, 3*H*)-dione (*enantiomer 1*), and its salts, solvates and solvates of the salts.

10. Compound according to Claim 1, where this compound has the following structure: 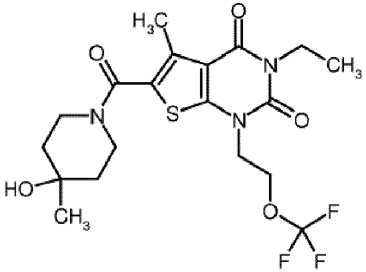 3-ethyl-6-[4-hydroxy-4-methylpiperidin-1-yl)carbonyl]-5-methyl-1-[2-(trifluormethoxy)ethyl]thieno[2,3-d]pyrimidine-2,4(1*H*, 3*H*)-dione, and its salts, solvates and solvates of the salts.

11. Compound according to Claim 1, where this compound has the following structure: 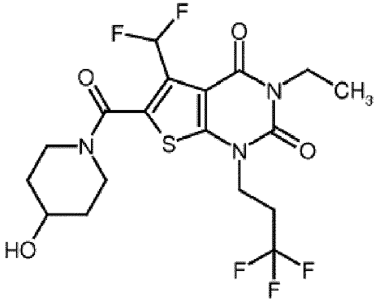 5-(difluoromethyl)-3-ethyl-6-[(4-hydroxypiperidin-1-yl)carbonyl]-1-(3,3,3-trifluoropropyl)-thieno[2,3-d]pyrimidine-2,4(1*H*, 3*H*)-dione, and its salts, solvates and solvates of the salts.

12. Compound according to Claim 1, where this compound has the following structure: 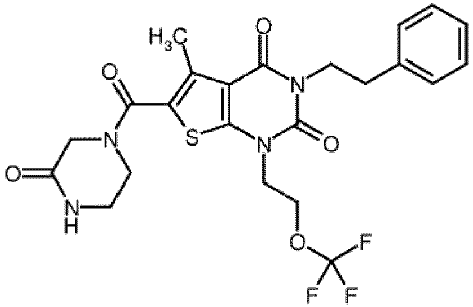 5-methyl-6-[3-oxopiperazin-1-yl)carbonyl]-3-(2-phenylethyl)-1-[2-(trifluoromethoxy)ethyl]thieno[2,3-d]pyrimidine-2,4(1*H*, 3*H*)-dione, and its salts, solvates and solvates of the salts.

13. Process for preparing a compound of the formula (I) as defined in any of Claims 1 to 12, **characterized in that** either
[A-1]a compound of the formula (II) 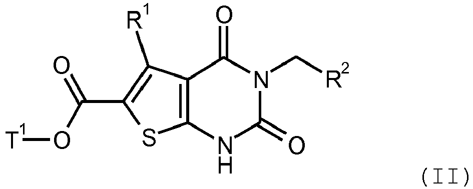 in which R¹ and R² have the meanings given in any of Claims 1 to 3
and
T¹ represents (C₁-C₄)-alkyl or benzyl is alkylated in the presence of a base with a compound of the formula (III) 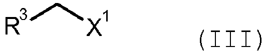 in which R³ has the meaning given in any of Claims 1 to 3
and
X¹ represents a leaving group, for example chlorine, bromine, iodine, mesylate, triflate or tosylate,
to give a compound of the formula (IV) 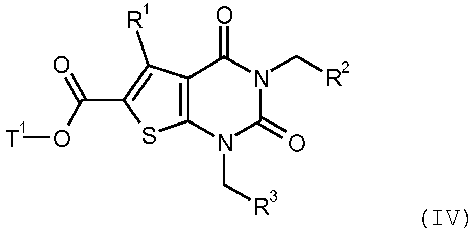 in which R¹, R², R³ and T¹ have the meanings given above,
then the ester radical T¹ is cleaved off and the carboxylic acid of the formula (V) obtained in this manner 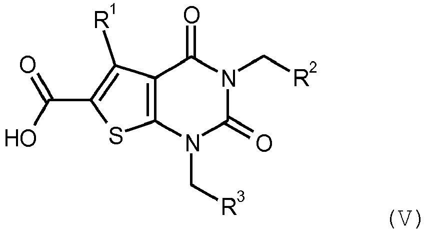 in which R¹, R² and R³ have the meanings given above
is then coupled with activation of the carboxyl function with an amine of the formula (VI) 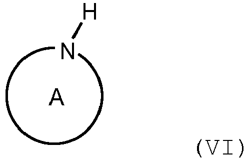 in which the ring A has the meaning given in any of Claims 1 to 3,
or
[A-2]the compound of the formula (II) 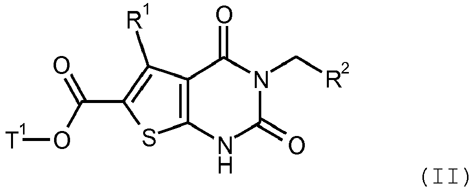 in which R¹, R² and T¹ have the meanings given above
is initially converted by cleavage of the ester radical T¹ into the carboxylic acid of the formula (VII) 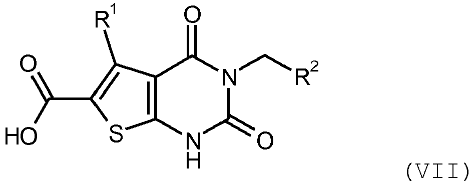 in which R¹ and R² have the meanings given above,
then coupled with activation of the carboxyl function with an amine of the formula (VI) 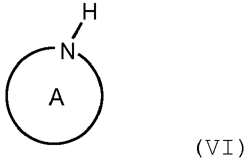 in which the ring A has the meaning given above
to give a compound of the formula (VIII) 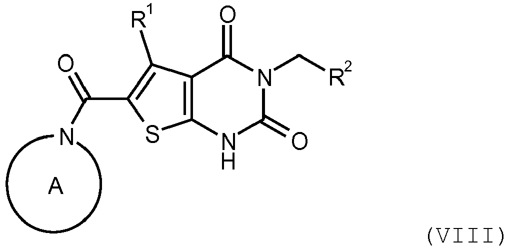 in which R¹, R² and the ring A have the meanings given above
and this is then alkylated in the presence of a base with a compound of the formula (III) 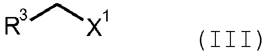 in which R³ and X¹ have the meanings given above
or
[B] initially an *N³* protected compound of the formula (IX) 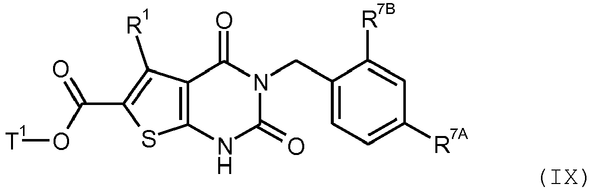 in which R¹ and T¹ have the meanings given above
and
R^{7A} and R^{7B} independently of one another represent hydrogen or methoxy
is alkylated in the presence of a base with a compound of the formula (III) 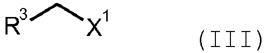 in which R³ and X¹ have the meanings given above
to give a compound of the formula (X) 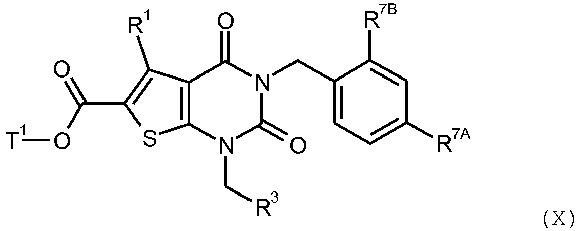 in which R¹, R³, R^{7A}, R^{7B} and T¹ have the meanings given above,
the *N³*-benzyl group and the ester radical T¹ are then cleaved off simultaneously by treatment with a strong Lewis acid such as aluminium trichloride, the carboxylic acid of the formula (XI) obtained in this manner 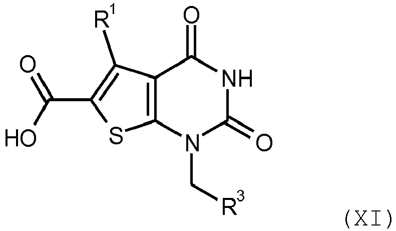 in which R¹ and R³ have the meanings given above,
is then coupled with activation of the carboxyl function with an amine of the formula (VI) 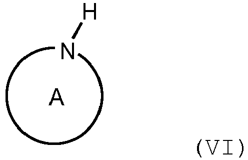 in which the ring A has the meaning given above
to give a compound of the formula (XII) 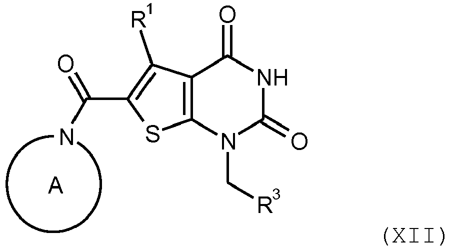 in which R¹, R³ and the ring A have the meanings given above
and the latter is then either reacted (*a*) in the presence of a base with a compound of the formula (XIII) 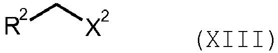 in which R² has the meaning given above
and
X² represents a leaving group, for example chlorine, bromine, iodine, mesylate, triflate or tosylate,
or (*b*) in the presence of a suitable phosphine and an azodicarboxylate with a compound of the formula (XIV) 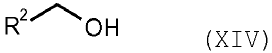 in which R² has the meaning given above, and the compounds of the formula (I) prepared in this manner are optionally converted with the appropriate (*i*) solvents and/or (*ii*) acids into their solvates, salts and/or solvates of the salts.

14. Process for preparing compounds of the formula (II-C) 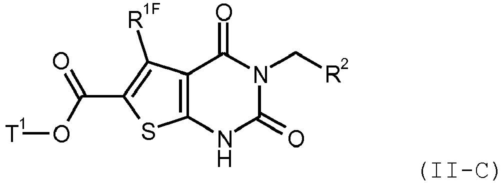 in which R² has the meaning given in any of Claims 1 to 3
R^{1F} represents difluoromethyl or trifluoromethyl
and
T¹ represents (C₁-C₄)-alkyl or benzyl,
**characterized in that** a compound of the formula (XIX) 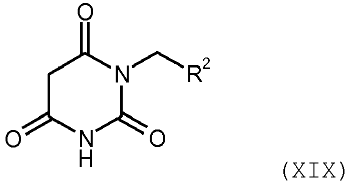 in which R² has the meaning given above,
is converted with phosphorus oxychloride into a compound of the formula (XXIV) 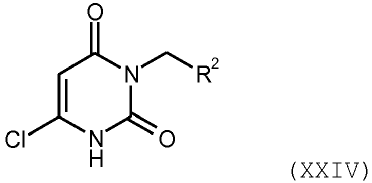 in which R² has the meaning given above,
then in the presence of excess pyridine is reacted with an anhydride of the formula (XXV) 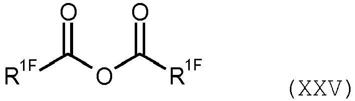 in which R^{1F} has the meaning given above
to give a betaine compound of the formula (XXVI) 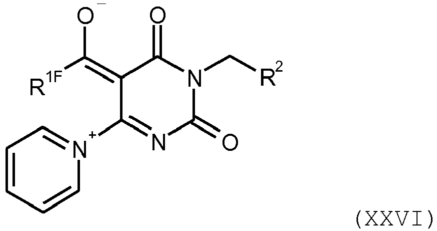 in which R^{1F} and R² have the meanings given above
and these are then condensed in the presence of a base with an α-mercaptoacetic ester of the formula (XXI) 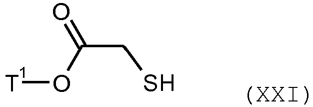 in which T¹ has the meaning given above
to give the compound (II-C).

15. Compound as defined in any of Claims 1 to 12 for use in a method for the treatment and/or prevention of diseases.

16. Compound as defined in any of Claims 1 to 12 for use in a method for the treatment and/or prevention of idiopathic pulmonary fibrosis, pulmonary hypertension, Bronchiolitis obliterans syndrome, chronic-obstructive pulmonary disease, asthma, cystic fibrosis, myocardial infarction, heart failure and sickle cell anaemia.

17. Use of a compound as defined in any of Claims 1 to 12 for preparing a medicament for the treatment and/or prevention of idiopathic pulmonary fibrosis, pulmonary hypertension, Bronchiolitis obliterans syndrome, chronic-obstructive pulmonary disease, asthma, cystic fibrosis, myocardial infarction, heart failure and sickle cell anaemia.

18. Medicament comprising a compound as defined in any of Claims 1 to 12 in combination with one or more inert, nontoxic, pharmaceutically suitable excipients.

19. Medicament comprising a compound as defined in any of Claims 1 to 12 in combination with one or more further active compounds selected from the group of the PDE 5 inhibitors, sGC activators, sGC stimulators, prostacyclin analogs, endothelin antagonists, of the antifibrotic agents, the antiinflammatory, immunomodulating, immunosuppressive and/or cytotoxic agents and/or the signal transduction cascade-inhibiting compounds.

20. Medicament according to Claim 18 or 19 for use in a method for the treatment and/or prevention of idiopathic pulmonary fibrosis, pulmonary hypertension, Bronchiolitis obliterans syndrome, chronic-obstructive pulmonary disease, asthma, cystic fibrosis, myocardial infarction, heart failure and sickle cell anaemia.

## Revendications

1. Composé de formule (I) 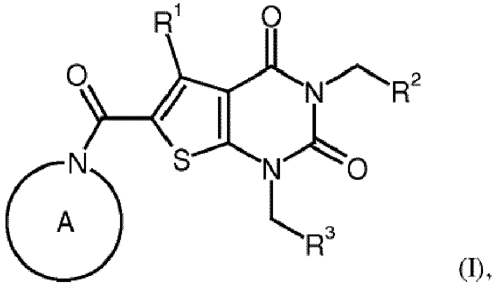 dans lequel
R¹ représente hydrogène, méthyle ou éthyle, le méthyle et l'éthyle pouvant être substitués jusqu'à trois fois avec fluor,
R² représente méthyle, éthyle, n-propyle, isopropyle, cyclopropyle, cyclopropylméthyle, 2,2-difluorovinyle, 3,3-difluoroallyle ou propargyle ou un groupe de formule 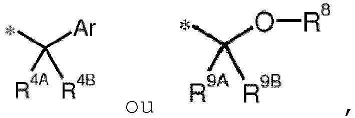
dans lesquelles * marque la liaison au groupe CH₂,
Ar signifie phényle ou hétéroaryle à 5 ou 6 chaînons contenant jusqu'à deux atomes d'azote de cycle,
le phényle et l'hétéroaryle pouvant être substitués une ou deux fois, de manière identique ou différente, avec un radical choisi dans la série constituée par fluor, chlore, méthyle, trifluorométhyle, méthoxy et trifluorométhoxy, R^{4A} signifie hydrogène, fluor ou méthyle,
R^{4B} signifie hydrogène, fluor, méthyle, trifluorométhyle, hydroxy ou méthoxy,
ou
R^{4A} et R^{4B} sont reliés l'un avec l'autre et forment ensemble avec l'atome de carbone auquel ils sont reliés un cycle cyclopropyle ou cyclobutyle,
R⁸ signifie méthyle ou trifluorométhyle,
et
R^{9A} et R^{9B} signifient indépendamment l'un de l'autre hydrogène, méthyle ou trifluorométhyle,
R³ représente alkyle en (C₁-C₆), alcényle en (C₂-C₆) , cycloalkyle en (C₃-C₇) ou [cycloalkyle en (C₃-C₇) ] méthyle,
l'alkyle et l'alcényle pouvant être substitués jusqu'à trois fois et le cycloalkyle jusqu'à deux fois avec fluor,
et
dans l'alkyle et le cycloalkyle, jusqu'à deux groupes CH₂ pouvant être remplacés par -O- ou -S-, à condition qu'au moins deux atomes de carbone se trouvent entre de tels hétéroatomes, y compris l'atome N¹ de l'uracile,
et
le cycle A représente un hétérocycle aza mono- ou bicyclique de formule 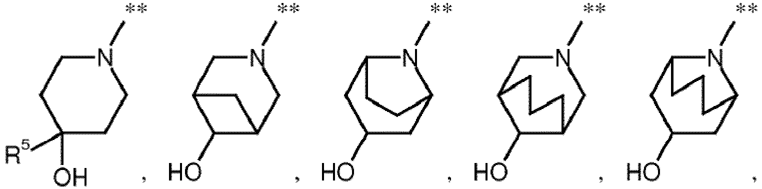 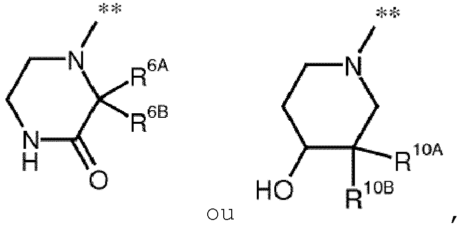
dans lesquelles ** marque la liaison au groupe carbonyle,
R⁵ signifie hydrogène, méthyle, trifluorométhyle, hydroxyméthyle ou éthyle,
R^{6A} et R^{6B} signifient indépendamment l'un de l'autre hydrogène, méthyle ou éthyle,
R^{10A} signifie méthyle, éthyle, hydroxy ou méthoxy, et
R^{10B} signifie hydrogène, méthyle ou éthyle,
ainsi que ses sels, solvates et solvates des sels.

2. Composé de formule (I) selon la revendication 1, dans lequel
R¹ représente méthyle, difluorométhyle, trifluorométhyle, éthyle ou 1-fluoroéthyle,
R² représente méthyle, éthyle, n-propyle, isopropyle, 3,3-difluoroallyle ou propargyle ou un groupe de formule 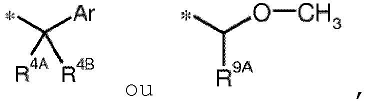
dans lesquelles * marque la liaison au groupe CH₂,
Ar signifie phényle ou pyridyle,
le phényle et le pyridyle pouvant être substitués avec fluor, chlore, méthyle ou méthoxy, R^{4A} signifie hydrogène, fluor ou méthyle,
R^{4B} signifie hydrogène, fluor, méthyle, hydroxy ou méthoxy,
ou
R^{4A} et R^{4B} sont reliés l'un avec l'autre et forment ensemble avec l'atome de carbone auquel ils sont reliés un cycle cyclopropyle,
et
R^{9A} signifie hydrogène, méthyle ou trifluorométhyle,
R³ représente alkyle en (C₂-C₄), alcényle en (C₂-C₄) , cycloalkyle en (C₃-C₆) ou [cycloalkyle en (C₃-C₆)]méthyle,
l'alkyle et l'alcényle pouvant être substitués jusqu'à trois fois et le cycloalkyle jusqu'à deux fois avec fluor,
et
dans l'alkyle et le cycloalkyle, un groupe CH₂ pouvant être remplacé par -O- ou -S-, à condition qu'au moins deux atomes de carbone se trouvent entre un tel hétéroatome et l'atome N¹ de l'uracile,
et
le cycle A représente un hétérocycle aza de formule 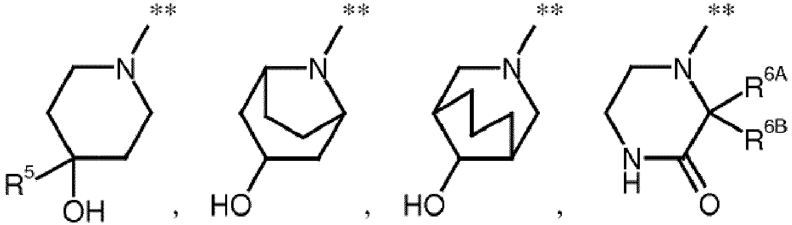 ou 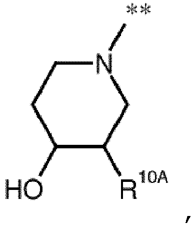
dans lesquelles ** marque la liaison au groupe carbonyle,
R⁵ signifie hydrogène, méthyle, trifluorométhyle, hydroxyméthyle ou éthyle,
R^{6A} et R^{6B} signifient indépendamment l'un de l'autre hydrogène, méthyle ou éthyle,
et
R^{10A} signifie méthyle ou éthyle,
ainsi que ses sels, solvates et solvates des sels.

3. Composé de formule (I) selon la revendication 1 ou 2, dans lequel
R¹ représente méthyle, difluorométhyle, trifluorométhyle ou éthyle,
R² représente méthyle, éthyle, isopropyle ou propargyle ou un groupe de formule 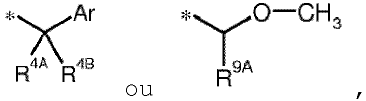
dans lesquelles * marque la liaison au groupe CH₂,
Ar signifie phényle, 3-pyridyle ou 4-pyridyle,
le phényle pouvant être substitué en positon *méta* ou para avec fluor ou en position *ortho* avec fluor, chlore ou méthyle,
R^{4A} signifie hydrogène, fluor ou méthyle,
R^{4B} signifie hydrogène, fluor, méthyle, hydroxy ou méthoxy,
ou
R^{4A} et R^{4B} sont reliés l'un avec l'autre et forment ensemble avec l'atome de carbone auquel ils sont reliés un cycle cyclopropyle,
et
R^{9A} signifie méthyle ou trifluorométhyle,
R³ représente 2,2,2-trifluoroéthyle, 3,3-difluoropro-2-én-1-yle, méthoxyméthyle, (trifluorométhoxy)méthyle ou [(trifluorométhyl)sulfanyl]méthyle,
et
le cycle A représente un hétérocycle aza de formule 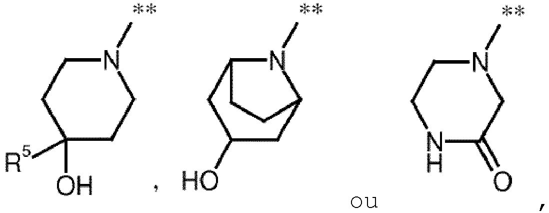
dans lesquelles ** marque la liaison au groupe carbonyle,
et
R⁵ signifie hydrogène, méthyle, trifluorométhyle, hydroxyméthyle ou éthyle,
ainsi que ses sels, solvates et solvates des sels.

4. Composé tel que dans la revendication 1, ce composé ayant la structure suivante : 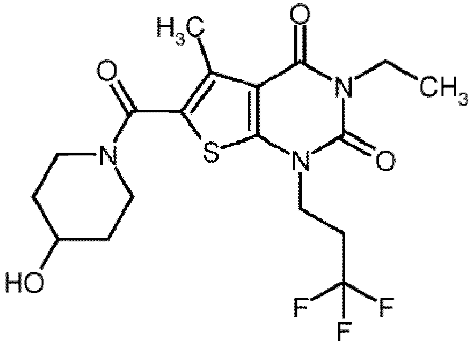 3-éthyl-6-[(4-hydroxypipéridin-1-yl)carbonyl]-5-méthyl-1-(3,3,3-trifluoropropyl)thiéno[2,3-d]pyrimidine-2,4(1H, 3H)-dione, ainsi que ses sels, solvates et solvates des sels.

5. Composé tel que dans la revendication 1, ce composé ayant la structure suivante : 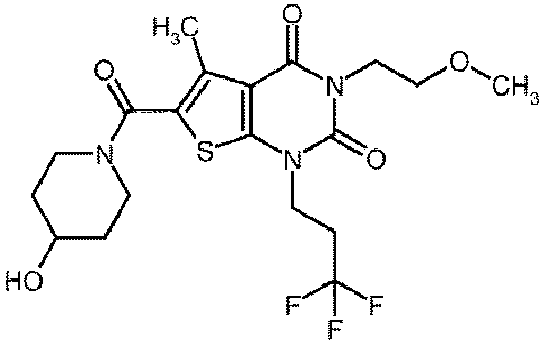 6-[(4-hydroxypipéridin-1-yl)carbonyl]-3-(2-méthoxyéthyl)-5-méthyl-1-(3,3,3-trifluoropropyl)thiéno[2,3-d]pyrimidine-2,4(1H,3H)-dione, ainsi que ses sels, solvates et solvates des sels.

6. Composé tel que dans la revendication 1, ce composé ayant la structure suivante : 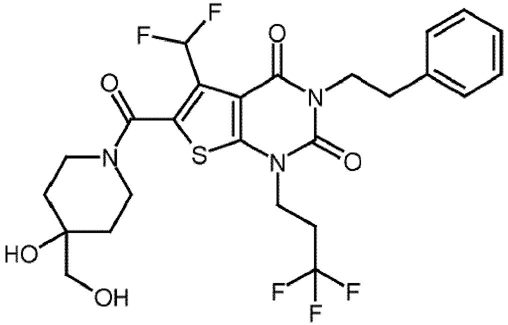 5-(difluorométhyl)-6-{[4-hydroxy-4-(hydroxyméthyl)pipéridin-1-yl]carbonyl}-3-(2-phényléthyl)-1-(3,3,3-trifluoropropyl)thiéno[2,3-d]pyrimidine-2,4(1H,3H)-dione, ainsi que ses sels, solvates et solvates des sels.

7. Composé tel que dans la revendication 1, ce composé ayant la structure suivante : 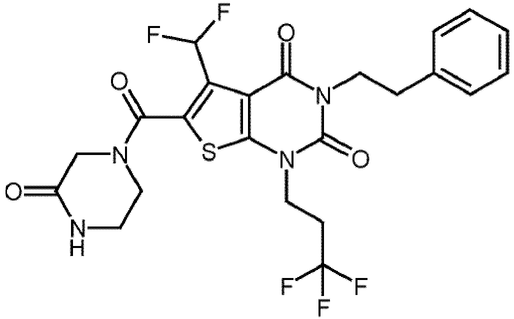 5-(difluorométhyl)-6-[(3-oxopipérazin-1-yl)carbonyl]-3-(2-phényléthyl)-1-(3,3,3-trifluoropropyl)thiéno[2,3-d]pyrimidine-2,4(1H,3H)-dione, ainsi que ses sels, solvates et solvates des sels.

8. Composé tel que dans la revendication 1, ce composé ayant la structure suivante : 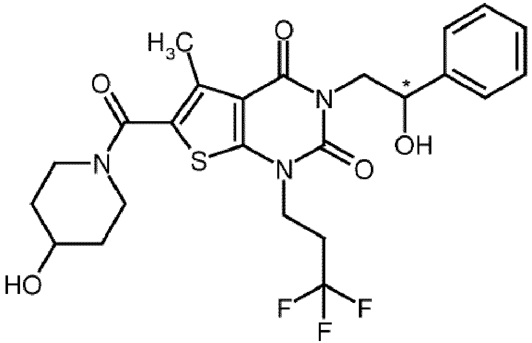 3-(2-hydroxy-2-phényléthyl)-6-[(4-hydroxypipéridin-1-yl)carbonyl]-5-méthyl-1-(3,3,3-trifluoropropyl)thiéno[2,3-d]pyrimidine-2,4(1H,3H)-dione (énantiomère 1), ainsi que ses sels, solvates et solvates des sels.

9. Composé tel que dans la revendication 1, ce composé ayant la structure suivante : 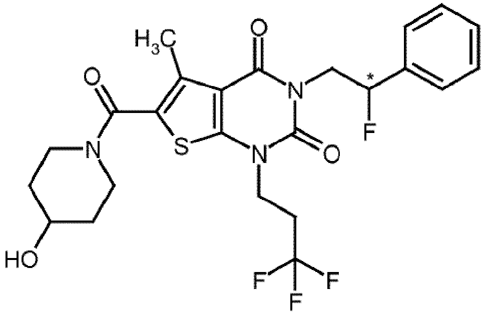 3-(2-fluoro-2-phényléthyl)-6-[(4-hydroxypipéridin-1-yl)carbonyl]-5-méthyl-1-(3,3,3-trifluoropropyl)thiéno[2,3-d]pyrimidine-2,4(1H,3H)-dione (énantiomère 1), ainsi que ses sels, solvates et solvates des sels.

10. Composé tel que dans la revendication 1, ce composé ayant la structure suivante : 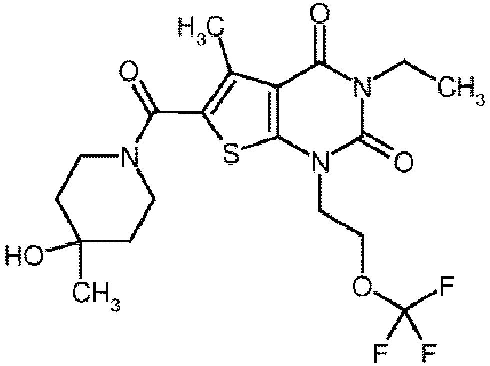 3-éthyl-6-[(4-hydroxy-4-méthylpipéridin-1-yl)carbonyl]-5-méthyl-1-[2-(trifluorométhoxy)éthyl]thiéno[2,3-d]pyrimidine-2,4(1H,3H)-dione, ainsi que ses sels, solvates et solvates des sels.

11. Composé tel que dans la revendication 1, ce composé ayant la structure suivante : 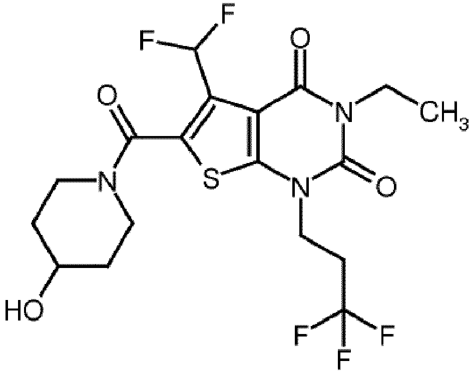 5-(difluorométhyl)-3-éthyl-6-[(4-hydroxypipéridin-1-yl)carbonyl]-1-(3,3,3-trifluoropropyl)-thiéno[2,3-d]pyrimidine-2,4(1H, 3H)-dione, ainsi que ses sels, solvates et solvates des sels.

12. Composé tel que dans la revendication 1, ce composé ayant la structure suivante : 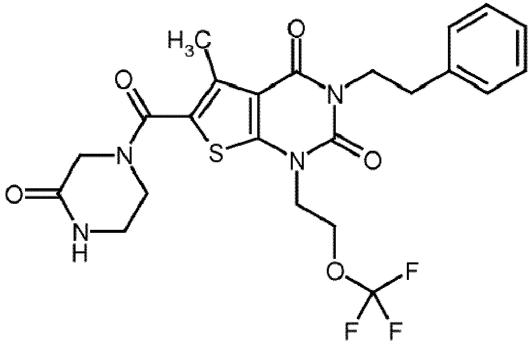 5-méthyl-6-[(3-oxopipérazin-1-yl)carbonyl]-3-(2-phényléthyl)-1-[2-(trifluorométhoxy)éthyl]thiéno[2,3-d]pyrimidin-2,4(1H,3H)-dione, ainsi que ses sels, solvates et solvates des sels.

13. Procédé de fabrication d'un composé de formule (I), tel que défini dans les revendications 1 à 12, **caractérisé en ce que** soit
[A-1] un composé de formule (II) 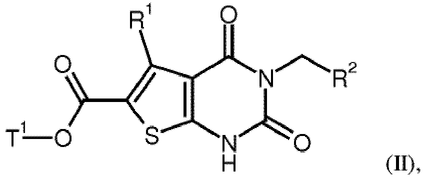
dans laquelle R¹ et R² ont les significations indiquées dans les revendications 1 à 3,
et
T¹ représente alkyle en (C₁-C₄) ou benzyle,
est alkylé en présence d'une base avec un composé de formule (III) 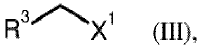
dans laquelle R³ a la signification indiquée dans les revendications 1 à 3,
et
X¹ représente un groupe partant, tel que par exemple chlore, brome, iode, mésylate, triflate ou tosylate,
pour former un composé de formule (IV) 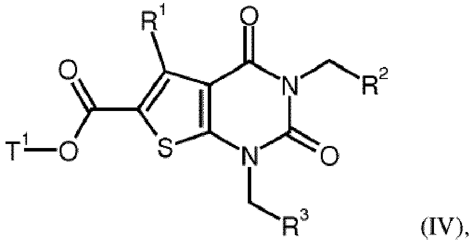
dans laquelle R¹, R², R³ et T¹ ont les significations indiquées précédemment,
puis le radical ester T¹ est clivé et l'acide carboxylique de formule (V) ainsi obtenu 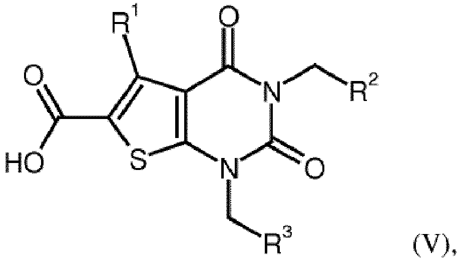
dans laquelle R¹, R² et R³ ont les significations indiquées précédemment,
est ensuite couplé avec activation de la fonction carboxyle avec une amine de formule (VI) 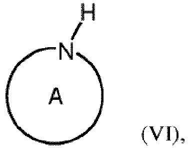
dans laquelle le cycle A a la signification indiquée dans les revendications 1 à 3,
soit
[A-2] le composé de formule (II) 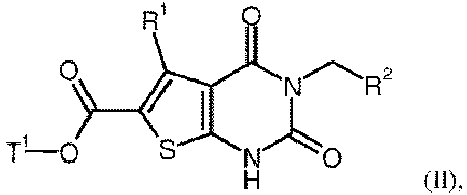
dans laquelle R¹, R² et T¹ ont les significations indiquées précédemment,
est tout d'abord transformé par clivage du radical ester T¹ en l'acide carboxylique de formule (VII) 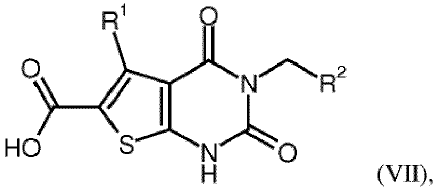
dans laquelle R¹ et R² ont les significations indiquées précédemment,
puis couplé avec activation de la fonction carboxyle avec une amine de formule (VI) 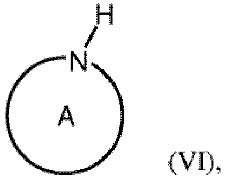
dans laquelle le cycle A a la signification indiquée précédemment,
pour former un composé de formule (VIII) 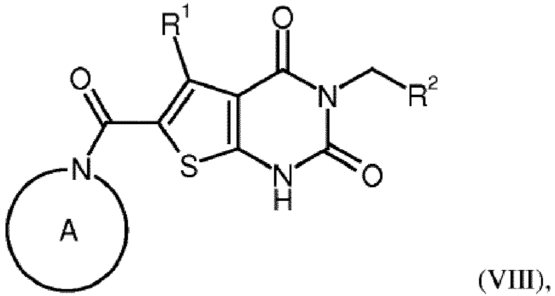
dans laquelle R¹, R² et le cycle A ont les significations indiquées précédemment,
puis celui-ci est alkylé en présence d'une base avec un composé de formule (III) 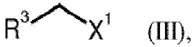
dans laquelle R³ et X¹ ont les significations indiquées précédemment,
soit
[B] un composé N³-protégé de formule (IX) 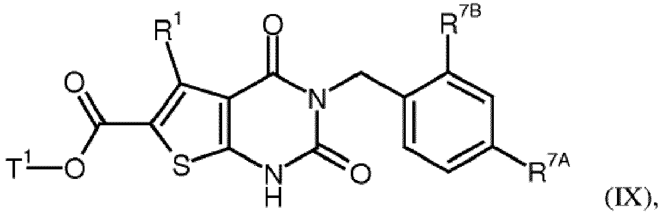
dans laquelle R¹ et T¹ ont les significations indiquées précédemment
et
R^{7A} et R^{7B} représentent indépendamment l'un de l'autre hydrogène ou méthoxy,
est tout d'abord alkylé en présence d'une base avec un composé de formule (III) 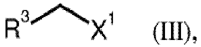
dans laquelle R³ et X¹ ont les significations indiquées précédemment,
pour former un composé de formule (X) 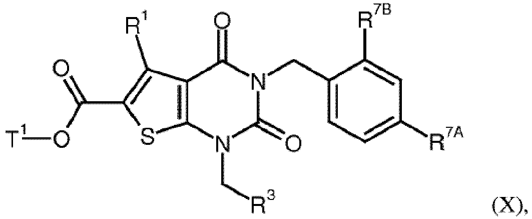
dans laquelle R¹, R³, R^{7A}, R^{7B} et T¹ ont les significations indiquées précédemment,
puis le groupe N³-benzyle et le radical ester T¹ sont clivés simultanément par traitement avec un acide de Lewis fort tel que le trichlorure d'aluminium, puis l'acide carboxylique de formule (XI) ainsi obtenu 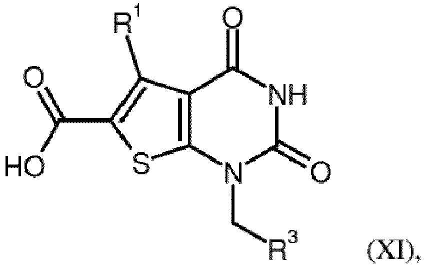
dans laquelle R¹ et R³ ont les significations indiquées précédemment,
est couplé avec activation de la fonction carboxyle avec une amine de formule (VI) 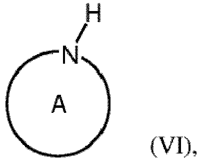
dans laquelle le cycle A a la signification indiquée précédemment,
pour former un composé de formule (XII) 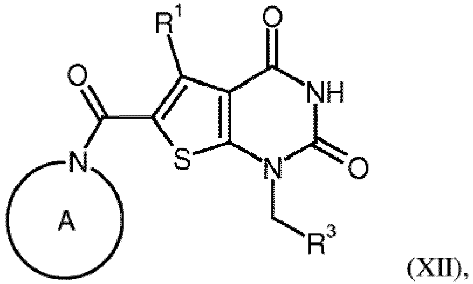
dans laquelle R¹, R³ et le cycle A ont les significations indiquées précédemment,
puis ce dernier est mis en réaction soit (a) en présence d'une base avec un composé de formule (XIII) 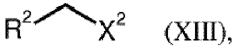
dans laquelle R² a la signification indiquée précédemment,
et
X² représente un groupe partant, tel que par exemple chlore, brome, iode, mésylate, triflate ou tosylate,
soit (b) en présence d'une phosphine appropriée et d'un azodicarboxylate avec un composé de formule (XIV) 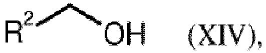
dans laquelle R² a la signification indiquée précédemment,
et les composés de formule (I) ainsi fabriqués sont éventuellement transformés avec (i) les solvants et/ou (ii) les acides appropriés en leurs solvates, sels et/ou solvates des sels.

14. Procédé de fabrication de composés de formule (II-C) 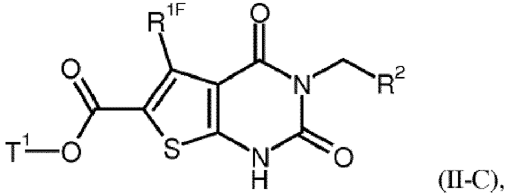
dans laquelle R² a la signification indiquée dans les revendications 1 à 3,
R^{1F} représente difluorométhyle ou trifluorométhyle, et
T¹ représente alkyle en (C₁-C₄) ou benzyle, **caractérisé en ce qu'**un composé de formule (XIX) 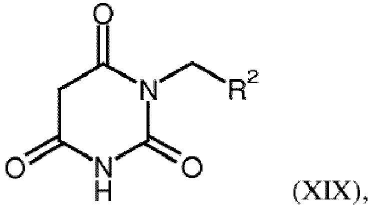
dans laquelle R² a la signification indiquée précédemment,
est transformé avec de l'oxychlorure de phosphore en un composé de formule (XXIV) 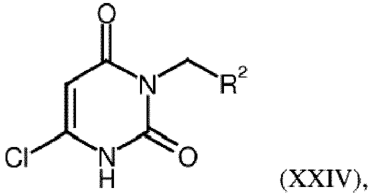
dans laquelle R² a la signification indiquée précédemment,
puis mis en réaction en présence de pyridine en excès avec un anhydride de formule (XXV) 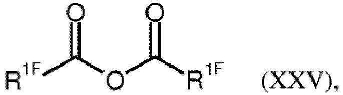
dans laquelle R^{1F} a la signification indiquée précédemment,
pour former un composé de bétaïne de formule (XXVI) 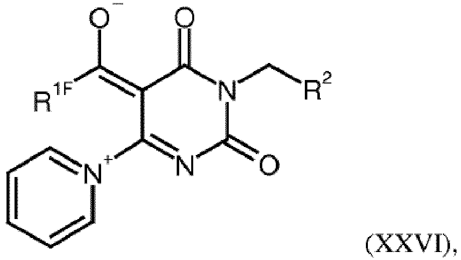
dans laquelle R^{1F} et R² ont les significations indiquées précédemment,
et celui-ci est condensé en présence d'une base avec un ester de l'acide α-mercaptoacétique de formule (XXI) 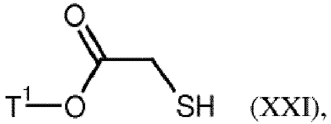
dans laquelle T¹ a la signification indiquée précédemment,
pour former le composé (II-C).

15. Composé, tel que défini dans l'une quelconque des revendications 1 à 12, destiné à une utilisation dans un procédé de traitement et/ou de prévention de maladies.

16. Composé, tel que défini dans l'une quelconque des revendications 1 à 12, destiné à une utilisation dans un procédé de traitement et/ou de prévention de la fibrose pulmonaire idiopathique, de l'hypertension pulmonaire, du syndrome de la bronchiolite oblitérante, de la maladie pulmonaire obstructive chronique, de l'asthme, de la fibrose cystique, de l'infarctus du myocarde, de l'insuffisance cardiaque et de l'anémie à hématies falciformes.

17. Utilisation d'un composé tel que défini dans l'une quelconque des revendications 1 à 12, pour la fabrication d'un médicament pour le traitement et/ou la prévention de la fibrose pulmonaire idiopathique, de l'hypertension pulmonaire, du syndrome de la bronchiolite oblitérante, la maladie pulmonaire obstructive chronique, l'asthme, la fibrose cystique, l'infarctus du myocarde, l'insuffisance cardiaque et l'anémie à hématies falciformes.

18. Médicament contenant un composé, tel que défini dans l'une quelconque des revendications 1 à 12, en combinaison avec un ou plusieurs adjuvants inertes, non toxiques, pharmaceutiquement appropriés.

19. Médicament contenant un composé, tel que défini dans l'une quelconque des revendications 1 à 12, en combinaison avec un ou plusieurs agents actifs supplémentaires choisis dans le groupe constitué par les inhibiteurs de PDE 5, les activateurs de sGC, les stimulateurs de sGC, les analogues de prostacycline, les antagonistes d'endothéline, les agents à effet antifibrotique, les agents à effet anti-inflammatoire, immunomodulateur, immunosuppresseur et/ou cytotoxique, et/ou les composés inhibant la cascade de transduction de signaux.

20. Médicament selon la revendication 18 ou 19, destiné à utilisation dans un procédé de traitement et/ou de prévention de la fibrose pulmonaire idiopathique, de l'hypertension pulmonaire, du syndrome de la bronchiolite oblitérante, de la maladie pulmonaire obstructive chronique, de l'asthme, de la fibrose cystique, de l'infarctus du myocarde, de l'insuffisance cardiaque et de l'anémie à hématies falciformes.
